# EUROPÄISCHE PATENTSCHRIFT

(11) **EP 2 903 440 B1**
(45) Veröffentlichungstag und Bekanntmachung des Hinweises auf die Patenterteilung: **06.09.2017**
(21) Anmeldenummer: 13770492.0
(22) Anmeldetag: 30.09.2013
(51) Int. Cl.: A01N 43/56, A01N 43/58, A01N 43/647, A01N 43/76, A01N 43/78, A01N 43/82, C07D 231/06, C07D 231/22, C07D 231/40, C07D 231/52, C07D 231/56, C07D 249/06, C07D 401/04, C07D 401/12, C07D 403/04, C07D 403/12, C07D 405/12, C07D 409/12, C07D 417/12, A01P 7/00

(54) **HETEROCYCLISCHE VERBINDUNGEN ALS SCHÄDLINGSBEKÄMPFUNGSMITTEL**
HETEROCYCLIC COMPOUNDS AS PEST CONTROLLING AGENTS
COMPOSÉS HÉTÉROCYCLIQUES EN TANT QUE MOYEN DE LUTTE CONTRE LES PARASITES

(30) Priorität: 02.10.2012 EP 12186946; 05.06.2013 EP 13170565
(43) Veröffentlichungstag der Anmeldung: 12.08.2015
(73) Patentinhaber: Bayer CropScience AG, 40789 Monheim am Rhein (DE)
(72) Erfinder: HEILMANN, Eike Kevin, 40229 Düsseldorf (DE); GREUL, Jörg, 51381 Leverkusen (DE); TRAUTWEIN, Axel, 40593 Düsseldorf (DE); SCHWARZ, Hans-Georg, 46282 Dorsten (DE); ADELT, Isabelle, 42781 Haan (DE); ANDREE, Roland, 40764 Langenfeld (DE); LÜMMEN, Peter, 65510 Idstein (DE); HINK, Maike, 71706 Markgröningen (DE); ADAMCZEWSKI, Martin, 51067 Köln (DE); DREWES, Mark, 40764 Langenfeld (DE); BECKER, Angela, 40235 Düsseldorf (DE); VOERSTE, Arnd, 50674 Köln (DE); GÖRGENS, Ulrich, 40882 Ratingen (DE); ILG, Kerstin, 50670 Köln (DE); JANSEN, Johannes-Rudolf, 40789 Monheim (DE); PORTZ, Daniela, 52391 Vettweiß (DE)
(74) Vertreter: BIP Patents
(86) Internationale Anmeldenummer: PCT/EP2013/070371
(87) Internationale Veröffentlichungsnummer: WO 2014/053450

(56) Entgegenhaltungen:
- DD-A1- 159 875
- DE-A1-102008 041 216
- US-A- 4 663 272
- JOANNA MATYSIAK ET AL: "Synthesis and antimycotic activity of N-azolyl-2,4-dihydroxythiobenzamides", BIOORGANIC & MEDICINAL CHEMISTRY, Bd. 11, Nr. 10, 1. Mai 2003 (2003-05-01), Seiten 2285-2291, XP055054430, ISSN: 0968-0896, DOI: 10.1016/S0968-0896(03)00110-X in der Anmeldung erwähnt
- DATABASE CAS REGISTRY [Online] 16. November 1984 (1984-11-16), "Benzamide, 2-chloro-N-[4,5-dihydro-5-oxo-1-(2,4,6-tri chlorophenyl)-1H-pyrazol-3-yl]-5-[(2-methy l-1-oxo-2-propen-1-yl)amino]-", XP002692801, gefunden im STN Database accession no. 86025-05-0
- DATABASE CAS REGISTRY [Online] 16. November 1984 (1984-11-16), "Benzamide, N-[1-(3,4-dichloro-2,5-dimethoxyphenyl)-4, 5-dihydro-5-oxo-1H-pyrazol-3-yl]-2-nitro-" , XP002692793, gefunden im STN Database accession no. 74202-18-9 -& BERGHMANS P ET AL: "Magenta-forming couplers for use in colour photography (P Berghmans, L Luyten, R Van Poucke and Agfa-Gevaert NV, Belgium)", RESEARCH DISCLOSURE, MASON PUBLICATIONS, HAMPSHIRE, GB, Bd. 193, Nr. 11, 1. Mai 1980 (1980-05-01), XP007107216, ISSN: 0374-4353 in der Anmeldung erwähnt
- DATABASE CAS REGISTRY [Online] 29. Juli 2009 (2009-07-29), "Benzamide, N-[1-(2,4-difluorophenyl)-5-methyl-1H-pyra zol-3-yl]-2,4-dimethoxy-", XP002692794, gefunden im STN Database accession no. 1170047-96-7 -& "Amb10178760", , 1. Juli 2009 (2009-07-01), Seiten 1-1, XP055054696, Gefunden im Internet: URL:http://www.ambinter.com/reference/1017 8760 [gefunden am 2013-02-27]
- DATABASE REGISTRY; CAS [Online] "Benzamide, 2-chloro-N-[4,5-dihydro-5-oxo-1-(2,4,6-tri chlorophenyl)-1H-pyrazol-3-yl]-5-[(1-oxo-2 -propen-1-yl)amino]-", XP002692791, retrieved from REGISTRY; STN Database accession no. 101750-33-8

## Beschreibung

Die vorliegende Anmeldung betrifft die nicht-therapeutische Verwendung heterocyclischer Verbindungen zur Bekämpfung von tierischen Schädlingen, zu denen Arthropoden, Insekten und Nematoden zählen, neue heterocyclische Verbindungen, Verfahren zu deren Herstellung sowie Zwischenprodukte zur Herstellung der heterocyclischen Verbindungen.

Die heterocyclischen Verbindungen der Formeln (W) und (W2) sind bekannt (vgl. für die Verbindungen der Formel (W) Registry Numbers 1189645-25-7, 1189474-83-6, 1193202-69-5, 1172407-07-6, 1185158-40-0, 1185036-12-7, 1170986-74-9, 1193179-17-7, 1189458-68-1, 1189956-23-7, 1189915-26-1, 1170047-96-7). Eine Verwendung für diese Verbindungen wurde nicht angegeben.

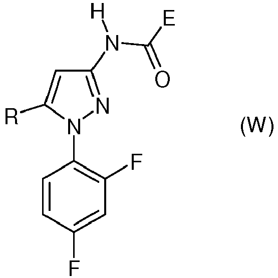

| Nr. | R | E |
|---|---|---|
| W-1 | Ethyl | 2-(Trifluormethyl)phenyl |
| W-2 | Ethyl | 2-Bromphenyl |
| W-3 | Ethyl | 2-Fluorphenyl |
| W-4 | Ethyl | 3-Methyl-2-thienyl |
| W-5 | Ethyl | 2,5-Dimethyl-3-furanyl |
| W-6 | H | 2,4-Dimethoxyphenyl |
| W-7 | H | 2-(Trifluormethyl)phenyl |
| W-8 | H | 2,4-Difluorphenyl |
| W-9 | Methyl | 2-Bromphenyl |
| W-10 | Ethyl | 2,4-Difluorphenyl |
| W-11 | H | 2,5-Dimethyl-3-furanyl |
| W-12 | Methyl | 2,4-Dimethoxyphenyl |

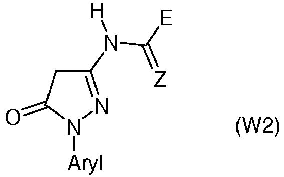

| Nr. | CAS-No. | Referenz | Z | E | Aryl |
|---|---|---|---|---|---|
| W2-1 | 74202-18-9 | Research Disclosure 1980, 193011,165.) | O | 2-nitrophenyl | 3,4-Dichlor-2,5-dimethoxyphenyl |
| W2-2 | 612092-81-6 | Bioorg. Med. Chem. 2003, 2285. | S | 2,4-Dihydroxyphenyl | 2,4,6-Trichlorphenyl |
| W2-3 | 111243-44-8 | JP 62-090657 | O | 2-Chlor-4-aminophenyl | 2,4,6-Trichlorphenyl |
| W2-4 | 101750-33-8 | JP 60-218646 | O | 2-Chlor-5-(acryloylamino)phenyl | 2,4,6-Trichlorphenyl |
| W2-5 | 87756-39-6 | DE 3226163 | O | 2-Chlor-5-aminophenyl | 2,4,6-Trichlorphenyl |
| W2-6 | 87756-30-7 | DD 159875 | O | 2-Chlor-5-nitrophenyl | 2,4,6-Trichlorphenyl |
| W2-7 | 86025-05-0 | DE 3226163 | O | 2-Chlor-5-(methacryloylamino)phenyl | 2,4,6-Trichlorphenyl |

In DE 10 2008 041216 A1 werden insektizide Indol-Carboxamide offenbart.

Derivate von Pyrazol-4-carboxamiden sind aus JP 2010-202649 bekannt. Die dort beschriebenen Verbindungen weisen eine bakterizide Wirkung gegen pflanzenschädigende Bakterien auf. Zwischenprodukte für ihre Herstellung sind in JP 2010-202648 beschrieben.

Pflanzenschutzmittel, zu denen auch Schädlingsbekämpfungsmittel gehören, müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch die nicht-therapeutische Verwendung von neuen und bekannten Verbindungen der Formel (I) 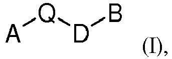 worin
A für einen Rest aus der Reihe 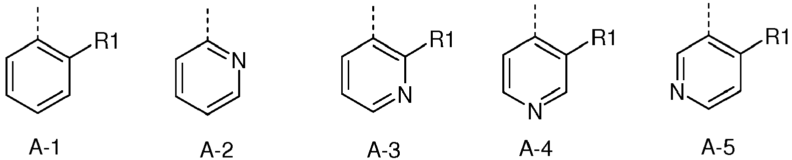 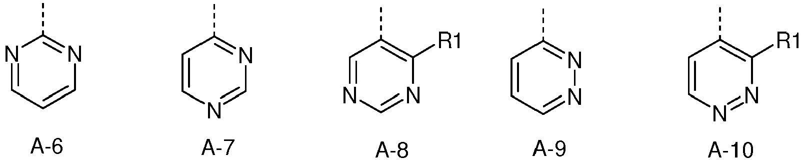 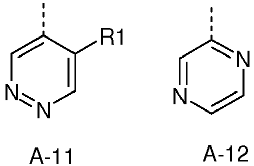 steht, in dem die gestrichelte Linie die Bindung zu Q bedeutet und worin A weiterhin m Substituenten R2 trägt,
Q für einen Rest aus der Reihe 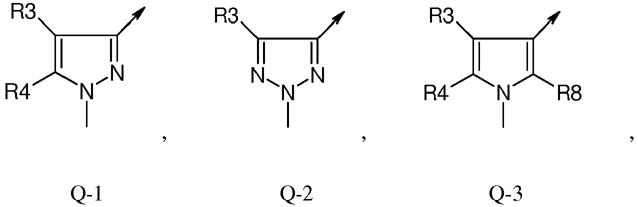 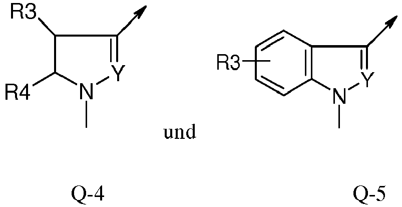 steht, worin der Stickstoff mit dem Ring A verknüpft ist und der Pfeil jeweils die Bindung zu D bedeutet, und
D für den Rest der Formel 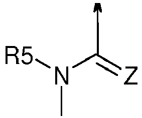 steht, worin der Stickstoff mit Q verbunden ist und der Pfeil die Bindung zu B bedeutet,
B für einen Rest aus der Reihe 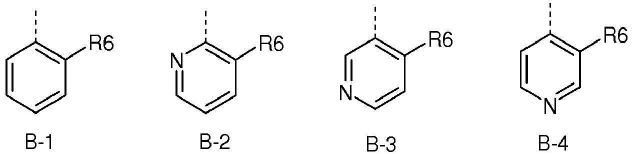 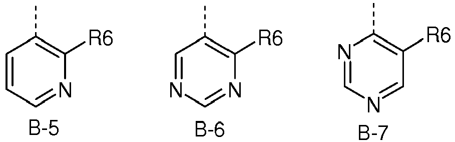 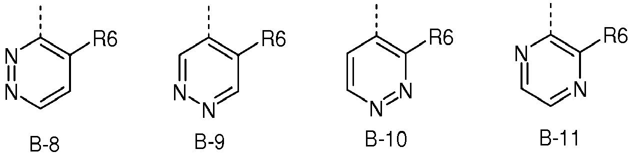 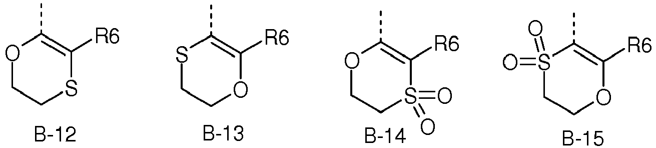 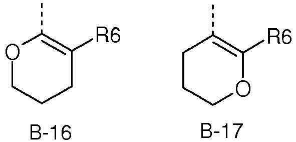 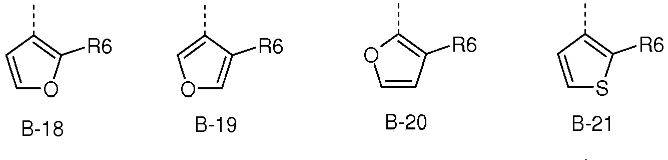 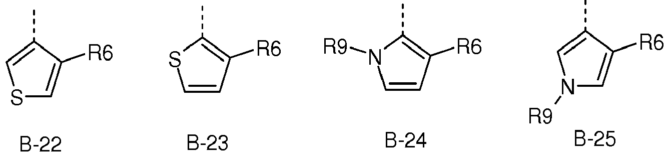 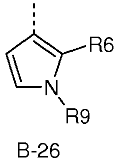 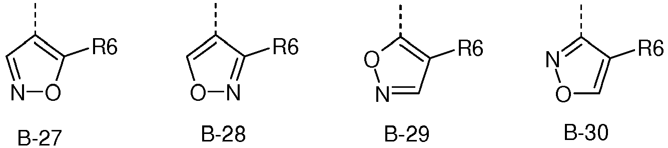 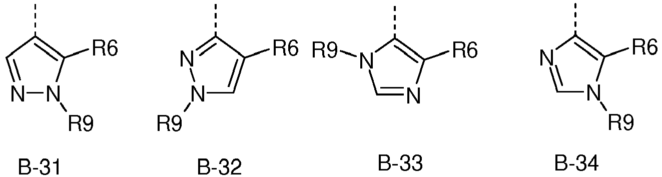 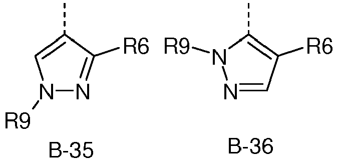 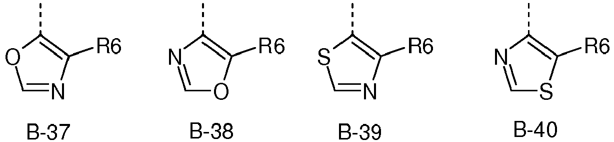 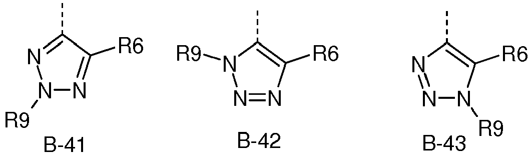 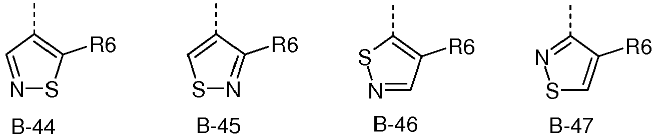 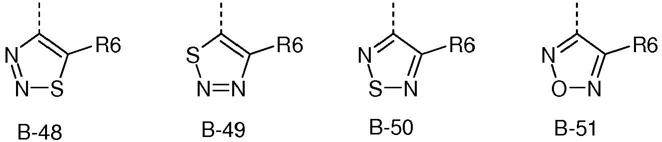 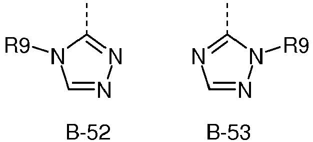 steht, worin die gestrichelte Linie die Bindung zu D bedeutet und wobei B weiterhin n Substituenten R7 trägt,
Y im Rest Q-4 für CR8 oder für Stickstoff steht,
Y im Rest Q-5 für Stickstoff steht,
Z für Sauerstoff oder Schwefel steht,
R1 für einen Rest aus der Reihe Halogen, Cyano, Nitro, Amino, Hydroxy, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, C₄-C₁₂-Bicycloalkyl, Aryl, Aryloxy, Arylamino, Arylthio, Heteroaryl, Heteroaryloxy, Heteroarylamino und Heteroarylthio steht, wobei die Substituenten unabhängig voneinander ausgewählt sind aus Halogen, Cyano, Nitro, Hydroxy, Amino, C₁-C₆-Alkoxy, C₃-C₆-Cycloalkyl, C₁-C₆-Halogenalkoxy, C₁-C₆-Alkylthio, Aryl, Aryloxy, Arylthio, Heteroaryl, Heteroaryloxy und Heteroarylthio,
R2 für einen Rest aus der Reihe Halogen, Cyano, Nitro, Amino, Hydroxy, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenoxy, C₃-C₆-Alkinoxy, C₃-C₆-Cycloalkoxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, Di-(C₁-C₆-alkyl)-amino, C₃-C₆-Alkenylamino, C₃-C₆-Alkinylamino, C₃-C₆-Cycloalkylamino, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, C₄-C₁₂-Bicycloalkyl, Aryl, Aryloxy, Arylamino, Arylthio, Heteroaryl, Heteroaryloxy, Heteroarylamino und Heteroarylthio steht, wobei die Substituenten unabhängig voneinander ausgewählt sind aus Halogen, Cyano, Nitro, Hydroxy, Amino, C₁-C₆-Alkyl, C₁-C₆-Alkoxy, C₃-C₆-Cycloalkyl, C₁-C₆-Halogenalkoxy und C₁-C₆-Alkylthio, Aryl, Aryloxy, Arylthio, Heteroaryl, Heteroaryloxy und Heteroarylthio,
R3 für einen Rest aus der Reihe Wasserstoff, Halogen, Cyano, Nitro, Amino, Hydroxy, C₁-C₆-Alkyl, C₁-C₆-Alkoxy, C₁-C₆-Halogenalkyl und C₁-C₆-Halogenalkoxy steht,
R4 für einen Rest aus der Reihe Wasserstoff, Halogen, Amino, Hydroxy, C₁-C₆-Alkyl, C₁-C₆-Alkoxy, C₁-C₆-Halogenalkyl undC₁-C₆-Halogenalkoxy steht,
R5 für einen Rest aus der Reihe Wasserstoff, C₁-C₆-Alkyl, C₃-C₆-Cycloalkyl, C₁-C₆-Halogenalkyl, C₂-C₆-Alkenyl, C₃-C₆-Alkinyl, C₃-C₆-Cycloalkyl-C₁-C₆-alkyl, Cyano-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl und C₁-C₆-Halogenalkylsulfonyl oder für C(=O)-B steht,
R6 für einen Rest aus der Reihe Wasserstoff (nur in den Resten B-26, B-33, B-36 und B-42), Halogen, Cyano, Nitro, Amino, Hydroxy, Carboxyl, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenoxy, C₃-C₆-Alkinoxy, C₃-C₆-Cycloalkoxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₁-C₆-Alkylsulfinyl, C₁-C₆-Alkylsulfonyl, C₁-C₆-Alkylcarbonyl, C₁-C₆-Alkoxyimino-C₁-C₆₋alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Halogenalkylcarbonylamino, C₁-C₆-Alkylthiocarbonylamino, C₄-C₁₂-Bicycloalkyl, Aryl, Aryloxy, Heteroaryl, Heteroaryloxy steht, wobei die Substituenten unabhängig voneinander ausgewählt sind aus Halogen, Cyano, Nitro, Hydroxy, Amino, C₁-C₆-Alkyl, C₁-C₆-Alkoxy, C₃-C₆-Cycloalkyl, C₁-C₆-Halogenalkoxy und C₁-C₆-Alkylthio,
R7 für einen Rest aus der Reihe Halogen, Nitro, Cyano, Amino, Hydroxy, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenoxy, C₃-C₆-Alkinoxy, C₃-C₆-Cycloalkoxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl, Heteroaryloxy, C₄-C₁₂-Bicycloalkyl steht, wobei die Substituenten unabhängig voneinander ausgewählt sind aus Halogen, Cyano, Nitro, Hydroxy, Amino, C₁-C₆-Alkyl, C₁-C₆-Alkoxy, C₃-C₆-Cycloalkyl, C₁-C₆-Halogenalkoxy und C₁-C₆-Alkylthio,
R8 für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy steht,
R9 für einen Rest aus der Reihe Wasserstoff, C₁-C₆-Alkyl, C₃-C₆-Cycloalkyl, Halogenalkyl, C₃-C₆-Alkenyl, C₃-C₆-Alkinyl, C₃-C₆-Cycloalkyl-C₁-C₆-alkyl, Cyano-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl und C₁-C₆-Halogengenalkylsufonyl steht,
m für eine Zahl aus der Reihe 0, 1, 2 und 3 steht, wobei für m > 1 die Reste R2 gleich oder verschieden sein können und
n für eine Zahl aus der Reihe 0, 1, 2 und 3 steht, wobei für n > 1 die Reste R7 gleich oder verschieden sein können,
   zur Bekämpfung von tierischen Schädlingen.

Es wurde gefunden, dass die bekannten und die neuen Verbindungen der Formel (I) stark ausgeprägte biologische Eigenschaften besitzen und vor allem zur nicht-therapeutischen Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die Verwendung der reinen Isomeren als auch die der Isomerengemische.

Bevorzugte Substituenten bzw. Bereiche der in den Verbindungen der Formel (I) aufgeführten Reste werden im Folgenden erläutert.
A steht für einen Rest aus der Reihe 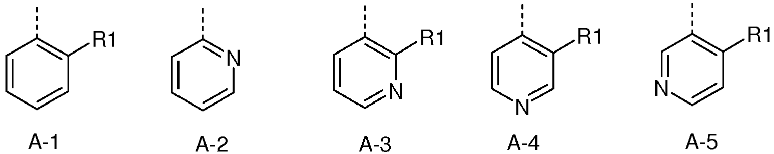 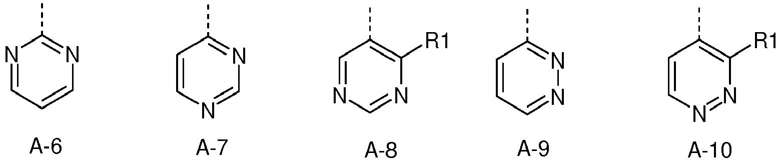 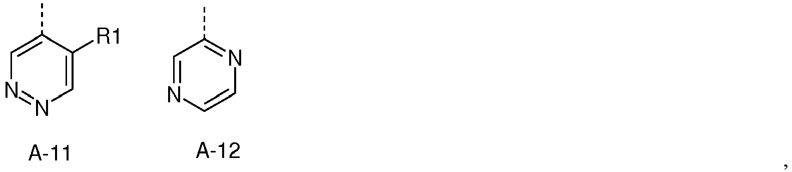 in dem die gestrichelte Linie die Bindung zu Q bedeutet und wobei A weiterhin m Substituenten R2 trägt.
Q steht für einen Rest aus der Reihe 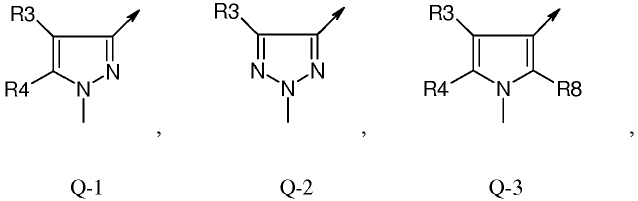 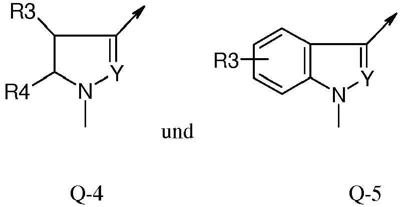 worin der Stickstoff mit dem Ring A verknüpft ist und der Pfeil jeweils die Bindung zu D bedeutet.
D steht für den Rest der Formel 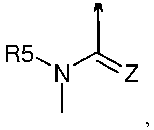 worin der Stickstoff mit Q verbunden ist und der Pfeil die Bindung zu B bedeutet.
B steht für einen Rest aus der Reihe 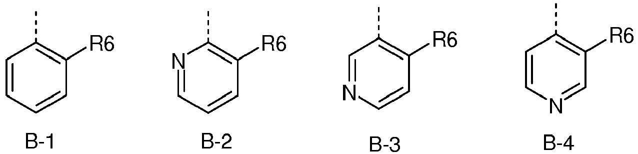 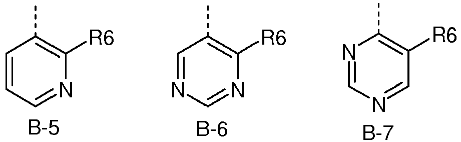 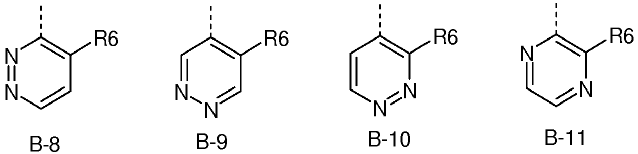 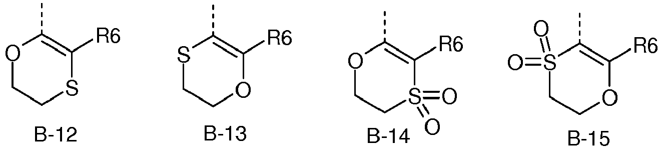 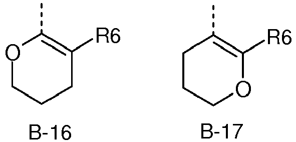 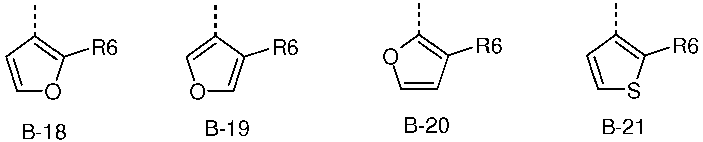 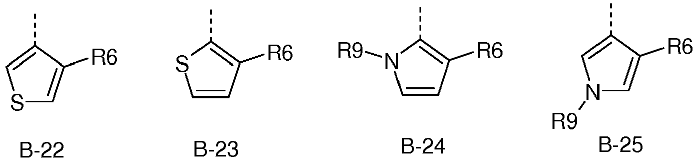 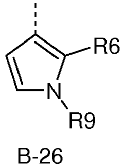 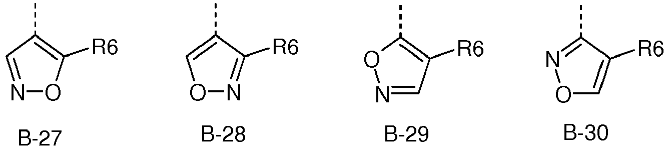 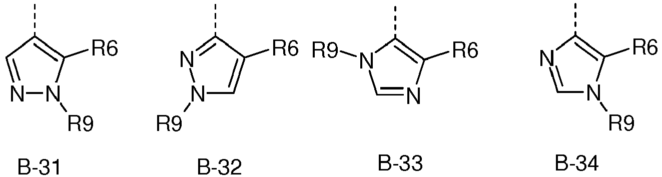 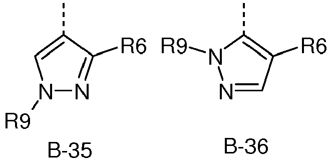 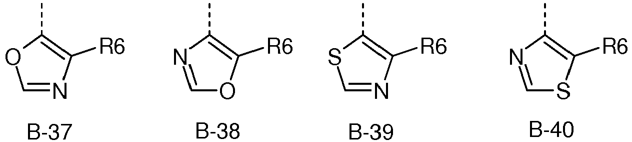 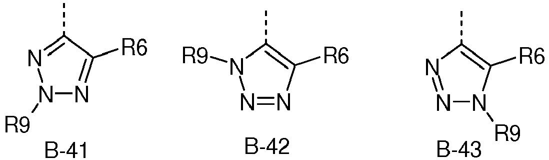 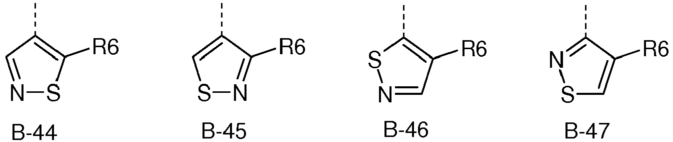 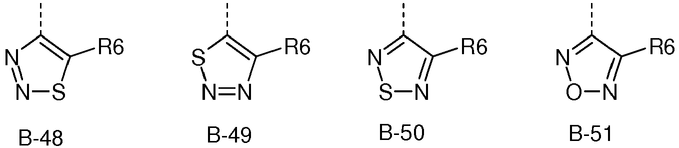 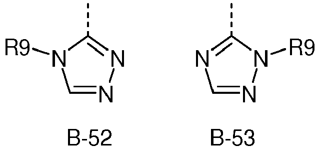 worin die gestrichelte Linie die Bindung zu D bedeutet und wobei B weiterhin n Substituenten R7 trägt.
Y steht im Rest Q-4 für CR8 oder für Stickstoff.
Y steht im Rest Q-5 für Stickstoff.
Z steht für Sauerstoff oder Schwefel.
R1 steht für einen Rest aus der Reihe Halogen, Cyano, Nitro, Amino, Hydroxy, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C6-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl und Heteroaryloxy.
R2 steht für einen Rest aus der Reihe Halogen, Cyano, Nitro, Amino, Hydroxy, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, Di-(C₁-C₆-alkyl)-amino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl und Heteroaryloxy.
R3 steht für einen Rest aus der Reihe Wasserstoff, Halogen, Cyano, Nitro, Amino, C₁₋C₆-Alkyl und C₁-C₆-Halogenalkyl.
R4 steht für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy.
R5 steht für einen Rest aus der Reihe Wasserstoff, C₁-C₆-Alkyl, C₁-C₆-Halogenalkyl, Cyano-C₁-C₆-alkyl, C₂-C₆-Alkenyl, C₃-C₆-Alkinyl, C₃-C₆-Cycloalkyl, C₃-C₆-Cycloalkyl-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl, C₁-C₆-Halogengenalkylsufonyl und C(=O)-B.
R6 steht für einen Rest aus der Reihe Wasserstoff (nur in den Resten B-26, B-33, B-36 und B-42), Halogen, Cyano, Nitro, Amino, Hydroxy, Carboxyl, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₄-Halogenalkylcarbonylamino, C₁-C₆-Alkylthiocarbonylamino, Aryl, Aryloxy, Heteroaryl und Heteroaryloxy.
R7 steht für einen Rest aus der Reihe Halogen, Cyano, Nitro, Amino, Hydroxy, C₁-C₆-Alkyl, C₁-C₆-Halogenalkyl, Cyano-C₁-C₆-alkyl, C₂-C₆-Alkenyl, C₃-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl und Heteroaryloxy.
R8 steht für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy.
R9 steht für einen Rest aus der Reihe Wasserstoff, C₁-C₆-Alkyl, C₃-C₆-Cycloalkyl, C₁-C₆-Halogenalkyl, C₃-C₆-Alkenyl, C₃-C₆-Alkinyl, C₃-C₆-Cycloalkyl-C₁-C₆-alkyl, Cyano-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl und C₁-C₆-Halogengenalkylsufonyl.
m steht für eine Zahl aus der Reihe 0, 1, 2 und 3, wobei für m > 1 die Reste R2 gleich oder verschieden sein können.
n steht für eine Zahl aus der Reihe 0, 1, 2 und 3, wobei für n > 1 die Reste R7 gleich oder verschieden sein können.

Besonders bevorzugte Substituenten bzw. Bereiche der in den Verbindungen der Formel (I) aufgeführten Reste werden im Folgenden erläutert.
A steht für einen Rest aus der Reihe 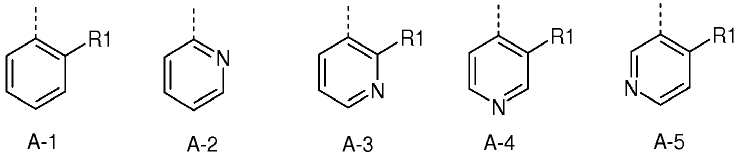 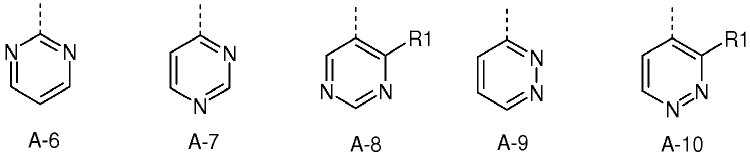 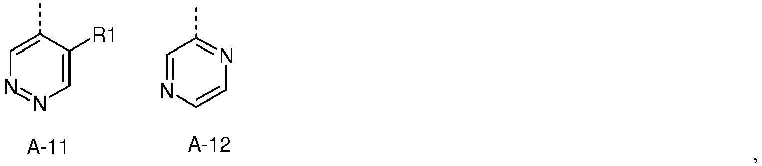 in dem die gestrichelte Linie die Bindung zu Q bedeutet und wobei A weiterhin m Substituenten R2 trägt.
Q steht für einen Rest aus der Reihe 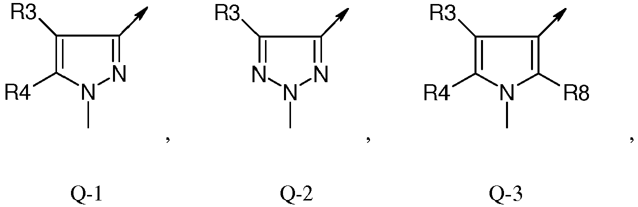 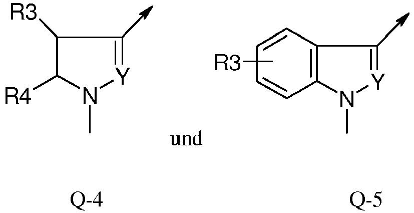 worin der Stickstoff mit dem Ring A verknüpft ist und der Pfeil jeweils die Bindung zu D bedeutet.
D steht für den Rest der Formel 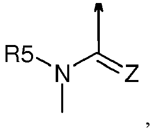 worin der Stickstoff mit Q verbunden ist und der Pfeil die Bindung zu B bedeutet.
B steht für einen Rest aus der Reihe 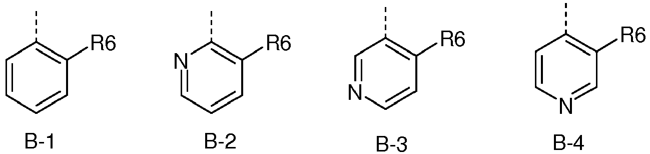 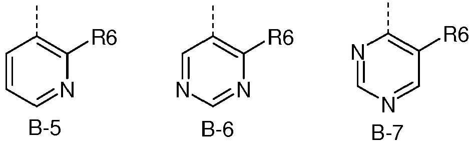 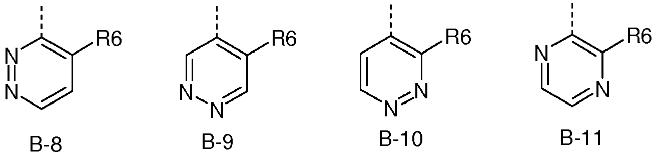 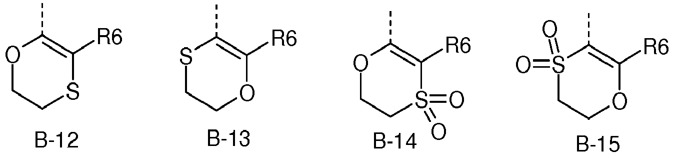 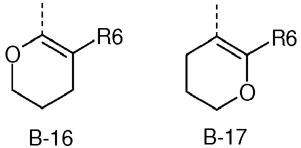 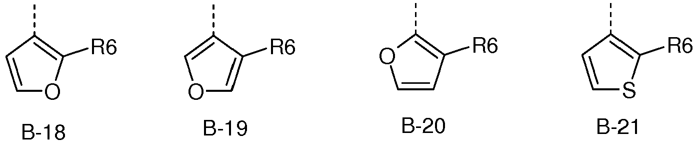 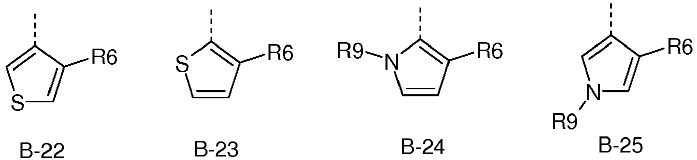 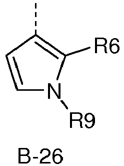 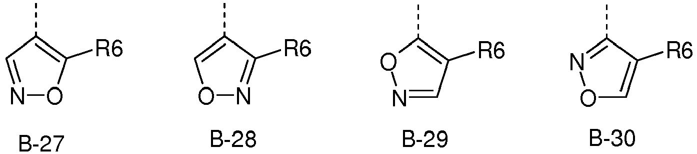 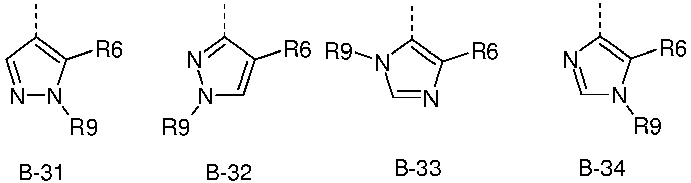 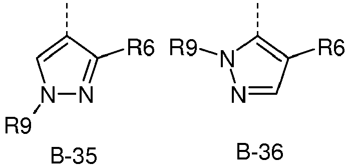 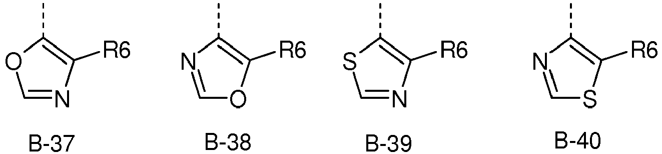 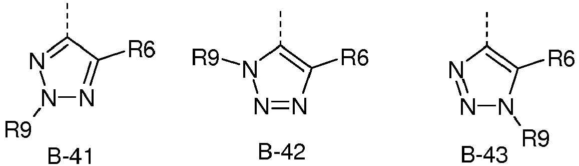 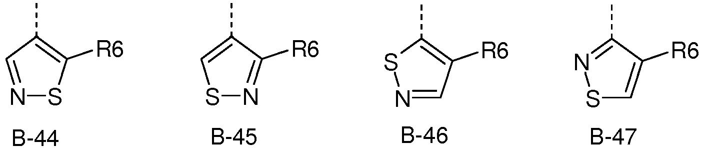 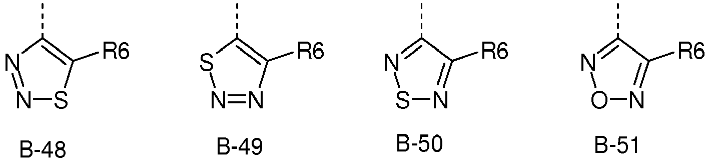 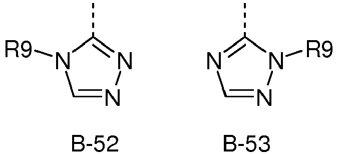 worin die gestrichelte Linie die Bindung zu D bedeutet und wobei B weiterhin n Substituenten R7 trägt.
Y steht im Rest Q-4 für CR8 oder für Stickstoff.
Y steht im Rest Q-5 für Stickstoff.
Z steht für Sauerstoff oder Schwefel.
R1 steht für einen Rest aus der Reihe Halogen, Cyano, Nitro, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylthio, C₁-C₆-Halogenalkylthio, C₁-C₆-Alkylsulfinyl, C₁-C₆-Alkylsulfonyl, C₁-C₆-Alkoxyimino-C₁-C₆-alkyl, Aryl, Aryloxy, Heteroaryl und Heteroaryloxy.
R2 steht für einen Rest aus der Reihe Halogen, Cyano, Nitro, Amino, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylthio, C₁-C₆-Halogenalkylthio, C₁-C₆-Alkylsulfinyl, C₁-C₆-Alkylsulfonyl, C₁-C₆-Alkylcarbonyl, Di-(C₁-C₆-alkyl)-amino, C₁-C₆-Alkoxyimino-C₁-C₆-alkyl, Aryl, Aryloxy, Heteroaryl und Heteroaryloxy.
R3 steht für einen Rest aus der Reihe Wasserstoff, Halogen, Cyano, Nitro, Amino, C₁-C₆-Alkyl und C₁-C₆-Halogenalkyl.
R4 steht für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy.
R5 steht für einen Rest aus der Reihe Wasserstoff, C₁-C₆-Alkyl, C₁-C₆-Halogenalkyl, Cyano-C₁-C₆-alkyl, C₂-C₆-Alkenyl, C₃-C₆-Alkinyl, C₃-C₆-Cycloalkyl, C₃-C₆-Cycloalkyl-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl, C₁-C₆-Halogengenalkylsufonyl und C(=O)-B.
R6 steht für einen Rest aus der Reihe Wasserstoff (nur in den Resten B-26, B-33, B-36 und B-42), Halogen, Cyano, Nitro, Hydroxy, Carboxyl, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylsulfinyl, C₁-C₆-Alkylthio, C₁-C₆-Halogenalkylthio, C₁-C₆-Alkylsulfonyl, C₁-C₆-Alkylcarbonyl, C₁-C₆-Alkoxyimino-C₁-C₆-alkyl, C₁-C₆-Alkylcarbonylamino, C₁-C₆-Halogenalkylcarbonylamino, Aryl, Aryloxy, Heteroaryl und Heteroaryloxy.
R7 steht für einen Rest aus der Reihe Halogen, Cyano, Nitro, Hydroxy, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylthio, C₁-C₆-Halogenalkylthio, C₁-C₆-Alkylsulfinyl, C₁-C₆-Alkylsulfonyl, C₁-C₆-Alkoxyimino-C₁-C₆-alkyl, Aryl, Aryloxy, Heteroaryl und Heteroaryloxy.
R8 steht für einen Rest aus der Reihe Wasserstoff, C₁-C₆-Alkyl, C₁-C₆-Alkoxy, C₁-C₆-Halogenalkyl und C₁-C₆-Halogenalkoxy.
R9 steht für einen Rest aus der Reihe Wasserstoff, C₁-C₆-Alkyl, C₃-C₆-Cycloalkyl und C₁-C₆-Halogenalkyl.
m steht für eine Zahl aus der Reihe 0, 1, 2 und 3, wobei für m > 1 die Reste R2 gleich oder verschieden sein können.
n steht für eine Zahl aus der Reihe 0, 1, 2 und 3, wobei für n > 1 die Reste R7 gleich oder verschieden sein können.

Ganz besonders bevorzugte Substituenten bzw. Bereiche der in den Verbindungen der Formel (I) aufgeführten Reste werden im Folgenden erläutert.
A steht für einen Rest aus der Reihe 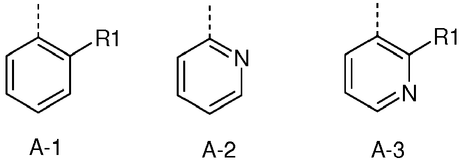 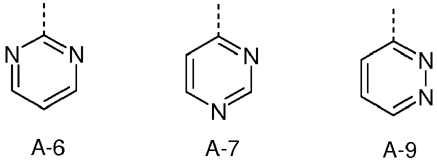 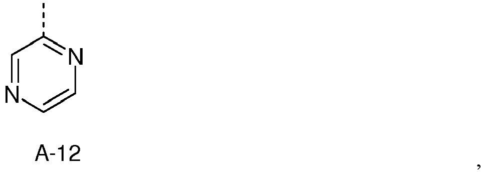 in dem die gestrichelte Linie die Bindung zu Q bedeutet und wobei A weiterhin m Substituenten R2 trägt.
Q steht für einen Rest aus der Reihe 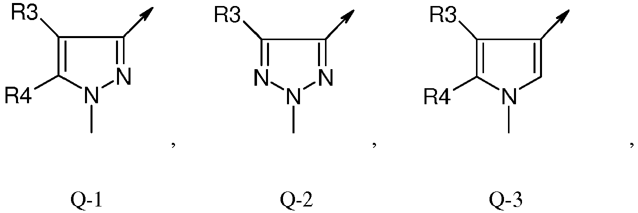 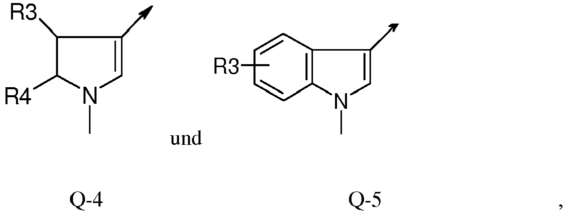 worin der Stickstoff mit dem Ring A verknüpft ist und der Pfeil jeweils die Bindung zu D bedeutet.
D steht für den Rest der Formel 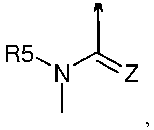 worin der Stickstoff mit Q verbunden ist und der Pfeil die Bindung zu B bedeutet.
B steht für einen Rest aus der Reihe 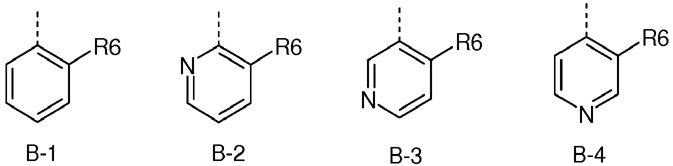 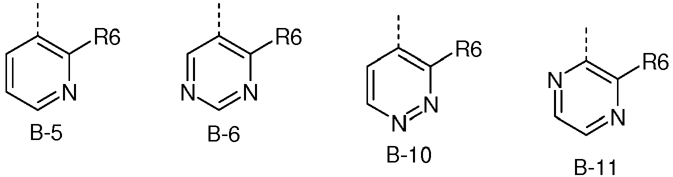 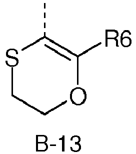 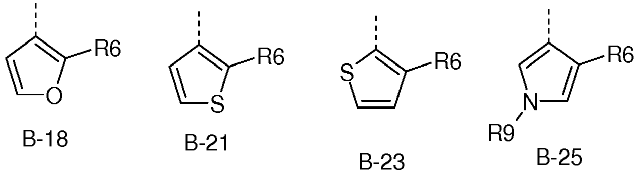 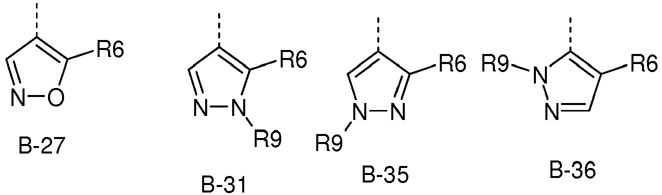 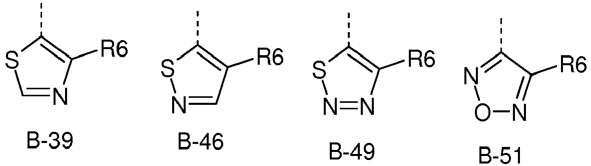 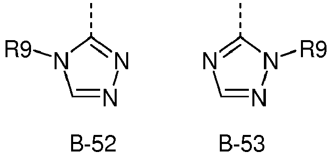 worin die gestrichelte Linie die Bindung zu D bedeutet und wobei B weiterhin n Substituenten R7 trägt.
Z steht für Sauerstoff oder Schwefel.
R1 steht für einen Rest aus der Reihe Halogen, Cyano, Nitro, C₁-C₄-Alkyl, C₁-C₄-Halogenalkyl, C₁-C₄-Alkoxy und C₁-C₄-Alkylsulfonyl.
R2 steht für einen Rest aus der Reihe Halogen, C₁-C₄-Alkyl und C₁-C₄-Alkoxy.
R3 steht für einen Rest aus der Reihe Wasserstoff und Halogen.
R4 steht für einen Rest aus der Reihe Wasserstoff und C₁-C₄-Alkyl.
R5 steht für einen Rest aus der Reihe Wasserstoff, C₁-C₄-Alkyl, C₃-C₄-Alkenyl, C₃-C₄-Alkinyl, C₃-C₆-Cycloalkyl-C₁-C₄-alkyl, C₁-C₄-Alkylcarbonyl, Cyano-C₁-C₄-alkyl und C(=O)-B.
R6 steht für einen Rest aus der Reihe Halogen, Nitro, Hydroxy, C₁-C₄-Alkyl, C₁-C₄-Alkoxy, C₁-C₄-Alkylthio, C₁-C₄-Alkylsulfonyl, C₁-C₄-Halogenalkyl, C₁-C₄-Halogenalkoxy, C₁-C₄-Halogenalkylthio und Heteroaryl.
R7 steht für einen Rest aus der Reihe Halogen, C₁-C₄-Alkyl und C₁-C₄-Halogenalkyl.
R9 steht für einen Rest aus der Reihe C₁-C₄-Alkyl.
m steht für eine Zahl aus der Reihe 0, 1, 2 und 3, wobei für m > 1 die Reste R2 gleich oder verschieden sein können.
n steht für eine Zahl aus der Reihe 0 und 1.

Eine weitere Gruppe ganz besonders bevorzugter Substituenten bzw. Bereiche der in den Verbindungen der Formel (I) aufgeführten Reste wird im Folgenden erläutert.
A steht für einen Rest aus der Reihe 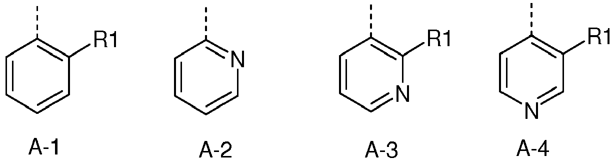 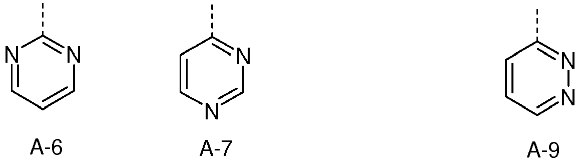 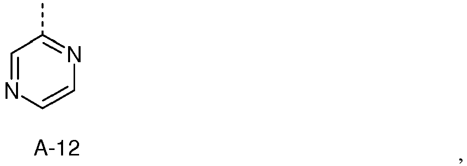 in dem die gestrichelte Linie die Bindung zu Q bedeutet und wobei A weiterhin m Substituenten R2 trägt.
Q steht für einen Rest aus der Reihe 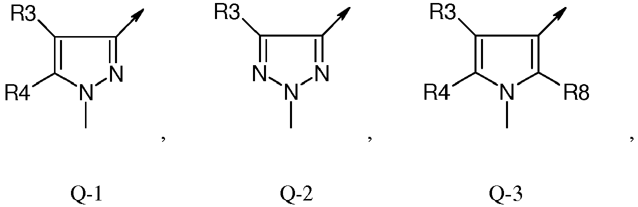 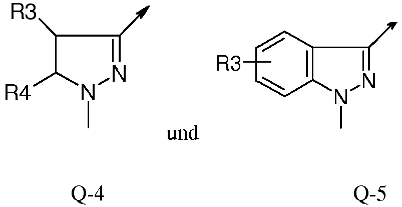 worin der Stickstoff mit dem Ring A verknüpft ist und der Pfeil jeweils die Bindung zu D bedeutet.
D steht für den Rest der Formel 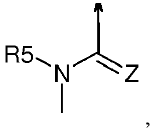 worin der Stickstoff mit Q verbunden ist und der Pfeil die Bindung zu B bedeutet.
B steht für einen Rest aus der Reihe 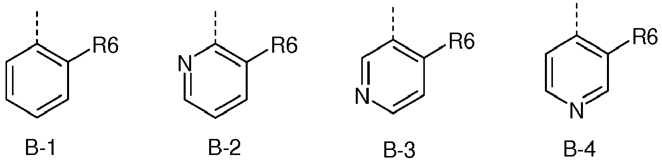 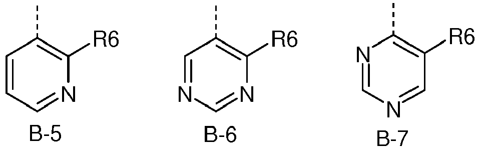 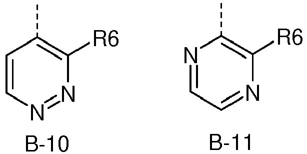 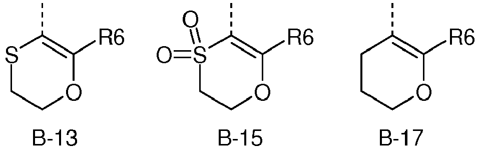 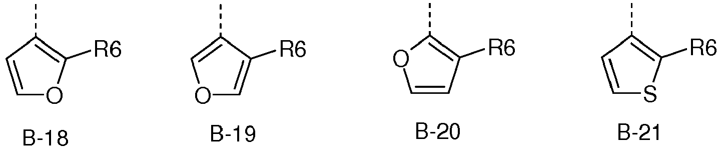 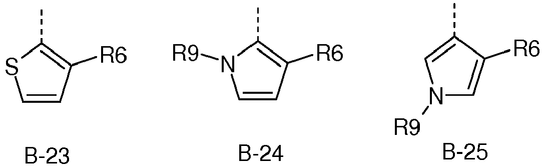 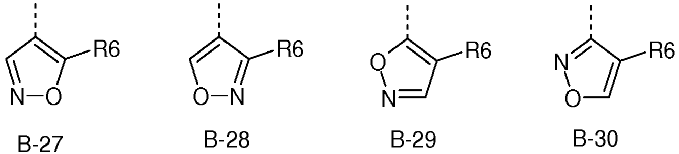 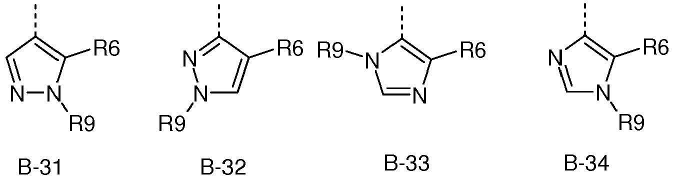 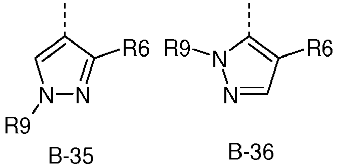 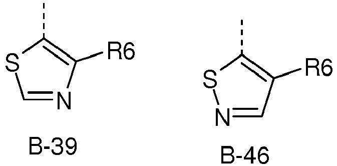 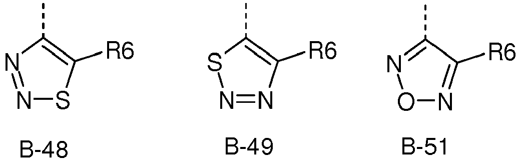 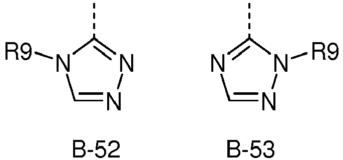 worin die gestrichelte Linie die Bindung zu D bedeutet und wobei B weiterhin n Substituenten R7 trägt.
Z steht für Sauerstoff oder Schwefel.
R1 steht für einen Rest aus der Reihe Halogen, Cyano, Nitro, C₁-C₄-Alkyl, C₁-C₄-Halogenalkyl, C₁-C₄-Alkoxy und C₁-C₄-Alkylsulfonyl.
R2 steht für einen Rest aus der Reihe Halogen, Cyano, Nitro, Amino, C₁-C₄-Alkyl, C₁-C₄-Halogenalkyl, C₃-C₆-Cycloalkyl, C₁-C₄-Alkoxy, Di-(C₁-C₆-alkyl)-amino, Acetyl, Aryl.
R3 steht für einen Rest aus der Reihe Wasserstoff, Halogen, Cyano, Nitro, C₁-C₄-Alkyl.
R4 steht für einen Rest aus der Reihe Wasserstoff, Amino, C₁-C₄-Alkyl.
R5 steht für einen Rest aus der Reihe Wasserstoff, C₁-C₄-Alkyl, C₃-C₄-Alkenyl, C₃-C₄-Alkinyl, C₃-C₆-Cycloalkyl-C₁-C₄-alkyl, C₁-C₄-Alkylcarbonyl, Cyano-C₁-C₄-alkyl und C(=O)-B.
R6 steht für einen Rest aus der Reihe Wasserstoff (nur in den Resten B-33 und B-36), Halogen, Cyano, Nitro, Hydroxy, Carboxyl, C₁-C₄-Alkyl, C₁-C₄-Alkoxy, C₁-C₄-Alkylthio, C₁-C₄-Alkylsulfonyl, C₁-C₄-Halogenalkyl, C₃-C₆-Cycloalkyl, C₁-C₄-Halogenalkoxy, C₁-C₄-Halogenalkylthio, Acetyl, C₁-C₄-Alkylcarbonylamino, C₁-C₄-Halogenalkylcarbonylamino und Heteroaryl.
R7 steht für einen Rest aus der Reihe Halogen, Cyano, Nitro, Hydroxy, C₁-C₄-Alkyl, C₁-C₄-Halogenalkyl und C₁-C₄-Alkoxy.
R9 steht für einen Rest aus der Reihe C₁-C₄-Alkyl und C₁-C₄-Halogenalkyl.
m steht für eine Zahl aus der Reihe 0, 1, 2 und 3, wobei für m > 1 die Reste R2 gleich oder verschieden sein können.
n steht für eine Zahl aus der Reihe 0, 1, 2 und 3, wobei für n > 1 die Reste R7 gleich oder verschieden sein können.

In den bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Heteroaryloxy) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl und Pyrazinyl.

In den besonders bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Het(ero)aryl (auch als Teil einer größeren Einheit, wie beispielsweise Heteroaryloxy) ausgewählt aus der Reihe Furyl, Thienyl, Pyrrolyl, Pyrazolyl, Imidazolyl, Oxadiazolyl, 1,2,3-Triazolyl, 1,2,4-Triazolyl, Oxazolyl, Isoxazolyl und Thiazolyl.

In den ganz besonders bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom, und
Het(ero)aryl (auch als Teil einer größeren Einheit, wie beispielsweise Heteroaryloxy) steht für 1,2,4-Triazolyl.

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt.

Erfindungsgemäß besonders bevorzugt verwendet werden Verbindungen der Formel (I), in welchen eine Kombination der vorstehend als besonders bevorzugt aufgeführten Bedeutungen vorliegt.

Erfindungsgemäß ganz besonders bevorzugt verwendet werden Verbindungen der Formel (I), in welchen eine Kombination der vorstehend als ganz besonders bevorzugt aufgeführten Bedeutungen vorliegt.

Gegenstand der vorliegenden Erfindung sind auch neue Verbindungen der Formel (I), in welchen A, Q, D und B die oben genannten Bedeutungen haben, ausgenommen die oben beschriebenen Verbindungen W und W2, insbesondere W-1 bis W-12, W2-1 bis W2-3, W2-5 und W2-6. Die oben aufgeführten allgemeinen oder in Vorzugsbereichen aufgeführten Restedefinitionen bzw. Erläuterungen gelten auch für die neuen Verbindungen der Formel (I). Wenn in der vorliegenden Anmeldung von erfindungsgemäß zu verwendenden Verbindungen die Rede ist, sind damit, sofern nichts anderes gesagt wird, jeweils sowohl die Verbindungen der Formel (I) als auch die neuen Verbindungen der Formel (I) eingeschlossen.

In den folgenden Gruppen von Verbindungen haben die einzelnen Reste R und Z die oben angegebenen Bedeutungen.

In einer hervorgehobenen Gruppe von erfindungsgemäß zu verwendenden Verbindungen der Formel (I) steht Q für Q-1

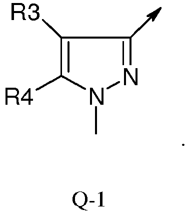

In einer weiteren hervorgehobenen Gruppe von erfindungsgemäß zu verwendenden Verbindungen der Formel (I) steht Q für Q-2

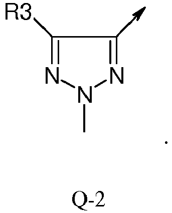

In einer weiteren hervorgehobenen Gruppe von erfindungsgemäß zu verwendenden Verbindungen der Formel (I) steht Q für Q-3

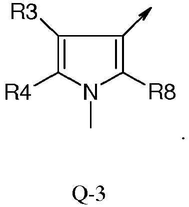

In einer weiteren hervorgehobenen Gruppe von erfindungsgemäß zu verwendenden Verbindungen der Formel (I) steht Q für Q-4

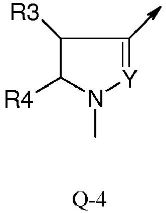

In einer weiteren hervorgehobenen Gruppe von erfindungsgemäß zu verwendenden Verbindungen der Formel (I) steht Q für Q-5

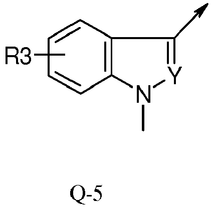

In einer weiteren hervorgehobenen Gruppe von erfindungsgemäß zu verwendenden Verbindungen der Formel (I) steht A für 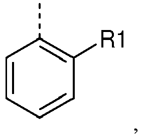 wobei dieser Rest m Substituenten R2 trägt.

In einer weiteren hervorgehobenen Gruppe von erfindungsgemäß zu verwendenden Verbindungen der Formel (I) steht A für 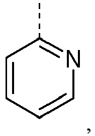 wobei dieser Rest m Substituenten R2 trägt.

In einer weiteren hervorgehobenen Gruppe von erfindungsgemäß zu verwendenden Verbindungen der Formel (I) steht A für 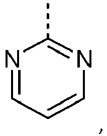 wobei dieser Rest m Substituenten R2 trägt.

In den folgenden hervorgehobenen Gruppen von Verbindungen der Formel (I), die jeweils eine bevorzugte Ausführungsform darstellen, können die Reste A bzw. B wie oben ausgeführt weitere Substituenten R2 bzw. R7 tragen.

Eine hervorgehobene Gruppe von Verbindungen der Formel (I) sind Verbindungen der Formel (A-1)-(Q-1)-D-(B-1)

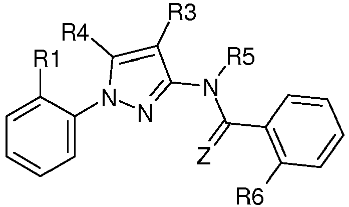

Eine weitere hervorgehobene Gruppe von Verbindungen der Formel (I) sind Verbindungen der Formel (A-2)-(Q-1)-D-(B-1)

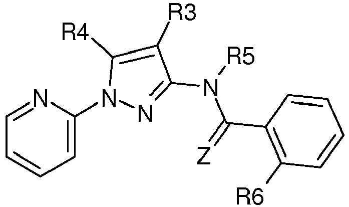

Eine weitere hervorgehobene Gruppe von Verbindungen der Formel (I) sind Verbindungen der Formel (A-1)-(Q-1)-D-(B-2)

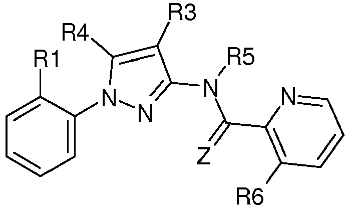

Eine weitere hervorgehobene Gruppe von Verbindungen der Formel (I) sind Verbindungen der Formel (A-1)-(Q-1)-D-(B-39)

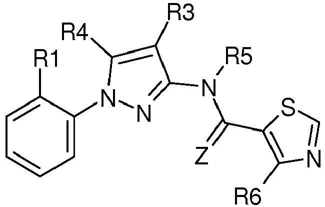

Eine weitere hervorgehobene Gruppe von Verbindungen der Formel (I) sind Verbindungen der Formel (A-1)-(Q-1)-D-(B-23)

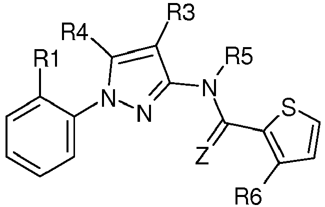

Eine weitere hervorgehobene Gruppe von Verbindungen der Formel (I) sind Verbindungen der Formel (A-1)-(Q-1)-D-(B-21)

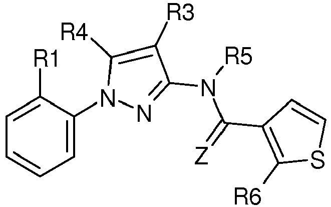

Eine weitere hervorgehobene Gruppe von Verbindungen der Formel (I) sind Verbindungen der Formel (A-1)-(Q-1)-D-(B-18)

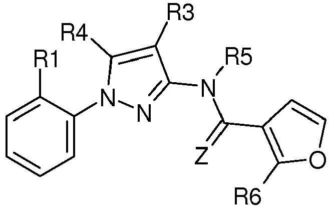

Eine weitere hervorgehobene Gruppe von Verbindungen der Formel (I) sind Verbindungen der Formel (A-1)-(Q-1)-D-(B-3)

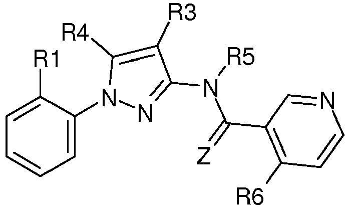

Eine weitere hervorgehobene Gruppe von Verbindungen der Formel (I) sind Verbindungen der Formel (A-1)-(Q-1)-D-(B-11)

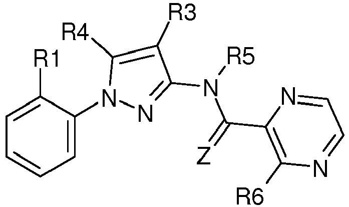

Eine weitere hervorgehobene Gruppe von Verbindungen der Formel (I) sind Verbindungen der Formel (A-1)-(Q-1)-D-(B-5)

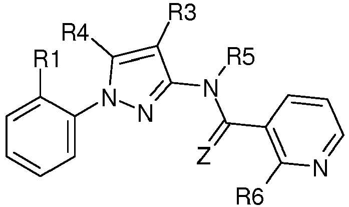

Eine weitere hervorgehobene Gruppe von Verbindungen der Formel (I) sind Verbindungen der Formel (A-6)-(Q-1)-D-(B-1)

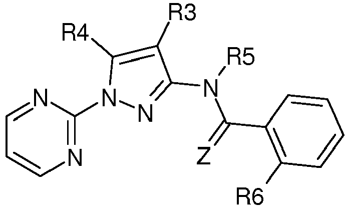

Eine weitere hervorgehobene Gruppe von Verbindungen der Formel (I) sind Verbindungen der Formel (A-2)-(Q-1)-D-(B-3)

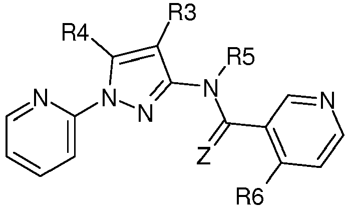

Eine weitere hervorgehobene Gruppe von Verbindungen der Formel (I) sind Verbindungen der Formel (A-1)-(Q-4)-D-(B-1)

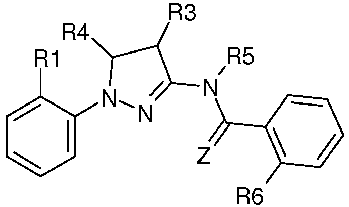

Eine weitere hervorgehobene Gruppe von Verbindungen der Formel (I) sind Verbindungen der Formel (A-1)-(Q-2)-D-(B-1)

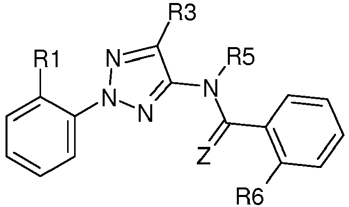

Eine weitere hervorgehobene Gruppe von Verbindungen der Formel (I) sind Verbindungen der Formel (A-1)-(Q-2)-D-(B-5)

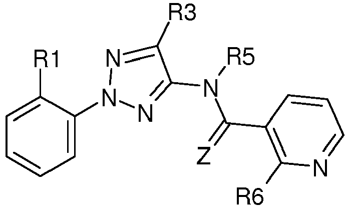

Eine weitere hervorgehobene Gruppe von Verbindungen der Formel (I) sind Verbindungen der Formel (A-2)-(Q-5)-D-(B-1)

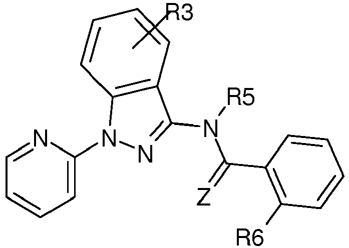

In gleicher Weise wie bei den vorhergehenden hervorgehobenen Gruppen von Verbindungen lassen sich bei den folgenden Gruppen von Verbindungen die jeweiligen Strukturformeln in einfacher Weise erstellen.

Eine weitere hervorgehobene Gruppe von Verbindungen der Formel (I) sind Verbindungen der Formel (A-3)-(Q-1)-D-(B-1).

Eine weitere hervorgehobene Gruppe von Verbindungen der Formel (I) sind Verbindungen der Formel (A-1)-(Q-1)-D-(B-36).

Eine weitere hervorgehobene Gruppe von Verbindungen der Formel (I) sind Verbindungen der Formel (A-1)-(Q-1)-D-(B-46).

Eine weitere hervorgehobene Gruppe von Verbindungen der Formel (I) sind Verbindungen der Formel (A-1)-(Q-1)-D-(B-1).

Eine weitere hervorgehobene Gruppe von Verbindungen der Formel (I) sind Verbindungen der Formel (A-1)-(Q-1)-D-(B-27).

Eine weitere hervorgehobene Gruppe von Verbindungen der Formel (I) sind Verbindungen der Formel (A-1)-(Q-1)-D-(B-51).

Eine weitere hervorgehobene Gruppe von Verbindungen der Formel (I) sind Verbindungen der Formel (A-1)-(Q-1)-D-(B-4).

Eine weitere hervorgehobene Gruppe von Verbindungen der Formel (I) sind Verbindungen der Formel (A-1)-(Q-1)-D-(B-13).

Eine weitere hervorgehobene Gruppe von Verbindungen der Formel (I) sind Verbindungen der Formel (A-1)-(Q-1)-D-(B-31).

Eine weitere hervorgehobene Gruppe von Verbindungen der Formel (I) sind Verbindungen der Formel (A-1)-(Q-1)-D-(B-35).

Eine weitere hervorgehobene Gruppe von Verbindungen der Formel (I) sind Verbindungen der Formel (A-1)-(Q-1)-D-(B-10).

Eine weitere hervorgehobene Gruppe von Verbindungen der Formel (I) sind Verbindungen der Formel (A-12)-(Q-1)-D-(B-1).

Eine weitere hervorgehobene Gruppe von Verbindungen der Formel (I) sind Verbindungen der Formel (A-12)-(Q-1)-D-(B-2).

Eine weitere hervorgehobene Gruppe von Verbindungen der Formel (I) sind Verbindungen der Formel (A-2)-(Q-1)-D-(B-49).

Eine weitere hervorgehobene Gruppe von Verbindungen der Formel (I) sind Verbindungen der Formel (A-2)-(Q-1)-D-(B-46).

Eine weitere hervorgehobene Gruppe von Verbindungen der Formel (I) sind Verbindungen der Formel (A-2)-(Q-1)-D-(B-13).

Eine weitere hervorgehobene Gruppe von Verbindungen der Formel (I) sind Verbindungen der Formel (A-2)-(Q-1)-D-(B-4).

Eine weitere hervorgehobene Gruppe von Verbindungen der Formel (I) sind Verbindungen der Formel (A-2)-(Q-1)-D-(B-6).

Eine weitere hervorgehobene Gruppe von Verbindungen der Formel (I) sind Verbindungen der Formel (A-2)-(Q-1)-D-(B-25).

Eine weitere hervorgehobene Gruppe von Verbindungen der Formel (I) sind Verbindungen der Formel (A-2)-(Q-1)-D-(B-31).

Eine weitere hervorgehobene Gruppe von Verbindungen der Formel (I) sind Verbindungen der Formel (A-2)-(Q-1)-D-(B-35).

Eine weitere hervorgehobene Gruppe von Verbindungen der Formel (I) sind Verbindungen der Formel (A-2)-(Q-1)-D-(B-27).

Eine weitere hervorgehobene Gruppe von Verbindungen der Formel (I) sind Verbindungen der Formel (A-7)-(Q-1)-D-(B-1).

Eine weitere hervorgehobene Gruppe von Verbindungen der Formel (I) sind Verbindungen der Formel (A-9)-(Q-1)-D-(B-1).

Wenn R4 oder R3 in der Gruppe Q-1 für Hydroxy steht, kann Q-1 auch in der Keto-Form vorliegen:

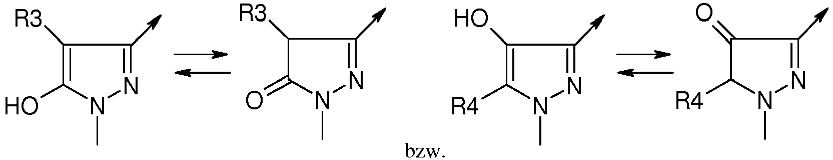

Weiter wurde gefunden, dass man die Verbindungen der Formel (I) nach den im Folgenden beschriebenen Verfahren erhalten kann.

Die Erfindung betrifft demnach auch Verfahren zur Herstellung von Verbindungen der Formel (I), in denen Z für O steht, 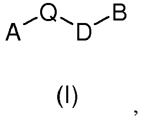 wobei A, Q, D und B die oben beschriebenen Bedeutungen haben,
durch Umsetzung von Aminen der Formeln (II-1) bis (II-5) 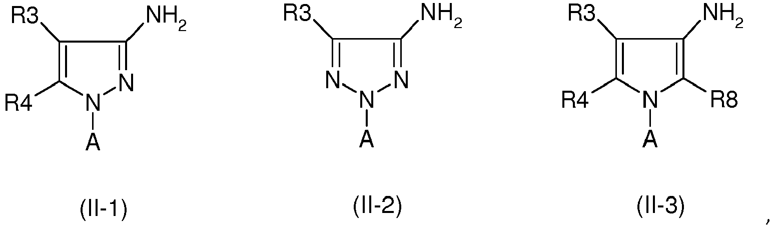 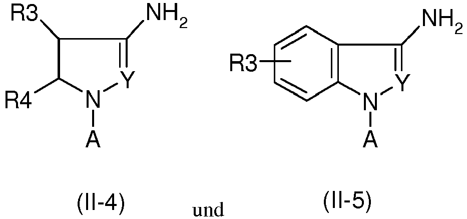 mit Carbonsäuren oder Carbonsäurehalogeniden der Formel (III), 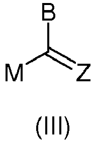 worin
M für Halogen, Hydroxy, Alkoxy, Alkylsufanyl, Acyloxy, Sulfonyloxy, N-Heterocyclyl (z.B. Imidazolyl) oder für Hydroxy steht,
B die oben angegebenen Bedeutungen hat und
Z für O steht.

Hierbei können Verbindungen der Formel (III) voraktiviert sein oder *in situ* aktiviert werden. Beispielsweise können Verbindungen der Formel (III) als Säurehalogenide (z.B. M = Chlor) eingesetzt werden. Die Umsetzung wird dann vorteilhaft in Gegenwart einer Base, wie z.B. Triethylamin oder Natriumhydroxid durchgeführt. Es können aber auch Carbonsäuren (M = OH) in Gegenwart von Kupplungsreagenzien wie z.B. Dicyclohexylcarbodiimid und Additiven wie 1-Hydroxy-1-H-benzotriazol eingesetzt werden (W. König, R. Geiger, Chem. Ber. 1970, 103, 788). Verwendbar sind ferner Kupplungsreagenzien wie 1-Ethyl-3-(3-dimethylaminopropyl)carbodiimid, 1,1'-Carbonyl-1H-imidazol, O-(Benzotriazol-1-yl)-N,N,N',N'-tetramethyluroniumhexafluorophosphat und ähnliche Verbindungen. Als Kupplungsreagenzien zur Durchführung des Herstellungsverfahrens sind grundsätzlich alle Verbindungen geeignet, die die Bildung einer Amidbindung ermöglichen (vgl. z. B. E. Valeur, M. Bradley Chem. Soc. Rev. 2009, 38, 606; S.-Y. Han, Y.-A. Kim Tetrahedron 2004, 60, 2447). Weiterhin können auch symmetrische oder gemischte Anhydride zur Darstellung von Verbindungen der Formel (I) verwendet werden (G. W. Anderson, J. E. Zimmerman, F. M. Calahan, J. Am. Chem. Soc. 1967, 89, 5012.). Dabei können verschiedene Chlorameisensäureester zum Einsatz kommen, wie z.B. Chlorameisensäure*iso*butylester und Chlorameisensäure-*sec*-butylester. Ebenfalls können beispielsweise Isovalerylchlorid und Pivaloylchlorid verwendet werden.
b) Gegebenenfalls können anschließend die Verbindungen der Formel (I), in denen Z für O (Sauerstoffatom) steht, mit einem Schwefelungsreagenz, wie z.B. Diphosphorpentasulfid oder Lawessons Reagenz (vgl. C. P. Dell in Comprehensive Orgauic Funktional Group Transformations, Vol. 5, Hrsg.: A. L. Katritzky, O. Meth-Cohn, C. W. Rees, Pergamon, Oxford, 1995, S. 565; M. Jesberger, T. P. Davis, L. Barner, Synthesis 2003, 13, 1929), zu Verbindungen der Formel (I), in denen Z für S (Schwefelatom) steht, umgesetzt werden.
c) Verbindungen der Formeln (1-1), (1-2) und (1-3) 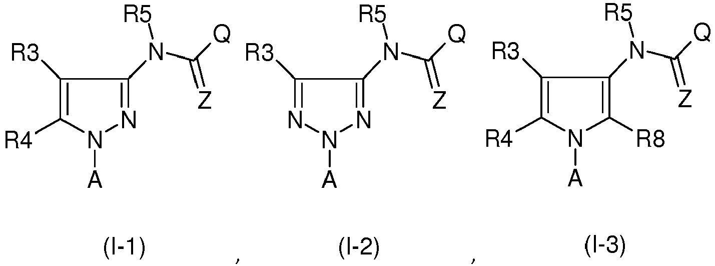 in denen R3 für Halogen steht, können entweder nach dem oben beschriebenen Herstellungsweg aus den entsprechenden Aminen der Formeln (II-1), (II-2) und (II-3) erhalten werden, in denen R3 für Halogen (F, Cl, Br oder I) steht oder aus Amiden der Formeln (1-1), (1-2) und (1-3), in denen R3 für H (Wasserstoff) steht, durch Umsetzung mit einem Halogenierungsreagenz, wie z.B. N-Halosuccinimide (vgl. z.B. WO2008/092888, Z.-G- Zhao, Z.-X. Wang, Synth. Comm. 2007, 37, 137) oder 1-Chlormethyl-4-fluordiazoniabicyclo[2.2.2]octan bis(tetrafluoroborate) (P. T. Nyffeler, S. Gonzalez Durón, M. D. Burkart, S. P. Vincent, C-H. Wong, Angew. Chem. Int. Ed. 2005, 44, 192), synthetisiert werden.
d) Verbindungen der Formeln (I-1), (I-2) und (I-3) in denen für R5 für Wasserstoff steht, können in Anwesenheit einer Base (z.B. Natriumhydrid) und einem Alkylierungs- (z.B. Methyliodid) oder Acylierungsmittel (z.B. Acetanhydrid) zu Verbindungen der Formeln (I-1), (I-2) und (I-3) in denen R5 für Alkyl oder Acyl werden (vgl. z.B. WO 2005/092863).
e) Verbindungen der Formel (I-1) können alternativ auch erhalten werden, indem 1H-Pyrazole der Formel (IV-1) 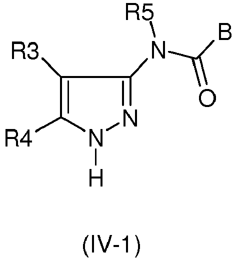 mit Aromaten oder Heteroaromaten der Formel (V) 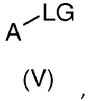 umgesetzt werden, die mit einer geeigneten Gruppe LG substituiert sind. Die Reaktion kann entweder in Anwesenheit einer Base (z.B. Kaliumcarbonat für z.B. LG = Fluor (vgl. WO 2011/060035) oder gegebenenfalls eines Katalysators (z.B. CuI/1,2-Cyclohexanediamine, Kaliumcarbonat für z.B. LG = Brom (vgl. WO 2007/039146) oder Cu(OAc)₂/Pyridin für z.B. LG = B(OH)₂ (vgl. WO 2005/092863)) durchgeführt werden.

Amine der Formel (II-1) 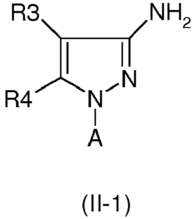 sind kommerziell erhältlich, literaturbekannt oder können anhand literaturbekannter Verfahren synthetisiert werden. Als Beispiele seien genannt:
1-(2-Methylphenyl)-1H-pyrazol-3-amin (WO 2004/037794),
1-(2-Methoxyphenyl)-1H-pyrazol-3-amin (WO 2004/037794),
1-(2-(Trifluormethyl)phenyl)-1H-pyrazol-3-amin (kommerziell erhältlich),
1-(2-Cyanophenyl)-1H-pyrazol-3-amin (kommerziell erhältlich),
1-(2-Fluorphenyl)-1H-pyrazol-3-amin (WO 2004/037794),
1-(2-Chlorphenyl)-1H-pyrazol-3-amin (C. Albert, C. Tironi, Farmaco Sci. 1964, 19, 618),
1-(2-Chlorphenyl)-5-methyl-1H-pyrazol-3-amin (kommerziell erhältlich) 1-(2-Bromphenyl)-1H-pyrazol-3-amin (kommerziell erhältlich),
1-(2,4-Difluorphenyl)-1H-pyrazol-3-amin (kommerziell erhältlich),
1-(2,5-Dichlorphenyl)-1H-pyrazol-3-amin (kommerziell erhältlich),
1-(Pyridin-2-yl)-1H-pyrazol-3-amin (WO 2004/037794),
1-(3-Chlorpyridin-2-yl)-1H-pyrazol-3-amin (kommerziell erhältlich),
1-(3-Fluorpyridin-2-yl)-1H-pyrazol-3-amin (kommerziell erhältlich),
1-(Pyrimidin-2-yl)-1H-pyrazol-3-amin (kommerziell erhältlich),
1-(Pyridazin-3-yl)-1H-pyrazol-3-amin (kommerziell erhältlich),
1-[2-(Methylsulfonyl)phenyl]-1H-pyrazol-3-amin (kommerziell erhältlich),
1-(2,3-Difluorphenyl)-1H-pyrazol-3-amin (kommerziell erhältlich),
1-(2,4,5-Trifluorphenyl)-1H-pyrazol-3-amin (kommerziell erhältlich),
1-(2,4-Dichlorphenyl)-1H-pyrazol-3-amin (kommerziell erhältlich),
1-(2,5-Difluorphenyl)-1H-pyrazol-3-amin (kommerziell erhältlich),
1-(2,4-Difluorphenyl)-5-ethyl-1H-pyrazol-3-amin (kommerziell erhältlich),
1-(2,4-Difluorphenyl)-5-methyl-1H-pyrazol-3-amin (kommerziell erhältlich),
1-(2-Nitrophenyl)-1H-pyrazol-3-amin (kommerziell erhältlich),
1-[3-Chlor-5-(trifluormethyl)pyridin-2-yl]-1H-pyrazol-3-amin (kommerziell erhältlich),
1-[3-(Trifluormethyl)pyridin-2-yl]-1H-pyrazol-3-amin (kommerziell erhältlich),
1-(3,5-Dichlorpyridin-2-yl)-1H-pyrazol-3-amin (kommerziell erhältlich),
1-(3-Brompyridin-2-yl)-1H-pyrazol-3-amin (kommerziell erhältlich),
2-(3-Amino-1H-pyrazol-1-yl)nicotinonitril (kommerziell erhältlich),
3-(3-Amino-1H-pyrazol-1-yl)pyrazin-2-carbonitril (kommerziell erhältlich),
2-(3-Amino-1H-pyrazol-1-yl)-6-methylnicotinonitril (kommerziell erhältlich),
2-(3-Amino-1H-pyrazol-1-yl)-4,6-dimethylnicotinonitril (kommerziell erhältlich),
1-(5-Fluorpyridin-2-yl)-1H-pyrazol-3-amin (kommerziell erhältlich),
Neue 1H-Pyrazol-3-amine der Formel (II-1) können wie in Syntheseschema 1 dargestellt erhalten werden.

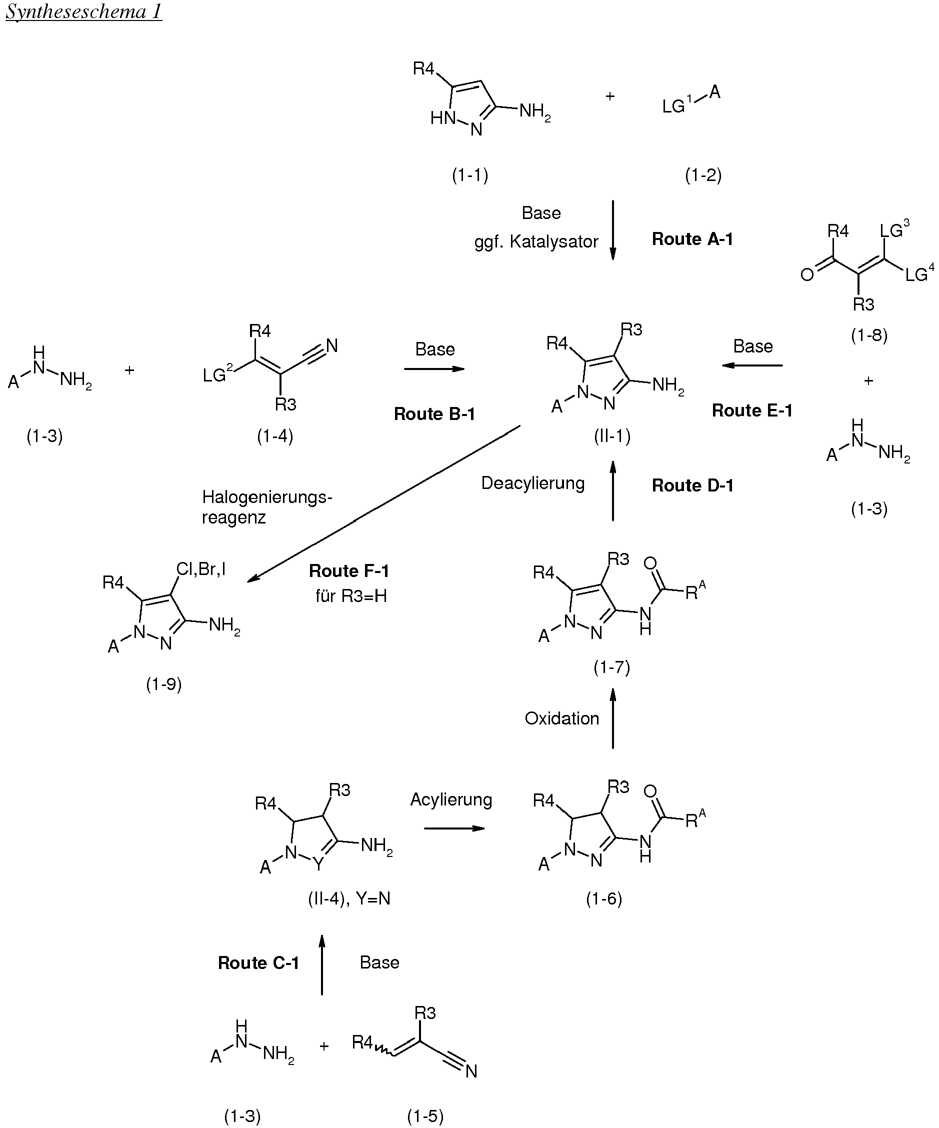

Gemäß Route A-1 können 1H-Pyrazol-3-amine der Formel (1-1) mit Aromaten oder Heteroaromaten der Formel (1-2) umgesetzt werden, die mit einer geeigneten Gruppe LG¹ substituiert sind, um in Anwesenheit einer Base (z.B. Kalium-*tert*-butoxid für z.B. LG¹ = Chlor (vgl. WO 2009/012482) oder z.B. Cäsiumcarbonat für z.B. LG¹ = Chlor (vgl. WO 2007/056155)) und gegebenenfalls eines Katalysators (z.B. CuI/N,N-Dimethylethan-1,2-diamin, Kaliumcarbonat für z.B. LG¹ = Iod (vgl. WO2008/153042) oder Pd₂(dba)₃/Xantphos, Natriumcarbonat für z.B. LG¹ = Chlor (vgl. Z. Shen, Y. Hong, X. He, W. Mo, B. Hu, N. Sun, X. Hu, Org. Lett. 2010, 12, 552)) zu Verbindungen der Formel (II-1) zu reagieren.

In einer alternativen Route B-1 kann ein Aryl- oder Hetarylhydrazin der Formel (1-3), das als freies Hydrazin oder als Salz (z.B. als Hydrochlorid) vorliegen kann, mit einem Acrylnitril der Formel (1-4), das mit einer Abgangsgruppe LG² (z.B. LG² = OR mit R = Alkyl, Acyl, Sulfonyl, etc.; LG² = SR mit R = Alkyl, Acyl, etc.; LG² = NHR oder NR₂ mit R = Alkyl, Acyl, Sulfonyl; LG² = Halogen oder Cyan) substituiert ist, in Gegenwart einer geeigneten Base (z.B. Natriumethylat vgl. z.B. WO 2004/037794 oder Kalium-*tert*-butoxid vgl. z.B. WO 2008/046527) zum 1H-Pyrazol-3-amin der Formel (II-1) umgesetzt werden.

4,5-Dihydro-1H-pyrazol-3-amine der Formel (II-4), in denen Y für N steht, lassen sich gemäß Route C-1 aus Aryl- oder Hetarylhydrazinen (1-3), die als freie Hydrazine oder Salze (z.B. als Hydrochloride) vorliegen können, mit Acrylnitrilen der Formel (1-5) in Gegenwart einer geeigneten Base (beispielsweise Cholin) gewinnen (vgl. C. Albert, C. Tironi, Farmaco Sci. 1964, 19, 618). Verbindungen der Formel (II-4), in denen Y für N steht, lassen sich in einer Sequenz aus Acylierung mit einem geeigneten Acylierungsreagenz (z.B. Acetanhydrid für R^{A} = Methyl), Oxidation mit einem angemessenen Oxidationsmittel (z.B. 2,3-Dichlor-5,6-dicyano-1,4-benzochinon) und Abspaltung der Acylgruppe mittels geeigneter Methoden (z.B. Erhitzen in Salzsäure vgl. Greene's protective groups in organic synthesis, 4th ed., P.G.M. Wuts, T.W. Greene, John Wiley & Sons, Inc., Hoboken, New Jersey, 2007) ebenfalls zu 1H-Pyrazol-3-aminen der Formel (II-1) umsetzen.

Ein weiterer Zugang zu 1H-Pyrazol-3-aminen der Formel (II-1) besteht gemäß Route E-1 ausgehend von Ketoverbindungen der Formel (1-8), die mit einer Abgangsgruppe LG^{3/4} (z.B. LG^{3/4} = OR mit R = Alkyl, Acyl, Sulfonyl, etc.; LG^{3/4} = SR mit R = Alkyl, Acyl, etc.; LG^{3/4} = NHR oder NR₂ mit R = Wasserstoff, Alkyl, Acyl, Sulfonyl; LG^{3/4} = Halogen) substituiert sind. Diese werden zunächst mit Ammoniak über β-Enaminoketone (LG³ = NH₂/LG⁴ = Alkoxy) aktiviert (vgl. M. A. P. Martins, W. Cunico, S. Brondani, R. L. Peres, N. Zimmermann, F. A. Rosa, G. F. Fiss, N. Zanatta, H. G. Bonacorso Synthesis 2006, 1485) um dann in Gegenwart geeigneter Basen (z.B. Triethylamin) mit freien Hydrazinen oder deren Salzen (z.B. Hydrochloride) zu 1H-Pyrazol-3-aminen der Formel (II-1) umgesetzt zu werden.

1H-Pyrazol-3-amine der Formel (II-1) können gegebenenfalls gemäß Route F-1, wenn R3 für H (Wasserstoff) steht, durch geeignete Halogenierungsreagenzien (z.B. N-Halosuccinimide) in der 3-Position zu Verbindungen der Formel (1-8) halogeniert werden, in denen R3 für Chlor, Brom oder Iod steht (vgl. J. Velcicky, R. Feifel, S. Hawtin, R. Heng, C. Huppertz, G. Koch, M. Kroemer, H. Moebitz, L. Revesz, C. Scheufler, A. Schlapbach Bioorg. Med. Chem. Letters 2010, 20, 1293).

### Amine der Formel (II-2)

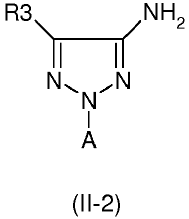

sind ebenfalls kommerziell erhältlich. Als Beispiele seien genannt:
2-(2-Bromphenyl)-2H-1,2,3-triazol-4-amin (kommerziell erhältlich)
2-(2-Chlorphenyl)-2H-1,2,3-triazol-4-amin (kommerziell erhältlich)
2-(2-Bromphenyl)-5-chlor-2H-1,2,3-triazol-4-amin (kommerziell erhältlich)
2-(3-Chlorpyridin-2-yl)-2H-1,2,3-triazol-4-amin (kommerziell erhältlich)
2-(3-Brompyridin-2-yl)-2H-1,2,3-triazol-4-amin (kommerziell erhältlich)
5-Chlor-2-(3-chlorpyridin-2-yl)-2H-1,2,3-triazol-4-amin (kommerziell erhältlich)
Neue Amine der Formel (II-2) können gemäß Syntheseschema 3 erhalten werden:

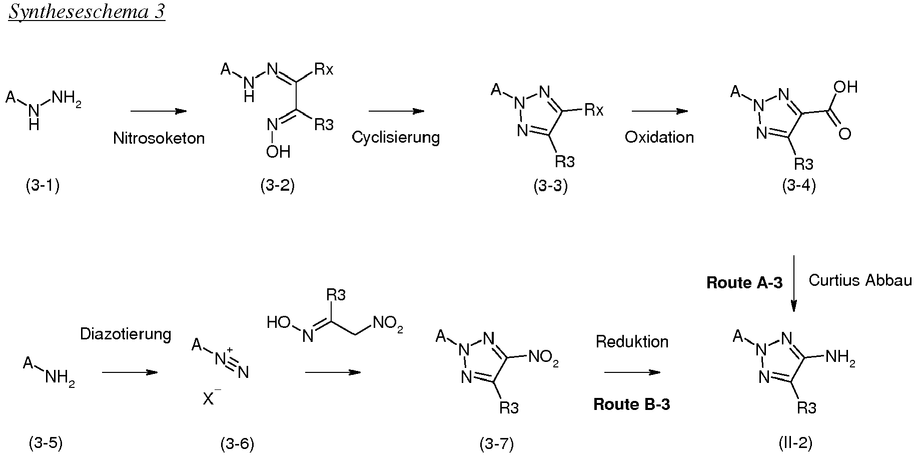

Gemäß Route A-3 können aromatische Hydrazine der Formel (3-1) z.B. mit einem Nitrosoketon (z.B. Nitrosoaceton für R3=H, vgl. M. Begtrup, J. Holm J. Chem. Soc., Perkin Trans. 1, 1981, 503.) umgesetzt werden, die dann mit Hilfe von Kondensationsmitteln (z.B. Acetanhydrid vgl. D. L. Swartz, A. R. Karash, L. A. Berry, D. L. Jaeger J. Heterocyclic Chem. 1983, 20, 1561) zu 2H-1,2,3-triazolen der Formel (3-3) cyclisiert werden. Oxidation (z.B mit Natriumchromat(VI) vgl. Comprehensive Organic Transformations: A Guide to Functional Group Preparations; Larock, R. C., Ed.; Wiley-VCH: New York, 1999) von (3-3) liefert die Carbonsäure (3-4), die dann durch Curtius-Abbau in das 4-Amino-2H-1,2,3-triazol (II-2) überführt werden kann (vgl. P. A. S. Smith Org. React. 1946, 337).

Alternativ kann auch Route B-3 zur Darstellung der 4-Amino-2H-1,2,3-triazole (II-2) gefolgt werden (vgl. V. M. Nikitin, A. V. Zavodov, L. I. Vereshchagin Zhurnal Organicheskoi Khimii 1992, 28, 2334.). Hierfür wird ein aromatisches Amin (3-5) diazotiert und das Diazoniumsalz (3-6) mit z.B. Metazonsäure (für R3=H) umgesetzt. Das entstandene 4-Nitro-2H-1,2,3-triazol (3-7) kann dann z.B. mit Zinn(II)chlorid zum 4-Amino-2H-1,2,3-triazol (II-2) reduziert werden.

### Amine der Formel (II-3)

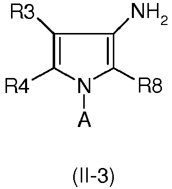

können gemäß Syntheseschema 4 erhalten werden.

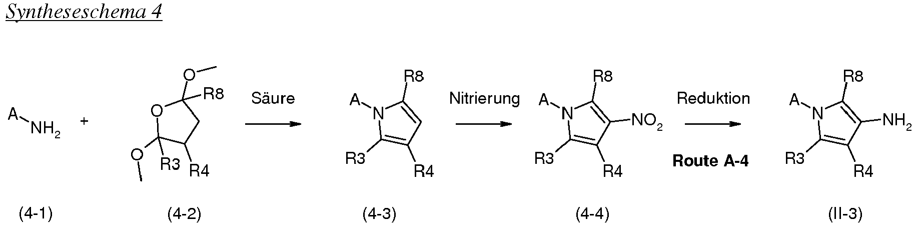

Hierfür werden aromatische Amine der Formel (4-1) z.B. mit cyclischen Acetalen der Formel (4-2) unter sauren Bedingungen zum Pyrrol (4-3) umgesetzt (z.B. FeCl₃ vgl. N. Azizi, A. Khajeh-Amiria, H. Ghafurib, M. Bolourtchiana, M. R. Saidi Synlett 2009, 14, 2245). Nitrierung des Pyrrols (4-3) zum 3-Nitro-1H-pyrrol (4-4) (z.B. mittels Acetylnitrat vgl. D. Korakas, G. Varvounis J. Heterocyclic Chem. 1996, 33, 611) und anschließende Reduktion (z.B. mit SnCl₂ vgl. WO 2009/136995) liefert das 3-Amino-1H-pyrrol (II-3).

### Amine der Formel (II-5)

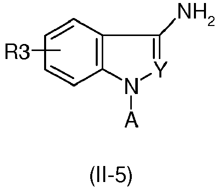

sind ebenfalls kommerziell erhältlich. Neue 1H-Indazol-3-amine der Formel (II-5) können gemäß Syntheseschema 2 erhalten werden.

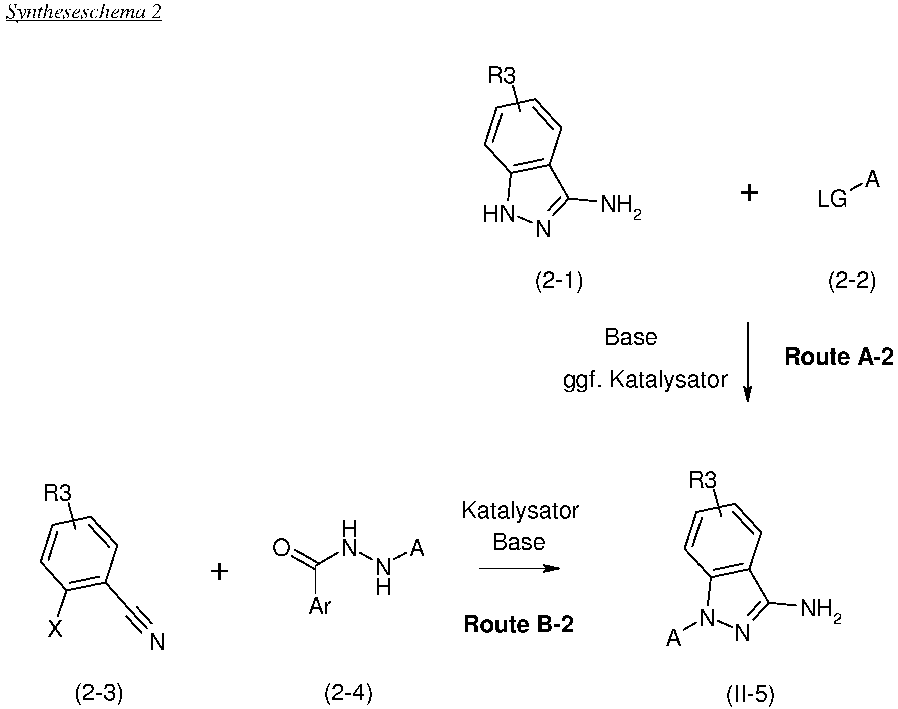

Hierbei werden Verbindungen der Formel (2-1) gemäß Route A-2 mit Aromaten oder Heteroaromaten der Formel (2-2) umgesetzt, die mit einer geeigneten Abgangsgruppe LG substituiert sind, um in Anwesenheit einer Base (z.B. Natriumhydrid für z.B. LG = Chlor (vgl.WO 2008/068171)) und gegebenenfalls eines Katalysators (z.B. CuI/N,N-Dimethylethan-1,2-diamin, Kaliumcarbonat für z.B. LG = Brom (vgl. WO 2010/098367) oder Pda(dba)₃/X-Phos, Cäsiumcarbonat für z.B. LG = Chlor (vgl. DE 10 2009/004245)) zu Verbindungen der Formel (II-5) zu reagieren. 1H-Indazol-3-amine der Formel (2-1) sowie Aromaten oder Heteroaromaten der Formel (2-2) sind kommerziell verfügbar oder können nach bekannten Verfahren erhalten werden.

Gemäß Route B-2 können alternativ ortho-Halobenzonitrile der Formel (2-3) mit Hydraziden der Formel (2-4) in Gegenwart eines Katalysators und einer Base (z.B. Kupfer(I)bromid/4-Hydroxy-L-Prolin; Kaliumcarbonat für X=Br oder I und Ar=Phenyl vgl. L. Xu, Y. Peng, Q. Pan, Y. Jiang, D. Ma J. Org. Chem. 2013, 78, 3400) zu 1H-Indazol-3-aminen der Formel (II-5) umgesetzt werden.

Die erfindungsgemäßen Verfahren zur Herstellung der neuen Verbindungen der Formel (I) werden vorzugsweise unter Verwendung eines Verdünnungsmittels durchgeführt. Als Verdünnungsmittel zur Durchführung der erfindungsgemäßen Verfahren kommen neben Wasser alle inerten Lösungsmittel in Frage. Als Beispiele sind zu nennen: Halogenkohlenwasserstoffe (z.B. Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-*tert*-butylether, Anisol, Phenetol, Cyclohexylmethylether, Dimethylether, Diethylether, Diproplether, Diisopropylether, Di-*n*-butylether, Diisobutylether, Diisoamylether, Ethylenglycoldimethylether, Tetrahydrofuran, 1,4-Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, *o-*Nitrotoluol); Nitrile (wie z.B. Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Octan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeintervalles von 70°C bis 190°C, Cyclohexan, Methylcyclohexan, Petrolether, Ligroin, Benzol, Toluol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäure-triamid, Formamid, N-Methylformamid, N,N-Dimethylformamid, N,N-Dipropylformamid, N,N-Dibutylformamid, N-Methylpyrrolidin, N-Methylcaprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formylpiperidin, N,N'-Diformylpiperazin) und Ketone (z.B. Aceton, Acetophenon, Methylethylketon, Methylbutylketon).

Selbstverständlich kann man das erfindungsgemäße Verfahren auch in Gemischen der genannten Lösungs- und Verdünnungsmittel durchführen.

Die Reaktionstemperaturen können bei der Durchführung des erfindungsgemäßen Verfahrens in einem größeren Bereich variiert werden. Im Allgemeinen arbeitet man bei Temperaturen zwischen -30 °C und +150 °C, vorzugsweise zwischen -10 °C und +100 °C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen bei absoluten Drücken zwischen 0,1 bar und 15 bar - durchzuführen.

Zur Durchführung des erfindungsgemäßen Verfahrens werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels, gegebenenfalls auch unter einer Schutzgasatmosphäre (z.B. unter Stickstoff, Argon oder Helium) durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)-octan (DABCO), 1,8-Diazabicyclo(5.4.0)undecen (DBU), Cyclohexyltetrabutyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyltoluidin, N,N-Dimethyl-*p*-aminopyridin, N-Methyl-pyrrolidin, N-Methylpiperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, 2-Picolin, 3-Picolin, Pyrimidin, Acridin, N,N,N',N'-Tetramethylendiamin, N,N,N',N'-Tetraethylendiamin, Chinoxalin, N-Propyldiisopropylamin, N-Ethyl-diisopropylamin, N,N'-Dimethyl-cyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoffsäuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)chlorid, Bortrifluorid oder sein Etherat, Titan(IV)chlorid, Zinn(IV)chlorid) und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure) eingesetzt werden.

Die erfindungsgemäßen bzw. erfindungsgemäß zu verwendenden Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., Epitrimerus pyri, Eutetranychus spp., Eriophyes spp., Glycyphagus domesticus, Halotydeus destructor, Hemitarsonemus spp., Hyalomma spp., Ixodes spp., Latrodectus spp., Loxosceles spp., Metatetranychus spp., Neutrombicula autumnalis, Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., Tetranychus spp., Trombicula alfreddugesi, Vaejovis spp., Vasates lycopersici.;
aus der Klasse der Chilopoda z.B. Geophilus spp., Scutigera spp.;
aus der Ordnung oder der Klasse der Collembola z.B. Onychiurus armatus.;
aus der Klasse der Diplopoda z.B. Blaniulus guttulatus;
aus der Klasse der Insecta, z.B. aus der Ordnung der Blattodea z.B. Blattella asahinai, Blattella germanica, Blatta orientalis, Leucophaea maderae, Panchlora spp., Parcoblatta spp., Periplaneta spp., Supella longipalpa;
aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptolestes ferrugineus, Cryptorhynchus lapathi, Cylindrocopturus spp., Dermestes spp., Diabrotica spp., Dichocrocis spp., Dicladispa armigera, Diloboderus spp., Epilachna spp., Epitrix spp., Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., Lachnosterna consanguinea, Lasioderma serricorne, Latheticus oryzae, Lathridius spp., Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Necrobia spp., Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllophaga helleri, Phyllotreta spp., Popillia japonica, Premnotrypes spp., Prostephanus truncatus, Psylliodes spp., Ptinus spp., Rhizobius ventralis, Rhizopertha dominica, Sitophilus spp., Sitophilus oryzae, Sphenophorus spp., Stegobium paniceum, Sternechus spp., Symphyletes spp., Tanymecus spp., Tenebrio molitor, Tenebrioides mauretanicus, Tribolium spp., Trogoderma spp., Tychius spp., Xylotrechus spp., Zabrus spp.;
aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Calliphora vicina, Ceratitis capitata, Chironomus spp., Chrysomyia spp., Chrysops spp., Chrysozona pluvialis, Cochliomyia spp., Contarinia spp., Cordylobia anthropophaga, Cricotopus sylvestris, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., Lucilia spp., Lutzomyia spp., Mansonia spp., Musca spp., Oestrus spp., Oscinella frit, Paratanytarsus spp., Paralauterborniella subcincta, Pegomyia spp., Phlebotomus spp., Phorbia spp., Phormia spp., Piophila casei, Prodiplosis spp., Psila rosae, Rhagoletis spp., Sarcophaga spp., Simulium spp., Stomoxys spp., Tabanus spp., Tetanops spp., Tipula spp.;
aus der Ordnung der Heteroptera z.B. Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptocorisa varicornis, Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Homoptera z.B. Acizzia acaciaebaileyanae, Acizzia dodonaeae, Acizzia uncatoides, Acrida turrita, Acyrthosipon spp., Acrogonia spp., Aeneolamia spp., Agonoscena spp., Aleyrodes proletella, Aleurolobus barodensis, Aleurothrixus floccosus, Allocaridara malayensis, Amrasca spp., Anuraphis cardui, Aonidiella spp., Aphanostigma piri, Aphis spp., Arboridia apicalis, Arytainilla spp., Aspidiella spp., Aspidiotus spp., Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., Cryptomyzus ribis, Cryptoneossa spp., Ctenarytaina spp., Dalbulus spp., Dialeurodes citri, Diaphorina citri, Diaspis spp., Drosicha spp., Dysaphis spp., Dysmicoccus spp., Empoasca spp., Eriosoma spp., Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Macrosteles facifrons, Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nettigoniclla spectra, Nilaparvata lugens, Oncometopia spp., Orthezia praelonga, Oxya chinensis, Pachypsylla spp., Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Prosopidopsylla flava, Protopulvinaria pyriformis, Pseudaulacaspis pentagona, Pseudococcus spp., Psyllopsis spp., Psylla spp., Pteromalus spp., Pyrilla spp., Quadraspidiotus spp., Quesada gigas, Rastrococcus spp., Rhopalosiphum spp., Saissetia spp., Scaphoideus titanus, Schizaphis graminum, Selenaspidus articulatus, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., Trialeurodes vaporariorum, Trioza spp., Typhlocyba spp., Unaspis spp., Viteus vitifolii, Zygina spp.;
aus der Ordnung der Hymenoptera z.B. Acromyrmex spp., Athalia spp., Atta spp., Diprion spp., Hoplocampa spp., Lasius spp., Monomorium pharaonis, Sirex spp., Solenopsis invicta, Tapinoma spp., Urocerus spp., Vespa spp., Xeris spp.;
aus der Ordnung der Isopoda z.B. Armadillidium vulgare, Oniscus asellus, Porcellio scaber;
aus der Ordnung der Isoptera z.B. Coptotermes spp., Cornitermes cumulans, Cryptotermes spp., Incisitermes spp., Microtermes obesi, Odontotermes spp., Reticulitermes spp.;
aus der Ordnung der Lepidoptera z.B. Achroia grisella, Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamstra brassicae, Melanitis leda, Mocis spp., Monopis obviella, Mythimna separata, Nemapogon cloacellus, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudaletia unipuncta, Pseudoplusia includens, Pyrausta nubilalis, Rachiplusia nu, Schoenobius spp., Scirpophaga spp., Scirpophaga innotata, Scotia segetum, Sesamia spp., Sesamia inferens, Sparganothis spp., Spodoptera spp., Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., Tryporyza incertulas, Tuta absoluta, Virachola spp.;
aus der Ordnung der Orthoptera oder Saltatoria z.B. Acheta domesticus, Dichroplus spp., Gryllotalpa spp., Hieroglyphus spp., Locusta spp., Melanoplus spp., Schistocerca gregaria;
aus der Ordnung der Phthiraptera z.B. Damalinia spp., Haematopinus spp., Linognathus spp., Pediculus spp., Phylloera vastatrix, Phtirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., Pulex irritans, Tunga penetrans, Xenopsylla cheopsis;
aus der Ordnung der Thysanoptera z.B. Anaphothrips obscurus, Baliothrips biformis, Drepanothrips reuteri, Enneothrips flavens, Frankliniella spp., Heliothrips spp., Hercinothrips femoralis, Rhipiphorothrips cruentatus, Scirtothrips spp., Taeniothrips cardamomi, Thrips spp.;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp.;
Schädlinge aus dem Stamm der Mollusca, insbesondere aus der Klasse der Bivalvia, z.B. Dreissena spp., sowie aus der Klasse der Gastropoda z.B. Arion spp., Biomphalaria spp., Bulinus spp., Deroceras spp., Galba spp., Lymnaea spp., Oncomelania spp., Pomacea spp., Succinea spp.;
Tierparasiten aus den Stämmen der Plathelminthes und Nematoda, z.B. Ancylostoma duodenale, Ancylostoma ceylanicum, Acylostoma braziliensis, Ancylostoma spp., Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti;
Pflanzenschädlinge aus dem Stamm der Nematoda, d.h. pflanzenparasitäre Nematoden, insbesondere Aphelenchoides spp., Bursaphelenchus spp., Ditylenchus spp., Globodera spp., Heterodera spp., Longidorus spp., Meloidogyne spp., Pratylenchus spp., Radopholus spp., Trichodorus spp., Tylenchulus spp., Xiphinema spp., Helicotylenchus spp., Tylenchorhynchus spp., Scutellonema spp., Paratrichodorus spp., Meloinema spp., Paraphelenchus spp., Aglenchus spp., Belonolaimus spp., Nacobbus spp., Rotylenchulus spp., Rotylenchus spp., Neotylenchus spp., Paraphelenchus spp., Dolichodorus spp., Hoplolaimus spp., Punctodera spp., Criconemella spp., Quinisulcius spp., Hemicycliophora spp., Anguina spp., Subanguina spp., Hemicriconemoides spp., Psilenchus spp., Pseudohalenchus spp., Criconemoides spp., Cacopaurus spp.

Weiterhin lässt sich aus dem Unterreich der Protozoa die Ordnung der Coccidia z.B. Eimeria spp. bekämpfen.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die die erfindungsgemäßen bzw. erfindungsgemäß zu verwendenden Wirkstoffe können als solche oder in ihren Formulierungen auch in Mischung mit einem oder mehreren geeigneten Fungiziden, Bakteriziden, Akariziden, Molluskiziden, Nematiziden, Insektiziden, Mikrobiologika, Nützlingen, Düngemitteln, Vogelrepellentien, Phytotonics, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. Durch Kombination der erfindungsgemäßen bzw. erfindungsgemäß zu verwendenden Wirkstoffe mit Mischpartnern erhält man synergistische Effekte, d.h. die Wirksamkeit der jeweiligen Mischung ist größer als aufgrund der Wirksamkeiten der Einzelkomponenten zu erwarten war. Generell können die Kombinationen sowohl in Vor-, Tank- oder Fertigmischungen als auch in Saatgutanwendungen verwendet werden.

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

Als Mischpartner geeignete Insektizide / Akarizide / Nematizide sind
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
   Cyclodien-organochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin; oder
   Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder
   Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder Etoxazole.
(11) Mikrobielle Disruptoren der Insektendarmmembran, z.B. Bacillus thuringiensis Subspezies israelensis, Bacillus thuringiensis Subspezies aizawai, Bacillus thuringiensis Subspezies kurstaki, Bacillus thuringiensis Subspezies tenebrionis und B.t. Pflanzenproteine: Cry1Ab, Cry1Ac, Cry1Fa, Cry1A.105, Cry2Ab, Vip3A, mCry3A, Cry3Ab, Cry3Bb, Cry34 Ab1/35Ab1; oder
   Bacillus sphaericus.
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder
   Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
   Tetron- und Tetramsäurederivate, z.B. Spirodiclofen, Spiromesifen und Spirotetramat.
(24) Komplex-IV-Elektronentransportinhibitoren, wie beispielsweise
   Phosphine, z.B. Aluminiumphosphid, Calciumphosphid, Phosphin und Zinkphosphid; oder
   Cyanid.
(25) Komplex-II-Elektronentransportinhibitoren, wie beispielsweise Cyenopyrafen und Cyflumetofen.
(28) Ryanodinrezeptor-Effektoren, wie beispielsweise
   Diamide, z.B. Chlorantraniliprole, Cyantraniliprole und Flubendiamide.

Weitere Wirkstoffe, wie beispielsweise Amidoflumet, Azadirachtin, Benclothiaz, Benzoximate, Bifenazate, Bromopropylate, Chinomethionat, Cryolite, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), Afidopyropen (bekannt aus WO2008/066153), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluor-ethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), Pyflubumide (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazin-carboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}}amino)benzoyl]-1,2-diethyl-hydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), Methyl-2-[3,5-dibrom-2-({[3-brom-1-(3-chlorpyridin-2-yl)-1H-pyrazol-5-yl]carbonyl}amino)benzoyl]-2-ethyl-1-methyl-hydrazincarboxylat (bekannt aus WO2011/049233), Heptafluthrin, Pyriminostrobin, Flufenoxystrobin und 3-Chlor-N²-(2-cyanpropan-2-yl)-N¹-[4-(1,1,1,2,3,3,3-heptafluorpropan-2-yl)-2-methylphenyl]phthalamid (bekannt aus WO2012/034472).

Als Mischpartner geeignete Fungizide sind:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethyl-phenoxy)methyl]phenyl}-2-methoxy-N-methylacetamid und (2R)-2-{2-[(2,5-Dimethyl-phenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim, Pyrimethanil und 3-(5-Fluor-3,3,4,4-tetramethyl-3,4-dihydroisoquinolin-1-yl)quinolin.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxy-phenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropyl-methoxy)imino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydro-naphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valinamid, 4-Oxo-4-[(2-phenylethyl)amino]butansäure und But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Die Behandlung der Pflanzen und Pflanzenteile mit den erfindungsgemäßen bzw. erfindungsgemäß zu verwendenden Wirkstoffen, Wirkstoffkombinationen bzw. Mitteln erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Eine bevorzugte direkte Behandlung der Pflanzen ist die Blattapplikation, d.h. die Wirkstoffe, Wirkstoffkombinationen bzw. Mittel werden auf das Blattwerk aufgebracht, wobei die Behandlungsfrequenz und die Aufwandmenge auf den Befallsdruck des jeweiligen Pathogens, Schädlings bzw. Unkrauts abgestimmt sein kann.

Bei systemisch wirksamen Verbindungen gelangen die Wirkstoffe, Wirkstoffkombinationen bzw. Mittel über das Wurzelwerk in die Pflanzen. Die Behandlung der Pflanzen erfolgt dann durch Einwirkung der Wirkstoffe, Wirkstoffkombinationen bzw. Mittel auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Wirkstoffe, Wirkstoffkombinationen bzw. Mittel getränkt, oder durch die Bodenapplikation, d.h. die erfindungsgemäßen Wirkstoffe, Wirkstoffkombinationen bzw. Mittel werden in fester Form, (z.B. in Form eines Granulats) in den Standort der Pflanzen eingebracht. Bei Wasserreiskulturen kann das auch durch Zudosieren der Erfindung in einer festen Anwendungsform (z.B. als Granulat) in ein überflutetes Reisfeld sein.

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch Tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen bzw. erfindungsgemäß zu verwendenden Wirkstoff behandelt wird. Das erfindungsgemäße Verfahren zum Schutz von Saatgut und keimenden Pflanzen vor dem Befall von Schädlingen umfasst ein Verfahren, in dem das Saatgut gleichzeitig in einem Vorgang mit einem Wirkstoff der Formel I und Mischungspartner behandelt wird. Es umfasst auch ein Verfahren, in dem das Saatgut zu unterschiedlichen Zeiten mit einem Wirkstoff der Formel I und Mischungspartner behandelt wird.

Die Erfindung bezieht sich ebenfalls auf die Verwendung der erfindungsgemäßen Wirkstoffe zur Behandlung von Saatgut zum Schutz des Saatguts und der daraus entstehenden Pflanze vor Tierischen Schädlingen.

Weiterhin bezieht sich die Erfindung auf Saatgut, welches zum Schutz vor tierischen Schädlingen mit einem erfindungsgemäßen Wirkstoff behandelt wurde. Die Erfindung bezieht sich auch auf Saatgut, welches zur gleichen Zeit mit einem Wirkstoff der Formel I und Mischungspartner behandelt wurde. Die Erfindung bezieht sich weiterhin auf Saatgut, welches zu unterschiedlichen Zeiten mit einem Wirkstoff der Formel I und Mischungspartner behandelt wurde. Bei Saatgut, welches zu unterschiedlichen Zeiten mit einem Wirkstoff der Formel I und Mischungspartner behandelt wurde, können die einzelnen Wirkstoffe des erfindungsgemäßen Mittels in unterschiedlichen Schichten auf dem Saatgut enthalten sein. Dabei können die Schichten, die einen Wirkstoff der Formel (I) und Mischungspartner enthalten, gegebenenfalls durch eine Zwischenschicht getrennt sein. Die Erfindung bezieht sich auch auf Saatgut, bei dem ein Wirkstoff der Formel I und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit dem Wirkstoff der Formel (I) bzw. einer ihn enthaltenden Wirkstoffkombination einem Filmcoating - Verfahren unterzogen wurde, um Staubabrieb am Saatgut zu vermeiden.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit Wirkstoff der Formel (I) bzw. ihn enthaltenden Wirkstoffkombination Keimung und Auflauf des behandelten Saatguts gefördert werden können.

Ebenso ist es als vorteilhaft anzusehen, dass Wirkstoffe der Formel (I) und die genannten Wirkstoffkombinationen insbesondere auch bei transgenem Saatgut eingesetzt werden können.

Zu nennen ist auch, dass Wirkstoffe der Formel (I) in Kombination mit Mitteln der Signaltechnologie eingesetzt werden können, wodurch beispielhaft eine bessere Besiedlung mit Symbionten, wie zum Beispiel Rhizobien, Mycorrhiza und/oder endophytischen Bakterien, stattfindet und/oder es zu einer optimierten Stickstofffixierung kommt.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit Wirkstoffen der Formel (I) bzw. einer Wirkstoffkombination eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt. Besonders bevorzugt handelt es sich dabei um ein heterologes Gen, das aus *Bacillus thuringiensis* stammt.

Im Rahmen der vorliegenden Erfindung wird der Wirkstoff der Formel (I) allein (bzw. als Wirkstoffkombination) oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe/Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe/ Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl-oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt des/der Wirkstoffs/Wirkstoffe in den Formulierungen und nach dem Saatgut. Die Aufwandmengen bei Wirkstoffen/Wirkstoffkombinationen liegen im Allgemeinen zwischen 0,001 und 50 g pro Kilogramm Saatgut, vorzugsweise zwischen 0,01 und 15 g pro Kilogramm Saatgut.

Aus dem Stand der Technik ist nicht bekannt, dass die Wirkstoffe der Formel (I) gegen biotische Stressfaktoren und/oder abiotischen Stress von Pflanzen oder im Hinblick auf das Pflanzenwachstum eine Wirkung zeigen.

Es wurde nun gefunden, dass die erfindungsgemäßen Wirkstoffe der Formel (I) zur Steigerung der pflanzeneigenen Abwehrkräfte (Pathogenabwehr in Pflanzen) geeignet sind.

Es ist bekannt, dass Pflanzen auf natürliche Stressbedingungen, wie beispielsweise Kälte, Hitze, Trockenheit, Verwundung, Pathogenbefall (Viren, Bakterien, Pilze), Insekten etc. aber auch auf Herbizide mit spezifischen oder unspezifischen Abwehrmechanismen reagieren (Pflanzenbiochemie, S. 393-462, Spektrum Akademischer Verlag, Heidelberg, Berlin, Oxford, Hans W. Heldt, 1996.; Biochemistry and Molecular Biology of Plants, S. 1102-1203, American Society of Plant Physiologists, Rockville, Maryland, eds. Buchanan, Gruissem, Jones, 2000). Dabei dienen z. B. durch Verwundung entstandene Zellwandbestandteile oder spezifische vom Pathogen stammende Signalsubstanzen als Induktoren pflanzlicher Signaltransduktionsketten, die am Ende zur Bildung von gegen den Stressfaktor gerichteten Abwehrmolekülen führen. Hierbei kann es sich beispielsweise um (a) niedermolekulare Substanzen, wie z.B. Phytoalexine, (b) nicht-enzymatische Proteine, wie z. B. "Pathogenesis-related proteins" (PR-Proteine), (c) enzymatische Proteine, wie beispielsweise Chitinasen, Glucanasen, oder (d) um spezifische Inhibitoren essentieller Proteine, wie beispielsweise um Protease-Inhibitoren, Xylanase-Inhibitoren, handeln, welche das Pathogen direkt angreifen oder seine Proliferation behindern (Dangl and Jones, Nature 411, 826-833, 2001; Kessler and Baldwin, Annual Review of Plant Biology, 53, 299-328,2003).

Ein zusätzlicher Abwehrmechanismus ist die sogenannte hypersensitive Reaktion (HR), die über oxidativen Stress vermittelt wird und zum Absterben von Pflanzengewebe im Bereich eines Infektionsherdes führt, wodurch eine Ausbreitung von Pflanzenpathogenen, die auf lebende Zellen angewiesen sind, verhindert wird (Pennazio, New Microbiol. 18, 229-240, 1995).

Im weiteren Verlauf einer Infektion werden durch pflanzeneigene Botenstoffe Signale in nicht befallene Gewebe weitergegeben, die auch dort zur Auslösung von Abwehrreaktionen führen und die Entstehung von Sekundärinfektionen behindern (Systemic acquired resistance, SAR) (Ryals et al., The Plant Cell 8, 1809-1819, 1996).

Eine Reihe von pflanzenendogenen Signalstoffen, die in die Stresstoleranz bzw. die Pathogenabwehr involviert sind, sind bereits bekannt. Zu nennen sind hier beispielsweise Salicylsäure, Benzoesäure, Jasmonsäure oder Ethylen (Biochemistry and Molecular Biology of Plants, S. 850-929, American Society of Plant Physiologists, Rockville, Maryland, eds. Buchanan, Gruissem, Jones, 2000). Einige dieser Substanzen oder deren stabile synthetische Derivate und abgeleitete Strukturen sind auch bei externer Applikation auf Pflanzen oder Saatgutbeizung wirksam und aktivieren Abwehrreaktionen, die eine erhöhte Stress- bzw. Pathogentoleranz der Pflanze zur Folge haben (Sembdner, Parthier, Ann. Rev. Plant Physiol. Plant Mol. Biol. 44, 569-589, 1993). Die Salicylat-vermittelte Abwehr richtet sich besonders gegen phytopathogene Pilze, Bakterien und Viren (Ryals et al., The Plant Cell 8, 1809-1819, 1996).

Ein bekanntes synthetisches Produkt, das eine der Salicylsäure vergleichbare Funktion übernimmt und eine Schutzwirkung gegen phytopathogene Pilze, Bakterien und Viren vermitteln kann, ist Benzothiadiazol (CGA 245704; Common name: Acibenzolar-*S*-methyl; Handelsname: Bion^{®}) (Achuo et al., Plant Pathology 53 (1), 65-72, 2004; Tamblyn et al., Pesticide Science 55 (6), 676-677, 1999; EP-OS 0313512).

Andere Verbindungen, die in die Gruppe der Oxylipine gehören, wie z.B. Jasmonsäure, und die durch sie ausgelösten Schutzmechanismen sind besonders gegen Schadinsekten wirksam (Walling, J. Plant Growth Regul. 19, 195-216, 2000).

Desweiteren ist bekannt, dass die Behandlung von Pflanzen mit Insektiziden aus der Reihe der Neonikotinoide (Chlornikotinyle) zu einer erhöhten Resistenz der Pflanze gegenüber abiotischem Stress führt. Insbesondere gilt dies für das Imidacloprid (Brown et al., Beltwide Cotton Conference Proceedings 2231-2237, 2004). Dieser Schutz erfolgt durch Beeinflussung physiologischer und biochemischer Eigenschaften der Pflanzenzellen wie z.B. durch Verbesserung der Membranstabilität, Erhöhung der Kohlenhydratkonzentration, Steigerung der Polyolkonzentration und Antioxidantienaktivität (Gonias et al., Beltwide Cotton Conference Proceedings 2225-2229, 2004).

Darüber hinaus ist der Effekt von Chlornikotinylen gegen biotische Stressfaktoren bekannt (Crop Protection 19 (5), 349-354, 2000; Journal of Entomological Science 37(1), 101-112, 2002; Annals of Biology (Hisar, India) 19 (2), 179-181, 2003). Beispielsweise führen Insektizide aus der Reihe der Neonikotinoide (Chlornikotinyle) zu einer erhöhten Expression von Genen aus der Reihe der "Pathogenesis-related Proteins" (PR-Proteine). PR-Proteine unterstützen die Pflanzen primär in der Abwehr von biotischen Stressoren, wie z.B. phytopathogene Pilze, Bakterien und Viren (DE 10 2005 045 174 A; DE 10 2005 022 994 A und WO 2006/122662 A; Thielert Pflanzenschutz-Nachrichten Bayer, 59 (1), 73-86, 2006; Francis et al., European Journal of Plant Pathology, publ. online 23.1.2009).

Des Weiteren ist bekannt, dass die Behandlung von genetisch modifizierten Pflanzen mit Insektiziden aus der Reihe der Neonikotinoide (Chlornikotinyle) zu einer verbesserten Stresstoleranz der Pflanze führt (EP 1 731 037 A), beispielsweise auch gegenüber dem Herbizid Glyphosat (WO 2006/015697 A).

Somit ist bekannt, dass Pflanzen über mehrere endogene Reaktionsmechanismen verfügen, die eine wirksame Abwehr gegenüber verschiedensten Schadorganismen (biotischer Stress) und/oder abiotischem Stress bewirken können.

Die Anzucht von gesunden und gleichmäßig gewachsenen Jungpflanzen bildet eine wesentliche Voraussetzung für den großflächigen Anbau und die ökonomische Bestandesführung landwirtschaftlicher, gartenbaulicher und forstwirtschaftlicher Kulturpflanzen.

Zahlreiche Jungpflanzen-Anzuchtverfahren sind in der Land- und Forstwirtschaft sowie im Gartenbau etabliert. Hierbei werden als Anzuchtsubstrate neben gedämpfter Erde auch spezielle Substrate u.a. auf Basis von Torfmoosen, Kokosfasern, Steinwolle, wie z.B. Grodan^{®}, Bims, Blähton, wie z.B. Lecaton^{®} oder Lecadan^{®}, Tongranulate, wie z.B. Seramis^{®}, Schaumstoffe, wie z.B. Baystrat^{®}, Vermiculite, Perlite, künstliche Erden, wie z.B. Hygromull^{®}, oder Kombinationen dieser Substrate eingesetzt, in das entweder mit Fungiziden und/oder Insektiziden gebeiztes oder ungebeiztes Saatgut ausgesät wird.

In speziellen Kulturen, wie z.B. Tabak, werden Jungpflanzen zunehmend im sogenannten "Float-Verfahren" oder "Floating-Verfahren" angezogen (Leal, R. S., The use of Confidor S in the float , a new tobacco seedlings production system in the South of Brazil. Pflanzenschutz-Nachrichten Bayer (Deutsche Ausgabe) (2001), 54(3), Seiten 337 bis 352; Rudolph, R. D.; Rogers, W. D.; The efficacy of imidacloprid treatment for reduction in the severity of insect vectored virus diseases of tobacco. Pflanzenschutz-Nachrichten Bayer (Deutsche Ausgabe) (2001), 54(3), Seiten 311 bis 336). Bei diesem Verfahren wird das Saatgut in speziellen Behältern, z.B. Styropor-Lochtabletts, in spezieller Anzuchterde auf Basis Torf-Kultur-Substrat ausgesät und anschließend in Containern mit geeigneter Nährlösung bis zum Erreichen der gewünschten Verpflanzungsgröße kultiviert (Abbildung 1). Dabei gestattet man den Behältern auf der Nährlösung zu treiben, wovon sich der Name der Anzuchtmethode ableitet (Leal, 2001, s.o.). In Floating-Verfahren werden seit einigen Jahren zur Bekämpfung von saugenden Schädlingen Insektizide aus der Klasse der Neonicotiniode (Chlornikotinyle) eingesetzt. Üblicherweise werden die Pflanzen im Float-Verfahren kurz vor dem Verpflanzen mit Neonikotinoid (Chlornikotinyle) Insektiziden besprüht oder unmittelbar vor oder beim Verpflanzen ins Feld mit Neonikotinoid (Chlornikotinyle) Insektiziden angegossen, was als "Drenching" bezeichnet wird (Leal, 2001, s.o.; Rudolph and Rogers, 2001, s.o.). Beide Applikationsverfahren sind technisch relativ aufwendig.

Zum Schutz des auflaufenden Saat- oder Pflanzgutes vor pilzlichen Krankheitserregern und Schädlingen werden hierbei bis zur Verpflanzung Fungizide und Insektizide verwendet. Die Wahl der Pflanzenschutzmittel, der Ort und Zeitpunkt der Anwendung sowie die Aufwandmenge der Mittel richten sich hierbei vor allem nach der Art der auftretenden Pilzkrankheiten und Schädlinge, der spezifischen Wirkungsweise und Wirkungsdauer der Mittel sowie deren Pflanzenverträglichkeit, und kann somit unmittelbar an die spezifischen Erfordernisse unterschiedlicher Kulturen und Regionen angepasst werden.

Die Wirkstoffeder Formel (I) führen dabei unabhängig von einer Insektenbekämpfung zu einem guten Schutz der Pflanze vor Schäden durch pilzliche, bakterielle oder virale Pathogene.

Ohne an eine Theorie gebunden sein zu wollen wird zurzeit davon ausgegangen, dass die Abwehr der Pathogene durch die Induktion von PR Proteinen als Folge einer Behandlung mit mindestens einem Wirkstoff der Formel (I) erfolgt.

Insbesondere zeigt die erfindungsgemäße Verwendung in der Saatgutbehandlung, in der Bodenbehandlung, in speziellen Anzucht- und Kultivierungsverfahren (z.B. Floating Box, Rockwool, Hydroponic), aber auch Stamm- und Blattbehandlung die beschriebenen Vorteile. Kombinationen eines Wirkstoffs der Formel (I) unter anderem mit Insektiziden, Fungiziden und Bakteriziden zeigen synergistische Wirkung bei der Bekämpfung von Pflanzenkrankheiten. Die kombinierte Verwendung der Wirkstoffe der Formel (I) mit gentechnisch veränderten Sorten in Bezug auf erhöhte abiotische Stresstoleranz führt darüberhinaus zu einer synergistischen Verbesserung des Wachstums.

Schließlich wurde erfindungsgemäß auch gefunden, dass die Wirkstoffe der Formel (I) nicht nur zur Steigerung der Pathogenabwehr in Pflanzen, sondern auch zur Verbesserung des Pflanzenwachstums und/oder zur Steigerung der Widerstandfähigkeit von Pflanzen gegenüber Pflanzenkrankheiten, welche durch Pilze, Bakterien, Viren, MLO (Mycoplasma-like organisms) und/oder RLO (Rickettsia-like organisms) verursacht werden, insbesondere gegenüber bodenbürtigen Pilzkrankheiten, und/oder zur Erhöhung der Widerstandsfähigkeit von Pflanzen gegenüber abiotischen Stressfaktoren geeignet sind.

Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Gegenstand der vorliegenden Erfindung ist somit zunächst die Verwendung von mindestens einem Wirkstoff der Formel (I) zur Steigerung von pflanzeneigenen Abwehrkräften und/oder zur Verbesserung des Pflanzenwachstums und/oder zur Steigerung der Widerstandfähigkeit von Pflanzen gegenüber Pflanzenkrankheiten, welche durch Pilze, Bakterien, Viren, MLO (Mycoplasma-like organisms) und/oder RLO (Rickettsia-like organisms) verursacht werden, insbesondere gegenüber bodenbürtige Pilzkrankheiten, und/oder zur Steigerung der Widerstandsfähigkeit von Pflanzen gegenüber abiotischen Stressfaktoren.

Unter der Bezeichnung Pflanzenwachstum werden im Rahmen der vorliegenden Erfindung verschiedenartige Vorteile für Pflanzen verstanden, die nicht unmittelbar mit der bekannten pestiziden Wirksamkeit, bevorzugt der insektiziden Wirksamkeit der Wirkstoffe der Formel (I) verbunden sind. Solche vorteilhaften Eigenschaften sind beispielsweise die nachfolgend genannten verbesserten Pflanzencharakteristika: beschleunigte Keimung und Auflaufen des Saat- und Pflanzgutes, verbessertes Wurzelwachstum hinsichtlich Oberfläche und Tiefe, vermehrte Ausläuferbildung oder Bestockung, stärkere und produktivere Ausläufer und Bestockungstriebe, Verbesserung des Sprosswachstums, erhöhte Standfestigkeit, vergrößerter Sprossbasisdurchmesser, vergrößerte Blattfläche, grünere Blattfarbe, höhere Erträge an Nähr- und Inhaltsstoffen, wie z.B. Kohlenhydrate, Fette, Öle, Proteine, Vitamine, Mineralstoffe, ätherische Öle, Farbstoffe, Fasern, bessere Faserqualität, früheres Blühen, gesteigerte Blütenanzahl, reduzierter Gehalt an toxischen Produkten wie Mycotoxine, reduzierter Gehalt an Rückständen oder unvorteilhaften Bestandteilen jeglicher Art oder bessere Verdaulichkeit, verbesserte Lagerstabilität des Erntegutes, verbesserte Toleranz gegenüber unvorteilhaften Temperaturen, verbesserte Toleranz gegenüber Dürre und Trockenheit wie auch Sauerstoffmangel durch Wasserüberschuss, verbesserte Toleranz gegenüber erhöhten Salzgehalten in Böden und Wasser, gesteigerte Toleranz gegenüber UV-Strahlung, gesteigerte Toleranz gegenüber Ozonstress, verbesserte Verträglichkeit gegenüber Herbiziden und anderen Pflanzenbehandlungsmitteln, verbesserte Wasseraufnahme und Photosyntheseleistung, vorteilhafte Pflanzeneigenschaften, wie beispielsweise Beschleunigung der Reifung, gleichmäßigere Abreife, größere Anziehungskraft für Nützlinge, verbesserte Bestäubung oder andere Vorteile, die einem Fachmann durchaus bekannt sind.

Die weiter oben genannten verschiedenartigen Vorteile für Pflanzen lassen sich bekannterweise partiell zusammenfassen und mit allgemein gültigen Begriffen belegen. Soche Begriffe sind beispielsweise die nachfolgend aufgeführten Bezeichnungen: phytotonischer Effekt, Widerstandsfähigkeit gegenüber Stressfaktoren, weniger Pflanzenstress, Pflanzengesundheit, gesunde Pflanzen, Pflanzenfitness ("Plant Fitness"), "Plant Wellness", "Plant Concept", "Vigor Effect", "Stress Shield", Schutzschild, "Crop Health", "Crop Health Properties", "Crop Health Products", "Crop Health Management", "Crop Health Therapy", "Plant Health", "Plant Health Properties", "Plant Health Products", "Plant Health Management", "Plant Health Therapy", Grünungseffekt ("Greening Effect" oder "Re-greening Effect"), "Freshness" oder andere Begriffe, die einem Fachmann durchaus bekannt sind.

Es wurde ferner gefunden, dass Wirkstoffe der Formel (I) zu einer erhöhten Expression von Genen aus der Reihe der "Pathogenesis-related proteins" (PR-Proteine) führen. PR-Proteine unterstützen die Pflanzen primär in der Abwehr von biotischen Stressoren, wie z.B. phytopathogene Pilze, Bakterien und Viren. Dies hat zur Folge, dass Pflanzen nach Anwendung von Wirkstoffen der Formel (I) besser geschützt sind vor Infektionen phytopathogener Pilze, Bakterien und Viren. Bei notwendigem Einsatz von Insektiziden, Fungiziden und Bakteriziden in Mischung wie auch bei sequentieller Anwendung mit Wirkstoffen der Formel (I) wird deren Wirkung unterstützt.

Erfindunsgemäß wurde darüber hinaus gefunden, dass die Anwendung der Wirkstoffe der Formel (I) in Kombination mit einem Düngemittel wie weiter unten stehend definiert auf Pflanzen oder in deren Umgebung einen synergistischen wachstumssteigernden Effekt bewirkt.

Düngemittel die erfindungsgemäß zusammen mit den oben näher erläuterten Wirkstoffen oder Mitteln verwendet werden können sind im Allgemeinen organische und anorganische Stickstoff-haltige Verbindungen wie beispielsweise Harnstoffe, Harnstoff-Formaldehyd-Kondensationsprodukte, Aminosäuren, Ammoniumsalze und -nitrate, Kaliumsalze (bevorzugt Chloride, Sulfate, Nitrate), Phosphorsäuresalze und/oder Salze von Phosphoriger Säure (bevorzugt Kaliumsalze und Ammoniumsalze). Insbesondere zu nennen sind in diesem Zusammenhang die NPK-Dünger, d.h. Düngemittel, die Stickstoff, Phosphor und Kalium enthalten, Kalkammonsalpeter, d.h. Düngemittel, die noch Calcium enthalten, Ammonsulfatsalpeter (Allgemeine Formel (NH₄)₂SO₄ NH₄NO₃), Ammonphosphat und Ammonsulfat. Diese Düngemittel sind dem Fachmann allgemein bekannt, siehe auch beispielsweise Ullmann's Encyclopedia of Industrial Chemistry, 5. Edition, Vol. A 10, Seiten 323 bis 431, Verlagsgesellschaft, Weinheim, 1987.

Die Düngemittel können auch Salze aus Mikronährstoffen (bevorzugt Calcium, Schwefel, Bor, Mangan, Magnesium, Eisen, Bor, Kupfer, Zink, Molybdän und Kobalt) und Phytohormonen (z. B. Vitamin B1 und Indol-3-ylessigsäure (IAA)) oder Gemische davon enthalten. Erfindungsgemäß eingesetzte Düngemittel können auch weitere Salze wie Monoammoniumphosphat (MAP), Diammoniumphosphat (DAP), Kaliumsulfat, Kaliumchlorid oder Magnesiumsulfat enthalten. Geeignete Mengen für die sekundären Nährstoffe oder Spurenelemente sind Mengen von 0,5 bis 5 Gew.-%, bezogen auf das gesamte Düngemittel. Weitere mögliche Inhaltsstoffe sind Pflanzenschutzmittel, Insektizide oder Fungizide, Wachstumsregulatoren oder Gemische davon. Hierzu folgen weiter unten weitergehende Ausführungen.

Die Düngemittel können beispielsweise in Form von Pulvern, Granulaten, Prills oder Kompaktaten eingesetzt werden. Die Düngemittel können jedoch auch in flüssiger Form, gelöst in einem wässrigen Medium, eingesetzt werden. In diesem Fall kann auch verdünnter wässriger Ammoniak als Stickstoffdüngemittel eingesetzt werden. Weitere mögliche Inhaltsstoffe für Düngemittel sind beispielsweise in Ullmann's Encyclopedia of Industrial Chemistry, 5. Auflage, 1987, Band A 10, Seiten 363 bis 401, DE-A 41 28 828, DE-A 19 05 834 und DE-A 196 31 764 beschrieben.

Die allgemeine Zusammensetzung der Düngemittel, bei welchen es sich im Rahmen der vorliegenden Erfindung um Einzelnährstoff- und/oder Mehrnährstoffdünger handeln kann, beispielsweise aus Stickstoff, Kalium oder Phosphor, kann innerhalb eines breiten Bereichs variieren. Im Allgemeinen ist ein Gehalt von 1 bis 30 Gew.-% Stickstoff (bevorzugt 5 bis 20 Gew.-%), von 1 bis 20 Gew.-% Kalium (bevorzugt 3 bis 15 Gew.-%) und ein Gehalt von 1 bis 20 Gew.-% Phosphor (bevorzugt 3 bis 10 Gew.- %) vorteilhaft. Der Gehalt von Mikroelementen ist üblicherweise im ppm Bereich, bevorzugt im Bereich von 1 bis 1000 ppm.

Im Rahmen der vorliegenden Erfindung kann das Düngemittel sowie der Wirkstoff der Formel (I) zeitgleich, d.h. synchron, verabreicht werden. Es ist jedoch auch möglich, zunächst das Düngemittel und dann den Wirkstoff der Formel (I) oder zunächst den Wirkstoff der Formel (I) und dann das Düngemittel anzuwenden. Bei nicht zeitgleicher Anwendung des Wirkstoffs der Formel (I) und des Düngemittels erfolgt im Rahmen der vorliegenden Erfindung jedoch die Anwendung in funktionellem Zusammenhang, insbesondere innerhalb eines Zeitraums von im Allgemeinen 24 Stunden, bevorzugt 18 Stunden, besonders bevorzugt 12 Stunden, speziell 6 Stunden, noch spezieller 4 Stunden, noch weiter spezieller innerhalb 2 Stunden. In ganz besonderen Ausführungsformen der vorliegenden Erfindung erfolgt die Anwendung der erfindungsgemäßen Wirkstoffe der allgemeinen Formel (I) und des Düngemittels in einem zeitlichen Rahmen von weniger als 1 Stunde, vorzugsweise weniger als 30 Minuten, besonders bevorzugt weniger als 15 Minuten.

Darüber hinaus ist es möglich, formstabile Mischungen, beispielsweise in der Form von Stäbchen, Granulaten, Tabletten etc., ausgehend von mindestens einem erfindungsgemäß zu verwendenden Wirkstoff und mindestens einem Düngemittel herzustellen. Um eine entsprechende formstabile Mischung herzustellen, können die entsprechenden Bestandteile miteinander gemischt und gegebenenfalls extrudiert werden bzw. kann der mindestens eine erfindungsgemäß zu verwendende Wirkstoff der Formel (I) auf das Düngemittel aufgezogen werden. Gegebenenfalls können auch Formulierungshilfsmittel in den formstabilen Mischungen, wie beispielsweise Streckmittel oder Haftkleber, verwendet werden, um eine Formstabilität der resultierenden Mischung zu erreichen. Durch die entsprechende Formstabilität eignen sich entsprechende Mischungen insbesondere für die Anwendung im Bereich "Home & Garden", d.h. bei einem Privatanwender oder Hobbygärtner, welche die formstabile Mischung bzw. die darin enthaltenden Bestandteile mit einer vorgegebenen, klar definierten Menge und ohne besondere Hilfsmittel verwenden können.

Unabhängig hiervon können die Mischungen aus mindestens einem der erfindungsgemäß zu verwendenden Wirkstoffe und dem mindestens einen Düngemittel auch flüssig vorliegen, so dass - beispielsweise bei einem professionellen Anwender im Bereich der Landwirtschaft - die resultierende Mischung als so genannte Tanklösung ausgebracht werden kann.

Durch die Verwendung mindestens eines der erfindungsgemäß zu verwendenen Wirkstoffe und mindestens einem Düngemittel wird ein vergrössertes Wurzelwachstum ermöglicht, welches wiederum eine höhere Nährstoffaufnahme ermöglicht und damit das Planzenwachstum fördert.

Die erfindungsgemäß zu verwendenden Wirkstoffe können, gegebenenfalls in Kombination mit Düngemitteln, bevorzugt an folgenden Pflanzen angewendet werden, wobei die folgende Aufzählung nicht beschränkend ist.

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten, die aus Teilen der Bäume hergestellt werden.

Der Begriff Nutzpflanzen, wie hier verwendet, bezeichnet Kulturpflanzen, die als Pflanzen für die Gewinnung von Nahrungsmitteln, Futtermitteln, Treibstoffen oder für technische Zwecke eingesetzt werden.

Zu den Nutzpflanzen zählen z.B. folgende Pflanzenarten: Turf, Reben, Getreide, beispielsweise Weizen, Gerste, Roggen, Hafer, Triticale, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen sind folgende Pflanzen anzusehen: Bamwolle, Aubergine, Turf, Kernobst, Steinobst, Beerenobst, Mais, Weizen, Gerste, Gurke, Tabak, Reben, Reis, Getreide, Birne, Bohnen, Sojabohnen, Raps, Tomate, Paprika, Melonen, Kohl, Kartoffel und Apfel.

Als Bäume seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus.

Als besonders bevorzugte Bäume können genannt werden: Aus der Baumart Pinus: P. radiate, P. ponderosa, P. contorta, P. sylvestre, P. strobes; aus der Baumart Eucalyptus: E. grandis, E. globulus, E. camadentis.

Als ganz besonders bevorzugte Bäume können genannt werden: Rosskastanie, Platanengewächse, Linde, Ahornbaum.

Die vorliegende Erfindung kann auch an beliebigen Rasenarten ("turfgrasses") durchgeführt werden, einschließlich "cool season turfgrasses" und "warm season turfgrasses". Beispiele für Rasenarten für die kalte Jahreszeit sind Blaugräser ("blue grasses"; *Poa spp*.), wie "Kentucky bluegrass" (*Poa pratensis* L.), "rough bluegrass" (*Poa trivialis* L.), "Canada bluegrass" (*Poa compressa* L.), "annual bluegrass" (*Poa annua* L.), "upland bluegrass" (Poa *glaucantha Gaudin*), "wood bluegrass" (*Poa nemoralis* L.) und "bulbous bluegrass" (*Poa bulbosa* L.); Straussgräser ("Bentgrass", *Agrostis spp*.), wie "creeping bentgrass" (*Agrostis palustris* Huds.), "colonial bentgrass" (*Agrostis tenuis* Sibth.), "velvet bentgrass" (*Agrostis canina* L.), "South German Mixed Bentgrass" (*Agrostis spp.* einschließlich *Agrostis tenius* Sibth., *Agrostis canina* L., und *Agrostis palustris* Huds.), und "redtop" (*Agrostis alba* L.);
Schwingel ("Fescues", *Festucu spp.*), wie "red fescue" (*Festuca rubra* L. spp. *rubra*), "creeping fescue" (*Festuca rubra* L.), "chewings fescue" (*Festuca rubra commutata* Gaud.), "sheep fescue" (*Festuca ovina* L.), "hard fescue" (*Festuca longifolia* Thuill.), "hair fescue" *(Festucu capillata* Lam.), "tall fescue" (*Festuca arundinacea* Schreb.) und "meadow fescue" (*Festuca elanor* L.);
Lolch ("ryegrasses", *Lolium* spp.), wie "annual ryegrass" (*Lolium multiflorum* Lam.), "perennial ryegrass" *(Lolium perenne* L.) und "italian ryegrass" *(Lolium multiflorum* Lam.);
und Weizengräser ("wheatgrasses", *Agropyron* spp..), wie "fairway wheatgrass" (*Agropyron cristatum* (L.) Gaertn.), "crested wheatgrass" (*Agropyron desertorum* (Fisch.) Schult.) und "western wheatgrass" (*Agropyron smithii* Rydb.).

Beispiele für weitere "cool season turfgrasses" sind "beachgrass" (*Ammophila breviligulata* Fern.), "smooth bromegrass" (*Bromus inermis* Leyss.), Schilf ("cattails") wie "Timothy" (*Phleum pratense* L.), "sand cattail" (*Phleum subulatum* L.), "orchardgrass" (*Dactylis glomerata* L.), "weeping alkaligrass" (*Puccinellia distans* (L.) Parl.) und "crested dog's-tail" (*Cynosurus cristatus* L.).

Beispiele für "warm season turfgrasses" sind "Bermudagrass" (*Cynodon spp.* L. C. Rich), "zoysiagrass" (*Zoysia spp.* Willd.), "St. Augustine grass" (*Stenotaphrum secundatum* Walt Kuntze), "centipedegrass" (*Eremochloa ophiuroides* Munro Hack.), "carpetgrass" (*Axonopus affinis* Chase), "Bahia grass" (*Paspalum notatum* Flugge), "Kikuyugrass" *(Pennisetum clandestinum* Hochst. ex Chiov.), "buffalo grass" *(Buchloe dactyloids* (Nutt.) Engelm.), "Blue gramma" *(Bouteloua gracilis* (H.B.K.) Lag. ex Griffiths), "seashore paspalum" (*Paspalum vaginatum* Swartz) und "sideoats grama" (*Bouteloua curtipendula* (Michx. Torr.). "Cool season turfgrasses" sind für die erfindungsgemäße Verwendung im Allgemeinen bevorzugt. Besonders bevorzugt sind Blaugras, Straussgras und "redtop", Schwingel und Lolch. Straussgras ist insbesondere bevorzugt.

Die Wirkstoffe der Formel (I) und ihre Zusammensetzungen eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Wirkstoffe oder Zusammensetzungen allein oder in Kombination mit anderen Wirk- und/oder Hilfsstoffen verwendet. Bevorzugt werden sie in Haushaltsinsektizid-Produkten verwendet. Die erfindungsgemäßen Wirkstoffe sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Darüber hinaus können die Wirkstoffe der Formel (I) zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Auf dem Gebiet der Tiergesundheit, d.h. dem Gebiet der Tiermedizin, sind die erfindungsgemäßen Wirkstoffe gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die erfindungsgemäßen Verbindungen, die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z.B. in der Aquakultur; oder gegebenenfalls auch Insekten wie Bienen.

Zu den Haustieren zählen zum Beispiel Säugetiere wie Hamster, Meerschweinchen, Ratten, Mäuse, Chinchillas, Frettchen oder insbesondere Hunde, Katzen; Stubenvögel; Reptilien; Amphibien oder Aquariumfische.

Gemäß einer bevorzugten Ausführungsform werden die erfindungsgemäßen Verbindungen an Säugetiere verabreicht.

Gemäß einer weiteren bevorzugten Ausführungsform werden die erfindungsgemäßen Verbindungen an Vögel, nämlich Stubenvögel oder insbesondere Geflügel, verabreicht.

Durch Verwendung der erfindungsgemäßen Wirkstoffe für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Wirkstoffe wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass der Wirkstoff den jeweiligen Parasiten abtöten, sein Wachstum verhindern oder seine Vermehrung verhindern kann.

Zu Beispielen für Arthropoden zählen, jedoch ohne Einschränkung:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.; aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin sind unter den Arthropoden die folgenden Akari beispielhaft, jedoch ohne Einschränkung, zu nennen:
aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Zu Beispielen für parasitäre Protozoen zählen, jedoch ohne Einschränkung:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis.

Sarcomastigophora (Rhizopoda), wie Entamoebidae, zum Beispiel Entamoeba histolytica, Hartmanellidae, zum Beispiel Acanthamoeba sp., Harmanella sp.

Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec.

Zu Beispielen für pathogene Endoparasiten, bei denen es sich um Helminthen handelt, zählen Plattwürmer (z.B. Monogenea, Cestodes und Trematodes), Rundwürmer, Acanthocephala und Pentastoma. Zu weiteren Helminthen zählen, jedoch ohne Einschränkung:
Monogenea: z.B.: Gyrodactylus spp., Dactylogyrus spp., Polystoma spp.

Cestodes: aus der Ordnung Pseudophyllidea zum Beispiel: Diphyllobothrium spp., Spirometra spp., Schistocephalus spp., Ligula spp., Bothridium spp., Diplogonoporus spp.

Aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.

Ründwürmer: Trichinellida zum Beispiel: Trichuris spp., Capillaria spp., Trichomosoides spp., Trichinella spp.

Aus der Ordnung Tylenchida zum Beispiel: Micronema spp., Strongyloides spp.

Aus der Ordnung Rhabditina zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp.

Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.,

Aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der erfindungsgemäßen Wirkstoffe nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf erfindungsgemäße Verbindungen für die Verwendung als Arzneimittel.

Ein weiterer Aspekt bezieht sich auf erfindungsgemäße Verbindungen für die Verwendung als Antiendoparasitikum, insbesondere ein Helminthizid oder ein Mittel gegen Protozoen. Zum Beispiel erfindungsgemäße Verbindungen für die Verwendung als Antiendoparasitikum, insbesondere ein Helminthizid oder Mittel gegen Protozoen, z.B. in der Tierzucht, in der Tierhaltung, in Ställen und auf dem Hygienesektor.

Ein weiterer Aspekt wiederum betrifft erfindungsgemäße Verbindungen für die Verwendung als Antiektoparasitikum, insbesondere ein Arthropodizid wie ein Insektizid oder ein Akarizid. Zum Beispiel erfindungsgemäße Verbindungen für die Verwendung als Antiektoparasitikum, insbesondere ein Arthropodizid wie ein Insektizid oder Akarizid, zum Beispiel in der Tierhaltung, in der Tierzucht, in Ställen, auf dem Hygienesektor.

Die Wirkstoffe der Formel (I) und sie enthaltende Zusammensetzungen eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer erfindungsgemäßen Ausführungsform enthalten die erfindungsgemäßen Zusammensetzungen oder Mittel noch mindestens ein weiteres Insektizid und/oder mindestens ein Fungizid.

In einer weiteren Ausführungsform ist diese erfindungsgemäße Zusammensetzung eine anwendungsfertige (ready-to-use) Zusammensetzung, d.h., sie kann ohne weitere Änderungen auf das entsprechende Material aufgebracht werden. Als weitere Insektizide oder als Fungizide kommen die oben genannten in Frage.

Überraschenderweise wurde auch gefunden, dass die erfindungsgemäßen Wirkstoffe und Zusammensetzungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die erfindungsgemäßen Wirkstoffe und Zusammensetzungen allein oder in Kombinationen mit anderen Wirkstoffen als Antifouling-Mittel eingesetzt werden.

Die folgenden Herstellungs- und Verwendungsbeispiele illustrieren die Erfindung.

### Herstellungsbeispiele

### Synthesebeispiel 1

### 2-Brom-N-[1-(2-methylphenyl)-1H-pyrazol-3-yl]benzamid (Verbindung I-1-1 in Tabelle 1)

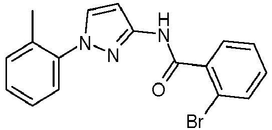

1-(2-Methylphenyl)-1H-pyrazol-3-amin (255 mg) wurde in N,N-Dimethylformamid (2 mL) vorgelegt, bei Raumtemperatur mit 2-(7-Aza-1H-benzotriazol-1-yl)-1,1,3,3-tetramethyluronium hexafluorophosphat (482 mg) und Diisopropylethylamin (0,59 mL) versetzt und für 1 h gerührt. Dann wurde eine Lösung von 2-Brombenzoesäure (200 mg) in N,N-Dimethylformamid (1 mL) zugegeben und für weitere 2 h gerührt. Anschließend wurde das Reaktionsgemisch mit eiskaltem Wasser versetzt und mit Dichlormethan extrahiert. Die vereinigten organischen Phasen wurden nacheinander mit Natriumhydrogencarbonatlösung, Wasser und gesättigter Natriumchloridlösung gewaschen, über Natriumsulfat getrocknet und im Vakuum zur Trockne eingeengt. Der Rückstand wurde an Kieselgel mit Petrolether/Ethylacetat als Laufmittel säulenchromatographisch (isochratisch bei 12 % Ethylacetat in Petrolether) gereinigt. Es wurden 150 mg der Titelverbindung erhalten. HPLC-MS: logP = 2,79; Masse (m/z): 356,0 (M+H)⁺; ¹H-NMR (DMSO-D₆) 2.26 (s, 3H), 6.87 (d, 1H), 7.32 - 7.42 (m, 5H), 7.44 - 7.48 (m, 1H), 7.52 - 7.54 (m, 1H), 7.68 - 7.70 (m, 1H), 7.98 (d, 1H), 11.13 (br. s, 1H).

### Synthesebeispiel 2

### 2-Chlor-N-[1-(2,6-dimethylphenyl)-1H-pyrazol-3-yl]benzamid (Verbindung I-1-61 in Tabelle 1)

### Stufe 1: 1-(2,6-Dimethylphenyl)-1H-pyrazol-3-amin (gemäß Route B-1)

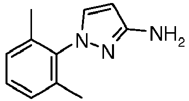

2,6-Dimethylphenylhydrazinhydrochlorid (3,50 g) wurde in Ethanol (30 mL) vorgelegt, mit Natriummethylatlösung (3,29 g in 20 mL Ethanol) und 3-Ethoxyacrylsäurenitril (2,95 g) versetzt, für 9 h unter Rückfluss erhitzt und anschließend über Nacht bei Raumtemperatur gerührt. Das Reaktionsgemisch wurde anschließend im Vakuum eingeengt, in Ethylacetat aufgenommen, mit Wasser gewaschen, über Natriumsulfat getrocknet und im Vakuum zur Trockne eingeengt. Der Rückstand wurde zunächst an Kieselgel mit Cyclohexan/Ethylacetat als Laufmittel säulenchromatographisch (Gradient = 2 h von 100 % Cyclohexan auf 100 % Ethylacetat) gereinigt und dann an einer präparativen HPLC mit Wasser/Acetonitril als Laufmittel chromatographiert (Gradient = 40 min von 10 % Acetonitril in Wasser auf 100 % Acetonitril). Es wurden 39 mg der Titelverbindung erhalten. HPLC-MS: logP = 0,62; Masse (m/z): 188,1 (M+H)⁺; ¹H-NMR (CD₃CN) 2.00 (s, 6H), 4.03 (br. s, 2H), 5.57 (d, 1H), 7.21 - 7.23 (m, 2H), 7.30 - 7.33 (m, 1H), 7.36 (d, 1H).

### Stufe 2: 2-Chlor-N-[1-(2,6-dimethylphenyl)-1H-pyrazol-3-yl]benzamid

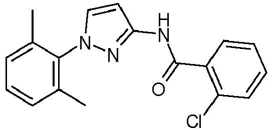

1-(2,6-Dimethylphenyl)-1H-pyrazol-3-amin (37 mg) wurde in Dichlormethan (2 mL) vorgelegt, bei 0 °C mit Triethylamin (0,14 mL) und einer Lösung von 2-Chlorbenzoylchlorid (35 mg) in Dichlormethan (1 mL) versetzt und über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 15 mg der Titelverbindung erhalten. HPLC-MS: logP = 2,63; Masse (m/z): 324,1 (M+H)⁺; ¹H-NMR (CD₃CN) 1.96 (s, 6H), 6.71 (d, 1H), 7.18 - 7.20 (m, 2H), 7.29 - 7.44 (m, 6H), 7.65 - 7.66 (d, 1H), 8.46 (br. s, 1H).

### Synthesebeispiel 3

### 2-Brom-N-[1-(2,3-difluorphenyl)-1H-pyrazol-3-yl]benzamid (Verbindung I-1-63 in Tabelle 1)

### Stufe1: 1-(2,3-Difluorphenyl)-1H-pyrazol-3-amin (gemäß Route B-1)

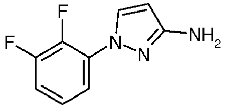

2,3-Difluorphenylhydrazinhydrochlorid (5,00 g) wurde in Ethanol (30 mL) vorgelegt, mit Natriummethylatlösung (4,49 g in 20 mL Ethanol) und 3-Ethoxyacrylsäurenitril (4,03 g) versetzt und über Nacht unter Rückfluss erhitzt. Das Reaktionsgemisch wurde anschließend im Vakuum eingeengt, in Ethylacetat aufgenommen, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 1,79 g der Titelverbindung erhalten. HPLC-MS: logP = 1,56; Masse (m/z): 196,0 (M+H)⁺; ¹H-NMR (CD₃CN) 4.26 (br. s, 2H), 5.89 (d, 1H), 7.05 - 7.12 (m, 1H), 7.16 - 7.22 (m, 1H), 7.54 - 7.59 (m, 1H), 7.82 - 7.84 (m, 1H). Als Nebenprodukt wurden 193 mg 1-(2-Ethoxy-3-fluorphenyl)-1H-pyrazol-3-amin erhalten: HPLC-MS: logP = 2,04; Masse (m/z): 222,1 (M+H)⁺; ¹H-NMR (CD₃CN) 1.32 (t, 3H), 4.08 (q, 2H), 4.56 (br. s, 2H), 4.65 (d, 1H), 7.00 - 7.11 (m, 3H), 7.46 (d, 1H).

### Stufe 2: 2-Brom-N-[1-(2,3-difluorphenyl)-1H-pyrazol-3-yl]benzamid

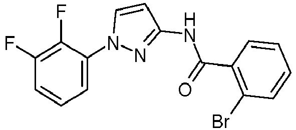

1-(2,3-Difluorphenyl)-1H-pyrazol-3-amin (100 mg) wurde in Dichlormethan (2 mL) vorgelegt, bei 0 °C mit Triethylamin (0,36 mL) und einer Lösung von 2-Brombenzoylchlorid (113 mg) in Dichlormethan (1 mL) versetzt und über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 116 mg der Titelverbindung erhalten. HPLC-MS: logP = 2,97; Masse (m/z): 376,0 (M+H)⁺; ¹H-NMR (CD₃CN) 7.03 - 7.04 (m, 1H), 7.20 - 7.29 (m, 2H), 7.36 - 7.47 (m, 2H), 7.53 - 7.58 (m, 2H), 7.66 - 7.68 (m, 1H), 8.03 - 8.04 (m, 1H), 9.30 (br. s, 1H).

### Synthesebeispiel 4

### N-[1-(2,5-Difluorphenyl)-1H-pyrazol-3-yl]-5-fluor-1-methyl-3-(trifluormethyl)-1H-pyrazol-4-carboxamid (Verbindung I-1-72 in Tabelle 1)

### Stufe 1: 1-(2,5-Difluorphenyl)-1H-pyrazol-3-amin (gemäß Route B-1)

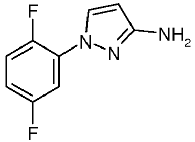

2,5-Difluorphenylhydrazin (3,62 g) wurde in Ethanol (10 mL) vorgelegt, mit Natriummethylatlösung (4,07 g in 10 mL Ethanol) und 3-Ethoxyacrylsäurenitril (3,66 g) versetzt und über Nacht unter Rückfluss erhitzt. Das Reaktionsgemisch wurde anschließend im Vakuum eingeengt, in Ethylacetat aufgenommen, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 2,88 g der Titelverbindung erhalten. HPLC-MS: logP = 1,58; Masse (m/z): 196,1 (M+H)⁺; ¹H-NMR (CD₃CN) 4.26 (br. s, 2H), 5.87 (d, 1H), 6.87 - 6.93 (m, 1H), 7.21 - 7.28 (m, 1H), 7.53 - 7.58 (m, 1H), 7.86 - 7.88 (m, 1H).

### Stufe 2: N-[1-(2,5-Difluorphenyl)-1H-pyrazol-3-yl]-5-fluor-1-methyl-3-(trifluormethyl)-1H-pyrazol-4-carboxamid

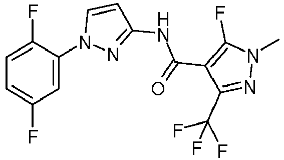

5-Fluor-1-methyl-3-(trifluormethyl)-1H-pyrazol-4-carbonsäure (130 mg) wurde in Dichlormethan (4 mL) vorgelegt, bei 0 °C mit 1-Ethyl-3-(3-dimethylaminopropyl)carbodiimid (147 mg) und 1-Hydroxy-1-H-benzotriazol (104 mg) versetzt und für 30 min gerührt. Dann wurde eine Lösung von 1-(2,5-Difluorphenyl)-1H-pyrazol-3-amin (100 mg) in Dichlormethan (1 mL) zugegeben und über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde zunächst an Kieselgel mit Cyclohexan/Ethylacetat als Laufmittel säulenchromatographisch (Gradient = 2 h von 100 % Cyclohexan auf 100 % Ethylacetat) gereinigt und dann an einer präparativen HPLC mit Wasser/Acetonitril als Laufmittel chromatographiert (Gradient = 40 min von 10 % Acetonitril in Wasser auf 100 % Acetonitril). Es wurden 16 mg der Titelverbindung erhalten. HPLC-MS: logP = 2,93; Masse (m/z): 390,1 (M+H)⁺; ¹H-NMR (CD₃CN) 3.85 (s, 3H), 6.97 (d, 1H), 7.07 - 7.14 (m, 1H), 7.34 - 7.40 (m, 1H), 7.61 - 7.66 (m, 1H), 8.09 - 8.10 (m, 1H), 9.08 (br. s, 1H).

### Synthesebeispiel 5

### 2-Chlor-N-[1-(2-ethoxy-6-nuorphenyl)-1H-pyrazol-3-yl]benzamid (Verbindung I-1-117 in Tabelle 1)

### Stufe 1: 1-(2-Ethoxy-6-fluorphenyl)-1H-pyrazol-3-amin (gemäß Route B-1)

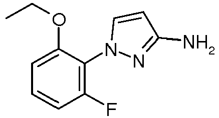

2,6-Difluorphenylhydrazin (5,00 g) wurde in Ethanol (30 mL) vorgelegt, mit Natriummethylatlösung (4,49 g in 20 mL Ethanol) und 3-Ethoxyacrylsäurenitril (4,03 g) versetzt und über Nacht unter Rückfluss erhitzt. Das Reaktionsgemisch wurde anschließend im Vakuum eingeengt, in Ethylacetat aufgenommen, mit Wasser gewaschen, über Natriumsulfat getrocknet und im Vakuum zur Trockne eingeengt. Der Rückstand wurde anschließend erneut in Ethanol (50 mL) gelöst und eine weitere Nacht unter Rückfluss erhitzt. Das Reaktionsgemisch wurde dann wieder im Vakuum eingeengt, in Ethylacetat aufgenommen, mit Wasser gewaschen, über Natriumsulfat getrocknet und im Vakuum zur Trockne eingeengt. Der Rückstand wurde danach an Kieselgel mit Cyclohexan/Ethylacetat als Laufmittel säulenchromatographisch (Gradient = 2 h von 100 % Cyclohexan auf 100 % Ethylacetat) gereinigt. Es wurden 152 mg der Titelverbindung erhalten. HPLC-MS: logP = 1,47; Masse (m/z): 222,1 (M+H)⁺; ¹H-NMR (CD₃CN) 1.27 (t, 3H), 3.80 - 4.20 (m, 4H), 5.75 (d, 1H), 6.81 - 6.86 (m, 1H), 6.90 - 6.92 (m, 1H), 7.31 - 7.36 (m, 2H).

### Stufe 2: 2-Chlor-N-[1-(2-ethoxy-6-fluorphenyl)-1H-pyrazol-3-yl]benzamid

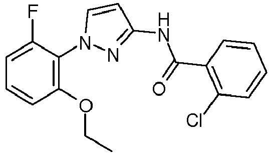

1-(2-Ethoxy-6-fluorphenyl)-1H-pyrazol-3-amin (71 mg) wurde in Dichlormethan (2 mL) vorgelegt, bei 0 °C mit Triethylamin (0,22 mL) und einer Lösung von 2-Chlorbenzoylchlorid (56 mg) in Dichlormethan (1 mL) versetzt und über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 60 mg der Titelverbindung erhalten. HPLC-MS: logP = 2,84; Masse (m/z): 360,1 (M+H)⁺; ¹H-NMR (CD₃CN) 1.30 (t, 3H), 4.12 (q, 2H), 6.89 - 7.00 (m, 3H), 7.42 - 7.53 (m, 4H), 7.61 - 7.62 (m, 1H), 7.66 - 7.67 (m, 1H), 9.31 (br. s, 1H).

### Synthesebeispiel 6

### 2-Brom-N-[1-(2,6-difluorphenyl)-4,5-dihydro-1H-pyrazol-3-yl]benzamid (Verbindung I-4-2 in Tabelle 2)

### Stufe 1: 1-(2,6-Difluorphenyl)-4,5-dihydro-1H-pyrazol-3-amin (gemäß Route C-1)

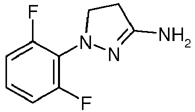

2,6-Difluorphenylhydrazinhydrochlorid (2,00 g) wurde in Ethanol (20 mL) vorgelegt, bei Raumtemperatur langsam Natriumethylat (21%ig in Ethanol, 3,02 g) zugetropft, für 10 min nachgerührt, mit Acrylsäurenitril (0,80 mL) versetzt und über Nacht unter Rückfluss erhitzt. Das Reaktionsgemisch wurde anschließend im Vakuum eingeengt, in Dichlormethan aufgenommen, mit Wasser gewaschen, über Natriumsulfat getrocknet und im Vakuum zur Trockne eingeengt. Es verblieben 1.40 g des Rohprodukts, das ohne weitere Reinigung für den nächsten Schritt eingesetzt wurde.

### Stufe 2: 2-Brom-N-[1-(2,6-difluorphenyl)-4,5-dihydro-1H-pyrazol-3-yl]benzamid

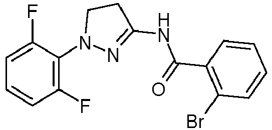

1-(2,6-Difluorphenyl)-4,5-dihydro-1H-pyrazol-3-amin (150 mg) wurde in Dichlormethan (2 mL) vorgelegt, bei 0 °C mit Triethylamin (0,32 mL) und einer Lösung von 2-Brombenzoylchlorid (167 mg) in Dichlormethan (1 mL) versetzt und für 6 h bei 0 °C sowie über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 15 mg der Titelverbindung erhalten. HPLC-MS: logP = 2,80; Masse (m/z): 380,0 (M+H)⁺; ¹H-NMR (CD₃CN) 3.45 (t, 2H), 3.79 (t, 2H), 6.93 - 7.01 (m, 2H), 7.10 - 7.18 (m, 1H), 7.36 - 7.40 (m, 1H), 7.43 - 7.47 (m, 1H), 7.50 - 7.53 (m, 1H), 7.66 - 7.68 (m, 1H), 9.04 (br. s, 1H).

### Synthesebeispiel 7

### N-[1-(2,6-Difluorphenyl)-1H-pyrazol-3-yl]-3-(trifluormethyl)pyridin-2-carboxamid (Verbindung I-1-91 in Tabelle 1)

### Stufe 1: N-[1-(2,6-Difluorphenyl)-4,5-dihydro-1H-pyrazol-3-yl]acetamid

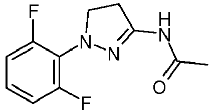

1-(2,6-Difluorphenyl)-4,5-dihydro-1H-pyrazol-3-amin (1,40 g als Rohgemisch aus Synthesebeispiel 6, Stufe 1) wurde unter Eiskühlung in Acetanhydrid (6 mL) gelöst und über Nacht bei Raumtemperatur gerührt. Dann wurde der Ansatz mit Ethylacetat verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 537 mg der Titelverbindung erhalten. HPLC-MS: logP = 1,46; Masse (m/z): 240,0 (M+H)⁺; ¹H-NMR (DMSO-D₆) 1.99 (s, 3H), 3.26 (t, 2H), 3.65 (t, 2H), 7.04 - 7.10 (m, 2H), 7.11 -7.18 (m, 1H), 10.59 (br. s, 1H).

### Stufe 2: N-[1-(2,6-Difluorphenyl)-1H-pyrazol-3-yl]acetamid

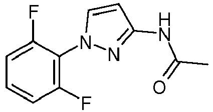

N-[1-(2,6-Difluorphenyl)-4,5-dihydro-1H-pyrazol-3-yl]acetamid (200 mg) wurde in 1,4-Dioxan (1 mL) vorgelegt, mit 2,3-Dichlor-5,6-dicyano-1,4-benzochinon (209 mg) versetzt und für 30 min bei Raumtemperatur gerührt. Das Reaktionsgemisch wurde filtriert und das Filtrat im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 140 mg der Titelverbindung erhalten. HPLC-MS: logP = 1,33; Masse (m/z): 238,0 (M+H)⁺; ¹H-NMR (CD₃CN) 2.07 (s, 3H), 6.84 (d, 1H), 7.14 - 7.20 (m, 2H), 7.47 - 7.53 (m, 1H), 7.65 - 7.66 (m, 1H), 8.76 (br. s, 1H).

### Stufe 3: 1-(2,6-Difluorphenyl)-1H-pyrazol-3-amin (gemäß Route D-1)

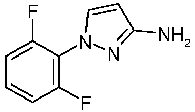

N-[1-(2,6-Difluorphenyl)-1H-pyrazol-3-yl]acetamid (50 mg) wurde in Wasser (1 mL) vorgelegt, mit konzentrierter Salzsäure (0,07 mL) versetzt und 8 h unter Rückfluss erhitzt. Das Reaktionsgemisch wurde dann mit konzentrierter Natronlauge alkalisch gestellt und mit Dichlormethan extrahiert. Die organische Phase wurde im Vakuum zur Trockne eingeengt. Es wurden 25 mg der Titelverbindung erhalten. HPLC-MS: logP = 1,13; Masse (m/z): 196,1 (M+H)⁺; ¹H-NMR (CD₃CN) 4.12 (br. s, 2H), 5.83 (d, 1H), 7.09 - 7.16 (m, 2H), 7.37 - 7.44 (m, 1H), 7.46 - 7.47 (m, 1H).

### Stufe 4: N-[1-(2,6-Difluorphenyl)-1H-pyrazol-3-yl]-3-(trifluormethyl)pyridin-2-carboxamid

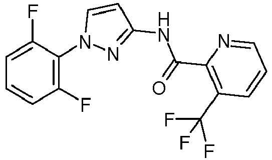

3-(Trifluormethyl)pyridin-2-carbonsäure (76 mg) wurde in Dichlormethan (2 mL) vorgelegt, bei 0 °C mit 1-Ethyl-3-(3-dimethylaminopropyl)carbodiimid (95 mg) und 1-Hydroxy-1H-benzotriazol (67 mg) versetzt und für 1 h gerührt. Dann wurde eine Lösung von 1-(2,6-Difluorphenyl)-1H-pyrazol-3-amin (66 mg) in Dichlormethan (1 mL) zugegeben und über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde zunächst an Kieselgel mit Cyclohexan/Ethylacetat als Laufmittel säulenchromatographisch (Gradient = 2 h von 100 % Cyclohexan auf 100 % Ethylacetat) gereinigt und dann an einer präparativen HPLC mit Wasser/Acetonitril als Laufmittel chromatographiert (Gradient = 40 min von 10 % Acetonitril in Wasser auf 100 % Acetonitril). Es wurden 55 mg der Titelverbindung erhalten. HPLC-MS: logP = 2,22; Masse (m/z): 369,0 (M+H)⁺; ¹H-NMR (CD₃CN) 6.98 - 6.99 (m, 1H), 7.17 - 7.21 (m, 2H), 7.48 - 7.55 (m, 1H), 7.66 - 7.73 (m, 1H), 7.76 - 7.77 (m, 1H), 8.08 - 8.10 (m, 1H), 8.80 - 8.82 (m, 1H), 9.30 (br. s, 1H).

### Synthesebeispiel 8

### N-[1-(2,6-Dichlorphenyl)-1H-pyrazol-3-yl]-2-iodbenzamid (Verbindung I-1-123 in Tabelle 1)

### Stufe 1: 1-(2,6-Dichlorphenyl)-1H-pyrazol-3-amin (gemäß Route B-1)

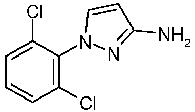

2,6-Dichlorphenylhydrazin (10,0 g) wurde in Ethanol (65 mL) vorgelegt, mit Natriummethylatlösung (3,04 g in 35 mL Ethanol) und mit 3-Ethoxyacrylsäurenitril (6,82 g) versetzt, für 12 h bei 80°C und für 2 d bei Raumtemperatur gerührt. Das Reaktionsgemisch wurde anschließend im Vakuum eingeengt, in Ethylacetat aufgenommen, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 832 mg der Titelverbindung erhalten. HPLC-MS: logP = 1,47; Masse (m/z): 228,1 (M+H)⁺; ¹H-NMR (CD₃CN) 4.09 (br. s, 2H), 5.81 (d, 1H), 7.35 - 7.43 (m, 2H), 7.51 - 7.53 (m, 2H).

### Stufe 2: N-[1-(2,6-Dichlorphenyl)-1H-pyrazol-3-yl]-2-iodbenzamid

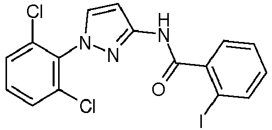

1-(2,6-Dichlorphenyl)-1H-pyrazol-3-amin (150 mg) wurde in Dichlormethan (2 mL) vorgelegt, bei 0 °C mit Triethylamin (0,28 mL) und einer Lösung von 2-Iodbenzoylchlorid (175 mg) in Dichlormethan (1 mL) versetzt und über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 150 mg der Titelverbindung erhalten. HPLC-MS: logP = 3,04; Masse (m/z): 457,9 (M+H)⁺; ¹H-NMR (CD₃CN) 6.99 - 7.00 (m, 1H), 7.18 - 7.23 (m, 1H), 7.46 - 7.52 (m, 3H), 7.56 - 7.58 (m, 2H), 7.66 - 7.67 (m, 1H), 7.94 - 7.96 (m, 1H), 9.16 (br. s, 1H).

### Synthesebeispiel 9

### N-[1-(3,5-Difluorpyridin-2-yl)-1H-pyrazol-3-yl]-2,6-difluorbenzamid (Verbindung I-1-146 in Tabelle 1)

### Stufe 1: 1-(3,5-Difluorpyridin-2-yl)-1H-pyrazol-3-amin (gemäß Route A-1)

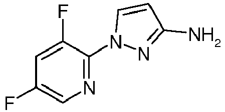

1H-Pyrazol-3-amin (1,80 g) wurde in Acetonitril (50 mL) vorgelegt, mit 2,3,5-Trifluorpyridin (2,88 g) und Kaliumcarbonat (5,99 g) versetzt und über Nacht unter Rückfluss erhitzt. Das Reaktionsgemisch wurde anschließend im Vakuum eingeengt, in Dichlormethan aufgenommen, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 610 mg der Titelverbindung erhalten. HPLC-MS: logP = 0,81; Masse (m/z): 197,1 (M+H)⁺; ¹H-NMR (CD₃CN) 4.27 (br. s, 2H), 5.88 (d, 1H), 7.56 - 7.62 (m, 1H), 7.99 - 8.00 (m, 1H), 8.17 - 8.18 (m, 1H).

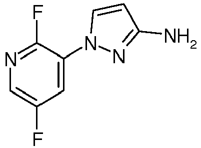

Als weiteres Produkt wurden 410 mg des isomeren 1-(2,5-Difluorpyridin-3-yl)-1H-pyrazol-3-amins isoliert. HPLC-MS: logP = 1,15; Masse (m/z): 197,0 (M+H)⁺; 1H-NMR (CD₃CN) 4.39 (br. s, 2H), 5.92 (d, 1H), 7.80 - 7.82 (m, 1H), 7.94 - 7.95 (m, 1H), 8.02 - 8.07 (m, 1H).

### Stufe 2: N-(1-(3,5-Difluorpyridin-2-yl)-1H-pyrazol-3-yl)-2,6-difluorbenzamid

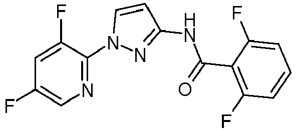

1-(3,5-Difluorpyridin-2-yl)-1H-pyrazol-3-amin (66 mg) wurde in Dichlormethan (2 mL) vorgelegt, bei 0°C mit Triethylamin (0,14 mL) und einer Lösung von 2,6-Difluorbenzoylchlorid (59 mg) in Dichlormethan (1 mL) versetzt und über Nacht bei Raumtemperatur gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 107 mg der Titelverbindung erhalten. HPLC-MS: logP = 2,19; Masse (m/z): 337,1 (M+H)⁺; ¹H-NMR (CD₃CN) 7.02 - 7.03 (m, 1H), 7.06 - 7.12 (m, 2H), 7.48 - 7.56 (m, 1H), 7.65 - 7.70 (m, 1H), 8.21 - 8.22 (m, 1H), 8.26 - 8.27 (m, 1H), 9.51 (br. s, 1H).

### Synthesebeispiel 10

### 2-Brom-N-[1-(3,5,6-trifluorpyridin-2-yl)-1H-pyrazol-3-yl]benzamid (Verbindung I-1-164 in Tabelle 1)

### Stufe 1: 1-(3,5,6-Trifluorpyridin-2-yl)-1H-pyrazol-3-amin (gemäß Route A-1)

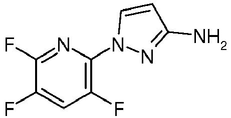

1H-Pyrazol-3-amin (2,00 g) wurde in Acetonitril (75 mL) vorgelegt, mit 2,3,5,6-Tetrafluorpyridin (3,64 g) und Kaliumcarbonat (6,65 g) versetzt und über Nacht unter Rückfluss erhitzt. Das Reaktionsgemisch wurde anschließend mit tert-Butylmethylether verrührt und filtriert. Das Filtrat wurde im Vakuum zur Trockne eingeengt. Es verbleiben 2,51 g der Titelverbindung. HPLC-MS: logP = 1,15; Masse (m/z): 215,1 (M+H)⁺; ¹H-NMR (DMSO-D₆) 5.35 (br. s, 2H), 5.86 (d, 1H), 8.00 (d, 1H), 8.40 - 8.45 (m, 1H).

### Stufe 2: 2-Brom-N-[1-(3,5,6-trifluorpyridin-2-yl)-1H-pyrazol-3-yl]benzamid

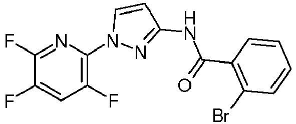

Analog zu Synthesebeispiel 9 Stufe 2 wurde 1-(3,5,6-Trifluorpyridin-2-yl)-1H-pyrazol-3-amin (150 mg) mit 2-Brombenzoylchlorid (169 mg) und Triethylamin (0,29 mL) in Dichlormethan umgesetzt. Nach säulenchromatographischer Reinigung wurden 145 mg der Titelverbindung erhalten. HPLC-MS: logP = 2.68; Masse (m/z): 396,9 (M+H)⁺; 1H-NMR [DMSO-D_{6]} 7.02 (d, 1H), 7.38 - 7.43 (m, 1H), 7.45 - 7.49 (m, 1H), 7.53-7.55 (m, 1H), 7.68 - 7.70 (m, 1H), 8.32 (d, 1H), 8.55 - 8.61 (m, 1H), 11.41 (s, 1H).

### Synthesebeispiel 11

### N-[1-(2-Chlorpyridin-3-yl)-1H-pyrazol-3-yl]-2-iodbenzamid (Verbindung I-1-139 in Tabelle 1)

### Stufe 1: 1-(2-Chlorpyridin-3-yl)-1H-pyrazol-3-amin (gemäß Route A-1)

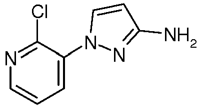

1H-Pyrazol-3-amin (2,00 g) wurde in Dimethylsulfoxid (25 mL) vorgelegt, mit 2-Chlor-3-fluorpyridin (3,17 g) und Kaliumcarbonat (6,65 g) versetzt und über Nacht bei 120 °C gerührt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 2,16 g der Titelverbindung erhalten. HPLC-MS: logP = 0,62; Masse (m/z): 179,1 (M+H)⁺; ¹H-NMR (CD₃CN) 4.23 (br. s, 2H), 5.87 (d, 1H), 7.41 - 7.44 (m, 1H), 7.86 (d, 1H), 7.92 - 7.94 (m, 1H), 8.28 - 8.30 (m, 1H).

### Stufe 2: N-(1-(2-Chlorpyridin-3-yl)-1H-pyrazol-3-yl)-2-iodbenzamid

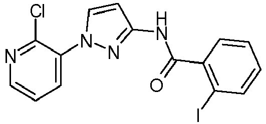

Analog zu Synthesebeispiel 9 Stufe 2 wurde 1-(2-Chlorpyridin-3-yl)-1H-pyrazol-3-amin (100 mg) mit 2-Iodbenzoylchlorid (137 mg) und Triethylamin (0,22 mL) in Dichlormethan umgesetzt. Nach säulenchromatographischer Reinigung wurden 96 mg der Titelverbindung erhalten. HPLC-MS: logP = 2,27; Masse (m/z): 424,9 (M+H)⁺; 1H-NMR [CD₃CN] 7.02 (d, 1H), 7.19 - 7.24 (m, 1H), 7.49 - 7.52 (m, 3H), 7.94 - 7.96 (m, 2H), 8.00 - 8.01 (m, 1H), 8.42 - 8.44 (m, 1H), 9.21 (s, 1H).

### Synthesebeispiel 12

### N-[1-(5,6-Difluorpyrimidin-4-yl)-1H-pyrazol-3-yl]-2-(trifluormethyl)benzamid (Verbindung I-1-169 in Tabelle 1)

### Stufe 1: 1-(5,6-Difluorpyrimidiu-4-yl)-1H-pyrazol-3-amin (gemäß Route A-1)

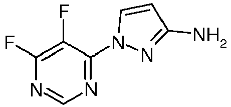

1H-Pyrazol-3-amin (1,25 g) wurde in Acetonitril (75 mL) vorgelegt, mit 4,5,6-Trifluorpyrimidin (2,02 g) und Kaliumcarbonat (4,16 g) versetzt und über Nacht unter Rückfluss erhitzt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 379 mg der Titelverbindung erhalten. HPLC-MS: logP = 0,86; Masse (m/z): 198,1 (M+H)⁺; ¹H-NMR (DMSO-D₆) 5.73 (br. s, 2H), 6.01 (d, 1H), 8.34 (d, 1H), 8.46 (s, 1H).

### Stufe 2: N-[1-(5,6-Difluorpyrimidin-4-yl)-1H-pyrazol-3-yl]-2-(trifluormethyl)benzamid

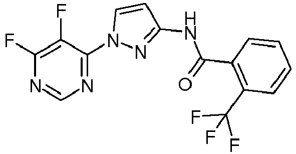

Analog zu Synthesebeispiel 9 Stufe 2 1-(5,6-Difluorpyrimidin-4-yl)-1H-pyrazol-3-amin (62 mg) mit 2-(Trifluormethyl)benzoylchlorid (66 mg) und Triethylamin (0,13 mL) in Dichlormethan umgesetzt. Nach säulenchromatographischer Reinigung wurden 6 mg der Titelverbindung erhalten. HPLC-MS: logP = 2.62; Masse (m/z): 370,1 (M+H)⁺; 1H-NMR [DMSO-D₆] 7.12 (d, 1H), 7.69 - 7.84 (m, 4H), 8.66 - 8.67 (m, 2H), 11.69 (s, 1H).

### Synthesebeispiel 13

### 2-Chlor-N-[1-(3-fluorpyrazin-2-yl)-1H-pyrazol-3-yl]benzamid (Verbindung I-1-174 in Tabelle 1)

### Stufe 1: 1-(3-Fluorpyrazin-2-yl)-1H-pyrazol-3-amin (gemäß Route A-1)

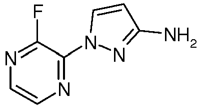

1H-Pyrazol-3-amin (2,00 g) wurde in Acetonitril (75 mL) vorgelegt, mit 2,3-Difluorpyrazin (2,79 g) und Kaliumcarbonat (6,65 g) versetzt und über Nacht unter Rückfluss erhitzt. Das Reaktionsgemisch wurde anschließend mit Dichlormethan verdünnt, mit Wasser gewaschen, über Natriumsulfat getrocknet und im Vakuum zur Trockne eingeengt. Der Rückstand wurde mit tert-Butylmethylether verrührt und der ausgefallene Feststoff abgetrennt, gewaschen und getrocknet. Es wurden 2,24 g der Titelverbindung erhalten. HPLC-MS: logP = 0,49; Masse (m/z): 180,0 (M+H)⁺; ¹H-NMR [DMSO-D_{6]} 5.44 (br. s, 2H), 5.92 (d, 1H), 8.07 - 8.09 (m, 1H), 8.22 (d, 1H), 8.52 - 8.54 (s, 1H).

### Stufe 2: 2-Chlor-N-[1-(3-fluorpyrazin-2-yl)-1H-pyrazol-3-yl]benzamid

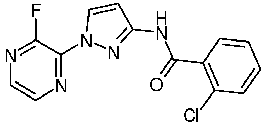

Analog zu Synthesebeispiel 9 Stufe 2 1-(3-Fluorpyrazin-2-yl)-1H-pyrazol-3-amin (150 mg) mit 2-Chlorbenzoylchlorid (162 mg) und Triethylamin (0,35 mL) in Dichlormethan umgesetzt. Nach säulenchromatographischer Reinigung wurden 188 mg der Titelverbindung erhalten. HPLC-MS: logP = 2.03; Masse (m/z): 318,1 (M+H)⁺; 1H-NMR [DMSO-D_{6]} 7.08 (d, 1H), 7.41 - 7.59 (m, 4H), 8.30 - 8.31 (m, 1H), 8.51 (d, 1H), 8.54 - 8.55 (m, 1H), 11.49 (s, 1H).

### Synthesebeispiel 14

### 2-Chlor-N-[1-(2-chlorphenyl)-1H-pyrazol-3-yl]benzamid (Verbindung I-1-20 in Tabelle 1)

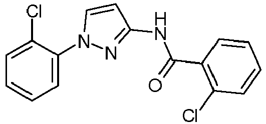

Analog zu Synthesebeipiel 9 Stufe 2 wurde 1-(2-Chlorphenyl)-1H-pyrazol-3-amin (300 mg) mit 2-Chlorbenzoylchlorid (271 mg) und Triethylamin (1,08 mL) in Dichlormethan umgesetzt. Nach säulenchromatographischer Reinigung wurden 386 mg der Titelverbindung erhalten. HPLC-MS: logP = 2,80; Masse (m/z): 332,0 (M+H)⁺; ¹H-NMR (CD₃CN) 6.97 (d, 1H), 7.34 - 7.55 (m, 6H), 7.58 - 7.61 (m, 2H), 7.88 (d, 1H), 9.22 (br. s, 1H).

### Synthesebeispiel 15

### 2-Chlor-N-[1-(2-chlorphenyl)-1H-pyrazol-3-yl]-N-methylbenzamid (Verbindung I-1-21 in Tabelle 1)

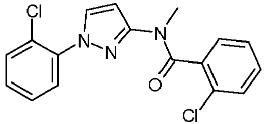

2-Chlor-N-[1-(2-chlorphenyl)-1H-pyrazol-3-yl]benzamid (100 mg) wurde in Tetrahydrofuran (2 mL) vorgelegt, bei 0 °C mit Natriumhydrid (13 mg) versetzt und für 30 min unter Kühlung gerührt. Dann wurde Iodmethan (0,02 mL) zugegeben, für weitere 6 h bei 0 °C und über Nacht bei Raumtemperatur gerührt. Anschließend wurde mit Ethylacetat verdünnt, mit Wasser gewaschen, über Natriumsulfat getrocknet und im Vakuum zur Trockne eingeengt. Der Rückstand wurde an Kieselgel mit Cyclohexan/Ethylacetat als Laufmittel säulenchromatographisch (Gradient = 2 h von 100 % Cyclohexan auf 100 % Ethylacetat) gereinigt. Es wurden 84 mg der Titelverbindung erhalten. HPLC-MS: logP = 3,14; Masse (m/z): 346,0 (M+H)⁺; ¹H-NMR (CD₃CN) (2 Rotamere ca. 2:1) 3.28; 3.46 (2 s, 3H), 6.02; 7.94 (2 m, 1H), 7.14 - 7.63 (m, 9H).

### Synthesebeispiel 16

### 2-Chlor-N-[1-(2-chlorphenyl)-4-fluor-1H-pyrazol-3-yl]benzamid (Verbindung I-1-27 in Tabelle 1)

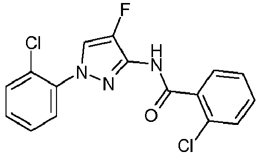

2-Chlor-N-[1-(2-chlorphenyl)-1H-pyrazol-3-yl]benzamid (151 mg) wurde unter Argon in Acetonitril (2 mL) vorgelegt, mit Selectfluor (646 mg) versetzt und für 2 d bei Raumtemperatur nachgerührt. Dann wurde das Reaktionsgemisch in Dichlormethan aufgenommen, filtriert und das Filtrat im Vakuum zur Trockne eingeengt. Der Rückstand wurde zunächst an Kieselgel mit Cyclohexan/Ethylacetat als Laufmittel säulenchromatographisch (Gradient = 2 h von 100 % Cyclohexan auf 100 % Ethylacetat) gereinigt und dann an einer präparativen HPLC mit Wasser/Acetonitril als Laufmittel chromatographiert (Gradient = 40 min von 10 % Acetonitril in Wasser auf 100 % Acetonitril). Es wurden 12 mg der Titelverbindung erhalten. HPLC-MS: logP = 2,70; Masse (m/z): 350,0 (M+H)⁺; ¹H-NMR (DMSO-D₆) 7.44 - 7. 62 (m, 7H), 7.68 - 7.71 (m, 1H), 8.39 (d, 1H), 10.73 (s, 1H).

### Synthesebeispiel 17

### N-[4-Chlor-1-(2-chlorphenyl)-1H-pyrazol-3-yl]-2-(trifluormethyl)benzamid (Verbindung I-1-15 in Tabelle 1)

### Stufe 1: 4-Chlor-1-(2-chlorphenyl)-1H-pyrazol-3-amin (gemäß Route F-1)

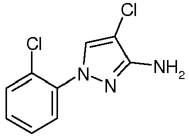

1-(2-Chlorphenyl)-1H-pyrazol-3-amin (266 mg) wurde in Tetrahydrofuran (9 mL) vorgelegt, mit N-Chlorsuccinimid (202 mg) versetzt und für 4 h bei Raumtemperatur gerührt. Danach wurde das Reaktionsgemisch auf Eis gegeben, kräftig gerührt und mit Ethylacetat extrahiert. Die organische Phase wurde über Natriumsulfat getrocknet und im Vakuum zur Trockne eingeengt. Es wurden 413 mg der rohen Titelverbindung erhalten, die ohne weitere Reinigung weiter umgesetzt wurden. HPLC-MS: logP = 2,14; Masse (m/z): 228,1 (M+H)⁺; ¹H-NMR (CD₃CN) 7.34 - 7.43 (m, 2H), 7.50 - 7.56 (m, 2H), 7.83 (s, 1H).

### Stufe 2: N-[4-Chlor-1-(2-chlorphenyl)-1H-pyrazol-3-yl]-2-(trijluormethyl)benzamid

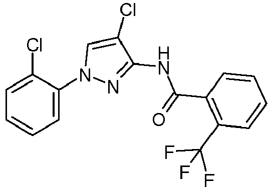

4-Chlor-1-(2-chlorphenyl)-1H-pyrazol-3-amin (66 mg) wurde in Toluol (2 mL) vorgelegt, bei 0 °C mit Triethylamin (0,28 mL) und einer Lösung von 2-(Trifluormethyl)benzoylchlorid (140 mg) in Toluol (1 mL) versetzt und über Nacht bei 80 °C gerührt. Dann wurde Silber(I)cyanid (117 mg) zugegeben und 2 weitere Tage bei Raumtemperatur gerührt. Das Reaktionsgemisch wurde anschließend mit Ethylacetat verdünnt, mit Wasser gewaschen, über Natriumsulfat getrocknet und im Vakuum zur Trockne eingeengt. Der Rückstand wurde zunächst an Kieselgel mit Cyclohexan/Ethylacetat als Laufmittel säulenchromatographisch (Gradient = 2 h von 100 % Cyclohexan auf 100 % Ethylacetat) gereinigt und dann an einer präparativen HPLC mit Wasser/Acetonitril als Laufmittel chromatographiert (Gradient = 40 min von 10 % Acetonitril in Wasser auf 100 % Acetonitril). Es wurden 16 mg der Titelverbindung erhalten. HPLC-MS: logP = 3,06; Masse (m/z): 399,9 (M+H)⁺; ¹H-NMR (CD₃CN) 7.48 - 7.89 (m, 8H), 8.05 (s, 1H), 8.68 (br. s, 1H).

### Synthesebeispiel 18

### N-[4-Brom-1-(2-chlorphenyl)-1H-pyrazol-3-yl]-2-(trifluormethyl)benzamid (Verbindung I-1-16 in Tabelle 1)

### Stufe 1: 4-Brom-1-(2-chlorphenyl)-1H-pyrazol-3-amin (gemäß Route F-1)

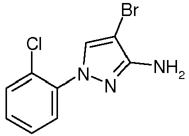

1-(2-Chlorphenyl)-1H-pyrazol-3-amin (266 mg) wurde in Tetrahydrofuran (7 mL) vorgelegt, mit N-Bromsuccinimid (269 mg) versetzt und für 3 h bei Raumtemperatur gerührt. Danach wurde das Reaktionsgemisch auf Eis gegeben, kräftig gerührt und mit Ethylacetat extrahiert. Die organische Phase wurde über Natriumsulfat getrocknet und im Vakuum zur Trockne eingeengt. Es wurden 442 mg der rohen Titelverbindung erhalten, die ohne weitere Reinigung weiter umgesetzt wurden. HPLC-MS: logP = 2,23; Masse (m/z): 272,0 (M+H)⁺; ¹H-NMR (CD₃CN) 4.27 (br. s, 2H), 7.35 - 7.43 (m, 2H), 7.50 - 7.56 (m, 2H), 7.83 (s, 1H).

### Stufe 2: N-[4-Brom-1-(2-chlorphenyl)-1H-pyrazol-3-yl]-2-(trifluormethyl)benzamid

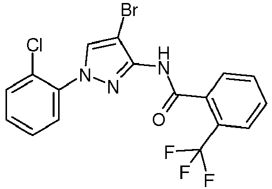

4-Brom-1-(2-chlorphenyl)-1H-pyrazol-3-amin (165 mg) wurde in Toluol (2 mL) vorgelegt, bei 0 °C mit Triethylamin (0,25 mL) und einer Lösung von 2-(Trifluormethyl)benzoylchlorid (126 mg) in Toluol (1 mL) versetzt und über Nacht bei 80 °C gerührt. Dann wurde Silber(I)cyanid (97 mg) zugegeben und 2 weitere Tage bei Raumtemperatur gerührt. Das Reaktionsgemisch wurde anschließend mit Ethylacetat verdünnt, mit Wasser gewaschen, über Natriumsulfat getrocknet und im Vakuum zur Trockne eingeengt. Der Rückstand wurde zunächst an Kieselgel mit Cyclohexan/Ethylacetat als Laufmittel säulenchromatographisch (Gradient = 2 h von 100 % Cyclohexan auf 100 % Ethylacetat) gereinigt und dann an einer präparativen HPLC mit Wasser/Acetonitril als Laufmittel chromatographiert (Gradient = 40 min von 10 % Acetonitril in Wasser auf 100 % Acetonitril). Es wurden 18 mg der Titelverbindung erhalten. HPLC-MS: logP = 3,11; Masse (m/z): 444,0 (M+H)⁺; ¹H-NMR (CD₃CN) 7.48 - 7.49 (m, 2H), 7.57 - 7.58 (m, 1H), 7.62 - 7.63 (m, 1H), 7.69 - 7.70 (m, 1H), 7.75 - 7.76 (m, 2H), 7.83 - 7.84 (m, 1H), 8.06 (s, 1H), 8.59 (br. s, 1H).

### Synthesebeispiel 19

### N-[4-Iod-1-(2-chlorphenyl)-1H-pyrazol-3-yl]-2-(trifluormethyl)benzamid (Verbindung I-1-17 in Tabelle 1)

### Stufe 1: 4-Iod-1-(2-chlorpheuyl)-1H-pyrazol-3-amin (gemäß Route F-1)

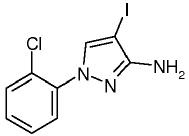

1-(2-Chlorphenyl)-1H-pyrazol-3-amin (266 mg) wurde in Tetrahydrofuran (7 mL) vorgelegt, mit N-Iodsuccinimid (340 mg) versetzt und für 3 h bei Raumtemperatur gerührt. Danach wurde das Reaktionsgemisch auf Eis gegeben, kräftig gerührt und mit Ethylacetat extrahiert. Die organische Phase wurde über Natriumsulfat getrocknet und im Vakuum zur Trockne eingeengt. Es wurden 557 mg der rohen Titelverbindung erhalten, die ohne weitere Reinigung weiter umgesetzt wurden. HPLC-MS: logP = 2,36; Masse (m/z): 320,0 (M+H)⁺; ¹H-NMR (CD₃CN) 7.35 - 7.42 (m, 2H), 7.49 - 7.56 (m, 2H), 7.81 (s, 1H).

### Stufe 2: N-[4-Iod-1-(2-chlorphenyl)-1H-pyrazol-3-yl]-2-(trifluormethyl)benzamid

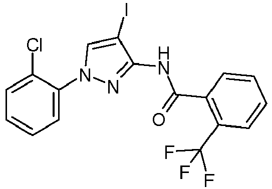

4-Iod-1-(2-chlorphenyl)-1H-pyrazol-3-amin (180 mg) wurde in Toluol (3 mL) vorgelegt, bei 0 °C mit Triethylamin (0,24 mL) und einer Lösung von 2-(Trifluormethyl)benzoylchlorid (117 mg) in Toluol (2 mL) versetzt und über Nacht bei 80 °C gerührt. Dann wurde Silber(I)cyanid (91 mg) zugegeben und 2 weitere Tage bei Raumtemperatur gerührt. Das Reaktionsgemisch wurde anschließend mit Ethylacetat verdünnt, mit Wasser gewaschen, über Natriumsulfat getrocknet und im Vakuum zur Trockne eingeengt. Der Rückstand wurde zunächst an Kieselgel mit Cyclohexan/Ethylacetat als Laufmittel säulenchromatographisch (Gradient = 2 h von 100 % Cyclohexan auf 100 % Ethylacetat) gereinigt und dann an einer präparativen HPLC mit Wasser/Acetonitril als Laufmittel chromatographiert (Gradient = 40 min von 10 % Acetonitril in Wasser auf 100 % Acetonitril). Es wurden 11 mg der Titelverbindung erhalten. HPLC-MS: logP = 3,16; Masse (m/z): 491,9 (M+H)⁺; ¹H-NMR (CD₃CN) 7.48 - 7.49 (m, 2H), 7.56 - 7.57 (m, 1H), 7.61 - 7.64 (m, 1H), 7.68 - 7.71 (m, 1H), 7.75 - 7.78 (m, 2H), 7.83 - 7.85 (m, 1H), 8.05 (s, 1H), 8.60 (br. s, 1H).

Die folgenden neuen 1H-Pyrazol-3-amine der Formel (II-1) wurden analog zu den angegebenen Synthesebeispielen erhalten:1-(2,3-Dichlorphenyl)-1H-pyrazol-3-amin: analog zu Synthesebeispiel 3, Stufe 1 ausgehend von 2,3-Dichlorphenylhydrazinhydrochlorid: HPLC-MS: logP = 1,97; Masse (m/z): 228,0 (M+H)⁺; ¹H-NMR (CD₃CN) 4.16 (br. s, 2H), 5.84 (d, 1H), 7.39 - 7.41 (m, 1H), 7.50 - 7.52 (m, 1H), 7.59 - 7.60 (m, 1H), 7.73 (d, 1H).

1-(3-Chlor-2-fluorphenyl)-1H-pyrazol-3-amin: analog zu Synthesebeispiel 3, Stufe 1 ausgehend von 3-Chlor-2-fluorphenylhydrazinhydrochlorid: HPLC-MS: logP = 1,90; Masse (m/z): 212,0 (M+H)⁺; ¹H-NMR (DMSO-D₆) 5.22 (br. s, 2H), 5.82 (d, 1H), 7.25 - 7.30 (m, 1H), 7.36 - 7.40 (m, 1H), 7.67 - 7.71 (m, 1H), 7.90 - 7.10 (m, 1H). Als Nebenprodukt wurde 1-(3-Chlor-2-ethoxyphenyl)-1H-pyrazol-3-amin erhalten: HPLC-MS: logP = 2,13; Masse (m/z): 238,0 (M+H)⁺; ¹H-NMR (DMSO-D₆) 1.24 (t, 3H), 3.78 (q, 2H), 5.08 (br. s, 2H), 5.78 (d, 1H), 7.17 - 7.21 (m, 1H), 7.30 - 7.33 (m, 1H), 7.56 - 7.59 (m, 1H), 7.97 (d, 1H).

1-(2-Chlor-6-ethoxyphenyl)-1H-pyrazol-3-amin: analog zu Synthesebeispiel 5, Stufe 1 ausgehend von 2-Chlor-6-fluorphenylhydrazinhydrochlorid: HPLC-MS: logP = 2,13; Masse (m/z): 238,0 (M+H)⁺; ¹H-NMR (DMSO-D₆) 1.34 (t, 3H), 4.11 (q, 2H), 4.99 (br. s, 2H), 7.09 - 7.12 (m, 2H), 7.24 - 7.33 (m, 3H).

1-(2-Chlor-4-methylphenyl)-1H-pyrazol-3-amin: analog zu Synthesebeispiel 3, Stufe 1 ausgehend von 2-Chlor-4-methylphenylhydrazinhydrochlorid: HPLC-MS: logP = 1,85; Masse (m/z): 208,1 (M+H)⁺; ¹H-NMR (CD₃CN) 2.35 (s, 3H), 4.08 (br. s, 2H), 5.79 (d, 1H), 7.19 - 7.20 (m, 1H), 7.36 - 7.38 (m, 2H), 7.64 (d, 1H).

1-(3,5-Difluor-6-methylpyridin-2-yl)-1H-pyrazol-3-amin: analog zu Synthesebeispiel 9, Stufe 1 ausgehend von 2,3,5-Trifluor-6-methylpyridin: HPLC-MS: logP = 1,25; Masse (m/z): 211,1 (M+H)⁺; ¹H-NMR (DMSO-D₆) 2.40 - 2.41 (m, 3H), 5.18 (br. s, 2H), 5.80 (d, 1H), 7.97 - 8.02 (m, 2H). Als Nebenprodukt wurde 1-(2,5-Difluor-6-methylpyridin-3-yl)-1H-pyrazol-3-amin erhalten: HPLC-MS: logP = 1,50; Masse (m/z): 211,1 (M+H)⁺; ¹H-NMR (DMSO-D₆) 2.36 - 2.37 (m, 3H), 5.34 (br. s, 2H), 5.84 (d, 1H), 7.92 - 7.98 (m, 2H).

1-(5-Chlorpyrazin-2-yl)-1H-pyrazol-3-amin: analog zu Synthesebeispiel 13, Stufe 1 ausgehend von 2,5-Dichlorpyrazin: HPLC-MS: (m/z): 196,1 (M+H)⁺.

1-(6-Fluorpyrazin-2-yl)-1H-pyrazol-3-amin: analog zu Synthesebeispiel 13, Stufe 1 ausgehend von 2,6-Difluorpyrazin: HPLC-MS: logP = 0,90; Masse (m/z): 180,1 (M+H)⁺; ¹H-NMR (DMSO-D₆) 5.61 (br. s, 2H), 5.94 (d, 1H), 8.18 (d, 1H), 8.36 (d, 1H), 8.78 (d, 1H).

1-(3-Methoxypyrazin-2-yl)-1H-pyrazol-3-amin: analog zu Synthesebeispiel 13, Stufe 1 ausgehend von 2-Chlor-3-methoxypyrazin.

4-(3-Amino-1H-pyrazol-1-yl)pyrimidin-5-carbonitril: analog zu Synthesebeispiel 12, Stufe 1 ausgehend von 4-Chlorpyrimidin-5-carbonitril: HPLC-MS: logP = 0,59; Masse (m/z): 187,1.

1-(5-Chlor-3-fluorpyridin-2-yl)-1H-pyrazol-3-amin: analog zu Synthesebeispiel 9, Stufe 1 ausgehend von 5-Chlor-2,3-difluorpyridin: HPLC-MS: logP = 1,27; Masse (m/z): 213,0 (M+H)⁺; ¹H-NMR (DMSO-D₆) 5.30 (s, 2H), 5.86 (d, 1H), 8.12 (d, 1H), 8.17 - 8.20 (m, 1H), 8.31 (d, 1H).

1-(3-Chlor-5-fluorpyridin-2-yl)-1H-pyrazol-3-amin: analog zu Synthesebeispiel 9, Stufe 1 ausgehend von 3-Chlor-2,5-difluorpyridin: HPLC-MS: logP = 0,98; Masse (m/z): 213,0 (M+H)⁺; ¹H-NMR (DMSO-D₆) 5.11 (s, 2H), 5.76 - 5.78 (m, 1H), 7.91- 7.92 (m, 1H), 8.23 - 8.26 (m, 1H), 8.47 - 8.48 (m, 1H).

### Synthesebeispiel 20

### N-[2-(2-Chlorphenyl)-2H-1,2,3-triazol-4-yl]-2-(trifluormethyl)benzamid (Verbindung I-2-1 in Tabelle 3)

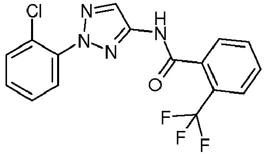

Analog zu Synthesebeispiel 9 Stufe 2 wurde 2-(2-Chlorphenyl)-2H-1,2,3-triazol-4-amin (150 mg) mit 2-(Trifluormethyl)benzoylchlorid (160 mg) und Triethylamin (0,32 mL) in Dichlormethan umgesetzt. Nach säulenchromatographischer Reinigung und präparativer HPLC wurden 171 mg der Titelverbindung erhalten. HPLC-MS: logP = 3,02; Masse (m/z): 367,0 (M+H)⁺; ¹H-NMR (CD₃CN) 7.48 - 7.55 (m, 2H), 7.60 - 7.66 (m, 2H), 7.68 - 7.77 (m, 3H), 7.83 - 7.85 (m, 1H), 8.36 (s, 1H), 9.54 (br. s, 1H).

### Synthesebeispiel 21

### N-[2-(2,6-Difluorphenyl)-2H-1,2,3-triazol-4-yl]-2-(trifluormethyl)benzamid (Verbindung I-2-35 in Tabelle 5)

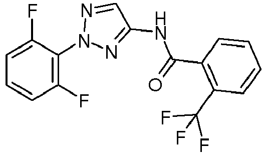

### Syntheseschema des Zwischeuprodukts 2-(2,6-Difluorphenyl)-2H-1,2,3-triazol-4-amin (IX) (gemäβ Route A-3)

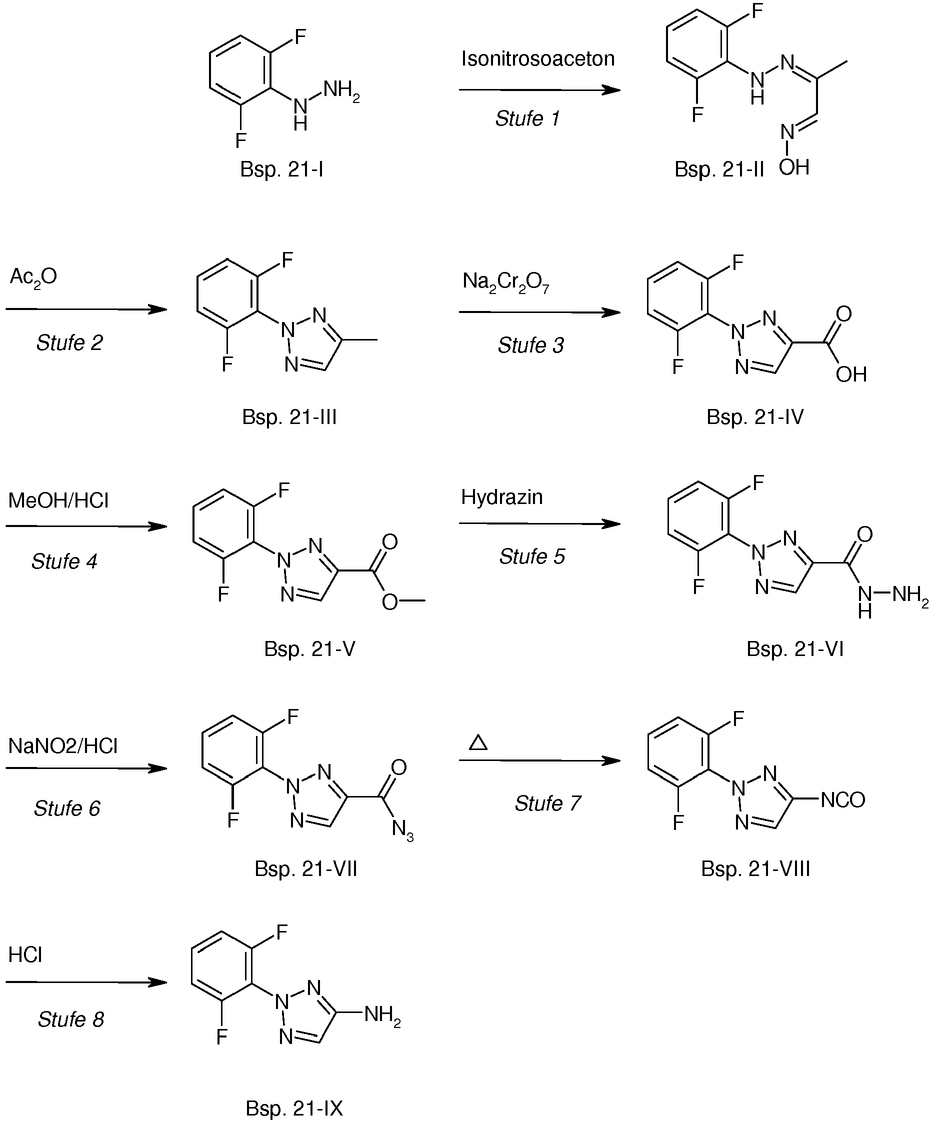

### Stufe 1: 2-[2-(2,6-Difluorphenyl)hydrazinyliden]propanaloxim (Bsp. 21-II)

0,1 Mol 2,6-Difluorphenylhydrazin (Bsp. 21-1) und 0,12 Mol Isonitrosoaceton wurden in Ethanol für 3 h unter Rückfluss erhitzt. Nach Abkühlung auf Raumtemperatur wurde der ausgefallene Feststoff abfiltriert, mit Ethanol nachgewaschen und getrocknet. Man erhielt 75 % der Theorie des Hydrazonoximes (Bsp. 21-II).

### Stufe 2: 2-(2,6-Difluorphenyl)-4-methyl-2H-1,2,3-triazol (Bsp. 21-III)

Eine Lösung von 0,1 Mol des Hydrazonoximes (Bsp. 21-II) in Essigsäureanhydrid wurde langsam auf 120°C erhitzt und für 2 h bei dieser Temperatur gerührt. Überschüssiges Essigsäureanhydrid wurde am Rotationsverdampfer entfernt. Das entstandene Methyltriazol (Bsp. 21-III) (65 % der Theorie) wurde ohne weitere Aufarbeitung weiterverwendet.

### Stufe 3: 2-(2,6-Difluorphenyl)-2H-1,2,3-triazol-4-carbonsäure (Bsp. 21-IV)

0,2 Mols Natriumdichromat wurden in kleinen Portionen zu einer gut gerührten Lösung von 0,1 Mol Methyltriazol (Bsp. 21-III) in 66%iger Schwefelsäure gegeben. Jede einzelne Portion Dichromat wurde erst zugegeben, nachdem die gelb-orange Farbe des Cr⁶⁺ im Kolben verschwand. Die Portionierung wurde außerdem so dosiert, dass die Temperatur im Kolben bei ca. 80-90°C blieb. Anschließend wurde auf dem Dampfbad für 1 h erhitzt. Nach dem Abkühlen wurde die Mischung in etwa dieselbe Menge Eis gegossen und über Nacht stehengelassen. Die ausgefallene Säure (Bsp. 21-IV) wurde abfiltriert, mit Wasser gewaschen und getrocknet. Man erhielt 50% der Theorie der Säure (Bsp. 21-IV).

### Stufe 4: Methyl-2-(2,6-difluorphenyl)-2H-1,2,3-triazol-4-carboxylat (Bsp. 21-V)

Chlorwasserstoff wurde für 2 h durch eine kochende Lösung der Säure (Bsp. 21-IV) in Methanol geleitet. Nach dem Abkühlen wurden weiße Kristalle des Esters (Bsp. 21-V) abfiltriert (85% der Theorie).

### Stufe 5: 2-(2,6-Difluorphenyl)-2H-1,2,3-triazol-4-carbohydrazid (Bsp. 21-VI)

Der Ester (Bsp. 21-V) wurde mit einem Überschuss von 1.5 Äq. Hydrazinhydrat für 4 h in Ethanol gekocht. Nach dem Abkühlen wurden die Kristalle des Hydrazides (Bsp. 21-VI) mit Wasser gewaschen und getrocknet. Man erhielt 90% der Theorie.

### Stufe 6: 2-(2,6-Difluorphenyl)-2H-1,2,3-triazol-4-carbonylazid (Bsp. 21-VII)

Zu einer Suspension des Hydrazids (Bsp. 21-VI) in 20%iger wässriger Salzsäure wurde eine wässrige Lösung von Natriumnitrit gegeben. Nach weiterem Rühren bei 10°C konnten Kristalle des Acylazids (Bsp. 21-VII) abfiltriert, mit Wasser gewaschen und im Vakuum bei Raumtemperatur getrocknet werden. Man erhielt 75% der Theorie.

### Stufe 7: 2-(2,6-Difluorphenyl)-2H-1,2,3-triazol-4-carbonylisocyanat (Bsp. 21-VIII)

Das getrocknete Acylazid (Bsp. 21-VII) wurde in Toluol gekocht bis die Gasentwicklung aufhörte (ca. 2 h). Anschließend wurde das Toluol am Rotationsverdampfer entfernt und der zähe Rückstand des Isocyanats (Bsp. 21-VIII) ohne weitere Reinigung direkt weiter umgesetzt. Man erhielt ca.90% der Theorie.

### Stufe 8: 2-(2,6-Difluorphenyl)-2H-1,2,3-triazol-4-amin (Bsp. 21-IX)

Das Isocyanat (Bsp. 21-VIII) wurde durch 30 min Kochen in Salzsäure hydrolysiert. Restliche flüchtige Substanzen wurden am Rotationsverdampfer entfernt und der Rückstand wurde mit Natriumcarbonatlösung behandelt. Die ausgefallenen Ksristalle wurden abfiltriert, mit Wasser gewaschen und aus Hexan umkristallisiert. Man erhielt 70 % der Theorie des Amins (Bsp. 21-IX). HPLC-MS: logP = 1,16; Masse (m/z): 197,0 (M+H)⁺; ¹H-NMR (CD₃CN) 5,46 (b, 2H), 7.33 - 7.38 (m, 3H), 7.56 - 7.64 (m, 1H).

### N-(2-(2,6-Difluorphenyl)-2H-1,2,3-triazol-4-yl)-2-(trifluormethyl)benzamid (Verbindung I-2-35 in Tabelle 5)

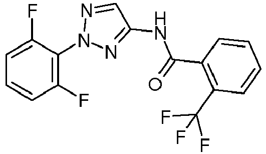

Analog zu Synthesebeispiel 9 Stufe 2 wurde 2-(2,6-Difluorphenyl)-2H-1,2,3-triazol-4-amin (150 mg) mit 2-(Trifluormethyl)benzoylchlorid (160 mg) und Triethylamin (0,21 mL) in 3,9 ml Dichlormethan umgesetzt. Nach säulenchromatographischer Reinigung und präparativer HPLC wurden 143 mg der Titelverbindung erhalten. HPLC-MS: logP = 2,80; Masse (m/z): 369,1 (M+H)⁺; ¹H-NMR (CD₃CN) 7.45 - 7.49 (m, 2H), 7.70 - 7.77 (m, 4H), 7.78 - 7.88 (m, 1H), 8.45 (s, 1H), 11.83 (s, 1H).

### Synthesebeispiel 22

### N-[1-(3,5-Difluorpyridin-2-yl)-5-fluor-1H-indazol-3-yl]-2-(trifluormethyl)benzamid (Verbindung I-5-2 in Tabelle 4)

### Stufe 1: 1-(3,5-Difluorpyridin-2-yl)-5-fluor-1H-indazol-3-amin (gemäß Route A-2)

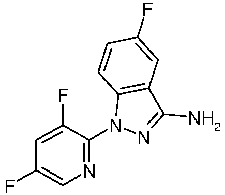

5-Fluor-1H-indazol-3-amin (100 mg) wurde unter Argon in N,N-Dimethylformamid (1 mL) vorgelegt, mit Natriumhydrid (16 mg) versetzt und für 30 min bei Raumtemperatur gerührt. Dann wurde eine Lösung von 2,3,5-Trifluorpyridin (88 mg in 0,5 mL N,N-Dimethylformamid) zugetropft und für 2 Tage bei Raumtemperatur gerührt Das Reaktionsgemisch wurde anschließend in Ethylacetat aufgenommen, mit Wasser gewaschen, über Natriumsulfat getrocknet und im Vakuum zur Trockne eingeengt. Es wurden 121 mg der rohen Titelverbindung erhalten, die ohne weitere Reinigung weiter umgesetzt wurden. HPLC-MS: logP = 2,09; Masse (m/z): 265,1 (M+H)⁺; ¹H-NMR (DMSO-D₆) 6.14 (br. s, 2H), 7.31 - 7.39 (m, 1H), 7.41 - 7.47 (m, 1H), 7.63 - 7.71 (m, 1H), 8.07 - 8.16 (m, 1H), 8.20 - 8.22 (m, 1H).

### Stufe 2: N-(1-(3,5-Difluorpyridin-2-yl)-5-fluor-1H-indazol-3-yl)-2-(trifluormethyl)benzamid

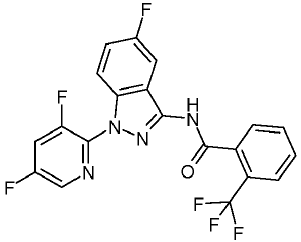

1-(3,5-Difluorpyridin-2-yl)-1H-pyrazol-3-amin (121 mg) wurde in Acetonitril (3 mL) vorgelegt und bei Raumtemperatur mit Kaliumcarbonat (284 mg) und 2-(Trifluormethyl)benzoylchlorid (287 mg) versetzt und über Nacht gerührt. Das Reaktionsgemisch wurde anschließend mit Ethylacetat verdünnt, mit Wasser gewaschen, über Natriumsulfat getrocknet und im Vakuum zur Trockne eingeengt. Der Rückstand wurde an einer präparativen HPLC mit Wasser/Acetonitril als Laufmittel chromatographiert (Gradient = 40 min von 10 % Acetonitril in Wasser auf 100 % Acetonitril). Es wurden 13 mg der Titelverbindung erhalten. HPLC-MS: logP = 3,37; Masse (m/z): 437,1 (M+H)⁺; ¹H-NMR (DMSO-D₆) 7.47 - 7.52 (m, 1H), 7.64 - 7.67 (m, 1H), 7.74 - 7.77 (m, 1H), 7.81 - 7.89 (m, 3H), 7.98 - 8.05 (m, 1H), 8.32-8.45 (m, 1H), 8.57 - 8.58 (m, 1H), 11.47 (s, 1H).

### Synthesebeispiel 23

### N-{1-[3-Fluor-4-(4-fluorphenyl)pyridin-2-yl]-1H-pyrazol-3-yl}-2-(trifluormethyl)benzamid (Verbindung I-1-640 in Tabelle 1)

### Stufe 1: 1-(3-Fluor-4-iodpyridin-2-yl)-1H-pyrazol-3-amin(gemäß A-1)

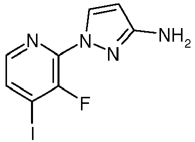

Eine Mischung aus 3-Aminopyrazol (0,50 g), 2,3-Difluor-4-iodpyridin (1,45 g), Kaliumcarbonat (3,3 g) und 10 ml wasserfreiem Dimethylsulfoxid wurde bei 85°C für 48 Stunden gerührt. Zur Aufarbeitung fügte man bei Raumtemperatur 100 ml Wasser hinzu und extrahierte mit Essigsäureethylester. Nach Trocknen der organischen Phase mit Magnesiumsulfat wurde vollständig eingedampft und an Kieselgel mit einem Essigsäureethylester/Methanol Gradienten chromatographiert. Es wurden 705 mg der Titelverbindung erhalten. HPLC-MS: logP = 1,38; Masse (m/z): 304,9 (M+H)⁺; ¹H-NMR (DMSO-D₆) 5.91 (m, 1H), 7.62 (m, 1H), 7.85 (m, 1H), 8.08 (m, 1H).

### Stufe 2: N-(1-(3-Fluor-4-iodpyridin-2-yl)-1H-pyrazol-3-yl)-2-(trifluormethyl)benzamid (Verbindung I-1-576 in Tabelle 1)

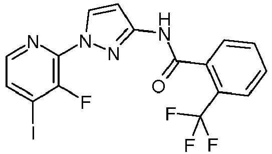

Zu einer Lösung von 1-(3-Fluor-4-iodpyridin-2-yl)-1H-pyrazol-3-amin (600 mg) und Triethylamin (0,83 ml) in Dichlormethan (12 ml) gab man unter Eiskühlung 2-(Trifluormethyl)benzoylchlorid (412 mg), rührte noch eine Stunde bei 0°C und ließ dann über Nacht auf Raumtemperatur erwärmen. Der Ansatz wurde vollständig eingedampft und der so erhaltene Rückstand zwischen Essigsäureethylester und halbgesättigter Natriumhydrogencarbonatlösung verteilt. Die organische Phase wurde eingedampft und an Kieselgel mit einem Methylenchlorid/Methanol Gradienten chromatographiert. Es wurden 650 mg der Titelverbindung erhalten. HPLC-MS: logP = 2,96; Masse (m/z): 476,9 (M+H)⁺; ¹H-NMR (CD₃CN) 7.05 (m, 1H), 7.6 (br m, 3H), 7.76 (br m, 2H), 7.92 (m, 1H), 8.29 (m, 1H), 9.54 (br s, 1H).

### Stufe 3: N-(1-(3-Fluor-4-(4-fluorphenyl)pyridin-2-yl)-1H-pyrazol-3-yl}-2-(trifluormethyl)benzamid

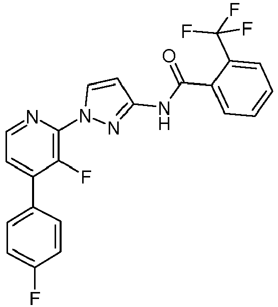

Eine Mischung aus N-[1-(3-Fluor-4-iodpyridin-2-yl)-1H-pyrazol-3-yl]-2-(trifluormethyl)benzamid (200 mg), 4-Fluorboronsäure (117 mg), Natriumcarbonat (170 mg), 1,1'-Bis-(diphenylphosphino)-ferrocen-palladium(II)chlorid (15 mg), 1,2-Dimethoxyethan (3 ml) und Wasser (1 ml) wurde für 6 Stunden auf 85°C erhitzt. Das Reaktionsgemisch wurde eingedampft, der Rückstand mit Essigester verrührt, abfiltriert und das Filtrat mit Wasser gewaschen, getrocknet und erneut eingedampft. Der so erhaltene Rückstand wurde an Kieselgel mit einem Cyclohexan/Essigester Gradienten chromatographiert. Es wurden 81 mg der Titelverbindung erhalten. HPLC-MS: logP = 3,40; Masse (m/z): 445,1 (M+H)⁺; ¹H-NMR (DMSO-D₆) 6.99 (m, 1H), 7.42 (m, 2H), 7.61 - 7.85 (m, 7H), 8.42 (m, 2H), 11.46 (s, 1 H).

### Synthesebeispiel 24

### 2-{[1-(4-Amino-2,6-difluorphenyl)-1H-pyrazol-3-yl]carbamoyl}benzoesäure (Verbindung I-1-643 in Tabelle 1)

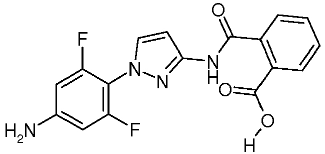

### Stufe 1: 2-{[1-(2,6-Difluor-4-uitropheuyl)-1H-pyrazol-3-yl]carbamoyl}benzoesäure

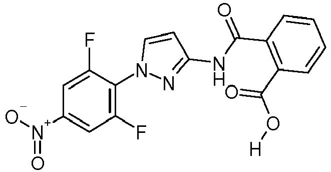

Zu einer Lösung von 2-(1H-Pyrazol-3-yl)-1H-isoindol-1,3(2H)-dion (5.90 g) und 3,4,5-Trifluornitrobenzol (5.05 g) in Tetrahydrofuran (120 ml) gab man unter Argon 60 proz. Natriumhydrid (2.27 g) bei - 20°C portionsweise hinzu. Die Temperatur stieg dabei nicht über -5°C. Nach 6 Stunden ließ man auf Raumtemperatur erwärmen, rührte das Reaktionsgemisch in 300ml Wasser ein und extrahierte mit Essigsäureethylester. Die vereinigten organischen Phasen wurden nach Trocknen über Magnesiumsulfat filtriert und das Filtrat vollständig eingeengt. Es wurden 8.5 g der Titelverbindung erhalten. HPLC-MS: logP = 1,85, Masse (m/z): 389,0 (M+H)⁺. Der so erhaltene Rückstand wurde ohne weitere Reinigung in der nächsten Stufe eingesetzt.

### Stufe 2: 2-{[1-(4-Amino-2,6-difluorphenyl)-1H-pyrazol-3-yl)carbamoyl}benzoesäure

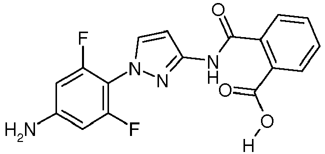

Eine Mischung aus obiger 2-1[1-(2,6-Difluor-4-nitrophenyl)-1H-pyrazol-3-yl]carbamoyllbenzoesäure (6.56 g), Methanol (50 ml) und 250 mg Pd/C (10 proz.) wurde in einem Autoklaven unter 4 bar Wasserstoff bei Raumtemperatur für 4 Tage hydriert. Nach Abfiltrieren vom Katalysator und Eindampfen der organischen Phase erhielt man 6.2 g der Titelverbindung mit einem Gehalt von 70 Proz. d. Theorie. HPLC-MS: logP = 1,38, Masse (m/z): 359,1 (M+H)⁺ ; ¹H-NMR (DMSO-D₆) 4.10 (m, 2H), 6.07 (s, 2H), 6.33 (m, 2H), 6.85 (m, 1H), 7.41 (m, 2H), 7.67 (m, 1H), 7.74 (m, 1H), 7.77 (br s, 1H)

### Synthesebeispiel 25

### N-[1-(3-Cyanpyridin-2-yl)-1H-pyrazol-3-yl]-2-(trifluormethyl)benzamid (Verbindung (I-1-575) in Tabelle 1)

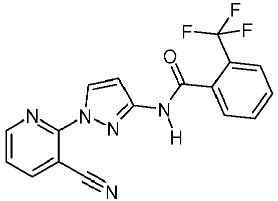

### Stufe 1: N-(1H-Pyrazol-3-yl)-2-(trifluormethyl)benzamid

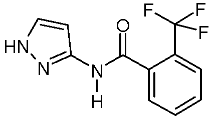

Eine Lösung von 3-Aminopyrazol (43.9 g) und Triethylamin (52.4 g) in Acetonitril (400 ml) wurde unter Feuchtigkeitsausschluß bei 0°C für eine Stunde gerührt. Nachfolgend tropfte man ein Lösung von 2-(Trifluormethyl)-benzoesäurechlorid (109.1 g) in Acetonitril (50 ml) so zu, dass die Innentemperatur nicht über 7°C stieg. Über Nacht ließ man die Reaktion auf Raumtemperatur erwärmen, verdünnte mit 600 ml Wasser und extrahierte mit Essigsäureethylester. Die vereinigten organischen Phasen wurden über Magnesiumsulfat getrocknet, filtriert und eingedampft. Der so erhaltene Rückstand wurde in Isopropanol (500 ml) unter Rückfluss gelöst. Nach Abkühlen auf Raumtemperatur isolierte man die abgeschiedenen Kristalle (48.6 g). Es handelte sich dabei um die Titelverbindung mit einem Molequivalent Kristallisopropanol. HPLC-MS: logP = 1,33, Masse (m/z): 255,9 (M+H)⁺ ; ¹H-NMR (DMSO-D₆) 1.03 (m, 6H), 3.77 (m, 1H), 4.33 (m, 1H), 6.60 (m, 1H), 7.60-7.81 (m, 5H), 10.97 (s, 1H), 12.41 (m, 1H),

### Stufe 2: N-[1-(3-Cyanpyridin-2-yl)-1H-pyrazol-3-yl]-2-(trifluormethyl)benzamid

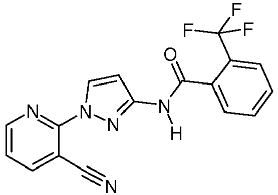

Eine Lösung von N-(1H-Pyrazol-3-yl)-2-(trifluormethyl)benzamid x iso-propanol (222 mg) in 50 ml Dimethylformamid wurde im Vakuum zur Trockene eingedampft. Der Rückstand wurde in wasserfreiem Dimethylformamid (3 ml) aufgenommen, mit Kaliumcarbonat versetzt und das so entstandene Gemisch für 5 Minuten intensiv gerührt. Anschliessend gab man 2-Chlornicotinsaeurenitril (88 mg) hinzu und ließ für 6 Stunden bei 50°C rühren, fügte dann bei Raumtemperatur Wasser (50 ml) hinzu, extrahierte mit Essigsäureethylester, trocknete die vereinigten organischen Phasen über Magnesiumsulfat und dampfte zur Trockene ein. Durch Chromatographie an Kieselgel mit einem Cyclohexan/Essigsäureethylester Gradienten erhielt man die Titelverbindung (149 mg). HPLC-MS: logP = 2,55, Masse (m/z): 358,0 (M+H)' ; ¹H-NMR (DMSO-D₆) 7.06 (m, 1H), 7.53 (m, 1H), 7.69 - 7.84 (m, 4H), 8.46 (m, 1H), 7.60-7.81 (m, 4H), 8.46 (m, 1H), 8.60 (m, 1H), 8.74 (m, 1H), 11.61 (s, 1H).

Die folgenden neuen 2-(Trifluormethyl)benzamide wurden analog zu den angegebenen Synthesebeispielen erhalten:

### Synthesebeispiel 26:

### N-[1-(2,6-Difluor-4-nitrophenyl)-1H-pyrazol-3-yl]-2-(trifluormethyl)benzamid (Verbindung (I-1-229) in Tabelle 1)

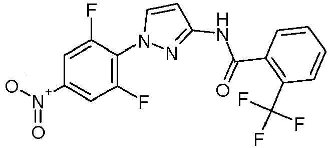

Analog zu Synthesebeispiel 25, Stufe 2 ausgehend von 3,4,5-Trifluornitrobenzol: HPLC-MS: logP = 2,86; Masse (m/z): 413,0 (M+H)+; 1H-NMR (CD₃CN) 7.02 (m, 1H), 7.60 - 7.95 (m, 4H), 8.13 (m, 1H), 8.36 (m, 1H), 11.45 (s, 1H).

### Synthesebeispiel 27:

### N-{1-[2,6-Difluor-4-(trifluormethyl)phenyl]-1H-pyrazol-3-yl}-2-(trifluormethyl)benzamid (Verbindung (I-1-230) in Tabelle 1)

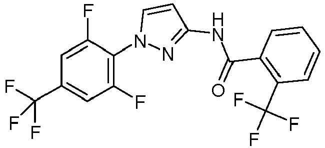

Analog zu Synthesebeispiel 25, Stufe 2 ausgehend von 1,2,3-Trifluor-5-(trifluormethyl)benzol : HPLC-MS: logP = 3,39; Masse (m/z): 436,0 (M+H)⁺; 1H-NMR (DMSO-D₆) 6.98 (m, 1H), 7.68 - 7.14 (m, 2H), 7.77 (m, 1H), 7.81 (m, 1H), 7.97 (m, 2H), 8.14 (m, 1 H), 11.40 (s, 1H).

### Synthesebeispiel 28:

### :N-[1-(4-Cyan-2,6-difluorphenyl)-1H-pyrazol-3-yl]-2-(trifluormethyl)benzamid (Verbindung (I-1-228) in Tabelle 1)

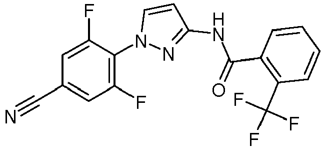

Analog zu Synthesebeispiel 25, Stufe 2 ausgehend von 3,4,5-Trifluorobenzonitril: HPLC-MS: logP = 2,66; Masse (m/z): 393,0 (M+H)+; 1H-NMR (DMSO-D₆) 6.99 (m, 1H), 7.68 - 7.71 (m, 2H), 7.75 (m, 1H), 7.82 (m, 1H), 8.09 - 8.14 (m, 3H), 11.41 (s, 1H).

### Synthesebeispiel 29:

### N-[1-(2-Cyan-4,6-difluorphenyl)-1H-pyrazol-3-yl]-2-(trifluormethyl)benzamid (Verbindung (I-1-393) in Tabelle 1)

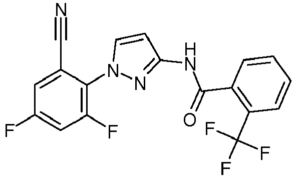

Analog zu Synthesebeispiel 25, Stufe 2 ausgehend von 2,3,5-Trifluorobenzonitril: HPLC-MS: logP = 2,65; Masse (m/z): 393,0 (M+H)+; 1H-NMR (DMSO-D₆) 7.00 (m, 1H), 7.68 - 7.71 (m, 2H), 7.75 (m, 1H), 7.83 (m, 1H), 8.04 - 8.11 (m, 2H), 8.17 (m, 1H), 11.44 (s, 1H).

### Synthesebeispiel 30:

### N-[1-(4-Acetyl-2,6-difluorphenyl)-1H-pyrazol-3-yl]-2-(trifluormethyl)benzamid (Verbindung (I-1-584) in Tabelle 1)

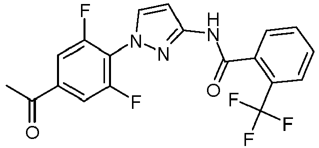

Analog zu Synthesebeispiel 25, Stufe 2 ausgehend von 3,4,5-Trifluoroacetophenon: HPLC-MS: logP = 2,70; Masse (m/z): 410,0 (M+H)+; 1H-NMR (DMSO-D₆) 2.65 (s, 3H), 6.99 (m, 1H), 7.68 - 7.71 (m, 2H), 7.75 (m, 1H), 7.83 (m, 1H), 7.90-7.93 (m, 2H), 8.11 (m, 1H), 11.44 (s, 1H).

### Synthesebeispiel 31:

### N-{1-[3-Cyan-6-(trifluormethyl)pyridin-2-yl]-1H-pyrazol-3-yl}-2-(trifluormethyl)benzamid (Verbindung (I-1-569) in Tabelle 1)

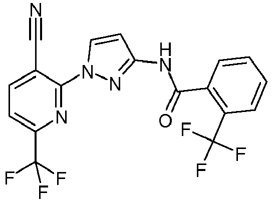

Analog zu Synthesebeispiel 25, Stufe 2 ausgehend von 2-Chlor-6-(trifluormethyl)nicotinonitril: HPLC-MS: logP = 3,41; Masse (m/z): 426,1 (M+H)+; 1H-NMR (DMSO-D₆) 7.11 (m, 1H), 7.67 - 7.73 (m, 2H), 7.77 (m, 1H), 7.84 (m, 1H), 7.99 (m, 1H), 8.60 (m, 1H), 8.77 (m, 1H), 11.71 (s, 1H).

### Synthesebeispiel 32:

### N-{1-[3-Cyan-4-(4-fluorphenyl)pyridin-2-yl]-1H-pyrazol-3-yl}-2-(trifluormethyl)benzamid (Verbindung (I-1-641) in Tabelle 1)

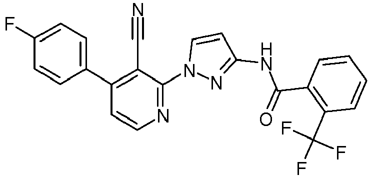

Analog zu Synthesebeispiel 25, Stufe 2 ausgehend von 2-Chlor-4-(4-fluorphenyl)nicotinonitril: HPLC-MS: logP = 3,41; Masse (m/z): 426,1 (M+H)+; 1H-NMR (DMSO-D₆) 7.11 (m, 1H), 7.67 - 7.73 (m, 2H), 7.77 (m, 1H), 7.84 (m, 1H), 7.99 (m, 1H), 8.60 (m, 1H), 8.77 (m, 1H), 11.71 (s, 1H).

### Synthesebeispiel 33:

### N-{1-[3-Cyan-6-methyl-4-(trifluormethyl)pyridin-2-yl]-1H-pyrazol-3-yl}-2-(trifluormethyl)-benzamid (Verbindung (I-1-571) in Tabelle 1)

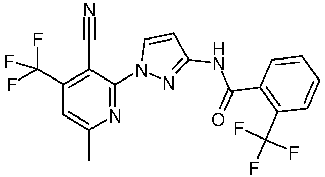

Analog zu Synthesebeispiel 25, Stufe 2 ausgehend von 2-Chlor-6-methyl-4-(trifluormethyl)nicotino-nitril: HPLC-MS: logP = 3,49; Masse (m/z): 440,0 (M+H)+; 1H-NMR (DMSO-D₆) 2.70 (s, 3H), 7.09 (m, 1H), 7.69-7.73 (m, 2H), 7.76 (m, 1H), 7.83 (m, 1H), 7.89 (m, 1H), 8.62 (m, 1H), 11.66 (s, 1H).

### Synthesebeispiel 34:

### N-[1-(6-Chlor-3-cyanpyridin-2-yl)-1H-pyrazol-3-yl]-2-(trifluormethyl)benzamid (Verbindung (I-1-607) in Tabelle 1)

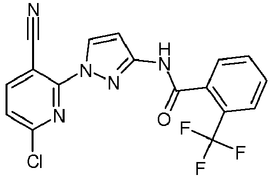

Analog zu Synthesebeispiel 25, Stufe 2 ausgehend von 2,6-Dichlornicotinonitril: HPLC-MS: logP = 3,14; Masse (m/z): 392.0/394.1 (M+H)⁺; 1H-NMR (DMSO-D₆) 7.08 (m, 1H), 7.64 (m, 1H), 7.69 - 7.72 (m, 2H), 7.76 (m, 1H), 7.82 (m, 1H), 8.48 (m, 1H), 8.55 (m, 1H), 11.66 (s, 1H).

### Synthesebeispiel 35:

### N-[1-(6-Chlor-5-cyanpyridin-2-yl)-1H-pyrazol-3-yl]-2-(trifluormethyl)benzamid (Verbindung (I-1-608) in Tabelle 1)

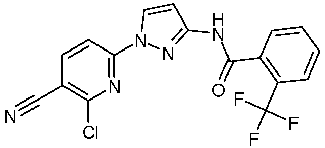

Analog zu Synthesebeispiel 25, Stufe 2 ebenfalls ausgehend von 2,6-Dichlornicotinonitril im Gemisch mit Synthesebeispiel 34: HPLC-MS: logP = 3,17; Masse (m/z): 392.0/394.1 (M+H)+; 1H-NMR (DMSO-D₆) 7.06 (m, 1H), 7.70 - 7.86 (m, 5H), 8.54 (m, 1H), 8.60 (m, 1H), 11.62 (s, 1H).

### Synthesebeispiel 36:

### N-{1-[3-Cyan-6-(diethylamino)pyridin-2-yl]-1H-pyrazol-3-yl}-2-(trifluormethyl)benzamid (Verbindung (I-1-568) in Tabelle 1)

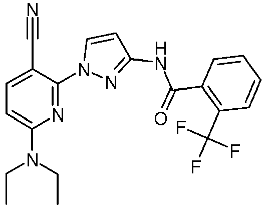

Analog zu Synthesebeispiel 25, Stufe 2 ausgehend von 2-Chlor-6-(diethylamino)nicotinonitril: HPLC-MS: logP = 3,76; Masse (m/z): 429.1 (M+H)+; 1H-NMR (DMSO-D₆) 1.17 (m, 6H), 3.60 (br, 4 H), 6.66 (m, 1H), 7.00 (m, 1H), 7.69 (m, 2H), 7.76 (m, 1H), 7.81 (m, 1H), 7.86 (m, 1H), 8.48 (m, 1H), 11.52 (s, 1H).

### Synthesebeispiel 37:

### N-[1-(2-Chlor-3-cyanpyridin-4-yl)-1H-pyrazol-3-yl]-2-(trifluormethyl)benzamid (Verbindung (I-1-585) in Tabelle 1)

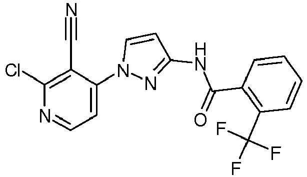

Analog zu Synthesebeispiel 25, Stufe 2 ausgehend von 2,4-Dichlornicotinonitril: HPLC-MS: logP = 2,67; Masse (m/z): 391.9/394.0 (M+H)⁺; 1H-NMR (DMSO-D₆) 7.15 (m, 1H), 7.71 (m, 2H), 7.79 (m, 1H), 7.84 (m, 1H), 7.95 (m, 1H), 8.67 - 8.70 (m, 2H), 11.69 (s, 1H).

### Synthesebeispiel 38:

### N-[1-(4-Chlor-3-cyanpyridin-2-yl)-1H-pyrazol-3-yl]-2-(trifluormethyl)benzamid (Verbindung (I-1-586) in Tabelle 1)

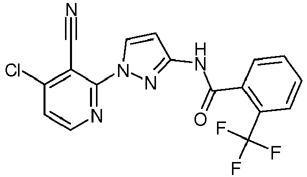

Analog zu Synthesebeispiel 25, Stufe 2 ebenfalls ausgehend von 2,4-Dichlornicotinonitril im Gemisch mit Synthesebeispiel 36 entstanden und durch Chromatographie an Kieselgel mit einem Cyclohexan/Essigsäureethylester Gradienten isoliert.: HPLC-MS: logP = 2,67; Masse (m/z): 391.9/394.0 (M+H)⁺; 1H-NMR (DMSO-D₆) 7.08 (m, 1H), 7.71 (m, 2H), 7.78 (m, 1H), 7.85 (m, 1H), 8.58 (m, 1H), 8.68 (m, 2H), 11.66 (s, 1H).

### Synthesebeispiel 39:

### N-[1-(3-Cyan-6-propylpyridin-2-yl)-1H-pyrazol-3-yl]-2-(trifluormethyl)benzamid (Verbindung (I-1-573) in Tabelle 1)

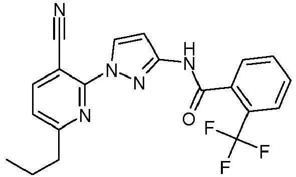

Analog zu Synthesebeispiel 25, Stufe 2 ausgehend von 2-Chlor-6-propylnicotinonitril: HPLC-MS: logP = 3.80; Masse (m/z): 400.1 (M+H)⁺; 1H-NMR (DMSO-D₆) 0.95 (m, 3H), 1.77 (m, 2H), 2.82 (m, 2H), 7.05 (m, 1H), 7.40 (m, 1H), 7.70 (m, 2H), 7.76 (m, 1H), 7.83 (m, 1H), 7.95 (m, 1H), 8.32 (m, 1H), 8.58 (m, 1H), 11.59 (s, 1H).

### Synthesebeispiel 40:

### N-{1-[3-Cyan-6-cyclopropyl-4-(trifluormethyl)pyridin-2-yl]-1H-pyrazol-3-yl}-2-(trifluormethyl)-benzamid (Verbindung (I-1-570) in Tabelle 1)

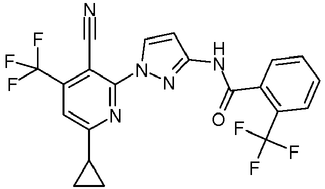

Analog zu Synthesebeispiel 25, Stufe 2 ausgehend von 2-Chlor-6-cyclopropyl-4-(trifluormethyl)nico-tinonitril: HPLC-MS: logP = 3.99; Masse (m/z): 466.1 (M+H)⁺; 1H-NMR (DMSO-D₆) 1.24 (m, 4H), 2.47 (m, 1H), 7.07 (m, 1H), 7.77 (m, 2H), 7.83 (m, 1H), 7.96 (s, 1H), 8.59 (m, 1H), 8.58 (m, 1H), 11.64 (s, 1H).

### Synthesebeispiel 41:

### N-[1-(4-Amino-2,6-difluorphenyl)-1H-pyrazol-3-yl]-2-(trifluormethyl)benzamid (Verbindung (I-1-227) in Tabelle 1)

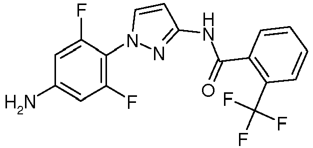

Analog zu Synthesebeispiel 24, Stufe 2, ausgehend von N-[1-(2,6-Difluor-4-nitrophenyl)-1H-pyrazol-3-yl]-2-(trifluormethyl)benzamid (Synthesebeispiel 26, 1.00 g) erhielt man 830 mg der Titelverbindung : HPLC-MS: logP = 2,32; Masse (m/z): 383,1 (M+H)⁺; 1H-NMR (DMSO-D₆) 6.09 (m, 2H), 6.33 (m, 2H), 6.80 (m, 1H), 7.68 (m, 2H), 7.55 (m, 1H), 7.81 (m, 2H), 11.18 (s, 1H).

### Synthesebeispiel 42:

### N-[1-(4-Acetamido-2,6-difluorphenyl)-1H-pyrazol-3-yl]-2-(trifluormethyl)benzamid (Verbindung (I-1-583) in Tabelle 1)

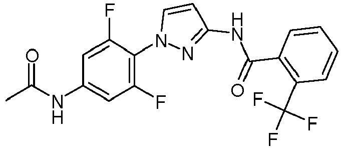

Eine Mischung von N-[1-(4-Amino-2,6-difluorphenyl)-1H-pyrazol-3-yl]-2-(trifluormethyl)benzamid (100 mg) und Essigsäureanhydrid (1 g) wurde bei Raumtemperatur für eine Stunde gerührt, mit Wasser (30 ml) verdünnt und die Titelverbindung (98 mg) durch Filtration isoliert: HPLC-MS: logP = 2,28; Masse (m/z): 425,1 (M+H)⁺; 1H-NMR (DMSO-D₆) 2.11 (s, 3H), 6.89 (m, 1H), 7.52 (m, 2H), 7.69 (m, 2H), 7.76 (m, 1H), 7.82 (m, 1H), 7.99 (m, 1H), 10.51 (s, 1H), 11.96 (s, 1H).

### Synthesebeispiel 43:

### N-[1-(3-Cyanpyrazin-2-yl)-1H-pyrazol-3-yl]-2-(trifluormethyl)benzamid (Verbindung (I-1-574) in Tabelle 1)

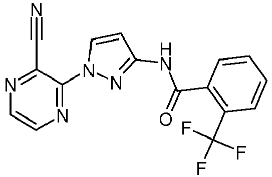

Analog zu Synthesebeispiel 25, Stufe 2 ausgehend von 3-Chlorpyrazin-2-carbonitril: HPLC-MS: logP = 2,47; Masse (m/z): 359,0 (M+H)⁺; 1H-NMR (DMSO-D₆) 7.12 (m, 1H), 7.72 (m, 2H), 7.78 (m, 1H), 7.83 (m, 1H), 8.63 (m, 1H), 8.75 (m, 1H), 8.85 (m, 1H), 11.71 (s, 1H).

### Synthesebeispiel 44:

### N-[1-(6-Chlor-5-cyan-3-fluorpyridin-2-yl)-1H-pyrazol-3-yl]-2-(trifluormethyl)benzamid (Verbindung (I-1-606) in Tabelle 1)

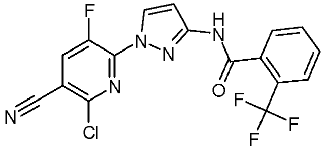

Analog zu Synthesebeispiel 25, Stufe 2 ausgehend von 2,6-Dichlor-5-fluornicotinonitril: HPLC-MS: logP = 2,94; Masse (m/z): 410,0/411,9 (M+H)⁺; 1H-NMR (DMSO-D₆) 7.11 (m, 1H), 7.70 (m, 2H), 7.77 (m, 1H), 7.83 (m, 1H), 8.52 (m, 1H), 8.80 (m, 1H), 8.85 (m, 1H), 11.89 (s, 1H).

### Synthesebeispiel 45:

### N-[2-(3,5-Difluorpyridin-2-yl)-2H-1,2,3-triazol-4-yl]-2-(trifluormethyl)benzamid (Verbindung (I-2-63) in Tabelle 3)

### Syntheseschema des Zwischeuprodukts 2-(3,5-Difluorpyridin-2-yl)-2H-1,2,3-triazol-4-amin (Bsp.45-V) (gemäß Route A-3)

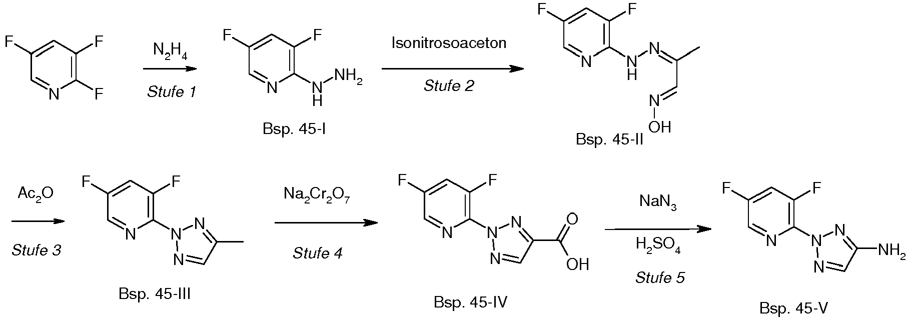

### Stufe 1: 3,5-Difluor-2-hydrazinopyridin (Bsp. 45-I)

25g 2,3,5-Trifluorpyridin wurden gemeinsam mit einem Überschuss an Hydrazinhydrat für 1 h unter Rückfluss erhitzt. Anschließend wurde die Reaktionsmischung wasserdampfdestilliert und ca. 1 1 Destillat wurde gesammelt. Das Destillat wurde 5-mal mit jeweils 250 ml Dichlormethan extrahiert, die organischen Phasen über Natriumsulfat getrocknet und anschließend das Lösungsmittel am Rotationsverdampfer entfernt. Man erhielt rohes Hydrazino-3,5-difluorpyridin (48% der Theorie), welches ohne weitere Aufarbeitung in der nächsten Stufe umgesetzt wurde.

### Stufe 2: 2-[2-(3,5-Difluorpyridin-2-yl)hydrazinyliden]propanaloxim (Bsp. 45-II)

Das Hydrazino-3,5-difluorpyridin aus der ersten Stufe (Bsp. 45-I) wurde mit einem 20%igen Überschuss an Isonitrosoaceton in Ethanol 3 h unter Rückfluss erhitzt. Nach dem Abkühlen erhielt man einen Niederschlag, der abfiltriert und mit Ethanol gewaschen wurde. Man erhielt rohes Propanaloxim (65% der Theorie) welches ohne weitere Aufarbeitung in der nächsten Stufe umgesetzt wurde.

### Stufe 3: 3,5-Difluor-2-(4-methyl-2H-1,2,3-triazol-2-yl)(Bsp. 45-III)

Eine Lösung von 0,1 Mol des Hydrazonoximes *(Bsp. 45-II)* in Essigsäureanhydrid wurde langsam auf 130°C erhitzt und für 2 h bei dieser Temperatur gerührt. Überschüssiges Essigsäureanhydrid wurde am Rotationsverdampfer entfernt und das entstandene Methyltriazol (Bsp. 45-III) (70 % der Theorie) wurde ohne weitere Aufarbeitung weiter verwendet.

### Stufe 4: 2-(3,5-Difluorpyridin-2-yl)-2H-1,2,3-triazol-4-carbousäure (Bsp. 45-IV)

Natriumdichromat (0.2 mol) wurde in kleinen Portionen zu einer gut gerührten Lösung von Methyltriazol (Bsp. 45-III, 0,1 mol) in 66%iger Schwefelsäure gegeben. Jede einzelne Portion Dichromat wurde erst zugegeben, nachdem die gelb-orange Farbe des Cr⁶⁺ im Kolben verschwunden war. Die Portionierung wurde außerdem so dosiert, dass die Temperatur im Kolben bei ca. 80-90°C blieb. Anschließend wurde auf dem Dampfbad für 1 h erhitzt. Nach dem Abkühlen wurde die Mischung in etwa dieselbe Menge Eis gegossen und über Nacht stehengelassen. Die ausgefallene Säure wurde abfiltriert, mit Wasser gewaschen und getrocknet. Man erhielt 50% der Theorie der Säure (Bsp 45-IV).

### Stufe 5: 2-(3,5-Difluorpyridin-2-yl)-2H-1,2,3-triazol-4-amin (Bsp.45-V)

Zu einer gerührten Mischung der Säure aus der Vorstufe *(Bsp. 45-IV)* in 50 ml Chloroform und 50 ml konz. Schwefelsäure wurde Natriumazid innerhalb von 2 h in kleinen Portionen zugegeben. Die Reaktion wurde für weitere 2 h bei 55°C gerührt. Nach dem Abkühlen wurde die Mischung in etwa dieselbe Menge Eis gegossen und über Nacht stehen gelassen, anschließend von Feststoffen abfiltriert und die Mutterlauge mit Ammoniak-Lösung alkalisch gestellt. Das ausgefallene Amin wurde abfiltriert, mit Wasser gewaschen und getrocknet. Man erhielt 70% der Theorie. HPLC-MS: logP = 0,65; Masse (m/z): 198,0 (M+H)⁺; ¹H-NMR (DMSO-D₆) 5.61 (s, 2H), 7.38 (s, 1H), 8.21 - 8.25 (m, 1H), 8.45 (s, 1H).

### N-[2-(3,5-Difluorpyridin-2-yl)-2H-1,2,3-triazol-4-yl]-2-(trifluormethyl)benzamid (Verbindung (I-2-63) in Tabelle 3)

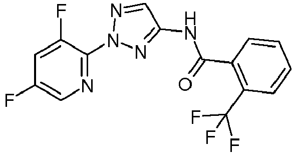

Analog zu Synthesebeispiel 9 Stufe 2 wurde 2-(3,5-Difluorpyridin-2-yl)-2H-1,2,3-triazol-4-amin (Bsp. 45-V; 174 mg) mit 2-(Trifluormethyl)benzoylchlorid (150 mg) und Triethylamin (0,23 mL) in 4 ml Dichlormethan umgesetzt. Nach säulenchromatographischer Reinigung und präparativer HPLC wurden 207 mg der Titelverbindung erhalten. HPLC-MS: logP = 2,38; Masse (m/z): 370,0 (M+H)⁺; ¹H-NMR (DMSO-D₆) 7.72 - 7.83 (m, 3H), 8.35 - 8.40 (m, 1H), 8.45 - 8.47 (m, 1H), 8.57-8-58 (s, 1H), 11.96-11-97 (d, 1H).

Auf analoge Weise zu Bsp. 45-V erhielt man die folgenden neuen Zwischenprodukte (gemäß Route A-3):

### 2-(3-Fluorpyridin-2-yl)-2H-1,2,3-triazol-4-amin:

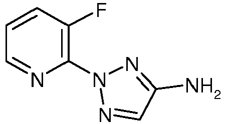

HPLC-MS: logP = 0,40; Masse (m/z): 180,0 (M+H)⁺; ¹H-NMR (DMSO-D₆) 5.61 (s, 2H), 7.39 (s, 1H), 7.48-7.52 (m, 1H), 7.95-8.00 (m, 1H), 8.35-8.36 (m, 1H).

### 2-[5-(Trifluormethyl)pyridin-2-yl)-2H-1,2,3-triazol-4-amin:

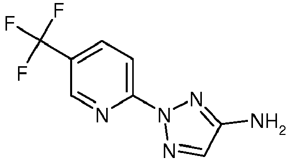

HPLC-MS: logP = 1.53; Masse (m/z): 230,0 (M+H)⁺; ¹H-NMR (DMSO-D₆) 5.86 (s, 2H), 7.48 (s, 1H), 7.91-7.94 (m, 1H), 8.29-8.31 (m, 1H), 8.8 (s, 1H).

### 2-(3,5-Dichlorpyridin-2-yl)-2H-1,2,3-triazol-4-amin:

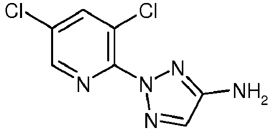

HPLC-MS: logP = 1.38; Masse (m/z): 229,9 (M+H)⁺; ¹H-NMR (DMSO-D₆) 5.60 (s, 2H), 7.38 (s, 1H), 8.48-8.49 (d, 1H), 8.58 - 8.59 (d, 1H).

### 2-[3-Chlor-5-(trifluormethyl)pyridin-2-yl)-2H-1,2,3-triazol-4-amin:

HPLC-MS: logP = 1.74; Masse (m/z): 263,9 (M+H)⁺; ¹H-NMR (DMSO-D₆) 5.76 (s, 2H), 7.48 (s, 1H), 8.68-8.69 (d, 1H), 8.89 - 8.90 (d, 1H).

### Synthesebeispiel 46

### N-[1-(2,6-Difluorphenyl)-1H-pyrrol-3-yl]-2-(trifluormethyl)benzamid (Verbindung (I-3-1) in Tabelle 5)

### Syntheseschema des Zwischeuprodukts: 1-(2,6-Difluorphenyl)-1H-pyrrol-3-amin (Bsp.46-III) (gemäβ Route A-4)

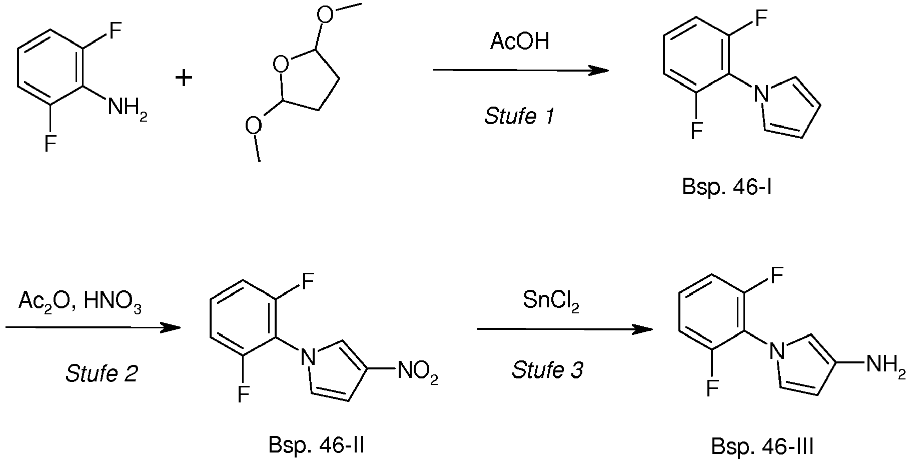

### Stufe 1: 1-(2,6-Difluorphenyl)-1H-pyrrol (Bsp. 46-I)

Zu 0,39 Mol 2,6-Difluoranilin in 100 ml Eisessig tropfte man unter Rühren langsam 0,39 Mol 2,5-Dimethoxytetrahydrofuran und rührte noch 1 h bei 100°C. Nach Abkühlung auf Raumtemperatur wurde der Eisessig am Rotationsverdampfer entfernt und der Rückstand noch mehrmals mit Toluol eingedampft. Nach säulenchromatographischer Reinigung des Rückstandes mit Cyclohexan / Essigester (10:1 bis 1:1) erhielt man 5,6 g, 78 % der Theorie des gewünschten Pyrrols mit einer Reinheit von 96% (LCMS). HPLC-MS: logP = 2,98; ¹H-NMR (CD₃CN) 6,28 - 6,29 (m, 2H), 6,95 - 7.00 (m, 2H), 7.30 - 7.35 (m, 2H), 7,45-7,52 (m, 1H).

### Stufe 2: 1-(2,6-Difluorphenyl)-3-nitro-1H-pyrrol (Bsp. 46-II)

2 ml konz. Salpetersäure wurden bei -10 °C zu 162 ml Acetanhydrid vorsichtig zugetropft. Diese Mischung wurde dann in eine Lösung aus 5,4 g (0,31 Mol) 1-(2,6-Difluorphenyl)-1H-pyrrol in 80 ml Acetanhydrid bei -40°C zugetropft. Anschließend ließ man weitere 2 h bei dieser Temperatur rühren.

Die Reaktionsmischung wurde auf Eis gegossen und dreimal mit Chloroform extrahiert. Nach dem Trocknen der organischen Phase über Natriumsulfat wurde das Lösungsmittel am Rotationsverdampfer entfernt. Nach säulenchromatographischer Reinigung des Rückstandes mit Cyclohexan / Essigester (10:1 bis 1:1) erhielt man neben dem 2-Nitro Isomer 3,2 g, (48 % der Theorie) des gewünschten 3-Nitropyrrols mit einer Reinheit von 99% (LCMS). HPLC-MS: logP = 2,58; Masse (m/z): 225,1 (M+H)⁺; ¹H-NMR (CD₃CN) 6,60 - 6,62 (m, 1H), 7,37 - 7.42 (m, 2H), 7.55 - 7.58 (m, 2H), 7,62-7,70 (m, 1H).

### Stufe 3: 1-(2,6-Difluorphenyl)-1H-pyrrol-3-amin (Bsp. 46-III)

575 mg (2,57 mMol) des 3-Nitropyrrols wurden mit 2,43 g (12,8 mMol) Zinnchlorid bei 80 °C in 17 ml Essigsäureethylester über Nacht gerührt. Nach dem Abkühlen wurde abfiltriert und das Lösungsmittel am Rotationsverdampfer entfernt. Der so erhaltene Rückstand wurde direkt und ohne weitere Aufarbeitung in der Folgereaktion eingesetzt.

### N-[1-(2,6-Difluorphenyl)-1H-pyrrol-3-yl]-2-(trifluormethyl)benzamid (Verbindung (I-3-1) in Tabelle 5)

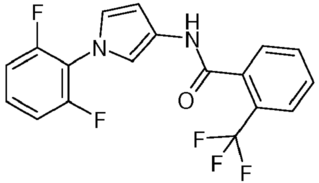

Analog zu Synthesebeispiel 9 Stufe 2 wurde das rohe *1-(2,6-Difluorphenyl)-1H-pyrrol-3-amin* (85 mg) mit 2-(Trifluormethyl)benzoylchlorid (92 mg) und Triethylamin (0,12 mL) in 1,3 ml Dichlormethan umgesetzt. Nach säulenchromatographischer Reinigung und präparativer HPLC wurden 26 mg der Titelverbindung erhalten. HPLC-MS: logP = 2,96; Masse (m/z): 367,0 (M+H)⁺; ¹H-NMR (DMSO-D₆) 6,31-6,32 (m, 1H), 6,92-6,94 (m, 1H), 7,31-7,39 (m, 3H), 7,48-7,52 (m, 1H), 7,66-7,84 (m, 4H).

### Synthesebeispiel 47

### N-(1-(2-Chlorphenyl)-1H-indazol-3-yl)-2-(trifluormethyl)benzamid (Verbindung (I-5-8) in Tabelle 4)

### Stufe 1: 1-(2-Chlorphenyl)-1H-indazol-3-amin (gemäß Route B-2)

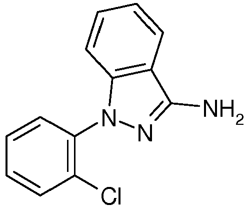

2-Iodbenzonitril (500 mg), N'-(2-Chlorphenyl)benzohydrazid (646 mg), Kaliumcarbonat (1207 mg), Kupfer(I)bromid (31 mg) und trans-4-Hydroxy-L-prolin (57 mg) wurden in Dimethylsulfoxid (7,5 mL) für 48 h bei 90°C unter Argon gerührt. Nach dem Abkühlen wurde das Reaktionsgemisch in Ethylacetat aufgenommen, mit gesättigter Ammoniumchloridlösung und gesättigter Natriumchlorid gewaschen, auf Kieselgel adsorbiert und an Kieselgel mit Cyclohexan/Ethylacetat als Laufmittel säulenchromatographisch gereinigt. Es wurden 226 mg der Titelverbindung erhalten. HPLC-MS: logP = 2,32; Masse (m/z): 244,0 (M+H)⁺; ¹H-NMR (DMSO-D₆) 5.83 (br. s, 2H), 7.01 - 7.09 (m, 2H), 7.31 - 7.35 (m, 1H), 7.42 - 7.49 (m, 3H), 7.67 - 7.69 (m, 1H), 7.82 - 7.84 (m, 1H).

### Stufe 2: N-[1-(2-Chlorphenyl)-1H-indazol-3-yl]-2-(trifluormethyl)benzamid (Verbindung 1-5-8 in Tabelle 4)

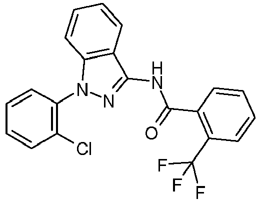

Analog zu Synthesebeispiel 9 Stufe 2 wurde 1-(2-Chlorphenyl)-1H-indazol-3-amin (174 mg) mit 2-(Trifluormethyl)benzoylchlorid (47 mg) und Triethylamin (0,6 mL) in 4 ml Dichlormethan umgesetzt. Nach säulenchromatographischer Reinigung und präparativer HPLC wurden 20 mg der Titelverbindung erhalten. HPLC-MS: logP = 3,54; Masse (m/z): 416,0 (M+H)⁺; ¹H-NMR (DMSO-D₆) 7.19 - 7.28 (m, 2H), 7.45 - 7.49 (m, 1H), 7.57 - 7.63 (m, 3H), 7.65 - 7.95 (m, 6H), 11.31 (s, 1H).

Auf analoge Weise zu Bsp. 47 Stufe 1 kann aus 2-Fluor-6-iodbenzonitril und N'-(4-Cyan-2,5-difluorphenyl)benzohydrazid 4-(3-Amino-4-fluor-1H-indazol-1-yl)-2,5-difluorbenzonitril erhalten werden.

### Synthesebeispiel 48

### N-[1-(3,5-Difluor-1-oxidopyridin-2-yl)-1H-pyrazol-3-yl]-2-(trifluormethyl)benzamid (Verbindung I-1-438 in Tabelle 1)

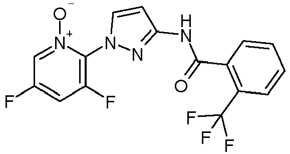

N-[1-(3,5-Difluorpyridin-2-yl)-1H-pyrazol-3-yl]-2-(trifluormethyl)benzamid (1-1-75 in Tabelle 1; 150 mg) wurden mit Wasserstoffperoxid-Harnstoff (153 mg) in in 3 ml Dichlormethan 30 min gerührt. Anschließend wurde auf dem Eisbad bei 0-5°C Trifluoressigsäureanhydrid (342 mg) zugetropft und über Nacht gerührt. Die Mischung wurde mit Natriumsulfit-Lösung versetzt, mit Wasser gewaschen, über Natriumsulfat getrocknet und anschließend am Rotationsverdampfer eingeengt. Nach säulenchromatographischer Reinigung und präparativer HPLC wurden 35 mg der Titelverbindung erhalten. HPLC-MS: logP = 1,69; Masse (m/z): 385,1 (M+H)⁺; ¹H-NMR (DMSO-D₆) 6.95 - 6.96 (d, 1H), 7.68 - 7.78 (m, 4H), 7.82 - 7.96 (m, 1H), 8.05 - 8.06 (m, 1H), 8.83 - 8.85 (m, 1H), 11.33 (s, 1H).

Die in den Tabellen 1 bis 5 beschriebenen Verbindungen der Formeln (I-1), (I-4), (I-2), (I-5) und (I-3) sind ebenfalls bevorzugte Verbindungen, die gemäß oder in Analogie zu den oben beschriebenen Synthesebeispielen erhalten wurden.

**Tabelle 1**

| | | | | | | |
|---|---|---|---|---|---|---|
| 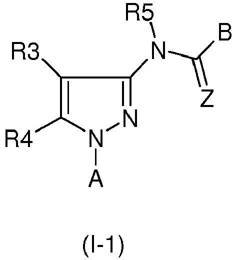 | | | | | | |

| **Verbindung Nr.** | **A** | **B** | **R3** | **R4** | **R5** | **Z** |
|---|---|---|---|---|---|---|
| I-1-1 Synthesebeispiel 1 | 2-Methylphenyl | 2-Bromphenyl | H | H | H | O |
| I-1-2 | 2-Methoxyphenyl | 2-Bromphenyl | H | H | H | O |
| I-1-3 | 2-Methoxyphenyl | 2,5-Dimethyl-3-furanyl | H | H | H | O |
| I-1-4 | 2-(Trifluormethyl)phenyl | 2-Chlorphenyl | H | H | H | O |
| I-1-5 | 2-(Trifluormethyl)phenyl | 2-lodphenyl | H | H | H | O |
| I-1-6 | 2-(Trifluormethyl)phenyl | 2-(Trifluormethyl)phenyl | H | H | H | O |
| I-1-7 | 2-(Trifluormethyl)phenyl | 1-(Difluormethyl)-1H-pyrazol-5-yl | H | H | H | O |
| I-1-8 | 2-Cyanphenyl | 2-Chlorphenyl | H | H | H | O |
| I-1-9 | 2-Cyanphenyl | 2-(Trifluormethyl)phenyl | H | H | H | O |
| I-1-10 | 2-Fluorphenyl | 2-Bromphenyl | H | H | H | O |
| I-1-11 | 2-Chlorphenyl | 2-Methylphenyl | H | H | H | O |
| I-1-12 | 2-Chlorphenyl | 2-Ethylphenyl | H | H | H | O |
| I-1-13 | 2-Chlorphenyl | 2-Difluormethylphenyl | H | H | H | O |
| I-1-14 | 2-Chlorphenyl | 2-(Trifluormethyl)phenyl | H | H | H | O |
| I-1-15 Synthesebeispiel 17 | 2-Chlorphenyl | 2-(Trifluormethyl)phenyl | Cl | H | H | O |
| I-1-16 Synthesebeispiel 18 | 2-Chlorphenyl | 2-(Trifluormethyl)phenyl | Br | H | H | O |
| I-1-17 Synthesebeispiel 19 | 2-Chlorphenyl | 2-(Trifluormethyl)phenyl | I | H | H | O |
| I-1-18 | 2-Chlorphenyl | 2-Trifluormethoxyphenyl | H | H | H | O |
| I-1-19 | 2-Chlorphenyl | 2-Fluorphenyl | H | H | H | O |
| I-1-20 Synthesebeispiel 14 | 2-Chlorphenyl | 2-Chlorphenyl | H | H | H | O |
| I-1-21 Synthesebeispiel 15 | 2-Chlorphenyl | 2-Chlorphenyl | H | H | Methyl | O |
| I-1-22 | 2-Chlorphenyl | 2-Chlorphenyl | H | H | Ethyl | O |
| I-1-23 | 2-Chlorphenyl | 2-Chlorphenyl | H | H | 1-Propin-2-yl | O |
| I-1-24 | 2-Chlorphenyl | 2-Chlorphenyl | H | H | Cyclopropyl methyl | O |
| I-1-25 | 2-Chlorphenyl | 2-Chlorphenyl | H | H | Cyanmethyl | O |
| I-1-26 | 2-Chlorphenyl | 2-Chlorphenyl | H | H | Allyl | O |
| I-1-27 Synthesebeispiel 16 | 2-Chlorphenyl | 2-Chlorphenyl | F | H | H | O |
| I-1-28 | 2-Chlorphenyl | 2-Chlorphenyl | Cl | H | H | O |
| I-1-29 | 2-Chlorphenyl | 2-Chlorphenyl | Br | H | H | O |
| I-1-30 | 2-Chlorphenyl | 2-Chlorphenyl | I | H | H | O |
| I-1-31 | 2-Chlorphenyl | 2-Chlorphenyl | H | Methyl | H | O |
| I-1-32 | 2-Chlorphenyl | 2-Bromphenyl | H | H | H | O |
| I-1-33 | 2-Chlorphenyl | 2-lodphenyl | H | H | H | O |
| I-1-34 | 2-Chlorphenyl | 2-lodphenyl | Cl | H | H | O |
| I-1-35 | 2-Chlorphenyl | 2-lodphenyl | H | Methyl | H | O |
| I-1-36 | 2-Chlorphenyl | 2,6-Difluorphenyl | H | H | H | O |
| I-1-37 | 2-Chlorphenyl | 2-Chlor-6-fluorphenyl | H | H | H | O |
| I-1-38 | 2-Chlorphenyl | 3-Methylpyridin-2-yl | H | H | H | O |
| I-1-39 | 2-Chlorphenyl | 3-Trifluormethylpyridin-2-yl | H | H | H | O |
| I-1-40 | 2-Chlorphenyl | 3-Chlorpyridin-2-yl | H | H | H | O |
| I-1-41 | 2-Chlorphenyl | 2-Methylpyridin-3-yl | H | H | H | O |
| I-1-42 | 2-Chlorphenyl | 2-(Trifluormethyl)pyridin-3-yl | H | H | H | O |
| I-1-43 | 2-Chlorphenyl | 2-Chlorpyridin-3-yl | H | H | H | O |
| I-1-44 | 2-Chlorphenyl | 3-Trifluormethyl-2-pyrazinyl | H | H | H | O |
| I-1-45 | 2-Chlorphenyl | 2,5-Dimethyl-3-furanyl | H | H | H | O |
| I-1-46 | 2-Chlorphenyl | 3-Methyl-2-thienyl | H | H | H | O |
| I-1-47 | 2-Chlorphenyl | 2-lod-3-thienyl | H | H | H | O |
| I-1-48 | 2-Chlorphenyl | 5-Chlor-1,3-dimethyl-1H-pyrazol-4-yl | H | H | H | O |
| I-1-49 | 2-Chlorphenyl | 3-Difluormethyl-5-fluor-1-methyl-1H-pyrazol-4-yl | H | H | H | O |
| I-1-50 | 2-Bromphenyl | 2-(Trifluormethyl)phenyl | H | H | H | O |
| I-1-51 | 2-Bromphenyl | 2-Chlorphenyl | H | H | H | O |
| I-1-52 | 2-Bromphenyl | 2-Bromphenyl | H | H | H | O |
| I-1-53 | 2-Bromphenyl | 2-lodphenyl | H | H | H | O |
| I-1-54 | 2-Bromphenyl | 3-(Trifluormethyl)pyridine-2-yl | H | H | H | O |
| I-1-55 | 2-Bromphenyl | 3-Chlorpyridin-2-yl | H | H | H | O |
| I-1-56 | 2-Bromphenyl | 2-(Trifluormethyl)pyridin-3-yl | H | H | H | O |
| I-1-57 | 2-Bromphenyl | 2-Chlorpyridin-3-yl | H | H | H | O |
| I-1-58 | 2-Bromphenyl | 3-Difluormethyl-5-fluor-1-methyl-1H-pyrazol-4-yl | H | H | H | O |
| I-1-59 | 2-Bromphenyl | 5-fluor-1-methyl-3-trifluormethyl-1H-pyrazol-4-yl | H | H | H | O |
| I-1-60 | 2-Bromphenyl | 3-Methyl-2-thienyl | H | H | H | O |
| I-1-61 Synthesebeispiel 2 | 2,6-Dimethylphenyl | 2-Chlorphenyl | H | H | H | O |
| I-1-62 | 2,3-Difluorphenyl | 2-(Trifluormethyl)phenyl | H | H | H | O |
| I-1-63 Synthesebeispiel 3 | 2,3-Difluorphenyl | 2-Bromphenyl | H | H | H | O |
| I-1-64 | 2,4-Difluorphenyl | 2-(Trifluormethyl)phenyl | H | H | H | O |
| I-1-65 | 2,5-Difluorphenyl | 2-(Trifluormethyl)phenyl | H | H | H | O |
| I-1-66 | 2,5-Difluorphenyl | 2-Chlorphenyl | H | H | H | O |
| I-1-67 | 2,5-Difluorphenyl | 2-lodphenyl | H | H | H | O |
| I-1-68 | 2,5-Difluorphenyl | 3-(Trifluormethyl)pyridin-2-yl | H | H | H | O |
| I-1-69 | 2,5-Difluorphenyl | 2-Methylpyridin-3-yl | H | H | H | O |
| I-1-70 | 2,5-Difluorphenyl | 2-(Trifluormethyl)pyridin-3-yl | H | H | H | O |
| I-1-71 | 2,5-Difluorphenyl | 3-Methyl-2-thienyl | H | H | H | O |
| I-1-72 Synthesebeispiel 4 | 2,5-Difluorphenyl | 5-fluor-1-methyl-3-trifluormethyl-1H-pyrazol-4-yl | H | H | H | O |
| I-1-73 | 2,6-Difluorphenyl | 2-Methylphenyl | H | H | H | O |
| I-1-74 | 2,6-Difluorphenyl | 2-Difluormethylphenyl | H | H | H | O |
| I-1-75 | 2,6-Difluorphenyl | 2-(Trifluormethyl)phenyl | H | H | H | O |
| I-1-76 | 2,6-Difluorphenyl | 2-(Trifluormethyl)phenyl | F | H | H | O |
| I-1-77 | 2,6-Difluorphenyl | 2-(Trifluormethyl)phenyl | H | H | H | S |
| I-1-78 | 2,6-Difluorphenyl | 2-Ethylphenyl | H | H | H | O |
| I-1-79 | 2,6-Difluorphenyl | 2-Trifluormethoxyphenyl | H | H | H | O |
| I-1-80 | 2,6-Difluorphenyl | 2-(Methylsulfonyl)phenyl | H | H | H | O |
| I-1-81 | 2,6-Difluorphenyl | 2-Chlorphenyl | H | H | H | O |
| I-1-82 | 2,6-Difluorphenyl | 2-Bromphenyl | H | H | H | O |
| I-1-83 | 2,6-Difluorphenyl | 2-lodphenyl | H | H | H | O |
| I-1-84 | 2,6-Difluorphenyl | 2,6-Difluorphenyl | H | H | H | O |
| I-1-85 | 2,6-Difluorphenyl | 2-Chlor-6-fluorphenyl | H | H | H | O |
| I-1-86 | 2,6-Difluorphenyl | 2-Nitrophenyl | H | H | H | O |
| I-1-87 | 2,6-Difluorphenyl | 2-Hydroxyphenyl | H | H | H | O |
| I-1-88 | 2,6-Difluorphenyl | 2-[(Trifluormethyl)sulfanyl]ph enyl | H | H | H | O |
| I-1-89 | 2,6-Difluorphenyl | 2-(1H-1,2,4-triazol-1-yl)phenyl | H | H | H | O |
| I-1-90 | 2,6-Difluorphenyl | 3-Methylpyridin-2-yl | H | H | H | O |
| I-1-91 Synthesebeispiel 7 | 2,6-Difluorphenyl | 3-(Trifluormethyl)pyridin-2-yl | H | H | H | O |
| I-1-92 | 2,6-Difluorphenyl | 3-Chlorpyridin-2-yl | H | H | H | O |
| I-1-93 | 2,6-Difluorphenyl | 2-(Trifluormethyl)pyridin-3-yl | H | H | H | O |
| I-1-94 | 2,6-Difluorphenyl | 4-(Trifluormethyl)pyridin-3-yl | H | H | H | O |
| I-1-95 | 2,6-Difluorphenyl | 2-Chlorpyridin-3-yl | H | H | H | O |
| I-1-96 | 2,6-Difluorphenyl | 2-Brompyridin-3-yl | H | H | H | O |
| I-1-97 | 2,6-Difluorphenyl | 3-(Trifluormethyl)pyridine-4-yl | H | H | H | O |
| I-1-98 | 2,6-Difluorphenyl | 3-Ethylpyridazin-4-yl | H | H | H | O |
| I-1-99 | 2,6-Difluorphenyl | 3-(Trifluormethyl)pyrazin-2-yl | H | H | H | O |
| I-1-100 | 2,6-Difluorphenyl | 3-Chlorpyrazin-2-yl | H | H | H | O |
| I-1-101 | 2,6-Difluorphenyl | 2-Methyl-5,6-dihydro-1,4-oxathiin-3-yl | H | H | H | O |
| I-1-102 | 2,6-Difluorphenyl | 2-(Trifluormethyl)-5,6-dihydro-1,4-oxathiin-3-yl | H | H | H | O |
| I-1-103 | 2,6-Difluorphenyl | 3-Methyl-2-thienyl | H | H | H | O |
| I-1-104 | 2,6-Difluorphenyl | 3-lod-2-thienyl | H | H | H | O |
| I-1-105 | 2,6-Difluorphenyl | 2-lod-3-thienyl | H | H | H | O |
| I-1-106 | 2,6-Difluorphenyl | 5-(Difluormethyl)-3-methyl-1, 2-oxa zo I-4-yl | H | H | H | O |
| I-1-107 | 2,6-Difluorphenyl | 1,3-Dimethyl-1H-pyrazol-4-yl | H | H | H | O |
| I-1-108 | 2,6-Difluorphenyl | 3-lod-1-methyl-1H-pyrazol-4-yl | H | H | H | O |
| I-1-109 | 2,6-Difluorphenyl | 1,3,5-Trimethyl-1H-pyrazol-4-yl | H | H | H | O |
| I-1-110 | 2,6-Difluorphenyl | 1,3-Dimethyl-5-fluor-1H-pyrazol-4-yl | H | H | H | O |
| I-1-111 | 2,6-Difluorphenyl | 1-(Difluormethyl)-1H-pyrazol-5-yl | H | H | H | O |
| I-1-112 | 2,6-Difluorphenyl | 5-Fluor-l-methyl-3-(trifluormethyl)-1H-pyrazol-4-yl | H | H | H | O |
| I-1-113 | 2,6-Difluorphenyl | 2,4-Dimethyl-1,3-thiazol-5-yl | H | H | H | O |
| I-1-114 | 2,6-Difluorphenyl | 2-Methyl-4-trifluormethyl-1,3-thiazol-5-yl | H | H | H | O |
| I-1-115 | 2,6-Difluorphenyl | 3,4-Dichlor-1,2-thiazol-5-yl | H | H | H | O |
| I-1-116 | 2,6-Difluorphenyl | 4-Methyl-1,2,5-oxadiazol-3-yl | H | H | H | O |
| I-1-117 Synthesebeispiel 5 | 2-Ethoxy-6-fluorphenyl | 2-Chlorphenyl | H | H | H | O |
| I-1-118 | 2,5-Dichlorphenyl | 2-Chlorphenyl | H | H | H | O |
| I-1-119 | 2,5-Dichlorphenyl | 2-Bromphenyl | H | H | H | O |
| I-1-120 | 2,6-Dichlorphenyl | 2-(Trifluormethyl)phenyl | H | H | H | O |
| I-1-121 | 2,6-Dichlorphenyl | 2-Chlorphenyl | H | H | H | O |
| I-1-122 | 2,6-Dichlorphenyl | 2-Bromphenyl | H | H | H | O |
| I-1-123 Synthesebeispiel 8 | 2,6-Dichlorphenyl | 2-lodphenyl | H | H | H | O |
| I-1-124 | 2,6-Dichlorphenyl | 2-(Trifluormethyl)pyridin-3-yl | H | H | H | O |
| I-1-125 | 2-Nitrophenyl | 2-Chlorphenyl | H | H | H | O |
| I-1-126 | 2-(Methylsulfonyl)phenyl | 2-Chlorphenyl | H | H | H | O |
| I-1-127 | 2-Pyridyl | 2-Bromphenyl | H | H | H | O |
| I-1-128 | 3-Fluorpyridin-2-yl | 2-(Trifluormethyl)phenyl | H | H | H | O |
| I-1-129 | 3-Fluorpyridin-2-yl | 2-Chlorphenyl | H | H | H | O |
| I-1-130 | 3-Fluorpyridin-2-yl | 2-Bromphenyl | H | H | H | O |
| I-1-131 | 3-Fluorpyridin-2-yl | 2-lodphenyl | H | H | H | O |
| I-1-132 | 3-Fluorpyridin-2-yl | 2,6-Difluorphenyl | H | H | H | O |
| I-1-133 | 3-Fluorpyridin-2-yl | 2-(Trifluormethyl)pyridin-3-yl | H | H | H | O |
| I-1-134 | 3-Fluorpyridin-2-yl | 2-(Trifluormethyl)pyridin-3-yl | H | H | 2-(Trifluormet hyl)-pyridin-3-ylcarbonyl | O |
| I-1-135 | 3-Chlorpyridin-2-yl | 2-(Trifluormethyl)phenyl | H | H | H | O |
| I-1-136 | 3-Chlorpyridin-2-yl | 2-Chlorphenyl | H | H | H | O |
| I-1-137 | 3-Chlorpyridin-2-yl | 2-Bromphenyl | H | H | H | O |
| I-1-138 | 3-Chlorpyridin-2-yl | 2-Iodphenyl | H | H | H | O |
| I-1-139 Synthesebeispiel 11 | 2-Chlorpyridin-3-yl | 2-Iodphenyl | H | H | H | O |
| I-1-140 | 3,5-Difluorpyridin-2-yl | 2-(Trifluormethyl)phenyl | H | H | H | O |
| I-1-141 | 3,5-Difluorpyridin-2-yl | 2-(Trifluormethyl)phenyl | I | H | H | O |
| I-1-142 | 3,5-Difluorpyridin-2-yl | 2-(Trifluormethyl)phenyl | H | H | Acetyl | O |
| I-1-143 | 3,5-Difluorpyridin-2-yl | 2-Chlorphenyl | H | H | H | O |
| I-1-144 | 3,5-Difluorpyridin-2-yl | 2-Bromphenyl | H | H | H | O |
| I-1-145 | 3,5-Difluorpyridin-2-yl | 2-Iodphenyl | H | H | H | O |
| I-1-146 Synthesebeispiel 9 | 3,5-Difluorpyridin-2-yl | 2,6-Difluorphenyl | H | H | H | O |
| I-1-147 | 3,5-Difluorpyridin-2-yl | 2-Nitrophenyl | H | H | H | O |
| I-1-148 | 3,5-Difluorpyridin-2-yl | 2-Hydroxyphenyl | H | H | H | O |
| I-1-149 | 3,5-Difluorpyridin-2-yl | 2-[(Trifluormethyl)sulfanyl]ph enyl | H | H | H | O |
| I-1-150 | 3,5-Difluorpyridin-2-yl | 2-(1H-1,2,4-triazol-1-yl)phenyl | H | H | H | O |
| I-1-151 | 3,5-Difluorpyridin-2-yl | 2-(Trifluormethyl)pyridin-3-yl | H | H | H | O |
| I-1-152 | 3,5-Difluorpyridin-2-yl | 2-Chlorpyridin-3-yl | H | H | H | O |
| I-1-153 | 3,5-Difluorpyridin-2-yl | 3-(Trifluormethyl)pyridin-4-yl | H | H | H | O |
| I-1-154 | 3,5-Difluorpyridin-2-yl | 4-(Trifluormethyl)pyrimidin-5-yl | H | H | H | O |
| I-1-155 | 3,5-Difluorpyridin-2-yl | 2-(Trifluormethyl)-5,6-dihydro-1,4-oxathiin-3-yl | H | H | H | O |
| I-1-156 | 3,5-Difluorpyridin-2-yl | 1-Methyl-4-(trifluormethyl)-1H-pyrrol-3-yl | H | H | H | O |
| I-1-157 | 3,5-Difluorpyridin-2-yl | 5-(Difluormethyl)-3-methyl-1,2-oxazol-4-yl | H | H | H | O |
| I-1-158 | 3,5-Difluorpyridin-2-yl | S-Fluor-1-methyl-3-(trifluormethyl)-1H-pyrazol-4-yl | H | H | H | O |
| I-1-159 | 3,5-Difluorpyridin-2-yl | 3,4-Dichlor-1,2-thiazol-5-yl | H | H | H | O |
| I-1-160 | 3,5-Difluorpyridin-2-yl | 4-Methyl-1,2,3-thiadiazol-5-yl | H | H | H | O |
| I-1-161 | 2,5-Difluorpyridin-3-yl | 2-Iodphenyl | H | H | H | O |
| I-1-162 | 3,5,6-Trifluorpyridin-2-yl | 2-(Trifluormethyl)phenyl | H | H | H | O |
| I-1-163 | 3,5,6-Trifluorpyridin-2-yl | 2-Chlorphenyl | H | H | H | O |
| I-1-164 Synthesebeispiel 10 | 3,5,6-Trifluorpyridin-2-yl | 2-Bromphenyl | H | H | H | O |
| I-1-165 | 3,5,6-Trifluorpyridin-2-yl | 2-Iodphenyl | H | H | H | O |
| I-1-166 | 3,5,6-Trifluorpyridin-2-yl | 2,6-Difluorphenyl | H | H | H | O |
| I-1-167 | 3,5,6-Trifluorpyridin-2-yl | 3-(Trifluormethyl)pyridin-2-yl | H | H | H | O |
| I-1-168 | 2-Pyrimidinyl | 2-Bromphenyl | H | H | H | O |
| I-1-169 Synthesebeispiel 12 | 5,6-Difluorpyrimidin-4-yl | 2-(Trifluormethyl)phenyl | H | H | H | O |
| I-1-170 | 5,6-Difluorpyrimidin-4-yl | 2-Bromphenyl | H | H | H | O |
| I-1-171 | 5,6-Difluorpyrimidin-4-yl | 2,6-Difluorphenyl | H | H | H | O |
| I-1-172 | 3-Pyridazinyl | 2-Chlorphenyl | H | H | H | O |
| I-1-173 | 3-Fluorpyrazin-2-yl | 2-(Trifluormethyl)phenyl | H | H | H | O |
| I-1-174 Synthesebeispiel 13 | 3-Fluorpyrazin-2-yl | 2-Chlorphenyl | H | H | H | O |
| I-1-175 | 3-Fluorpyrazin-2-yl | 2-Bromphenyl | H | H | H | O |
| I-1-176 | 3-Fluorpyrazin-2-yl | 2-Iodphenyl | H | H | H | O |
| I-1-177 | 3-Fluorpyrazin-2-yl | 2,6-Difluorphenyl | H | H | H | O |
| I-1-178 | 3-Fluorpyrazin-2-yl | 3-(Trifluormethyl)pyridin-2-yl | H | H | H | O |
| I-1-179 | 2-(Methylsulfonyl)phenyl | 2-(Trifluormethyl)phenyl | H | H | H | O |
| I-1-180 | 2-(Methylsulfonyl)phenyl | 2-(Trifluormethyl)pyridin-3-yl | H | H | H | O |
| I-1-181 | 2-(Methylsulfonyl)phenyl | 2,6-Difluorphenyl | H | H | H | O |
| I-1-182 | 2-(Methylsulfonyl)phenyl | 2-Bromphenyl | H | H | H | O |
| I-1-183 | 3-Chlor-5-fluorpyridin-2-yl | 2-(Trifluormethyl)phenyl | H | H | H | O |
| I-1-184 | 2-(Methylsulfonyl)phenyl | 2-lodphenyl | H | H | H | O |
| I-1-185 | 2-(Trifluormethyl)phenyl | 1,3,5-Trimethyl-1H-pyrazol-4-yl | H | H | H | O |
| I-1-186 | 2-(Trifluormethyl)phenyl | 2-Bromphenyl | H | H | H | O |
| I-1-187 | 2,3-Dichlorphenyl | 2-Bromphenyl | H | H | H | O |
| I-1-188 | 2,3-Dichlorphenyl | 2-Chlorphenyl | H | H | H | O |
| I-1-189 | 2,3-Difluorphenyl | 2-Chlorphenyl | H | H | H | O |
| I-1-190 | 2,3-Difluorphenyl | 2-Iodphenyl | H | H | H | O |
| I-1-191 | 2,4,5-Trifluorphenyl | 2-(Trifluormethyl)phenyl | H | H | H | O |
| I-1-192 | 2,4,5-Trifluorphenyl | 2-(Trifluormethyl)pyridin-3-yl | H | H | H | O |
| I-1-193 | 2,4,5-Trifluorphenyl | 2,6-Difluorphenyl | H | H | H | O |
| I-1-194 | 2,4,5-Trifluorphenyl | 2-Bromphenyl | H | H | H | O |
| I-1-195 | 2,4,5-Trifluorphenyl | 2-Chlorphenyl | H | H | H | O |
| I-1-196 | 2,4,5-Trifluorphenyl | 2-Iodphenyl | H | H | H | O |
| I-1-197 | 2,4-Dichlorphenyl | 2-(Trifluormethyl)phenyl | H | H | H | O |
| I-1-198 | 2,4-Dichlorphenyl | 2,6-Difluorphenyl | H | H | H | O |
| I-1-199 | 2,4-Dichlorphenyl | 2-Bromphenyl | H | H | H | O |
| I-1-200 | 2,4-Dichlorphenyl | 2-Chlorphenyl | H | H | H | O |
| I-1-201 | 2,4-Difluorphenyl | 2-(Trifluormethyl)phenyl | H | Ethyl | H | O |
| I-1-202 | 2,4-Difluorphenyl | 2-(Trifluormethyl)phenyl | H | Methyl | H | O |
| I-1-203 | 2,4-Difluorphenyl | 2-(Trifluormethyl)pyridin-3-yl | H | Methyl | H | O |
| I-1-204 | 2,4-Difluorphenyl | 2-(Trifluormethyl)pyridin-3-yl | H | Ethy | H | O |
| I-1-205 | 2,4-Difluorphenyl | 2,4-Difluorphenyl | H | H | H | O |
| I-1-206 | 2,4-Difluorphenyl | 2,6-Difluorphenyl | H | Methyl | H | O |
| I-1-207 | 2,4-Difluorphenyl | 2,6-Difluorphenyl | H | Ethyl | H | O |
| I-1-208 | 2,4-Difluorphenyl | 2-Bromphenyl | H | Methyl | H | O |
| I-1-209 | 2,4-Difluorphenyl | 2-Bromphenyl | H | H | H | O |
| I-1-210 | 2,4-Difluorphenyl | 2-Brompyridin-3-yl | H | Methyl | H | O |
| I-1-211 | 2,4-Difluorphenyl | 2-Chlorphenyl | H | H | H | O |
| I-1-212 | 2,4-Difluorphenyl | 2-Chlorphenyl | H | Methyl | H | O |
| I-1-213 | 2,4-Difluorphenyl | 2-Chlorphenyl | H | Ethyl | H | O |
| I-1-214 | 2,4-Difluorphenyl | 2-Iodphenyl | H | Methyl | H | O |
| I-1-215 | 2,4-Difluorphenyl | 2-Iodphenyl | H | Ethyl | H | O |
| I-1-216 | 2,4-Difluorphenyl | 3-Methyl-2-thienyl | H | Ethyl | H | O |
| I-1-217 | 2,5-Difluor-4-methylpyridin-3-yl | 2-(Trifluormethyl)phenyl | H | H | H | O |
| I-1-218 | 2,5-Difluorphenyl | 2-Chlorpyridin-3-yl | H | H | H | O |
| I-1-219 | 2,5-Difluorphenyl | 2-Iod-3-thienyl | H | H | H | O |
| I-1-220 | 2,5-Difluorphenyl | 3-Chlorpyridin-2-yl | H | H | H | O |
| I-1-221 | 2,5-Difluorphenyl | 3-Iod-2-thienyl | H | H | H | O |
| I-1-222 | 2,5-Difluorphenyl | 3-Iodfuran-2-yl | H | H | H | O |
| I-1-223 | 2,5-Difluorphenyl | 5-Fluor-1-methyl-3-(difluormethyl)-1H-pyrazol-4-yl | H | H | H | O |
| I-1-224 | 2,5-Difluorpyridin-3-yl | 2-(Trifluormethyl)phenyl | H | H | H | O |
| I-1-225 | 2,5-Difluorpyridin-3-yl | 2-Chlorphenyl | H | H | H | O |
| I-1-226 | 2,6-Dichlorphenyl | 2-(Trifluormethoxy)pyridin-3-yl | H | H | H | O |
| I-1-227 Synthesebeispiel 41 | 2,6-Difluor-4-aminophenyl | 2-(Trifluormethyl)phenyl | H | H | H | O |
| I-1-228 Synthesebeispiel 28 | 2,6-Difluor-4-cyanphenyl | 2-(Trifluormethyl)phenyl | H | H | H | O |
| I-1-229 Synthesebeispiel 26 | 2,6-Difluor-4-nitrophenyl | 2-(Trifluormethyl)phenyl | H | H | H | O |
| I-1-230 Synthesebeispiel 27 | 2,6-Difluor-4-trifluormethyl)phenyl | 2-(Trifluormethyl)phenyl | H | H | H | O |
| I-1-231 | 2,6-Difluorphenyl | 1-(Difluormethyl)-4-fluor-3-methyl-1H-pyrazol-5-yl | H | H | H | O |
| I-1-232 | 2,6-Difluorphenyl | 1,3-Dimethyl-1H-pyrazol-5-yl | H | H | H | O |
| I-1-233 | 2,6-Difluorphenyl | 1,3-Dimethyl-4-nitro-1H-pyrazol-5-yl | H | H | H | O |
| I-1-234 | 2,6-Difluorphenyl | 1,5-Dimethyl-4-nitro-1H-pyrazol-3-yl | H | H | H | O |
| I-1-235 | 2,6-Difluorphenyl | 1-Ethyl-4-iod-1H-pyrazol-5-yl | H | H | H | O |
| I-1-236 | 2,6-Difluorphenyl | 1-Methyl-1H-1,2,4-triazol-5-yl | H | H | H | O |
| I-1-237 | 2,6-Difluorphenyl | 1-Methyl-3-(pentafluorethyl)-4-(trifluormethyl)-1H-pyrazol-5-yl | H | H | H | O |
| I-1-238 | 2,6-Difluorphenyl | 1-Methyl-3,5-bis(trifluormethyl)-1H-pyrazol-4-yl | H | H | H | O |
| I-1-239 | 2,6-Difluorphenyl | 1-Methyl-3-nitro-1H-pyrrol-2-yl | H | H | H | O |
| I-1-240 | 2,6-Difluorphenyl | 1-Methyl-4-(trifluormethyl)-1H-pyrrol-3-yl | H | H | H | O |
| I-1-241 | 2,6-Difluorphenyl | 1-Methyl-4-nitro-1H-pyrazol-3-yl | H | H | H | O |
| I-1-242 | 2,6-Difluorphenyl | 1-Methyl-5-nitro-1H-imidazol-4-yl | H | H | H | O |
| I-1-243 | 2,6-Difluorphenyl | 1-Oxido-2-(trifluormethyl)pyridin-3-yl | H | H | H | O |
| I-1-244 | 2,6-Difluorphenyl | 1-sec-Butyl-4-chlor-1H-pyrazol-5-yl | H | H | H | O |
| I-1-245 | 2,6-Difluorphenyl | 1-sec-Butyl-4-iod-1H-pyrazol-5-yl | H | H | H | O |
| I-1-246 | 2,6-Difluorphenyl | 2-(2,2,2-Trifluorethyl)pyridin-3-yl | H | H | H | O |
| I-1-247 | 2,6-Difluorphenyl | 2-(Difluormethoxy)phenyl | H | H | H | O |
| I-1-248 | 2,6-Difluorphenyl | 2-(Difluormethyl)pyridin-3-yl | H | H | H | O |
| I-1-249 | 2,6-Difluorphenyl | 2-(Methylsulfanyl)pyridin-3-yl | H | H | H | O |
| I-1-250 | 2,6-Difluorphenyl | 2-(Trifluormethoxy)pyridin-3-yl | H | H | H | O |
| I-1-251 | 2,6-Difluorphenyl | 2-(Trifluormethyl)phenyl | Cl | H | H | O |
| I-1-252 | 2,6-Difluorphenyl | 2-(Trifluormethyl)phenyl | Br | H | H | O |
| I-1-253 | 2,6-Difluorphenyl | 2-(Trifluormethyl)phenyl | H | H | Methyl | O |
| I-1-254 | 2,6-Difluorphenyl | 2-(Trifluormethyl)phenyl | H | H | Ethyl | O |
| I-1-255 | 2,6-Difluorphenyl | 2-(Trifluormethyl)phenyl | H | H | Allyl | O |
| I-1-256 | 2,6-Difluorphenyl | 2-(Trifluormethyl)phenyl | H | H | 1-Propin-2-yl | O |
| I-1-257 | 2,6-Difluorphenyl | 2-(Trifluormethyl)phenyl | I | H | H | O |
| I-1-258 | 2,6-Difluorphenyl | 2-(Trifluormethyl)phenyl | H | H | Cyclopropyl methyl | O |
| I-1-259 | 2,6-Difluorphenyl | 2-(Trifluormethyl)phenyl | H | H | Cyanmethyl | O |
| I-1-260 | 2,6-Difluorphenyl | 2-(Trifluormethyl)phenyl | H | H | 2-Propyl | O |
| I-1-261 | 2,6-Difluorphenyl | 2-(Trifluormethyl)phenyl | H | H | 1-Propyl | O |
| I-1-262 | 2,6-Difluorphenyl | 2-(Trifluormethyl)phenyl | H | H | Ethoxymeth yl | O |
| I-1-263 | 2,6-Difluorphenyl | 2-(Trifluormethyl)phenyl | H | H | But-2-in-1-yl | O |
| I-1-264 | 2,6-Difluorphenyl | 2-(Trifluormethyl)phenyl | H | H | 2,2-Difluorethyl | O |
| I-1-265 | 2,6-Difluorphenyl | 2-(Trifluormethyl)phenyl | H | H | 2,2,2-Trifluorethyl | O |
| I-1-266 | 2,6-Difluorphenyl | 2,3-Dichlorphenyl | H | H | H | O |
| I-1-267 | 2,6-Difluorphenyl | 2,6-Dichlorphenyl | H | H | H | O |
| I-1-268 | 2,6-Difluorphenyl | 2,6-Dimethylphenyl | H | H | H | O |
| I-1-269 | 2,6-Difluorphenyl | 2-Acetamidophenyl | H | H | H | O |
| I-1-270 | 2,6-Difluorphenyl | 2-Acetylphenyl | H | H | H | O |
| I-1-271 | 2,6-Difluorphenyl | 2-Chlor-6-methoxyphenyl | H | H | H | O |
| I-1-272 | 2,6-Difluorphenyl | 2-Cyanphenyl | H | H | H | O |
| I-1-273 | 2,6-Difluorphenyl | 2-Cyclopropylphenyl | H | H | H | O |
| I-1-274 | 2,6-Difluorphenyl | 2-Ethyl-6-fluorphenyl | H | H | H | O |
| I-1-275 | 2,6-Difluorphenyl | 2-Ethylpyridin-3-yl | H | H | H | O |
| I-1-276 | 2,6-Difluorphenyl | 2-Fluor-6-(trifluormethyl)phenyl | H | H | H | O |
| I-1-277 | 2,6-Difluorphenyl | 2-Fluor-6-iodphenyl | H | H | H | O |
| I-1-278 | 2,6-Difluorphenyl | 2-Fluorphenyl | H | H | H | O |
| I-1-279 | 2,6-Difluorphenyl | 2-Fluorpyridin-3-yl | H | H | H | O |
| I-1-280 | 2,6-Difluorphenyl | 2-Methoxyphenyl | H | H | H | O |
| I-1-281 | 2,6-Difluorphenyl | 2-Methyl-3-furanyl | H | H | H | O |
| I-1-282 | 2,6-Difluorphenyl | 3-(Difluormethyl)-2-thienyl | H | H | H | O |
| I-1-283 | 2,6-Difluorphenyl | 3-(Difluormethyl)-5-fluor-1-methylpyrazol-4-yl | H | H | H | O |
| I-1-284 | 2,6-Difluorphenyl | 3-(Trifluormethyl)-2-thienyl | H | H | H | O |
| I-1-285 | 2,6-Difluorphenyl | 3-Ethylpyrazin-2-yl | H | H | H | O |
| I-1-286 | 3-Chlor-5-fluorpyridin-2-yl | 2,6-Difluorphenyl | H | H | H | O |
| I-1-287 | 2,6-Difluorphenyl | 3-Fluor-2-(Trifluormethyl)phenyl | H | H | H | O |
| I-1-288 | 2,6-Difluorphenyl | 3-lod-2-furanyl | H | H | H | O |
| I-1-289 | 2,6-Difluorphenyl | 3-Isobutyl-1-methyl-4-nitro-1H-pyrazol-5-yl | H | H | H | O |
| I-1-290 | 2,6-Difluorphenyl | 3-Isopropyl-1-methyl-4-nitro-1H-pyrazol-5-yl | H | H | H | O |
| I-1-291 | 2,6-Difluorphenyl | 3-Methyl-5-(trifluormethyl)-1,2-oxazol-4-yl | H | H | H | O |
| I-1-292 | 2,6-Difluorphenyl | 3-tert-Butyl-1-methyl-4-nitro-1H-pyrazol-5-yl | H | H | H | O |
| I-1-293 | 2,6-Difluorphenyl | 3-tert-Butyl-4-chlor-1-methyl-1H-pyrazol-5-yl | H | H | H | O |
| I-1-294 | 2,6-Difluorphenyl | 4-(Difluormethyl)-1,3-dimethyl-1H-pyrazol-5-yl | H | H | H | O |
| I-1-295 | 2,6-Difluorphenyl | 4-(Trifluormethyl)pyrimidin-5-yl | H | H | H | O |
| I-1-296 | 2,6-Difluorphenyl | 4,5,6-Trifluor-2-(trifluormethyl)phenyl | H | H | H | O |
| I-1-297 | 2,6-Difluorphenyl | 4,5-Dimethyl-1,2-oxazol-3-yl | H | H | H | O |
| I-1-298 | 2,6-Difluorphenyl | 4-Brom-1,3-dimethy)-1H-pyrazol-5-yl | H | H | H | O |
| I-1-299 | 2,6-Difluorphenyl | 4-Brom-1-ethyl-1H-pyrazol-5-yl | H | H | H | O |
| I-1-300 | 2,6-Difluorphenyl | 4-Brom-1-ethyl-3-methyl-1H-pyrazol-5-yl | H | H | H | O |
| I-1-301 | 2,6-Difluorphenyl | 4-Brom-1-methyl-1H-pyrazol-3-yl | H | H | H | O |
| I-1-302 | 2,6-Difluorphenyl | 4-Brom-1-methyl-1H-pyrazol-5-yl | H | H | H | O |
| I-1-303 | 2,6-Difluorphenyl | 4-Brom-1-methyl-3-(trifluormethyl)-1H-pyrazol-5-yl | H | H | H | O |
| I-1-304 | 2,6-Difluorphenyl | 4-Brom-2,5-dimethyl-3-furanyl | H | H | H | O |
| I-1-305 | 2,6-Difluorphenyl | 4-Bromthiophen-3-yl | H | H | H | O |
| I-1-306 | 2,6-Difluorphenyl | 4-Chlor-1-(difluormethyl)-3-methyl-1H-pyrazol-5-yl | H | H | H | O |
| I-1-307 | 2,6-Difluorphenyl | 4-Chlor-1,3-dimethyl-1H-pyrazol-5-yl | H | H | H | O |
| I-1-308 | 2,6-Difluorphenyl | 4-Chlor-1-ethyl-1H-pyrazol-5-yl | H | H | H | O |
| I-1-309 | 2,6-Difluorphenyl | 4-Chlor-1-ethyl-3-methyl-1H-pyrazol-5-yl | H | H | H | O |
| I-1-310 | 2,6-Difluorphenyl | 4-Chlor-1-isopropyl-3-nitro-1H-pyrazol-5-yl | H | H | H | O |
| I-1-311 | 2,6-Difluorphenyl | 4-Chlor-1-methyl-1H-pyrazol-5-yl | H | H | H | O |
| I-1-312 | 2,6-Difluorphenyl | 4-Chlor-1-methyl-3-nitro-1H-pyrazol-5-yl | H | H | H | O |
| I-1-313 | 2,6-Difluorphenyl | 4-Chlor-1-methyl-3-propyl-1H-pyrazol-5-yl | H | H | H | O |
| I-1-314 | 2,6-Difluorphenyl | 4-Chlor-1-methyl-5-propyl-1H-pyrazol-3-yl | H | H | H | O |
| I-1-315 | 2,6-Difluorphenyl | 4-Chlor-1-propyl-1H-pyrazol-3-yl | H | H | H | O |
| I-1-316 | 2,6-Difluorphenyl | 4-Chlor-3-ethyl-1-methyl-1H-pyrazol-5-yl | H | H | H | O |
| I-1-317 | 2,6-Difluorphenyl | 4-Cyan-1-methyl-3-(pentafluorethyl)-1H-pyrazol-5-yl | H | H | H | O |
| I-1-318 | 2,6-Difluorphenyl | 4-Cyan-1-methyl-3-(trifluormethyl)-1H-pyrazol-5-yl | H | H | H | O |
| I-1-319 | 2,6-Difluorphenyl | 4-Cyclopropyl-1,2,3-thiadiazol-5-yl | H | H | H | O |
| I-1-320 | 2,6-Difluorphenyl | 4-Fluor-1,3-dimethyl-1H-pyrazol-5-yl | H | H | H | O |
| I-1-321 | 2,6-Difluorphenyl | 4-Fluor-1-methyl-1H-pyrazol-5-yl | H | H | H | O |
| I-1-322 | 2,6-Difluorphenyl | 4-Fluor-2-(Trifluormethyl)phenyl | H | H | H | O |
| I-1-323 | 2,6-Difluorphenyl | 4-Iod-1-isobutyl-1H-pyrazol-5-yl | H | H | H | O |
| I-1-324 | 2,6-Difluorphenyl | 4-Iod-1-methyl-1H-pyrazol-5-yl | H | H | H | O |
| I-1-325 | 2,6-Difluorphenyl | 4-Iod-1-propyl-1H-pyrazol-5-yl | H | H | H | O |
| I-1-326 | 2,6-Difluorphenyl | 4-Methyl-1,2,3-thiadiazol-5-yl | H | H | H | O |
| I-1-327 | 2,6-Difluorphenyl | 4-Methyl-1,2-oxazol-5-yl | H | H | H | O |
| I-1-328 | 2,6-Difluorphenyl | 4-Methyl-4H-1,2,4-triazol-3-yl | H | H | H | O |
| I-1-329 | 2,6-Difluorphenyl | 5-Chlor-2-(Trifluormethyl)phenyl | H | H | H | O |
| I-1-330 | 2,6-Difluorphenyl | 5-Chlorpyrimidin-4-yl | H | H | H | O |
| I-1-331 | 2,6-Difluorphenyl | 5-Fluor-2-(Trifluormethyl)phenyl | H | H | H | O |
| I-1-332 | 2,6-Difluorphenyl | 5-Methyl-1,2,3-thiadiazol-4-yl | H | H | H | O |
| I-1-333 | 2,6-Difluorphenyl | 6-Methyl-3,4-dihydro-2H-pyran-5-yl | H | H | H | O |
| I-1-334 | 2-Bromphenyl | 2,6-Difluorphenyl | H | H | H | O |
| I-1-335 | 2-Bromphenyl | 2-Fluor-6-(trifluormethyl)phenyl | H | H | H | O |
| I-1-336 | 2-Bromphenyl | 3-Methylpyridin-2-yl | H | H | H | O |
| I-1-337 | 2-Chlor-3-cyanpyridin-4-yl Synthesebeispiel 37 | 2-(Trifluormethyl)phenyl | H | H | H | O |
| I-1-338 | 2-Chlor-4-methylphenyl | 2-Bromphenyl | H | H | H | O |
| I-1-339 | 2-Chlor-4-methylphenyl | 2-Chlorphenyl | H | H | H | O |
| I-1-340 | 2-Chlor-6-ethoxyphenyl | 2-(Trifluormethyl)phenyl | H | H | H | O |
| I-1-341 | 2-Chlorphenyl | 2-(Methylsulfonyl)phenyl | H | H | H | O |
| I-1-342 | 2-Chlorphenyl | 2-(Methylsulfonyl)phenyl | H | Methyl | H | O |
| I-1-343 | 2-Chlorphenyl | 2-(Trifluormethoxy)phenyl | H | Methyl | H | O |
| I-1-344 | 2-Chlorphenyl | 2-(Trifluormethoxy)pyridin-3-yl | H | H | H | O |
| I-1-345 | 2-Chlorphenyl | 2-(Trifluormethyl)phenyl | Met hyl | H | H | O |
| I-1-346 | 2-Chlorphenyl | 2-(Trifluormethyl)phenyl | Cya n | H | H | O |
| I-1-347 | 2-Chlorphenyl | 2-(Trifluormethyl)phenyl | H | Methyl | H | O |
| I-1-348 | 2-Chlorphenyl | 2-(Trifluormethyl)pyridin-3-yl | Met hyl | H | H | O |
| I-1-349 | 2-Chlorphenyl | 2-(Trifluormethyl)pyridin-3-yl | H | Methyl | H | O |
| I-1-350 | 2-Chlorphenyl | 2,3-Dichlorphenyl | H | H | H | O |
| I-1-351 | 2-Chlorphenyl | 2,6-Dichlorphenyl | H | H | H | O |
| I-1-352 | 2-Chlorphenyl | 2,6-Difluorphenyl | Met hyl | H | H | O |
| I-1-353 | 2-Chlorphenyl | 2,6-Dimethylphenyl | H | H | H | O |
| I-1-354 | 2-Chlorphenyl | 2-Bromphenyl | Met hyl | H | H | O |
| I-1-355 | 2-Chlorphenyl | 2-Bromphenyl | H | Amino | H | O |
| I-1-356 | 2-Chlorphenyl | 2-Bromphenyl | H | Methyl | H | O |
| I-1-357 | 2-Chlorphenyl | 2-Brompyridin-3-yl | Met hyl | H | H | O |
| I-1-358 | 2-Chlorphenyl | 2-Brompyridin-3-yl | H | H | H | O |
| I-1-359 | 2-Chlorphenyl | 2-Brompyridin-3-yl | H | Methyl | H | O |
| I-1-360 | 2-Chlorphenyl | 2-Chlorphenyl | Met hyl | H | H | O |
| I-1-361 | 2-Chlorphenyl | 2-Chlorphenyl | H | Amino | H | O |
| I-1-362 | 2-Chlorphenyl | 2-Chlorphenyl | Cya n | H | H | O |
| I-1-363 | 2-Chlorphenyl | 2-Chlorpyrazin-3-yl | H | H | H | O |
| I-1-364 | 2-Chlorphenyl | 2-Fluorpyridin-3-yl | H | H | H | O |
| I-1-365 | 2-Chlorphenyl | 2-Iodphenyl | Met hyl | H | H | O |
| I-1-366 | 2-Chlorphenyl | 2-Iodphenyl | Br | H | H | O |
| I-1-367 | 2-Chlorphenyl | 2-Iodphenyl | I | H | H | O |
| I-1-368 | 2-Chlorphenyl | 2-Iodphenyl | Cya n | H | H | O |
| I-1-369 | 2-Chlorphenyl | 2-Iodpyridin-3-yl | H | H | H | O |
| I-1-370 | 2-Chlorphenyl | 2-Methyl-3-furanyl | H | H | H | O |
| I-1-371 | 2-Chlorphenyl | 2-Methyl-4-(trifluormethyl)-1,3-thiazol-5-yl | H | H | H | O |
| I-1-372 | 2-Chlorphenyl | 2-Methyl-5,6-dihydro-1,4-oxathiin-3-yl | H | H | H | O |
| I-1-373 | 2-Chlorphenyl | 2-*tert*-Butylphenyl | H | H | H | O |
| I-1-374 | 2-Chlorphenyl | 3-(Difluormethyl)-2-thienyl | H | H | H | O |
| I-1-375 | 2-Chlorphenyl | 3-(Trifluormethyl)-2-thienyl | H | H | H | O |
| I-1-376 | 2-Chlorphenyl | 3-(Trifluormethyl)pyridin-2-yl | H | Methyl | H | O |
| I-1-377 | 2-Chlorphenyl | 3-Chlor-2-thienyl | H | H | H | O |
| I-1-378 | 2-Chlorphenyl | 3-Iod-2-furanyl | H | H | H | O |
| I-1-379 | 2-Chlorphenyl | 3-Iod-2-thienyl | H | H | H | O |
| I-1-380 | 2-Chlorphenyl | 4-(Trifluormethyl)pyridin-3-yl | H | Methyl | H | O |
| I-1-381 | 2-Chlorphenyl | 4-Chlorpyridin-3-yl | H | H | H | O |
| I-1-382 | 2-Chlorphenyl | 5-Fluor-1,3-dimethyl-1H-pyrazol-4-yl | H | H | H | O |
| I-1-383 | 2-Chlorphenyl | 5-Fluor-1-methyl-3-(trifluormethyl)-1H-pyrazol-4-yl | H | H | H | O |
| I-1-384 | 2-Chlorphenyl | 6-Fluor-2-(Trifluormethyl)phenyl | H | H | H | O |
| I-1-385 | 2-Chlorpyrazin-5-yl | 2-(Trifluormethyl)phenyl | H | H | H | O |
| I-1-386 | 2-Chlorpyrazin-5-yl | 2-(Trifluormethyl)pyridin-3-yl | H | H | H | O |
| I-1-387 | 2-Chlorpyrazin-5-yl | 2,6-Difluorphenyl | H | H | H | O |
| I-1-388 | 2-Chlorpyrazin-5-yl | 2-Bromphenyl | H | H | H | O |
| I-1-389 | 2-Chlorpyrazin-5-yl | 2-Chlorphenyl | H | H | H | O |
| I-1-390 | 2-Chlorpyrazin-5-yl | 2-Iodphenyl | H | H | H | O |
| I-1-391 | 2-Chlorpyridin-3-yl | 2-(Trifluormethyl)phenyl | H | H | H | O |
| I-1-392 | 2-Chlorpyridin-3-yl | 2-Chlorphenyl | H | H | H | O |
| I-1-393 Synthesebeispiel 29 | 2-Cyan-4,6-difluorphenyl | 2-(Trifluormethyl)phenyl | H | H | H | O |
| I-1-394 | 2-Cyanphenyl | 2-Iodphenyl | H | H | H | O |
| I-1-395 | 2-Ethoxy-3-chlorphenyl | 2-(Trifluormethyl)pyridin-3-yl | H | H | H | O |
| I-1-396 | 2-Ethoxy-3-fluorphenyl | 2-Bromphenyl | H | H | H | O |
| I-1-397 | 2-Ethoxy-3-fluorphenyl | 2-Chlorphenyl | H | H | H | O |
| I-1-398 | 2-Chlor-6-ethoxyphenyl | 2-Bromphenyl | H | H | H | O |
| I-1-399 | 2-Ethoxy-6-fluorphenyl | 2-Bromphenyl | H | H | H | O |
| I-1-400 | 2-Fluorphenyl | 2,5-Dimethylfuran-3-yl | H | H | H | O |
| I-1-401 | 2-Fluorpyrazin-6-yl | 2-(Trifluormethyl)phenyl | H | H | H | O |
| I-1-402 | 2-Fluorpyrazin-6-yl | 2-(Trifluormethyl)pyridin-3-yl | H | H | H | O |
| I-1-403 | 2-Fluorpyrazin-6-yl | 2,6-Difluorphenyl | H | H | H | O |
| I-1-404 | 2-Fluorpyrazin-6-yl | 2-Brompyridin-3-yl | H | H | H | O |
| I-1-405 | 2-Fluorpyrazin-6-yl | 2-Iodphenyl | H | H | H | O |
| I-1-406 | 2-Methoxyphenyl | 2-(Trifluormethyl)phenyl | H | H | H | O |
| I-1-407 | 2-Methoxyphenyl | 2-(Trifluormethyl)pyridin-3-yl | H | H | H | O |
| I-1-408 | 2-Methoxyphenyl | 2,6-Difluorphenyl | H | H | H | O |
| I-1-409 | 2-Methoxyphenyl | 2-Brompyridin-3-yl | H | H | H | O |
| I-1-410 | 2-Methoxyphenyl | 2-Chlorphenyl | H | H | H | O |
| I-1-411 | 2-Methoxyphenyl | 2-Iodphenyl | H | H | H | O |
| I-1-412 | 2-Methoxypyrazin-3-yl | 2,5-Dimethylfuran-3-yl | H | H | H | O |
| I-1-413 | 2-Methoxypyrazin-3-yl | 2-Bromphenyl | H | H | H | O |
| I-1-414 | 2-Methylphenyl | 2,5-Dimethylfuran-3-yl | H | H | H | O |
| I-1-415 | 2-Nitrophenyl | 2-(Trifluormethyl)phenyl | H | H | H | O |
| I-1-416 | 2-Nitrophenyl | 2-(Trifluormethyl)pyridin-3-yl | H | H | H | O |
| I-1-417 | 2-Nitrophenyl | 2,6-Difluorphenyl | H | H | H | O |
| I-1-418 | 2-Nitrophenyl | 2-Bromphenyl | H | H | H | O |
| I-1-419 | 2-Nitrophenyl | 2-Iodphenyl | H | H | H | O |
| I-1-420 | 2-Pyridyl | 2-Chlorphenyl | H | H | H | O |
| I-1-421 | 3-Chlor-5-(trifluormethyl)pyridin-2-yl | 2-Iodphenyl | H | H | H | O |
| I-1-422 | 3-(Trifluormethyl)pyridin -2-yl | 2-(Trifluormethyl)phenyl | H | H | H | O |
| I-1-423 | 3-(Trifluormethyl)pyridin-2-yl | 2,6-Difluorphenyl | H | H | H | O |
| I-1-424 | 3-(Trifluormethyl)pyridin -2-yl | 2-Bromphenyl | H | H | H | O |
| I-1-425 | 3-(Trifluormethyl)pyridin -2-yl | 2-Chlorphenyl | H | H | H | O |
| I-1-426 | 3-(Trifluormethyl)pyridin -2-yl | 2-(Trifluormethyl)pyridin-3-yl | H | H | H | O |
| I-1-427 | 3-(Trifluormethyl)pyridin -2-yl | 2-lodphenyl | H | H | H | O |
| I-1-428 | 3,5-Dichlorpyridin-2-yl | 2-(Trifluormethyl)phenyl | H | H | H | O |
| I-1-429 | 3,5-Dichlorpyridin-2-yl | 2,6-Difluorphenyl | H | H | H | O |
| I-1-430 | 3,5-Dichlorpyridin-2-yl | 2-Bromphenyl | H | H | H | O |
| I-1-431 | 3,5-Dichlorpyridin-2-yl | 2-Brompyridin-3-yl | H | H | H | O |
| I-1-432 | 3,5-Dichlorpyridin-2-yl | 2-Chlorphenyl | H | H | H | O |
| I-1-433 | 3,5-Dichlorpyridin-2-yl | 2-Iodphenyl | H | H | H | O |
| I-1-434 | 3,5-Dichlorpyridin-2-yl | 3-Fluor-2-(Trifluormethyl)phenyl | H | H | H | O |
| I-1-435 | 3,5-Dichlorpyridin-2-yl | 3-Iod-1-methyl-1H-pyrazol-4-yl | H | H | H | O |
| I-1-436 | 3,5-Dichlorpyridin-2-yl | 4-Fluor-2-(Trifluormethyl)phenyl | H | H | H | O |
| I-1-437 | 3,5-Dichlorpyridin-2-yl | 5-Chlor-2-(Trifluormethyl)phenyl | H | H | H | O |
| I-1-438 | 3,5-Difluor-1-oxidopyridin-2-yl Synthesebeispiel 48 | 2-(Trifluormethyl)phenyl | H | H | H | O |
| I-1-439 | 3,5-difluor-6-methylpyridin-2-yl | 2-(Trifluormethyl)phenyl | H | H | H | O |
| I-1-440 | 3,5-Difluor-6-methylpyridin-2-yl | 2-(Trifluormethyl)pyridin-3-yl | H | H | H | O |
| I-1-441 | 3,5-Difluor-6-methylpyridin-2-yl | 2,6-Difluorphenyl | H | H | H | O |
| I-1-442 | 3,5-Difluor-6-methylpyridin-2-yl | 2-Bromphenyl | H | H | H | O |
| I-1-443 | 3,5-Difluorpyridin-2-yl | 1-(Difluormethyl)-1H-pyrazol-5-yl | H | H | H | O |
| I-1-444 | 3,5-Difluorpyridin-2-yl | 1-(Difluormethyl)-4-fluor-3-methyl-1H-pyrazol-5-yl | H | H | H | O |
| I-1-445 | 3,5-Difluorpyridin-2-yl | 1,3,5-Trimethyl-1H-pyrazol-4-yl | H | H | H | O |
| I-1-446 | 3,5-Difluorpyridin-2-yl | 1,3-Dimethyl-1H-pyrazol-4-yl | H | H | H | O |
| I-1-447 | 3,5-Difluorpyridin-2-yl | 1,3-Dimethyl-4-nitro-1H-pyrazol-5-yl | H | H | H | O |
| I-1-448 | 3,5-Difluorpyridin-2-yl | 1,5-Dimethyl-4-nitro-1H-pyrazol-3-yl | H | H | H | O |
| I-1-449 | 3,5-Difluorpyridin-2-yl | 1-Methyl-1H-1,2,4-triazol-5-yl | H | H | H | O |
| I-1-450 | 3,5-Difluorpyridin-2-yl | 1-Methyl-3-(pentafluorethyl)-4-(trifluormethyl)-1H-pyrazol-5-yl | H | H | H | O |
| I-1-451 | 3,5-Difluorpyridin-2-yl | 1-Methyl-3,5-bis(trifluormethyl)-1H-pyrazol-4-yl | H | H | H | O |
| I-1-452 | 3,5-Difluorpyridin-2-yl | 1-Methyl-3-nitro-1H-pyrrol-2-yl | H | H | H | O |
| I-1-453 | 3,5-Difluorpyridin-2-yl | 1-Methyl-4-nitro-1H-pyrazol-3-yl | H | H | H | O |
| I-1-454 | 3,5-Difluorpyridin-2-yl | 1-Methyl-5-nitro-1H-imidazol-4-yl | H | H | H | O |
| I-1-455 | 3,5-Difluorpyridin-2-yl | 1-sec-Butyl-4-chlor-1H-pyrazol-5-yl | H | H | H | O |
| I-1-456 | 3,5-Difluorpyridin-2-yl | 1-sec-Butyl-4-iod-1H-pyrazol-5-yl | H | H | H | O |
| I-1-457 | 3,5-Difluorpyridin-2-yl | 2-(2,2,2-Trifluorethyl)pyridin-3-yl | H | H | H | O |
| I-1-458 | 3,5-Difluorpyridin-2-yl | 2-(Difluormethoxy)phenyl | H | H | H | O |
| I-1-459 | 3,5-Difluorpyridin-2-yl | 2-(Difluormethyl)phenyl | H | H | H | O |
| I-1-460 | 3,5-Difluorpyridin-2-yl | 2-(Difluormethyl)pyridin-3-yl | H | H | H | O |
| I-1-461 | 3,5-Difluorpyridin-2-yl | 2-(Methylsulfanyl)pyridin-3-yl | H | H | H | O |
| I-1-462 | 3,5-Difluorpyridin-2-yl | 2-(Methylsulfonyl)phenyl | H | H | H | O |
| I-1-463 | 3,5-Difluorpyridin-2-yl | 2-(Trifluormethoxy)phenyl | H | H | H | O |
| I-1-464 | 3,5-Difluorpyridin-2-yl | 2-(Trifluormethoxy)pyridin-3-yl | H | H | H | O |
| I-1-465 | 3,5-Difluorpyridin-2-yl | 2-(Trifluormethyl)phenyl | F | H | H | O |
| I-1-466 | 3,5-Difluorpyridin-2-yl | 2-(Trifluormethyl)phenyl | H | H | H | S |
| I-1-467 | 3,5-Difluorpyridin-2-yl | 2-(Trifluormethyl)phenyl | H | H | Methyl | O |
| I-1-468 | 3,5-Difluorpyridin-2-yl | 2-(Trifluormethyl)phenyl | H | H | Ethyl | O |
| I-1-469 | 3,5-Difluorpyridin-2-yl | 2-(Trifluormethyl)phenyl | H | H | Allyl | O |
| I-1-470 | 3,5-Difluorpyridin-2-yl | 2-(Trifluormethyl)phenyl | H | H | 1-Propin-2-yl | O |
| I-1-471 | 3,5-Difluorpyridin-2-yl | 2-(Trifluormethyl)phenyl | H | H | Cyclopropylmethyl | O |
| I-1-472 | 3,5-Difluorpyridin-2-yl | 2-(Trifluormethyl)phenyl | H | H | Cyanmethyl | O |
| I-1-473 | 3,5-Difluorpyridin-2-yl | 2-(Trifluormethyl)phenyl | Cl | H | H | O |
| I-1-474 | 3,5-Difluorpyridin-2-yl | 2-(Trifluormethyl)phenyl | Br | H | H | O |
| I-1-475 | 3,5-Difluorpyridin-2-yl | 2-(Trifluormethyl)phenyl | Nitr o | H | H | O |
| I-1-476 | 3,5-Difluorpyridin-2-yl | 2,3-Dichlorphenyl | H | H | H | O |
| I-1-477 | 3,5-Difluorpyridin-2-yl | 2,3-Dichlorphenyl | H | H | 2,3-Dichlorphenylcarbonyl | O |
| I-1-478 | 3,5-Difluorpyridin-2-yl | 2,4-Dimethyl-1,3-thiazol-5-yl | H | H | H | O |
| I-1-479 | 3,5-Difluorpyridin-2-yl | 2,6-Dichlorphenyl | H | H | H | O |
| I-1-480 | 3,5-Difluorpyridin-2-yl | 2,6-Dimethylphenyl | H | H | H | O |
| I-1-481 | 3,5-Difluorpyridin-2-yl | 2-Acetylphenyl | H | H | H | O |
| I-1-482 | 3,5-Difluorpyridin-2-yl | 2-Brompyridin-3-yl | H | H | H | O |
| I-1-483 | 3,5-Difluorpyridin-2-yl | 2-Chlor-5-methoxyphenyl | H | H | H | O |
| I-1-484 | 3,5-Difluorpyridin-2-yl | 2-Chlor-6-fluorphenyl | H | H | H | O |
| I-1-485 | 3,5-Difluorpyridin-2-yl | 2-Chlorpyrazin-3-yl | H | H | H | O |
| I-1-486 | 3,5-Difluorpyridin-2-yl | 2-Cyclopropylphenyl | H | H | H | O |
| I-1-487 | 3,5-Difluorpyridin-2-yl | 2-Ethyl-6-fluorphenyl | H | H | H | O |
| I-1-488 | 3,5-Difluorpyridin-2-yl | 2-Ethylphenyl | H | H | H | O |
| I-1-489 | 3,5-Difluorpyridin-2-yl | 2-Ethylpyrazin-3-yl | H | H | H | O |
| I-1-490 | 3,5-Difluorpyridin-2-yl | 2-Ethylpyridin-3-yl | H | H | H | O |
| I-1-491 | 3,5-Difluorpyridin-2-yl | 2-Fluor-6-(trifluormethyl)phenyl | H | H | H | O |
| I-1-492 | 3,5-Difluorpyridin-2-yl | 2-Fluor-6-iodphenyl | H | H | H | O |
| I-1-493 | 3,5-Difluorpyridin-2-yl | 2-Fluorphenyl | H | H | H | O |
| I-1-494 | 3,5-Difluorpyridin-2-yl | 2-Iod-3-thienyl | H | H | H | O |
| I-1-495 | 3,5-Difluorpyridin-2-yl | 2-Iodpyridin-3-yl | H | H | H | O |
| I-1-496 | 3,5-Difluorpyridin-2-yl | 2-Methoxyphenyl | H | H | H | O |
| I-1-497 | 3,5-Difluorpyridin-2-yl | 2-Methyl-4-(trifluormethyl)-1,3-thiazol-5-yl | H | H | H | O |
| I-1-498 | 3,5-Difluorpyridin-2-yl | 2-Methyl-5,6-dihydro-1,4-oxathiin-3-yl | H | H | H | O |
| I-1-499 | 3,5-Difluorpyridin-2-yl | 2-Methylphenyl | H | H | H | O |
| I-1-500 | 3,5-Difluorpyridin-2-yl | 3-(Difluormethyl)-5-fluor-1-methylpyrazol-4-yl | H | H | H | O |
| I-1-501 | 3,5-Difluorpyridin-2-yl | 3-(Trifluormethyl)pyrazin-2-yl | H | H | H | O |
| I-1-502 | 3,5-Difluorpyridin-2-yl | 3-(Trifluormethyl)pyridin-2-yl | H | H | H | O |
| I-1-503 | 3,5-Difluorpyridin-2-yl | 3-Chlor-2-thienyl | H | H | H | O |
| I-1-504 | 3,5-Difluorpyridin-2-yl | 3-Chlorpyridin-2-yl | H | H | H | O |
| I-1-505 | 3,5-Difluorpyridin-2-yl | 3-Ethylpyridazin-4-yl | H | H | H | O |
| I-1-506 | 3,5-Difluorpyridin-2-yl | 3-Fluor-6-(trifluormethyl)phenyl | H | H | H | O |
| I-1-507 | 3,5-Difluorpyridin-2-yl | 3-Iod-2-thienyl | H | H | H | O |
| I-1-508 | 3,5-Difluorpyridin-2-yl | 3-Isobutyl-1-methyl-4-nitro-1H-pyrazol-5-yl | H | H | H | O |
| I-1-509 | 3,5-Difluorpyridin-2-yl | 3-Isopropyl-1-methyl-4-nitro-1H-pyrazol-5-yl | H | H | H | O |
| I-1-510 | 3,5-Difluorpyridin-2-yl | 3-Methyl-2-thienyl | H | H | H | O |
| I-1-511 | 3,5-Difluorpyridin-2-yl | 3-Methyl-5-(trifluormethyl)-1,2-oxazol-4-yl | H | H | H | O |
| I-1-512 | 3,5-Difluorpyridin-2-yl | 3-tert-Butyl-1-methyl-4-nitro-1H-pyrazol-5-yl | H | H | H | O |
| I-1-513 | 3,5-Difluorpyridin-2-yl | 3-tert-Butyl-4-chlor-1-methyl-1H-pyrazol-5-yl | H | H | H | O |
| I-1-514 | 3,5-Difluorpyridin-2-yl | 4-(Difluormethyl)-1,3-dimethyl-1H-pyrazol-5-yl | H | H | H | O |
| I-1-515 | 3,5-Difluorpyridin-2-yl | 4-(Trifluormethyl)pyridin-3-yl | H | H | H | O |
| I-1-516 | 3,5-Difluorpyridin-2-yl | 4,5,6-Trifluor-2-(trifluormethyl)phenyl | H | H | H | O |
| I-1-517 | 3,5-Difluorpyridin-2-yl | 4-Brom-1,3-dimethyl-1H-pyrazol-5-yl | H | H | H | O |
| I-1-518 | 3,5-Difluorpyridin-2-yl | 4-Brom-1-ethyl-1H-pyrazol-5-yl | H | H | H | O |
| I-1-519 | 3,5-Difluorpyridin-2-yl | 4-Brom-1-ethyl-3-methyl-1H-pyrazol-5-yl | H | H | H | O |
| I-1-520 | 3,5-Difluorpyridin-2-yl | 4-Brom-1-methyl-1H-pyrazol-3-yl | H | H | H | O |
| I-1-521 | 3,5-Difluorpyridin-2-yl | 4-Brom-1-methyl-1H-pyrazol-5-yl | H | H | H | O |
| I-1-522 | 3,5-Difluorpyridin-2-yl | 4-Brom-1-methyl-3-(trifluormethyl)-1H-pyrazol-5-yl | H | H | H | O |
| I-1-523 | 3,5-Difluorpyridin-2-yl | 4-Brom-2,5-dimethyl-3-furanyl | H | H | H | O |
| I-1-524 | 3,5-Difluorpyridin-2-yl | 4-Bromthiophen-3-yl | H | H | H | O |
| I-1-525 | 3,5-Difluorpyridin-2-yl | 4-Chlor-1-(difluormethyl)-3-methyl-1H-pyrazol-5-yl | H | H | H | O |
| I-1-526 | 3,5-Difluorpyridin-2-yl | 4-Chlor-1-ethyl-1H-pyrazol-5-yl | H | H | H | O |
| I-1-527 | 3,5-Difluorpyridin-2-yl | 4-Chlor-1-ethyl-3-methyl-1H-pyrazol-5-yl | H | H | H | O |
| I-1-528 | 3,5-Difluorpyridin-2-yl | 4-Chlor-1-isopropyl-3-nitro-1H-pyrazol-5-yl | H | H | H | O |
| I-1-529 | 3,5-Difluorpyridin-2-yl | 4-Chlor-1-methyl-1H-pyrazol-5-yl | H | H | H | O |
| I-1-530 | 3,5-Difluorpyridin-2-yl | 4-Chlor-1-methyl-3-nitro-1H-pyrazol-5-yl | H | H | H | O |
| I-1-531 | 3,5-Difluorpyridin-2-yl | 4-Chlor-1-methyl-3-propyl-1H-pyrazol-5-yl | H | H | H | O |
| I-1-532 | 3,5-Difluorpyridin-2-yl | 4-Chlor-1-methyl-5-propyl-1H-pyrazol-3-yl | H | H | H | O |
| I-1-533 | 3,5-Difluorpyridin-2-yl | 4-Chlor-1-propyl-1H-pyrazol-3-yl | H | H | H | O |
| I-1-534 | 3,5-Difluorpyridin-2-yl | 4-Chlor-3-ethyl-1-methyl-1H-pyrazol-5-yl | H | H | H | O |
| I-1-535 | 3,5-Difluorpyridin-2-yl | 4-Cyan-1-methyl-3-(pentafluorethyl)-1H-pyrazol-5-yl | H | H | H | O |
| I-1-536 | 3,5-Difluorpyridin-2-yl | 4-Cyan-1-methyl-3-(trifluormethyl)-1H-pyrazol-5-yl | H | H | H | O |
| I-1-537 | 3,5-Difluorpyridin-2-yl | 4-Cyclopropyl-1,2,3-thiadiazol-5-yl | H | H | H | O |
| I-1-538 | 3,5-Difluorpyridin-2-yl | 4-Fluor-1-methyl-1H-pyrazol-5-yl | H | H | H | O |
| I-1-539 | 3,5-Difluorpyridin-2-yl | 4-Iod-1-isobutyl-1H-pyrazol-5-yl | H | H | H | O |
| I-1-540 | 3,5-Difluorpyridin-2-yl | 4-Iod-1-methyl-1H-pyrazol-5-yl | H | H | H | O |
| I-1-541 | 3,5-Difluorpyridin-2-yl | 4-Iod-1-propyl-1H-pyrazol-5-yl | H | H | H | O |
| I-1-542 | 3,5-Difluorpyridin-2-yl | 4-Methyl-1,2,5-oxadiazol-3-yl | H | H | H | O |
| I-1-543 | 3,5-Difluorpyridin-2-yl | 4-Methyl-1,2-oxazol-5-yl | H | H | H | O |
| I-1-544 | 3,5-Difluorpyridin-2-yl | 4-Methyl-4H-1,2,4-triazol-3-yl | H | H | H | O |
| I-1-545 | 3,5-Difluorpyridin-2-yl | 5-Chlorpyrimidin-4-yl | H | H | H | O |
| I-1-546 | 3,5-Difluorpyridin-2-yl | 5-Methyl-1,2,3-thiadiazol-4-yl | H | H | H | O |
| I-1-547 | 3,5-Difluorpyridin-2-yl | 5-Methyl-1,2-oxazol-4-yl | H | H | H | O |
| I-1-548 | 3,5-Difluorpyridin-2-yl | 6-Methyl-3,4-dihydro-2H-pyran-5-yl | H | H | H | O |
| I-1-549 | 3-Brompyridin-2-yl | 2-(Trifluormethyl)phenyl | H | H | H | O |
| I-1-550 | 3-Brompyridin-2-yl | 2-(Trifluormethyl)pyridin-3-yl | H | H | H | O |
| I-1-551 | 3-Brompyridin-2-yl | 2,6-Difluorphenyl | H | H | H | O |
| I-1-552 | 3-Brompyridin-2-yl | 2-Bromphenyl | H | H | H | O |
| I-1-553 | 3-Brompyridin-2-yl | 2-Chlorphenyl | H | H | H | O |
| I-1-554 | 3-Brompyridin-2-yl | 2-Iodphenyl | H | H | H | O |
| I-1-555 | 3-Chlor-2-fluorphenyl | 2-(Trifluormethyl)phenyl | H | H | H | O |
| I-1-556 | 3-Chlor-2-fluorphenyl | 2,6-Difluorphenyl | H | H | H | O |
| I-1-557 | 3-Chlor-2-fluorphenyl | 2-Bromphenyl | H | H | H | O |
| I-1-558 | 3-Chlor-2-fluorphenyl | 2-Brompyridin-3-yl | H | H | H | O |
| I-1-559 | 3-Chlor-2-fluorphenyl | 2-Chlorphenyl | H | H | H | O |
| I-1-560 | 3-Chlor-5-(trifluormethyl)pyridin-2-yl | 2-(Trifluormethyl)phenyl | H | H | H | O |
| I-1-561 | 3-Chlor-5-(trifluormethyl)pyridin-2-yl | 2-Chlorphenyl | H | H | H | O |
| I-1-562 | 3-Chlorpyridin-2-yl | 2-(Trifluormethyl)pyridin-3-yl | H | H | H | O |
| I-1-563 | 3-Chlorpyridin-2-yl | 2,5-Dimethylfuran-3-yl | H | H | H | O |
| I-1-564 | 3-Chlorpyridin-2-yl | 2,6-Difluorphenyl | H | H | H | O |
| I-1-565 | 3-Chlorpyridin-2-yl | 2-Brompyridin-3-yl | H | H | H | O |
| I-1-566 | 3-Chlorpyridin-2-yl | 2-Iod-3-thienyl | H | H | H | O |
| I-1-567 | 3-Cyan-4,6-dimethylpyridin-2-yl | 2-(Trifluormethyl)phenyl | H | H | H | O |
| I-1-568 Synthesebeispiel 36 | 3-Cyan-6-(diethylamino)pyridin-2-yl | 2-(Trifluormethyl)phenyl | H | H | H | O |
| I-1-569 Synthesebeispiel 31 | 3-Cyan-6-(trifluormethyl)pyridin-2-yl | 2-(Trifluormethyl)phenyl | H | H | H | O |
| I-1-570 Synthesebeispiel 40 | 3-Cyan-6-cyclopropyl-4-(trifluormethyl)pyridin-2-yl | 2-(Trifluormethyl)phenyl | H | H | H | O |
| I-1-571 Synthesebeispiel 33 | 3-Cyan-6-methyl-4-(trifluormethyl)pyridin-2-yl | 2-(Trifluormethyl)phenyl | H | H | H | O |
| I-1-572 | 3-Cyan-6-methylpyridin-2-yl | 2-(Trifluormethyl)phenyl | H | H | H | O |
| I-1-573 Synthesebeispiel 39 | 3-Cyan-6-propylpyridin-2-yl | 2-(Trifluormethyl)phenyl | H | H | H | O |
| I-1-574 Synthesebeispiel 43 | 3-Cyanpyrazin-2-yl | 2-(Trifluormethyl)phenyl | H | H | H | O |
| I-1-575 Synthesebeispiel 25 | 3-Cyanpyridin-2-yl | 2-(Trifluormethyl)phenyl | H | H | H | O |
| I-1-576 | 3-Fluor-4-iodpyridin-2-yl | 2-(Trifluormethyl)phenyl | H | H | H | O |
| I-1-577 | 3-Fluorpyridin-2-yl | 2-(Trifluormethoxy)pyridin-3-yl | H | H | H | O |
| I-1-578 | 3-Fluorpyridin-2-yl | 2-Brompyridin-3-yl | H | H | H | O |
| I-1-579 | 3-Fluorpyridin-2-yl | 2-Chlorpyridin-3-yl | H | H | H | O |
| I-1-580 | 3-Fluorpyridin-2-yl | 2-Fluor-6-(trifluormethyl)phenyl | H | H | H | O |
| I-1-581 | 3-Fluorpyridin-2-yl | 2-Iod-3-thienyl | H | H | H | O |
| I-1-582 | 3-Fluorpyridin-2-yl | 3-Iod-2-furanyl | H | H | H | O |
| I-1-583 Synthesebeispiel 42 | 4-Acetamido-2,6-difluorphenyl | 2-(Trifluormethyl)phenyl | H | H | H | O |
| I-1-584 Synthesebeispiel 30 | 4-Acetyl-2,6-difluorphenyl | 2-(Trifluormethyl)phenyl | H | H | H | O |
| I-1-585 Synthesebeispiel 37 | 2-Chlor-3-cyanpyridin-4-yl | 2-(Trifluormethyl)phenyl | H | H | H | O |
| I-1-586 Synthesebeispiel 38 | 4-Chlor-3-cyanpyridin-2-yl | 2-(Trifluormethyl)phenyl | H | H | H | O |
| I-1-587 | 5-Chlor-3-fluorpyridin-2-yl | 2-(Trifluormethyl)phenyl | H | H | H | O |
| I-1-588 | 5-Chlor-3-fluorpyridin-2-yl | 2,6-Difluorphenyl | H | H | H | O |
| I-1-589 | 5-Chlor-3-fluorpyridin-2-yl | 2-Bromphenyl | H | H | H | O |
| I-1-590 | 5-Chlor-3-fluorpyridin-2-yl | 2-Chlorphenyl | H | H | H | O |
| I-1-591 | 5-Chlor-3-fluorpyridin-2-yl | 2-Fluor-6-(trifluormethyl)phenyl | H | H | H | O |
| I-1-592 | 5-Chlor-3-fluorpyridin-2-yl | 2-Iod-3-thienyl | H | H | H | O |
| I-1-593 | 5-Chlor-3-fluorpyridin-2-yl | 2-Iodphenyl | H | H | H | O |
| I-1-594 | 5-Cyanpyrimidin-4-yl | 2-(Trifluormethyl)phenyl | H | H | H | O |
| I-1-595 | 5-Cyanpyrimidin-4-yl | 2,6-Difluorphenyl | H | H | H | O |
| I-1-596 | 5-Fluorpyridin-2-yl | 2-(Trifluormethyl)phenyl | H | H | H | O |
| I-1-597 | 5-Fluorpyridin-2-yl | 2-(Trifluormethylpyridin-3-yl | H | H | H | O |
| I-1-598 | 5-Fluorpyridin-2-yl | 2,6-Difluorphenyl | H | H | H | O |
| I-1-599 | 5-Fluorpyridin-2-yl | 2-Iodphenyl | H | H | H | O |
| I-1-600 | 5-Fluorpyrimidin-2-yl | 2-(Trifluormethyl)phenyl | H | H | H | O |
| I-1-601 | 5-Fluorpyrimidin-2-yl | 2-(Trifluormethyl)pyridin-3-yl | H | H | H | O |
| I-1-602 | 5-Fluorpyrimidin-2-yl | 2,6-Difluorphenyl | H | H | H | O |
| I-1-603 | 5-Fluorpyrimidin-2-yl | 2-Bromphenyl | H | H | H | O |
| I-1-604 | 5-Fluorpyrimidin-2-yl | 2-Chlorphenyl | H | H | H | O |
| I-1-605 | 5-Fluorpyrimidin-2-yl | 2-Iodphenyl | H | H | H | O |
| I-1-606 Synthesebeispiel 44 | 6-Chlor-5-cyan-3-fluorpyridin-2-yl | 2-(Trifluormethyl)phenyl | H | H | H | O |
| I-1-607 Synthesebeispiel 34 | 6-Chlor-3-cyanpyridin-2-yl | 2-(Trifluormethyl)phenyl | H | H | H | O |
| I-1-608 Synthesebeispiel 35 | 6-Chlor-5-cyanpyridin-2-yl | 2-(Trifluormethyl)phenyl | H | H | H | O |
| I-1-609 | Pyrimidin-2-yl | 2,5-Dimethylfuran-3-yl | H | H | H | O |
| I-1-610 | 2,6-Difluorphenyl | 2-(2,2,2-Trifluorethyl)phenyl | H | H | H | O |
| I-1-611 | 2,6-Difluorphenyl | 2-(Difluormethyl)-5,6-dihydro-1,4-oxathiin-3-yl | H | H | H | O |
| I-1-612 | 2,6-Difluorphenyl | 2-[(Trifluoracetyl)amino]phenyl | H | H | H | O |
| I-1-613 | 2,6-Difluorphenyl | 2-Brom-6-fluorphenyl | H | H | H | O |
| I-1-614 | 2,6-Difluorphenyl | 2-Chlor-6-(trifluormethyl)phenyl | H | H | H | O |
| I-1-615 | 2,6-Difluorphenyl | 3-Methyl-5,6-dihydro-1,4-dioxin-2-yl | H | H | H | O |
| I-1-616 | 2,6-Difluorphenyl | 2-Methyl-5,6-dihydro-4,4-dioxo-1,4-oxathiin-3-yl | H | H | H | O |
| I-1-617 | 2-Cyan-3-fluorphenyl | 2-(Trifluormethyl)phenyl | H | H | H | O |
| I-1-618 | 2-Cyan-3-fluorphenyl | 2-(Trifluormethyl)pyridin-3-yl | H | H | H | O |
| I-1-619 | 2-Cyan-3-fluorphenyl | 2,6-Difluorphenyl | H | H | H | O |
| I-1-620 | 2-Cyan-3-fluorphenyl | 2-Bromphenyl | H | H | H | O |
| I-1-621 | 2-Cyan-3-fluorphenyl | 2-Chlorphenyl | H | H | H | O |
| I-1-622 | 2-Cyan-3-fluorphenyl | 2-Chlorpyridin-3-yl | H | H | H | O |
| I-1-623 | 2-Cyan-3-fluorphenyl | 2-Fluor-6-(trifluormethyl)phenyl | H | H | H | O |
| I-1-624 | 2-Cyan-3-fluorphenyl | 2-Iod-3-thienyl | H | H | H | O |
| I-1-625 | 2-Cyan-3-fluorphenyl | 2-Iodphenyl | H | H | H | O |
| I-1-626 | 2-Cyan-4-fluorphenyl | 2-(Trifluormethyl)phenyl | H | H | H | O |
| I-1-627 | 2-Cyan-4-fluorphenyl | 2-(Trifluormethyl)pyridin-3-yl | H | H | H | O |
| I-1-628 | 2-Cyan-4-fluorphenyl | 2,6-Difluorphenyl | H | H | H | O |
| I-1-629 | 2-Cyan-4-fluorphenyl | 2-Bromphenyl | H | H | H | O |
| I-1-630 | 2-Cyan-4-fluorphenyl | 2-Chlorphenyl | H | H | H | O |
| I-1-631 | 2-Cyan-4-fluorphenyl | 2-Chlorpyridin-3-yl | H | H | H | O |
| I-1-632 | 2-Cyan-4-fluorphenyl | 2-Fluor-6-(trifluormethyl)phenyl | H | H | H | O |
| I-1-633 | 2-Cyan-4-fluorphenyl | 2-Iod-3-thienyl | H | H | H | O |
| I-1-634 | 2-Cyan-4-fluorphenyl | 2-Iodphenyl | H | H | H | O |
| I-1-635 | 3,5-Difluorpyridin-2-yl | 2-(2,2,2-Trifluorethyl)phenyl | H | H | H | O |
| I-1-636 | 3,5-Difluorpyridin-2-yl | 2-(Difluormethyl)-5,6-dihydro-1,4-oxathiin-3-yl | H | H | H | O |
| I-1-637 | 3,5-Difluorpyridin-2-yl | 2-Brom-6-fluorphenyl | H | H | H | O |
| I-1-638 | 3,5-Difluorpyridin-2-yl | 2-Chlor-6-(trifluormethyl)phenyl | H | H | H | O |
| I-1-639 | 3,5-Difluorpyridin-2-yl | 3-Methyl-5,6-dihydro-1,4-dioxin-2-yl | H | H | H | O |
| I-1-640 Synthesebeispiel 23 | 3,5-Difluorpyridin-2-yl | 2-Methyl-5,6-dihydro-4,4-dioxo-1,4-oxathiin-3-yl | H | H | H | O |
| I-1-641 Synthesebeispiel 32 | 3-Cyan-4-(4-fluorphenyl)pyridin-2-yl | 2-(Trifluormethyl)phenyl | H | H | H | O |
| I-1-642 | 3-Fluor-4-(4-fluorphenyl)pyridin-2-yl | 2-(Trifluormethyl)phenyl | H | H | H | O |
| I-1-643 Synthesebeispiel 24 | 4-Amino-2,6-difluorphenyl | 2-Carboxyphenyl | H | H | H | O |
| I-1-644 | 2,6-Difluorphenyl | 1-Methyl-1H-imidazol-5-yl | H | H | H | O |
| I-1-645 | 2,6-Difluorphenyl | 1-Methyl-1H-pyrrol-2-yl | H | H | H | O |
| I-1-646 | 3,5-Difluorpyridin-2-yl | 1-Methyl-1H-imidazol-5-yl | H | H | H | O |
| I-1-647 | 3,5-Difluorpyridin-2-yl | 1-Methyl-1H-pyrrol-2-yl | H | H | H | O |

**Tabelle 2**

| | | | | | | |
|---|---|---|---|---|---|---|
| 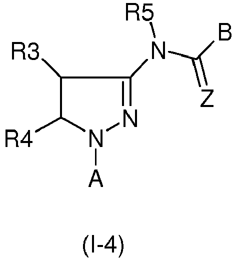 | | | | | | |

| **Verbindung Nr.** | **A** | **B** | **R 3** | **R4** | **R5** | **Z** |
|---|---|---|---|---|---|---|
| I-4-1 | 2,6-Difluorphenyl | 2-Chlorphenyl | H | H | H | O |
| I-4-2 Synthesebeispiel 6 | 2,6-Difluorphenyl | 2-Bromphenyl | H | H | H | O |
| I-4-3 | 2,6-Difluorphenyl | 2,6-Difluorphenyl | H | H | H | O |
| I-4-4 | 2,6-Difluorphenyl | 2-Iodphenyl | H | H | H | O |
| I-4-5 | 2,6-Difluorphenyl | 2-Methylphenyl | H | H | H | O |
| I-4-6 | 2,6-Difluorphenyl | 2-Methylphenyl | H | H | 2-Methylpyridin-3-ylcarbonyl | O |
| I-4-7 | 2,6-Difluorphenyl | 2-(Trifluormethyl)phenyl | H | H | H | O |
| I-4-8 | 2-Chlorphenyl | 2-Bromphenyl | H | H | H | O |

**Tabelle 3**

| | | | | | |
|---|---|---|---|---|---|
| 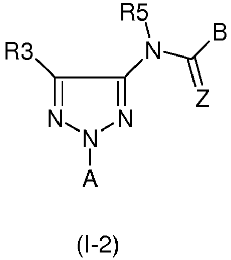 | | | | | |

| **Verbindung Nr.** | **A** | **B** | **R3** | **R5** | **Z** |
|---|---|---|---|---|---|
| I-2-1 Synthesebeispiel 20 | 2-Chlorphenyl | 2-(Trifluormethyl)phenyl | H | H | O |
| I-2-2 | 2-Chlorphenyl | 2-Chlorphenyl | H | H | O |
| I-2-3 | 2-Chlorphenyl | 2-Iodphenyl | H | H | O |
| I-2-4 | 2-Chlorphenyl | 2-(Trifluormethyl)pyridin-3-yl | H | H | O |
| I-2-5 | 2-Chlorphenyl | 2-Chlorpyridin-3-yl | H | H | O |
| I-2-6 | 2-Bromphenyl | 2-(Trifluormethyl)phenyl | H | H | O |
| I-2-7 | 2-Bromphenyl | 2-Chlorphenyl | H | H | O |
| I-2-8 | 2-Bromphenyl | 2-(Trifluormethyl)pyridin-3-yl | H | H | O |
| I-2-9 | 2-Bromphenyl | 2-Chlorpyridin-3-yl | H | H | O |
| I-2-10 | 3-Chlorpyridin-2-yl | 2-(Trifluormethyl)phenyl | H | H | O |
| I-2-11 | 3-Chlorpyridin-2-yl | 2-(Trifluormethyl)phenyl | Cl | H | O |
| I-2-12 | 3-Chlorpyridin-2-yl | 2-Chlorphenyl | H | H | O |
| I-2-13 | 3-Chlorpyridin-2-yl | 2-Bromphenyl | H | H | O |
| I-2-14 | 3-Chlorpyridin-2-yl | 2-Iodphenyl | H | H | O |
| I-2-15 | 3-Chlorpyridin-2-yl | 2,6-Difluorphenyl | H | H | O |
| I-2-16 | 3-Chlorpyridin-2-yl | 5-Fluor-1-methyl-3-(trifluormethyl)-1H-pyrazol-4-yl | H | H | O |
| I-2-17 | 3-Brompyridin-2-yl | 2-(Trifluormethyl)phenyl | H | H | O |
| I-2-18 | 3-Brompyridin-2-yl | 2-Iodphenyl | H | H | O |
| I-2-19 | 2-Chlorphenyl | 2-(Trifluormethyl)phenyl | Cl | H | O |
| I-2-20 | 2-Chlorphenyl | 2-(Trifluormethyl)phenyl | Cl | 2-(Trifluormethy l)phenylcarbonyl | O |
| I-2-21 | 2-Chlorphenyl | 2-(Trifluormethyl)phenyl | Methyl | H | O |
| I-2-22 | 2-Chlorphenyl | 2-(Trifluormethyl)phenyl | Methyl | 2-(Trifluormethy l)phenylcarbonyl | O |
| I-2-23 | 2-Bromphenyl | 2-Chlorpyridin-3-yl | Cl | 2-Chlorpyridin-3-ylcarbonyl | O |
| I-2-24 | 2-Chlorphenyl | 2-Iodphenyl | Methyl | H | O |
| I-2-25 | 2-Chlorphenyl | 2-Iodphenyl | Methyl | 2-Iodphenylcarbonyl | O |
| I-2-26 | 2-Bromphenyl | 2-(Trifluormethyl)phenyl | Cl | H | O |
| I-2-27 | 2-Bromphenyl | 2-(Trifluormethyl)phenyl | Cl | 2-(Trifluormethy l)phenylcarbonyl | O |
| I-2-28 | 2-Bromphenyl | 2-(Trifluormethyl)pyridin-3-yl | Cl | H | O |
| I-2-29 | 2-Bromphenyl | 2-Chlorphenyl | Cl | H | O |
| I-2-30 | 2-Bromphenyl | 2-Chlorphenyl | Cl | 2-Chlorphenylcarbonyl | O |
| I-2-31 | 2-Bromphenyl | 2-Chlorpyridin-3-yl | Cl | H | O |
| I-2-32 | 2-Bromphenyl | 2-Iodphenyl | Cl | H | O |
| I-2-33 | 2-Bromphenyl | 2-Iodphenyl | H | H | O |
| I-2-34 | 2-Bromphenyl | 2-Iodphenyl | Cl | 2-Iodphenylcarbonyl | O |
| I-2-35 Synthesebeispiel 21 | 2,6-Difluorphenyl | 2-(Trifluormethyl)phenyl | H | H | O |
| I-2-36 | 2,6-Difluorphenyl | 2-Bromphenyl | H | H | O |
| I-2-37 | 2,6-Difluorphenyl | 2-Brompyridin-3-yl | H | H | O |
| I-2-38 | 2,6-Difluorphenyl | 2-Chlorphenyl | H | H | O |
| I-2-39 | 2,6-Difluorphenyl | 2-Fluor-6-(trifluormethyl)phenyl | H | H | O |
| I-2-40 | 2,6-Difluorphenyl | 2-Iod-3-thienyl | H | H | O |
| I-2-41 | 2,6-Difluorphenyl | 2-Iodphenyl | H | H | O |
| I-2-42 | 5-(Trifluormethyl)pyridin-2-yl | 2-(Trifluormethyl)phenyl | H | H | O |
| I-2-43 | 5-(Trifluormethyl)pyridin-2-yl | 2-Iodphenyl | H | H | O |
| I-2-44 | 3-Fluorpyridin-2-yl | 2-(Trifluormethyl)phenyl | H | H | O |
| I-2-45 | 3-Fluorpyridin-2-yl | 2-(Trifluormethyl)pyridin-3-yl | H | H | O |
| I-2-46 | 3-Fluorpyridin-2-yl | 2-Bromphenyl | H | H | O |
| I-2-47 | 3-Fluorpyridin-2-yl | 2-Brompyridin-3-yl | H | H | O |
| I-2-48 | 3-Fluorpyridin-2-yl | 2-Chlorphenyl | H | H | O |
| I-2-49 | 3-Fluorpyridin-2-yl | 2-Fluor-6-(trifluormethyl)phenyl | H | H | O |
| I-2-50 | 3-Fluorpyridin-2-yl | 2-Iod-3-thienyl | H | H | O |
| I-2-51 | 3-Fluorpyridin-2-yl | 2-Iodphenyl | H | H | O |
| I-2-52 | 3-Chlorpyridin-2-yl | 2-(Trifluormethyl)phenyl | H | 2-(Trifluormethy l)phenylcarbonyl | O |
| I-2-53 | 3-Chlorpyridin-2-yl | 2-(Trifluormethyl)phenyl | Methyl | H | O |
| I-2-54 | 3-Chlorpyridin-2-yl | 2-(Trifluormethyl)phenyl | Methyl | 2-(Trifluormethy l)phenylcarbonyl | O |
| I-2-55 | 3-Chlorpyridin-2-yl | 2-(Trifluormethyl)pyridin-3-yl | H | H | O |
| I-2-56 | 3-Chlorpyridin-2-yl | 2-Chlorpyridin-3-yl | H | H | O |
| I-2-57 | 3-Chlorpyridin-2-yl | 2-Fluor-6-(trifluormethyl)phenyl | H | H | O |
| I-2-58 | 3-Chlorpyridin-2-yl | 2-Iod-3-thienyl | H | H | O |
| I-2-59 | 3-Chlorpyridin-2-yl | 2-Iodphenyl | Methyl | H | O |
| I-2-60 | 3-Chlorpyridin-2-yl | 2-Iodphenyl | Cl | H | O |
| I-2-61 | 3-Chlorpyridin-2-yl | 2-lodphenyl | Methyl | 2-Iodphenylcarbonyl | O |
| I-2-62 | 2,5-Difluorpyridin-3-yl | 2-(Trifluormethyl)phenyl | H | H | O |
| I-2-63 Synthesebeispiel 45 | 3,5-Dichlorpyridin-2-yl | 2-(Trifluormethyl)phenyl | H | H | O |
| I-2-64 | 3,5-Dichlorpyridin-2-yl | 2-(Trifluormethyl)pyridin-3-yl | H | H | O |
| I-2-65 | 3,5-Dichlorpyridin-2-yl | 2-Iodphenyl | H | H | O |
| I-2-66 | 3,5-Difluorpyridin-2-yl | 2-(Trifluormethyl)phenyl | H | H | O |
| I-2-67 | 3,5-Difluorpyridin-2-yl | 2-(Trifluormethyl)pyridin-3-yl | H | H | O |
| I-2-68 | 3,5-Difluorpyridin-2-yl | 2-Bromphenyl | H | H | O |
| I-2-69 | 3,5-Difluorpyridin-2-yl | 2-Brompyridin-3-yl | H | H | O |
| I-2-70 | 3,5-Difluorpyridin-2-yl | 2-Chlorphenyl | H | H | O |
| I-2-71 | 3,5-Difluorpyridin-2-yl | 2-Fluor-6-(trifluormethyl)phenyl | H | H | O |
| I-2-72 | 3,5-Difluorpyridin-2-yl | 2-Iod-3-thienyl | H | H | O |
| I-2-73 | 3,5-Difluorpyridin-2-yl | 2-Iodphenyl | H | H | O |
| I-2-74 | 3-Chlor-5-(trifluormethyl)pyridin-2-yl | 2-(Trifluormethyl)phenyl | H | H | O |
| I-2-75 | 3-Chlor-5-(trifluormethyl)pyridin-2-yl | 2-Iodphenyl | H | H | O |
| I-2-76 | 2,6-Difluorphenyl | 2-(Trifluormethyl)pyridin-3-yl | H | H | O |
| I-2-77 | 2,6-Difluorphenyl | 2,6-Difluorphenyl | H | H | O |
| I-2-78 | 2,6-Difluorphenyl | 3-(Difluormethyl)-1-methyl-1H-pyrazol-4-yl | H | H | O |
| I-2-79 | 2,6-Difluorphenyl | 3-(Trifluormethyl)pyrazin-2-yl | H | H | O |
| I-2-80 | 2,6-Difluorphenyl | 3-(Trifluormethyl)pyridin-2-yl | H | H | O |
| I-2-81 | 2,6-Difluorphenyl | 3-(Trifluormethyl)pyridin-4-yl | H | H | O |
| I-2-82 | 2,6-Difluorphenyl | 3-(Trifluormethyl)thiophen-2-yl | H | H | O |
| I-2-83 | 2,6-Difluorphenyl | 4-(Trifluormethyl)pyridin-3-yl | H | H | O |
| I-2-84 | 3,5-Difluorpyridin-2-yl | 3-(Trifluormethyl)pyridin-2-yl | H | H | O |
| I-2-85 | 3,5-Difluorpyridin-2-yl | 3-(Trifluormethyl)thiophen-2-yl | H | H | O |

**Tabelle 4**

| | | | | | |
|---|---|---|---|---|---|
| 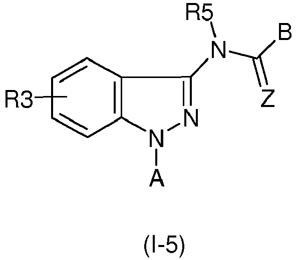 | | | | | |

| **Verbindung Nr.** | **A** | **B** | **R3** | **R5** | **Z** |
|---|---|---|---|---|---|
| I-5-1 | 3,5-Difluorpyridin-2-yl | 2-(Trifluormethyl)phenyl | H | H | O |
| I-5-2 Synthesebeispiel 22 | 3,5-Difluorpyridin-2-yl | 2-(Trifluormethyl)phenyl | 5-F | H | O |
| I-5-3 | 3,5-Difluorpyridin-2-yl | 2-Bromphenyl | H | H | O |
| I-5-4 | 3-Fluorpyridin-2-yl | 2-Bromphenyl | H | H | O |
| I-5-5 | 3-Chlor-5-(trifluormethyl)pyridin-2-yl | 2-Bromphenyl | H | H | O |
| I-5-6 | 2,5-Difluorpyridin-3-yl | 2-Bromphenyl | H | H | O |
| I-5-7 | 2,5-Difluorpyridin-3-yl | 2-(Trifluormethyl)phenyl | 5-F | H | O |
| I-5-8 Synthesebeispiel 47 | 2-Chlorphenyl | 2-(Trifluormethyl)phenyl | H | H | O |
| I-5-9 | 2-Chlorphenyl | 2-(Trifluormethyl)pyridin-3-yl | H | H | O |
| I-5-10 | 2,5-Difluor-4-cyanphenyl | 2-(Trifluormethyl)phenyl | 4-F | H | O |
| I-5-11 | 2,5-Difluor-4-cyanphenyl | 2,6-Difluorphenyl | 4-F | H | O |

**Tabelle5**

| | | | | | | | |
|---|---|---|---|---|---|---|---|
| 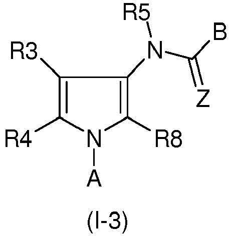 | | | | | | | |

| **Verbindung Nr.** | **A** | **B** | **R3** | **R4** | **R5** | **R8** | **Z** |
|---|---|---|---|---|---|---|---|
| I-3-1 Synthesebeispiel 46 | 2,6-Difluorphenyl | 2-(Trifluormethyl)phenyl | H | H | H | H | O |
| I-3-2 | 2,6-Difluorphenyl | 2,6-Difluorphenyl | H | H | H | H | O |
| I-3-3 | 2,6-Difluorphenyl | 2-Iodphenyl | H | H | H | H | O |
| I-3-4 | 2,6-Difluorphenyl | 2-Chlor-4-methoxyphenyl | H | H | H | H | O |
| I-3-5 | 2,6-Difluorphenyl | 2-(Trifluormethyl)pyridin-3-yl | H | H | H | H | O |
| I-3-6 | 2,6-Difluorphenyl | 3-Iod-2-furanyl | H | H | H | H | O |
| I-3-7 | 2,6-Difluorphenyl | 2-Iod-3-thienyl | H | H | H | H | O |

| | | | | | | | |
|---|---|---|---|---|---|---|---|
| *HPLC- MS*¹⁾ *und ¹H-NMR Daten* ²⁾ | | | | | | | |

| **Verbindung Nr.** |
|---|
| **HPLC-MS¹⁾ und ¹H-NMR Daten²⁾** |
| I-1-1 |
| siehe Synthesebeispiel 1 |
| I-1-2 |
| HPLC-MS: logP = 2,69; Masse (m/z): 372,0 (M+H)⁺; ¹H-NMR [DMSO-D₆] 3.33 (s, 3H), 6.86 (d, 1H), 7.05 - 7.09 (m, 1H), 7.24 - 7.26 (m, 1H), 7.32 - 7.42 (m, 2H), 7.46 - 7.49 (m, 1H), 7.51 - 7.54 (m, 1H), 7.58-7.60 (m, 1H), 7.68-7.70 (m, 1H), 8.12 (d, 1H), 11.17 (s, 1H). |
| I-1-3 |
| HPLC-MS: logP = 2,87; Masse (m/z): 312,1 (M+H)⁺; ¹H-NMR [DMSO-D₆] 2.24 (s, 3H), 2.52 (s, 3H), 3.88 (s, 3H), 6.79 - 6.80 (m, 1H), 6.83-6.84 (m, 1H), 7.05-7.10 (m, 1H), 7.23 - 7.25 (m, 1H), 7.31 - 7.35 (m, 1H), 7.62 - 7.65 (m, 1H), 8.10 (d, 1H), 10.48 (s, 1H). |
| I-1-4 |
| HPLC-MS: logP = 2,92; Masse (m/z): 366,0 (M+H)⁺; ¹H-NMR [CD₃CN] 6.97 (d, 1H), 7.40 - 7.60 (m, 5H), 7.64 - 7.68 (m, 1H), 7.74 - 7.78 (m, 2H), 7.86 - 7.88 (m, 1H), 9.18 (br. s, 1H). |
| I-1-5 |
| HPLC-MS: logP = 3,05; Masse (m/z): 458,0 (M+H)⁺; ¹H-NMR [CD₃CN] 6.96 - 6.97 (m, 1H), 7.17 - 7.24 (m, 1H), 7.45 - 7.50 (m, 2H), 7.54-7.56 (m, 1H), 7.64 - 7.68 (m, 1H), 7.74 - 7.78 (m, 2H), 7.86 - 7.88 (m, 1H), 7.93 - 7.95 (m, 1H), 9.17 (br. s, 1H). |
| I-1-6 |
| HPLC-MS: logP = 2.75; Masse (m/z): 372,1 (M+H)⁺; ¹H-NMR [DMSO-D₆] 6.90 (d, 1H), 7.41 (s, 1H), 7.64 - 7.67 (m, 1H), 7.73 - 7.77 (m, 1H), 7.85 - 7.89 (m, 1H), 7.92 - 7.96 (m, 2H), 8.00 (d, 1H), 8.32 (t, 1H), 11.53 (s, 1H). |
| I-1-7 |
| HPLC-MS: logP = 3,05; Masse (m/z): 400,1 (M+H)⁺; ¹H-NMR [CD₃CN] 6.94 - 6.95 (m, 1H), 7.54 - 7.55 (m, 1H), 7.64 - 7.82 (m, 7H), 7.86-7.88 (m, 1H), 9.23 (br. s, 1H). |
| I-1-8 |
| HPLC-MS: logP = 2,38; Masse (m/z): 323,1 (M+H)⁺; ¹H-NMR [CD₃CN] 7.07 (d, 1H), 7.40 - 7.52 (m, 4H), 7.59 - 7.62 (m, 1H), 7.69 - 7.71 (m, 1H), 7.75 - 7.79 (m, 1H), 7.85 - 7.87 (m, 1H), 8.12 (d, 1H), 9.39 (br. s, 1H). |
| I-1-9 |
| HPLC-MS: logP = 2,55; Masse (m/z): 357,1 (M+H)⁺; ¹H-NMR [CD₃CN] 7.05 (d, 1H), 7.48 - 7.52 (m, 1H), 7.65 - 7.86 (m, 7H), 8.11 (d, 1H), 9.39 (br. s, 1H). |
| I-1-10 |
| HPLC-MS: logP = 2,78; Masse (m/z): 360,01 (M+H)⁺; ¹H-NMR [DMSO-D₆] 6.95 (d, 1H), 7.34 - 7.50 (m, 5H), 7.53 - 7.55 (m, 1H), 7.69-7.71 (m, 1H), 7.73 - 7.77 (m, 1H), 8.16-8.17 (m, 1H), 11.27 (s, 1H). |
| I-1-11 |
| HPLC-MS: logP = 2,86; Masse (m/z): 312,0 (M+H)⁺; ¹H-NMR [CD₃CN] 2.45 (s, 3H), 6.98-6.99 (m, 1H), 7.25 - 7.31 (m, 3H), 7.36 - 7.45 (m, 3H), 7.47 - 7.54 (m, 2H), 7.58 - 7.60 (m, 1H), 7.88 (d, 1H), 9.07 (br. s, 1H). |
| I-1-12 |
| HPLC-MS: logP = 3,30; Masse (m/z): 326,1 (M+H)⁺; ¹H-NMR [CD₃CN] 1.25 (t, 3H), 2.84 (q, 2H), 7.01 (d, 1H), 7.28 - 7.32 (m, 1H), 7.37-7.38 (m, 1H), 7.43 - 7.52 (m, 4H), 7.55 - 7.58 (m, 1H), 7.61 - 7.64 (m, 1H), 7.91 (d, 1H), 9.13 (br. s, 1H). |
| I-1-13 |
| HPLC-MS: logP = 3,03; Masse (m/z): 348,1 (M+H)⁺; ¹H-NMR [CD₃CN] 6.97 (d, 1H), 7.30 (t, 1H), 7.41 - 7.48 (m, 2H), 7.52-7.56 (m, 1H), 7.59 - 7.69 (m, 3H), 7.76 - 7.81 (m, 2H), 7.81 - 7.89 (m, 1H), 9.32 (br. s, 1H). |
| I-1-14 |
| HPLC-MS: logP = 2,96; Masse (m/z): 366,0 (M+H)⁺; ¹H-NMR [CD₃CN] 6.95 (d, 1H), 7.41 - 7.47 (m, 2H), 7.51 - 7.54 (m, 1H), 7.57 - 7.60 (m, 1H), 7.65 - 7.74 (m, 3H), 7.80 - 7.82 (m, 1H), 7.88 (d, 1H), 9.26 (br. s, 1H). |
| I-1-15 |
| siehe Synthesebeispiel 17 |
| I-1-16 |
| siehe Synthesebeispiel 18 |
| I-1-17 |
| siehe Synthesebeispiel 19 |
| I-1-18 |
| HPLC-MS: logP = 3,28; Masse (m/z): 382,0 (M+H)⁺; ¹H-NMR [CD₃CN] 6.96 (d, 1H), 7.40 - 7.56 (m, 4H), 7.59 - 7.65 (m, 3H), 7.77 - 7.80 (m, 1H), 7.89 (d, 1H), 9.23 (br. s, 1H). |
| I-1-19 |
| HPLC-MS: logP = 2,89; Masse (m/z): 316,0 (M+H)⁺; ¹H-NMR [CD₃CN] 6.99 (d, 1H), 7.24 - 7.29 (m, 1H), 7.32 - 7.36 (m, 1H), 7.41 - 7.48 (m, 2H), 7.53 - 7.62 (m, 3H), 7.89 - 7.93 (m, 2H), 9.17 (br. s, 1H). |
| I-1-20 |
| siehe Synthesebeispiel 14 |
| I-1-21 |
| siehe Synthesebeispiel 15 |
| I-1-22 |
| HPLC-MS: logP = 3,41; Masse (m/z): 360,0 (M+H)⁺; ¹H-NMR [CD₃CN] 1.28 (t, 3H), 3.95 (q, 2H), 6.10 (d, 1H), 7.21 -7.33 (m, 4H), 7.37-7.54 (m, 4H), 7.61 - 7.64 (m, 1H). |
| I-1-23 |
| HPLC-MS: logP = 3,25; Masse (m/z): 370,0 (M+H)⁺; ¹H-NMR [CD₃CN] 2.54 (s, 1H), 4.72 (s, 2H), 6.15 (s, 1H), 7.22-7.34 (m, 4H), 7.39-7.41 (m, 3H), 7.53 - 7.54 (m, 1H), 7.66 (s, 1H). |
| I-1-24 |
| HPLC-MS: logP = 3,85; Masse (m/z): 386,1 (M+H)⁺; ¹H-NMR [CD₃CN] 0.29 - 0.30 (m, 2H), 0.48 - 0.50 (m, 2H), 1.13 - 1.21 (m, 1H), 3.81 (d, 2H), 6.13 (d, 1H), 7.15 - 7.34 (m, 5H), 7.39 - 7.41 (m, 2H), 7.52 - 7.55 (m, 1H), 7.62 (d, 1H). |
| I-1-25 |
| HPLC-MS: logP = 3,04; Masse (m/z): 371,0 (M+H)⁺; ¹H-NMR [CD₃CN] 4.87 (s, 2H), 5.99 (s, 1H), 7.24 - 7.45 (m, 7H), 7.55 - 7.56 (m, 1H), 7.66 (s, 1H). |
| I-1-26 |
| HPLC-MS: logP = 3,56; Masse (m/z): 372,1 (M+H)⁺; ¹H-NMR [DMSO-D₆] 4.51 (d, 2H), 5.18 (d, 1H), 5.33 (d, 1H), 5.89 - 5.99 (m, 1H), 6.12 (d, 1H), 7.19 - 7.50 (m, 7H), 7.59 - 7.61 (m, 1H), 7.89 (d, 1H). |
| I-1-27 |
| siehe Synthesebeispiel 16 |
| I-1-28 |
| HPLC-MS: logP =2,88; Masse (m/z): 366,0 (M+H)⁺; ¹H-NMR [CD₃CN] 7.42-7.63 (m, 8H), 8.05 (s, 1H), 8.62 (br. s, 1H). |
| I-1-29 |
| HPLC-MS: logP = 2,99; Masse (m/z):409,9 (M+H)⁺; ¹H-NMR [CD₃CN] 7.44 - 7.63 (m, 8H), 8.06 (s, 1H), 8.58 (br. s, 1H). |
| I-1-30 |
| HPLC-MS: logP = 3,01; Masse (m/z): 457,9 (M+H)⁺; ¹H-NMR [CD₃CN] 7.42-7.66 (m, 8H), 8.05 (s, 1H), 8.55 (br. s, 1H). |
| I-1-31 |
| HPLC-MS: logP = 2,45; Masse (m/z): 346,1 (M+H)⁺; ¹H-NMR [CD₃CN] 2.28 (s, 3H), 6.48 (s, 1H), 7.32-7.36 (m, 1H), 7.39-7.50 (m, 5H), 7.56 - 7.59 (m, 1H), 8.50 (br. s, 1H). |
| I-1-32 |
| HPLC-MS: logP = 2,83; Masse (m/z): 376,0 (M+H)⁺; ¹H-NMR [CD₃CN] 6.97 (d, 1H), 7.37 -7.47 (m, 4H), 7.53 - 7.56 (m, 2H), 7.59 - 7.60 (m, 1H), 7.68 - 7.69 (m, 1H), 7.89 (d, 1H), 9.20 (br. s, 1H). |
| I-1-33 |
| HPLC-MS: logP = 2,95; Masse (m/z): 423,9 (M+H)⁺; ¹H-NMR [CD₃CN] 6.97 (d, 1H), 7.18 - 7.24 (m, 1H), 7.39 - 7.55 (m, 5H), 7.58 - 7.61 (m, 1H), 7.89 (d, 1H), 7.93 - 7.95 (m, 1H), 9.17 (br. s, 1H). |
| I-1-34 |
| HPLC-MS: logP = 3,10; Masse (m/z): 457,9 (M+H)⁺; ¹H-NMR [CD₃CN] 7.21 - 7.25 (m, 1H), 7.48 - 7.62 (m, 6H), 7.97 - 7.98 (m, 1H), 8.05 (s, 1H), 8.56 (br. s, 1H). |
| I-1-35 |
| HPLC-MS: logP = 2,53; Masse (m/z): 438,0 (M+H)⁺; ¹H-NMR [CD₃CN] 2.28 (s, 3H), 6.49 (s, 1H), 7.12 - 7.17 (m, 1H), 7.24 - 7.29 (m, 1H), 7.38 - 7.49 (m, 4H), 7.56 - 7.58 (m, 1H), 7.84 - 7.86 (m, 1H), 8.46 (br. s, 1H). |
| I-1-36 |
| HPLC-MS: logP = 2,65; Masse (m/z): 334,0 (M+H)⁺; ¹H-NMR [CD₃CN] 6.95 (d, 1H), 7.03 - 7.13 (m, 2H), 7.41 - 7.49 (m, 2H), 7.50 - 7.56 (m, 2H), 7.59 - 7.62 (m, 1H), 7.88 (d, 1H), 9.46 (br. s, 1H). |
| I-1-37 |
| HPLC-MS: logP = 2,84; Masse (m/z): 350,0 (M+H)⁺; ¹H-NMR [CD₃CN] 6.97 (d, 1H), 7.20 - 7.23 (m, 1H), 7.35 - 7.37 (m, 1H), 7.42 - 7.50 (m, 3H), 7.52 - 7.55 (m, 1H), 7.59 - 7.61 (m, 1H), 7.89 (d, 1H), 9.37 (br. s, 1H). |
| I-1-38 |
| HPLC-MS: logP = 3,24; Masse (m/z): 313,1 (M+H)⁺; ¹H-NMR [CD₃CN] 2.78 (s, 3H), 7.06 (d, 1H), 7.43 - 7.52 (m, 3H), 7.61 - 7.65 (m, 2H), 7.78 - 7.80 (m, 1H), 7.94 (d, 1H), 8.52 - 8.54 (m, 1H), 10.51 (br. s, 1H). |
| I-1-39 |
| HPLC-MS: logP = 2,99; Masse (m/z): 367,0 (M+H)⁺; ¹H-NMR [CD₃CN] 7.02 (d, 1H), 7.44 - 7.52 (m, 2H), 7.57 - 7.65 (m, 2H), 7.75 - 7.78 (m, 1H), 7.93 - 7.94 (m, 1H), 8.31 - 8.34 (m, 1H), 8.88 - 8.89 (m, 1H), 10.04 (br. s, 1H). |
| I-1-40 |
| HPLC-MS: logP = 2,79; Masse (m/z): 333,1 (M+H)⁺; ¹H-NMR [CD₃CN] 7.03 (d, 1H), 7.42 - 7.48 (m, 2H), 7.54 - 7.61 (m, 3H), 7.92 (d, 1H), 7.97 -7.99 (m, 1H), 8.58-8.59 (m, 1H), 10.09 (br. s, 1H). |
| I-1-41 |
| HPLC-MS: logP = 1,44; Masse (m/z): 313,1 (M+H)⁺; ¹H-NMR [CD₃CN] 2.62 (s, 3H), 6.98 (d, 1H), 7.24 - 7.27 (m, 1H), 7.40 - 7.47 (m, 2H), 7.52 -7.54 (m, 1H), 7.58 - 7.60 (m, 1H), 7.83 - 7.89 (m, 2H), 8.53 - 8.55 (m, 1H), 9.25 (br. s, 1H). |
| I-1-42 |
| HPLC-MS: logP = 2,48; Masse (m/z):367,1 (M+H)⁺; ¹H-NMR [CD₃CN] 6.95 (d, 1H), 7.42 - 7.47 (m, 2H), 7.53 - 7.54 (m, 1H), 7.59 - 7.61 (m, 1H), 7.71 - 7.73 (m, 1H), 7.89 (d, 1H), 8.08 - 8.10 (m, 1H), 8.81 - 8.82 (m, 1H), 9.35 (br. s, 1H). |
| I-1-43 |
| HPLC-MS: logP = 2,19; Masse (m/z): 333,0 (M+H)⁺; ¹H-NMR [CD₃CN] 6.97 (d, 1H), 7.38 - 7.48 (m, 3H), 7.51 - 7.55 (m, 1H), 7.59 -7.62 (m, 1H), 7.89 (d, 1H), 7.97 - 7.99 (m, 1H), 8.48 - 8.50 (m, 1H), 9.35 (br. s, 1H). |
| I-1-44 |
| HPLC-MS: logP = 2,74; Masse (m/z): 368,0 (M+H)⁺; ¹H-NMR [CD₃CN] 6.99 (d, 1H), 7.41 - 7.49 (m, 2H), 7.55 - 7.62 (m, 2H), 7.92 (d, 1H), 8.90 (s, 2H), 9.84 (br. s, 1H). |
| I-1-45 |
| HPLC-MS: logP = 3,05; Masse (m/z): 316,1 (M+H)⁺; ¹H-NMR [DMSO-D₆] 2.23 (s, 3H), 2.52 (s, 3H), 6.78 (s, 1H), 6.88 (d, 1H), 7.45 - 7.53 (m, 2H), 7.60 - 7.62 (m, 1H), 7.67 - 7.69 (m, 1H), 8.05 (d, 1H), 10.51 (br. s, 1H). |
| I-1-46 |
| HPLC-MS: logP = 2,96; Masse (m/z): 318,0 (M+H)⁺; ¹H-NMR [CD₃CN] 2.53 (s, 3H), 6.92 (d, 1H), 6.99 (d, 1H), 7.40 - 7.49 (m, 3H), 7.54-7.60 (m, 2H), 7.87 - 7.88 (m, 1H), 8.76 (br. s, 1H). |
| I-1-47 |
| HPLC-MS: logP = 3,05; Masse (m/z): 430,0 (M+H)⁺; ¹H-NMR [CD₃CN] 6.96 (d, 1H), 7.25 (d, 1H), 7.40 - 7.48 (m, 2H), 7.53 - 7.56 (m, 1H), 7.58 - 7.61 (m, 1H), 7.63 - 7.64 (m, 1H), 7.88 (d, 1H), 9.08 (br. s, 1H). |
| I-1-48 |
| HPLC-MS: logP = 2,46; Masse (m/z): 350,0 (M+H)⁺; ¹H-NMR [CD₃CN] 2.38 (s, 3H), 3.77 (s, 3H), 6.93 (d, 1H), 7.39 - 7.47 (m, 2H), 7.53-7.55 (m, 1H), 7.58 - 7.60 (m, 1H), 7.86 (d, 1H), 8.75 (br. s, 1H). |
| I-1-49 |
| HPLC-MS: logP = 2,59; Masse (m/z): 370,0 (M+H)⁺; ¹H-NMR [CD₃CN] 3.79 (s, 3H), 6.89 (d, 1H), 7.10 (t, 1H), 7.40 - 7.48 (m, 2H), 7.53-7.56 (m, 1H), 7.58 - 7.61 (m, 1H), 7.87 (d, 1H), 8.62 (br. s, 1H). |
| I-1-50 |
| HPLC-MS: logP = 3,07; Masse (m/z): 410,1 (M+H)⁺; ¹H-NMR [CD₃CN] 6.94 (d, 1H), 7.36 - 7.40 (m, 1H), 7.48 - 7.52 (m, 2H), 7.65 - 7.83 (m, 6H), 9.22 (br. s, 1H). |
| I-1-51 |
| HPLC-MS: logP = 2,83; Masse (m/z): 376,0 (M+H)⁺; ¹H-NMR [CD₃CN] 6.97 (d, 1H), 7.36 - 7.39 (m, 1H), 7.41 - 7.43 (m, 1H), 7.46 - 7.52 (m, 4H), 7.59 - 7.61 (m, 1H), 7.76 - 7.78 (m, 1H), 7.83 (d, 1H), 9.19 (br. s, 1H). |
| I-1-52 |
| HPLC-MS: logP = 2,86; Masse (m/z): 419.9 (M+H)⁺; ¹H-NMR [CD₃CN] 6.96 (d, 1H), 7.36 - 7.40 (m, 2H), 7.45 - 7.47 (m, 1H), 7.49 - 7.51 (m, 2H), 7.55 - 7.56 (m, 1H), 7.68 - 7.69 (m, 1H), 7.76 - 7.78 (m, 1H), 7.83 (d, 1H), 9.17 (br. s, 1H). |
| I-1-53 |
| HPLC-MS: logP = 3,07; Masse (m/z): 467.9 (M+H)⁺; ¹H-NMR [CD₃CN] 6.96 (d, 1H), 7.19 - 7.23 (m, 1H), 7.35 - 7.40 (m, 1H), 7.46 - 7.51 (m, 4H), 7.76 - 7.78 (m, 1H), 7.84 (d, 1H), 7.93 - 7.95 (m, 1H), 9.19 (br. s, 1H). |
| I-1-54 |
| HPLC-MS: logP = 2,99; Masse (m/z): 411.0 (M+H)⁺; ¹H-NMR [CD₃CN] 6.99 (d, 1H), 7.35 - 7.40 (m, 1H), 7.48 - 7.54 (m, 3H), 7.72 - 7.79 (m, 2H), 7.86 (d, 1H), 8.28 - 8.31 (m, 1H), 8.85 - 8.86 (m, 1H), 10.01 (br. s, 1H). |
| I-1-55 |
| HPLC-MS: logP = 2,76; Masse (m/z): 377.0 (M+H)⁺; ¹H-NMR [CD₃CN] 7.01 (d, 1H), 7.35 - 7.40 (m, 1H), 7.48 - 7.56 (m, 3H), 7.77 - 7.79 (m, 1H), 7.86 (d, 1H), 7.96 - 7.99 (m, 1H), 8.58 - 8.59 (m, 1H), 10.08 (br. s, 1H). |
| I-1-56 |
| HPLC-MS: logP = 2,54; Masse (m/z): 411.0 (M+H)⁺; ¹H-NMR [CD₃CN] 6.94 (d, 1H), 7.36 - 7.40 (m, 1H), 7.48 - 7.53 (m, 2H), 7.70 - 7.73 (m, 1H), 7.76 - 7.79 (m, 1H), 7.84 (d, 1H), 8.08 - 8.10 (m, 1H), 8.80 - 8.82 (m, 1H), 9.30 (br. s, 1H). |
| I-1-57 |
| HPLC-MS: logP = 2,37; Masse (m/z): 377.0 (M+H)⁺; ¹H-NMR [CD₃CN] 6.96 (d, 1H), 7.35 - 7.41 (m, 1H), 7.44 - 7.47 (m, 1H), 7.49 - 7.51 (m, 2H), 7.76 - 7.78 (m, 1H), 7.84 (d, 1H), 7.97 - 8.00 (m, 1H), 8.48 - 8.50 (m, 1H), 9.34 (br. s, 1H). |
| I-1-58 |
| HPLC-MS: logP = 2,63; Masse (m/z): 414.0 (M+H)⁺; ¹H-NMR [CD₃CN] 3.79 (s, 3H), 6.88 (d, 1H), 7.11 (t, 1H), 7.35 - 7.41 (m, 1H), 7.49-7.51 (m, 2H), 7.76 - 7.79 (m, 1H), 7.82 (d, 1H), 8.69 (br. s, 1H). |
| I-1-59 |
| HPLC-MS: logP = 3,00; Masse (m/z): 431.9 (M+H)⁺; ¹H-NMR [CD₃CN] 3.81 (s, 3H), 6.88 (d, 1H), 7.36 - 7.41 (m, 1H), 7.49 - 7.51 (m, 2H), 7.76 - 7.78 (m, 1H), 7.82 (d, 1H), 9.00 (br. s, 1H). |
| I-1-60 |
| HPLC-MS: logP = 3,16; Masse (m/z): 362.0 (M+H)⁺; ¹H-NMR [CD₃CN] 2.53 (s, 3H), 6.91 (d, 1H), 6.98 - 6.99 (m, 1H), 7.34 - 7.38 (m, 1H), 7.48 - 7.50 (m, 3H), 7.75 - 7.77 (m, 1H), 7.82 (d, 1H), 8.77 (br. s, 1H). |
| I-1-61 |
| siehe Synthesebeispiel 2 |
| I-1-62 |
| HPLC-MS: logP = 3,07; Masse (m/z): 368.1 (M+H)⁺; ¹H-NMR [CD₃CN] 7.02 (d, 1H), 7.20 - 7.30 (m, 2H), 7.54 - 7.58 (m, 1H), 7.66 - 7.76 (m, 3H), 7.80 - 7.82 (m, 1H), 8.03 - 8.04 (m, 1H), 9.34 (br. s, 1H). |
| I-1-63 |
| siehe Synthesebeispiel 3 |
| I-1-64 |
| HPLC-MS: logP = 3,02; Masse (m/z): 368.0 (M+H)⁺; ¹H-NMR [DMSO-D₆] 6.92 (d, 1H), 7.24 - 7.29 (m, 1H), 7.54 - 7.60 (m, 1H), 7.67 - 7.71 (m, 2H), 7.74 - 7.84 (m, 3H), 8.13 - 8.14 (m, 1H), 11.35 (br. s, 1H). |
| I-1-65 |
| HPLC-MS: logP = 3,13; Masse (m/z): 368.1 (M+H)⁺; ¹H-NMR [CD₃CN] 7.01 (d, 1H), 7.03 - 7.09 (m, 1H), 7.30 - 7.36 (m, 1H), 7.52 - 7.57 (m, 1H), 7.66 - 7.74 (m, 3H), 7.80 - 7.82 (m, 1H), 8.07 - 8.08 (m, 1H), 9.39 (br. s, 1H). |
| I-1-66 |
| HPLC-MS: logP = 3,02; Masse (m/z): 334.0 (M+H)⁺; ¹H-NMR [CD₃CN] 7.03 - 7.09 (m, 2H), 7.31 - 7.36 (m, 1H), 7.39 - 7.60 (m, 5H), 8.07-8.08 (m, 1H), 9.37 (br. s, 1H). |
| I-1-67 |
| HPLC-MS: logP = 3,10; Masse (m/z): 426.0 (M+H)⁺; ¹H-NMR [CD₃CN] 7.03 - 7.09 (m, 2H), 7.18 - 7.24 (m, 1H), 7.30 - 7.36 (m, 1H), 7.45-7.50 (m, 2H), 7.53 - 7.58 (m, 1H), 7.93 - 7.95 (m, 1H), 8.07 - 8.09 (m, 1H), 9.30 (br. s, 1H). |
| I-1-68 |
| HPLC-MS: logP = 3,29; Masse (m/z): 369.0 (M+H)⁺; ¹H-NMR [CD₃CN] 7.05 - 7.10 (m, 2H), 7.31 - 7.37 (m, 1H), 7.60 - 7.65 (m, 1H), 7.78-7.79 (m, 1H), 8.11 (s, 1H), 8.30-8.32 (m, 1H), 8.92 (br. s, 1H), 10.14 (s, 1H). |
| I-1-69 |
| HPLC-MS: logP = 1,53; Masse (m/z): 315.1 (M+H)⁺; ¹H-NMR [CD₃CN] 7.04 - 7.09 (m, 2H), 7.24 - 7.28 (m, 1H), 7.30 - 7.37 (m, 1H), 7.54-7.58 (m, 1H), 7.83 - 7.85 (m, 1H), 8.07 - 8.08 (m, 1H), 8.54 - 8.56 (m, 1H), 9.33 (br. s, 1H). |
| I-1-70 |
| HPLC-MS: logP = 2,60; Masse (m/z): 369.1 (M+H)⁺; ¹H-NMR [CD₃CN] 7.01 (d, 1H), 7.05 - 7.11 (m, 1H), 7.31 - 7.37 (m, 1H), 7.53 - 7.58 (m, 1H), 7.70 - 7.74 (m, 1H), 8.08 - 8.09 (m, 2H), 8.81 - 8.82 (m, 1H), 9.47 (br. s, 1H). |
| I-1-71 |
| HPLC-MS: logP = 3,24; Masse (m/z): 320.0 (M+H)⁺; ¹H-NMR [CD₃CN] 2.54 (s, 3H), 6.97 - 7.00 (m, 2H), 7.03 - 7.09 (m, 1H), 7.30 - 7.36 (m, 1H), 7.49 - 7.51 (m, 1H), 7.57 - 7.62 (m, 1H), 8.05 - 8.07 (m, 1H), 8.83 (br. s, 1H). |
| I-1-72 |
| siehe Synthesebeispiel 4 |
| I-1-73 |
| HPLC-MS: logP = 2,60; Masse (m/z): 314.1 (M+H)⁺; ¹H-NMR [CD₃CN] 2.40 (s, 3H), 6.95 - 6.96 (m, 1H), 7.24 - 7.38 (m, 5H), 7.43 - 7.47 (m, 1H), 7.54 - 7.61 (m, 1H), 7.98 - 7.99 (m, 1H), 10.94 (br. s, 1H). |
| I-1-74 |
| HPLC-MS: logP = 2,73; Masse (m/z): 350.0 (M+H)⁺; ¹H-NMR [CD₃CN] 7.01 (d, 1H), 7.15 - 7.48 (m, 3H), 7.49 - 7.55 (m, 1H), 7.61 - 7.69 (m, 2H), 7.77 - 7.81 (m, 3H), 9.37 (br. s, 1H). |
| I-1-75 |
| HPLC-MS: logP = 2,68; Masse (m/z): 368.1 (M+H)⁺; ¹H-NMR [CD₃CN] 6.99 (d, 1H), 7.16 - 7.22 (m, 2H), 7.47 - 7.54 (m, 1H), 7.65 - 7.76 (m, 4H), 7.80 - 7.82 (m, 1H), 9.30 (br. s, 1H). |
| I-1-76 |
| HPLC-MS: logP = 2,61; Masse (m/z): 386.1 (M+H)⁺; ¹H-NMR [CD₃CN] 7.13 - 7.23 (m, 2H), 7.50 - 7.57 (m, 1H), 7.67 - 7.77 (m, 3H), 7.82-7.84 (m, 2H), 8.76 (br. s, 1H). |
| I-1-77 |
| HPLC-MS: logP = 3,33; Masse (m/z): 384.0 (M+H)⁺; ¹H-NMR [CD₃CN] 7.18 - 7.24 (m, 2H), 7.50 - 7.60 (m, 4H), 7.65 - 7.69 (m, 1H), 7.73-7.75 (m, 1H), 7.83 - 7.84 (m, 1H), 10.67 (br. s, 1H). |
| I-1-78 |
| HPLC-MS: logP = 2,92; Masse (m/z): 328.1 (M+H)⁺; ¹H-NMR [CD₃CN] 1.22 (t, 3H), 2.81 (q, 2H), 7.01 (d, 1H), 7.16 - 7.21 (m, 2H), 7.25-7.29 (m, 1H), 7.33 - 7.35 (m, 1H), 7.40 - 7.44 (m, 1H), 7.47 - 7.54 (m, 2H), 7.74 - 7.75 (m, 1H), 9.07 (br. s, 1H). |
| I-1-79 |
| HPLC-MS: logP = 2,96; Masse (m/z): 384.0 (M+H)⁺; ¹H-NMR [CD₃CN] 7.00 (d, 1H), 7.15 - 7.21 (m, 2H), 7.43 - 7.55 (m, 3H), 7.60 - 7.65 (m, 1H), 7.76 - 7.80 (m, 2H), 9.21 (br. s, 1H). |
| I-1-80 |
| HPLC-MS: logP = 1,95; Masse (m/z): 378.0 (M+H)⁺; ¹H-NMR [CD₃CN] 3.31 (s, 3H), 6.98 (d, 1H), 7.17 - 7.22 (m, 2H), 7.49 - 7.53 (m, 1H), 7.70 - 7.81 (m, 4H), 8.06 - 8.08 (m, 1H), 9.35 (br. s, 1H). |
| I-1-81 |
| HPLC-MS: logP = 2,55; Masse (m/z): 334.0 (M+H)⁺; ¹H-NMR [CD₃CN] 7.01 (d, 1H), 7.17 - 7.21 (m, 2H), 7.41 - 7.43 (m, 1H), 7.46 - 7.44 (m, 3H), 7.60 - 7.61 (m, 1H), 7.76 (d, 1H), 9.20 (br. s, 1H). |
| I-1-82 |
| HPLC-MS: logP = 2,59; Masse (m/z): 378.0 (M+H)⁺; ¹H-NMR [CD₃CN] 7.01 (d, 1H), 7.17 - 7.21 (m, 2H), 7.38 - 7.40 (m, 1H), 7.45 - 7.48 (m, 1H), 7.49 - 7.54 (m, 1H), 7.55 - 7.57 (m, 1H), 7.68-7.69 (m, 1H), 7.76 (d, 1H), 9.18 (br. s, 1H). |
| I-1-83 |
| HPLC-MS: logP = 2,67; Masse (m/z): 426.1 (M+H)⁺; ¹H-NMR [CD₃CN] 7.01 (d, 1H), 7.16 - 7.23 (m, 3H), 7.46 - 7.54 (m, 3H), 7.76 (d, 1H), 7.93 - 7.95 (m, 1H), 9.15 (br. s, 1H). |
| I-1-84 |
| HPLC-MS: logP = 2,42; Masse (m/z): 336.1 (M+H)⁺; ¹H-NMR [CD₃CN] 6.99 (d, 1H), 7.08 - 7.13 (m, 2H), 7.17 - 7.22 (m, 2H), 7.48 - 7.57 (m, 2H), 7.77 (d, 1H), 9.40 (br. s, 1H). |
| I-1-85 |
| HPLC-MS: logP = 2,60; Masse (m/z): 352.0 (M+H)⁺; ¹H-NMR [CD₃CN] 7.02 (d, 1H), 7.20 - 7.24 (m, 3H), 7.37 - 7.39 (m, 1H), 7.49 - 7.54 (m, 2H), 7.80 (d, 1H), 9.62 (br. s, 1H). |
| I-1-86 |
| HPLC-MS: logP = 2.20; Masse (m/z): 345,0 (M+H)⁺; ¹H-NMR [DMSO-D₆] 6.92 (d, 1H), 7.32 - 7.41 (m, 2H), 7.56 - 7.64 (m, 1H), 7.71 - 7.77 (m, 2H), 7.82 - 7.87 (m, 1H), 8.07 (d, 1H), 8.11 - 8.13 (m, 1H), 11.46 (s, 1H). |
| I-1-87 |
| HPLC-MS: logP = 2.66; Masse (m/z): 316,0 (M+H)⁺; ¹H-NMR [DMSO-D₆] 6.95 - 7.02 (m, 3H), 7.36 - 7.47 (m, 3H), 7.58 - 7.65 (m, 1H), 8.05 - 8.08 (m, 2H), 11.02 (s, 1H), 11.89 (s, 1H). |
| I-1-88 |
| HPLC-MS: logP = 3.15; Masse (m/z): 400,0 (M+H)⁺; ¹H-NMR [DMSO-D₆] 6.95 (d, 1H), 7.36 - 7.40 (m, 2H), 7.57 - 7.68 (m, 3H), 7.74 - 7.81 (m, 2H), 8.07 - 8.08 (m, 1H), 11.34 (s, 1H). |
| I-1-89 |
| HPLC-MS: logP = 1.66; Masse (m/z): 367,1 (M+H)⁺; ¹H-NMR [DMSO-D₆] 6.78 (d, 1H), 7.32 - 7.37 (m, 2H), 7.55 - 7.60 (m, 2H), 7.64 - 7.71 (m, 3H), 7.98 (d, 1H), 8.13 (s, 1H), 8.88 (s, 1H), 11.13 (s, 1H). |
| I-1-90 |
| HPLC-MS: logP = 2,86; Masse (m/z): 315.1 (M+H)⁺; ¹H-NMR [CD₃CN] 2.74 (s, 3H), 7.06 (d, 1H), 7.17-7.23 (m, 2H), 7.45-7.54 (m, 2H), 7.75 - 7.77 (m, 2H), 8.48 - 8.50 (m, 1H), 10.47 (br. s, 1H). |
| I-1-91 |
| siehe Synthesebeispiel 7 |
| I-1-92 |
| HPLC-MS: logP = 2,40; Masse (m/z): 335.1 (M+H)⁺; ¹H-NMR [CD₃CN] 7.01 (d, 1H), 7.20 - 7.26 (m, 2H), 7.50 - 7.59 (m, 2H), 7.81 (d, 1H), 7.99-8.02 (m, 1H), 8.60-8.62 (m, 1H), 10.15 (br. s, 1H). |
| I-1-93 |
| HPLC-MS: logP = 2,68; Masse (m/z): 369.1 (M+H)⁺; 1H-NMR [CD₃CN] 7.03 (d, 1H), 7.17 - 7.23 (m, 2H), 7.48 - 7.55 (m, 1H), 7.72 - 7.75 (m, 1H), 7.78 (d, 1H), 8.28 - 8.30 (m, 1H), 8.85 - 8.86 (m, 1H), 10.04 (br. s, 1H). |
| I-1-94 |
| HPLC-MS: logP = 2,16; Masse (m/z): 369.0 (M+H)⁺; ¹H-NMR [CD₃CN] 7.00 (d, 1H), 7.17 - 7.22 (m, 2H), 7.48 - 7.55 (m, 1H), 7.74 - 7.78 (m, 2H), 8.91 - 8.94 (m, 2H), 9.44 (br. s, 1H). |
| I-1-95 |
| HPLC-MS: logP = 1,96; Masse (m/z): 335.1 (M+H)⁺; ¹H-NMR [CD₃CN] 7.01 (d, 1H), 7.17 - 7.21 (m, 2H), 7.44 - 7.55 (m, 2H), 7.77 (d, 1H), 7.97 - 8.00 (m, 1H), 8.48 - 8.50 (m, 1H), 9.36 (br. s, 1H). |
| I-1-96 |
| HPLC-MS: logP = 1,97; Masse (m/z): 378.9 (M+H)⁺; ¹H-NMR [CD₃CN] 7.00 (d, 1H), 7.16 - 7.22 (m, 2H), 7.46 - 7.54 (m, 2H), 7.77 (d, 1H), 7.89 - 7.91 (m, 1H), 8.45 - 8.46 (m, 1H), 9.32 (br. s, 1H). |
| I-1-97 |
| HPLC-MS: logP = 2.16; Masse (m/z): 368,1 (M+H)⁺; ¹H-NMR [DMSO-D₆] 6.92 (d, 1H), 7.36 - 7.41 (m, 2H), 7.57 - 7.64 (m, 1H), 7.77 - 7.79 (m, 1H), 8.10 (d, 1H), 8.98 - 9.00 (m, 1H), 9.07 (s, 1H), 11.56 (s, 1H). |
| I-1-98 |
| HPLC-MS: logP = 1.73; Masse (m/z): 330,1 (M+H)⁺; ¹H-NMR [DMSO-D₆] 1.30 (t, 3H), 3.05 (q, 2H), 6.96 (d, 1H), 7.36 - 7.40 (m, 2H), 7.57-7.65 (m, 1H), 7.75 (d, 1H), 8.10 (d, 1H), 9.24 - 9.26 (m, 1H), 11.54 (s, 1H). |
| I-1-99 |
| HPLC-MS: logP = 2,45; Masse (m/z): 370.0 (M+H)⁺; ¹H-NMR [CD₃CN] 7.02 (d, 1H), 7.16 - 7.23 (m, 2H), 7.48 - 7.56 (m, 1H), 7.80 (d, 1H), 8.89 - 8.90 (m, 2H), 9.86 (br. s, 1H). |
| I-1-100 |
| HPLC-MS: logP = 2,15; Masse (m/z): 336.0 (M+H)⁺; ¹H-NMR [CD₃CN] 7.05 (d, 1H), 7.17 - 7.24 (m, 2H), 7.50 - 7.56 (m, 1H), 7.79 (d, 1H), 8.61 - 8.63 (m, 2H), 9.92 (br. s, 1H). |
| I-1-101 |
| HPLC-MS: logP = 2.41; Masse (m/z): 338,1 (M+H)⁺; ¹H-NMR [DMSO-D₆] 2.01 (s, 3H) 3.02 - 3.04 (m, 2H), 4.26 - 4.28 (m, 2H), 6.79 (d, 1H), 7.34 - 7.39 (m, 2H), 7.55 - 7.61 (m, 1H), 7.99 (d, 1H), 10.40 (s, 1H). |
| I-1-102 |
| HPLC-MS: logP = 2.56; Masse (m/z): 391,0 (M+H)⁺; ¹H-NMR [DMSO-D₆] 3.23 - 3.25 (m, 2H), 4.36 - 4.39 (m, 2H), 6.78 (d, 1H), 7.34 - 7.40 (m, 2H), 7.56 - 7.63 (m, 1H), 8.03 (d, 1H), 11.38 (s, 1H). |
| I-1-103 |
| HPLC-MS: logP = 2,64; Masse (m/z): 320.1 (M+H)⁺; ¹H-NMR [CD₃CN] 2.53 (s, 3H), 6.95 (d, 1H), 6.98 - 7.00 (m, 1H), 7.17 - 7.22 (m, 2H), 7.47 - 7.54 (m, 2H), 7.73 (d, 1H), 8.72 (br. s, 1H). |
| I-1-104 |
| HPLC-MS: logP = 2,96; Masse (m/z): 431.8 (M+H)⁺; ¹H-NMR [CD₃CN] 6.96 (d, 1H), 7.18 - 7.23 (m, 2H), 7.26 - 7.27 (m, 1H), 7.48 - 7.55 (m, 1H), 7.61 - 7.63 (m, 1H), 7.76 (d, 1H), 9.23 (br. s, 1H). |
| I-1-105 |
| HPLC-MS: logP = 2,76; Masse (m/z): 431.9 (M+H)⁺; ¹H-NMR [CD₃CN] 6.99 (d, 1H), 7.16 - 7.22 (m, 2H), 7.25 - 7.27 (m, 1H), 7.47 - 7.55 (m, 1H), 7.63 - 7.64 (m, 1H), 7.75 (d, 1H), 9.12 (br. s, 1H). |
| I-1-106 |
| HPLC-MS: logP = 2.53; Masse (m/z): 355,0 (M+H)⁺; ¹H-NMR [DMSO-D₆] 2.43 (s, 3H), 6.92 (d, 1H), 7.29 - 7.55 (m, 3H), 7.58 - 7.65 (m, 1H), 8.10 (d, 1H), 11.39 (s, 1H). |
| I-1-107 |
| HPLC-MS: logP = 1,71; Masse (m/z): 318.1 (M+H)⁺; ¹H-NMR [CD₃CN] 2.41 (s, 3H), 3.80 (s, 3H), 6.96 (d, 1H), 7.16 - 7.22 (m, 2H), 7.48-7.53 (m, 1H), 7.70 (d, 1H), 7.96 (s, 1H), 8.69 (br. s, 1H). |
| I-1-108 |
| HPLC-MS: logP = 2,03; Masse (m/z): 430.0 (M+H)⁺; ¹H-NMR [DMSO-D₆] 3.89 (s, 3H), 6.91 (d, 1H), 7.35 - 7.39 (m, 2H), 7.56 - 7.63 (m, 1H), 8.03 (d, 1H), 8.38 (s, 1H), 10.72 (br. s, 1H). |
| I-1-109 |
| HPLC-MS: logP = 1,77; Masse (m/z): 332.1 (M+H)⁺; ¹H-NMR [DMSO-D₆] 2.25 (s, 3H), 2.36 (s, 3H), 3.67 (s, 3H), 6.88 (d, 1H), 7.35 - 7.39 (m, 2H), 7.55 - 7.63 (m, 1H), 8.00 (d, 1H), 10.22 (br. s, 1H). |
| I-1-110 |
| HPLC-MS: logP = 1,98; Masse (m/z): 336.0 (M+H)⁺; ¹H-NMR [CD₃CN] 2.36 (s, 3H), 3.65 (s, 3H), 6.93 (d, 1H), 7.15 - 7.21 (m, 2H), 7.46-7.53 (m, 1H), 7.71 - 7.72 (m, 1H), 8.41 (br. s, 1H). |
| I-1-111 |
| HPLC-MS: logP = 2.40; Masse (m/z): 340,1 (M+H)⁺; ¹H-NMR [DMSO-D₆] 6.93 (d, 1H), 7.37 - 7.41 (m, 3H), 7.58 - 7.65 (m, 1H), 7.93 (d, 1H), 8.11 (d, 1H), 8.32 (t, 1H), 11.57 (s, 1H). |
| I-1-112 |
| HPLC-MS: logP = 2.53; Masse (m/z): 390,0 (M+H)⁺; ¹H-NMR [DMSO-D₆] 3.85 (s, 3H), 6.87 (d, 1H), 7.36 - 7.40 (m, 2H), 7.57 - 7.64 (m, 1H), 8.06 (d, 1H), 11.09 (s, 1H). |
| I-1-113 |
| HPLC-MS: logP = 2,09; Masse (m/z): 335.1 (M+H)⁺; ¹H-NMR [CD₃CN] 2.60 (s, 3H), 2.64 (s, 3H), 6.93 (d, 1H), 7.15 - 7.21 (m, 2H), 7.46-7.54 (m, 1H), 7.73-7.74 (m, 1H), 8.91 (br. s, 1H). |
| I-1-114 |
| HPLC-MS: logP = 2,58; Masse (m/z): 389.1 (M+H)⁺; ¹H-NMR [CD₃CN] 2.73 (s, 3H), 6.92-6.93 (m, 1H), 7.17 - 7.22 (m, 2H), 7.48 - 7.55 (m, 1H), 7.75 - 7.76 (m, 1H), 9.42 (br. s, 1H). |
| I-1-115 |
| HPLC-MS: logP = 3.33; Masse (m/z): 374,0 (M+H)⁺; ¹H-NMR [DMSO-D₆] 6.92 (d, 1H), 7.37 - 7.42 (m, 2H), 7.58 - 7.65 (m, 1H), 8.12 (d, 1H), 11.65 (s, 1H). |
| I-1-116 |
| HPLC-MS: logP = 2.53; Masse (m/z): 306,1 (M+H)⁺; ¹H-NMR [DMSO-D₆]2.55 (s, 3H) 6.92 (d, 1H), 7.36 - 7.42 (m, 2H), 7.58 - 7.66 (m, 1H), 8.12 (d, 1H), 11.79 (s, 1H). |
| I-1-117 |
| siehe Synthesebeispiel 5 |
| I-1-118 |
| HPLC-MS: logP = 3,36; Masse (m/z): 366.0 (M+H)⁺; ¹H-NMR [CD₃CN] 7.00 (d, 1H), 7.41 - 7.44 (m, 2H), 7.46-7.52 (m, 2H), 7.56 - 7.60 (m, 3H), 7.95 (d, 1H), 9.27 (br. s, 1H). |
| I-1-119 |
| HPLC-MS: logP = 3,40; Masse (m/z): 409.9 (M+H)⁺; ¹H-NMR [CD₃CN] 7.00 (d, 1H), 7.38 - 7.48 (m, 3H), 7.55 - 7.58 (m, 2H), 7.60 - 7.61 (m, 1H), 7.68 - 7.70 (m, 1H), 7.95 (d, 1H), 9.22 (br. s, 1H). |
| I-1-120 |
| HPLC-MS: logP = 3,05; Masse (m/z): 399.9 (M+H)⁺; ¹H-NMR [CD₃CN] 6.97 (d, 1H), 7.46 - 7.50 (m, 1H), 7.56 - 7.58 (m, 1H), 7.65 - 7.74 (m, 1H), 7.80 - 7.82 (m, 1H), 9.30 (br. s, 1H). |
| I-1-121 |
| HPLC-MS: logP = 2,84; Masse (m/z): 366.0 (M+H)⁺; ¹H-NMR [CD₃CN] 7.00 (d, 1H), 7.40 - 7.52 (m, 4H), 7.56 - 7.61 (m, 3H), 7.66 (d, 1H), 9.25 (br.s, 1H). |
| I-1-122 |
| HPLC-MS: logP = 2,88; Masse (m/z): 410.0 (M+H)⁺; ¹H-NMR [CD₃CN] 6.99 (d, 1H), 7.38 - 7.41 (m, 1H), 7.44 - 7.50 (m, 2H), 7.55 - 7.58 (m, 3H), 7.66 - 7.69 (m, 2H), 9.18 (br. s, 1H). |
| I-1-123 |
| siehe Synthesebeispiel 8 |
| I-1-124 |
| HPLC-MS: logP = 2,57; Masse (m/z): 401.0 (M+H)⁺; ¹H-NMR [CD₃CN] 6.97 (d, 1H), 7.47 - 7.51 (m, 1H), 7.57 - 7.59 (m, 2H), 7.67 (d, 1H), 7.70-7.73 (m, 1H), 8.08 - 8.10 (m, 1H), 8.80 - 8.82 (m, 1H), 9.36 (br. s, 1H). |
| I-1-125 |
| HPLC-MS: logP = 2,46; Masse (m/z): 343.0 (M+H)⁺; ¹H-NMR [DMSO-D₆] 6.96 (d, 1H), 7.39 - 7.55 (m, 4H), 7.61 - 7.65 (m, 1H), 7.79-7.86 (m, 2H), 8.00 - 8.03 (m, 1H), 8.24 - 8.26 (m, 1H), 11.26 (s, 1H). |
| I-1-126 |
| HPLC-MS: logP = 1,96; Masse (m/z): 376.1 (M+H)⁺; ¹H-NMR [DMSO-D₆] 2.54 (s, 3H), 6.88 (d, 1H), 7.43 - 7.65 (m, 5H), 7.72 - 7.77 (m, 1H), 7.86 - 7.90 (m, 1H), 8.05 (d, 1H), 8.10 - 8.12 (m, 1H), 11.20 (s, 1H). |
| I-1-127 |
| HPLC-MS: logP = 2,36; Masse (m/z): 343.0 (M+H)⁺; ¹H-NMR [CD₃CN] 7.00 (d, 1H), 7.24 - 7.26 (m, 1H), 7.38 - 7.41 (m, 1H), 7.46 - 7.48 (m, 1H), 7.56 - 7.57 (m, 1H), 7.69 - 7.70 (m, 1H), 7.77 - 7.79 (m, 1H), 7.88 - 7.90 (m, 1H), 8.41 - 8.42 (m, 1H), 8.52 (d, 1H), 9.24 (br. s, 1H). |
| I-1-128 |
| HPLC-MS: logP = 2,24; Masse (m/z): 351.0 (M+H)⁺; ¹H-NMR [CD₃CN] 7.03 (d, 1H), 7.33 - 7.37 (m, 1H), 7.60 - 7.77 (m, 5H), 8.27 - 8.30 (m, 2H), 9.53 (br. s, 1H). |
| I-1-129 |
| HPLC-MS: logP = 2,05; Masse (m/z): 317.0 (M+H)⁺; ¹H-NMR [CD₃CN] 7.06 (d, 1H), 7.34 - 7.38 (m, 2H), 7.40 - 7.47 (m, 2H), 7.55 - 7.57 (m, 1H), 7.69 - 7.74 (m, 1H), 8.28 - 8.31 (m, 2H), 9.54 (br. s, 1H). |
| I-1-130 |
| HPLC-MS: logP = 2,12; Masse (m/z): 361.0 (M+H)⁺; ¹H-NMR [CD₃CN] 7.05 (d, 1H), 7.32 - 7.42 (m, 3H), 7.50 - 7.53 (m, 1H), 7.62 - 7.64 (m, 1H), 7.69 - 7.74 (m, 1H), 8.28 - 8.31 (m, 2H), 9.45 (br. s, 1H). |
| I-1-131 |
| HPLC-MS: logP = 2,22; Masse (m/z): 409.0 (M+H)⁺; ¹H-NMR [CD₃CN] 7.05 (d, 1H), 7.15 - 7.20 (m, 1H), 7.34 - 7.38 (m, 1H), 7.42 - 7.49 (m, 2H), 7.70 - 7.75 (m, 1H), 7.90 - 7.92 (m, 1H), 8.29 - 8.31 (m, 2H), 9.40 (br. s, 1H). |
| I-1-132 |
| HPLC-MS: logP = 1,99; Masse (m/z):319.0 (M+H)⁺; ¹H-NMR [CD₃CN] 7.03 - 7.07 (m, 3H), 7.35 - 7.39 (m, 1H), 7.45 - 7.52 (m, 1H), 7.70-7.75 (m, 1H), 8.28 - 8.31 (m, 2H), 9.62 (br. s, 1H). |
| I-1-133 |
| HPLC-MS: logP = 1,83; Masse (m/z):352.0 (M+H)⁺; ¹H-NMR [CD₃CN] 6.99 (d, 1H), 7.36 - 7.40 (m, 1H), 7.69 - 7.75 (m, 2H), 8.07 - 8.09 (m, 1H), 8.30 - 8.31 (m, 2H), 8.79 - 8.80 (m, 1H), 9.52 (br. s, 1H). |
| I-1-134 |
| HPLC-MS: logP = 2,93; Masse (m/z): 525.1 (M+H)⁺; ¹H-NMR [CD₃CN] 6.61 (d, 1H), 7.40 - 7.45 (m, 1H), 7.62 - 7.65 (m, 2H), 7.70 - 7.75 (m, 1H), 8.07 - 8.09 (m, 2H), 8.21 (d, 1H), 8.26 - 8.28 (m, 1H), 8.73 - 8.81 (m, 2H). |
| I-1-135 |
| HPLC-MS: logP = 2,41; Masse (m/z): 367.0 (M+H)⁺; ¹H-NMR [CD₃CN] 6.99 (d, 1H), 7.37 - 7.43 (m, 1H), 7.63 - 7.72 (m, 3H), 7.78 - 7.80 (m, 1H), 7.98-8.01 (m, 1H), 8.10 (d, 1H), 8.43-8.45 (m, 1H), 9.44 (br. s, 1H). |
| I-1-136 |
| HPLC-MS: logP = 2,21; Masse (m/z): 333.0 (M+H)⁺; ¹H-NMR [CD₃CN] 7.01 (d, 1H), 7.38 - 7.51 (m, 4H), 7.58 - 7.60 (m, 1H), 7.99 - 8.02 (m, 1H), 8.10 (d, 1H), 8.44-8.45 (m, 1H), 9.31 (br. s, 1H). |
| I-1-137 |
| HPLC-MS: logP = 2,23; Masse (m/z):376.9 (M+H)⁺; ¹H-NMR [DMSO-D₆] 6.95 (d, 1H), 7.38 - 7.42 (m, 1H), 7.45 - 7.49 (m, 1H), 7.52 - 7.55 (m, 2H), 7.68 - 7.70 (m, 1H), 8.19 - 8.22 (m, 1H), 8.26 (d, 1H), 8.52 - 8.53 (m, 1H), 11.28 (br. s, 1H). |
| I-1-138 |
| HPLC-MS: logP = 2,36; Masse (m/z): 424.9 (M+H)⁺; ¹H-NMR [CD₃CN] 7.01 (d, 1H), 7.18-7.22 (m, 1H), 7.38 - 7.41 (m, 1H), 7.45 - 7.51 (m, 2H), 7.92 - 7.94 (m, 1H), 8.00 - 8.02 (m, 1H), 8.11 (d, 1H), 8.44 - 8.46 (m, 1H), 9.29 (br. s, 1H). |
| I-1-139 |
| siehe Synthesebeispiel 11 |
| I-1-140 |
| HPLC-MS: logP = 2,48; Masse (m/z): 369.1 (M+H)⁺; ¹H-NMR [CD₃CN] 7.03 (d, 1H), 7.62 - 7.70 (m, 4H), 7.77 - 7.79 (m, 1H), 8.20 (d, 1H), 8.25 (d, 1H), 9.47 (br. s, 1H). |
| I-1-141 |
| HPLC-MS: logP = 2,72; Masse (m/z): 495.0 (M+H)⁺; ¹H-NMR [CD₃CN] 7.70 - 7.91 (m, 5H), 8.30 (s, 1H), 8.37 (s, 1H), 8.65 (br. s, 1H). |
| I-1-142 |
| HPLC-MS: logP = 2.97; Masse (m/z): 411,1 (M+H)⁺; ¹H-NMR [DMSO-D₆] 2.31 (s, 3H), 6.67 (d, 1H), 7.59 - 7.70 (m, 3H), 7.76 - 7.78 (m, 1H), 8.25 - 8.30 (m, 1H), 8.38 (d, 1H), 8.49 (d, 1H). |
| I-1-143 |
| HPLC-MS: logP = 2,32; Masse (m/z): 335.0 (M+H)⁺; ¹H-NMR [CD₃CN] 7.06 (d, 1H), 7.34 - 7.38 (m, 1H), 7.40 - 7.47 (m, 2H), 7.54 - 7.56 (m, 1H), 7.62 - 7.67 (m, 1H), 8.21 (d, 1H), 8.25 (d, 1H), 9.55 (br. s, 1H). |
| I-1-144 |
| HPLC-MS: logP = 2,36; Masse (m/z): 378.9 (M+H)⁺; ¹H-NMR [CD₃CN] 7.05 (d, 1H), 7.34 - 7.38 (m, 1H), 7.41 - 7.45 (m, 1H), 7.52 - 7.54 (m, 1H), 7.64 - 7.69 (m, 2H), 8.21 (d, 1H), 8.26 (d, 1H), 9.42 (br. s, 1H). |
| I-1-145 |
| HPLC-MS: logP = 2,45; Masse (m/z): 426.9 (M+H)⁺; ¹H-NMR [CD₃CN] 7.05 (d, 1H), 7.15 - 7.19 (m, 1H), 7.42 - 7.48 (m, 2H), 7.64 - 7.69 (m, 1H), 7.89 - 7.91 (m, 1H), 8.22 (d, 1H), 8.26 (d, 1H), 9.42 (br. s, 1H). |
| I-1-146 |
| siehe Synthesebeispiel 9 |
| I-1-147 |
| HPLC-MS: logP = 2.00; Masse (m/z): 346,0 (M+H)⁺; ¹H-NMR [DMSO-D₆] 6.98 (d, 1H), 7.72 - 7.77 (m, 2H), 7.83 - 7.87 (m, 1H), 8.12 - 8.14 (m, 1H), 8.23 - 8.28 (m, 1H), 8.36 - 8.37 (m, 1H), 8.48 (d, 1H), 11.58 (s, 1H). |
| I-1-148 |
| HPLC-MS: logP =2.48; Masse (m/z): 317,1 (M+H)⁺; ¹H-NMR [DMSO-D₆] 6.96 - 7.03 (m, 3H), 7.44 - 7.48 (m, 1H), 8.06 - 8.09 (m, 1H), 8.25 - 8.30 (m, 1H), 8.35 - 8.36 (m, 1H), 8.49 (d, 1H), 11.08 (s, 1H), 11.89 (s, 1H). |
| I-1-149 |
| HPLC-MS: logP = 2.98; Masse (m/z): 401,0 (M+H)⁺; ¹H-NMR [DMSO-D₆] 6.95 (d, 1H), 7.62 - 7.68 (m, 2H), 7.75 - 7.81 (m, 2H), 8.23-8.29 (m, 1H), 8.36 (d, 1H), 8.48 - 8.49 (m, 1H), 11.46 (s, 1H). |
| I-1-150 |
| HPLC-MS: logP = 1.47; Masse (m/z): 368,1 (M+H)⁺; ¹H-NMR [DMSO-D₆] 6.84 (d, 1H), 7.58 - 7.60 (m, 1H), 7.65 - 7.70 (m, 3H), 8.11 (s, 1H), 8.19-8.27 (m, 2H), 8.44 (d, 1H), 8.88 (s, 1H), 11.22 (s, 1H). |
| I-1-151 |
| HPLC-MS: logP = 2,02; Masse (m/z): 370.1 (M+H)⁺; ¹H-NMR [CD₃CN] 6.99 - 7.02 (m, 1H), 7.65 - 7.73 (m, 2H), 8.08 - 8.10 (m, 1H), 8.22-8.28 (m, 2H), 8.80 - 8.82 (m, 1H), 9.44 (br. s, 1H). |
| I-1-152 |
| HPLC-MS: logP = 1,72; Masse (m/z): 336.0 (M+H)⁺; ¹H-NMR [CD₃CN] 7.04 (d, 1H), 7.43 - 7.46 (m, 1H), 7.65 - 7.70 (m, 1H), 7.97 - 7.99 (m, 1H), 8.22 (d, 1H), 8.27 (d, 1H), 8.48 - 8.49 (m, 1H), 9.40 (br. s, 1H). |
| I-1-153 |
| HPLC-MS: logP = 1.96; Masse (m/z): 369,1 (M+H)⁺; ¹H-NMR [DMSO-D₆] 6.97 (d, 1H), 7.77 (d, 1H), 8.24 - 8.29 (m, 1H), 8.37 - 8.38 (m, 1H), 8.49 (d, 1H), 8.99 (d, 1H), 9.07 (s, 1H), 11.70 (s, 1H). |
| I-1-154 |
| HPLC-MS: logP = 2.00; Masse (m/z): 370,1 (M+H)⁺; ¹H-NMR [DMSO-D₆] 6.99 (d, 1H), 8.24-8.30 (m, 1H), 8.40 - 8.41 (m, 1H), 8.49 (d, 1H), 9.39 (s, 1H), 9.56 (s, 1H), 11.80 (s, 1H). |
| I-1-155 |
| HPLC-MS: logP = 2.35; Masse (m/z): 392,0 (M+H)⁺; ¹H-NMR [DMSO-D₆] 3.23 - 3.26 (m, 2H), 4.37 - 4.39 (m, 2H), 6.83 (d, 1H), 8.23 - 8.29 (m, 1H), 8.32 (d, 1H), 8.47 (d, 1H), 11.51 (s, 1H). |
| I-1-156 |
| HPLC-MS: logP = 2.31; Masse (m/z): 371,1 (M+H)⁺; ¹H-NMR [DMSO-D₆] 3.70 (s, 3H), 6.95 (d, 1H), 7.40 (d, 1H), 7.85 (d, 1H), 8.22 - 8.29 (m, 2H), 8.47 (d, 1H), 10.71 (s, 1H). |
| I-1-157 |
| HPLC-MS: logP = 2.33; Masse (m/z): 356,0 (M+H)⁺; ¹H-NMR [DMSO-D₆] 2.42 (s, 3H), 6.96 (d, 1H), 7.42 (t, 1H), 8.24 - 8.30 (m, 1H), 8.37 (d, 1H), 8.48 - 8.49 (m, 1H), 11.51 (s, 1H). |
| I-1-158 |
| HPLC-MS: logP = 2.36; Masse (m/z): 391,0 (M+H)⁺; ¹H-NMR [DMSO-D₆] 3.86 (s, 3H), 6.92 (d, 1H), 8.24-8.29 (m, 1H), 8.35 (d, 1H), 8.47 - 8.48 (m, 1H), 11.21 (s, 1H). |
| I-1-159 |
| HPLC-MS: logP = 3.06; Masse (m/z): 375,0 (M+H)⁺; ¹H-NMR [DMSO-D₆]6.96 (d, 1H), 8.25-8.30 (m, 1H), 8.39 (d, 1H), 8.49 (d, 1H), 11.78 (s, 1H). |
| I-1-160 |
| HPLC-MS: logP = 1.91; Masse (m/z): 323,1 (M+H)⁺; ¹H-NMR [DMSO-D₆] 2.83 (s, 3H), 6.99 (d, 1H), 8.25 - 8.30 (m, 1H), 8.37 - 8.38 (m, 1H), 8.49 (d, 1H), 11.82 (s, 1H). |
| I-1-161 |
| HPLC-MS: logP = 2,75; Masse (m/z): 426,9 (M+H)⁺; ¹H-NMR [CD₃CN] 7.08 (d, 1H), 7.20 - 7.25 (m, 1H), 7.41 - 7.50 (m, 2H), 7.93 - 7.98 (m, 2H), 8.06 - 8.11 (m, 1H), 8.16 - 8.17 (m, 1H), 9.31 (s, 1H). |
| I-1-162 |
| HPLC-MS: logP = 2.78; Masse (m/z): 387,0 (M+H)⁺; ¹H-NMR [DMSO-D₆] 7.00 (d, 1H), 7.68 - 7.72 (m, 2H), 7.75 - 7.77 (m, 1H), 7.82 - 7.84 (m, 1H), 8.33 (d, 1H), 8.55 - 8.61 (m, 1H), 11.51 (s, 1H). |
| I-1-163 |
| HPLC-MS: logP = 2.64; Masse (m/z): 353,1 (M+H)⁺; ¹H-NMR [DMSO-D₆] 7.02 (d, 1H), 7.41 - 7.58 (m, 4H), 8.33 (d, 1H), 8.55 - 8.61 (m, 1H), 11.43 (s, 1H). |
| I-1-164 |
| siehe Synthesebeispiel 10 |
| I-1-165 |
| HPLC-MS: logP = 3.04; Masse (m/z):445,0 (M+H)⁺; ¹H-NMR [DMSO-D₆] 7.02 (d, 1H), 7.18-7.24 (m, 1H), 7.46 - 7.50 (m, 2H), 7.90 - 7.92 (m, 1H), 8.32 (d, 1H), 8.55 - 8.61 (m, 1H), 11.35 (s, 1H). |
| I-1-166 |
| HPLC-MS: logP = 2.51; Masse (m/z): 355,0 (M+H)⁺; ¹H-NMR [DMSO-D₆] 7.01 (d, 1H), 7.18 - 7.25 (m, 2H), 7.54 - 7.62 (m, 1H), 8.35 (d, 1H), 8.56 - 8.62 (m, 1H), 11.74 (s, 1H). |
| I-1-167 |
| HPLC-MS: logP = 3.88; Masse (m/z):388,0 (M+H)⁺; ¹H-NMR [DMSO-D₆] 7.00 (d, 1H), 7.80 - 7.84 (m, 1H), 8.00 - 8.01 (m, 1H), 8.19 - 8.22 (m, 1H), 8.34 - 8.35 (m, 1H), 8.39 - 8.45 (m, 1H), 8.56 - 8.62 (m, 1H), 8.84 (d, 1H), 11.68 (s, 1H). |
| I-1-168 |
| HPLC-MS: logP = 1,72; Masse (m/z): 344.0 (M+H)⁺; ¹H-NMR [DMSO-D₆] 7.02 (d, 1H), 7.42 - 7.58 (m, 4H), 7.70 - 7.75 (m, 1H), 8.64 (d, 1H), 8.84 (d, 2H), 11.44 (br. s, 1H). |
| I-1-169 |
| siehe Synthesebeispiel 12 |
| I-1-170 |
| HPLC-MS: logP = 2.46; Masse (m/z): 379,9 (M+H)⁺; ¹H-NMR [DMSO-D₆] 7.14 (d, 1H), 7.33 - 7.50 (m, 2H), 7.54 - 7.57 (m, 1H), 7.69 - 7.71 (m, 1H), 8.64 - 8.67 (m, 2H), 11.59 (s, 1H). |
| I-1-171 |
| HPLC-MS: logP = 2.29; Masse (m/z): 338,0 (M+H)⁺; ¹H-NMR [DMSO-D₆]7.12 (d, 1H), 7.20 - 7.26 (m, 2H), 7.55 - 7.63 (m, 1H), 8.66 - 8.69 (m, 2H), 11.91 (s, 1H). |
| I-1-173 |
| HPLC-MS: logP = 2.24; Masse (m/z): 352,0 (M+H)⁺; ¹H-NMR [DMSO-D₆]7.06 (d, 1H), 7.69 - 7.72 (m, 2H), 7.75 - 7.77 (m, 1H), 7.82 - 7.84 (m, 1H), 8.30 - 8.33 (m, 1H), 8.51 (d, 1H), 8.53 - 8.56 (m, 1H), 11.57 (s, 1H). |
| I-1-174 |
| siehe Synthesebeipiel 10d |
| I-1-175 |
| HPLC-MS: logP = 2.09; Masse (m/z):362,0 (M+H)⁺; ¹H-NMR [DMSO-D₆] 7.08 (d, 1H), 7.39 - 7.43 (m, 1H), 7.46 - 7.49 (m, 1H), 7.54 - 7.56 (m, 1H), 7.69 - 7.71 (m, 1H), 8.30 - 8.31 (m, 1H), 8.51 (d, 1H), 8.54 - 8.55 (m, 1H), 11.47 (s, 1H). |
| I-1-176 |
| HPLC-MS: logP = 2.18; Masse (m/z): 409,9 (M+H)⁺; ¹H-NMR [DMSO-D₆] 7.08 (d, 1H), 7.20 - 7.25 (m, 1H), 7.47 - 7.51 (m, 2H), 7.91 - 7.93 (m, 1H), 8.30 - 8.31 (m, 1H), 8.51 (d, 1H), 8.54 - 8.55 (m, 1H), 11.41 (s, 1H). |
| I-1-177 |
| HPLC-MS: logP = 1.94; Masse (m/z): 320,1 (M+H)⁺; ¹H-NMR [DMSO-D₆] 7.06 (d, 1H), 7.20 - 7.25 (m, 2H), 7.54 - 7.62 (m, 1H), 8.31 - 8.32 (m, 1H), 8.53 - 8.56 (m, 2H), 11.80 (s, 1H). |
| I-1-178 |
| HPLC-MS: logP = 1.77; Masse (m/z): 353,0 (M+H)⁺; ¹H-NMR [DMSO-D₆] 7.06 (d, 1H), 7.81 - 7.84 (m, 1H), 8.20 - 8.22 (m, 1H), 8.31 - 8.32 (m, 1H), 8.53 - 8.56 (m, 1H), 8.84 - 8.85 (m, 1H), 11.74 (s, 1H). |
| I-1-179: |
| HPLC-MS: logP = 2.13; Masse (m/z): 410,1 (M+H)⁺; ¹H-NMR(400,0 MHz, DMSO-D₆): |
| δ= 11,261(2,7);8,123(1,6);8,119(1,7);8,103(1,9);8,099(1,9);8,057(3,3);8,051(3,2);7,901(0,7);7,897(0,8);7,881(1,7);7,878(1,6);7,862(2,3);7,8 59(2,4);7,841(1,7);7,808(0,5);7,790(1,5);7,771(1,3);7,766(1,5);7,763(1,3);7,746(1,8);7,743(1,8);7,732(1,2);7,727(1,2);7,724(1,0);7,713(1,5); 7,705(1,9);7,694(0,6);7,686(1,2);7,656(2,0);7,653(1,9);7,636(1,8);7,633(1,6);6,861(3,5);6,855(3,5);3,456(16,0);3,324(82,6);2,675(0,4);2,670 (0,5);2,666(0,4);2,540(55,9);2,523(1,6);2,510(30,5);2,506(59,9);2,501(77,4);2,496(54,9);2,492(25,8);2,332(0,4);2,328(0,5);2,323(0,4);2,074( 0,5);0,008(0,6);0,000(15,6);-0,009(0,5) |
| I-1-180: |
| HPLC-MS: logP = 2.00; Masse (m/z): 411,1 (M+H)⁺; ¹H-NMR(400,0 MHz, DMSO-D₆): |
| δ= 11,337(2,8);8,938(1,5);8,928(1,5);8,390(1,5);8,372(1,6);8,129(1,6);8,125(1,7);8,109(1,9);8,105(1,9);8,074(3,3);8,067(3,2);7,908(0,7);7,9 04(0,8);7,889(1,7);7,885(1,7);7,870(1,2);7,866(1,1);7,821(1,0);7,808(1,1);7,801(1,0);7,789(1,0);7,776(1,2);7,773(1,3);7,757(1,7);7,754(1,8); 7,738(0,9);7,735(0,8);7,669(2,0);7,667(2,0);7,650(1,8);7,647(1,7);6,871(3,5);6,865(3,5);3,461(16,0);3,332(224,0);2,995(0,4);2,711(0,6);2,67 5(0,5);2,671(0,6);2,666(0,5);2,541(149,2);2,524(1,9);2,519(2,9);2,511(34,6);2,506(68,5);2,502(89,3);2,497(64,4);2,493(31,2);2,367(0,6);2,3 33(0,4);2,329(0,6);2,324(0,4);2,074(1,0);0,008(0,4);0,000(9,8) |
| I-1-181: |
| HPLC-MS: logP = 1.86; Masse (m/z): 378,1 (M+H)⁺;¹H-NMR(400,0 MHz, DMSO-D₆): |
| δ= 11,465(2,7);8,125(1,6);8,122(1,7);8,106(1,9);8,102(1,9);8,075(3,2);8,069(3,2);7,907(0,7);7,903(0,8);7,887(1,7);7,884(1,7);7,868(1,2);7,8 64(1,2);7,776(1,2);7,773(1,3);7,756(1,7);7,754(1,8);7,737(0,9);7,734(0,8);7,658(2,0);7,656(2,0);7,639(1,8);7,636(1,7);7,631(0,5);7,614(0,7); 7,609(0,7);7,593(1,3);7,576(0,7);7,572(0,8);7,555(0,4);7,274(0,5);7,268(2,3);7,248(3,3);7,227(2,0);7,220(0,4);6,870(3,5);6,864(3,5);3,456(1 6,0);3,367(0,7);3,334(243,8);3,303(0,7);3,289(0,3);2,995(0,5);2,711(0,6);2,675(0,4);2,671(0,5);2,666(0,4);2,541(168,5);2,524(2,6);2,511(32, 7);2,506(63,5);2,502(82,5);2,497(60,3);2,493(30,1);2,368(0,6);2,333(0,4);2,328(0,5);2,324(0,4);2,074(0,7);0,000(1,9) |
| I-1-182: |
| HPLC-MS: logP = 2.00; Masse (m/z): 422,1 (M+H)⁺; ¹H-NMR(400,0 MHz, DMSO-D₆): |
| δ= 11,188(2,9);8,121(1,7);8,118(1,8);8,101(1,9);8,098(1,9);8,056(3,3);8,050(3,3);7,900(0,8);7,896(0,8);7,881(1,7);7,877(1,7);7,862(1,2);7,8 58(1,1);7,764(1,2);7,761(1,3);7,744(1,8);7,741(1,9);7,725(2,6);7,707(2,1);7,705(2,0);7,651(2,0);7,648(2,0);7,631(1,8);7,629(1,6);7,566(1,1); 7,561(1,3);7,547(2,1);7,543(2,1);7,512(1,0);7,509(1,1);7,493(2,0);7,490(1,9);7,475(1,0);7,472(0,9);7,442(1,3);7,437(1,3);7,423(1,5);7,418(1, 5);7,404(0,7);7,399(0,7);6,882(3,5);6,876(3,5);3,475(16,0);3,327(122,0);2,675(0,3);2,670(0,5);2,666(0,3);2,541(54,6);2,524(1,5);2,519(2,3); 2,510(28,0);2,506(55,8);2,501(72,8);2,497(52,0);2,492(24,5);2,332(0,3);2,328(0,5);2,323(0,3);2,074(0,5);0,000(6,9) |
| I-1-183: |
| HPLC-MS: logP = 2.64; Masse (m/z): 384,9 (M+H)⁺; ¹H-NMR [DMSO-D₆] 6.93 (d, 1H), 7.67 - 7.84 (m, 4H),8.20 (d, 1H), 8.40 - 8.42 (m, 1H), 8.61 - 8.62 (m, 1H), 11.37 (s, 1H). |
| I-1-184: |
| HPLC-MS: logP = 2,10; Masse (m/z): 468,0 (M+H)⁺; ¹H-NMR(400,0 MHz, DMSO-D₆): |
| δ= 11,129(3,2);8,122(1,8);8,118(1,8);8,102(2,0);8,099(2,0);8,055(3,3);8,048(3,3);7,945(2,2);7,925(2,5);7,900(0,8);7,896(0,8);7,881(1,8);7,8 77(1,7);7,861(1,3);7,858(1,1);7,763(1,3);7,760(1,3);7,743(2,0);7,741(2,0);7,724(0,9);7,721(0,9);7,656(2,2);7,637(1,9);7,522(0,5);7,503(1,8); 7,485(4,3);7,471(0,7);7,251(1,0);7,244(1,0);7,234(1,1);7,231(1,2);7,224(1,0);7,215(0,9);7,208(0,8);6,880(3,4);6,874(3,4);3,482(16,0);3,335( 303,3);2,675(0,5);2,671(0,6);2,666(0,5);2,541(52,4);2,524(2,5);2,511(38,8);2,506(74,3);2,502(95,3);2,497(68,5);2,493(33,2);2,333(0,5);2,32 9(0,6);2,324(0,4);2,074(0,8);0,000(2,6) |
| I-1-185: |
| HPLC-MS: logP = 2,16; Masse (m/z): 364,1 (M+H)⁺; ¹H-NMR(400,0 MHz, DMSO-D₆): |
| δ= 10,164(2,5);8,315(0,6);7,941(1,2);7,938(1,2);7,921(1,4);7,918(1,4);7,901(2,0);7,895(1,9);7,860(0,5);7,841(1,3);7,822(0,9);7,730(0,9);7,7 11(1,3);7,691(0,5);7,644(1,6);7,624(1,3);6,857(3,0);6,850(3,0);5,756(0,5);3,667(14,4);3,321(243,6);2,689(0,7);2,680(0,7);2,675(1,5);2,670(2 ,1);2,666(1,4);2,661(0,7);2,524(6,3);2,519(10,2);2,510(112,4);2,506(224,0);2,501(294,8);2,497(210,2);2,492(98,1);2,360(15,9);2,337(0,8);2, 333(1,5);2,328(2,0);2,324(1,4);2,319(0,7);2,258(16,0);1,259(0,5);1,235(0,4);0,146(0,7);0,008(6,5);0,000(180,4);-0,009(5,4);-0,150(0,7) |
| I-1-186: |
| HPLC-MS: logP = 2,96; Masse (m/z): 411,9 (M+H)⁺;¹H-NMR(400,0 MHz, DMSO-D₆): |
| δ=11,178(12,3);7,967(10,7);7,961(10,7);7,945(6,5);7,927(7,4);7,925(7,5);7,868(2,8);7,866(2,8);7,848(6,7);7,829(4,5);7,740(4,7);7,721(7,0); 7,699(9,7);7,696(8,7);7,679(9,4);7,676(9,2);7,655(8,3);7,635(6,8);7,547(5,6);7,542(6,3);7,528(9,4);7,524(9,6);7,482(4,4);7,479(4,7);7,463(9, 2);7,460(8,8);7,445(5,1);7,442(4,7);7,430(0,3);7,427(0,3);7,416(6,1);7,411(6,1);7,396(7,1);7,392(7,0);7,377(3,4);7,373(3,1);6,917(16,0);6,91 0(15,8);5,756(9,8);4,038(0,7);4,020(0,7);3,325(108,8);2,675(0,6);2,670(0,8);2,666(0,6);2,524(2,6);2,510(44,5);2,506(89,2);2,501(117,8);2,4 97(85,2);2,492(40,8);2,333(0,6);2,328(0,8);2,323(0,6);1,989(3,2);1,397(2,1);1,336(1,4);1,299(0,4);1,259(0,6);1,250(1,8);1,235(0,6);1,192(0, 9);1,175(1,8);1,159(0,7);1,157(1,0);0,991(0,6);0,008(2,1);0,000(59,3);-0,009(1,9) |
| I-1-187: |
| HPLC-MS: logP = 3,41; Masse (m/z): 411,9(M+H)⁺; ¹H-NMR(601,6 MHz, CD₃CN): |
| δ= 9,253(3,0);9,237(0,9);7,894(11,2);7,889(11,3);7,685(6,3);7,684(6,5);7,672(7,6);7,669(13,4);7,665(12,6);7,624(0,4);7,623(0,4);7,612(0,5); 7,610(0,5);7,553(5,4);7,550(5,7);7,541(7,1);7,538(7,4);7,532(0,4);7,520(9,4);7,506(16,0);7,470(10,6);7,468(5,7);7,466(13,4);7,456(13,7);7,4 54(9,0);7,452(6,7);7,443(4,4);7,441(4,2);7,415(0,5);7,413(0,5);7,400(5,0);7,397(4,9);7,387(5,8);7,384(5,7);7,374(3,3);7,371(3,2);7,302(0,4); 7,277(0,4);6,987(12,3);6,983(12,1);2,185(0,7);2,183(0,9);2,174(405,2);2,056(0,4);2,052(0,6);2,048(0,4);1,973(1,1);1,966(3,4);1,958(2,7);1,9 54(3,3);1,950(35,9);1,946(67,5);1,942(102,0);1,938(69,7);1,934(34,2);1,929(1,0);1,925(0,4);1,831(0,4);1,827(0,6);1,823(0,4);1,436(14,3);1, 268(0,4);1,216(1,3);1,204(2,5);1,192(1,2);1,029(1,1);1,017(2,2);1,005(1,1);0,005(0,8);0,000(27,7);-0,006(0,7) |
| I-1-188: |
| HPLC-MS: logP = 3,38; Masse (m/z): 366,0(M+H)⁺;¹H-NMR(601,6MHz, CD₃CN): |
| δ= 9,307(3,0);9,242(0,4);7,891(11,4);7,886(11,5);7,663(11,7);7,660(12,1);7,590(5,4);7,588(5,6);7,578(6,5);7,575(6,6);7,514(9,2);7,509(3,8); 7,507(4,4);7,500(16,0);7,496(8,8);7,494(8,8);7,482(4,6);7,479(4,6);7,470(6,7);7,465(12,1);7,462(9,7);7,456(3,8);7,454(3,8);7,451(5,9);7,447 (5,7);7,443(0,5);7,441(0,4);7,421(5,2);7,419(5,1);7,409(7,1);7,407(6,9);7,402(0,6);7,396(3,1);7,394(3,0);7,390(0,4);7,386(0,4);6,990(12,1);6, 986(12,0);5,449(8,5);2,189(0,5);2,177(268,1);2,052(0,4);1,973(1,0);1,966(2,3);1,958(1,8);1,954(2,3);1,950(24,2);1,946(44,6);1,942(67,1);1, 938(46,0);1,934(22,7);1,929(0,6);1,827(0,4);1,435(1,7);1,266(0,4);1,215(0,4);1,210(0,7);1,203(0,6);1,198(1,4);1,191(0,3);1,186(0,7);1,024(0 ,7);1,012(1,4);1,000(0,7);0,005(0,5);0,000(17,8);-0,006(0,5) |
| I-1-189: |
| HPLC-MS: logP = 3,01; Masse (m/z): 334,0(M+H)⁺;¹H-NMR(400,0 MHz, CD₃CN): |
| δ= 9,336(3,6);8,042(7,0);8,036(12,5);8,030(7,0);7,598(6,8);7,596(6,9);7,580(10,1);7,576(11,3);7,567(3,0);7,559(6,2);7,543(2,7);7,539(4,2);7 ,534(2,6);7,516(2,9);7,512(4,1);7,496(12,1);7,492(16,0);7,487(8,4);7,473(10,4);7,469(9,4);7,453(4,5);7,449(4,1);7,429(8,0);7,424(7,2);7,410 (8,8);7,406(8,5);7,392(4,1);7,388(3,8);7,294(1,5);7,280(2,0);7,273(4,9);7,270(5,0);7,262(5,7);7,259(6,1);7,254(13,3);7,246(4,5);7,238(8,6);7, 232(4,9);7,225(2,3);7,221(4,3);7,216(4,5);7,200(1,6);7,195(1,3);7,042(12,4);7,035(12,2);5,447(1,9);2,155(42,5);1,964(1,9);1,958(2,5);1,952( 17,5);1,946(32,4);1,940(45,1);1,934(31,1);1,927(16,0);1,376(0,5);1,372(1,2);1,339(0,5);1,284(0,7);1,276(1,4);1,263(0,6);0,008(1,6);0,000(4 2,7);-0,009(1,3) |
| I-1-190: |
| HPLC-MS: logP = 3,09; Masse (m/z): 425,9(M+H)⁺;¹H-NMR(400,0 MHz, DMSO-D₆): |
| δ= 11,255(8,9);8,316(1,2);8,233(4,7);8,226(8,6);8,220(4,6);8,202(0,4);8,196(0,3);7,927(6,7);7,907(7,5);7,612(2,3);7,595(3,8);7,591(4,0);7,5 74(2,6);7,503(1,2);7,483(6,9);7,476(9,7);7,469(16,0);7,458(4,5);7,441 (2,3);7,437(2,8);7,433(2,8);7,415(1,7);7,411 (1,6);7,392(1,8);7,388(1,9) ;7,372(3,3);7,367(2,8);7,357(2,5);7,353(2,9);7,336(0,9);7,332(1,0);7,250(0,5);7,237(3,0);7,230(3,1);7,222(3,1);7,217(3,7);7,215(3,6);7,210(2 ,9);7,203(2,7);7,195(2,3);6,989(9,2);6,983(8,9);4,027(0,3);4,008(0,3);3,322(248,2);2,943(0,5);2,675(2,7);2,671(3,6);2,666(2,7);2,607(0,4);2, 541(3,1);2,506(397,8);2,502(510,3);2,497(379,1);2,333(2,6);2,328(3,5);2,324(2,6);1,989(0,7);1,398(12,3);1,257(0,4);1,239(0,8);1,222(0,4);1 ,175(0,4);0,000(37,9);-0,008(1,8) |
| I-1-191: |
| HPLC-MS: logP = 4,04; Masse (m/z): 386,1(M+H)⁺;¹H-NMR(400,0 MHz, DMSO-D₆): |
| δ= 11,402(13,3);8,316(0,4);8,187(7,5);8,181(14,2);8,174(7,3);7,937(2,9);7,918(3,2);7,909(5,4);7,891(6,2);7,882(3,9);7,875(5,0);7,866(4,6);7 ,856(3,8);7,844(8,7);7,823(9,8);7,790(2,7);7,771(7,5);7,753(6,4);7,719(5,7);7,700(7,2);7,684(11,0);7,666(6,2);6,951(16,0);6,944(15,9);3,384 (0,5);3,333(420,8);3,299(0,5);2,677(0,9);2,672(1,2);2,668(0,8);2,542(49,0);2,525(5,4);2,512(74,4);2,507(143,2);2,503(184,3);2,498(132,1);2 ,494(64,5);2,334(0,9);2,329(1,1);2,325(0,8);1,234(0,5);0,000(0,4) |
| I-1-192: |
| HPLC-MS: logP = 3,42; Masse (m/z): 387,1(M+H)⁺: ¹H-NMR(400,0 MHz, DMSO-D₆): |
| δ= 11,569(10,2);8,852(5,8);8,842(5,8);8,315(0,4);8,200(16,0);8,194(6,4);8,182(6,2);7,941(2,1);7,923(2,4);7,913(4,0);7,896(4,4);7,882(3,9);7 ,872(3,0);7,868(2,9);7,861(2,7);7,853(3,7);7,845(5,3);7,833(6,9);7,825(4,7);7,813(4,4);6,956(12,5);6,950(12,4);3,386(0,3);3,332(293,6);2,67 6(0,7);2,672(1,0);2,667(0,7);2,542(30,9);2,525(3,6);2,512(68,7);2,507(129,8);2,503(163,2);2,498(115,5);2,494(55,1);2,338(0,4);2,334(0,8);2 ,330(1,0);2,325(0,7);1,235(0,5);0,000(0,5) |
| I-1-193: |
| HPLC-MS: logP = 3,73; Masse (m/z): 354,0(M+H)⁺; ¹H-NMR(400,0 MHz, DMSO-D₆): |
| δ= 11,618(12,2);8,316(0,4);8,204(7,1);8,197(13,3);8,191(6,9);7,940(2,6);7,922(3,0);7,912(5,9);7,894(6,2);7,885(4,5);7,867(4,3);7,862(4,5);7 ,842(2,6);7,615(1,6);7,598(3,4);7,594(3,2);7,577(6,3);7,560(3,4);7,556(3,8);7,539(1,7);7,250(2,2);7,244(11,1);7,223(16,0);7,203(9,3);7,196( 2,0);6,956(15,3);6,949(15,1);3,367(0,5);3,330(314,3);3,297(0,3);2,712(0,7);2,676(0,8);2,672(1,0);2,668(0,7);2,542(179,6);2,525(3,3);2,511( 63,1);2,507(122,4);2,503(157,5);2,498(113,9);2,494(55,3);2,465(0,3);2,368(0,7);2,334(0,8);2,329(1,0);2,325(0,8);1,234(0,6);0,000(0,6) |
| I-1-194: |
| HPLC-MS: logP = 3,99; Masse (m/z): 396,0(M+H)⁺; ¹H-NMR(400,0 MHz, DMSO-D₆): |
| δ= 11,299(13,9);8,315(0,4);8,185(7,8);8,179(14,6);8,172(7,5);7,935(3,0);7,917(3,4);7,908(5,6);7,890(7,9);7,880(3,8);7,871(5,1);7,862(6,1);7 ,852(3,5);7,843(4,6);7,824(2,8);7,708(8,9);7,706(9,0);7,689(10,4);7,686(10,2);7,541(5,2);7,537(6,2);7,523(10,5);7,518(10,7);7,493(5,3);7,49 1(5,6);7,475(10,1);7,472(9,7);7,456(5,1);7,454(4,7);7,429(6,4);7,424(6,4);7,410(7,6);7,405(7,5);7,391(3,5);7,386(3,1);6,967(16,0);6,961(15, 7);3,385(0,3);3,368(0,8);3,332(495,1);3,300(0,9);3,284(0,4);2,996(0,4);2,711(1,4);2,676(0,9);2,671(1,2);2,667(0,9);2,561(1,9);2,542(342,9); 2,524(5,0);2,511(77,3);2,507(151,1);2,502(196,3);2,498(140,9);2,493(68,1);2,368(1,5);2,333(0,9);2,329(1,2);2,324(0,9);1,234(0,7);0,000(0,5 ) |
| I-1-195: |
| HPLC-MS: logP = 3,94; Masse (m/z): 352,1(M+H)⁺; ¹H-NMR(400,0 MHz, DMSO-D₆): |
| δ= 11,314(13,5);8,315(0,4);8,187(7,7);8,180(14,2);8,174(7,3);7,936(2,9);7,918(3,3);7,908(5,5);7,891(7,1);7,881(3,8);7,873(5,0);7,864(5,6);7 ,853(3,5);7,845(4,6);7,825(2,7);7,573(6,8);7,569(7,4);7,555(15,1);7,551(10,7);7,537(12,1);7,535(12,5);7,517(5,4);7,513(5,6);7,499(9,3);7,49 4(7,7);7,479(5,0);7,474(4,1);7,453(7,5);7,450(7,2);7,435(9,2);7,432(8,9);7,417(3,4);7,413(3,1);6,970(16,0);6,964(15,7);3,384(0,5);3,331(472 ,9);3,313(1,8);3,301(0,6);2,996(0,4);2,712(1,6);2,676(1,0);2,671(1,4);2,667(1,0);2,662(0,5);2,594(0,4);2,584(0,5);2,562(2,3);2,542(380,9);2, 511(86,0);2,507(165,9);2,502(213,6);2,498(151,8);2,493(71,8);2,368(1,6);2,334(1,0);2,329(1,4);2,325(1,0);1,234(0,7);0,000(0,5) |
| I-1-196: |
| HPLC-MS: logP = 4,10; Masse (m/z): 444,0(M+H)⁺; ¹H-NMR(400,0 MHz, DMSO-D₆): |
| δ= 11,240(10,7);8,315(0,5);8,185(5,8);8,179(11,0);8,173(5,7);7,936(2,7);7,926(8,3);7,918(3,1);7,908(11,7);7,891(5,7);7,881(2,9);7,873(3,7); 7,864(4,5);7,853(2,7);7,845(3,4);7,825(2,1);7,504(1,9);7,502(2,0);7,485(6,8);7,483(6,6);7,468(16,0);7,462(11,0);7,449(2,9);7,443(1,4);7,238 (4,1);7,232(3,9);7,221(4,4);7,218(4,8);7,215(4,6);7,212(4,1);7,202(3,6);7,196(3,3);6,967(12,7);6,961(12,6);3,380(0,6);3,363(1,2);3,331(509, 7);3,306(1,1);3,294(0,5);2,676(1,0);2,671(1,4);2,667(1,0);2,542(34,6);2,525(5,0);2,520(8,0);2,511(84,1);2,507(166,0);2,502(216,0);2,498(15 3,6);2,493(72,5);2,334(1,0);2,329(1,3);2,324(0,9);1,235(0,6);0,000(0,5) |
| I-1-197: |
| HPLC-MS: logP = 3,51; Masse (m/z): 400,0(M+H)⁺; ¹H-NMR(400,0 MHz, DMSO-D₆): |
| δ= 11,331(9,0);8,315(0,4);8,117(10,9);8,111(10,8);7,890(9,6);7,884(9,7);7,836(4,6);7,817(5,7);7,782(1,6);7,764(4,7);7,745(4,2);7,711(3,7);7 ,686(7,0);7,667(4,2);7,645(5,1);7,623(16,0);7,608(10,7);7,602(9,7);7,586(3,2);7,581(3,4);6,913(12,1);6,907(12,0);3,323(83,8);2,675(0,6);2,6 71(0,8);2,666(0,5);2,541(0,4);2,524(1,8);2,519(2,9);2,511(43,0);2,506(88,2);2,502(116,1);2,497(81,2);2,493(37,2);2,333(0,5);2,328(0,7);2,3 24(0,5);1,398(0,8);0,008(0,7);0,000(22,7);-0,009(0,6) |
| I-1-198: |
| HPLC-MS: logP = 3,25; Masse (m/z): 368,0(M+H)⁺; ¹H-NMR(400,0 MHz, DMSO-D₆): |
| δ= 11,549(8,6);8,315(0,8);8,130(10,9);8,124(10,7);7,895(9,5);7,889(9,7);7,649(4,9);7,627(16,0);7,612(10,9);7,606(10,8);7,590(5,3);7,585(5, 5);7,573(1,8);7,568(4,5);7,564(1,6);7,552(2,3);7,547(2,7);7,531(1,2);7,244(1,4);7,238(8,0);7,218(10,9);7,197(6,6);7,190(1,3);6,917(12,1);6,9 11(11,9);3,322(326,0);3,300(0,5);2,680(0,7);2,675(1,4);2,671(2,0);2,666(1,4);2,662(0,6);2,541(1,0);2,524(4,8);2,519(8,1);2,511(114,4);2,50 6(232,5);2,502(305,0);2,497(213,2);2,493(97,5);2,338(0,7);2,333(1,4);2,328(1,9);2,324(1,4);2,319(0,6);1,989(0,4);1,398(12,4);1,120(0,4);1, 104(0,4);0,008(2,0);0,000(60,1);-0,009(1,8) |
| I-1-199: |
| HPLC-MS: logP = 3,44; Masse (m/z): 411,9(M+H)⁺; ¹H-NMR(400,0 MHz, CD₃CN): |
| δ= 9,307(2,9);7,892(9,9);7,885(10,0);7,682(5,7);7,680(5,7);7,661(16,0);7,656(11,9);7,613(0,4);7,594(0,4);7,590(0,4);7,550(4,2);7,546(4,7);7 ,531(6,4);7,527(6,8);7,518(6,3);7,496(13,7);7,467(12,5);7,462(10,0);7,448(8,0);7,446(10,9);7,440(4,9);7,430(4,1);7,427(3,9);7,400(4,7);7,39 6(4,7);7,381(5,7);7,376(5,5);7,362(2,6);7,357(2,4);6,985(10,2);6,979(10,1);5,447(2,3);2,205(116,2);2,133(0,4);2,108(0,4);1,977(0,5);1,972(1 ,8);1,965(44,2);1,959(2,3);1,953(17,7);1,947(33,4);1,941(47,5);1,935(33,4);1,928(17,4);1,435(0,8);1,268(0,8);1,221(0,3);1,203(0,6);1,135(0, 4) |
| I-1-200: |
| HPLC-MS: logP = 3,40; Masse (m/z): 366,0(M+H)⁺; ¹H-NMR(400,0 MHz, CD₃CN): |
| δ= 9,281(3,7);7,892(11,4);7,886(11,9);7,666(11,8);7,661(13,0);7,616(0,4);7,600(5,5);7,597(6,0);7,582(6,7);7,578(7,3);7,527(7,3);7,519(3,4); 7,515(4,4);7,505(16,0);7,499(11,0);7,495(11,6);7,491(7,4);7,487(6,2);7,472(15,9);7,466(13,3);7,450(7,4);7,445(5,9);7,432(5,7);7,427(5,6);7, 413(7,0);7,409(7,0);7,395(2,9);7,391(2,9);7,377(0,4);6,988(11,5);6,982(11,8);5,447(3,2);3,282(1,3);3,268(1,4);2,193(194,5);2,133(1,1);2,12 3(0,3);2,120(0,4);2,114(0,6);2,108(0,7);2,102(0,5);2,095(0,3);1,976(1,7);1,965(126,9);1,953(32,6);1,947(59,7);1,940(84,0);1,934(60,0);1,92 8(32,1);1,894(0,4);1,792(0,8);1,775(0,4);1,769(0,6);1,763(0,4);1,436(5,1);1,292(0,5);1,269(1,3);1,200(0,4);1,101(0,5);1,014(0,3);0,911(0,4); |
| 0,000(1,1) |
| I-1-201: |
| HPLC-MS: logP = 4,15; Masse (m/z): 396,1(M+H)⁺; ¹H-NMR(400,0 MHz, DMSO-D₆): |
| δ= 11,145(5,8);7,835(3,0);7,816(3,7);7,780(1,1);7,762(3,1);7,744(2,5);7,708(2,4);7,691(3,7);7,676(1,9);7,669(3,9);7,659(4,1);7,655(3,9);7,6 47(2,2);7,641(2,9);7,632(1,6);7,610(1,5);7,603(1,5);7,588(1,8);7,584(2,0);7,581(2,0);7,577(1,8);7,561(1,4);7,555(1,4);7,307(1,2);7,304(1,1); 7,286(2,0);7,268(1,0);7,265(1,1);7,261(1,0);6,726(8,9);3,399(0,4);3,389(0,4);3,361(2,2);3,339(401,4);3,298(0,3);2,717(0,7);2,678(1,0);2,673 (0,8);2,571(0,7);2,548(146,2);2,513(121,0);2,508(156,2);2,504(114,3);2,482(6,2);2,463(6,0);2,444(2,0);2,374(0,7);2,340(0,8);2,335(1,0);2,3 31(0,8);2,090(0,6);1,955(0,7);1,243(0,5);1,165(7,7);1,146(16,0);1,127(7,4);1,115(0,4) |
| I-1-202: |
| HPLC-MS: logP=3,78; Masse (m/z):382,1(M+H)⁺; ¹H-NMR(400,0MHz,DMSO-D₆): |
| δ= 11,124(4,3);7,827(2,4);7,807(3,1);7,772(1,0);7,753(2,5);7,735(2,1);7,700(1,9);7,680(3,1);7,663(2,0);7,657(2,9);7,650(3,4);7,642(2,4);7,6 33(2,8);7,620(1,1);7,603(1,0);7,596(1,1);7,576(1,6);7,554(1,1);7,547(1,0);7,301(0,9);7,297(0,9);7,280(1,6);7,261(0,8);7,258(0,8);7,254(0,8); 6,710(6,2);3,332(91,6);2,541(26,7);2,506(38,9);2,502(48,6);2,498(35,5);2,329(0,3);2,165(16,0) |
| I-1-203: |
| HPLC-MS: logP = 3,19; Masse (m/z): 383,1(M+H)⁺; ¹H-NMR(400,0 MHz, DMSO-D₆): |
| δ= 11,293(4,8);8,835(2,4);8,825(2,5);8,166(2,3);8,149(2,5);7,828(2,1);7,816(2,1);7,809(2,0);7,797(1,9);7,686(1,0);7,671(1,1);7,664(2,1);7,6 49(2,1);7,642(1,2);7,627(1,1);7,607(1,1);7,600(1,1);7,584(1,3);7,580(1,6);7,574(1,3);7,558(1,1);7,551(1,1);7,307(0,8);7,304(0,9);7,300(0,8); 7,283(1,6);7,278(1,5);7,265(0,8);7,262(0,8);7,258(0,7);6,716(6,5);3,333(82,7);2,712(0,5);2,672(0,3);2,542(109,4);2,525(1,5);2,507(37,6);2,5 03(47,7);2,498(34,5);2,369(0,5);2,330(0,4);2,173(16,0) |
| I-1-204: |
| HPLC-MS: logP = 3,58; Masse (m/z): 397,1(M+H)⁺; ¹H-NMR(400,0 MHz, DMSO-D₆): |
| δ= 11,306(5,6);8,836(3,0);8,825(2,9);8,169(2,8);8,150(3,0);7,831(2,6);7,819(2,6);7,812(2,4);7,800(2,3);7,693(1,2);7,677(1,4);7,670(2,5);7,6 55(2,5);7,648(1,5);7,633(1,3);7,608(1,3);7,601(1,4);7,585(1,6);7,581(1,9);7,579(1,9);7,575(1,7);7,559(1,3);7,552(1,3);7,307(1,0);7,304(1,1); 7,300(1,0);7,283(1,9);7,278(1,8);7,264(0,9);7,261(1,0);7,257(0,9);6,725(8,7);3,328(102,1);2,712(0,7);2,676(0,4);2,672(0,6);2,667(0,4);2,582 (0,3);2,542(153,4);2,511(34,2);2,507(65,4);2,502(85,3);2,498(62,1);2,483(7,4);2,464(6,0);2,445(2,0);2,368(0,7);2,333(0,4);2,329(0,5);2,325( 0,4);1,159(7,8);1,140(16,0);1,122(7,4) |
| I-1-205: |
| HPLC-MS: Masse (m/z): 336,1(M+H)⁺; ¹H-NMR(601,6 MHz, DMSO-D₆): |
| δ= 11,203(2,4);8,144(8,6);8,140(15,6);8,136(8,6);7,803(3,2);7,793(3,9);7,788(6,8);7,779(8,6);7,773(4,9);7,766(8,2);7,755(7,1);7,741(3,4);7, 605(4,3);7,601(4,4);7,590(4,8);7,586(8,5);7,581(4,8);7,571(4,4);7,566(4,3);7,420(3,5);7,416(3,7);7,402(6,3);7,387(3,7);7,383(3,5);7,290(3,6) ;7,288(3,5);7,277(6,5);7,275(6,4);7,273(6,4);7,262(3,5);7,259(3,3);7,224(4,4);7,221(4,3);7,210(7,9);7,207(7,7);7,196(4,0);7,193(3,8);6,933(1 6,0);6,929(16,0);6,687(0,3);5,359(0,3);5,325(0,7);3,366(3290,1);2,998(0,5);2,619(3,5);2,616(4,8);2,613(3,5);2,544(8,8);2,525(8,2);2,522(10, 6);2,519(11,4);2,510(255,3);2,507(544,6);2,504(751,0);2,501(558,6);2,499(264,8);2,392(3,4);2,389(4,7);2,386(3,4);2,294(0,5);2,020(0,8);2,0 08(1,4);1,995(1,1);1,985(0,7);1,976(0,7);1,940(0,4);1,506(1,1);1,453(0,5);1,280(0,9);1,235(5,5);0,865(1,0);0,853(2,5);0,842(1,2);0,005(2,7); 0,000(80,1);-0,006(2,7) |
| I-1-206: |
| HPLC-MS: logP = 3,42; Masse (m/z): 350,1 (M+H)⁺; ¹H-NMR(400,0 MHz, DMSO-D₆): |
| δ= 11,338(4,4);7,685(1,0);7,670(1,1);7,663(2,2);7,648(2,2);7,641(1,2);7,626(1,1);7,608(1,1);7,601(1,2);7,594(0,7);7,585(1,4);7,578(2,6);7,5 74(2,2);7,557(2,7);7,552(2,0);7,540(1,1);7,535(1,3);7,519(0,6);7,308(0,8);7,305(0,9);7,301(0,8);7,298(0,8);7,285(1,5);7,279(1,4);7,266(0,8); 7,262(0,8);7,259(0,7);7,256(0,7);7,233(0,7);7,226(3,9);7,206(5,4);7,186(3,2);7,179(0,7);6,715(6,3);3,329(65,2);2,712(0,5);2,542(115,3);2,53 0(0,7);2,525(1,2);2,520(1,6);2,511(17,7);2,507(34,9);2,502(45,2);2,498(32,2);2,493(15,3);2,368(0,5);2,329(0,4);2,168(16,0) |
| I-1-207: |
| HPLC-MS: logP = 3,84; Masse (m/z): 364,1 (M+H)⁺; ¹H-NMR(400,0 MHz, DMSO-D₆): |
| δ= 11,351(5,0);7,689(1,1);7,674(1,3);7,667(2,4);7,652(2,4);7,645(1,4);7,630(1,3);7,608(1,3);7,601(1,4);7,596(0,9);7,585(1,7);7,579(3,0);7,5 75(2,7);7,558(3,5);7,553(2,0);7,541(1,3);7,537(1,5);7,520(0,7);7,307(1,0);7,304(1,0);7,300(1,0);7,284(1,7);7,278(1,7);7,265(0,9);7,262(0,9); 7,258(0,9);7,235(0,8);7,228(4,3);7,208(6,1);7,188(3,6);7,180(0,7);6,725(8,4);3,327(84,3);3,308(0,4);2,711(0,6);2,676(0,4);2,671(0,5);2,667( 0,3);2,565(0,6);2,542(146,2);2,511(28,7);2,507(55,8);2,502(72,0);2,498(51,9);2,493(25,2);2,477(5,6);2,458(5,5);2,439(1,9);2,368(0,6);2,333 (0,4);2,329(0,5);2,324(0,4);1,154(7,7);1,135(16,0);1,116(7,4) |
| I-1-208: |
| HPLC-MS: logP = 3,63; Masse (m/z): 392,0 (M+H)⁺; ¹H-NMR(400,0 MHz, DMSO-D₆): |
| δ= 11,020(4,4);7,691(2,9);7,672(4,2);7,657(1,4);7,650(2,2);7,635(2,3);7,628(1,3);7,613(1,2);7,603(1,1);7,597(1,1);7,581(1,3);7,574(1,6);7,5 70(1,3);7,554(1,1);7,547(1,1);7,510(1,3);7,505(1,6);7,491(3,7);7,486(3,8);7,477(2,0);7,475(2,0);7,459(3,1);7,456(2,9);7,440(1,4);7,438(1,3); 7,410(2,1);7,405(2,0);7,391(2,3);7,386(2,1);7,373(1,2);7,367(1,0);7,305(0,8);7,302(0,9);7,295(0,9);7,281(1,6);7,275(1,5);7,262(0,8);7,259(0, 8);7,255(0,8);6,723(6,2);3,328(209,9);3,298(0,3);3,292(0,4);2,711(0,6);2,676(0,6);2,671(0,8);2,666(0,6);2,559(0,8);2,541(146,5);2,524(2,9); 2,511(51,7);2,506(99,1);2,502(126,3);2,497(90,0);2,493(43,1);2,367(0,6);2,333(0,6);2,329(0,8);2,324(0,6);2,164(16,0);2,059(0,4);1,236(0,4) ;0,000(0,4) |
| I-1-209: |
| HPLC-MS: logP = 2,95; Masse (m/z): 378,0 (M+H)⁺; ¹H-NMR(400,0 MHz, CD₃CN): |
| δ= 9,270(0,3);9,223(3,6);7,952(7,8);7,946(14,9);7,940(7,9);7,765(3,7);7,750(4,1);7,743(7,9);7,728(7,9);7,720(4,3);7,705(4,2);7,694(9,4);7,6 92(9,1);7,675(10,8);7,672(10,5);7,596(0,3);7,589(0,3);7,564(6,7);7,560(7,2);7,545(10,0);7,541(10,5);7,482(5,6);7,479(5,9);7,463(11,8);7,46 |
| 0(11,4);7,444(6,6);7,441(5,9);7,411(8,0);7,407(8,0);7,391(9,0);7,387(8,7);7,373(4,4);7,368(4,0);7,206(4,2);7,199(4,8);7,183(4,5);7,176(8,8); 7,169(5,1);7,154(4,1);7,147(4,8);7,123(3,3);7,119(3,4);7,116(2,8);7,113(2,7);7,103(3,9);7,100(5,5);7,096(5,5);7,093(4,7);7,080(3,1);7,077(3, 1);7,074(2,7);7,070(2,6);6,992(16,0);6,985(15,7);2,207(0,5);2,199(0,7);2,191(1,4);2,174(186,7);2,170(344,6);2,168(275,4);2,167(301,3);2,13 3(0,5);2,120(0,5);2,114(0,8);2,108(1,3);2,101(0,8);2,095(0,5);1,964(34,9);1,958(4,1);1,952(68,7);1,946(134,9);1,940(198,0);1,934(134,1);1, 928(67,4);1,921(1,6);1,915(0,6);1,781(0,5);1,775(0,8);1,769(1,1);1,762(0,7);1,756(0,3);1,270(0,8);0,000(1,7) |
| I-1-210: |
| HPLC-MS: logP = 2,89; Masse (m/z): 395,0 (M+H)⁺; ¹H-NMR(400,0 MHz, DMSO-D₆): |
| δ= 11,199(4,5);8,471(3,0);8,466(3,2);8,459(3,2);8,454(3,1);7,959(2,9);7,954(3,0);7,940(3,4);7,935(3,1);7,680(1,0);7,664(1,1);7,657(2,2);7,6 42(2,1);7,635(1,2);7,620(1,1);7,607(1,1);7,600(1,1);7,584(1,3);7,580(1,5);7,578(1,5);7,574(1,3);7,556(3,9);7,551(1,4);7,544(3,2);7,538(3,0); 7,526(2,9);7,308(0,8);7,305(0,9);7,301(0,8);7,298(0,8);7,284(1,5);7,279(1,4);7,265(0,8);7,262(0,8);7,258(0,7);6,728(6,3);3,328(191,1);2,995 (0,4);2,711(0,7);2,676(0,6);2,671(0,8);2,667(0,6);2,572(0,4);2,565(0,5);2,541(178,2);2,524(2,8);2,511(47,3);2,507(94,0);2,502(122,0);2,497( 86,5);2,493(40,9);2,367(0,7);2,333(0,6);2,329(0,8);2,324(0,5);2,171(16,0);1,235(0,4);0,000(0,5) |
| I-1-211: |
| HPLC-MS:logP=2,91; Masse(m/z): 334,0 (M+H)⁺; ¹H-NMR(400,0 MHz, CD₃CN): |
| δ=9,251(3,9);7,952(8,2);7,945(15,2);7,939(8,1);7,766(3,8);7,751(4,1);7,743(7,8);7,728(7,9);7,720(4,3);7,706(4,1);7,607(7,5);7,603(7,2);7,5 88(9,5);7,584(9,5);7,524(3,7);7,520(5,2);7,504(13,1);7,501(14,7);7,496(9,3);7,492(8,5);7,479(12,6);7,474(10,8);7,459(5,8);7,454(4,8);7,438( 9,5);7,433(8,5);7,419(10,2);7,415(10,0);7,401(4,6);7,397(4,0);7,388(0,4);7,205(4,6);7,198(5,3);7,183(4,8);7,176(9,6);7,169(5,6);7,154(4,4);7 ,147(5,4);7,122(3,6);7,119(3,7);7,116(3,0);7,112(2,9);7,102(4,3);7,099(5,9);7,096(6,1);7,092(5,1);7,089(3,4);7,080(3,4);7,076(3,5);7,073(3,0 );7,069(2,8);6,995(16,0);6,988(15,6);5,447(1,9);4,068(0,5);4,050(0,5);2,213(0,5);2,203(0,7);2,175(222,4);2,170(346,5);2,169(324,8);2,147(0 ,6);2,133(0,4);2,120(0,5);2,114(0,8);2,108(1,1);2,101(0,8);2,095(0,3);1,976(0,5);1,972(2,7);1,965(30,8);1,958(4,1);1,953(65,1);1,946(125,9); 1,940(181,9);1,934(122,2);1,928(61,6);1,921(1,1);1,915(0,5);1,781(0,3);1,775(0,7);1,769(1,0);1,763(0,6);1,756(0,4);1,436(1,4);1,268(0,9);1, 221(0,7);1,204(1,3);1,186(0,6);0,000(1,8) |
| I-1-212: |
| HPLC-MS: logP = 3,58; Masse (m/z): 348,1 (M+H)⁺; ¹H-NMR(400,0 MHz, DMSO-D₆): |
| δ= 11,037(4,4);7,671(1,0);7,656(1,2);7,649(2,2);7,634(2,2);7,627(1,3);7,612(1,2);7,603(1,2);7,596(1,2);7,580(1,4);7,576(1,6);7,574(1,6);7,5 70(1,4);7,554(1,3);7,547(1,5);7,541(3,8);7,537(3,7);7,522(6,2);7,518(7,4);7,498(1,6);7,493(1,8);7,479(3,1);7,474(1,9);7,460(1,2);7,455(1,4); 7,437(2,4);7,434(2,4);7,419(2,9);7,416(2,5);7,400(0,8);7,397(1,0);7,305(0,8);7,302(0,9);7,298(0,9);7,295(0,8);7,281(1,5);7,276(1,5);7,262(0, 8);7,259(0,8);7,255(0,7);7,252(0,7);6,725(6,3);3,331(129,3);2,711(0,5);2,671(0,4);2,569(0,4);2,541(127,3);2,524(1,4);2,511(22,4);2,506(45, 1);2,502(59,2);2,497(42,4);2,493(20,2);2,367(0,5);2,329(0,4);2,324(0,3);2,164(16,0) |
| I-1-213: |
| HPLC-MS: logP = 3,99; Masse (m/z): 362,1 (M+H)⁺; ¹H-NMR(400,0 MHz, DMSO-D₆): |
| δ= 11,051(5,5);7,676(1,3);7,661(1,4);7,654(2,6);7,639(2,5);7,632(1,5);7,617(1,3);7,603(1,4);7,596(1,4);7,580(1,7);7,577(1,9);7,574(1,9);7,5 70(1,6);7,554(1,6);7,547(1,9);7,542(4,5);7,523(7,6);7,520(7,9);7,499(2,0);7,495(2,1);7,481(3,7);7,476(2,6);7,462(1,7);7,457(1,6);7,450(0,4); 7,440(2,8);7,436(2,7);7,421(3,4);7,418(2,9);7,402(1,1);7,400(1,1);7,305(1,0);7,301(1,1);7,297(1,1);7,281(1,9);7,275(1,8);7,262(1,0);7,259(1, 0);7,255(0,9);6,739(8,6);3,331(170,4);3,310(0,6);2,712(0,6);2,675(0,4);2,671(0,5);2,667(0,4);2,541(144,9);2,511(31,7);2,507(61,0);2,502(78 ,4);2,498(56,3);2,493(28,6);2,474(5,9);2,455(5,8);2,437(1,9);2,368(0,6);2,334(0,4);2,329(0,5);2,324(0,4);1,156(7,8);1,138(16,0);1,119(7,3);0 ,998(0,5) |
| I-1-214: |
| HPLC-MS: logP = 3,73; Masse (m/z): 440,1 (M+H)⁺; ¹H-NMR(400,0 MHz, DMSO-D₆): |
| δ= 10,966(4,7);7,912(3,2);7,894(3,4);7,675(1,0);7,660(1,1);7,653(2,1);7,638(2,1);7,631(1,4);7,616(1,1);7,601(1,1);7,595(1,1);7,578(1,4);7,5 74(1,6);7,552(1,1);7,545(1,1);7,488(1,0);7,486(1,0);7,469(2,9);7,451(2,8);7,449(2,7);7,438(3,5);7,433(4,0);7,419(1,7);7,414(1,5);7,304(0,9); 7,301(0,9);7,297(0,9);7,280(1,7);7,261(0,8);7,258(0,9);7,254(0,9);7,219(1,6);7,215(1,6);7,200(2,2);7,196(2,2);7,182(1,5);7,177(1,4);6,727(6, 4);3,332(58,6);2,711(0,4);2,541(83,7);2,524(0,8);2,511(13,1);2,506(25,4);2,502(32,7);2,497(23,4);2,493(11,2);2,368(0,4);2,167(16,0);2,120( 0,4);2,067(0,7) |
| I-1-215: |
| HPLC-MS: logP = 4,15; Masse (m/z): 454,0 (M+H)⁺; ¹H-NMR(400,0 MHz, DMSO-D₆): |
| δ= 10,985(6,3);7,921(4,2);7,901(4,5);7,688(1,2);7,673(1,5);7,666(2,6);7,651(2,6);7,644(1,7);7,629(1,5);7,610(1,5);7,603(1,6);7,583(2,4);7,5 81(2,4);7,561 (1,4);7,554(1,5);7,496(1,4);7,477(4,0);7,459(3,7);7,444(4,4);7,440(5,0);7,425(2,2);7,421 (1,8);7,307(1,3);7,304(1,4);7,287(2,4); 7,265(1,3);7,261(1,2);7,227(2,0);7,223(2,1);7,208(3,1);7,205(3,1);7,190(1,7);7,185(1,6);6,743(9,2);3,338(262,3);2,717(0,7);2,677(0,8);2,547 (136,3);2,512(96,7);2,508(122,2);2,504(95,1);2,482(7,1);2,463(6,5);2,445(2,3);2,374(0,7);2,335(0,8);2,091(0,6);2,026(0,7);1,241(0,4);1,168( 7,9);1,149(16,0);1,131(7,6);1,114(0,5) |
| I-1-217: |
| HPLC-MS: logP = 3,06; Masse (m/z): 383,0 (M+H)⁺; ¹H-NMR(400,0 MHz, DMSO-D₆): |
| δ= 11,451(6,6);8,316(0,4);8,254(3,6);8,248(6,4);8,242(3,4);8,156(2,6);8,134(3,5);8,115(2,6);7,984(0,5);7,965(0,6);7,961(0,6);7,942(0,6);7,9 32(0,7);7,925(1,3);7,918(0,7);7,843(3,6);7,824(4,6);7,790(1,3);7,772(3,6);7,754(3,2);7,721(2,8);7,701(3,5);7,685(5,4);7,667(3,0);6,979(7,7); 6,973(7,6);5,842(1,5);5,836(1,4);5,757(9,3);5,337(1,8);3,322(161,8);2,679(0,6);2,675(1,3);2,671(1,7);2,666(1,2);2,524(5,3);2,510(99,1);2,50 6(193,9);2,502(251,8);2,497(182,5);2,493(88,3);2,456(0,6);2,432(15,9);2,425(16,0);2,367(3,4);2,360(3,4);2,337(0,7);2,333(1,3);2,328(1,7);2 ,324(1,2);1,989(1,0);1,336(1,0);1,298(0,6);1,259(0,9);1,249(1,2);1,235(0,6);1,175(0,5);0,008(2,0);0,000(54,6);-0,008(1,9) |
| I-1-218: |
| HPLC-MS: logP = 2,28; Masse (m/z): 335,1 (M+H)⁺; ¹H-NMR(400,0 MHz, CD₃CN): |
| δ= 9,462(3,5);8,505(8,8);8,500(9,4);8,493(9,4);8,488(9,3);8,093(8,5);8,087(15,6);8,081(8,6);7,995(9,6);7,990(9,7);7,976(10,4);7,971(10,2);7 |
| ,593(3,7);7,585(4,0);7,577(4,2);7,569(7,4);7,561(4,2);7,553(3,9);7,545(3,8);7,475(11,1);7,463(11,2);7,456(10,7);7,444(10,1);7,371(3,8);7,35 9(4,0);7,348(4,8);7,342(4,4);7,336(5,0);7,330(4,2);7,319(4,6);7,307(4,5);7,105(2,6);7,097(4,8);7,088(3,4);7,086(3,6);7,082(3,2);7,077(5,5);7, 074(4,9);7,069(3,4);7,065(3,1);7,063(3,0);7,055(4,0);7,046(2,3);7,028(16,0);7,022(15,8);5,449(0,4);4,068(0,4);4,050(0,4);2,464(0,3);2,175(1 15,2);2,115(0,4);2,109(0,5);2,103(0,3);1,972(2,1);1,966(2,4);1,960(3,5);1,954(31,8);1,948(59,8);1,942(84,2);1,935(57,9);1,929(29,6);1,776( 0,3);1,770(0,5);1,764(0,3);1,436(0,7);1,372(5,4);1,340(0,7);1,284(1,0);1,276(6,2);1,221(0,5);1,204(0,9);1,186(0,5);0,146(0,6);0,008(5,5);0,0 00(139,7);-0,009(5,6);-0,150(0,6) |
| I-1-219: |
| HPLC-MS: logP = 3,25; Masse (m/z): 431,9 (M+H)⁺; ¹H-NMR(400,0 MHz, DMSO-D₆): |
| δ= 11,040(6,0);8,227(3,4);8,221(6,5);8,214(3,5);7,814(7,3);7,800(7,8);7,655(1,5);7,647(1,6);7,639(1,7);7,631(2,8);7,623(1,7);7,616(1,6);7,6 08(1,5);7,581(1,5);7,569(1,6);7,558(1,9);7,553(1,8);7,546(1,9);7,541(1,7);7,530(1,7);7,517(1,7);7,411(5,9);7,397(5,6);7,281 (1,0);7,273(1,8); 7,262(1,5);7,258(1,3);7,253(2,2);7,250(1,9);7,245(1,4);7,241(1,2);7,231(1,5);7,222(0,8);6,990(3,6);6,984(3,6);5,757(16,0);3,338(10,3);2,512 (11,5);2,507(14,9);2,503(11,0);1,992(0,4);1,396(1,3) |
| I-1-220: |
| HPLC-MS: logP = 3,00; Masse (m/z): 335,0 (M+H)⁺; ¹H-NMR(400,0 MHz, CD₃CN): |
| δ= 10,173(3,4);8,614(7,7);8,611(8,1);8,603(8,1);8,600(8,1);8,113(6,9);8,106(12,9);8,100(7,0);8,000(7,2);7,997(7,5);7,979(8,1);7,976(8,1);7, 664(3,2);7,656(3,4);7,648(3,5);7,640(6,4);7,632(3,6);7,624(3,4);7,616(3,3);7,578(8,1);7,567(8,1);7,558(7,6);7,546(7,3);7,425(0,5);7,419(0,5) ;7,376(3,0);7,364(3,2);7,353(3,9);7,347(3,5);7,341(4,0);7,335(3,4);7,324(3,7);7,312(3,6);7,240(0,4);7,235(0,4);7,172(0,4);7,165(0,4);7,102(2 ,2);7,093(4,2);7,082(16,0);7,076(15,6);7,066(3,1);7,061(2,7);7,051(3,2);7,043(1,7);5,448(1,0);2,468(0,4);2,463(0,5);2,459(0,4);2,149(379,2); 2,121(2,2);2,114(2,0);2,108(2,2);2,102(1,5);2,096(0,9);1,965(12,1);1,958(17,5);1,953(121,2);1,947(224,8);1,941(311,8);1,934(217,2);1,928( 114,5);1,781(0,7);1,775(1,3);1,769(1,8);1,763(1,3);1,757(0,7);1,372(11,0);1,340(1,2);1,285(1,9);1,276(11,2);1,269(3,1);1,216(1,7);0,881(0,5 );0,858(0,4);0,146(2,0);0,008(19,1);0,000(421,4);-0,008(20,7);-0,150(2,0) |
| I-1-221: |
| HPLC-MS: logP = 3,57; Masse (m/z): 403,1 (M+H)⁺; ¹H-NMR(400,0 MHz, DMSO-D₆): |
| δ= 11,061(8,8);8,237(5,0);8,230(9,8);8,224(5,1);7,809(13,3);7,796(13,7);7,656(2,3);7,648(2,5);7,640(2,6);7,632(4,5);7,624(2,5);7,617(2,5);7 ,609(2,3);7,587(2,1);7,575(2,3);7,564(2,7);7,559(2,5);7,552(2,7);7,546(2,5);7,536(2,5);7,523(2,4);7,293(2,0);7,286(16,0);7,273(15,3);7,265( 3,6);7,262(3,0);7,257(2,0);7,253(1,8);7,251(1,7);7,242(2,2);7,234(1,1);6,937(9,7);6,930(9,7);5,757(1,4);3,326(12,4);2,673(0,4);2,512(24,2);2 ,508(46,1);2,503(59,5);2,499(43,3);2,495(21,5);2,330(0,4);1,232(0,8);1,181(0,4);0,000(3,7) |
| I-1-222: |
| HPLC-MS: logP = 3,14; Masse (m/z): 415,9 (M+H)⁺; ¹H-NMR(400,0 MHz, DMSO-D₆): |
| δ= 10,963(7,2);8,316(0,6);8,224(4,4);8,218(9,1);8,211(4,6);7,945(11,9);7,940(12,1);7,666(1,9);7,657(2,1);7,650(2,2);7,642(4,0);7,634(2,2);7 ,626(2,2);7,618(2,0);7,584(1,8);7,572(2,0);7,561(2,2);7,555(2,1);7,549(2,3);7,543(2,1);7,532(2,2);7,520(2,1);7,284(1,3);7,276(2,4);7,267(1,6 );7,265(1,8);7,261(1,5);7,256(2,8);7,253(2,4);7,248(1,6);7,245(1,4);7,242(1,4);7,234(1,9);7,225(0,9);6,931(10,2);6,922(16,0);6,918(13,2);3,3 24(151,7);2,676(1,0);2,671(1,4);2,667(1,0);2,662(0,5);2,525(3,6);2,520(5,6);2,511(79,5);2,507(163,0);2,502(215,9);2,498(156,7);2,493(76,2 );2,338(0,5);2,333(1,0);2,329(1,4);2,324(1,0);1,398(0,4);1,135(0,5);1,117(1,3);1,099(0,7);0,008(1,1);0,000(35,4);-0,009(1,3) |
| I-1-223: |
| HPLC-MS: logP = 2,71; Masse (m/z): 372,1 (M+H)⁺; ¹H-NMR(400,0 MHz, CD₃CN): |
| δ= 8,672(1,2);8,068(2,8);8,062(5,3);8,055(2,8);7,625(1,4);7,617(1,4);7,610(1,4);7,602(2,7);7,594(1,4);7,586(1,4);7,578(1,4);7,426(0,4);7,42 0(0,4);7,368(1,3);7,355(1,4);7,345(1,6);7,339(1,4);7,332(1,6);7,327(1,4);7,316(1,5);7,304(1,5);7,235(3,4);7,101(6,6);7,090(1,7);7,082(1,1);7, 079(1,1);7,076(1,0);7,071(1,8);7,067(1,5);7,062(1,1);7,059(0,9);7,056(0,9);7,048(1,3);7,040(0,7);6,967(3,3);6,942(5,4);6,936(5,3);5,448(2,3) ;3,804(15,2);3,802(16,0);3,788(0,3);3,408(0,3);2,172(5,9);2,121(0,4);2,115(0,4);2,109(0,4);2,102(0,3);1,965(1,3);1,959(1,7);1,953(13,0);1,9 47(24,0);1,941(33,2);1,935(22,7);1,929(11,6);1,372(5,7);1,340(0,8);1,285(1,3);1,276(6,5);1,268(1,2);0,008(1,7);0,000(42,7);-0,009(1,6) |
| I-1-224: |
| HPLC-MS: logP = 2,72; Masse (m/z): 369,0 (M+H)⁺; ¹H-NMR(400,0 MHz, CD₃CN): |
| δ= 9,424(5,5);8,163(13,6);8,103(3,3);8,097(3,6);8,084(6,0);8,078(6,2);8,064(3,5);8,058(3,5);7,980(12,1);7,829(7,3);7,809(11,2);7,757(2,7);7 ,740(7,6);7,721(9,4);7,700(9,1);7,687(16,0);7,671(7,7);7,060(13,7);7,054(12,7);5,449(13,4);2,164(73,5);2,114(0,5);2,109(0,5);1,972(2,2);1,9 53(26,4);1,947(44,4);1,941(55,6);1,935(39,2);1,930(21,0);1,769(0,4);1,372(4,5);1,311(0,7);1,294(0,8);1,276(4,6);1,221(0,4);1,204(0,7);1,18 6(0,4);0,000(13,7) |
| I-1-225: |
| HPLC-MS: logP = 2,59; Masse (m/z): 335,0 (M+H)⁺; ¹H-NMR(400,0 MHz, CD₃CN): |
| δ= 9,382(3,2);8,168(5,8);8,162(10,4);8,155(6,2);8,105(2,4);8,098(2,8);8,085(4,1);8,079(4,5);8,066(2,5);8,059(2,7);7,982(6,3);7,975(10,4);7, 969(6,1);7,602(6,0);7,583(7,8);7,526(2,2);7,522(3,4);7,506(10,3);7,502(16,0);7,498(8,6);7,484(8,4);7,480(8,1);7,465(3,5);7,460(3,3);7,442(6 ,4);7,437(6,0);7,423(7,2);7,419(7,2);7,406(3,1);7,401(3,1);7,082(9,8);7,076(10,0);5,447(1,5);2,165(37,0);2,110(0,4);2,088(0,6);1,966(1,8);1, 960(2,2);1,955(16,0);1,948(30,2);1,942(42,2);1,936(29,7);1,930(15,7);0,008(0,5);0,000(13,2);-0,008(0,7) |
| I-1-226: |
| HPLC-MS: logP = 3,21; Masse (m/z): 417,0 (M+H)⁺; ¹H-NMR(400,0 MHz, CD₃CN): |
| δ= 9,373(2,7);8,428(4,0);8,424(4,3);8,416(4,4);8,412(4,2);8,286(4,2);8,282(4,1);8,267(4,5);8,263(4,2);7,675(7,4);7,669(7,5);7,589(7,8);7,58 7(7,6);7,568(16,0);7,512(6,2);7,494(4,9);7,489(4,2);7,479(4,5);7,470(4,1);7,467(4,7);7,460(4,3);7,448(3,8);7,426(0,4);7,000(7,5);6,994(7,4); 5,449(0,9);4,067(0,6);4,049(0,6);2,163(40,7);1,972(2,9);1,965(1,7);1,954(13,5);1,947(24,6);1,941(33,7);1,935(24,2);1,929(13,2);1,436(5,6); 1,372(3,8);1,308(0,3);1,291(0,4);1,276(4,0);1,221(0,8);1,203(1,5);1,186(0,8);0,000(8,2) |
| I-1-227 siehe Synthesebeispiel 41 |
| I-1-228 siehe Synthesebeispiel 28 |
| I-1-229 siehe Synthesebeispiel 26 |
| I-1-230 siehe Synthesebeispiel 27 |
| I-1-231: |
| HPLC-MS: logP = 2,89; Masse (m/z): 372,1 (M+H)⁺; ¹H-NMR(601,6 MHz, DMSO-D₆): |
| δ= 11,473(2,7);8,113(3,0);8,109(2,8);7,977(1,0);7,880(2,6);7,784(1,2);7,648(0,4);7,638(0,9);7,634(0,8);7,624(1,6);7,613(0,9);7,609(1,0);7,5 99(0,4);7,408(2,7);7,394(4,9);7,380(2,4);6,909(2,4);6,906(2,4);3,401(999,7);3,400(1251,1);3,395(262,8);3,394(254,6);3,002(1,3);2,625(0,6); 2,552(43,7);2,534(0,9);2,531(1,1);2,528(1,1);2,516(66,1);2,513(92,4);2,510(68,4);2,507(33,5);2,397(0,6);2,284(16,0);1,265(0,3);1,242(1,8); 0,861(0,4) |
| I-1-232: |
| HPLC-MS: logP = 2,10; Masse (m/z): 318,1 (M+H)⁺; ¹H-NMR(400,0 MHz, CD₃CN): |
| δ= 9,141(0,7);7,743(1,7);7,740(1,6);7,737(1,8);7,546(0,3);7,531(0,6);7,525(0,6);7,515(0,4);7,509(1,3);7,503(0,5);7,494(0,6);7,488(0,8);7,47 2(0,4);7,218(0,5);7,213(2,1);7,200(0,4);7,192(3,5);7,183(0,6);7,171(1,8);7,162(0,4);6,968(2,5);6,962(2,5);6,663(4,3);4,051(16,0);2,214(14,4) ;2,181(19,9);1,973(0,6);1,966(0,4);1,959(0,5);1,954(4,7);1,947(9,0);1,941(12,7);1,935(9,0);1,929(4,9);1,436(1,0);1,372(1,1);1,276(1,2);0,00 8(0,7);0,000(16,5);-0,008(1,0) |
| I-1-233: |
| HPLC-MS: logP = 2,23; Masse (m/z): 363,1 (M+H)⁺; ¹H-NMR(601,6 MHz, DMSO-D₆): |
| δ= 11,869(2,2);8,142(1,6);8,138(1,5);7,634(0,5);7,630(0,5);7,624(0,4);7,620(0,9);7,616(0,4);7,610(0,5);7,606(0,6);7,411(1,6);7,397(2,8);7,3 84(1,3);6,982(2,9);6,977(2,9);3,858(16,0);3,350(162,4);2,552(14,7);2,531(0,4);2,519(10,8);2,516(24,2);2,513(34,6);2,510(25,1);2,507(11,7); 2,458(14,3) |
| I-1-234: |
| HPLC-MS: logP = 2,03; Masse (m/z): 363,1 (M+H)⁺; ¹H-NMR(400,0 MHz, DMSO-D₆): |
| δ= 11,356(2,8);8,316(0,6);8,071(2,1);8,065(2,1);7,619(0,7);7,614(0,6);7,598(1,3);7,582(0,6);7,576(0,9);7,561(0,4);7,398(2,2);7,376(3,8);7,3 56(1,8);6,915(3,3);6,909(3,3);5,756(1,8);3,867(16,0);3,324(234,7);2,675(1,3);2,671(1,8);2,666(1,3);2,607(14,8);2,541(0,9);2,524(4,6);2,510( 106,6);2,506(210,0);2,502(272,4);2,497(195,8);2,440(0,4);2,333(1,3);2,328(1,8);2,324(1,3);0,146(1,0);0,008(8,2);0,000(205,9);-0,008(7,5);-0,150(1,0) |
| I-1-235: |
| HPLC-MS: logP = 2,76; Masse (m/z): 444,0 (M+H)⁺; ¹H-NMR(601,6 MHz, DMSO-D₆): |
| δ= 11,302(4,6);8,106(3,7);7,648(0,6);7,638(1,3);7,634(1,3);7,623(10,9);7,613(1,5);7,610(1,5);7,599(0,6);7,409(3,8);7,395(6,8);7,381(3,3);6, 947(4,2);6,943(4,2);4,287(1,8);4,275(5,5);4,263(5,5);4,251(1,9);3,371(0,7);3,347(1213,1);2,625(1,0);2,552(5,7);2,534(1,4);2,531(1,8);2,528( 1,9);2,519(53,5);2,516(114,3);2,513(158,9);2,510(116,8);2,507(56,9);2,397(1,0);1,356(7,5);1,344(16,0);1,332(7,6);1,246(0,7);0,011(0,7) |
| I-1-236: |
| HPLC-MS: logP = 1,93; Masse (m/z): 305,0 (M+H)⁺; ¹H-NMR(400,0 MHz, DMSO-D₆): |
| δ= 11,049(1,4);8,316(0,5);8,139(5,0);8,100(1,8);8,094(1,8);7,639(0,6);7,633(0,5);7,624(0,4);7,618(1,2);7,612(0,4);7,602(0,5);7,597(0,8);7,5 81(0,3);7,407(2,0);7,386(3,3);7,366(1,5);6,895(2,7);6,888(2,7);4,174(16,0);3,324(380,4);2,675(1,7);2,670(2,4);2,666(1,7);2,661(0,8);2,541(1 ,8);2,524(7,0);2,510(134,1);2,506(270,6);2,501(357,5);2,497(259,9);2,492(125,8);2,337(0,8);2,333(1,7);2,328(2,3);2,324(1,7);2,074(4,9);1,2 58(0,4);0,146(0,4);0,008(2,9);0,000(86,4);-0,008(2,7);-0,150(0,4) |
| I-1-237: |
| HPLC-MS: logP = 3,84; Masse (m/z): 490,1 (M+H)⁺; ¹H-NMR(601,6 MHz, DMSO-D₆): |
| δ= 12,018(3,8);8,157(2,7);8,154(2,6);7,652(0,4);7,641(0,8);7,638(0,8);7,627(1,4);7,616(0,8);7,613(0,8);7,602(0,4);7,416(2,4);7,401(4,3);7,3 88(2,0);6,953(4,1);6,949(3,9);4,017(16,0);3,884(0,5);3,349(1067,7);2,625(0,8);2,553(18,2);2,534(1,4);2,531(1,8);2,516(99,1);2,513(128,0);2 ,510(91,0);2,397(0,8);1,246(0,5);0,011(0,8) |
| I-1-238: |
| HPLC-MS: logP = 2,99; Masse (m/z): 440,0 (M+H)⁺; ¹H-NMR(400,0 MHz, DMSO-D₆): |
| δ= 11,589(5,2);8,083(3,7);8,080(3,3);8,077(3,7);7,638(0,6);7,622(1,3);7,616(1,0);7,606(0,9);7,601(2,6);7,595(1,0);7,584(1,1);7,579(1,7);7,5 64(0,8);7,408(0,7);7,399(4,4);7,378(7,3);7,357(3,4);6,876(7,0);6,870(6,9);5,757(0,4);4,126(16,0);4,092(0,9);4,021(0,4);3,325(34,8);2,982(2, 1);2,829(2,1);2,672(0,3);2,525(1,2);2,520(1,8);2,512(19,3);2,507(38,5);2,503(50,6);2,498(36,4);2,493(17,3);1,989(0,7);1,259(0,4);1,236(2,4) ;1,175(0,4);0,000(0,3) |
| I-1-239: |
| HPLC-MS: logP = 2,50; Masse (m/z): 348,1 (M+H)⁺; ¹H-NMR(400,0 MHz, DMSO-D₆): |
| δ= 11,094(3,0);8,230(2,8);8,225(2,9);8,055(2,2);8,050(2,2);7,826(3,4);7,821(3,3);7,626(0,7);7,621(0,6);7,610(0,5);7,604(1,4);7,589(0,7);7,5 83(0,9);7,567(0,4);7,404(2,2);7,382(3,9);7,362(1,7);6,896(3,4);6,890(3,4);5,757(6,0);3,975(16,0);3,328(95,8);2,675(0,5);2,671(0,7);2,541(0, 4);2,506(80,6);2,502(104,7);2,498(77,1);2,333(0,5);2,329(0,7);2,324(0,5);0,008(0,9);0,000(22,6);-0,008(1,0) |
| I-1-240: |
| HPLC-MS: logP = 2,52; Masse (m/z): 371,1 (M+H)⁺; ¹H-NMR(400,0 MHz, DMSO-D₆): |
| δ= 10,607(3,5);7,997(2,5);7,994(2,3);7,991(2,4);7,798(2,7);7,793(2,7);7,624(0,4);7,608(0,9);7,602(0,7);7,592(0,6);7,587(1,7);7,581(0,6);7,5 70(0,8);7,565(1,1);7,550(0,5);7,393(3,3);7,389(5,1);7,368(4,7);7,347(2,2);7,341(0,6);6,895(4,3);6,889(4,3);4,101(0,6);4,088(0,6);3,688(16,0) |
| ;3,327(7,0);3,178(3,0);3,165(2,9);2,525(0,4);2,512(6,2);2,507(12,1);2,503(15,9);2,498(11,6);2,494(5,6);1,250(0,4) |
| I-1-241: |
| HPLC-MS: logP = 1,90; Masse (m/z): 349,1 (M+H)⁺; ¹H-NMR(400,0 MHz, DMSO-D₆): |
| δ= 11,412(2,9);8,894(5,3);8,083(2,2);8,078(2,1);7,636(0,3);7,620(0,7);7,61 5(0,6);7,599(1,4);7,593(0,5);7,583(0,7);7,578(0,9);7,562(0,4);7,4 00(2,3);7,379(3,9);7,358(1,8);6,927(3,5);6,921(3,3);5,757(1,1);3,945(1 6,0);3,326(107,5);2,675(0,5);2,671(0,7);2,667(0,5);2,541(0,4);2,506(8 3,8);2,502(103,4);2,498(72,2);2,333(0,5);2,329(0,7);2,324(0,5);0,146(0,3);0,000(74,5);-0,008(2,8);-0,149(0,4) |
| I-1-242: |
| HPLC-MS: logP = 1,81; Masse (m/z): 349,1 (M+H)⁺; ¹H-NMR(400,0 MHz, DMSO-D₆): |
| δ= 11,214(3,1);8,316(0,6);8,082(5,3);8,073(2,4);8,067(2,4);7,634(0,4);7,618(0,8);7,612(0,7);7,602(0,5);7,596(1,5);7,591(0,6);7,580(0,7);7,5 75(1,0);7,559(0,4);7,397(2,6);7,376(4,3);7,355(2,0);6,925(3,9);6,919(3,9);5,756(0,4);3,926(16,0);3,323(161,8);2,675(1,0);2,671(1,4);2,666(1 ,0);2,541(0,7);2,524(3,2);2,519(5,2);2,511(79,2);2,506(163,8);2,502(217,8);2,497(156,8);2,493(75,7);2,333(1,0);2,328(1,4);2,324(1,0);0,146 (0,7);0,008(5,3);0,000(156,3);-0,009(5,9);-0,150(0,7) |
| I-1-243: |
| HPLC-MS: logP = 1,80; Masse (m/z): 385,0 (M+H)⁺; ¹H-NMR(400,0 MHz, DMSO-D₆): |
| δ= 11,619(12,2);11,093(0,4);11,082(1,0);8,623(8,5);8,607(8,6);8,490(1,2);8,316(0,9);8,097(9,2);8,092(9,2);7,890(0,3);7,825(6,9);7,805(10,1 );7,744(0,6);7,730(5,4);7,713(6,6);7,694(3,7);7,665(0,4);7,638(1,3);7,622(2,8);7,617(2,6);7,601(5,6);7,595(2,5);7,585(2,8);7,579(3,7);7,564( 1,6);7,443(0,7);7,422(1,3);7,402(9,8);7,381(16,0);7,360(7,2);7,261(0,4);7,240(0,5);6,905(13,8);6,899(13,6);6,075(0,4);6,069(0,4);3,322(135, 5);2,675(2,2);2,671(2,9);2,666(2,2);2,506(347,5);2,502(443,3);2,497(328,0);2,333(2,2);2,328(2,8);2,324(2,1);0,146(0,5);0,008(5,1);0,000(10 8,9);-0,008(5,0);-0,150(0,5) |
| I-1-244: |
| HPLC-MS: logP = 3,58; Masse (m/z): 380,1 (M+H)⁺; ¹H-NMR(601,6 MHz, DMSO-D₆): |
| δ= 11,415(5,6);8,107(4,4);8,104(4,5);7,700(9,1);7,649(0,6);7,638(1,4);7,634(1,3);7,624(2,5);7,614(1,4);7,610(1,6);7,600(0,7);7,410(4,1);7,3 96(7,3);7,382(3,5);6,948(5,3);6,944(5,4);4,550(0,9);4,539(1,5);4,525(1,5);4,515(1,0);3,345(469,7);2,625(0,6);2,552(11,6);2,534(0,9);2,531(1 ,2);2,528(1,2);2,519(32,6);2,516(72,2);2,513(101,6);2,510(75,7);2,507(37,7);2,397(0,6);1,879(0,9);1,866(1,3);1,856(1,5);1,841(1,7);1,829(1, 3);1,817(0,4);1,765(0,3);1,753(1,2);1,744(1,5);1,741(1,5);1,731(1,9);1,718(1,3);1,709(0,9);1,429(13,0);1,418(13,1);1,246(0,5);0,737(7,5);0,7 24(16,0);0,712(7,4);0,010(0,7) |
| I-1-245: |
| HPLC-MS: logP = 3,47; Masse (m/z): 472,1 (M+H)⁺; ¹H-NMR(601,6 MHz, DMSO-D₆): |
| δ= 11,343(6,8);8,102(4,9);8,099(4,9);7,641(11,4);7,624(2,7);7,613(1,5);7,610(1,7);7,599(0,7);7,410(4,4);7,395(7,9);7,382(3,8);6,947(6,4);6, 943(6,5);4,471(1,0);4,461(1,7);4,447(1,7);4,437(1,1);3,346(1159,5);2,625(1,1);2,552(81,9);2,534(1,7);2,531(2,2);2,528(2,1);2,519(58,6);2,5 16(125,8);2,513(173,8);2,510(126,2);2,507(59,8);2,397(1,1);1,871(0,9);1,858(1,3);1,848(1,6);1,834(1,7);1,821(1,3);1,809(0,4);1,753(0,3);1, 741(1,2);1,731(1,6);1,719(2,0);1,706(1,3);1,697(0,9);1,414(14,6);1,403(14,7);1,269(0,4);1,246(0,8);0,731(7,6);0,719(16,0);0,706(7,4);0,011( 1,3) |
| I-1-246: |
| HPLC-MS: logP = 2,35; Masse (m/z): 382,1 (M+H)⁺; ¹H-NMR(400,0 MHz, DMSO-D₆): |
| δ= 11,441(10,5);8,704(7,3);8,700(7,9);8,692(7,8);8,688(7,6);8,092(6,9);8,087(9,0);8,083(9,0);8,077(8,9);8,073(9,2);8,068(7,2);7,643(1,4);7, 627(3,1);7,621(2,4);7,611(2,1);7,606(6,0);7,600(2,3);7,589(2,6);7,584(3,9);7,569(1,8);7,540(6,7);7,528(6,7);7,520(6,5);7,508(6,3);7,413(1,6) ;7,404(9,8);7,383(16,0);7,362(7,4);6,962(13,3);6,955(13,2);4,143(3,2);4,115(10,1);4,087(10,5);4,058(3,6);3,328(195,5);2,676(0,6);2,671(0,9 );2,667(0,6);2,541(34,1);2,525(2,9);2,520(4,6);2,511(49,4);2,507(99,5);2,502(131,9);2,498(95,5);2,493(45,7);2,333(0,6);2,329(0,9);2,324(0, 6);0,008(0,9);0,000(27,4);-0,009(0,8) |
| I-1-247: |
| HPLC-MS: logP = 2,74; Masse (m/z): 366,1 (M+H)⁺; ¹H-NMR(400,0 MHz, DMSO-D₆): |
| δ= 11,038(10,5);8,056(8,2);8,050(8,2);7,636(6,9);7,632(6,8);7,617(8,1);7,613(9,5);7,604(2,3);7,598(5,9);7,592(2,4);7,581(5,5);7,577(6,7);7, 561(7,4);7,541(4,6);7,537(4,0);7,395(9,8);7,388(6,9);7,383(2,5);7,374(16,0);7,364(6,2);7,362(6,1);7,354(7,9);7,345(9,6);7,343(9,3);7,326(3, 8);7,324(3,8);7,278(7,4);7,258(6,5);7,204(12,5);7,019(6,2);6,941(13,1);6,935(13,0);5,756(3,7);4,038(0,4);4,020(0,4);3,324(103,6);2,679(0,4) ;2,675(0,8);2,670(1,1);2,666(0,8);2,524(3,3);2,519(5,3);2,510(62,0);2,506(123,2);2,501(161,2);2,497(115,7);2,492(54,7);2,337(0,4);2,333(0, 8);2,328(1,1);2,324(0,8);1,989(1,5);1,299(0,8);1,259(1,2);1,235(1,8);1,192(0,5);1,175(0,9);1,157(0,5);0,008(1,5);0,000(40,7);-0,009(1,2) |
| I-1-248: |
| HPLC-MS: logP = 1,99; Masse (m/z): 351,1 (M+H)⁺; ¹H-NMR(400,0 MHz, DMSO-D₆): |
| δ= 20,009(0,4);11,503(11,6);8,805(6,8);8,796(6,9);8,793(6,7);8,315(2,2);8,190(6,2);8,171(6,6);8,089(9,0);8,083(9,0);7,700(4,7);7,688(4,9);7 ,680(4,7);7,668(4,4);7,643(1,3);7,627(2,7);7,622(2,6);7,606(5,5);7,590(2,8);7,585(3,6);7,569(1,6);7,404(9,3);7,382(16,0);7,362(7,3);7,340(4, 9);7,205(10,7);7,070(5,3);6,961(12,9);6,955(12,8);3,321(780,9);2,675(5,7);2,670(7,7);2,666(5,7);2,540(4,4);2,506(888,4);2,501(1153,4);2,49 7(832,1);2,332(5,5);2,328(7,3);2,324(5,4);0,146(0,4);0,008(3,6);0,000(92,4);-0,009(3,1);-0,150(0,4) |
| I-1-249: |
| HPLC-MS: logP = 2,38; Masse (m/z): 347,0 (M+H)⁺; ¹H-NMR(400,0 MHz, DMSO-D₆): |
| δ= 11,252(2,1);8,579(1,6);8,575(1,8);8,567(1,7);8,563(1,7);8,071(2,0);8,066(2,1);7,966(1,4);7,962(1,4);7,947(1,5);7,943(1,5);7,624(0,6);7,6 19(0,6);7,608(0,5);7,603(1,3);7,598(0,5);7,587(0,6);7,581(0,8);7,566(0,4);7,401(2,2);7,380(3,7);7,359(1,7);7,233(1,6);7,220(1,6);7,214(1,6); 7,201 (1,5);6,947(1,7);6,941 (1,7);5,758(1,8);3,332(13,9);2,507(9,7);2,503(12,6);2,498(9,4);2,466(16,0);0,000(0,4) |
| I-1-250: |
| HPLC-MS: logP = 2,77; Masse (m/z): 385,0 (M+H)⁺; ¹H-NMR(400,0 MHz, CD₃CN): |
| δ= 9,340(3,1);8,430(6,0);8,426(6,1);8,418(6,3);8,414(5,9);8,288(6,3);8,283(5,9);8,269(6,6);8,264(6,0);7,769(8,7);7,553(1,4);7,538(3,0);7,53 2(3,0);7,522(2,2);7,516(6,2);7,510(2,2);7,501(3,2);7,495(3,7);7,480(7,9);7,467(6,4);7,461(6,2);7,448(5,8);7,426(0,3);7,241(0,3);7,235(0,4);7, 216(8,9);7,195(16,0);7,174(7,9);7,010(10,8);7,003(10,2);5,447(1,3);2,140(48,9); 2,119(0,3);2,113(0,5);2,107(0,6);2,101(0,4);1,963(1,2);1,95 2(27,6);1,946(51,2);1,939(71,3);1,933(48,7);1,927(24,7);1,768(0,4);1,372(4,7);1,341(0,8);1,285(1,2);1,277(5,0);0,146(0,6);0,008(5,6);0,000( 118,5);-0,008(4,4);-0,149(0,6) |
| I-1-251: |
| HPLC-MS: logP = 2,73; Masse (m/z): 402,0 (M+H)⁺; ¹H-NMR(400,0 MHz, CD₃CN): |
| δ= 8,661(4,1);7,944(10,5);7,841(5,5);7,822(7,1);7,740(16,0);7,704(4,8);7,691(4,9);7,591(1,4);7,571(3,4);7,555(5,6);7,538(3,7);7,534(3,8);7, 518(1,8);7,426(0,6);7,420(0,6);7,239(7,3);7,217(12,7);7,196(6,5);7,171(0,8);7,165(0,7);7,150(0,5);7,144(0,4);5,446(0,6);4,085(0,5);4,067(1, 5);4,049(1,6);4,032(0,5);2,145(72,1);2,119(0,7);2,113(0,7);2,106(0,8);2,100(0,6);2,094(0,3);1,971(7,4);1,963(4,1);1,951(40,1);1,945(73,6);1, 939(100,3);1,933(68,8);1,927(35,3);1,774(0,4);1,767(0,6);1,761(0,4);1,436(3,7);1,372(8,6);1,340(1,7);1,307(0,4);1,285(2,6);1,276(9,8);1,27 1(3,8);1,221(1,9);1,203(3,6);1,185(1,8);0,881(0,5);0,146(0,8);0,008(9,1);0,000(174,0);-0,009(6,7);-0,150(0,8) |
| I-1-252: |
| HPLC-MS: logP = 2,77; Masse (m/z): 447,9 (M+H)⁺; ¹H-NMR(400,0 MHz, CD₃CN): |
| δ= 8,586(5,1);7,956(11,2);7,841(5,9);7,822(7,7);7,748(16,0);7,711(4,4);7,701(5,4);7,693(4,9);7,683(4,2);7,593(1,6);7,577(3,5);7,572(3,7);7, 556(6,3);7,540(4,0);7,535(4,1);7,519(1,9);7,447(0,4);7,426(0,8);7,420(0,7);7,240(8,2);7,219(13,8);7,197(7,0);7,171(1,0);7,165(0,8);7,150(0, 6);7,144(0,5);3,908(0,7);3,850(0,8);2,133(57,0);2,119(0,7);2,112(0,9);2,106(1,1);2,100(0,8);2,094(0,4);1,963(7,2);1,957(9,7);1,951(68,3);1,9 45(125,7);1,939(172,2);1,933(116,7);1,927(59,3);1,914(1,0);1,780(0,4);1,773(0,7);1,767(1,0);1,761(0,7);1,755(0,4);1,372(13,0);1,340(2,6);1 ,285(3,6);1,276(14,9);1,270(3,8);1,216(0,5);1,162(0,5);1,146(0,4);0,881(0,6);0,866(0,6);0,848(0,4);0,146(1,2);0,008(10,1);0,000(270,2);-0,009(8,6);-0,150(1,2) |
| I-1-253: |
| HPLC-MS: logP = 3,08; Masse (m/z): 382,1 (M+H)⁺; ¹H-NMR(400,0 MHz, DMSO-D₆): |
| δ= 8,130(3,2);7,907(2,1);7,887(2,7);7,868(4,4);7,845(1,0);7,826(2,2);7,807(1,7);7,745(1,6);7,719(3,6);7,702(4,0);7,684(2,7);7,655(0,5);7,63 4(1,0);7,618(1,7);7,597(1,4);7,576(2,2);7,558(5,8);7,550(6,7);7,540(5,3);7,519(1,1);7,407(2,5);7,385(4,7);7,365(4,6);7,313(3,7);7,291(6,2);7, 271(2,9);7,097(3,5);7,092(3,4);5,984(4,9);5,978(4,8);5,758(1,0);4,038(0,5);4,020(0,6);3,409(24,8);3,323(260,0);3,145(16,0);2,680(0,9);2,67 5(1,9);2,671(2,6);2,666(1,9);2,662(0,9);2,524(7,2);2,519(11,4);2,511(145,7);2,506(294,0);2,502(387,7);2,497(279,3);2,493(133,4);2,338(0,9 );2,333(1,9);2,328(2,6);2,324(1,9);2,320(0,9);1,989(2,3);1,398(1,1);1,351(0,4);1,298(0,3);1,259(0,5);1,235(0,9);1,192(0,7);1,175(1,3);1,157( 0,7);0,146(0,7);0,008(5,3);0,000(156,8);-0,009(4,8);-0,150(0,7) |
| I-1-254: |
| HPLC-MS: logP = 3,31; Masse (m/z): 396,1 (M+H)⁺; ¹H-NMR(400,0 MHz, DMSO-D₆): |
| δ= 8,317(0,6);8,121(1,2);7,907(0,8);7,880(5,4);7,875(5,1);7,846(0,4);7,830(0,8);7,811(0,6);7,751(0,7);7,731(1,7);7,711(1,1);7,689(2,6);7,67 6(2,8);7,666(2,9);7,634(0,4);7,615(0,7);7,597(1,1);7,581(1,7);7,576(1,7);7,560(3,1);7,544(2,3);7,531(5,4);7,521(7,1);7,511(5,1);7,495(0,6);7, 409(0,9);7,388(1,5);7,366(0,8);7,334(3,2);7,324(4,2);7,316(6,6);7,295(8,0);7,274(3,8);7,007(1,2);7,002(1,2);6,061(6,5);6,055(6,3);3,895(2,4) ;3,883(2,4);3,793(0,4);3,778(0,4);3,757(0,4);3,740(0,3);3,472(0,4);3,454(0,3);3,322(129,0);2,680(0,8);2,675(1,7);2,671(2,3);2,666(1,7);2,66 2(0,8);2,524(10,5);2,511(139,7);2,506(267,9);2,502(343,4);2,497(243,9);2,493(114,9);2,337(0,9);2,333(1,8);2,328(2,3);2,324(1,7);2,319(0,8 );2,074(0,7);1,754(0,5);1,212(7,7);1,194(16,0);1,177(7,4);1,096(1,5);1,080(2,6);1,063(1,3);0,146(0,9);0,008(9,2);0,000(222,2);-0,009(7,7);-0,150(0,9) |
| I-1-255: |
| HPLC-MS: logP = 3,44; Masse (m/z): 408,2 (M+H)⁺; ¹H-NMR(400,0 MHz, DMSO-D₆): |
| δ= 8,316(0,5);8,121(2,1);7,864(11,6);7,799(1,6);7,782(1,3);7,703(6,1);7,691(6,7);7,647(2,1);7,629(2,3);7,606(1,8);7,586(2,8);7,542(15,4);7, 532(16,0);7,400(1,9);7,378(3,2);7,352(7,7);7,344(7,3);7,304(8,3);7,283(13,3);7,261(6,5);7,008(2,1);6,080(12,1);5,954(1,3);5,941(2,2);5,928( 2,4);5,915(3,2);5,900(3,2);5,885(2,5);5,872(2,5);5,859(1,3);5,848(0,4);5,785(0,7);5,772(0,7);5,756(0,6);5,305(6,5);5,262(5,7);5,188(6,7);5,1 62(6,3);5,064(1,4);5,039(1,2);4,892(1,2);4,849(1,1);4,507(10,9);4,434(0,7);4,419(0,8);4,382(0,9);4,069(0,8);4,033(0,6);3,323(65,6);2,671(1, 9);2,629(0,5);2,506(264,1);2,502(261,6);2,359(0,5);2,331(1,9);1,236(1,0);0,002(3,1);0,000(4,3) |
| I-1-256: |
| HPLC-MS: logP = 3,14; Masse (m/z): 406,1 (M+H)⁺; ¹H-NMR(400,0 MHz, DMSO-D₆): |
| δ= 8,315(3,3);8,151(1,3);8,121(0,3);7,903(12,0);7,847(1,0);7,760(1,1);7,714(5,9);7,701(6,9);7,692(6,4);7,599(2,2);7,577(4,5);7,559(16,0);7, 545(15,4);7,536(12,8);7,392(1,7);7,371(1,4);7,340(6,7);7,327(7,9);7,316(12,7);7,294(15,6);7,273(7,2);7,018(1,2);6,132(12,3);6,126(12,2);4, 749(0,3);4,680(15,5);4,532(0,5);4,491(0,6);4,314(0,4);4,296(0,6);4,289(0,6);4,267(0,4);4,258(0,4);4,243(0,4);3,322(466,1);3,248(11,4);3,18 0(1,2);2,680(1,9);2,675(4,3);2,671(6,2);2,666(4,4);2,662(2,1);2,524(16,1);2,519(24,9);2,511(350,8);2,506(722,0);2,502(959,4);2,497(692,0); 2,493(333,3);2,409(0,6);2,398(0,5);2,360(0,4);2,338(2,3);2,333(4,6);2,329(6,3);2,324(4,7);2,320(2,3);2,074(0,5);0,146(0,6);0,008(4,5);0,000 (152,1);-0,008(5,7);-0,150(0,7) |
| I-1-257: |
| HPLC-MS: logP = 2,86; Masse (m/z): 493,9 (M+H)⁺; ¹H-NMR(400,0 MHz, CD₃CN): |
| δ= 8,644(1,8);7,945(4,2);7,843(2,3);7,824(3,1);7,779(4,8);7,761(3,0);7,744(1,4);7,712(1,9);7,694(2,4);7,677(1,2);7,589(0,6);7,568(1,4);7,55 3(2,3);7,532(1,5);7,515(0,7);7,426(0,8);7,420(0,8);7,262(0,5);7,256(0,6);7,237(3,2);7,216(5,2);7,195(2,7);7,171(0,8);7,165(0,7);7,150(0,4);7, 144(0,4);6,985(0,3);4,292(0,9);4,275(1,7);4,257(0,9);4,085(0,5);4,067(1,6);4,049(1,6);4,032(0,6);3,372(0,8);2,566(2,1);2,512(1,5);2,422(0,5) ;2,403(1,2);2,383(0,8);2,212(0,7);2,192(1,0);2,160(74,4);2,113(0,4);2,107(0,4);1,971(7,3);1,964(2,9);1,958(3,9);1,952(23,6);1,946(43,1);1,9 40(58,7);1,933(40,1);1,927(20,5);1,768(0,4);1,436(3,3);1,383(0,6);1,372(14,8);1,1,340(2,8);1,309(0,4);1,285(3,9);1,276(16,0);1,221(2,0);1,21 7(0,8);1,203(3,8);1,185(1,9);0,882(0,4);0,146(0,4);0,008(3,3);0,000(80,1);-0,009(2,8);-0,150(0,4) |
| I-1-258: |
| HPLC-MS: logP = 3,68; Masse (m/z): 422,1 (M+H)⁺; ¹H-NMR(400,0 MHz, DMSO-D₆): |
| δ= 8,316(0,9);8,126(1,1);7,895(0,9);7,870(9,8);7,864(9,5);7,822(0,7);7,804(0,6);7,731(1,6);7,702(4,7);7,691(5,1);7,688(5,0);7,680(5,2);7,61 7(0,8);7,600(1,7);7,584(3,0);7,579(3,0);7,563(5,4);7,547(4,4);7,536(10,8);7,525(11,4);7,514(9,8);7,410(0,8);7,390(1,3);7,367(0,9);7,319(12, 5);7,299(16,0);7,277(6,7);6,958(1,1);6,047(11,5);6,041(11,4);3,769(4,8);3,561(0,5);3,545(0,5);3,466(0,5);3,451(0,5);3,323(194,1);2,676(1,7) ;2,671(2,3);2,666(1,7);2,662(0,8);2,541(1,4);2,524(6,6);2,511(135,1);2,507(274,6);2,502(362,1);2,497(258,9);2,493(123,1);2,333(1,6);2,329( 2,2);2,324(1,6);1,118(1,2);1,107(2,3);1,100(2,3);1,089(3,5);1,077(2,5);1,070(2,5);1,058(1,4);1,039(0,5);0,999(0,4);0,985(0,5);0,456(9,7);0,4 36(9,1);0,390(0,3);0,311(1,2);0,295(1,1);0,257(3,4);0,245(11,0);0,233(10,3);0,222(2,7);0,000(1,5);-0,029(1,4) |
| I-1-259: |
| HPLC-MS: logP = 2,90; Masse (m/z): 407,1 (M+H)⁺; ¹H-NMR(400,0 MHz, DMSO-D₆): |
| δ= 8,316(0,7);8,223(0,4);7,951(8,4);7,898(0,5);7,882(0,5);7,875(0,5);7,846(0,5);7,749(5,2);7,741(4,7);7,710(0,6);7,617(9,7);7,605(12,0);7,5 86(6,5);7,568(3,3);7,549(1,3);7,450(4,9);7,338(6,6);7,316(10,7);7,295(5,1);7,074(0,4);6,067(8,2);5,002(16,0);4,938(0,3);4,738(0,4);4,702(0, 6);3,323(155,0);2,675(1,5);2,671(2,0);2,510(135,9);2,506(260,7);2,502(335,1);2,497(240,7);2,463(1,0);2,333(1,6);2,329(2,2);2,074(4,3);1,75 5(0,7);0,146(0,8);0,025(0,6);0,008(8,1);0,000(183,5);-0,007(6,5);-0,008(7,1);-0,149(0,9) |
| I-1-260: |
| HPLC-MS: logP = 3,50; Masse (m/z): 410,1 (M+H)⁺; ¹H-NMR(601,6 MHz, DMSO-D₆): |
| δ= 19,970(0,9);8,314(1,7);7,884(4,8);7,880(4,9);7,623(2,8);7,611(3,2);7,588(1,4);7,577(2,4);7,562(1,5);7,486(1,0);7,474(3,0);7,464(5,3);7,4 53(2,8);7,318(6,6);7,305(9,3);7,291(3,3);6,204(5,6);6,200(5,8);4,922(0,8);4,911(2,1);4,900(2,9);4,889(2,1);3,324(377,8);3,320(448,0);3,319( 443,5);2,616(3,5);2,613(5,1);2,610(3,9);2,541(1,4);2,522(6,9);2,519(9,1);2,516(8,6);2,507(239,3);2,504(543,6);2,501(773,8);2,498(589,8);2, 496(299,2);2,389(3,6);2,386(5,1);2,383(3,9);1,163(15,8);1,152(16,0);0,005(3,2);0,000(118,1) |
| I-1-261: |
| HPLC-MS: logP = 3,64; Masse (m/z): 410,1 (M+H)⁺; ¹H-NMR(601,6 MHz, DMSO-D₆): |
| δ= 8,115(0,6);7,901(0,4);7,887(0,5);7,874(4,5);7,870(4,5);7,827(0,4);7,733(0,4);7,708(0,5);7,684(2,3);7,676(2,5);7,669(2,6);7,581(0,6);7,57 1(1,3);7,567(1,3);7,556(2,4);7,546(1,4);7,542(1,5);7,531(1,0);7,521 (4,5);7,513(5,6);7,506(4,8);7,496(0,6);7,400(0,4);7,385(0,7);7,372(0,4);7, 312(2,7);7,305(5,7);7,298(3,1);7,291(7,1);7,277(3,5);6,969(0,6);6,086(6,2);6,082(6,2);3,836(1,6);3,322(54,5);2,614(0,4);2,611(0,3);2,523(0, 7);2,520(0,8);2,517(0,7);2,508(21,2);2,505(47,3);2,502(67,5);2,499(48,6);2,496(22,9);2,386(0,4);2,383(0,3);1,645(0,5);1,633(2,6);1,621(5,4) ;1,608(5,6);1,596(3,0);1,584(0,8);0,936(7,7);0,923(16,0);0,911(7,3);0,643(0,7);0,631(1,3);0,619(0,7);0,005(0,5);0,000(19,7);-0,006(0,7) |
| I-1-262: |
| HPLC-MS: logP = 3,40; Masse (m/z): 426,1 (M+H)⁺; ¹H-NMR(400,0 MHz, DMSO-D₆): |
| δ= 8,316(0,4);8,143(1,0);7,900(7,4);7,807(0,9);7,687(5,1);7,587(1,7);7,568(3,0);7,550(4,5);7,527(9,5);7,518(9,3);7,391(1,5);7,326(5,3);7,30 6(6,4);7,284(8,9);7,263(4,3);6,896(0,9);6,270(7,3);5,278(13,6);5,114(0,4);4,887(0,4);3,699(3,1);3,682(8,0);3,665(8,1);3,648(3,3);3,322(104, 8);2,675(1,3);2,671(1,6);2,666(1,2);2,540(0,9);2,506(201,4);2,502(251,0);2,497(182,3);2,333(1,2);2,328(1,6);2,324(1,2);1,158(8,6);1,141(16 ,0);1,124(8,9);0,954(2,2);0,146(0,5);0,007(8,4);-0,001(103,4);-0,150(0,5) |
| I-1-263: |
| HPLC-MS: logP = 3,40; Masse (m/z): 420,1 (M+H)⁺; ¹H-NMR(400,0 MHz, DMSO-D₆): |
| δ= 8,133(1,0);7,887(6,4);7,834(0,8);7,687(3,8);7,681(3,8);7,539(8,9);7,391(1,3);7,314(7,2);7,292(6,8);7,272(3,1);7,015(0,9);6,135(6,0);4,62 8(8,9);4,482(0,3);4,474(0,3);4,443(0,5);4,271(0,5);3,322(31,8);3,314(26,7);2,667(1,1);2,502(153,1);2,498(159,5);2,329(1,1);2,325(1,1);1,78 2(16,0);1,688(2,9);0,000(6,1);-0,003(3,6);-0,008(4,3) |
| I-1-264: |
| HPLC-MS: logP = 3,38; Masse (m/z): 432,1 (M+H)⁺; ¹H-NMR(400,0 MHz, DMSO-D₆): |
| δ= 8,316(0,5);8,189(0,4);7,898(10,3);7,893(10,1);7,849(0,4);7,837(0,4);7,765(0,5);7,728(4,7);7,718(5,5);7,714(5,7);7,706(5,9);7,601(1,8);7, 580(5,3);7,571(14,2);7,564(16,0);7,558(13,3);7,549(14,3);7,528(1,8);7,370(5,8);7,362(5,9);7,358(5,7);7,349(4,9);7,319(8,8);7,298(15,1);7,2 77(7,0);7,021(0,4);6,449(1,4);6,439(2,7);6,429(1,4);6,310(2,8);6,300(5,6);6,290(2,9);6,171(1,3);6,161(2,8);6,151(1,5);6,092(11,9);6,086(11, 7);4,355(3,3);4,346(3,6);4,319(6,7);4,310(6,7);4,282(3,7);4,273(3,4);3,322(79,8);2,676(0,9);2,671(1,3);2,666(0,9);2,662(0,4);2,541(0,7);2,52 4(3,3);2,511(72,5);2,506(146,7);2,502(194,1);2,497(140,3);2,493(67,6);2,338(0,4);2,333(0,9);2,329(1,3);2,324(0,9);0,008(0,5);0,000(15,4);-0,009(0,6) |
| I-1-265: |
| HPLC-MS: logP = 3,59; Masse (m/z): 450,0 (M+H)⁺; ¹H-NMR(400,0 MHz, DMSO-D₆): |
| δ= 8,316(0,5);7,920(10,3);7,916(10,2);7,732(4,8);7,723(5,4);7,710(6,1);7,605(1,7);7,589(3,9);7,583(4,6);7,565(14,1);7,553(13,5);7,546(13,0 );7,530(3,1);7,347(6,1);7,334(5,9);7,324(6,6);7,315(9,9);7,294(16,0);7,273(7,5);6,213(11,6);6,207(11,4);4,774(4,2);4,756(4,2);3,322(90,8);2, 675(1,0);2,671(1,4);2,666(1,1);2,541(0,7);2,524(3,7);2,510(83,4);2,506(165,6);2,502(217,9);2,497(158,5);2,493(77,0);2,333(1,1);2,328(1,5); 2,324(1,1);0,008(0,6);0,000(16,1);-0,009(0,6) |
| I-1-266: |
| HPLC-MS: logP = 2,93; Masse (m/z): 368,0 (M+H)⁺; ¹H-NMR(400,0 MHz, DMSO-D₆): |
| δ= 11,332(11,3);8,212(0,5);8,206(0,6);8,077(8,5);8,071(8,6);7,757(7,2);7,753(7,8);7,737(8,5);7,733(8,7);7,640(1,5);7,624(3,1);7,618(2,5);7, 608(2,1);7,603(5,8);7,597(2,3);7,586(2,7);7,581(3,7);7,563(6,8);7,559(6,9);7,544(10,2);7,540(9,2);7,478(9,6);7,459(13,1);7,439(6,0);7,417(0 ,8);7,399(9,9);7,378(16,0);7,357(7,4);6,946(14,3);6,940(14,4);6,928(1,2);6,729(0,9);5,757(6,7);3,323(58,3);2,675(0,7);2,671(0,9);2,666(0,7); 2,541(31,2);2,524(2,8);2,511(53,6);2,506(107,0);2,502(140,6);2,497(102,8);2,493(50,5);2,348(3,6);2,337(0,5);2,333(0,8);2,328(1,0);2,324(0, 7);0,008(1,4);0,000(39,7);-0,009(1,4) |
| I-1-267: |
| HPLC-MS: logP = 2,72; Masse (m/z): 368,1 (M+H)⁺; ¹H-NMR(400,0 MHz, DMSO-D₆): |
| δ= 11,468(5,9);8,080(4,4);8,077(3,8);8,074(4,4);7,640(0,8);7,624(1,7);7,618(1,2);7,608(1,1);7,602(3,3);7,597(1,2);7,586(1,3);7,581(2,2);7,5 64(7,7);7,560(8,8);7,543(15,4);7,541(16,0);7,497(8,3);7,480(5,2);7,474(4,2);7,457(3,1);7,410(0,8);7,406(1,4);7,401(5,5);7,388(1,0);7,380(8, 8);7,359(4,1);7,354(1,1);6,949(8,8);6,943(8,7);3,406(0,3);3,390(0,6);3,343(380,7);3,321(1,7);3,301(0,5);3,291(0,4);2,676(0,4);2,672(0,6);2,6 67(0,4);2,542(50,0);2,532(0,5);2,525(1,8);2,520(3,0);2,512(34,4);2,507(69,0);2,503(90,9);2,498(65,3);2,493(30,6);2,334(0,4);2,329(0,6);2,3 25(0,4);0,000(0,9) |
| I-1-268: |
| HPLC-MS: logP = 2,68; Masse (m/z): 328,1 (M+H)⁺; ¹H-NMR(400,0 MHz, CD₃CN): |
| δ= 9,056(0,4);7,752(1,1);7,749(1,0);7,746(1,1);7,523(0,4);7,518(0,4);7,502(0,9);7,486(0,4);7,480(0,5);7,238(0,6);7,219(1,1);7,210(0,4);7,20 5(1,5);7,200(1,2);7,192(0,3);7,184(2,3);7,163(1,2);7,101(2,3);7,082(1,7);7,042(1,8);7,035(1,8);2,316(16,0);2,150(7,5);1,963(0,5);1,957(0,7); 1,951(3,7);1,945(6,6);1,939(9,0);1,933(6,2);1,927(3,2);1,372(0,8);1,276(0,8);0,008(0,5);0,000(11,0);-0,009(0,5) |
| I-1-269: |
| HPLC-MS: logP = 2,20; Masse (m/z): 357,0 (M+H)⁺; ¹H-NMR(400,0 MHz, DMSO-D₆): |
| δ= 11,210(1,8);10,533(1,1);8,177(1,1);8,156(1,2);8,072(2,3);8,066(2,3);7,823(1,5);7,805(1,6);7,646(0,4);7,631(0,7);7,625(0,7);7,615(0,6);7, 609(1,4);7,593(0,7);7,588(0,9);7,573(0,5);7,524(0,9);7,521(0,9);7,503(1,7);7,485(1,0);7,482(0,9);7,405(2,5);7,384(4,1);7,363(1,9);7,188(1,1) ;7,170(1,9);7,152(0,9);6,940(2,2);6,934(2,2);3,325(42,1);2,675(0,4);2,671(0,5);2,541(0,5);2,506(53,8);2,502(68,6);2,497(49,8);2,328(0,4);2, 260(0,5);2,087(16,0);2,027(0,3);1,909(2,2);1,235(1,0);0,008(2,9);0,000(61,0);-0,008(2,6) |
| I-1-270: |
| HPLC-MS: logP = 2,38; Masse (m/z): 342,1 (M+H)⁺; ¹H-NMR(400,0 MHz, DMSO-D₆): |
| δ= 8,316(0,7);7,804(2,8);7,785(3,3);7,770(1,1);7,768(1,1);7,752(2,5);7,750(2,4);7,733(1,9);7,731(1,7);7,646(3,5);7,628(3,5);7,610(5,1);7,60 3(2,8);7,594(1,3);7,515(4,3);7,482(0,5);7,466(1,0);7,460(0,8);7,450(0,7);7,445(2,0);7,439(0,8);7,428(0,9);7,423(1,3);7,408(0,6);7,263(0,9);7, 257(3,4);7,236(5,7);7,215(2,5);7,209(0,6);5,757(1,5);5,130(4,5);5,124(4,4);3,322(330,5);2,680(1,2);2,675(2,4);2,671(3,2);2,666(2,3);2,661(1 ,2);2,612(0,4);2,524(10,6);2,519(16,2);2,510(174,5);2,506(347,1);2,501(454,2);2,497(323,8);2,492(151,3);2,337(0,9);2,333(2,1);2,328(2,9);2 ,324(2,1);2,319(0,9);1,903(16,0);1,298(1,4);1,259(2,1);1,235(1,1);0,146(0,9);0,008(8,0);0,000(229,0);-0,009(7,1);-0,150(0,9) |
| I-1-271: |
| HPLC-MS: logP = 2,47; Masse (m/z): 363,1 (M+H)⁺; ¹H-NMR(400,0 MHz, DMSO-D₆): |
| δ= 11,146(2,3);8,038(1,7);8,032(1,8);7,617(0,6);7,611(0,5);7,602(0,4);7,596(1,2);7,580(0,5);7,575(0,8);7,559(0,4);7,423(1,4);7,402(2,8);7,3 94(2,1);7,382(2,3);7,373(3,4);7,352(1,6);7,102(2,4);7,096(2,8);7,080(2,0);7,076(2,5);6,936(3,2);6,930(3,3);3,811(16,0);3,328(486,3);2,995(0 ,4);2,680(0,7);2,675(1,2);2,671(1,6);2,666(1,2);2,541(53,6);2,524(5,1);2,519(8,0);2,511(91,0);2,506(182,0);2,502(239,8);2,497(172,9);2,493 (82,2);2,338(0,5);2,333(1,1);2,328(1,5);2,324(1,1);0,008(0,6);0,000(16,7);-0,009(0,4) |
| I-1-272: |
| HPLC-MS: logP = 2,48; Masse (m/z): 325,1 (M+H)⁺; ¹H-NMR(400,0 MHz, CD₃CN): |
| δ= 10,113(5,2);9,387(0,4);8,021(7,9);8,003(9,3);7,945(10,2);7,932(4,2);7,914(7,5);7,896(9,4);7,883(2,7);7,875(1,5);7,869(1,5);7,861(1,4);7, 830(3,3);7,812(7,7);7,794(5,3);7,779(8,6);7,776(8,5);7,761(10,4);7,758(9,4);7,742(3,6);7,740(3,2);7,591(1,8);7,576(4,0);7,570(3,7);7,560(2, 8);7,554(8,1);7,548(2,9);7,539(4,0);7,533(4,7);7,517(2,3);7,266(8,9);7,245(16,0);7,231(3,6);7,224(7,8);7,210(1,6);7,045(8,0);7,039(7,8);6,67 5(1,8);6,669(1,8);2,727(0,4);2,463(0,4);2,184(0,7);2,153(83,5);2,120(0,5);2,114(0,7);2,108(0,8);2,101(0,6);1,964(5,2);1,958(6,4);1,953(47,9) ;1,946(89,3);1,940(124,6);1,934(86,0);1,928(44,1);1,775(0,5);1,769(0,7);1,763(0,5);1,267(0,4);0,008(1,5);0,000(42,2);-0,009(1,4) |
| I-1-273: |
| HPLC-MS: logP = 2,89; Masse (m/z): 340,2 (M+H)⁺; ¹H-NMR(400,0 MHz, DMSO-D₆): |
| δ= 11,037(8,6);8,039(7,7);7,635(1,1);7,619(2,5);7,614(2,2);7,597(4,8);7,581(2,5);7,576(3,1);7,560(1,4);7,401(7,3);7,393(8,7);7,382(8,5);7,3 72(16,0);7,351(11,4);7,335(4,2);7,234(4,5);7,216(7,0);7,197(3,0);6,981(7,4);6,969(10,7);6,963(14,8);5,756(3,7);3,323(59,0);2,675(0,8);2,67 0(1,0);2,666(0,8);2,540(0,8);2,506(126,6);2,501(157,1);2,497(113,6);2,332(0,8);2,328(1,0);2,324(0,8);2,257(0,9);2,244(2,0);2,236(2,3);2,22 3(3,8);2,210(2,3);2,202(2,0);2,189(1,0);1,989(0,7);1,175(0,4);0,954(2,5);0,943(7,7);0,938(7,9);0,928(4,7);0,922(7,6);0,917(7,6);0,907(2,9);0, 705(3,1);0,691(9,4);0,682(8,8);0,678(8,6);0,666(2,5);0,146(0,4);0,008(4,3);0,000(84,1);-0,008(3,2);-0,150(0,4) |
| I-1-274: |
| HPLC-MS: logP = 2,85; Masse (m/z): 346,1 (M+H)⁺; ¹H-NMR(400,0 MHz, DMSO-D₆): |
| δ= 11,297(4,4);8,315(0,3);8,062(3,4);8,056(3,5);7,637(0,6);7,621(1,2);7,616(1,0);7,605(0,8);7,600(2,3);7,594(0,9);7,583(1,0);7,578(1,5);7,5 63(0,7);7,437(1,1);7,421(1,4);7,417(2,2);7,402(3,3);7,397(5,5);7,381(2,5);7,376(6,6);7,355(3,1);7,171(3,6);7,153(3,1);7,144(2,0);7,121(3,0); 7,100(1,6);6,953(5,9);6,947(5,9);3,327(306,3);2,678(2,1);2,671(1,8);2,666(1,7);2,660(5,5);2,641(5,4);2,622(1,8);2,541(42,5);2,524(4,3);2,51 9(7,1);2,511(82,9);2,506(164,3);2,502(213,7);2,497(155,5);2,493(76,3);2,337(0,5);2,333(1,0);2,328(1,4);2,324(1,0);2,074(7,1);1,259(0,4);1, 235(0,6);1,203(7,2);1,184(16,0);1,165(7,0);1,149(0,7);0,008(1,1);0,000(32,5);-0,009(1,3) |
| I-1-275: |
| HPLC-MS: logP = 1,47; Masse (m/z): 329,1 (M+H)⁺; ¹H-NMR(400,0 MHz, DMSO-D₆): |
| δ= 11,235(3,8);8,593(2,5);8,588(2,7);8,580(2,7);8,576(2,7);8,315(0,7);8,068(3,1);8,062(3,1);7,872(1,8);7,868(1,9);7,853(2,0);7,849(2,0);7,6 39(0,5);7,623(1,2);7,617(0,9);7,607(0,8);7,602(2,4);7,596(0,9);7,585(1,1);7,580(1,6);7,564(0,7);7,398(3,8);7,377(6,3);7,357(3,0);7,335(2,0); 7,323(2,1);7,316(2,1);7,304(1,9);6,965(4,9);6,958(4,9);3,334(505,9);2,915(1,7);2,897(5,3);2,878(5,4);2,859(1,8);2,680(1,2);2,675(2,5);2,671 (3,5);2,666(2,5);2,662(1,2);2,541(60,7);2,524(10,3);2,519(15,9);2,511(199,0);2,506(400,9);2,502(526,4);2,497(373,9);2,492(174,8);2,419(0, 4);2,338(1,2);2,333(2,5);2,328(3,5);2,324(2,4);2,319(1,1);2,074(13,8);1,298(0,6);1,259(1,0);1,248(7,5);1,229(16,0);1,210(7,1);1,147(0,3);0,0 08(2,8);0,000(83,4);-0,009(2,2) |
| I-1-276: |
| HPLC-MS: logP = 2,72; Masse (m/z): 386,1 (M+H)⁺; ¹H-NMR(400,0 MHz, DMSO-D₆): |
| δ= 11,563(9,8);8,090(8,0);8,084(8,0);7,774(0,8);7,754(2,5);7,746(1,4);7,735(5,8);7,721(9,3);7,701(16,0);7,685(4,5);7,680(3,4);7,639(1,3);7, 623(2,6);7,617(2,3);7,607(1,8);7,601(5,3);7,596(2,2);7,585(2,4);7,580(3,4);7,564(1,5);7,401(8,6);7,379(14,4);7,359(6,5);6,927(13,6);6,921(1 3,5);3,517(0,6);3,351(103,3);2,714(0,5);2,544(105,9);2,527(0,8);2,522(0,8);2,513(10,6);2,509(21,8);2,504(29,2);2,500(21,8);2,496(10,8);2,3 70(0,5);0,000(2,0) |
| I-1-277: |
| HPLC-MS: logP = 2,74; Masse (m/z): 431,8 (M+H)⁺; ¹H-NMR(400,0 MHz, DMSO-D₆): |
| δ= 11,408(2,5);8,080(1,8);8,074(1,8);7,740(1,9);7,722(1,9);7,720(2,0);7,623(0,6);7,617(0,5);7,608(0,4);7,602(1,2);7,596(0,5);7,586(0,5);7,5 81(0,8);7,565(0,4);7,410(0,3);7,401(2,1);7,379(4,1);7,359(3,3);7,337(1,1);7,335(1,1);7,284(1,0);7,268(1,1);7,264(1,2);7,249(1,2);7,243(0,6); 7,228(0,6);6,943(3,3);6,937(3,2);4,116(1,0);4,103(3,1);4,089(3,1);4,076(1,1);3,328(12,9);3,176(16,0);3,163(15,4);2,524(0,3);2,510(5,9);2,50 6(11,7);2,501(15,3);2,497(10,9);2,492(5,1);0,008(0,3);0,000(9,3) |
| I-1-278: |
| HPLC-MS: logP = 2,60; Masse (m/z): 318,1 (M+H)⁺; ¹H-NMR(400,0 MHz, DMSO-D₆): |
| δ= 11,109(8,0);8,066(7,6);8,060(7,5);7,695(2,5);7,691(2,9);7,676(4,8);7,672(5,4);7,657(3,0);7,653(3,0);7,640(1,5);7,625(3,1);7,618(2,4);7,6 09(2,0);7,603(6,2);7,597(3,6);7,592(2,3);7,586(3,0);7,582(5,1);7,579(4,3);7,576(3,2);7,572(2,7);7,565(3,9);7,560(3,0);7,558(3,8);7,553(2,2); 7,544(2,1);7,539(1,8);7,409(1,5);7,405(2,6);7,400(9,8);7,387(2,0);7,379(16,0);7,358(7,6);7,352(2,5);7,346(4,6);7,326(9,0);7,321(5,3);7,319( 4,5);7,310(9,0);7,308(8,3);7,300(3,7);7,298(3,6);7,291(4,5);7,289(3,7);6,945(10,0);6,939(9,8);3,424(0,4);3,412(0,5);3,399(0,6);3,344(657,4); 2,712(0,4);2,681(0,3);2,676(0,7);2,672(1,0);2,667(0,8);2,663(0,4);2,542(109,0);2,525(2,9);2,520(4,5);2,512(57,6);2,507(116,9);2,503(155,0) ;2,498(111,8);2,494(53,0);2,368(0,4);2,339(0,4);2,334(0,8);2,330(1,0);2,325(0,8);2,320(0,4);0,000(4,6) |
| I-1-279: |
| HPLC-MS: logP = 2,00; Masse (m/z): 319,1 (M+H)⁺; ¹H-NMR(400,0 MHz, DMSO-D₆): |
| δ= 11,330(9,6);8,391(5,3);8,381(5,1);8,379(5,2);8,317(0,4);8,269(3,1);8,265(3,0);8,251(3,8);8,246(5,8);8,241(3,3);8,227(3,3);8,222(2,9);8,0 88(8,6);8,083(8,4);7,643(1,5);7,627(3,1);7,621(2,7);7,612(2,3);7,606(6,1);7,600(2,6);7,590(2,9);7,585(3,9);7,569(1,9);7,519(0,4);7,507(3,7); 7,502(3,8);7,494(3,9);7,489(5,6);7,484(3,8);7,476(3,5);7,471(3,3);7,404(9,6);7,383(16,0);7,362(7,4);6,946(12,0);6,940(11,6);3,324(126,4);3, 063(2,4);2,998(0,5);2,897(0,5);2,680(0,7);2,675(1,3);2,671(1,7);2,666(1,3);2,662(0,6);2,541(32,6);2,524(6,8);2,511(100,3);2,506(194,4);2,5 02(252,1);2,497(182,2);2,493(87,8);2,338(0,6);2,333(1,2);2,329(1,7);2,324(1,2);2,320(0,6);0,008(2,8);0,000(68,7);-0,008(2,4) |
| I-1-280 |
| HPLC-MS: logP = 2,75; Masse (m/z): 330,1 (M+H)⁺; ¹H-NMR(400,0 MHz, DMSO-D₆): |
| δ= 10,544(2,3);8,047(1,9);8,042(1,9);7,782(1,5);7,777(1,6);7,762(1,6);7,758(1,6);7,642(0,4);7,627(0,7);7,620(0,6);7,611(0,5);7,605(1,5);7,5 99(0,5);7,589(0,6);7,584(0,9);7,568(0,4);7,553(0,8);7,548(0,8);7,534(1,1);7,532(1,3);7,528(1,1);7,514(1,0);7,509(1,0);7,405(0,4);7,400(0,6); 7,395(2,4);7,383(0,5);7,374(3,9);7,354(1,8);7,348(0,5);7,210(2,1);7,189(1,8);7,106(1,2);7,104(1,2);7,087(2,0);7,086(2,0);7,069(1,0);7,067(1, 0);6,960(3,1);6,953(3,1);5,756(0,6);3,931(16,0);3,324(20,7);2,524(0,7);2,519(1,1);2,511(12,2);2,506(24,6);2,502(32,6);2,497(23,4);2,493(11 ,1);0,008(0,6);0,000(18,2);-0,009(0,6) |
| I-1-281: |
| HPLC-MS: logP = 2,43; Masse (m/z): 304,1 (M+H)⁺; ¹H-NMR(400,0 MHz, CD₃CN): |
| δ= 8,776(0,7);7,719(1,8);7,716(1,8);7,713(1,7);7,523(0,6);7,517(0,6);7,507(0,4);7,502(1,3);7,496(0,5);7,486(0,7);7,480(0,8);7,465(0,4);7,39 0(2,6);7,385(2,5);7,218(0,4);7,208(2,1);7,196(0,5);7,187(3,6);7,178(0,4);7,166(1,8);6,977(2,6);6,971(2,5);6,820(2,7);6,815(2,5);5,446(1,0);2, 666(0,5);2,587(16,0);2,522(1,4);2,138(3,5);1,963(0,7);1,952(5,6);1,945(10,3);1,939(13,9);1,933(9,4);1,927(4,8);1,372(0,7);1,276(0,9);1,269( 0,5);0,000(5,1) |
| I-1-282: |
| HPLC-MS: logP = 2,80; Masse (m/z): 356,0 (M+H)⁺; ¹H-NMR(400,0 MHz, DMSO-D₆): |
| δ= 11,324(9,3);11,220(0,4);8,212(0,5);8,205(0,5);8,094(0,4);8,082(7,5);8,076(7,4);7,917(10,0);7,905(10,6);7,649(1,3);7,633(2,7);7,628(2,2); 7,617(1,9);7,612(5,3);7,606(2,1);7,595(2,3);7,590(3,4);7,575(1,6);7,565(4,2);7,426(9,5);7,416(2,4);7,409(16,0);7,396(12,8);7,386(14,6);7,36 5(6,8);7,288(4,4);6,934(0,9);6,927(0,9);6,895(12,3);6,889(12,5);6,729(0,8);5,757(2,8);3,324(116,7);3,051(0,4);2,680(0,5);2,675(1,0);2,671(1 ,3);2,666(1,0);2,662(0,5);2,541(7,1);2,524(4,0);2,519(6,4);2,511(76,7);2,506(152,6);2,502(199,6);2,497(143,6);2,493(68,4);2,347(2,9);2,338 (0,6);2,333(1,0);2,328(1,4);2,324(1,0);2,320(0,5);0,008(1,8);0,000(53,6);-0,009(1,7) |
| I-1-283: |
| HPLC-MS: logP = 2,27; Masse (m/z): 372,0 (M+H)⁺; ¹H-NMR(400,0 MHz, DMSO-D₆): |
| δ= 10,784(4,2);8,317(0,5);8,057(3,6);8,051(3,6);7,639(0,6);7,623(1,2);7,618(1,1);7,607(1,0);7,602(2,4);7,586(1,3);7,580(1,6);7,565(0,8);7,3 99(4,1);7,378(6,8);7,357(3,3);7,266(2,1);7,131(4,8);6,997(2,3);6,861(4,2);6,855(4,1);4,170(0,4);3,810(16,0);3,537(0,6);3,321(520,2);3,035(0 ,4);2,861(0,3);2,795(0,4);2,718(0,4);2,675(6,3);2,670(8,4);2,666(6,1);2,541(12,5);2,523(32,0);2,510(491,8);2,506(943,2);2,501(1214,7);2,49 7(877,5);2,492(424,1);2,412(0,5);2,332(5,9);2,328(8,0);2,323(5,8);1,235(0,3);1,147(0,8);0,933(0,5);0,146(1,3);0,008(12,2);0,000(297,3);-0,009(10,4);-0,150(1,3) |
| I-1-284: |
| HPLC-MS: logP = 2,82; Masse (m/z): 374,0 (M+H)⁺; ¹H-NMR(400,0 MHz, DMSO-D₆): |
| δ= 11,493(7,8);8,316(1,2);8,082(8,3);8,076(8,1);7,923(7,9);7,910(8,3);7,646(1,4);7,631(2,9);7,625(2,3);7,615(1,9);7,609(5,7);7,603(2,1);7,5 93(2,5);7,588(3,7);7,572(1,6);7,448(15,3);7,435(14,5);7,404(9,7);7,383(16,0);7,362(7,3);6,888(6,9);6,882(6,8);3,321(329,2);2,679(2,0);2,67 5(4,3);2,670(5,8);2,666(4,3);2,661(2,0);2,596(0,3);2,524(18,6);2,510(332,4);2,506(666,1);2,501(876,1);2,497(627,6);2,492(297,5);2,417(0,5 );2,337(2,0);2,333(4,1);2,328(5,8);2,324(4,1);2,319(1,9);1,398(0,9);1,336(0,6);1,298(0,4);1,258(0,5);1,250(0,7);0,146(2,3);0,008(19,4);0,000 (544,0);-0,009(17,4);-0,021(0,7);-0,150(2,3) |
| I-1-285: |
| HPLC-MS: logP = 2,65; Masse (m/z): 330,1 (M+H)⁺; ¹H-NMR(400,0 MHz, DMSO-D₆): |
| δ= 11,119(3,4);8,758(5,0);8,752(5,2);8,583(4,6);8,577(4,2);8,316(0,4);8,098(2,8);8,092(2,8);7,645(0,5);7,629(1,0);7,623(0,8);7,613(0,7);7,6 08(2,0);7,602(0,7);7,591(0,8);7,586(1,3);7,571(0,6);7,414(0,5);7,404(3,4);7,383(5,5);7,362(2,6);7,357(0,7);6,975(5,2);6,969(5,1);3,324(200, 9);3,310(1,5);3,106(1,7);3,087(5,3);3,068(5,4);3,050(1,8);2,680(0,4);2,675(0,9);2,671(1,3);2,666(0,9);2,662(0,4);2,541(8,9);2,524(3,3);2,519 (5,5);2,511(71,7);2,506(145,0);2,502(190,3);2,497(134,9);2,493(63,2);2,338(0,4);2,333(0,9);2,328(1,2);2,324(0,9);2,319(0,4);2,074(6,1);1,2 87(7,3);1,268(16,0);1,259(0,7);1,249(7,3);1,235(0,6);0,008(1,5);0,000(45,7);-0,009(1,3) |
| I-1-286: |
| HPLC-MS: logP = 2.33; Masse (m/z): 352,9 (M+H)⁺; ¹H-NMR [CD₃CN] 6.98 (d, 1H), 7.05 - 7.11 (m, 2H), 7.50 - 7.55 (m, 1H), 7.91 - 7.92 (m, 1H), 8.03 (d, 1H), 8.39 (d, 1H), 9.43 (br. s, 1H). |
| I-1-287: |
| HPLC-MS: logP = 2,76; Masse (m/z): 386,1 (M+H)⁺; ¹H-NMR(400,0 MHz, DMSO-D₆): |
| δ= 11,415(10,6);8,074(8,2);8,068(8,1);7,851(1,9);7,838(2,2);7,831(3,9);7,818(3,9);7,811(2,7);7,798(2,4);7,634(3,8);7,623(3,2);7,616(4,3);7, 607(5,5);7,601(7,1);7,596(2,4);7,585(5,3);7,580(4,6);7,564(1,7);7,497(7,0);7,478(6,3);7,408(1,5);7,399(9,6);7,378(16,0);7,357(7,5);6,905(15 ,3);6,899(15,2);4,583(0,4);3,512(3,8);3,492(0,6);3,478(0,7);3,466(0,5);3,463(0,4);3,456(0,4);3,424(1,1);3,411(1,2);3,397(1,3);3,339(1044,4); 3,277(0,5);2,995(0,4);2,712(0,5);2,681(0,6);2,676(1,3);2,672(1,8);2,667(1,3);2,663(0,6);2,542(136,0);2,525(5,1);2,520(7,9);2,512(98,7);2,50 7(197,8);2,502(262,0);2,498(190,3);2,493(90,8);2,368(0,5);2,338(0,6);2,334(1,2);2,329(1,7);2,325(1,2);2,320(0,6);2,074(0,6);1,235(0,8);0,0 08(0,8);0,000(25,2);-0,009(0,7) |
| I-1-288: |
| HPLC-MS: logP = 2,64; Masse (m/z): 415,9 (M+H)⁺; ¹H-NMR(400,0 MHz, DMSO-D₆): |
| δ= 10,883(10,6);8,053(7,9);8,047(7,8);7,922(15,8);7,917(16,0);7,641(1,2);7,625(2,6);7,619(2,2);7,610(1,8);7,604(5,1);7,598(2,1);7,588(2,4); 7,583(3,3);7,567(1,5);7,408(1,5);7,399(8,8);7,378(14,3);7,357(6,6);6,909(16,0);6,904(15,9);6,887(12,9);6,881(12,6);5,757(15,4);3,329(22,4) ;2,512(19,9);2,508(38,2);2,503(49,3);2,499(35,5);2,495(17,0);2,087(2,7);1,990(1,1);1,397(0,7);1,175(0,5);0,008(1,6);0,000(31,5);-0,008(1,1) |
| I-1-289: |
| HPLC-MS: logP = 3,19; Masse (m/z): 405,1 (M+H)⁺; ¹H-NMR(601,6 MHz, DMSO-D₆): |
| δ= 11,886(2,7);8,140(1,6);8,136(1,7);7,631(0,5);7,627(0,5);7,617(1,0);7,606(0,5);7,603(0,6);7,410(1,7);7,396(2,9);7,382(1,4);6,980(2,9);6,9 76(3,0);3,870(16,0);3,358(196,3);2,760(3,9);2,748(4,1);2,553(49,9);2,534(0,4);2,531(0,5);2,528(0,4);2,519(11,2);2,516(25,8);2,513(37,1);2, 510(27,8);2,507(13,9);2,046(0,4);2,035(0,7);2,024(0,9);2,013(0,8);2,001(0,4);0,951(13,6);0,940(13,5) |
| I-1-290: |
| HPLC-MS: logP = 2,93; Masse (m/z): 391,1 (M+H)⁺; ¹H-NMR(601,6 MHz, DMSO-D₆): |
| δ= 11,859(2,7);8,138(1,8);8,134(1,8);7,633(0,5);7,629(0,5);7,619(1,0);7,608(0,5);7,605(0,7);7,410(1,8);7,396(3,1);7,382(1,5);6,978(3,0);6,9 74(3,0);3,869(16,0);3,519(0,4);3,508(1,0);3,496(1,4);3,485(1,0);3,473(0,4);3,349(170,5);2,552(53,4);2,534(0,5);2,531(0,5);2,519(8,2);2,516( 17,4);2,513(24,1);2,510(18,0);2,507(8,9);1,285(14,7);1,273(14,7) |
| I-1-291: |
| HPLC-MS: logP = 2,80; Masse (m/z): 373,1 (M+H)⁺; ¹H-NMR(400,0 MHz, DMSO-D₆): |
| δ= 11,653(2,1);8,316(0,3);8,112(1,9);8,106(1,9);7,631(0,6);7,625(0,6);7,615(0,5);7,609(1,3);7,604(0,5);7,593(0,6);7,588(0,8);7,572(0,4);7,4 06(2,1);7,385(3,5);7,364(1,6);6,912(3,1);6,905(3,1);3,326(270,7);2,680(0,6);2,675(1,2);2,671(1,7);2,666(1,2);2,662(0,6);2,541(2,4);2,524(5, 2);2,519(8,2);2,511(94,7);2,506(189,4);2,502(249,0);2,497(180,2);2,493(87,3);2,422(16,0);2,337(0,5);2,333(1,2);2,328(1,6);2,324(1,2);2,319 (0,5);2,074(3,4);1,235(0,4);0,008(1,4);0,000(40,9);-0,009(1,4) |
| I-1-292: |
| HPLC-MS: logP = 3,33; Masse (m/z): 405,1 (M+H)⁺; ¹H-NMR(601,6 MHz, DMSO-D₆): |
| δ= 11,866(1,1);8,130(0,8);8,126(0,8);7,616(0,4);7,408(0,7);7,393(1,3);7,380(0,6);6,962(1,3);6,958(1,3);3,841(6,8);3,3754(253,8);3,3745(256 ,5);2,552(20,7);2,534(0,4);2,531(0,5);2,528(0,5);2,519(8,5);2,516(17,7);2,513(24,3);2,510(18,0);2,507(8,8);1,401(16,0) |
| I-1-293: |
| HPLC-MS: logP = 4,15; Masse (m/z): 394,1 (M+H)⁺;¹H-NMR(601,6 MHz, DMSO-D₆): |
| δ= 11,298(0,9);8,087(0,8);8,084(0,8);7,611(0,5);7,396(0,8);7,382(1,4);7,368(0,7);6,923(0,8);6,920(0,8);3,843(5,2);3,340(158,7);2,542(10,3); 2,523(0,3);2,520(0,4);2,517(0,4);2,508(13,3);2,505(29,0);2,502(40,4);2,499(29,7);2,497(14,6);1,352(16,0) |
| I-1-294: |
| HPLC-MS: logP = 2,34; Masse (m/z): 368,1 (M+H)⁺; ¹H-NMR(601,6 MHz, DMSO-D₆): |
| δ= 11,537(3,0);8,107(3,1);8,104(3,1);7,646(0,5);7,636(1,2);7,632(1,1);7,621(2,1);7,611(1,1);7,607(1,4);7,597(0,6);7,408(3,5);7,393(6,2);7,3 80(3,0);7,109(1,1);7,018(2,4);6,941(2,6);6,938(2,6);6,927(1,3);3,858(15,0);3,349(973,1);2,628(0,7);2,625(1,0);2,622(0,8);2,552(19,9);2,534( 1,5);2,531(1,9);2,528(1,8);2,519(53,0);2,516(118,1);2,513(166,3);2,510(120,3);2,507(57,5);2,400(0,8);2,397(1,1);2,394(0,8);2,303(0,5);2,27 0(16,0);1,246(0,6);0,010(0,4) |
| I-1-295: |
| HPLC-MS: logP = 2,25; Masse (m/z): 370,0 (M+H)⁺; ¹H-NMR(400,0 MHz, CD₃CN): |
| δ= 9,479(1,6);9,444(13,2);9,182(16,0);7,788(7,7);7,785(7,4);7,783(7,4);7,559(1,3);7,550(0,4);7,543(2,8);7,537(2,5);7,528(1,9);7,522(5,6);7, 516(2,0);7,506(2,7);7,500(3,2);7,485(1,6);7,427(0,5);7,420(0,5);7,241(0,4);7,236(0,6);7,227(2,2);7,218(8,3);7,205(2,9);7,197(13,9);7,188(1, 8);7,176(7,1);7,167(1,4);6,993(10,7);6,986(10,7);6,972(1,2);6,966(1,1);5,447(12,0);4,085(0,4);4,067(1,3);4,050(1,3);4,032(0,4);2,147(20,3); |
| 2,143(17,2);2,107(0,4);1,971(5,9);1,964(2,2);1,958(2,7);1,952(20,5);1,946(38,1);1,940(53,1);1,933(36,7);1,927(19,0);1,436(1,9);1,383(0,3); 1,372(8,7);1,340(1,5);1,300(0,4);1,285(2,2);1,277(9,3);1,221(1,6);1,217(0,4);1,203(3,0);1,185(1,5);0,008(0,7);0,000(17,7);-0,009(0,6) |
| I-1-296: |
| HPLC-MS: logP = 3,14; Masse (m/z): 422,0 (M+H)⁺; ¹H-NMR(400,0 MHz, DMSO-D₆): |
| δ= 11,952(0,3);11,701(11,4);8,316(0,6);8,112(8,3);8,106(8,4);8,090(2,3);8,086(2,5);8,069(2,8);8,065(2,8);8,060(2,6);8,047(2,3);7,642(1,3);7 ,626(2,8);7,620(2,3);7,610(1,9);7,604(5,6);7,599(2,1);7,588(2,5);7,583(3,6);7,567(1,6);7,404(9,5);7,383(16,0);7,362(7,3);7,280(0,3);6,910(1 4,6);6,904(14,6);5,756(0,5);3,323(119,2);3,021(0,5);2,829(0,5);2,676(1,0);2,671(1,4);2,667(1,0);2,541(0,8);2,524(3,7);2,511(80,1);2,507(16 1,9);2,502(212,2);2,498(150,7);2,493(71,2);2,333(1,0);2,329(1,4);2,324(1,0);2,179(0,5);1,259(0,5);1,236(7,0);0,854(0,7);0,146(0,3);0,008(2, 6);0,000(74,7);-0,008(2,4) |
| I-1-297: |
| HPLC-MS: logP = 2,76; Masse (m/z): 319,1 (M+H)⁺; ¹H-NMR(400,0 MHz, DMSO-D₆): |
| δ= 11,180(3,1);8,075(2,9);8,069(2,8);7,648(0,4);7,632(0,9);7,626(0,8);7,616(0,7);7,611(1,9);7,605(0,7);7,595(0,9);7,589(1,2);7,574(0,5);7,4 02(3,1);7,381(5,2);7,360(2,4);6,894(3,7);6,887(3,6);3,325(92,7);2,675(0,5);2,671(0,7);2,666(0,5);2,541(0,5);2,524(1,8);2,510(42,2);2,506(82 ,7);2,502(106,4);2,497(75,8);2,493(35,9);2,399(15,2);2,333(0,5);2,329(0,7);2,324(0,5);2,071(16,0);1,989(0,3);0,000(42,0);-0,008(1,4) |
| I-1-298: |
| HPLC-MS: logP = 2,76; Masse (m/z): 398,0 (M+H)⁺; ¹H-NMR(601,6 MHz, DMSO-D₆): |
| δ= 11,235(2,3);8,102(2,1);8,099(2,1);7,647(0,3);7,637(0,7);7,633(0,7);7,623(1,4);7,612(0,7);7,608(0,9);7,598(0,4);7,407(2,3);7,393(4,1);7,3 79(2,0);6,940(1,9);6,936(2,0);3,881(14,2);3,354(657,5);2,628(0,3);2,625(0,5);2,622(0,3);2,552(9,2);2,534(0,7);2,531(0,8);2,528(0,8);2,519(2 3,5);2,516(52,2);2,513(73,6);2,510(53,9);2,507(25,7);2,397(0,5);2,394(0,4);2,168(16,0);0,010(0,5) |
| I-1-299: |
| HPLC-MS: logP = 2,84; Masse (m/z): 398,0 (M+H)⁺; ¹H-NMR(601,6 MHz, DMSO-D₆): |
| δ= 11,354(4,1);8,108(3,4);8,105(3,4);7,672(7,7);7,648(0,5);7,638(1,2);7,634(1,1);7,623(2,1);7,613(1,2);7,609(1,4);7,599(0,6);7,408(3,5);7,3 94(6,2);7,381(3,0);6,947(3,8);6,943(3,8);4,294(1,6);4,282(5,1);4,270(5,2);4,258(1,8);3,355(364,0);3,353(606,6);3,005(0,9);2,625(0,5);2,552( 54,1);2,534(0,8);2,531(1,0);2,528(1,0);2,519(27,5);2,516(59,1);2,513(81,5);2,510(59,6);2,507(29,0);2,397(0,5);1,367(7,4);1,355(16,0);1,343 (7,5);1,246(0,4);0,010(0,5) |
| I-1-300: |
| HPLC-MS: logP = 3,15; Masse (m/z): 412,0 (M+H)⁺; ¹H-NMR(601,6 MHz, DMSO-D₆): |
| δ= 11,267(2,3);8,100(2,0);8,097(2,0);7,648(0,3);7,638(0,7);7,634(0,7);7,623(1,4);7,613(0,7);7,609(0,9);7,599(0,4);7,407(2,2);7,393(3,9);7,3 79(1,9);6,939(2,1);6,935(2,1);4,226(1,0);4,214(2,9);4,202(3,0);4,190(1,0);3,351(498,7);2,625(0,4);2,552(3,9);2,534(0,6);2,531(0,8);2,528(0, 8);2,519(21,5);2,516(47,9);2,513(67,3);2,510(48,7);2,507(23,6);2,397(0,4);2,181(16,0);1,346(4,7);1,334(10,3);1,322(4,7);0,010(0,5) |
| I-1-301: |
| HPLC-MS: logP = 2,37; Masse (m/z): 384,0 (M+H)⁺; ¹H-NMR(400,0 MHz, DMSO-D₆): |
| δ= 10,334(2,2);8,316(0,7);8,109(5,4);8,042(1,7);8,036(1,7);7,620(0,6);7,615(0,5);7,605(0,4);7,599(1,2);7,593(0,5);7,582(0,5);7,577(0,8);7,5 62(0,3);7,396(2,0);7,375(3,3);7,354(1,6);6,880(2,3);6,873(2,3);3,925(16,0);3,324(216,8);2,675(1,1);2,671(1,5);2,666(1,1);2,541(1,0);2,524(3 ,9);2,511(88,5);2,506(180,0);2,502(236,5);2,497(168,4);2,493(79,2);2,333(1,1);2,328(1,5);2,324(1,1);0,146(0,8);0,008(6,3);0,000(174,5);-0,009(5,7);-0,150(0,7) |
| I-1-302: |
| HPLC-MS: logP = 2,50; Masse (m/z): 382,0 (M+H)⁺; ¹H-NMR(601,6 MHz, DMSO-D₆): |
| δ= 11,319(2,5);8,110(2,2);8,106(2,3);7,657(5,0);7,648(0,5);7,638(0,8);7,634(0,7);7,623(1,5);7,613(0,8);7,609(1,0);7,599(0,4);7,409(2,5);7,3 95(4,3);7,381(2,1);6,949(2,2);6,945(2,3);3,949(16,0);3,351(381,4);3,005(0,7);2,625(0,3);2,552(35,7);2,534(0,6);2,531(0,7);2,528(0,6);2,519( 16,5);2,516(36,9);2,513(52,2);2,510(38,7);2,507(18,8);2,397(0,3) |
| I-1-303: |
| HPLC-MS: logP = 3,37; Masse (m/z): 452,0 (M+H)⁺; ¹H-NMR(601,6 MHz, DMSO-D₆): |
| δ= 11,645(3,6);8,129(2,6);8,125(2,6);7,643(0,4);7,632(0,8);7,629(0,8);7,618(1,5);7,608(0,8);7,604(0,9);7,594(0,4);7,403(2,5);7,389(4,3);7,3 75(2,1);6,950(4,0);6,946(4,0);4,024(16,0);3,340(237,0);2,995(1,4);2,614(0,4);2,542(95,9);2,524(0,6);2,520(0,8);2,517(0,7);2,509(21,2);2,50 6(47,7);2,503(67,6);2,499(48,5);2,496(23,1);2,387(0,4);2,384(0,3);0,000(0,7) |
| I-1-304: |
| HPLC-MS: logP = 3,38; Masse (m/z): 398,0 (M+H)⁺; ¹H-NMR(400,0 MHz, DMSO-D₆): |
| δ= 10,720(3,2);8,043(2,7);8,037(2,7);7,635(0,4);7,619(0,8);7,614(0,8);7,603(0,6);7,598(1,7);7,592(0,7);7,582(0,8);7,577(1,1);7,561(0,5);7,3 93(3,0);7,372(4,9);7,351(2,2);6,893(2,2);6,887(2,2);5,757(0,8);3,327(95,9);2,676(0,3);2,671(0,4);2,667(0,3);2,506(52,2);2,502(68,5);2,497(5 0,3);2,395(15,8);2,333(0,4);2,329(0,5);2,324(0,4);2,248(16,0);2,232(0,8);0,008(0,8);0,000(17,4);-0,008(0,7) |
| I-1-305: |
| HPLC-MS: logP = 2,61; Masse (m/z): 385,9 (M+H)⁺; ¹H-NMR(400,0 MHz, DMSO-D₆): |
| δ= 11,108(9,1);8,316(0,6);8,212(11,4);8,203(11,7);8,053(8,5);8,047(8,5);7,773(14,7);7,765(14,0);7,638(1,3);7,623(2,9);7,617(2,4);7,607(2,0 );7,601(5,7);7,596(2,2);7,585(2,6);7,580(3,7);7,564(1,6);7,399(9,7);7,377(16,0);7,357(7,2);6,922(7,2);6,917(7,2);5,757(1,0);4,056(0,4);4,038 (1,2);4,020(1,2);4,002(0,4);3,327(323,2);2,689(1,0);2,675(1,1);2,671(1,5);2,666(1,1);2,541(1,1);2,524(5,0);2,511(86,5);2,506(173,0);2,502(2 27,5);2,497(164,2);2,493(79,4);2,333(1,0);2,329(1,4);2,324(1,1);1,989(5,2);1,398(1,0);1,351(0,4);1,259(0,4);1,233(0,6);1,193(1,4);1,175(2,7 );1,157(1,4);0,008(2,7);0,000(66,0);-0,008(2,5) |
| I-1-306: |
| HPLC-MS:logP=3,11;Masse(m/z):388,0(M+H)⁺;¹H-NMR(601,6MHz,DMSO-D₆): |
| δ=11,612(3,2);8,121(2,6);8,117(2,8);7,943(1,0);7,847(2,4);7,752(1,2);7,651(0,4);7,641(0,8);7,637(0,8);7,626(1,5);7,616(0,8);7,612(1,0);7,6 02(0,4);7,409(2,5);7,395(4,5);7,381(2,1);6,931(3,4);6,927(3,5);3,409(2,1);3,406(2,9);3,385(2693,6);3,359(2,2);2,625(0,8);2,552(8,8);2,534(1 ,2);2,531(1,5);2,528(1,5);2,519(43,2);2,516(94,9);2,513(133,7);2,510(99,7);2,507(49,2);2,397(0,8);2,275(16,0);1,243(1,0);0,008(0,5) |
| I-1-307: |
| HPLC-MS: logP = 2,78; Masse (m/z): 352,1 (M+H)⁺; ¹H-NMR(400,0 MHz, CD₃CN): |
| δ= 9,141(0,8);7,772(1,9);7,769(1,8);7,766(1,9);7,552(0,3);7,536(0,7);7,530(0,7);7,521(0,4);7,515(1,5);7,509(0,5);7,499(0,7);7,493(0,9);7,47 8(0,4);7,222(0,4);7,218(0,6);7,213(2,3);7,200(0,5);7,192(3,8);7,183(0,5);7,171(1,9);7,162(0,4);6,987(1,8);6,981(1,8);4,014(16,0);2,210(17,3) ;2,177(0,7);2,156(4,7);1,959(0,4);1,953(3,6);1,947(6,7);1,941(9,5);1,935(6,6);1,929(3,5);1,371(1,0);1,276(1,1);0,008(0,5);0,000(12,8);-0,009(0,6) |
| I-1-308: |
| HPLC-MS: logP = 2,61; Masse (m/z): 352,1 (M+H)⁺; ¹H-NMR(601,6 MHz, DMSO-D₆): |
| δ= 10,355(3,9);8,180(10,0);8,055(2,7);8,051(2,7);7,633(0,4);7,623(0,9);7,619(0,8);7,612(0,6);7,608(1,7);7,605(0,6);7,598(0,8);7,594(1,1);7, 584(0,4);7,400(2,9);7,386(4,9);7,372(2,5);6,899(2,9);6,895(2,9);4,217(2,0);4,205(6,1);4,193(6,2);4,181(2,0);3,386(0,3);3,384(0,3);3,379(0,4) ;3,373(0,9);3,359(357,8);3,343(0,4);3,339(0,7);3,337(0,7);2,552(28,7);2,534(0,4);2,531(0,5);2,528(0,5);2,519(12,4);2,516(27,6);2,513(38,8); 2,510(27,9);2,507(12,9);1,446(7,3);1,434(16,0);1,422(7,4) |
| I-1-309: |
| HPLC-MS: logP = 3,15; Masse (m/z): 366,1 (M+H)⁺; ¹H-NMR(601,6 MHz, DMSO-D₆): |
| δ= 11,237(2,3);8,102(2,2);8,098(2,2);7,649(0,3);7,638(0,7);7,634(0,7);7,624(1,4);7,613(0,7);7,610(0,8);7,599(0,4);7,408(2,3);7,394(4,0);7,3 80(1,9);6,938(1,9);6,934(1,9);4,239(1,0);4,227(3,1);4,215(3,1);4,203(1,0);3,346(381,3);3,006(1,7);2,625(0,4);2,552(81,0);2,534(0,7);2,531(0 ,8);2,528(0,8);2,519(22,8);2,516(50,5);2,513(71,5);2,510(52,9);2,507(25,4);2,397(0,4);2,184(16,0);1,347(4,8);1,335(10,4);1,323(4,8);1,246( 0,3);0,011(0,5) |
| I-1-310: |
| HPLC-MS: logP = 3,28; Masse (m/z): 411,1 (M+H)⁺; ¹H-NMR(601,6 MHz, DMSO-D₆): |
| δ= 11,875(3,1);8,147(2,1);8,143(2,1);7,637(0,7);7,633(0,6);7,622(1,2);7,612(0,7);7,608(0,8);7,598(0,3);7,408(2,1);7,394(3,6);7,380(1,8);6,9 61(3,6);6,957(3,6);4,900(0,4);4,889(1,2);4,878(1,6);4,867(1,2);4,856(0,4);3,364(0,4);3,337(831,4);2,995(1,4);2,617(0,6);2,614(0,8);2,611(0, 6);2,542(64,8);2,523(1,4);2,520(1,7);2,517(1,7);2,508(42,8);2,505(93,3);2,502(129,3);2,499(92,8);2,496(43,5);2,390(0,6);2,386(0,8);2,383(0 ,6);1,477(15,9);1,467(16,0);1,235(0,5);0,000(1,3) |
| I-1-311: |
| HPLC-MS: logP = 2,46; Masse (m/z): 338,0 (M+H)+; ¹H-NMR(601,6 MHz, DMSO-D₆): |
| δ= 11,297(2,2);8,110(2,2);8,106(2,2);7,665(4,3);7,648(0,4);7,638(0,8);7,634(0,7);7,623(1,4);7,613(0,8);7,609(0,9);7,599(0,4);7,409(2,4);7,3 95(4,1);7,381(2,0);6,946(1,9);6,942(1,9);3,949(16,0);3,353(587,8);3,005(0,6);2,625(0,5);2,622(0,3);2,552(32,7);2,534(0,7);2,531(0,8);2,528( 0,8);2,519(26,8);2,516(56,6);2,513(77,3);2,510(56,3);2,507(27,1);2,400(0,4);2,397(0,5);2,394(0,4) |
| I-1-312: |
| HPLC-MS: logP = 2,70; Masse (m/z): 383,0 (M+H)⁺; ¹H-NMR(400,0 MHz, DMSO-D₆): |
| δ= 11,784(0,3);11,027(2,6);8,315(0,4);8,080(1,9);8,074(1,9);7,631(0,7);7,625(0,6);7,615(0,5);7,609(1,3);7,604(0,6);7,593(0,6);7,588(0,8);7, 572(0,4);7,414(0,7);7,405(2,2);7,384(3,6);7,363(1,7);6,955(0,4);6,949(0,4);6,900(2,1);6,894(2,0);5,756(0,8);4,230(16,0);4,078(1,9);3,321(13 4,4);2,675(0,9);2,671(1,1);2,666(0,8);2,541(0,7);2,523(3,8);2,510(67,6);2,506(130,2);2,502(168,1);2,497(120,8);2,493(58,3);2,333(0,8);2,32 8(1,1);2,324(0,8);1,234(0,4);0,008(3,2);0,000(70,6);-0,009(2,4);-0,150(0,3) |
| I-1-313: |
| HPLC-MS: logP = 3,68; Masse (m/z): 380,1 (M+H)⁺; ¹H-NMR(601,6 MHz, DMSO-D₆): |
| δ= 11,222(2,5);8,101(2,4);8,098(2,3);7,647(0,4);7,636(0,9);7,632(0,8);7,622(1,5);7,612(0,8);7,608(1,0);7,598(0,4);7,407(2,5);7,393(4,5);7,3 79(2,2);6,936(2,0);6,933(2,0);3,893(16,0);3,351(861,8);2,628(0,5);2,625(0,7);2,553(21,2);2,544(2,8);2,532(5,9);2,519(43,2);2,516(84,1);2,5 13(113,6);2,510(82,3);2,507(39,8);2,397(0,7);1,667(0,3);1,654(1,6);1,642(3,0);1,630(3,1);1,617(1,7);1,605(0,4);1,246(0,6);0,951(4,5);0,939( 9,0);0,927(4,3);0,011 (0,8) |
| I-1-314: |
| HPLC-MS: logP = 3,32; Masse (m/z): 380,1 (M+H)⁺; ¹H-NMR(400,0 MHz, DMSO-D₆): |
| δ= 10,247(2,4);8,040(1,9);8,034(1,8);7,619(0,6);7,614(0,5);7,604(0,4);7,598(1,2);7,592(0,5);7,582(0,6);7,577(0,8);7,561(0,3);7,396(2,0);7,3 75(3,4);7,354(1,5);6,880(2,4);6,874(2,4);5,758(0,9);3,883(16,0);3,327(27,1);2,710(1,7);2,692(2,9);2,673(2,0);2,511(13,2);2,507(26,1);2,502( 33,9);2,498(24,2);2,493(11,5);1,614(1,1);1,595(2,0);1,576(2,0);1,558(1,1);0,945(3,6);0,927(7,5);0,908(3,2);0,008(0,3);0,000(8,5) |
| I-1-315: |
| HPLC-MS: logP = 3,05; Masse (m/z): 366,0 (M+H)⁺; ¹H-NMR(400,0 MHz, DMSO-D₆): |
| δ= 10,341(4,9);8,170(11,5);8,049(3,7);8,043(3,8);7,636(0,6);7,621(1,2);7,615(1,1);7,605(0,8);7,599(2,4);7,594(1,0);7,583(1,1);7,578(1,6);7, 562(0,7);7,398(4,1);7,377(6,9);7,356(3,1);6,890(4,4);6,884(4,4);4,132(4,0);4,115(7,9);4,097(4,1);3,327(30,2);2,511(18,8);2,507(37,7);2,503( 49,7);2,498(36,5);1,895(0,5);1,876(2,5);1,858(4,9);1,840(5,0);1,822(2,6);1,804(0,6);0,865(7,8);0,847(16,0);0,828(7,2);0,008(0,5);0,000(12,0 );-0,008(0,5) |
| I-1-316: |
| HPLC-MS: logP = 3,24; Masse (m/z): 366,1 (M+H)⁺; ¹H-NMR(601,6 MHz, DMSO-D₆): |
| δ= 11,213(2,2);8,100(2,1);8,096(2,1);7,647(0,4);7,636(0,8);7,632(0,7);7,626(0,6);7,622(1,5);7,612(0,7);7,608(0,9);7,597(0,4);7,406(2,4);7,3 92(4,1);7,378(2,1);6,936(1,7);6,932(1,7);3,892(16,0);3,361(437,6);3,358(597,8);2,628(0,4);2,625(0,5);2,622(0,4);2,594(1,3);2,581(4,2);2,56 9(4,3);2,556(1,5);2,552(4,2);2,534(0,8);2,531(1,0);2,528(1,0);2,519(25,8);2,516(56,6);2,513(78,4);2,510(56,2);2,507(26,2);2,400(0,4);2,397( 0,5);2,394(0,4);1,245(0,4);1,210(5,4);1,198(11,6);1,185(5,4);0,010(0,6) |
| I-1-317: |
| HPLC-MS: logP = 3,37; Masse (m/z): 447,1 (M+H)⁺; ¹H-NMR(601,6 MHz, DMSO-D₆): |
| δ= 12,168(3,0);8,170(2,7);8,166(2,7);7,655(0,4);7,644(0,8);7,630(1,5);7,616(0,9);7,606(0,4);7,419(2,4);7,405(4,2);7,391(2,0);6,972(3,5);6,9 68(3,5);4,145(16,0);3,428(1,0);3,418(0,6);3,386(3294,8);3,385(2796,6);3,358(5,6);3,339(2,1);3,296(0,7);2,628(0,9);2,625(1,2);2,622(0,9);2, 552(101,5);2,534(2,1);2,531(2,6);2,528(2,5);2,519(62,7);2,516(136,0);2,513(190,4);2,510(138,3);2,507(65,1);2,436(0,4);2,397(1,2);2,394(0, 9);2,310(0,4);1,266(0,4);1,243(1,2);0,008(1,1) |
| I-1-318: |
| HPLC-MS: logP = 2,98; Masse (m/z): 397,1 (M+H)⁺; ¹H-NMR(601,6 MHz, DMSO-D₆): |
| δ= 12,125(3,0);8,303(1,3);8,150(2,7);8,147(2,7);7,649(0,4);7,638(0,8);7,634(0,8);7,624(1,5);7,613(0,9);7,610(1,0);7,599(0,4);7,406(2,4);7,3 92(4,3);7,378(2,1);6,971(3,4);6,967(3,4);4,130(16,0);3,395(2352,3);2,625(0,6);2,622(0,4);2,552(7,5);2,534(0,9);2,531(1,2);2,528(1,1);2,519( 32,1);2,516(70,3);2,513(97,0);2,510(69,9);2,507(32,5);2,400(0,5);2,397(0,6);1,241(1,5);0,861(0,4);0,005(0,5) |
| I-1-319: |
| HPLC-MS: logP = 2,68; Masse (m/z): 348,0 (M+H)⁺; ¹H-NMR(400,0 MHz, DMSO-D₆): |
| δ= 11,731(5,2);8,316(0,4);8,111(8,7);8,108(7,0);8,105(8,6);7,650(1,4);7,634(3,1);7,628(2,3);7,618(2,0);7,613(6,0);7,607(2,1);7,596(2,5);7,5 91(3,8);7,575(1,7);7,417(1,5);7,413(2,7);7,408(9,9);7,395(1,9);7,387(16,0);7,366(7,5);7,361(2,1);6,945(5,6);6,940(5,5);5,757(7,3);3,322(215 ,2);2,675(2,3);2,671(2,3);2,666(2,1);2,662(2,6);2,655(2,4);2,651(1,8);2,642(3,9);2,629(2,3);2,622(2,2);2,609(1,1);2,541(0,6);2,524(5,3);2,51 9(8,5);2,511(103,2);2,506(208,2);2,502(277,1);2,497(200,7);2,493(95,4);2,337(0,7);2,333(1,4);2,328(1,9);2,324(1,4);2,319(0,6);1,351(0,3);1 ,336(0,9);1,298(0,9);1,259(1,3);1,249(1,4);1,243(1,9);1,230(5,8);1,224(10,1);1,217(6,2);1,209(5,2);1,202(10,2);1,195(8,0);1,188(9,3);1,182( 10,5);1,176(10,4);1,170(6,3);1,156(1,4);1,147(0,6);0,008(2,7);0,000(81,8);-0,009(2,5) |
| 1-1-320: |
| HPLC-MS: logP = 2,20; Masse (m/z): 336,1 (M+H)⁺; ¹H-NMR(601,6 MHz, DMSO-D₆): |
| δ= 10,155(2,5);8,042(1,8);8,038(1,8);7,621(0,6);7,617(0,5);7,607(1,1);7,596(0,6);7,593(0,7);7,397(1,8);7,383(3,1);7,369(1,5);6,875(2,6);6,8 71(2,6);3,806(16,0);3,362(756,0);3,333(0,5);3,005(0,5);2,625(0,4);2,552(27,0);2,534(0,6);2,531(0,7);2,528(0,6);2,519(18,6);2,516(40,9);2,5 13(57,6);2,510(42,5);2,507(20,3);2,397(0,4);2,263(8,5);2,262(8,3);1,245(1,2) |
| I-1-321: |
| HPLC-MS: logP = 2,27; Masse (m/z): 322,1 (M+H)⁺; ¹H-NMR(601,6 MHz, DMSO-D₆): |
| δ= 11,051(2,4);8,099(2,3);8,095(2,3);7,645(0,4);7,635(0,7);7,631(0,7);7,623(3,0);7,616(2,9);7,610(0,8);7,606(0,8);7,596(0,4);7,408(2,2);7,3 94(4,0);7,380(1,9);6,922(1,8);6,918(1,8);3,977(16,0);3,391(0,3);3,353(724,3);2,625(0,6);2,552(4,9);2,534(0,8);2,531(1,0);2,528(1,0);2,519(3 0,3);2,516(64,8);2,513(90,0);2,510(66,1);2,507(31,9);2,397(0,6);1,245(0,9);0,010(0,5) |
| I-1-322: |
| HPLC-MS: logP = 2,80; Masse (m/z): 386,1 (M+H)⁺; ¹H-NMR(400,0 MHz, DMSO-D₆): |
| δ= 11,350(11,5);8,068(8,6);8,062(8,5);8,037(0,5);8,027(0,5);8,015(0,5);7,809(3,6);7,796(3,8);7,788(5,1);7,781(5,9);7,775(9,8);7,758(5,1);7, 752(5,3);7,668(2,8);7,661(2,6);7,647(5,3);7,640(5,1);7,622(4,6);7,606(2,4);7,600(6,2);7,595(2,5);7,585(2,8);7,579(4,0);7,563(1,9);7,399(9,8) ;7,378(16,0);7,357(7,5);7,340(0,8);7,319(1,0);7,298(0,6);6,917(15,2);6,911(15,5);6,700(0,9);6,694(1,0);4,578(0,4);3,511(4,0);3,491(0,4);3,4 77(0,5);3,465(0,5);3,423(0,7);3,411(0,8);3,399(0,6);3,330(1984,0);3,294(3,0);3,265(1,1);3,207(0,5);2,995(0,3);2,680(1,7);2,675(3,5);2,671(4 ,9);2,666(3,5);2,662(1,7);2,541(30,6);2,524(14,8);2,519(23,9);2,511(268,4);2,506(536,6);2,502(711,5);2,497(517,2);2,493(247,3);2,338(1,6); 2,333(3,4);2,329(4,7);2,324(3,3);2,319(1,5);2,289(0,4);2,074(2,2);1,336(0,5);1,298(0,5);1,258(0,7);1,249(0,8);1,235(2,0);1,147(0,5);0,008(0, 9);0,000(21,7);-0,009(0,6) |
| I-1-323: |
| HPLC-MS: logP = 3,47; Masse (m/z): 472,1 (M+H)⁺; ¹H-NMR(601,6 MHz, DMSO-D₆): |
| δ= 11,302(2,6);8,100(2,0);8,097(2,0);7,636(0,8);7,632(0,8);7,626(5,8);7,622(1,5);7,612(0,7);7,608(0,8);7,597(0,3);7,409(2,0);7,395(3,6);7,3 81(1,7);6,931(2,4);6,927(2,4);4,094(3,4);4,082(3,5);3,369(0,4);3,347(442,3);2,625(0,4);2,552(48,7);2,534(0,7);2,531(0,9);2,528(0,8);2,519(2 3,5);2,516(51,1);2,513(70,8);2,510(50,9);2,507(24,3);2,400(0,3);2,397(0,5);2,394(0,3);2,100(0,4);2,088(0,8);2,077(1,1);2,066(0,9);2,054(0,5 );1,246(0,3);0,830(16,0);0,819(16,0);0,011(0,5) |
| I-1-324: |
| HPLC-MS: logP = 2,46; Masse (m/z): 430,0 (M+H)⁺; ¹H-NMR(601,6 MHz, DMSO-D₆): |
| δ= 11,277(2,7);8,106(2,3);7,647(0,4);7,637(0,9);7,633(0,8);7,622(1,6);7,608(6,9);7,598(0,5);7,408(2,6);7,394(4,5);7,380(2,2);6,949(2,3);6,9 45(2,3);3,950(16,0);3,355(676,1);3,354(837,4);2,628(0,6);2,625(0,8);2,622(0,6);2,552(12,6);2,534(1,2);2,531(1,5);2,528(1,5);2,519(44,2);2, 516(96,6);2,513(134,8);2,510(97,2);2,507(46,3);2,400(0,6);2,397(0,8);2,394(0,6);1,245(0,3);0,010(0,9) |
| I-1-325: |
| HPLC-MS: logP = 3,11; Masse (m/z): 458,1 (M+H)⁺; ¹H-NMR(601,6 MHz, DMSO-D₆): |
| δ= 11,301(4,3);8,102(3,3);8,099(3,3);7,647(0,5);7,637(1,2);7,633(1,2);7,622(10,2);7,612(1,3);7,608(1,4);7,598(0,6);7,409(3,4);7,394(6,0);7, 381(2,9);6,939(3,8);6,935(3,9);4,224(2,9);4,212(5,6);4,201(3,0);3,349(680,2);2,625(0,6);2,552(18,8);2,534(0,8);2,531(1,1);2,528(1,1);2,519( 28,9);2,516(63,6);2,513(89,7);2,510(66,1);2,507(31,9);2,397(0,6);1,787(0,5);1,775(2,3);1,763(4,6);1,751(4,6);1,739(2,5);1,727(0,6);1,246(0, 4);0,832(7,5);0,819(16,0);0,807(7,3) |
| I-1-326: |
| HPLC-MS: logP = 2,15; Masse (m/z): 322,0 (M+H)⁺; ¹H-NMR(400,0 MHz, DMSO-D₆): |
| δ= 11,688(1,6);8,109(2,4);8,106(2,1);8,102(2,4);7,649(0,4);7,633(0,8);7,627(0,6);7,618(0,6);7,612(1,6);7,606(0,6);7,595(0,7);7,590(1,0);7,5 75(0,5);7,416(0,4);7,407(2,7);7,385(4,4);7,365(2,0);6,938(1,8);6,932(1,8);5,756(1,3);3,337(0,3);3,323(34,9);2,827(16,0);2,524(1,0);2,520(1, 4);2,511(16,7);2,507(33,4);2,502(44,1);2,497(32,6);2,493(16,1);0,008(0,5);0,000(16,6);-0,009(0,7) |
| I-1-327: |
| HPLC-MS: logP = 2,24; Masse (m/z): 305,0 (M+H)⁺; ¹H-NMR(400,0 MHz, DMSO-D₆): |
| δ= 11,382(2,5);8,677(5,4);8,086(2,3);8,080(2,4);7,637(0,7);7,631(0,7);7,616(1,4);7,599(0,7);7,594(0,9);7,578(0,4);7,408(2,4);7,386(4,0);7,3 65(1,8);6,896(2,7);6,890(2,7);3,326(33,4);2,506(38,2);2,502(48,8);2,498(36,7);2,328(0,3);2,279(16,0);0,000(9,5) |
| I-1-328: |
| HPLC-MS: logP = 1,51; Masse (m/z): 305,1 (M+H)⁺; ¹H-NMR(400,0 MHz, DMSO-D₆): |
| δ= 11,159(2,4);8,715(4,9);8,083(2,2);8,078(2,2);7,654(0,3);7,638(1,0);7,633(0,9);7,617(1,4);7,611(0,6);7,600(0,7);7,595(0,9);7,579(0,4);7,4 06(2,3);7,385(3,8);7,365(1,8);7,356(0,7);7,295(0,3);7,274(0,6);6,876(2,8);6,870(2,8);5,756(0,3);5,741(0,4);5,735(0,4);4,966(0,5);3,922(16,0) ;3,322(49,8);2,675(0,6);2,671(0,7);2,666(0,6);2,541(0,4);2,510(49,8);2,506(93,6);2,502(118,3);2,497(88,7);2,333(0,6);2,328(0,8);2,324(0,6); 2,027(1,2);0,000(55,9) |
| I-1-329: |
| HPLC-MS: logP = 3,11; Masse (m/z): 402,1 (M+H)⁺; ¹H-NMR(400,0 MHz, DMSO-D₆): |
| δ= 11,412(11,4);8,076(8,6);8,073(7,5);8,070(8,6);7,872(15,1);7,866(10,4);7,852(12,7);7,788(5,6);7,786(6,4);7,783(5,9);7,781(5,3);7,767(3,5 );7,765(3,9);7,762(3,8);7,760(3,4);7,639(1,5);7,624(3,1);7,617(2,4);7,608(2,1);7,602(6,0);7,596(2,2);7,586(2,6);7,581(3,9);7,565(1,7);7,408( 1,5);7,404(2,7);7,399(10,0);7,378(16,0);7,357(7,5);7,352(2,4);6,925(12,2);6,918(12,2);3,516(0,7);3,346(113,2);2,713(0,4);2,558(0,6);2,543( 103,6);2,530(0,6);2,527(0,7);2,522(0,9);2,513(12,3);2,509(25,3);2,504(33,9);2,499(25,1);2,495(12,3);2,369(0,4);0,000(3,6) |
| I-1-330: |
| HPLC-MS: logP = 2,14; Masse (m/z): 336,0 (M+H)⁺; ¹H-NMR(400,0 MHz, DMSO-D₆): |
| δ= 11,565(7,4);9,260(15,9);9,147(16,0);8,316(0,5);8,124(6,3);8,119(6,6);7,648(0,9);7,632(1,8);7,627(1,8);7,611(3,8);7,595(2,0);7,590(2,5);7 ,574(1,1);7,408(6,4);7,387(10,9);7,366(4,9);6,946(9,0);6,940(9,1);3,328(297,0);2,671(1,6);2,506(183,2);2,502(239,5);2,498(184,0);2,329(1,5 );0,000(5,0) |
| I-1-331: |
| HPLC-MS: logP = 2,80; Masse (m/z): 386,1 (M+H)⁺; ¹H-NMR(400,0 MHz, DMSO-D₆): |
| δ= 11,405(11,1);8,078(9,0);8,072(8,9);7,932(4,4);7,920(4,7);7,910(5,0);7,898(4,9);7,691(4,5);7,685(4,8);7,669(4,6);7,663(4,6);7,639(1,4);7, 623(3,1);7,617(2,5);7,608(2,1);7,602(6,0);7,596(2,3);7,586(2,7);7,581(4,0);7,565(3,7);7,546(4,6);7,540(4,0);7,524(2,3);7,519(2,0);7,408(1,6) ;7,399(9,9);7,378(16,0);7,357(7,5);6,928(14,5);6,922(14,4);3,516(0,7);3,340(70,2);2,543(60,1);2,529(0,5);2,526(0,7);2,521(0,9);2,512(12,0); 2,508(24,4);2,503(32,5);2,499(23,9);2,494(11,6);2,075(0,4);0,000(2,6) |
| I-1-332: |
| HPLC-MS: logP = 2,45; Masse (m/z): 322,0 (M+H)⁺; ¹H-NMR(400,0 MHz, DMSO-D₆): |
| δ= 11,196(2,1);8,097(1,9);8,091(1,9);7,636(0,7);7,631(0,5);7,620(0,5);7,615(1,4);7,609(0,5);7,598(0,6);7,593(0,9);7,577(0,4);7,418(0,4);7,4 09(2,2);7,388(3,6);7,367(1,7);7,361(0,5);6,953(2,3);6,947(2,3);3,324(5,8);2,903(16,0);2,512(5,5);2,507(10,8);2,503(14,3);2,498(10,4);2,494( 5,0);0,008(0,9);0,000(22,7);-0,009(0,8) |
| I-1-333: |
| HPLC-MS: logP = 2,30; Masse (m/z): 320.1 (M+H)⁺; ¹H-NMR [DMSO-D₆] 1.75 - 1.81 (m, 2H), 1.98 (s, 3H), 2.30 - 2.33 (m, 2H), 3.94 - 3.96 (m, 2H), 6.82 (d, 1H), 7.34 - 7.38 (m, 2H), 7.54 - 7.62 (m, 1H), 7.96 (d, 1H), 10.04 (s, 1H). |
| I-1-334: |
| HPLC-MS: logP = 2,70; Masse (m/z): 378,0 (M+H)⁺; ¹H-NMR(400,0 MHz, DMSO-D₆): |
| δ= 11,510(3,9);11,214(0,3);8,063(5,4);8,057(5,3);7,845(2,9);7,826(3,1);7,823(3,0);7,603(0,7);7,586(1,4);7,582(1,5);7,575(1,3);7,570(1,2);7, 565(3,3);7,561(7,6);7,559(4,3);7,555(5,0);7,545(4,2);7,542(3,6);7,526(1,2);7,522(1,0);7,446(2,1);7,440(2,0);7,430(1,6);7,426(2,1);7,424(1,8) ;7,420(1,9);7,410(1,5);7,404(1,4);7,246(0,6);7,243(0,8);7,236(4,1);7,217(5,3);7,196(3,4);7,189(0,6);6,895(5,9);6,889(5,9);5,756(6,9);3,326(1 2,6);2,524(0,5);2,519(0,8);2,511(8,7);2,506(17,4);2,502(22,9);2,497(16,5);2,493(7,8);1,989(0,4);1,397(16,0);1,234(0,3);1,071(0,4);0,008(0,5 );0,000(14,3);-0,009(0,4) |
| I-1-335: |
| HPLC-MS: logP = 3,02 (neutral); Masse (m/z): 430,0 (M+H)⁺; ¹H-NMR(400,0 MHz, DMSO-D₆): |
| δ= 11,523(10,4);8,065(13,6);8,059(13,6);7,845(7,0);7,842(7,2);7,825(7,9);7,822(7,9);7,768(0,9);7,749(2,6);7,739(1,4);7,729(5,7);7,715(9,7); 7,695(16,0);7,679(4,5);7,675(3,5);7,588(2,6);7,583(4,0);7,568(10,6);7,563(11,1);7,560(7,2);7,557(6,4);7,542(7,2);7,539(7,3);7,522(2,8);7,51 9(2,8);7,441(4,9);7,436(5,0);7,424(4,4);7,421(5,3);7,419(4,7);7,416(5,1);7,404(3,4);7,399(3,3);6,880(14,7);6,874(14,6);4,115(1,0);4,102(3,2) ;4,089(3,2);4,075(1,1);3,327(20,0);3,177(15,6);3,163(15,2);2,671(0,4);2,524(1,2);2,511(22,0);2,506(44,2);2,502(58,1);2,497(42,0);2,493(20, 2);2,329(0,4);0,008(1,8);0,000(48,4);-0,009(1,6) |
| I-1-336: |
| HPLC-MS: logP = 1,44; Masse (m/z): 357,0 (M+H)⁺; ¹H-NMR(400,0 MHz, CD₃CN): |
| δ= 9,331(0,8);8,555(1,7);8,551(1,8);8,543(1,8);8,539(1,8);7,861(1,6);7,858(1,6);7,839(4,2);7,834(3,2);7,782(2,3);7,763(2,8);7,501(3,1);7,49 0(5,5);7,393(1,5);7,384(1,1);7,379(1,1);7,372(1,6);7,370(1,4);7,363(1,0);7,360(1,1);7,350(1,0);7,275(1,5);7,263(1,5);7,256(1,5);7,244(1,4);6, 978(2,4);6,972(2,4);5,451(2,0);2,627(16,0);2,469(0,3);2,465(0,3);2,229(85,5);1,966(0,7);1,955(10,9);1,948(20,5);1,942(28,8);1,936(19,9);1, 930(10,3);0,000(4,9) |
| I-1-337: siehe Synthesebeispiel 37 |
| I-1-338: |
| HPLC-MS: logP = 3,23; Masse (m/z): 390,0 (M+H)⁺; ¹H-NMR(601,6 MHz, CD₃CN): |
| δ= 9,194(0,9);7,826(3,6);7,822(3,5);7,684(2,1);7,682(2,1);7,670(2,3);7,668(2,3);7,553(1,8);7,550(1,9);7,540(2,3);7,537(2,4);7,466(1,3);7,46 4(1,4);7,453(2,7);7,451 (2,7);7,441 (1,5);7,439(1,4);7,420(2,5);7,419(2,6);7,396(2,0);7,394(4,3);7,383(2,2);7,381(5,6);7,371 (1,2);7,368(1,1);7, 260(1,5);7,259(1,6);7,257(1,6);7,256(1,4);7,246(1,2);7,245(1,4);7,244(1,3);7,242(1,2);6,949(3,9);6,944(3,9);5,448(2,0);2,472(0,4);2,386(16, 0);2,159(51,2);1,972(0,5);1,965(1,0);1,957(0,9);1,953(1,1);1,949(11,0);1,945(21,2);1,941(31,5);1,937(21,1);1,933(10,2);1,436(1,8);0,000(2, 7) |
| I-1-339: |
| HPLC-MS: logP = 3,20; Masse (m/z): 346,0 (M+H)⁺; ¹H-NMR(601,6 MHz, CD₃CN): |
| δ= 9,228(1,0);7,824(3,3);7,820(3,3);7,594(1,6);7,591(1,7);7,581(1,9);7,579(2,0);7,509(1,3);7,496(2,7);7,481(1,1);7,478(1,2);7,468(1,7);7,46 6(1,8);7,455(0,9);7,453(0,8);7,419(3,8);7,409(2,2);7,392(2,9);7,379(3,4);7,256(1,7);7,243(1,4);6,952(3,3);6,947(3,3);5,448(0,6);2,471(0,5);2, 385(16,0);2,160(30,7);1,965(0,5);1,957(0,5);1,952(0,7);1,949(5,8);1,945(10,6);1,941(15,5);1,937(10,6);1,933(5,4);1,436(1,5);0,000(1,2) |
| I-1-340: |
| HPLC-MS: logP = 3,15; Masse (m/z): 410,1 (M+H)⁺; ¹H-NMR(400,0 MHz, DMSO-D₆): |
| δ= 11,192(5,8);10,394(1,0);10,385(1,0);8,316(0,8);7,828(2,7);7,809(4,1);7,797(1,1);7,778(7,4);7,772(7,6);7,753(3,0);7,735(2,9);7,699(2,7);7 ,685(6,2);7,667(3,6);7,592(1,1);7,574(0,9);7,495(2,5);7,474(5,2);7,453(3,4);7,228(4,8);7,219(5,0);7,207(4,0);7,199(4,1);7,024(1,3);7,015(1,3 );6,979(0,5);6,973(0,6);6,961(0,9);6,956(1,2);6,945(0,6);6,930(1,8);6,924(2,4);6,904(0,9);6,827(6,1);6,821(6,2);4,158(0,7);4,140(2,1);4,123( 2,2);4,112(2,5);4,106(1,4);4,094(7,3);4,077(7,4);4,059(2,4);3,321(177,0);2,890(0,5);2,731(0,5);2,675(2,2);2,670(2,9);2,666(2,3);2,540(2,5);2 ,505(343,0);2,501(452,3);2,497(347,8);2,328(2,8);2,324(2,2);1,753(1,0);1,384(2,2);1,367(4,5);1,349(2,2);1,221(7,8);1,204(16,0);1,187(7,6); 0,146(0,6);0,008(5,9);0,000(127,4);-0,150(0,6) |
| I-1-341: |
| HPLC-MS: logP = 2,17; Masse (m/z): 376,1 (M+H)⁺; ¹H-NMR(400,0 MHz, CD₃CN): |
| δ= 9,308(0,6);8,079(1,5);8,060(1,6);7,884(2,4);7,878(2,4);7,807(0,5);7,804(0,5);7,789(1,6);7,783(0,7);7,770(1,4);7,767(1,2);7,740(0,8);7,73 6(1,3);7,718(3,7);7,701(1,6);7,698(1,6);7,610(1,1);7,605(0,8);7,603(0,7);7,592(1,3);7,586(1,4);7,557(1,0);7,551(1,1);7,540(1,0);7,537(1,0);7, 533(1,6);7,478(0,4);7,473(0,6);7,459(1,5);7,454(1,4);7,447(1,5);7,441(2,3);7,435(1,1);7,429(1,3);7,423(1,1);7,410(0,4);6,948(2,5);6,942(2,4) ;5,446(1,3);4,085(0,4);4,067(1,1);4,049(1,2);4,031(0,4);3,310(16,0);2,137(1,9);2,135(1,7);1,971(5,1);1,951(4,6);1,945(8,8);1,939(12,5);1,93 3(8,6);1,926(4,4);1,436(2,8);1,372(0,9);1,284(0,3);1,276(1,1);1,221(1,4);1,203(2,7);1,185(1,3);0,008(0,6);0,000(16,0);-0,009(0,5) |
| I-1-342: |
| HPLC-MS: logP = 1,82; Masse (m/z): 390,0 (M+H)⁺; ¹H-NMR(400,0 MHz, CD₃CN): |
| δ= 8,643(0,6);7,999(1,0);7,996(0,8);7,981(1,2);7,977(1,0);7,711(0,3);7,708(0,4);7,692(1,0);7,689(1,1);7,675(1,3);7,672(1,7);7,667(1,4);7,65 3(1,3);7,649(1,2);7,634(0,5);7,630(0,4);7,599(0,9);7,594(0,6);7,586(0,4);7,583(0,5);7,579(0,9);7,574(0,8);7,491(0,4);7,484(0,7);7,477(0,8);7, 476(0,8);7,468(1,7);7,463(2,2);7,459(1,6);7,452(2,4);7,446(1,6);7,442(0,6);7,432(1,4);7,428(1,5);7,415(1,0);7,413(1,0);7,410(1,0);6,489(2,8) ;5,446(2,1);3,212(16,0);2,285(11,2);2,132(10,2);1,963(0,8);1,957(1,1);1,951(9,3);1,945(17,2);1,939(24,0);1,933(16,5);1,927(8,5);1,372(3,2); 1,340(1,2);1,285(1,6);1,277(3,6);1,271(0,7) |
| I-1-343: |
| HPLC-MS: logP = 2,87; Masse (m/z): 396,0 (M+H)⁺; ¹H-NMR(400,0 MHz, CD₃CN): |
| δ= 8,534(2,7);7,607(4,4);7,593(8,8);7,580(4,6);7,575(8,8);7,560(5,5);7,557(5,4);7,541(4,1);7,537(3,8);7,507(2,2);7,498(2,6);7,493(3,1);7,48 4(5,8);7,465(7,2);7,457(16,0);7,442(4,9);7,439(3,9);7,427(5,0);7,408(6,8);7,389(2,9);7,350(4,9);7,330(4,0);6,489(10,0);5,447(0,7);2,276(45, 3);2,146(34,7);2,113(0,5);2,107(0,4);1,963(1,9);1,957(2,6);1,951(18,2);1,945(34,7);1,939(49,0);1,933(35,8);1,927(19,6);1,372(5,9);1,340(0, 4);1,285(0,8);1,276(6,2);0,080(1,8);0,008(2,6);0,000(60,0) |
| I-1-344: |
| HPLC-MS: logP = 3,10; Masse (m/z): 383,0 (M+H)⁺; ¹H-NMR(400,0 MHz, CD₃CN): |
| δ= 9,373(3,8);8,426(6,9);8,414(7,0);8,282(6,8);8,263(7,0);7,893(10,8);7,888(11,0);7,610(5,5);7,607(5,0);7,590(6,8);7,574(0,7);7,556(5,1);7, 539(7,8);7,480(8,5);7,466(12,4);7,462(13,1);7,448(16,0);7,435(6,3);7,433(6,4);7,417(2,1);7,253(0,3);7,236(0,4);6,974(10,8);6,969(10,9);3,8 43(0,7);2,462(0,4);2,147(20,7);2,143(34,3);2,111(0,4);2,107(0,5);2,101(0,3);1,952(25,4);1,949(27,8);1,946(49,1);1,943(50,1);1,940(69,9);1, 937(66,5);1,934(50,4);1,931(45,8);1,928(27,0);1,925(23,2);1,768(0,4);1,436(0,7);1,372(5,8);1,349(0,5);1,340(0,9);1,282(1,5);1,276(6,0);0,1 46(0,5);0,000(89,3);-0,002(79,0);-0,150(0,5) |
| I-1-345: |
| HPLC-MS: logP = 3,01; Masse (m/z): 380,1 (M+H)⁺; ¹H-NMR(400,0 MHz, DMSO-D₆): |
| δ= 10,538(3,9);7,952(4,8);7,951(4,8);7,857(1,7);7,851(0,6);7,839(2,7);7,812(0,8);7,794(2,0);7,776(1,8);7,727(1,9);7,718(3,0);7,709(2,3);7,7 01(1,9);7,690(1,0);7,684(2,3);7,680(1,9);7,665(2,7);7,661(2,5);7,587(1,6);7,583(1,9);7,568(2,6);7,563(3,0);7,527(1,1);7,523(1,3);7,508(2,6); 7,504(2,3);7,489(1,9);7,485(3,4);7,480(2,1);7,466(2,2);7,461(2,1);7,447(0,8);7,442(0,7);5,753(0,6);4,056(0,4);4,038(1,2);4,020(1,2);4,003(0, 4);3,399(0,4);3,357(194,9);2,525(0,6);2,512(11,8);2,508(23,3);2,503(30,2);2,499(21,1);2,494(9,6);2,073(1,5);2,044(16,0);1,989(5,7);1,397(1 ,8);1,193(1,5);1,175(2,9);1,157(1,4);0,000(0,3) |
| I-1-346: |
| HPLC-MS: logP = 2,53; Masse (m/z): 391,0 (M+H)⁺; ¹H-NMR(400,0 MHz, CD₃CN): |
| δ= 8,968(2,5);8,115(0,4);8,089(16,0);7,780(3,6);7,764(4,6);7,758(5,1);7,745(1,1);7,727(0,6);7,706(0,4);7,692(1,4);7,677(5,6);7,672(7,4);7,6 63(13,4);7,659(8,7);7,656(10,5);7,639(9,0);7,637(8,6);7,591(3,3);7,585(3,6);7,573(4,8);7,568(6,4);7,549(6,3);7,548(6,3);7,536(9,0);7,523(8, 1);7,520(7,2);7,506(5,5);7,503(5,5);7,486(2,4);7,483(2,1);7,445(1,0);7,433(4,6);7,427(4,9);7,412(4,5);5,448(1,7);3,871(4,7);3,833(1,6);3,784 |
| (0,6);3,772(1,0);3,759(0,9);3,657(0,9);3,644(1,0);3,633(0,6);2,148(37,0);2,108(1,1);2,102(0,9);1,965(1,3);1,953(15,7);1,947(29,8);1,940(42, 2);1,934(30,8);1,928(17,5);1,1,372(4,6);1,340(0,6);1,285(0,9);1,276(5,1);0,008(2,2);0,000(51,7) |
| I-1-347: |
| HPLC-MS: logP = 2,58; Masse (m/z): 380,0 (M+H)⁺; ¹H-NMR(400,0 MHz, CD₃CN): |
| δ= 8,552(2,2);7,736(3,4);7,719(4,6);7,715(4,5);7,652(1,2);7,634(3,8);7,620(7,4);7,615(7,2);7,599(3,7);7,598(3,7);7,588(2,7);7,585(4,5);7,57 0(3,1);7,567(5,5);7,564(4,0);7,554(0,4);7,495(1,7);7,486(2,3);7,480(2,6);7,472(5,1);7,459(3,7);7,450(16,0);7,440(7,7);7,436(7,6);7,418(3,7); 6,483(12,4);5,447(3,2);2,279(50,1);2,152(135,0);2,118(0,5);2,114(0,4);2,107(0,5);2,101(0,3);1,964(3,3);1,958(3,7);1,952(27,2);1,946(50,7); 1,940(70,3);1,934(48,5);1,928(25,0);1,768(0,4);0,008(0,6);0,000(15,6);-0,009(0,6) |
| I-1-348: |
| HPLC-MS: logP = 2,53; Masse (m/z): 381,0 (M+H)⁺; ¹H-NMR(400,0 MHz, DMSO-D₆): |
| δ= 10,720(1,1);8,862(0,6);8,852(0,6);8,850(0,6);8,214(0,5);8,196(0,6);8,194(0,6);7,962(1,3);7,961(1,3);7,864(0,5);7,852(0,5);7,844(0,5);7,8 32(0,5);7,686(0,6);7,682(0,5);7,668(0,8);7,663(0,7);7,588(0,5);7,583(0,6);7,568(0,7);7,564(0,9);7,524(0,4);7,510(0,8);7,505(0,7);7,490(1,1); 7,485(0,9);7,471(0,6);7,466(0,6);5,755(16,0);3,323(3,9);2,519(0,4);2,511(4,8);2,506(9,7);2,502(12,7);2,497(9,0);2,493(4,2);2,057(4,5);1,989 (0,3);0,000(6,8) |
| I-1-349: |
| HPLC-MS: logP = 2,13; Masse (m/z): 381,0 (M+H)⁺; ¹H-NMR(400,0 MHz, CD₃CN): |
| δ= 8,739(4,5);8,728(4,6);8,664(2,1);7,847(4,0);7,829(4,7);7,828(4,8);7,639(4,1);7,627(4,2);7,619(3,7);7,607(3,4);7,585(4,8);7,570(3,7);7,56 7(6,1);7,501(1,9);7,491(2,8);7,488(2,9);7,482(1,3);7,478(5,1);7,473(1,6);7,468(2,3);7,459(6,8);7,452(15,9);7,450(16,0);7,443(5,2);7,441(4,8) ;7,439(5,0);7,435(2,9);7,419(0,5);7,416(0,5);6,520(13,7);5,447(7,5);2,281(53,3);2,149(45,2);2,120(0,6);2,113(0,4);2,107(0,4);1,964(1,9);1,9 57(2,4);1,952(19,1);1,946(35,7);1,939(50,0);1,933(34,9);1,927(18,3);0,008(2,2);0,000(58,1);-0,009(2,7) |
| I-1-350: |
| HPLC-MS: logP = 3,20; Masse (m/z): 366,0 (M+H)⁺; ¹H-NMR(601,6 MHz, CD₃CN): |
| δ= 9,244(3,5);9,200(0,6);7,890(13,3);7,885(13,4);7,665(7,2);7,662(7,7);7,651(8,1);7,649(8,5);7,604(6,3);7,601(5,4);7,592(7,8);7,589(7,7);7, 578(0,4);7,558(0,4);7,556(0,4);7,539(6,0);7,536(6,8);7,526(13,9);7,524(16,0);7,513(9,4);7,511(9,2);7,470(2,6);7,467(3,2);7,458(7,6);7,455(7 ,5);7,445(12,1);7,442(10,8);7,432(7,0);7,429(6,6);7,420(2,8);7,416(10,8);7,403(13,8);7,390(6,7);7,355(0,5);7,341(0,5);6,971(13,8);6,967(13, 6);5,449(0,7);2,152(176,2);2,056(0,5);2,052(0,8);2,048(0,5);1,1,972(0,4);1,965(5,0);1,957(4,0);1,953(5,3);1,949(49,8);1,945(92,0);1,941(132, 5);1,937(91,5);1,933(46,6);1,831(0,5);1,826(0,8);1,822(0,5);1,436(5,6);1,284(0,7);1,269(1,4);1,213(0,6);1,201(1,0);1,189(0,5);1,026(0,5);1,0 15(1,0);1,003(0,5);0,005(0,7);0,000(22,6);-0,006(0,7) |
| I-1-351: |
| HPLC-MS: logP = 3,00; Masse (m/z): 366,0 (M+H)⁺; ¹H-NMR(601,6 MHz, CD₃CN): |
| δ= 9,329(1,5);7,892(7,3);7,888(7,4);7,608(3,6);7,605(2,6);7,604(2,6);7,596(4,8);7,592(4,4);7,547(3,3);7,543(4,2);7,535(2,9);7,534(3,2);7,53 1(4,9);7,486(6,8);7,484(8,0);7,479(0,3);7,4721(15,4);7,4715(16,0);7,460(4,7);7,457(4,6);7,449(5,4);7,448(5,4);7,445(5,8);7,440(8,4);7,436(4 ,4);7,433(4,0);7,428(5,4);7,425(4,6);7,421(1,3);7,413(3,0);7,377(0,5);7,367(0,3);6,978(8,0);6,974(8,0);5,448(2,4);2,151(90,6);2,052(0,4);1,9 65(2,9);1,957(2,2);1,953(2,6);1,949(28,0);1,945(51,8);1,941(74,6);1,937(51,9);1,933(26,5);1,924(0,5);1,826(0,4);1,436(0,7);0,000(11,3);-0,006(0,4) |
| I-1-352: |
| HPLC-MS: logP = 2,70; Masse (m/z): 348,1 (M+H)⁺; ¹H-NMR(400,0 MHz, DMSO-D₆): |
| δ= 10,719(3,4);7,965(4,6);7,963(4,5);7,739(0,4);7,686(2,0);7,682(1,7);7,668(2,7);7,663(2,5);7,618(0,5);7,601(1,1);7,594(1,9);7,588(2,2);7,5 85(0,9);7,580(2,1);7,575(2,7);7,570(3,1);7,563(1,1);7,559(1,2);7,542(0,6);7,527(0,9);7,523(1,1);7,508(2,6);7,504(2,3);7,490(4,1);7,485(3,7); 7,471(2,2);7,466(2,1);7,453(0,8);7,448(0,7);7,383(0,3);7,265(0,6);7,258(3,2);7,238(4,3);7,218(2,7);7,210(0,5);7,040(0,4);5,753(0,5);3,354(3 07,1);2,526(0,6);2,521(1,1);2,512(17,0);2,508(34,7);2,503(45,6);2,499(32,2);2,494(15,0);2,073(1,3);2,031(16,0);1,994(1,4);1,044(0,3);1,028 (0,3);0,000(0,5) |
| I-1-353: |
| HPLC-MS: logP = 2,97; Masse (m/z): 326,1 (M+H)⁺; ¹H-NMR(601,6 MHz, CD₃CN): |
| δ= 9,063(0,4);7,895(1,7);7,894(1,7);7,891(1,8);7,890(1,7);7,603(0,9);7,600(0,8);7,590(1,3);7,587(1,1);7,534(0,9);7,531(1,1);7,522(1,0);7,52 1(1,0);7,519(1,2);7,466(0,5);7,463(0,6);7,453(1,3);7,451(1,1);7,441(0,9);7,438(0,8);7,434(1,1);7,431(1,1);7,421(1,0);7,418(1,1);7,409(0,4);7, 406(0,4);7,235(0,6);7,222(1,2);7,209(1,0);7,102(2,1);7,089(1,7);7,006(2,0);7,002(1,9);2,318(16,0);2,248(0,4);2,191(73,9);1,973(0,4);1,967(1 7,6);1,958(0,6);1,954(0,7);1,1,950(6,2);1,946(11,6);1,942(16,6);1,938(10,8);1,934(5,5);0,000(0,6) |
| I-1-354: |
| HPLC-MS: logP = 2,86; Masse (m/z): 390,0 (M+H)⁺; ¹H-NMR(400,0 MHz, DMSO-D₆): |
| δ= 10,442(3,7);8,310(0,4);7,948(4,8);7,727(2,4);7,726(2,4);7,708(2,8);7,706(2,7);7,682(2,0);7,678(1,9);7,663(2,5);7,659(2,4);7,586(1,6);7,5 82(1,9);7,567(4,0);7,562(4,4);7,548(3,0);7,544(2,9);7,525(1,2);7,521(1,4);7,515(1,7);7,512(1,8);7,507(2,7);7,503(2,5);7,496(2,9);7,494(2,8); 7,488(2,1);7,483(3,3);7,477(3,2);7,463(2,2);7,458(2,0);7,444(1,0);7,439(2,0);7,434(1,6);7,419(2,0);7,415(1,9);7,400(1, 0);7, 396(0,9);5,751 (6, 2);3,422(0,5);3,365(324,7);3,325(0,7);2,526(0,7);2,521(1,1);2,513(14,9);2,508(30,1);2,504(39,1);2,499(27,3);2,494(12,4);2,083(16,0);2,072( 2,4);2,063(0,4);1,989(0,9);1,397(0,7);1,175(0,4);0,000(0,6) |
| I-1-355: |
| HPLC-MS: logP = 1,94; Masse (m/z): 391,0 (M+H)⁺; ¹H-NMR(400,0 MHz, CD₃CN): |
| δ= 8,888(1,4);7,673(3,4);7,671(3,4);7,653(4,0);7,651(3,9);7,613(2,5);7,608(3,1);7,595(2,7);7,593(3,0);7,590(3,7);7,528(2,5);7,523(2,8);7,50 9(4,7);7,505(5,7);7,493(3,3);7,487(3,7);7,475(3,7);7,470(2,9);7,468(3,6);7,456(6,0);7,452(5,2);7,438(7,0);7,434(9,4);7,429(4,0);7,419(2,7);7, 416(3,3);7,409(1,3);7,385(2,8);7,380(2,8);7,365(3,3);7,360(3,2);7,346(1,6);7,342(1,4);6,085(11,7);5,446(16,0);4,257(1,1);4,067(0,3);2,155(4 |
| ,8);2,112(0,8);2,106(0,7);2,100(0,5);2,094(0,4);1,971(1,3);1,963(1,8);1,957(1,8);1,951(14,1);1,945(26,5);1,939(36,8);1,933(25,2);1,926(13,0 );1,913(0,3);1,436(0,4);1,372(1,5);1,340(0,6);1,285(0,8);1,276(1,9);1,269(0,8);1,221(0,4);1,203(0,7);1,185(0,4);0,008(0,5);0,000(16,3);-0,009(0,6) |
| I-1-356: |
| HPLC-MS: logP = 2,45; Masse (m/z): 390,0 (M+H)⁺; ¹H-NMR(400,0 MHz, CD₃CN): |
| δ= 8,467(3,2);7,588(7,7);7,580(5,9);7,568(10,6);7,561(6,8);7,556(5,5);7,493(2,4);7,486(2,9);7,484(2,6);7,477(5,4);7,471(8,5);7,464(9,5);7,4 59(9,6);7,454(16,0);7,449(10,3);7,441(2,7);7,438(3,5);7,433(3,2);7,418(1,5);7,414(1,2);7,396(1,5);7,377(5,3);7,362(14,1);7,355(12,3);7,343( 3,3);7,335(7,0);7,329(3,8);7,316(5,4);7,313(4,0);7,309(3,8);7,299(2,7);7,293(2,4);6,480(14,3);5,445(4,8);4,066(0,6);4,048(0,6);2,436(0,3);2, 279(53,7);2,176(0,9);2,117(0,5);1,969(2,8);1,962(1,2);1,955(1,7);1,950(12,8);1,944(23,9);1,938(33,5);1,932(23,6);1,925(12,6);1,436(1,5);1, 371(3,6);1,284(0,5);1,276(3,7);1,220(0,7);1,202(1,4);1,184(0,7);0,007(2,8);0,000(60,2);-0,008(3,7);-0,150(0,3) |
| I-1-357: |
| HPLC-MS: logP = 2,23; Masse (m/z): 392,9 (M+H)⁺; ¹H-NMR(400,0 MHz, DMSO-D₆): |
| δ= 10,632(3,7);8,501(2,1);8,496(2,3);8,489(2,3);8,484(2,2);8,315(0,3);8,006(2,1);8,001(2,2);7,987(2,4);7,982(2,3);7,956(4,6);7,685(1,9);7,6 81(1,7);7,666(2,4);7,662(2,3);7,589(2,7);7,581(2,2);7,577(2,6);7,570(2,9);7,562(3,1);7,558(2,6);7,526(0,9);7,522(1,0);7,508(2,3);7,503(2,1); 7,488(2,9);7,482(2,4);7,468(2,0);7,463(1,9);7,450(0,7);7,445(0,6);3,321(88,1);2,675(0,5);2,670(0,8);2,666(0,6);2,540(0,4);2,523(1,9);2,510( 46,9);2,506(94,1);2,501(123,0);2,497(87,4);2,492(41,3);2,332(0,6);2,328(0,8);2,323(0,6);2,094(16,0);2,074(0,3);2,041(0,6);0,000(0,6) |
| I-1-358: |
| HPLC-MS: logP = 2,22; Masse (m/z): 376,9 (M+H)⁺; ¹H-NMR(400,0 MHz, DMSO-D₆): |
| δ= 11,383(4,1);11,344(0,9);8,480(2,2);8,475(3,1);8,468(3,1);8,463(3,3);8,446(1,1);8,439(1,2);8,435(1,1);8,154(0,5);8,140(0,5);8,135(0,7);8, 129(0,7);8,108(3,7);8,102(5,2);8,085(1,0);8,079(1,1);8,069(1,1);7,995(2,2);7,990(3,1);7,976(2,5);7,971(3,4);7,955(0,9);7,936(0,7);7,697(2,1) ;7,691(2,6);7,679(2,5);7,674(3,3);7,639(1,0);7,612(2,0);7,606(2,7);7,593(2,6);7,588(3,7);7,565(3,2);7,560(2,7);7,553(3,4);7,547(4,1);7,534(3 ,7);7,530(3,7);7,517(3,7);7,512(4,6);7,505(4,4);7,498(5,3);7,493(4,8);7,486(3,8);7,481(3,8);7,467(2,1);7,462(2,0);7,351(0,4);7,332(0,7);7,18 1(0,7);7,176(0,7);6,921(3,6);6,915(5,2);6,882(1,0);5,756(0,3);4,056(0,5);4,051(0,4);4,038(1,1);4,034(0,9);4,020(1,2);4,015(0,9);4,002(0,6);3, 998(0,6);3,322(34,4);3,318(26,1);3,281(4,1);3,056(0,4);3,000(0,4);2,981(0,5);2,941(0,4);2,908(0,4);2,899(0,5);2,865(0,4);2,859(0,4);2,671(0 ,9);2,666(0,9);2,626(0,6);2,501(72,4);2,497(70,9);2,466(15,8);2,462(16,0);2,328(0,5);2,324(0,5);1,989(4,3);1,983(2,8);1,949(0,7);1,397(0,6); 1,336(4,4);1,297(1,7);1,258(2,1);1,249(5,3);1,245(4,2);1,210(1,6);1,193(1,9);1,188(1,5);1,175(2,8);1,170(2,0);1,157(1,8);1,153(1,4);1,135(0, 7);1,117(0,4);0,993(0,4);0,008(1,7);0,000(15,7);-0,005(9,5);-0,040(2,3) |
| I-1-359: |
| HPLC-MS: logP = 1,85; Masse (m/z): 390,9 (M+H)⁺; ¹H-NMR(400,0 MHz, CD₃CN): |
| δ= 8,679(1,9);8,386(5,8);8,381(5,9);8,374(6,1);8,369(5,8);7,714(4,6);7,709(4,6);7,695(5,3);7,690(5,1);7,657(1,7);7,652(1,7);7,638(1,9);7,63 3(1,9);7,589(4,2);7,585(2,5);7,578(1,5);7,570(5,0);7,565(3,9);7,506(2,0);7,499(2,2);7,490(3,0);7,483(6,8);7,479(3,3);7,473(7,6);7,467(13,6); 7,460(7,9);7,449(3,8);7,445(2,1);7,438(2,2);7,426(3,2);7,419(2,3);7,405(5,5);7,393(5,2);7,386(4,8);7,374(4,6);7,262(0,4);7,256(0,3);7,241(0, 6);7,235(0,6);7,171(0,5);7,165(0,4);6,514(12,4);4,085(0,8);4,067(2,4);4,050(2,4);4,032(0,8);3,697(0,4);3,681(0,4);3,664(0,3);3,627(0,9);3,35 1(0,3);3,334(0,4);3,318(0,4);3,189(0,3);3,171(0,6);3,153(0,6);3,134(0,4);3,117(0,6);3,099(0,6);3,082(0,3);2,589(0,5);2,470(1,9);2,460(0,6);2, 455(0,4);2,379(0,4);2,283(49,6);2,261(0,5);2,257(0,7);2,254(0,7);2,242(0,5);2,232(0,6);2,219(1,8);2,167(56,7);2,122(1,1);2,114(0,9);2,107(1 ,0);2,101(0,8);2,095(0,6);2,082(0,3);1,972(11,0);1,964(3,2);1,958(3,9);1,952(34,8);1,946(65,0);1,940(90,5);1,934(61,8);1,928(31,5);1,915(0, 7);1,775(0,4);1,768(0,6);1,762(0,4);1,386(0,7);1,372(14,8);1,340(6,3);1,321(0,5);1,303(0,7);1,285(8,3);1,276(16,0);1,270(4,8);1,229(5,7);1,2 21(3,3);1,216(1,5);1,211(11,4);1,203(6,2);1,193(5,6);1,186(3,1);1,052(5,5);1,034(11,1);1,017(5,3);0,918(2,5);0,898(0,4);0,881(0,9);0,874(0, 4);0,864(0,5);0,856(0,4);0,000(1,1) |
| I-1-360: |
| HPLC-MS: logP = 2,80; Masse (m/z): 346,1 (M+H)⁺; ¹H-NMR(400,0 MHz, DMSO-D₆): δ= 10,448(3,7);7,951(4,7);7,683(1,9);7,679(1,8);7,664(2,3);7,660(2,3);7,596(1,8);7,592(2,1);7,587(2,0);7,582(2,5);7,573(4,2);7,568(3,1);7,5 63(3,1);7,555(3,2);7,553(3,1);7,525(2,0);7,521(2,3);7,506(4,3);7,502(4,0);7,487(3,1);7,482(4,1);7,477(2,4);7,473(2,3);7,469(2,2);7,463(2,3); 7,458(2,4);7,454(2,6);7,451 (2,3);7,445(1,0);7,440(0,9);7,436(1,0);5,754(4,8);4,038(0,4);4,020(0,4);3,340(261,8);2,671 (0,4);2,525(0,9);2,520 (1,5);2,511(22,2);2,507(45,1);2,502(58,9);2,498(41,7);2,493(19,4);2,329(0,4);2,086(0,4);2,073(1,6);2,061(16,0);1,989(1,9);1,398(0,8);1,193( 0,5);1,175(1,0);1,157(0,5);0,000(1,1) |
| I-1-361: |
| HPLC-MS: logP = 1,88; Masse (m/z): 347,1 (M+H)⁺; ¹H-NMR(400,0 MHz, CD₃CN): |
| δ= 8,905(1,5);7,610(2,2);7,606(2,5);7,590(2,7);7,587(3,2);7,567(2,4);7,563(2,4);7,549(3,0);7,544(3,0);7,508(1,2);7,503(1,9);7,497(1,9);7,49 0(3,0);7,485(3,7);7,481(4,3);7,477(4,6);7,472(4,3);7,469(3,7);7,465(4,9);7,451(5,6);7,449(5,4);7,435(2,5);7,431(3,8);7,427(5,6);7,422(3,3);7, 412(3,0);7,408(3,8);7,403(1,5);7,393(3,1);7,390(2,9);7,375(1,2);7,371(1,1);6,088(8,5);5,446(16,0);4,252(4,4);2,146(21,2);1,963(0,9);1,957(1 ,1);1,951(8,5);1,945(15,8);1,938(21,9);1,932(14,9);1,926(7,6);1,340(0,7);1,285(0,9);1,268(0,8);1,254(0,4);1,247(0,4);0,008(0,7);0,000(18,9); -0,009(0,7) |
| I-1-362: |
| HPLC-MS: logP = 2,37; Masse (m/z): 357,0 (M+H)⁺; ¹H-NMR(400,0 MHz, CD₃CN): |
| δ= 8,954(0,5);8,109(0,8);8,088(11,5);7,642(2,6);7,633(0,8);7,623(4,1);7,618(3,6);7,599(0,3);7,596(0,4);7,582(1,8);7,577(2,2);7,564(3,8);7,5 60(4,9);7,549(4,2);7,545(5,3);7,543(5,7);7,531(0,6);7,520(3,8);7,516(3,8);7,503(2,5);7,499(2,9);7,490(0,7);7,482(1,3);7,479(1,3);7,472(0,4); 7,466(0,5);7,460(2,6);7,455(3,2);7,446(6,7);7,444(7,3);7,442(9,2);7,428(1,5);7,417(1,4);7,413(1,7);7,399(4,2);7,396(5,5);7,387(4,1);7,382(3, 2);7,373(2,5);7,367(2,9);7,361(1,1);7,355(1,0);7,348(0,9);5,448(16,0);3,829(3,3);2,154(15,2);2,114(0,6);2,108(0,5);2,101 (0,5);1,964(1,9);1,9 58(2,3);1,953(15,9);1,946(29,6);1,940(40,9);1,934(28,5);1,928(14,8);0,008(0,4);0,000(10,2);-0,009(0,4) |
| I-1-363: |
| HPLC-MS: logP = 2,45; Masse (m/z): 334,0 (M+H)⁺; ¹H-NMR(400,0 MHz, CD₃CN): |
| δ= 9,902(1,9);8,635(6,3);8,629(9,8);8,611(9,8);8,605(6,3);8,457(0,8);7,920(7,4);7,914(7,4);7,617(3,5);7,612(2,9);7,599(4,3);7,593(4,6);7,58 1(3,5);7,576(3,8);7,562(3,8);7,557(5,1);7,541(0,5);7,506(0,5);7,493(0,5);7,488(1,5);7,483(2,0);7,469(4, 6);7,465(4,4);7,455(5,0);7,452(5, 6);7, 449(5,9);7,436(4,2);7,431(3,6);7,418(1,9);7,413(1,1);7,261(0,4);7,256(0,4);7,240(0,7); 7,235(0,7);7,170(0,5);7,164(0,5);7,149(0,4);7,143(0,3) ;7,017(7,7);7,011(7,6);5,446(2,4);4,996(0,4);3,685(0,4);3,667(0,4);3,524(0,5);3,507(0,4);3,030(0,8);3,015(0,8);2,136(12,0);2,135(11,9);2,10 7(0,4);1,964(1,6);1,952(21,1);1,946(39,7);1,940(55,6);1,933(38,6);1,927(20,1);1,768(0,3);1,371(16,0);1,340(3,4);1,306(0,4);1,285(4,5);1,27 6(16,0);1,241(0,4);1,222(0,6);1,216(0,7);1,204(0,3);1,124(1,4);1,106(2,6);1,088(1,3);1,062(0,5);0,881(0,4);0,000(7,0) |
| I-1-364: |
| HPLC-MS: logP = 2,38; Masse (m/z): 317,1 (M+H)⁺; ¹H-NMR(400,0 MHz, CD₃CN): |
| δ= 9,311(2,2);8,436(2,5);8,431(3,4);8,417(2,9);8,408(15,1);8,402(4,4);8,395(11,0);7,929(13,1);7,923(13,2);7,644(7,3);7,640(5,2);7,638(4,9); 7,626(9,5);7,621(9,8);7,601(4,7);7,596(5,2);7,582(5,1);7,577(7,3);7,563(0,4);7,517(2,7);7,512(3,9);7,498(13,7);7,493(13,2);7,484(12,2);7,48 0(16,0);7,478(14,7);7,475(8,6);7,465(10,4);7,460(8,5);7,447(2,8);7,442(2,1);7,011(9,8);7,004(9,8);5,477(2,5);2,227(252,6);2,224(387,2);2,2 22(387,9);2,143(0,4);2,137(0,6);2,131(0,4);1,994(4,6);1,988(4,0);1,982(39,4);1,976(74,8);1,970(106,6);1,964(72,0);1,958(36,3);1,804(0,4);1 ,798(0,6);1,792(0,4);1,297(0,7);1,144(0,4) |
| I-1-365: |
| HPLC-MS: logP = 2,99; Masse (m/z): 438,0 (M+H)⁺; ¹H-NMR(400,0 MHz, DMSO-D₆): |
| δ= 10,397(4,1);7,948(6,7);7,930(3,0);7,682(1,9);7,679(1,9);7,663(2,4);7,659(2,3);7,589(1,5);7,584(1,7);7,569(2,5);7,565(2,7);7,525(1,4);7,5 22(1,3);7,507(4,9);7,500(4,2);7,492(6,8);7,482(3,2);7,477(2,2);7,462(2,1);7,458(1,9);7,444(0,8);7,439(0,7);7,247(1,1);7,239(1,2);7,232(1,3); 7,226(1,5);7,219(1,2);7,212(1,1);7,204(0,9);5,751(2,9);4,038(0,7);4,020(0,7);3,438(0,5);3,432(0,5);3,413(1,0);3,363(502,0);3,307(0,4);3,298 (0,5);2,673(0,4);2,512(22,2);2,508(43,0);2,504(55,2);2,499(39,5);2,495(18,8);2,330(0,4);2,114(16,0);2,072(1,9);1,989(3,2);1,397(0,7);1,193( 0,9);1,175(1,7);1,157(0,8);0,000(0,4) |
| I-1-366: |
| HPLC-MS: logP = 3,12; Masse (m/z): 501,9 (M+H)⁺; ¹H-NMR(601,6 MHz, CD₃CN): |
| δ= 8,524(1,6);8,074(1,2);8,061(3,7);8,057(3,5);8,014(2,2);8,013(2,2);8,001(2,4);7,999(2,4);7,981(2,7);7,970(2,9);7,944(0,7);7,937(0,5);7,89 3(0,7);7,888(0,7);7,876(0,5);7,844(0,6);7,841(0,6);7,831(0,7);7,828(0,6);7,726(1,8);7,723(1,8);7,713(2,0);7,710(2,1);7,619(3,5);7,574(4,5);7, 541(1,6);7,538(1,6);7,534(1,7);7,532(1,7);7,522(3,1);7,520(3,3);7,509(4,2);7,507(4,1);7,491(8,6);7,489(8,8);7,478(9,0);7,477(8,5);7,466(3,6) ;7,464(3,3);7,447(1,0);7,444(0,9);7,439(0,9);7,430(0,7);7,427(0,8);7,411(0,5);7,386(0,4);7,359(0,3);7,306(0,5);7,304(0,5);7,294(0,6);7,291(0 ,6);7,281(0,5);7,278(0,5);7,240(3,3);7,237(3,5);7,227(4,6);7,225(4,3);7,215(3,1);7,212(2,8);6,972(0,5);6,968(0,5);5,590(0,8);5,579(0,8);5,44 6(1,6);4,365(2,4);4,353(7,3);4,341(7,6);4,329(2,5);2,625(0,4);2,602(0,4);2,152(53,0);2,148(64,3);2,144(67,0);2,142(64,7);2,138(72,9);2,058( 0,6);2,054(1,2);2,050(1,8);2,046(1,2);2,042(0,6);1,964(9,2);1,956(8,1);1,951(9,4);1,948(118,8);1,943(217,3);1,939(304,0);1,935(198,5);1,93 1(96,9);1,926(3,0);1,922(1,4);1,833(0,6);1,829(1,2);1,825(1,8);1,820(1,2);1,816(0,6);1,736(4,2);1,725(4,0);1,639(0,4);1,627(0,4);1,373(7,8); 1,361(16,0);1,350(7,8);1,301(0,6);1,294(0,3);1,291(0,6);1,283(0,5);1,270(1,0);1,264(0,5);1,259(0,3);1,253(0,4);1,008(0,4);0,096(0,6);0,005( 4,3);0,000(160,3);-0,006(4,7);-0,100(0,6) |
| I-1-367: |
| HPLC-MS: logP = 3,18; Masse (m/z): 549,8 (M+H)⁺; ¹H-NMR(400,0 MHz, CD₃CN): |
| δ= 8,568(3,7);8,122(0,4);8,099(0,4);8,046(7,5);8,019(2,8);8,017(2,8);7,997(4,2);7,986(5,4);7,967(5,6);7,918(0,6);7,908(1,0);7,890(0,6);7,87 9(0,5);7,869(0,6);7,858(0,5);7,838(0,4);7,805(0,3);7,801(0,4);7,780(0,6);7,774(0,6);7,732(1,7);7,728(1,8);7,721(0,4);7,712(2,2);7,708(2,1);7, 696(0,5);7,685(0,5);7,622(7,6);7,617(7,9);7,610(8,5);7,584(9,2);7,538(5,7);7,529(5,7);7,515(7,1);7,508(6,9);7,491(14,9);7,480(16,0);7,431(1 ,3);7,423(1,3);7,411(1,0);7,404(1,0);7,389(0,8);7,385(0,8);7,380(0,7);7,371(0,7);7,366(0,7);7,346(0,6);7,342(0,6);7,325(0,5);7,320(0,5);7,31 0(0,5);7,249(4,9);7,244(4,8);7,230(7,3);7,226(6,3);7,217(3,7);7,211(4,1);7,206(3,3);7,149(0,8);7,146(0,7);7,135(0,7);7,131(0,8);7,110(0,4);6, 552(0,4);6,548(0,4);6,537(0,4);6,533(0,4);5,449(1,6);4,826(0,4);4,373(1,9);4,356(5,9);4,338(6,0);4,320(2,0);2,673(1,0);2,669(1,0);2,630(1,5) ;2,601(0,4);2,567(0,4);2,473(0,6);2,468(1,2);2,464(1,6);2,459(1,1);2,454(0,6);2,178(350,1);2,172(396,0);2,121(1,6);2,115(2,2);2,108(2,6);2, 102(1,9);2,096(1,2);2,016(0,4);1,965(14,1);1,959(15,8);1,953(134,8);1,947(253,2);1,941(354,6);1,935(244,9);1,929(126,5);1,854(0,3);1,848( 0,3);1,782(0,9);1,776(1,6);1,770(2,2);1,763(1,6);1,757(0,9);1,739(0,4);1,676(0,7);1,658(0,7);1,640(0,4);1,622(0,4);1,379(6,6);1,361(13,0);1, 343(6,6);1,331(0,3);1,325(0,4);1,300(0,6);1,282(1,1);1,268(3,8);1,258(1,0);1,247(1,2);1,244(1,0);1,229(1,6);1,226(1,4);1,219(0,5);1,211(1,0) ;1,209(0,8);1,200(0,4);1,195(0,4);1,101(0,3);1,085(0,3);1,025(0,7);1,014(1,5);1,007(1,3);0,997(1,5);0,989(0,8);0,898(0,4);0,891(0,4);0,881(0 ,7);0,858(0,5);0,840(0,5);0,774(0,4);0,770(0,4);0,757(0,4);0,751(0,5);0,740(0,4);0,731(0,3);0,723(0,4);0,146(0,6);0,008(4,4);0,000(149,5);-0,009(4,9);-0,149(0,6) |
| I-1-368: |
| HPLC-MS: logP = 2,51; Masse (m/z): 448,9 (M+H)⁺; ¹H-NMR(400,0 MHz, CD₃CN): |
| δ= 8,866(2,2);8,112(0,5);8,090(16,0);7,904(4,7);7,902(4,9);7,884(5,0);7,882(5,1);7,640(3,0);7,636(2,8);7,635(2,5);7,620(4,9);7,619(5,3);7,6 17(4,6);7,616(4,4);7,609(0,6);7,590(0,3);7,583(2,5);7,576(3,9);7,571(3,1);7,563(3,3);7,560(6,7);7,558(6,0);7,556(8,9);7,551(3,2);7,537(4,3); 7,532(2,4);7,523(5,1);7,518(5,1);7,513(0,8);7,504(3,1);7,503(2,9);7,500(4,7);7,485(1,7);7,481(1,6);7,455(2,5);7,452(2,6);7,446(0,4);7,436(5, 6);7,433(5,8);7,427(0,6);7,417(3,8);7,415(3,7);7,298(4,6);7,294(5,4);7,279(3,8);7,275(4,0);7,209(3,3);7,204(3,1);7,195(0,4);7,189(4,4);7,185 (4,3);7,170(2,8);7,166(2,7);5,447(2,2);4,066(0,6);4,048(0,6);3,843(2,0);2,146(19,9);1,971(3,0);1,965(1,0);1,958(1,2);1,953(11,1);1,946(21,1) ;1,940(29,8);1,934(20,8);1,928(10,8);1,436(1,4);1,372(3,6);1,340(0,3);1,284(0,5);1,276(4,1););1,221(0,8);1,203(1,6);1,185(0,8);0,936(0,3);0,0 08(1,5);0,000(37,8);-0,009(1,6) |
| I-1-369: |
| HPLC-MS: logP = 2,27; Masse (m/z): 424,9 (M+H)⁺; ¹H-NMR(400,0 MHz, CD₃CN): |
| δ= 9,215(3,3);8,425(6,2);8,420(6,4);8,413(6,5);8,408(6,2);7,901(11,5);7,895(11,4);7,768(6,4);7,763(6,3);7,749(7,3);7,744(6,9);7,615(5,3);7, 611(4,5);7,597(6,2);7,592(6,8);7,578(0,5);7,560(4,8);7,555(5,2);7,536(7,8);7,524(0,6);7,481(2,9);7,468(11,5);7,462(7,9);7,456(11,7);7,449(1 6,0);7,443(5,9);7,437(10,1);7,431(5,2);7,417(1,8);7,412(1,3);6,970(11,7);6,964(11,4);5,447(3,8);2,461(0,4);2,136(90,8);2,132(76,9);2,113(1, 7);2,107(2,2);2,101(1,6);2,095(0,9);1,963(9,1);1,952(125,9);1,945(233,6);1,939(326,4);1,933(224,8);1,9271(15,7);1,780(0,7);1,774(1,4);1,76 |
| 8(1,9);1,762(1,3);1,756(0,7);1,270(0,7);0,146(0,4);0,008(3,3);0,000(77,8);-0,150(0,4) |
| I-1-370: |
| HPLC-MS: logP = 2,71; Masse (m/z): 302,0 (M+H)⁺; ¹H-NMR(400,0 MHz, CD₃CN): |
| δ= 8,824(0,7);7,859(2,5);7,853(2,5);7,603(1,3);7,599(1,1);7,597(1,1);7,584(1,8);7,579(1,7);7,560(1,2);7,555(1,5);7,542(1,3);7,541(1,4);7,53 6(1,8);7,474(0,7);7,470(0,8);7,455(1,9);7,451(1,7);7,437(1,7);7,434(1,9);7,432(1,5);7,429(1,8);7,415(1,5);7,410(1,5);7,396(0,8);7,390(2,8);7, 384(2,7);6,941(2,6);6,935(2,5);6,821(2,5);6,816(2,5);5,446(0,5);2,587(16,0);2,169(25,7);1,964(4,0);1,958(0,8);1,952(4,1);1,946(7,5);1,939(1 0,4);1,933(7,1);1,927(3,7);1,436(0,4) |
| I-1-371: |
| HPLC-MS: logP = 2,88; Masse (m/z): 386,9 (M+H)⁺; ¹H-NMR(400,0 MHz, CD₃CN): |
| δ= 9,478(0,4);7,880(2,3);7,874(2,3);7,609(1,2);7,604(0,8);7,600(0,8);7,591(1,2);7,585(1,6);7,541(0,7);7,535(0,8);7,518(1,3);7,480(0,4);7,47 5(0,7);7,461(1,7);7,456(1,9);7,453(2,0);7,445(2,8);7,435(1,7);7,429(1,2);7,416(0,5);7,411(0,3);6,894(1,1);2,734(16,0);2,699(0,6);2,145(9,8); 1,971(0,5);1,964(0,5);1,952(7,9);1,946(14,9);1,940(21,0);1,933(14,6);1,927(7,7);1,372(5,7);1,340(1,2);1,285(1,6);1,276(5,8);0,000(6,5) |
| I-1-372: |
| HPLC-MS: logP = 2,75; Masse (m/z): 336,0 (M+H)⁺; ¹H-NMR(400,0 MHz, DMSO-D₆): |
| δ= 10,370(2,2);8,039(2,9);8,033(2,9);7,681(1,4);7,677(1,1);7,675(1,1);7,662(1,8);7,657(1,7);7,593(1,1);7,588(1,4);7,574(1,4);7,569(1,9);7,5 21(0,6);7,516(0,8);7,502(1,7);7,498(1,6);7,485(2,3);7,479(2,2);7,466(1,5);7,461(1,4);7,448(0,5);7,443(0,4);6,773(2,7);6,766(2,7);5,756(1,6); 4,287(2,1);4,276(2,4);4,265(2,2);3,323(16,3);3,044(2,3);3,032(2,4);3,021(2,1);2,524(0,5);2,511(11,3);2,506(22,3);2,502(29,0);2,497(20,6);2, 493(9,8);2,017(16,0);1,235(1,2);0,000(2,9) |
| I-1-373: |
| HPLC-MS: logP = 3,85; Masse (m/z): 354,1 (M+H)⁺; ¹H-NMR(601,6 MHz, CD₃CN): |
| δ= 7,892(0,8);7,888(0,8);7,602(0,4);7,600(0,4);7,589(0,5);7,587(0,5);7,566(0,4);7,565(0,4);7,553(0,5);7,552(0,5);7,535(0,4);7,532(0,4);7,52 3(0,5);7,520(0,5);7,453(0,5);7,450(0,5);7,440(0,4);7,433(0,4);7,429(0,4);7,420(0,4);7,417(0,4);7,401(0,3);7,399(0,4);7,338(0,4);7,336(0,4);7, 326(0,5);7,323(0,5);7,274(0,4);7,272(0,4);7,262(0,5);7,260(0,5);6,972(0,9);6,967(0,8);2,162(10,0);1,966(0,5);1,950(2,2);1,945(3,9);1,941(5, 7);1,937(3,9);1,933(2,0);1,436(0,7);1,429(16,0);1,344(0,5);0,000(2,6) |
| I-1-374: |
| HPLC-MS: logP = 3,15; Masse (m/z): 354,0 (M+H)⁺; ¹H-NMR(400,0 MHz, DMSO-D₆): |
| δ= 11,326(11,6);8,315(0,5);8,108(16,0);8,102(15,0);7,916(11,7);7,903(12,2);7,703(7,0);7,698(5,6);7,685(8,2);7,679(7,9);7,664(0,5);7,628(6, 1);7,622(6,4);7,611(6,9);7,608(6,9);7,604(8,8);7,591(0,6);7,571(5,0);7,542(2,6);7,537(3,3);7,523(8,6);7,519(8,1);7,510(9,3);7,505(11,3);7,50 0(6,5);7,492(7,6);7,487(6,1);7,473(2,5);7,468(1,8);7,432(10,7);7,410(13,2);7,397(12,4);7,294(5,2);6,871(14,9);6,865(13,9);5,756(1,8);3,322( 125,3);2,675(1,2);2,671(1,4);2,541(1,2);2,506(188,1);2,502(221,9);2,497(156,1);2,333(1,2);2,328(1,4);2,179(0,5);1,235(8,6);0,871(0,4);0,85 4(0,8);0,836(0,3);0,000(62,8);-0,008(2,9) |
| I-1-375: |
| HPLC-MS: logP = 3,15; Masse (m/z): 372,0 (M+H)⁺; ¹H-NMR(400,0 MHz, DMSO-D₆): |
| δ= 11,490(7,5);8,315(0,7);8,104(15,4);8,098(15,0);8,083(0,4);7,921(8,4);7,908(8,8);7,711(0,5);7,700(7,0);7,695(5,0);7,692(4,5);7,682(7,3);7 ,676(8,5);7,663(0,6);7,619(5,4);7,613(5,3);7,603(4,8);7,600(5,6);7,595(8,1);7,584(0,6);7,537(2,2);7,532(3,3);7,519(9,1);7,514(8,6);7,509(9,1 );7,502(14,8);7,495(6,5);7,490(7,8);7,485(6,5);7,471(2,3);7,466(1,7);7,447(16,0);7,434(15,2);6,889(0,5);6,883(0,6);6,861(7,0);6,856(6,7);5,7 56(4,6);3,322(168,7);2,675(1,3);2,671(1,7);2,666(1,3);2,541(1,2);2,524(5,3);2,510(104,8);2,506(203,1);2,502(261,3);2,497(184,6);2,493(86, 1);2,337(0,6);2,333(1,2);2,328(1,7);2,324(1,2);2,178(0,6);2,160(0,4);1,258(0,6);1,235(9,2);0,854(1,0);0,836(0,4);0,146(0,4);0,008(4,8);0,000 (94,9);-0,009(3,2);-0,150(0,4) |
| I-1-376: |
| HPLC-MS: logP = 2,57; Masse (m/z): 381,1 (M+H)⁺; ¹H-NMR(400,0 MHz, CD₃CN): |
| δ= 9,520(2,1);8,674(4,5);8,662(4,5);8,243(4,3);8,222(4,6);7,686(3,1);7,674(3,1);7,666(3,0);7,654(2,8);7,640(0,5);7,632(3,9);7,629(3,2);7,61 4(5,8);7,611(4,7);7,544(2,0);7,536(2,3);7,529(3,0);7,521(5,3);7,511(3,7);7,502(12,0);7,499(16,0);7,495(10,3);7,487(2,3);7,483(3,3);7,479(2, 3);7,468(0,4);7,464(0,7);7,460(0,8);7,441(0,4);7,436(0,4);6,558(13,5);6,395(0,8);5,447(1,4);2,449(0,3);2,291(53,9);2,254(3,1);2,147(46,9);2, 120(0,3);2,113(0,4);2,107(0,4);1,1,964(2,2);1,958(3,0);1,952(23,2);1,946(43,3);1,940(60,2);1,934(41,3);1,927(21,2);1,768(0,3);1,269(0,6);0,0 00(0,9) |
| I-1-377: |
| HPLC-MS: logP = 3,29; Masse (m/z): 338,0 (M+H)⁺; ¹H-NMR(400,0 MHz, CD₃CN): |
| δ= 9,252(2,3);7,896(9,6);7,890(9,6);7,728(10,8);7,714(11,3);7,615(4,6);7,610(3,7);7,609(3,4);7,596(6,1);7,591(6,0);7,575(4,5);7,569(5,1);7, 557(4,5);7,555(4,7);7,551(6,7);7,535(0,4);7,488(2,1);7,483(2,7);7,469(6,4);7,465(6,1);7,453(7,2);7,451(6,9);7,447(7,2);7,434(5,5);7,429(4,8) ;7,416(1,9);7,411(1,4);7,134(11,5);7,121(10,9);6,930(9,8);6,924(9,6);6,298(0,4);5,447(1,2);2,244(0,4);2,233(0,5);2,227(0,6);2,169(446,8);2, 120(0,9);2,114(0,9);2,107(0,9);2,101(0,7);2,095(0,6);2,087(0,4);2,084(0,4);2,076(0,3);1,972(1,2);1,964(3,8);1,958(5,0);1,952(26,5);1,946(48 ,4);1,940(66,4);1,934(46,0);1,928(23,9);1,775(0,4);1,768(0,5);1,762(0,4);1,690(0,4);1,437(16,0);1,269(0,5);0,911(0,3);0,000(1,5) |
| I-1-378: |
| HPLC-MS: logP = 2,96; Masse (m/z): 413,8 (M+H)⁺; ¹H-NMR(400,0 MHz, DMSO-D₆): |
| δ= 10,867(7,2);8,095(9,5);8,089(9,5);7,924(12,4);7,920(12,6);7,696(4,5);7,692(3,7);7,690(3,6);7,677(6,1);7,672(5,6);7,624(3,9);7,619(5,0);7 ,606(4,2);7,604(4,6);7,600(6,2);7,538(2,0);7,534(2,5);7,519(5,8);7,515(5,3);7,501(9,5);7,495(8,3);7,482(4,9);7,477(5,0);7,463(1,9);7,458(1,5 );6,910(13,2);6,906(13,1);6,865(9,9);6,859(10,0);5,757(16,0);4,039(0,4);4,021(0,4);3,330(13,5);2,526(0,6);2,521(1,0);2,513(11,5);2,508(23, 5);2,504(31,1);2,499(22,3);2,495(10,5);1,990(1,9);1,396(0,7);1,193(0,5);1,175(1,1);1,158(0,5);0,000(6,7) |
| I-1-379: |
| HPLC-MS: logP = 3,34; Masse (m/z): 429,9 (M+H)⁺; ¹H-NMR(600,1 MHz, CD₃CN): |
| δ= 9,213(2,3);8,519(0,4);8,517(0,4);8,512(0,5);8,509(0,5);8,035(0,5);8,033(0,5);8,021(0,6);8,019(0,6);7,894(11,3);7,890(11,2);7,626(15,0);7 ,621(0,7);7,617(15,9);7,613(6,2);7,611(5,2);7,610(5,3);7,600(7,6);7,597(7,1);7,572(5,6);7,569(6,5);7,559(6,3);7,556(7,5);7,546(0,6);7,538(0, 6);7,532(0,6);7,525(0,5);7,483(2,8);7,480(3,4);7,470(7,6);7,468(6,9);7,458(5,8);7,455(5,0);7,451(6,4);7,448(6,4);7,438(6,5);7,435(6,6);7,428 (1,4);7,426(2,9);7,423(2,4);7,272(16,0);7,264(15,4);7,103(1,1);7,095(1,0);6,929(10,4);6,925(10,3);5,446(7,3);2,127(117,8);2,058(0,5);2,054( 1,0);2,050(1,4);2,046(1,0);2,042(0,5);1,963(5,5);1,955(5,7);1,951(7,7);1,947(91,7);1,943(175,3);1,939(258,1);1,935(176,2);1,931(88,5);1,92 2(1,4);1,914(0,4);1,832(0,5);1,828(1,0);1,824(1,5);1,820(1,0);1,816(0,5);1,377(1,1);1,372(8,2);1,340(2,2);1,299(0,4);1,289(0,6);1,285(2,8);1, 277(8,7);1,264(0,3);1,217(1,2);0,097(0,8);0,005(5,9);0,000(202,9);-0,006(6,3);-0,100(0,8) |
| I-1-380: |
| HPLC-MS: logP = 2,09; Masse (m/z): 381,0 (M+H)⁺; ¹H-NMR(400,0 MHz, CD₃CN): |
| δ= 8,846(4,1);8,833(4,2);8,775(1,4);8,678(7,7);7,661(4,8);7,648(4,6);7,598(2,0);7,595(3,2);7,592(1,9);7,581(2,4);7,577(4,6);7,574(3,2);7,56 5(0,4);7,505(1,6);7,496(1,7);7,490(2,1);7,481(4,2);7,471(2,6);7,463(12,0);7,461(11,8);7,457(6,9);7,450(2,3);7,446(2,6);7,442(1,2);7,427(0,8) ;7,423(0,6);7,420(0,4);7,253(0,6);7,240(0,4);7,235(0,8);7,197(0,7);7,178(0,4);7,171(0,4);7,165(0,4);7,150(0,4);6,523(9,0);5,447(3,6);4,085(1 ,3);4,067(4,0);4,049(4,1);4,032(1,4);2,966(0,4);2,887(0,4);2,328(2,7);2,285(35,8);2,274(1,5);2,218(0,6);2,154(30,6);2,131(0,3);2,123(0,4);2, 107(0,4);1,971(18,0);1,964(1,6);1,957(1,9);1,952(16,5);1,946(30,9);1,939(43,1);1,933(29,7);1,927(15,3);1,913(0,8);1,553(1,1);1,437(16,0);1 ,372(6,0);1,341(1,4);1,296(0,4);1,285(1,9);1,276(6,9);1,250(0,6);1,231(0,3);1,221(4,9);1,203(9,6);1,185(4,7);0,881(0,3);0,000(1,9) |
| I-1-381: |
| HPLC-MS: logP = 2,19; Masse (m/z): 333,0 (M+H)⁺; ¹H-NMR(400,0 MHz, CD₃CN): |
| δ= 18,011(0,6);17,562(0,7);15,449(0,6);9,391(2,7);8,783(16,0);8,631(8,9);8,618(9,1);7,928(10,1);7,922(10,4);7,645(5,0);7,639(3,9);7,627(5, 8);7,621(6,3);7,587(4,3);7,581(4,8);7,570(14,0);7,563(7,9);7,556(9,9);7,510(2,4);7,496(6,3);7,491(6,1);7,485(6,8);7,479(10,4);7,472(4,6);7,4 67(5,8);7,462(4,3);7,448(1,4);7,011(10,3);7,005(9,8);5,477(2,0);4,097(0,6);4,079(0,7);3,306(0,7);2,332(0,7);2,318(0,9);2,290(1,3);2,229(869 ,1);2,223(1835,6);2,162(7,5);2,150(1,1);2,144(2,0);2,137(2,7);2,131(1,8);2,125(1,0);2,104(0,7);2,087(0,7);2,074(0,6);2,067(0,6);2,042(0,7);2 ,035(0,8);1,994(1128,9);1,982(176,2);1,976(314,2);1,970(437,3);1,964(294,7);1,957(147,0);1,920(0,7);1,863(0,6);1,822(6,6);1,815(0,7);1,80 5(1,8);1,798(2,4);1,792(1,9);1,786(1,1);1,710(0,6);1,317(0,6);1,299(2,2);1,251(0,8);1,233(1,5);1,214(0,6);0,941(1,0);0,029(1,8) |
| I-1-382: |
| HPLC-MS: logP = 2,15; Masse (m/z): 334,1 (M+H)⁺; ¹H-NMR(601,6 MHz, CD₃CN): |
| δ= 8,090(1,0);8,089(1,8);8,088(1,1);8,076(1,1);8,075(2,0);8,074(1,2);7,652(3,7);7,650(3,9);7,648(2,1);7,646(2,0);7,643(2,0);7,641(1,9);7,62 9(0,3);7,627(0,3);7,525(1,0);7,521(1,0);7,516(0,9);7,511(1,3);7,507(0,9);7,502(0,8);7,498(0,8);5,451(0,9);3,746(9,2);3,743(9,2);2,410(16,0); 2,177(4,9);1,967(10,4);1,959(0,3);1,955(0,4);1,951(4,6);1,947(8,5);1,943(12,4);1,939(8,6);1,935(4,4);0,000(1,1) |
| I-1-383: |
| HPLC-MS: logP = 2,86; Masse (m/z): 387,9 (M+H)⁺; ¹H-NMR(400,0 MHz, CD₃CN): |
| δ= 8,824(1,4);7,870(6,0);7,864(5,9);7,832(0,7);7,827(0,7);7,623(0,3);7,608(3,1);7,604(2,3);7,602(2,2);7,590(4,1);7,585(3,9);7,578(0,5);7,57 3(0,5);7,569(0,4);7,558(2,7);7,552(3,4);7,541(2,6);7,538(3,0);7,534(4,4);7,480(1,2);7,476(1,8);7,462(4,4);7,457(4,0);7,448(4,2);7,444(4,4);7, 442(4,5);7,438(3,0);7,429(3,7);7,424(3,2);7,416(0,4);7,410(1,1);7,406(0,8);7,402(0,4);6,892(4,4);6,886(4,4);5,446(1,3);3,813(15,9);3,811(16 ,0);3,408(0,3);3,321(1,5);2,150(235,5);2,132(1,3);2,119(0,5);2,113(0,4);2,107(0,5);2,101(0,4);2,074(0,5);1,976(1,1);1,972(1,5);1,964(158,3); 1,958(4,2);1,952(31,5);1,946(60,2);1,940(87,3);1,933(59,2);1,927(29,8);1,792(0,8);1,774(0,4);1,768(0,5);1,762(0,4);1,285(0,4);1,270(1,0);1, 135(0,4);1,117(0,8);1,099(0,4);0,000(2,6) |
| I-1-384: |
| HPLC-MS: logP = 3,05; Masse (m/z): 384,0 (M+H)⁺; ¹H-NMR(400,0 MHz, DMSO-D₆): |
| δ= 11,543(7,4);8,110(9,5);8,104(9,4);7,770(0,6);7,750(1,9);7,741(1,1);7,730(4,2);7,716(6,9);7,696(16,0);7,689(4,1);7,678(7,5);7,672(7,6);7, 660(0,5);7,624(4,0);7,618(4,0);7,607(3,3);7,605(3,8);7,600(5,9);7,588(0,4);7,531(1,3);7,526(2,0);7,512(5,4);7,507(5,2);7,502(5,5);7,495(9,1) ;7,488(4,0);7,483(4,9);7,478(4,1);7,464(1,5);7,459(1,0);6,892(10,3);6,885(10,2);5,756(9,7);3,323(19,7);2,675(0,5);2,671(0,6);2,666(0,4);2,5 24(1,8);2,511(37,1);2,506(74,9);2,502(98,1);2,497(69,8);2,493(33,0);2,333(0,5);2,329(0,6);2,324(0,4);1,989(1,3);1,193(0,4);1,175(0,7);1,15 7(0,4);0,008(2,3);0,000(63,6);-0,009(2,2) |
| I-1-385: |
| HPLC-MS: logP = 2,94; Masse (m/z): 368,0 (M+H)⁺; ¹H-NMR(400,0 MHz, DMSO-D₆): |
| δ= 11,590(1,4);8,840(1,7);8,837(1,5);8,696(1,8);8,693(1,4);8,579(1,5);8,572(1,3);7,855(0,8);7,836(1,0);7,803(0,3);7,785(0,8);7,766(0,7);7,7 34(0,7);7,716(1,8);7,697(0,8);7,049(1,5);7,042(1,4);3,336(13,5);2,510(4,7);2,505(5,6);2,501(3,8);0,000(0,6) |
| I-1-386: |
| HPLC-MS: logP = 2,45; Masse (m/z): 369,0 (M+H)⁺; ¹H-NMR(400,0 MHz, DMSO-D₆): |
| δ= 11,951(0,4);11,745(12,4);8,863(6,7);8,851(7,0);8,843(15,3);8,839(16,0);8,705(15,3);8,702(14,8);8,681(0,5);8,678(0,6);8,597(13,6);8,590( 13,5); 8,531(0,5);8,524(0,5);8,316(0,9);8,309(0,4);8,306(0,4);8,229(6,2);8,212(6,6);7,855(5,7);7,843(5,6);7,836(5,3);7,824(5,1);7,049(14,2);7 ,042(14,1);6,924(0,5);6,916(0,4);5,756(2,5);4,055(0,4);4,038(1,2);4,020(1,2);4,002(0,4);3,320(66,4);2,675(2,1);2,671(2,9);2,666(2,1);2,541( 1,5);2,524(7,0);2,510(163,2);2,506(331,2);2,502(437,3);2,497(315,2);2,493(151,7);2,333(2,0);2,328(2,8);2,324(2,1);2,197(0,4);2,179(0,8);2, 160(0,4);1,989(5,1);1,398(2,1);1,258(0,6);1,235(11,4);1,193(1,4);1,175(2,7);1,157(1,4);0,870(0,4);0,854(1,1);0,836(0,5);0,146(1,4);0,008(9, 9);0,000(306,1);-0,008(11,2);-0,150(1,4) |
| I-1-387: |
| HPLC-MS: logP = 2,67; Masse (m/z): 336,0 (M+H)⁺; ¹H-NMR(400,0 MHz, DMSO-D₆): |
| δ= 11,798(10,1);8,944(0,3);8,849(15,7);8,846(16,0);8,704(15,6);8,700(14,8);8,656(0,4);8,596(12,3);8,589(12,0);8,315(2,1);7,629(1,4);7,612( 3,0);7,608(2,6);7,591(5,4);7,574(2,8);7,570(3,2);7,554(1,4);7,255(9,6);7,235(13,5);7,214(7,8);7,207(1,5);7,049(14,1);7,042(13,7);5,755(0,7); 3,373(0,6);3,321(583,0);3,285(0,5);3,278(0,4);3,095(1,9);2,675(3,5);2,671(4,7);2,666(3,4);2,635(0,4);2,541(2,9);2,524(11,6);2,519(18,7);2,5 |
| 11(269,9);2,506(550,6);2,502(724,4);2,497(511,8);2,493(238,6);2,413(0,4);2,337(1,7);2,333(3,3);2,328(4,5);2,324(3,2);1,230(0,3);1,190(2,9 );1,171(4,9);1,154(2,9);0,008(0,5);0,000(12,3) |
| I-1-388: |
| HPLC-MS: logP = 2,84; Masse (m/z): 378,0 (M+H)⁺; ¹H-NMR(400,0 MHz, DMSO-D₆): |
| δ= 11,482(5,8);8,948(1,7);8,944(1,8);8,839(7,2);8,836(7,4);8,695(7,6);8,692(7,5);8,577(7,5);8,570(6,3);8,315(0,4);7,718(3,8);7,715(3,9);7,6 98(4,6);7,695(4,5);7,565(2,4);7,561(2,8);7,546(4,2);7,542(4,4);7,503(2,3);7,500(2,5);7,484(4,9);7,481(4,8);7,471(1,7);7,466(3,7);7,462(2,7); 7,441(3,3);7,436(3,3);7,421(3,7);7,417(3,7);7,402(1,8);7,398(1,7);7,064(6,6);7,057(6,6);6,647(1,2);5,755(16,0);5,449(1,9);5,445(1,9);3,346( 0,7);3,322(72,3);2,676(0,6);2,671(0,8);2,667(0,6);2,541(0,4);2,525(2,0);2,511(45,6);2,507(92,8);2,502(122,0);2,498(86,3);2,493(40,3);2,333 (0,6);2,329(0,8);2,324(0,6);1,506(1,2);0,000(0,8) |
| I-1-389: |
| HPLC-MS: logP = 2,82; Masse (m/z): 334,1 (M+H)⁺; ¹H-NMR(400,0 MHz, DMSO-D₆): |
| δ= 11,498(12,8);8,841(14,8);8,838(15,0);8,695(16,0);8,692(15,3);8,579(13,1);8,572(13,1);8,316(0,3);7,598(6,4);7,594(6,8);7,580(8,4);7,576( 8,9);7,568(5,4);7,565(6,0);7,548(11,9);7,545(12,2);7,529(5,7);7,525(5,8);7,511(9,3);7,507(8,0);7,491(4,9);7,487(4,3);7,463(7,4);7,459(7,1);7 ,444(9,5);7,441(9,1);7,426(3,6);7,423(3,3);7,068(13,4);7,061(13,2);5,756(1,1);3,323(80,8);2,676(0,7);2,672(0,9);2,668(0,7);2,542(0,6);2,525 (2,7);2,511(54,6);2,507(107,6);2,503(139,7);2,498(99,7);2,494(47,6);2,334(0,6);2,329(0,9);2,325(0,6);0,000(0,7) |
| I-1-390: |
| HPLC-MS: logP = 2,96; Masse (m/z): 425,9 (M+H)⁺; ¹H-NMR(400,0 MHz, DMSO-D₆): |
| δ= 11,424(9,2);9,024(0,4);8,840(10,6);8,837(10,2);8,747(0,5);8,744(0,4);8,694(11,0);8,691(10,3);8,576(8,6);8,570(8,5);7,935(7,3);7,916(8,0 );7,882(0,4);7,514(0,9);7,493(8,4);7,490(8,6);7,480(16,0);7,259(0,4);7,249(3,2);7,241(2,9);7,236(3,0);7,229(3,9);7,227(3,6);7,220(2,7);7,216 (3,0);7,207(2,6);7,064(8,8);7,058(8,7);4,056(0,4);4,038(1,1);4,020(1,1);4,002(0,4);3,322(45,6);2,671(0,6);2,667(0,4);2,541(0,4);2,506(75,6); 2,502(96,2);2,498(68,9);2,333(0,5);2,329(0,6);2,325(0,4);1,989(4,7);1,193(1,3);1,175(2,5);1,157(1,2);0,000(0,4) |
| I-1-391: |
| HPLC-MS: logP = 2,31; Masse (m/z): 367,0 (M+H)⁺; ¹H-NMR(400,0 MHz, CD₃CN): |
| δ= 9,277(4,2);8,436(6,5);8,432(7,2);8,425(7,1);8,420(7,0);7,997(11,4);7,991(11,8);7,953(7,0);7,948(7,4);7,933(7,8);7,929(7,9);7,824(5,8);7, 805(7,5);7,764(0,3);7,750(1,9);7,738(2,3);7,730(6,3);7,715(7,0);7,692(16,0);7,674(8,6);7,656(2,2);7,640(0,4);7,635(0,3);7,615(0,3);7,510(7, 3);7,498(7,4);7,490(7,1);7,479(6,7);7,426(0,5);7,420(0,5);7,241(0,3);7,236(0,4);7,003(12,0);6,996(12,3);5,448(7,9);2,150(57,8);2,114(0,4);2, 108(0,4);1,972(1,1);1,965(2,3);1,953(24,1);1,947(45,3);1,941(63,2);1,935(45,6);1,928(24,2);1,769(0,4);1,372(8,3);1,340(1,1);1,313(0,6);1,2 97(0,7);1,284(1,7);1,276(9,3);1,203(0,4);0,146(0,4);0,000(97,0);-0,150(0,4) |
| I-1-392: |
| HPLC-MS: logP = 2,13; Masse (m/z): 333,0 (M+H)⁺; ¹H-NMR(400,0 MHz, CD₃CN): |
| δ= 9,317(4,2);8,438(7,6);8,434(8,3);8,427(8,2);8,422(8,2);7,999(13,1);7,993(13,3);7,962(7,5);7,958(7,7);7,942(8,3);7,938(8,3);7,860(0,4);7, 854(0,4);7,631(0,8);7,613(7,0);7,610(7,3);7,595(8,4);7,591(8,8);7,527(4,6);7,514(10,9);7,511(12,7);7,507(14,1);7,503(13,4);7,496(12,7);7,4 83(16,0);7,479(9,3);7,471(1,9);7,463(4,5);7,459(3,8);7,444(7,0);7,440(6,5);7,426(9,5);7,422(8,9);7,408(3,8);7,404(3,5);7,389(0,4);7,384(0,4) ;7,368(0,4);7,364(0,4);7,350(0,4);7,345(0,4);7,261(0,3);7,256(0,4);7,240(0,5);7,235(0,6);7,172(0,5);7,166(0,5);7,025(13,4);7,019(13,4);6,98 5(0,5);6,633(0,4);6,626(0,4);5,449(3,7);4,085(0,4);4,067(1,2);4,049(1,2);4,031(0,4);3,993(0,4);3,259(0,4);2,473(0,5);2,468(0,9);2,463(1,3);2, 459(0,9);2,454(0,5);2,433(0,4);2,176(295,1);2,121(0,7);2,115(0,9);2,109(1,1);2,103(0,8);2,096(0,5);1,972(6,2);1,966(5,9);1,954(61,0);1,947( 113,7);1,941(158,7);1,935(112,0);1,929(59,9);1,782(0,4);1,776(0,7);1,770(1,0);1,764(0,7);1,757(0,4);1,436(2,7);1,385(0,7);1,371(14,6);1,34 0(1,9);1,310(2,8);1,293(3,1);1,285(3,1);1,276(14,4);1,221(1,5);1,216(1,0);1,204(2,8);1,186(1,4);0,881(0,6);0,858(0,5);0,146(1,3);0,008(12,5) ;0,000(261,9);-0,008(14,8);-0,150(1,3) |
| I-1-393 siehe Synthesebeispiel 29 |
| I-1-394: |
| HPLC-MS: logP = 2,53; Masse (m/z): 415,0 (M+H)⁺; ¹H-NMR(400,0 MHz, CD₃CN): |
| δ= 9,341(2,2);8,126(8,5);8,119(8,8);7,948(7,0);7,928(6,9);7,867(4,9);7,864(5,3);7,847(5,5);7,844(5,9);7,794(2,2);7,790(2,2);7,773(5,5);7,75 5(4,4);7,751(4,2);7,707(7,5);7,688(4,4);7,518(4,2);7,515(4,3);7,506(2,3);7,494(16,0);7,488(9,8);7,477(9,9);7,458(1,7);7,456(1,7);7,231(3,4); 7,224(3,0);7,215(3,1);7,211(4,1);7,208(3,9);7,205(3,3);7,195(2,8);7,188(2,8);7,074(8,6);7,068(8,7);5,448(5,4);2,168(30,1);2,109(0,3);1,965( 1,9);1,959(2,5);1,953(17,9);1,947(33,3);1,941(46,3);1,935(32,5);1,929(17,1);1,372(4,0);1,340(0,5);1,311(0,8);1,295(0,8);1,284(0,8);1,276(4, 5);0,008(0,5);0,000(13,4);-0,009(0,5) |
| I-1-395: |
| HPLC-MS: logP = 3,21; Masse (m/z): 411,0 (M+H)⁺; ¹H-NMR(400,0 MHz, DMSO-D₆): |
| δ= 11,571(0,7);11,504(4,3);8,849(3,2);8,838(3,3);8,254(0,6);8,247(1,1);8,217(7,9);8,211(7,7);8,195(3,4);7,841(2,4);7,829(2,6);7,822(2,3);7, 810(2,2);7,745(0,4);7,727(0,7);7,709(0,4);7,602(3,2);7,598(3,3);7,582(3,5);7,578(3,4);7,531(3,0);7,527(2,9);7,511(3,7);7,507(3,1);7,405(0,4) ;7,384(0,7);7,381(0,8);7,360(0,3);7,305(3,2);7,285(5,6);7,265(2,4);6,966(1,3);6,959(1,3);6,929(5,7);6,923(5,7);5,758(2,5);3,826(2,3);3,808(7 ,2);3,791(7,3);3,773(2,2);3,321(216,7);2,675(3,3);2,670(4,1);2,666(3,0);2,604(0,5);2,588(0,4);2,577(0,5);2,506(507,4);2,501(635,4);2,497(4 56,0);2,441(0,7);2,381(0,6);2,332(3,3);2,328(4,2);2,324(2,9);1,227(7,8);1,210(16,0);1,192(7,3);0,146(2,8);0,050(0,4);0,008(30,0);0,000(619, 9);-0,009(24,1);-0,030(1,0);-0,065(0,3);-0,150(3,0) |
| I-1-396: |
| HPLC-MS: logP = 2,91; Masse (m/z): 404,1 (M+H)⁺; ¹H-NMR(400,0 MHz, CD₃CN): |
| δ= 9,280(1,0);7,695(3,2);7,694(3,1);7,676(3,8);7,674(3,7);7,601(6,7);7,567(6,9);7,506(0,9);7,500(1,7);7,486(4,8);7,481(8,1);7,466(4,6);7,46 4(4,3);7,454(0,5);7,448(1,8);7,445(1,7);7,437(3,4);7,431(2,6);7,418(2,9);7,412(2,5);7,400(1,4);7,395(1,3);7,238(1,7);7,233(2,4);7,228(1,7);7, 218(2,8);7,217(2,9);7,213(3,0);7,210(2,7);7,151(1,0);7,136(1,4);7,130(3,6);7,117(4,3);7,115(4,6);7,112(5,0);7,096(3,2);7,091(3,1);7,086(3,1) ;7,070(1,0);7,065(0,7);5,446(0,6);4,785(5,2);4,751(5,1);4,227(2,2);4,225(2,1);4,209(6,1);4,207(6,1);4,192(6,1);4,189(6,1);4,174(2,0);4,172(2 |
| ,1);4,090(0,4);4,087(0,4);4,072(0,4);4,069(0,4);2,655(0,4);2,642(0,4);2,148(21,1);1,964(1,4);1,957(1,7);1,952(12,8);1,945(23,99);1,939(33,3); 1,933(23,0);1,927(11,9);1,874(1,3);1,860(1,3);1,486(0,4);1,360(8,1);1,359(8,1);1,3424(16,0);1,3416(15,9);1,325(8,0);1,261(0,6);1,259(0,4); 1,244(1,1);1,241(0,8);1,226(0,5);1,223(0,4);1,183(0,9);1,165(1,7);1,147(0,8);0,008(1,0);0,000(26,1);-0,009(0,9) |
| I-1-397: |
| HPLC-MS: logP = 2,74; Masse (m/z): 360,1 (M+H)⁺; ¹H-NMR(400,0 MHz, CD₃CN): |
| δ= 9,487(0,9);7,613(5,6);7,578(5,8);7,546(3,3);7,543(2,4);7,527(4,6);7,517(6,9);7,515(7,1);7,509(5,4);7,503(4,7);7,500(3,1);7,483(0,8);7,47 9(1,0);7,456(0,5);7,444(2,7);7,436(1,9);7,430(1,8);7,425(2,0);7,422(2,0);7,418(1,7);7,410(1,4);7,403(1,3);7,215(1,6);7,211(2,3);7,206(1,5);7, 195(2,7);7,193(3,0);7,191(2,8);7,187(2,6);7,147(1,1);7,132(1,4);7,126(3,3);7,111(5,7);7,104(3,4);7,092(2,5);7,084(3,0);7,079(2,7);7,063(1,0) ;7,058(0,8);5,448(1,9);4,741(5,3);4,707(5,2);4,224(2,3);4,208(6,2);4,207(6,2);4,191(6,1);4,189(6,1);4,173(2,1);4,172(2,1);2,196(21,5);2,189( 36,9);2,186(38,3);1,964(1,2);1,958(1,4);1,952(10,5);1,946(19,4);1,940(27,0);1,934(18,6);1,928(9,6);1,874(0,9);1,860(0,9);1,362(8,1);1,345( 16,0);1,327(8,0);1,262(0,4);1,245(0,7);1,240(0,4);1,227(0,4);1,182(0,4);1,164(0,8);1,147(0,6);0,008(0,9);0,000(23,3);-0,009(0,8) |
| I-1-398: |
| HPLC-MS: logP = 3,03; Masse (m/z): 420,0 (M+H)⁺; ¹H-NMR(400,0 MHz, DMSO-D₆): |
| δ= 11,097(2,3);7,775(2,7);7,769(2,7);7,696(1,5);7,694(1,5);7,677(1,8);7,674(1,8);7,546(1,0);7,542(1,2);7,527(1,8);7,523(1,8);7,495(1,4);7,4 75(2,9);7,461(2,0);7,458(2,0);7,454(2,0);7,442(1,1);7,439(1,1);7,411(1,1);7,406(1,1);7,391(1,3);7,387(1,3);7,372(0,6);7,368(0,6);7,227(2,2); 7,219(2,4);7,216(1,9);7,206(1,8);7,199(2,0);7,196(1,6);6,850(3,0);6,844(2,9);4,111(1,1);4,093(3,5);4,076(3,6);4,058(1,2);4,038(0,7);4,020(0, 7);3,321(53,8);3,233(0,5);3,019(0,5);2,675(0,6);2,670(0,9);2,666(0,7);2,523(2,5);2,510(51,4);2,506(102,4);2,501(134,1);2,497(96,8);2,492(4 6,5);2,332(0,7);2,328(0,9);2,323(0,6);1,989(2,9);1,398(16,0);1,330(0,4);1,221(3,7);1,204(7,9);1,192(1,3);1,187(3,7);1,179(0,8);1,175(1,7);1, 161(0,3);1,157(0,8);0,008(2,7);0,000(73,6);-0,009(2,4);-0,150(0,3) |
| I-1-399: |
| HPLC-MS: logP = 2,87; Masse (m/z): 404,0 (M+H)⁺; ¹H-NMR(400,0 MHz, CD₃CN): |
| δ= 4,132(0,4);4,115(0,4);2,327(16,0);2,324(15,8);1,982(0,6);1,976(1,2);1,970(1,6);1,964(1,1);1,958(0,6);1,319(0,4);1,302(0,9);1,284(0,4) |
| I-1-400: |
| HPLC-MS: Masse (m/z): 300,1 (M+H)⁺; ¹H-NMR(400,0 MHz, DMSO-D₆): |
| δ= 10,564(3,9);8,316(0,3);8,127(2,2);8,120(4,2);8,114(2,2);7,808(1,0);7,803(1,1);7,789(2,1);7,784(1,9);7,770(1,1);7,764(1,2);7,493(0,9);7,4 89(1,0);7,473(1,4);7,469(1,5);7,464(1,0);7,458(1,0);7,444(1,1);7,439(1,5);7,418(0,6);7,413(0,7);7,407(0,6);7,400(1,5);7,395(1,2);7,388(1,5); 7,382(1,6);7,376(2,8);7,371(2,9);7,363(1,1);7,357(2,3);7,352(2,1);7,338(0,8);7,334(0,7);6,930(4,8);6,924(4,8);6,801(4,5);3,903(2,2);3,328(1 94,0);2,675(0,7);2,671(0,9);2,667(0,7);2,523(23,9);2,511(64,2);2,507(123,5);2,502(157,3);2,498(112,4);2,436(0,5);2,333(0,8);2,329(1,0);2,3 24(0,7);2,315(0,5);2,241(16,0) |
| I-1-401: |
| HPLC-MS: logP = 3,33; Masse (m/z): 352,1 (M+H)⁺; ¹H-NMR(400,0 MHz, DMSO-D₆): |
| δ= 20,008(1,0);12,063(1,1);11,621(6,1);8,973(5,0);8,963(5,1);8,615(5,4);8,594(5,1);8,552(6,3);8,545(6,3);8,315(4,9);7,852(3,1);7,835(4,8);7 ,802(1,4);7,784(3,2);7,765(3,5);7,734(3,2);7,716(7,8);7,697(3,6);7,680(1,0);7,057(7,1);7,050(6,3);3,525(1,0);3,505(1,1);3,490(1,2);3,472(1,2 );3,447(1,3);3,430(1,4);3,406(3,2);3,398(2,2);3,393(2,1);3,329(5383,3);3,268(3,1);3,231(1,4);3,216(1,2);3,204(1,5);3,194(1,1);3,191(1,1);2,7 21(1,1);2,675(11,6);2,671(16,0);2,666(11,9);2,585(2,7);2,541(94,2);2,511(970,1);2,506(1928,6);2,502(2517,8);2,497(1817,5);2,493(878,5);2 ,444(3,4);2,383(1,3);2,370(1,4);2,333(11,5);2,329(16,0);2,324(11,1);2,290(1,0);1,297(1,5);1,259(2,3);1,235(2,5);0,827(1,0);0,000(6,0);-3,400(1,2) |
| I-1-402: |
| HPLC-MS: logP = 2,76; Masse (m/z): 353,1 (M+H)⁺; ¹H-NMR(400,0 MHz, DMSO-D₆): |
| δ= 16,885(1,2);11,782(9,5);8,977(8,5);8,967(8,0);8,865(5,3);8,854(5,6);8,626(8,2);8,606(8,3);8,572(9,9);8,566(10,3);8,315(5,9);8,239(5,6);8 ,220(5,7);7,858(4,4);7,847(4,7);7,839(4,5);7,826(4,2);7,062(11,0);7,055(11,0);3,573(1,1);3,537(1,4);3,481(1,6);3,473(1,1);3,440(1,9);3,423( 1,6);3,373(5,2);3,327(3958,0);3,305(11,2);3,290(4,8);3,278(1,7);3,250(1,4);3,135(1,1);2,788(1,1);2,675(11,9);2,671(16,0);2,635(1,6);2,616( 1,6);2,598(1,7);2,541(91,1);2,506(2024,8);2,502(2524,4);2,498(1779,8);2,431(1,6);2,422(1,7);2,333(11,7);2,329(15,5);1,301(1,5);1,259(2,3); 1,236(4,4);0,854(1,1);0,000(12,1) |
| I-1-403: |
| HPLC-MS: logP = 2,32; Masse (m/z): 320,1 (M+H)⁺; ¹H-NMR(400,0 MHz, DMSO-D₆): |
| δ= 11,207(1,6);11,202(1,2);9,078(1,6);8,717(1,7);8,696(1,9);8,315(4,8);7,903(1,6);7,639(1,2);7,598(1,2);7,300(1,3);7,281(2,0);7,260(1,3);6, 779(1,7);3,733(1,2);3,584(1,1);3,530(1,1);3,503(1,1);3,481(1,3);3,463(1,5);3,436(1,6);3,421(1,6);3,397(2,2);3,383(4,0);3,326(3356,3);3,301( 3,9);3,295(3,2);3,262(2,3);3,164(1,1);3,092(1,1);2,728(1,2);2,675(12,2);2,671(16,0);2,614(1,3);2,591(1,6);2,575(2,5);2,541(74,5);2,506(203 1,7);2,502(2577,3);2,497(1866,6);2,436(2,4);2,374(1,1);2,363(1,2);2,333(12,0);2,329(16,0);2,290(1,1);2,259(1,3);1,297(1,8);1,258(2,6);1,23 4(2,6);0,858(1,1);0,008(1,1);0,000(7,7);-1,654(1,1);-3,506(1,0) |
| I-1-404: |
| HPLC-MS: logP = 3,19; Masse (m/z): 364,0 (M+H)⁺; ¹H-NMR(400,0 MHz, DMSO-D₆): |
| δ= 11,519(13,3);8,973(11,1);8,963(11,1);8,947(0,9);8,612(12,1);8,592(12,1);8,551(14,5);8,544(14,2);8,480(1,2);8,315(5,5);7,718(9,4);7,701( 11,2);7,698(11,2);7,570(5,7);7,566(6,7);7,551(9,5);7,547(10,1);7,505(5,7);7,502(6,2);7,487(11,9);7,484(11,9);7,468(6,4);7,465(6,5);7,444(8, 3);7,439(8,1);7,424(9,0);7,420(8,9);7,405(4,4);7,401(3,8);7,076(15,2);7,069(14,8);5,071(0,9);4,612(0,9);3,550(1,2);3,534(0,9);3,516(1,2);3,4 95(1,4);3,486(1,3);3,473(1,2);3,468(1,7);3,453(1,3);3,439(1,8);3,433(1,3);3,423(1,5);3,415(1,9);3,408(3,2);3,394(4,3);3,373(7,0);3,357(17,8) ;3,329(5998,7);3,289(3,4);3,281(2,1);3,268(1,2);3,247(1,6);3,238(1,2);3,234(1,3);3,210(1,0);3,203(1,3);3,150(2,3);2,994(1,0);2,770(1,0);2,7 67(1,0);2,696(0,9);2,676(11,8);2,671(16,0);2,666(12,2);2,606(1,3);2,541(134,0);2,524(54,1);2,520(84,7);2,511(942,6);2,506(1902,0);2,502(2 501,8);2,497(1787,9);2,493(846,4);2,419(1,4);2,337(5,0);2,333(11,2);2,329(15,2);2,324(10,9);2,290(1,2);1,336(0,9);1,298(1,9);1,258(2,5);1, 235(5,2);0,854(1,0);0,000(8,6) |
| I-1-405: |
| HPLC-MS: logP = 3,28; Masse (m/z): 410,0 (M+H)⁺; ¹H-NMR(400,0 MHz, DMSO-D₆): |
| δ= 16,864(1,0);16,266(1,1);16,176(1,0);11,478(1,1);11,460(8,0);8,975(6,7);8,964(6,5);8,611(7,2);8,591(7,4);8,551(8,1);8,544(8,4);8,316(5,1 );7,937(7,0);7,917(7,7);7,495(10,8);7,484(15,5);7,252(3,7);7,242(3,2);7,239(3,5);7,232(4,0);7,220(3,8);7,209(3,2);7,076(8,8);7,069(8,4);3,47 1(1,1);3,445(1,2);3,429(1,1);3,418(1,3);3,403(1,4);3,370(2,4);3,324(2280,3);3,295(2,9);3,281(1,5);3,273(1,3);3,190(1,0);2,756(1,1);2,752(1, 2);2,675(11,7);2,671(16,0);2,666(11,6);2,628(1,8);2,606(1,6);2,541(99,5);2,524(54,6);2,511(999,9);2,506(1969,9);2,502(2550,4);2,497(1811 ,4);2,493(859,8);2,333(11,4);2,328(15,2);2,324(11,1);2,289(1,5);1,298(1,7);1,259(2,7);1,235(5,7);0,854(1,1);0,831(1,2);0,000(11,0) |
| I-1-406: |
| HPLC-MS: logP = 2,84; Masse (m/z): 362,1 (M+H)⁺; ¹H-NMR(400,0 MHz, DMSO-D₆): |
| δ= 11,269(2,4);8,126(2,9);8,120(2,9);7,832(1,3);7,812(1,6);7,779(0,5);7,761(1,3);7,742(1,2);7,706(1,0);7,686(1,4);7,678(1,8);7,668(0,6);7,6 59(1,2);7,603(1,5);7,599(1,6);7,584(1,7);7,580(1,7);7,365(0,6);7,361(0,6);7,344(1,3);7,340(1,1);7,326(1,1);7,322(1,1);7,258(1,8);7,256(2,0); 7,238(1,3);7,235(1,2);7,093(1,0);7,090(1,0);7,073(1,6);7,071(1,5);7,055(0,8);7,051(0,8);6,846(3,2);6,840(3,1);5,756(1,7);3,883(16,0);3,323( 8,7);2,524(0,4);2,510(8,3);2,506(16,5);2,501(21,6);2,497(15,3);2,492(7,2);0,008(1,0);0,000(24,9);-0,009(0,9) |
| I-1-407: |
| HPLC-MS: logP = 2,37; Masse (m/z): 363,1 (M+H)⁺; ¹H-NMR(400,0 MHz, DMSO-D₆): |
| δ= 11,445(2,1);8,843(1,1);8,840(1,2);8,831(1,2);8,829(1,2);8,192(1,1);8,189(1,1);8,172(1,2);8,170(1,2);8,142(2,8);8,136(2,8);7,834(1,1);7,8 22(1,1);7,814(1,0);7,802(1,0);7,597(1,5);7,593(1,6);7,577(1,7);7,573(1,6);7,371(0,6);7,367(0,7);7,353(0,9);7,351(1,2);7,346(1,1);7,332(1,2); 7,328(1,1);7,263(1,7);7,260(1,8);7,242(1,3);7,239(1,2);7,094(1,0);7,091(1,0);7,075(1,4);7,073(1,4);7,056(0,9);7,053(0,8);6,850(3,1);6,844(3, 1);3,884(16,0);3,869(0,8);3,322(12,7);2,524(0,5);2,519(0,8);2,511(10,9);2,506(22,2);2,502(29,3);2,497(20,7);2,492(9,6);1,989(1,2);1,235(0, 4);1,193(0,3);1,175(0,7);1,157(0,3);0,008(1,3);0,000(39,1);-0,009(1,2) |
| I-1-408: |
| HPLC-MS: logP = 2,57; Masse (m/z): 330,1 (M+H)⁺; ¹H-NMR(400,0 MHz, DMSO-D₆): |
| δ= 11,491(2,1);8,138(2,8);8,132(2,8);7,600(1,7);7,595(1,6);7,583(0,9);7,580(2,2);7,576(1,8);7,567(0,5);7,562(1,3);7,558(0,5);7,546(0,6);7,5 41(0,8);7,525(0,3);7,373(0,7);7,369(0,7);7,355(1,0);7,352(1,2);7,351(1,1);7,348(1,1);7,334(1,2);7,330(1,1);7,261(1,7);7,258(1,9);7,240(1,5); 7,237(1,5);7,232(2,3);7,212(2,9);7,192(1,8);7,184(0,4);7,096(1,0);7,093(1,0);7,076(1,5);7,074(1,4);7,058(0,9);7,054(0,8);6,851(3,2);6,845(3, 1);5,756(0,8);3,881(16,0);3,322(7,9);2,524(0,4);2,511(8,4);2,506(16,8);2,502(22,0);2,497(15,6);2,492(7,4);1,989(0,3);0,008(1,0);0,000(27,4) ;-0,009(1,0) |
| I-1-409: |
| HPLC-MS: logP = 2,12; Masse (m/z): 373,0 (M+H)⁺; ¹H-NMR(400,0 MHz, DMSO-D₆): |
| δ= 11,351(2,2);8,477(1,6);8,472(1,7);8,465(1,7);8,460(1,6);8,140(2,8);8,134(2,9);7,982(1,6);7,977(1,6);7,963(1,8);7,958(1,7);7,600(1,5);7,5 96(1,5);7,580(1,6);7,576(1,6);7,562(1,7);7,550(1,7);7,543(1,6);7,531(1,7);7,370(0,7);7,366(0,6);7,349(1,3);7,346(1,1);7,331(1,1);7,327(1,0); 7,262(1,9);7,259(2,0);7,241(1,3);7,238(1,2);7,096(1,0);7,093(1,0);7,077(1,6);7,074(1,5);7,058(0,9);7,055(0,8);6,865(3,0);6,859(3,0);5,755(1, 6);4,038(0,5);4,020(0,5);3,883(16,0);3,869(0,5);3,856(0,3);3,320(38,0);2,675(0,4);2,671(0,5);2,666(0,4);2,524(1,2);2,510(31,2);2,506(62,5); 2,501(81,5);2,497(58,0);2,492(27,4);2,333(0,4);2,328(0,5);2,324(0,4);1,988(2,1);1,193(0,6);1,175(1,1);1,157(0,5);0,146(0,4);0,008(3,4);0,00 0(89,0);-0,009(3,1);-0,150(0,4) |
| I-1-410: |
| HPLC-MS: logP = 2,70; Masse (m/z): 328,1 (M+H)⁺; ¹H-NMR(400,0 MHz, DMSO-D₆): |
| δ= 11,183(2,3);8,128(2,9);8,122(2,9);7,605(1,5);7,601(1,6);7,585(1,7);7,581(1,7);7,567(1,2);7,563(1,3);7,548(2,3);7,544(2,8);7,527(2,1);7,5 24(2,2);7,504(1,0);7,500(1,0);7,486(1,7);7,481(1,4);7,466(1,0);7,461(0,8);7,444(1,4);7,440(1,4);7,426(1,7);7,422(1,6);7,407(0,7);7,404(0,6); 7,364(0,7);7,360(0,7);7,345(1,0);7,343(1,3);7,339(1,2);7,325(1,2);7,321(1,1);7,257(1,8);7,254(2,0);7,236(1,3);7,233(1,2);7,093(1,0);7,090(1, 0);7,074(1,5);7,072(1,5);7,055(0,9);7,052(0,8);6,866(3,2);6,860(3,1);5,755(5,7);3,882(16,0);3,323(9,2);2,523(0,3);2,510(7,9);2,506(16,0);2,5 01(21,0);2,496(14,9);2,492(7,0);1,988(1,0);1,397(1,2);1,175(0,5);0,008(1,0);0,000(26,5);-0,009(0,9) |
| I-1-411: |
| HPLC-MS: logP = 2,83; Masse (m/z): 420,0 (M+H)⁺; ¹H-NMR(400,0 MHz, DMSO-D₆): |
| δ= 11,106(2,5);8,127(3,0);8,120(2,9);7,919(1,8);7,899(2,1);7,609(1,5);7,605(1,6);7,589(1,7);7,585(1,6);7,496(0,4);7,493(0,4);7,477(1,5);7,4 74(1,5);7,461(2,9);7,457(3,4);7,444(0,7);7,362(0,7);7,358(0,6);7,341(1,3);7,338(1,1);7,323(1,2);7,319(1,1);7,257(1,9);7,255(2,1);7,236(1,4); 7,234(1,3);7,226(1,0);7,219(0,9);7,209(1,0);7,206(1,1);7,203(1,0);7,199(0,9);7,190(0,8);7,183(0,8);7,094(1,0);7,091(1,0);7,074(1,6);7,072(1, 5);7,056(0,8);7,053(0,8);6,862(3,2);6,856(3,1);5,755(5,2);4,037(0,6);4,020(0,6);3,884(16,0);3,321(11,9);2,523(0,4);2,510(9,9);2,505(20,0);20, 501(26,3);2,496(18,6);2,492(8,7);1,988(2,7);1,397(1,1);1,192(0,7);1,175(1,5);1,157(0,7);0,008(1,2);0,000(31,6);-0,009(1,0) |
| I-1-412: |
| HPLC-MS: Masse (m/z): 314,0 (M+H)⁺; ¹H-NMR(400,0 MHz, DMSO-D₆): |
| δ= +10,718(2,3);8,379(2,7);8,373(2,6);8,229(2,8);8,223(3,3);8,160(3,3);8,153(2,8);6,971(2,8);6,964(2,8);6,835(2,7);4,039(16,0);3,903(2,9);3, 329(55,9);2,672(0,3);2,526(12,9);2,511(21,1);2,507(41,7);2,503(53,4);2,498(37,5);2,494(17,4);2,329(0,3);2,237(9,3) |
| I-1-413: |
| HPLC-MS: Masse (m/z): 374,0 (M+H)⁺; ¹H-NMR(400,0 MHz, DMSO-D₆): |
| δ= 11,377(2,4);8,401(2,7);8,395(2,7);8,257(3,1);8,250(3,6);8,175(3,5);8,168(3,0);7,709(1,6);7,707(1,6);7,689(1,9);7,687(1,8);7,552(1,0);7,5 47(1,1);7,533(1,8);7,528(1,9);7,495(0,9);7,492(0,9);7,476(1,9);7,474(1,7);7,458(1,0);7,455(0,9);7,429(1,2);7,424(1,1);7,409(1,4);7,405(1,3); 7,391(0,6);7,386(0,6);6,987(2,9);6,981(2,8);4,038(16,0);3,909(4,1);3,335(152,9);2,682(0,4);2,677(0,6);2,673(0,4);2,530(1,5);2,517(37,6);2,5 13(75,6);2,508(98,3);2,504(70,0);2,499(33,4);2,339(0,4);2,335(0,6);2,330(0,4) |
| I-1-414: |
| HPLC-MS: Masse (m/z): 296,1 (M+H)⁺; ¹H-NMR(400,0 MHz, DMSO-D₆): |
| δ= 10,423(2,8);7,937(3,5);7,931(3,5);7,406(0,8);7,399(1,1);7,388(1,9);7,384(2,3);7,374(1,7);7,366(3,4);7,354(3,2);7,349(3,3);7,340(3,4);7,3 31(1,5);7,327(0,9);6,839(3,6);6,833(3,5);6,781(3,2);3,909(2,2);3,333(66,9);2,682(0,3);2,677(0,4);2,526(17,6);2,513(52,6);2,508(66,7);2,504( 47,6);2,334(0,4);2,273(16,0);2,240(11,9) |
| I-1-415: |
| HPLC-MS: logP = 2,63; Masse (m/z): 377,1 (M+H)⁺; ¹H-NMR(400,0 MHz, DMSO-D₆): |
| δ= 11,332(10,6);8,257(10,7);8,250(11,0);8,025(6,7);8,005(7,4);7,862(1,7);7,859(1,8);7,842(5,9);7,839(5,7);7,821(16,0);7,815(11,9);7,802(8, 5);7,766(2,1);7,748(5,9);7,730(5,3);7,700(4,8);7,681(6,0);7,667(8,5);7,650(8,6);7,629(5,9);7,613(3,1);7,608(3,0);6,946(11,1);6,939(11,2);5,7 58(12,3);4,056(0,7);4,038(2,2);4,020(2,2);4,002(0,7);3,326(49,0);2,890(0,4);2,671(0,6);2,506(76,5);2,502(98,0);2,498(75,7);2,328(0,6);1,98 9(9,3);1,236(0,6);1,192(2,5);1,174(4,9);1,157(2,5);0,000(9,8) |
| I-1-416: |
| HPLC-MS: logP = 2,16; Masse (m/z): 378,1 (M+H)⁺; ¹H-NMR(400,0 MHz, DMSO-D₆): |
| δ= 11,478(11,1);8,833(6,0);8,823(5,9);8,821(5,9);8,287(12,7);8,281(12,7);8,193(5,7);8,175(6,1);8,173(6,1);8,029(6,6);8,027(7,0);8,009(7,4); 8,007(7,6);7,866(1,8);7,863(1,7);7,846(6,4);7,843(6,2);7,827(16,0);7,822(15,4);7,808(8,1);7,801(6,0);7,789(4,8);7,655(4,5);7,650(4,1);7,638 (4,5);7,635(5,5);7,630(4,1);7,619(3,7);7,613(3,3);6,950(13,6);6,944(13,6);5,758(11,6);3,323(112,0);2,675(1,8);2,671(2,5);2,666(1,8);2,524(8 ,8);2,510(143,1);2,506(280,1);2,501(363,7);2,497(264,2);2,493(129,3);2,333(1,8);2,328(2,4);2,324(1,8);0,146(0,7);0,008(6,5);0,000(168,4);-0,009(6,8);-0,150(0,7) |
| I-1-417: |
| HPLC-MS: logP = 2,34; Masse (m/z): 345,1 (M+H)⁺; ¹H-NMR(400,0 MHz, DMSO-D₆): |
| δ= 11,551(10,4);8,288(12,5);8,282(12,2);8,032(6,4);8,029(6,8);8,011(7,2);8,009(7,4);7,866(1,7);7,863(1,7);7,846(6,1);7,843(5,7);7,826(16,0 );7,821(11,3);7,805(3,3);7,801(1,7);7,658(4,3);7,653(4,0);7,640(4,4);7,637(5,5);7,633(4,1);7,621(3,5);7,615(3,2);7,599(1,4);7,582(3,0);7,578 (2,8);7,561(5,5);7,544(2,9);7,540(3,3);7,523(1,5);7,233(1,8);7,227(9,3);7,207(13,8);7,186(8,1);7,179(1,9);6,954(13,1);6,948(13,0);5,758(13, 8);4,038(0,8);4,020(0,9);3,326(54,5);2,676(0,6);2,671(0,7);2,666(0,6);2,524(2,6);2,511(42,7);2,506(82,2);2,502(105,8);2,497(76,7);2,493(37 ,2);2,333(0,5);2,329(0,7);2,324(0,5);1,989(3,7);1,235(0,5);1,192(1,0);1,175(2,0);1,157(1,0);0,008(0,5);0,000(12,7);-0,008(0,4) |
| I-1-418: |
| HPLC-MS: logP = 2,50; Masse (m/z): 389,0 (M+H)⁺; ¹H-NMR(400,0 MHz, DMSO-D₆): |
| δ= 11,243(12,7);11,214(0,3);8,253(13,1);8,246(12,9);8,025(8,0);8,023(7,8);8,005(9,0);7,860(2,4);7,857(2,2);7,840(7,5);7,823(8,7);7,820(8,8 );7,814(11,5);7,810(11,9);7,794(4,2);7,790(2,9);7,687(8,3);7,668(9,8);7,649(5,2);7,645(4,8);7,628(7,2);7,611(3,9);7,607(3,5);7,523(4,9);7,51 9(5,4);7,504(9,3);7,500(9,2);7,472(4,8);7,470(4,7);7,454(9,2);7,435(4,9);7,433(4,3);7,410(5,8);7,406(5,5);7,391(7,0);7,387(6,5);7,372(3,1);7, 368(2,7);6,963(13,2);6,957(13,0);5,757(16,0);4,055(0,4);4,038(1,3);4,020(1,3);4,002(0,4);3,324(99,1);2,675(0,9);2,670(1,2);2,666(0,9);2,50 6(140,0);2,501(173,0);2,497(127,7);2,328(1,2);2,324(0,9);1,989(5,4);1,236(0,6);1,192(1,5);1,174(2,9);1,157(1,5);0,000(17,8) |
| I-1-419: |
| HPLC-MS: logP = 2,61; Masse (m/z): 435,0 (M+H)⁺; ¹H-NMR(400,0 MHz, DMSO-D₆): |
| δ= 11,213(0,4);11,184(11,8);8,251(11,9);8,244(12,1);8,219(0,3);8,213(0,3);8,025(7,1);8,023(7,7);8,003(8,5);7,906(8,9);7,886(10,2);7,861(2, 1);7,858(2,1);7,840(6,6);7,838(6,1);7,823(8,0);7,817(11,1);7,812(11,4);7,796(3,6);7,792(2,4);7,648(4,5);7,643(4,4);7,627(6,5);7,610(3,6);7,6 06(3,5);7,481(1,9);7,462(7,3);7,448(14,9);7,444(16,0);7,431(3,0);7,425(1,1);7,415(0,5);7,220(4,0);7,213(3,8);7,203(4,3);7,200(5,1);7,197(4, 9);7,194(4,2);7,184(3,6);7,177(3,3);7,163(0,4);6,962(12,3);6,956(12,4);5,757(14,7);4,055(0,7);4,038(2,2);4,020(2,2);4,002(0,7);3,324(82,5); 2,675(0,9);2,670(1,3);2,666(1,0);2,506(140,5);2,501(183,1);2,497(136,5);2,332(0,9);2,328(1,2);2,324(0,9);1,989(9,3);1,235(0,6);1,192(2,5); 1,174(5,0);1,157(2,5);0,008(0,8);0,000(19,6);-0,008(0,9) |
| I-1-420: |
| HPLC-MS: logP = 2,33; Masse (m/z): 299,1 (M+H)⁺; ¹H-NMR(601,6 MHz, CD₃CN): |
| δ= 9,281(4,1);8,525(15,9);8,520(16,0);8,415(7,8);8,407(7,9);7,902(4,2);7,899(4,4);7,888(8,3);7,876(5,6);7,874(5,6);7,784(12,7);7,771(9,9);7 ,614(7,9);7,612(8,5);7,602(9,2);7,599(9,7);7,523(6,5);7,510(13,6);7,496(5,6);7,493(5,9);7,483(8,6);7,481(8,9);7,470(4,4);7,468(4,2);7,439(6, 7);7,437(7,1);7,427(9,9);7,425(10,5);7,414(4,0);7,413(4,2);7,262(7,0);7,254(7,4);7,250(7,2);7,241(6,7);7,005(15,8);7,001(15,8);5,449(0,5);2, 187(0,5);2,163(312,3);2,057(0,5);2,052(0,8);2,048(0,5);1,966(4,5);1,958(4,2);1,954(5,5);1,950(50,8);1,946(93,5);1,942(136,3);1,938(93,3);1 ,934(47,4);1,831(0,5);1,827(0,8);1,823(0,5);1,436(1,6);1,267(0,9);1,211(0,5);1,199(1,0);1,188(0,5);1,025(0,5);1,013(1,0);1,002(0,5);0,000(1 0,1);-0,006(0,3) |
| I-1-421: |
| HPLC-MS: logP = 3,43; Masse (m/z): 492,8 (M+H)⁺; ¹H-NMR(400,0 MHz, CD₃CN): |
| δ= 9,515(6,0);8,730(14,8);8,728(15,0);8,318(13,9);8,314(14,3);8,303(15,9);8,296(16,0);7,901(12,5);7,882(12,5);7,881(12,9);7,481(4,3);7,47 6(5,6);7,462(13,7);7,457(13,9);7,449(11,7);7,446(11,8);7,431(13,0);7,428(13,6);7,412(5,1);7,409(5,1);7,241(0,4);7,235(0,4);7,194(7,7);7,18 9(7,3);7,176(8,0);7,174(9,8);7,171(9,8);7,169(9,0);7,156(6,1);7,151(6,6);7,091(15,1);7,085(15,2);5,448(10,7);2,154(69,1);2,121(0,5);2,115(0 ,6);2,109(0,8);2,102(0,5);1,972(1,4);1,965(4,8);1,959(5,8);1,954(45,5);1,947(85,2);1,941(119,5);1,935(83,7);1,929(43,7);1,776(0,5);1,770(0, 7);1,763(0,5);1,436(1,1);1,372(9,7);1,340(0,9);1,284(1,4);1,276(11,0);1,221(0,4);1,216(0,7);1,204(0,6);1,186(0,4);0,008(1,2);0,000(37,1);-0,009(1,5) |
| I-1-422: |
| HPLC-MS: logP = 2,72; Masse (m/z): 401,2 (M+H)⁺; ¹H-NMR(400,0 MHz, DMSO-D₆): |
| δ= 11,341(9,6);8,818(5,1);8,815(5,4);8,806(5,3);8,803(5,2);8,457(5,4);8,453(5,4);8,437(5,7);8,433(5,5);8,343(10,7);8,337(10,7);7,835(5,1);7 ,816(7,0);7,783(1,9);7,766(5,5);7,747(5,0);7,714(8,4);7,696(16,0);7,680(7,7);6,964(11,6);6,958(11,6);3,326(425,1);2,995(0,5);2,711(1,0);2,6 80(0,7);2,675(1,4);2,671(1,9);2,666(1,4);2,589(0,4);2,541(276,2);2,529(2,9);2,524(5,9);2,511(110,0);2,506(219,0);2,502(286,3);2,497(211,3 );2,493(104,9);2,368(1,0);2,338(0,6);2,333(1,3);2,329(1,8);2,324(1,3);2,075(6,0);1,259(0,3);1,235(0,8);0,008(1,7);0,000(48,3);-0,009(1,9) |
| I-1-423: |
| HPLC-MS: logP = 2,46; Masse (m/z): 369,1 (M+H)⁺; ¹H-NMR(400,0 MHz, DMSO-D₆): |
| δ= 11,567(11,4);8,822(6,4);8,819(6,6);8,810(6,7);8,807(6,4);8,464(6,9);8,460(6,7);8,444(7,3);8,440(6,8);8,369(14,1);8,362(14,1);7,722(5,0); 7,710(5,0);7,703(4,9);7,690(4,6);7,614(1,5);7,597(3,4);7,593(3,1);7,576(6,3);7,559(3,3);7,555(3,7);7,538(1,6);7,250(2,1);7,243(11,0);7,223( 16,0);7,203(9,1);7,196(1,8);6,979(15,5);6,972(15,3);3,464(0,4);3,337(1001,1);2,996(0,5);2,712(2,3);2,676(1,5);2,672(2,0);2,667(1,5);2,585( 0,7);2,577(0,8);2,542(523,9);2,525(6,3);2,512(116,3);2,507(227,0);2,503(293,2);2,498(212,4);2,494(103,0);2,368(2,2);2,339(0,7);2,334(1,4); 2,329(1,8);2,325(1,3);2,075(2,0);1,259(0,3);1,235(0,7);0,000(5,2) |
| I-1-424: |
| HPLC-MS: logP = 2,61; Masse (m/z): 411,1 (M+H)⁺; ¹H-NMR(400,0 MHz, DMSO-D₆): |
| δ= 11,266(13,4);8,816(7,5);8,813(7,7);8,804(7,8);8,801(7,5);8,455(7,9);8,452(7,7);8,435(8,4);8,432(7,9);8,344(14,9);8,337(14,8);7,711(6,6); 7,701(13,6);7,691(6,7);7,681(14,3);7,553(5,9);7,549(6,6);7,534(10,1);7,530(10,2);7,489(5,0);7,486(5,1);7,471(10,2);7,468(9,6);7,452(5,5);7, 449(5,0);7,425(6,4);7,420(6,4);7,406(7,8);7,401(7,5);7,387(3,5);7,382(3,1);6,986(16,0);6,980(15,7);3,400(0,4);3,367(1,0);3,327(1143,5);2,6 75(3,1);2,671(4,3);2,666(3,1);2,662(1,4);2,541(14,5);2,524(12,6);2,511(258,5);2,506(511,5);2,502(663,7);2,497(475,1);2,493(225,0);2,422(0 ,4);2,419(0,4);2,338(1,6);2,333(3,2);2,328(4,3);2,324(3,1);2,288(0,4);2,075(5,8);1,298(0,5);1,258(0,6);1,235(0,6);1,147(0,5);0,008(2,6);0,00 0(78,5);-0,008(2,3) |
| I-1-425: |
| HPLC-MS: logP = 2,57; Masse (m/z): 367,1 (M+H)⁺; ¹H-NMR(400,0 MHz, DMSO-D₆): |
| δ= 11,281(13,6);8,816(8,2);8,814(8,4);8,805(8,5);8,456(8,5);8,452(8,3);8,436(9,0);8,433(8,4);8,344(15,1);8,338(14,9);7,713(6,6);7,700(6,6); 7,693(6,5);7,681(6,0);7,583(7,2);7,580(7,6);7,565(9,6);7,561(10,0);7,553(6,1);7,550(6,4);7,533(12,9);7,530(12,8);7,513(5,5);7,509(5,6);7,49 5(9,4);7,490(8,1);7,475(5,0);7,470(4,1);7,449(7,5);7,445(7,1);7,430(9,5);7,427(8,8);7,412(3,6);7,409(3,1);6,989(16,0);6,983(15,7);3,428(0,4) ;3,414(0,5);3,399(0,7);3,329(1012,7);2,711(0,5);2,675(2,4);2,671(3,3);2,667(2,4);2,662(1,2);2,541(109,9);2,524(11,1);2,511(199,6);2,506(38 7,5);2,502(498,8);2,497(358,8);2,493(172,4);2,368(0,4);2,333(2,4);2,329(3,2);2,324(2,3);2,075(3,0);1,298(0,4);1,258(0,5);1,236(0,6);1,148( 0,3);0,008(1,3);0,000(36,0);-0,009(1,2) |
| I-1-426: |
| HPLC-MS: logP = 2,69; Masse (m/z): 402,1 (M+H)⁺; ¹H-NMR(400,0 MHz, DMSO-D₆): |
| δ= 11,334(8,8);8,925(5,2);8,924(5,2);8,914(5,3);8,824(4,9);8,821(5,1);8,813(5,1);8,809(4,9);8,466(5,3);8,462(5,3);8,446(5,7);8,442(5,4);8,3 78(11,8);8,372(16,0);8,352(5,5);7,808(3,6);7,807(3,6);7,795(3,6);7,788(3,4);7,776(3,3);7,721(3,9);7,709(3,9);7,702(3,7);7,690(3,6);6,976(12 ,6);6,969(12,4);3,412(0,3);3,398(0,5);3,383(0,7);3,330(854,2);2,680(0,8);2,676(1,7);2,671(2,4);2,667(1,7);2,662(0,8);2,541(27,7);2,524(7,2); 2,520(11,5);2,511(138,4);2,507(274,7);2,502(357,8);2,497(254,2);2,493(118,8);2,338(0,8);2,333(1,7);2,329(2,3);2,324(1,6);2,320(0,7);2,075 (2,5);1,299(0,3);1,259(0,5);1,235(0,6);0,008(0,9);0,000(25,8);-0,009(0,8) |
| I-1-427: |
| HPLC-MS: logP = 2,72; Masse (m/z): 459,0 (M+H)⁺; ¹H-NMR(400,0 MHz, DMSO-D₆): |
| δ= 11,207(8,8);8,816(4,9);8,813(5,0);8,804(5,0);8,801(4,8);8,455(5,1);8,451(5,1);8,435(5,5);8,432(5,2);8,343(9,4);8,336(9,4);7,921(7,2);7,9 01(8,1);7,710(4,0);7,698(4,0);7,690(3,9);7,678(3,7);7,501(0,8);7,477(9,4);7,468(16,0);7,244(0,5);7,234(3,1);7,225(3,0);7,221(3,2);7,214(3,6) ;7,212(3,4);7,205(2,9);7,202(3,3);7,192(2,5);7,182(0,4);6,985(10,0);6,978(9,9);3,493(0,3);3,482(0,4);3,456(0,6);3,447(0,6);3,428(0,8);3,413( 1,0);3,331(2037,1);3,283(1,1);3,246(0,3);2,712(0,5);2,680(1,8);2,676(3,6);2,671(5,0);2,666(3,6);2,662(1,7);2,617(0,3);2,604(0,4);2,541(109, 5);2,524(16,4);2,519(26,0);2,511(293,4);2,506(579,0);2,502(751,7);2,497(537,4);2,493(254,0);2,432(0,3);2,368(0,4);2,333(3,5);2,329(4,8);2, 324(3,4);2,289(0,6);2,074(4,2);1,298(0,6);1,258(0,8);1,235(1,8);1,148(0,5);0,008(2,0);0,000(59,5);-0,009(1,8) |
| I-1-428: |
| HPLC-MS: logP = 3,05; Masse (m/z): 401,1 (M+H)⁺; ¹H-NMR(400,0 MHz, DMSO-D₆): |
| δ= 11,408(6,6);8,620(8,0);8,615(8,7);8,511(8,1);8,506(7,3);8,268(7,7);8,261(7,6);7,835(3,6);7,816(4,6);7,783(1,3);7,766(3,8);7,747(3,3);7,7 13(3,0);7,694(8,9);7,675(4,2);6,956(8,4);6,950(8,3);5,756(16,0);3,324(13,3);2,525(0,8);2,512(14,6);2,507(29,0);2,503(37,9);2,498(26,7);2,4 93(12,4);1,989(0,6);1,397(10,0);1,175(0,4);0,008(2,3);0,000(55,8);-0,009(1,8) |
| I-1-429: |
| HPLC-MS: logP = 2,77; Masse (m/z): 369,0 (M+H)⁺; ¹H-NMR(400,0 MHz, DMSO-D₆): |
| δ= 11,633(4,8);8,623(6,6);8,618(7,2);8,516(7,2);8,510(6,5);8,286(5,7);8,279(5,7);7,611(0,6);7,594(1,4);7,590(1,2);7,577(1,0);7,573(2,5);7,5 69(1,0);7,556(1,3);7,552(1,5);7,535(0,6);7,246(0,8);7,240(4,3);7,220(6,0);7,199(3,6);7,192(0,7);6,966(6,5);6,959(6,4);5,756(16,0);3,328(21, 7);2,526(0,4);2,513(7,8);2,508(15,6);2,504(20,4);2,499(14,4);2,495(6,8);0,008(1,1);0,000(26,5);-0,009(0,9) |
| I-1-430: |
| HPLC-MS: logP = 2,95; Masse (m/z): 410,8 (M+H)⁺; ¹H-NMR(400,0 MHz, DMSO-D₆): |
| δ= 11,308(4,6);8,618(5,5);8,613(5,9);8,508(6,0);8,503(5,4);8,264(5,1);8,258(5,0);7,703(2,8);7,700(2,8);7,683(3,3);7,680(3,2);7,550(2,0);7,5 45(2,2);7,531(3,4);7,527(3,4);7,488(1,6);7,485(1,7);7,470(3,3);7,467(3,1);7,451(1,8);7,448(1,6);7,423(2,2);7,418(2,2);7,403(2,5);7,399(2,5); 7,385(1,2);7,380(1,1);6,975(5,5);6,968(5,4);5,755(16,0);3,324(9,0);2,525(0,4);2,520(0,6);2,511(7,6);2,507(15,2);2,502(19,8);2,498(13,9);2,4 93(6,4);0,008(1,0);0,000(28,2);-0,009(0,9) |
| I-1-431: |
| HPLC-MS: logP = 2,29; Masse (m/z): 413,9 (M+H)⁺; ¹H-NMR(400,0 MHz, DMSO-D₆): |
| δ= 11,486(11,0);8,623(14,3);8,617(15,6);8,515(16,0);8,510(14,4);8,483(7,4);8,478(8,0);8,471(8,0);8,466(7,8);8,311(1,2);8,283(12,6);8,277(1 2,6);8,000(7,4);7,995(7,7);7,981(8,6);7,976(8,0);7,566(8,1);7,554(7,9);7,547(7,7);7,535(7,5);7,286(0,7);7,262(0,7);7,239(0,3);6,976(13,8);6, 969(13,8);3,594(0,3);3,589(0,3);3,578(0,4);3,533(0,6);3,518(0,4);3,509(0,5);3,496(0,4);3,486(0,5);3,474(0,8);3,456(0,8);3,439(1,1);3,420(1, 9);3,409(3,2);3,350(2513,1);3,284(1,5);3,268(1,5);3,253(0,9);3,248(0,9);3,238(1,0);3,219(0,6);3,207(0,7);3,199(0,5);3,186(0,4);3,179(0,4);3, 153(0,3);3,143(0,4);2,677(2,0);2,672(2,8);2,668(1,9);2,663(0,9);2,543(3,1);2,536(0,9);2,526(6,3);2,521(10,8);2,512(160,9);2,508(328,4);2,5 |
| 03(431,2);2,499(304,0);2,494(141,3);2,339(1,0);2,335(2,0);2,330(2,7);2,325(1,9);2,321(0,9);2,073(15,0);2,047(0,4);1,654(0,9);0,000(8,0) |
| I-1-432: |
| HPLC-MS: logP = 2,91; Masse (m/z): 366,9 (M+H)⁺; ¹H-NMR(400,0 MHz, DMSO-D₆): |
| δ= 11,326(4,6);8,618(5,2);8,613(5,6);8,508(5,7);8,503(5,1);8,266(5,0);8,259(4,9);7,582(2,2);7,578(2,3);7,563(3,0);7,559(3,1);7,551(1,8);7,5 48(2,0);7,531(3,8);7,528(3,9);7,511(1,8);7,506(1,8);7,492(3,1);7,488(2,7);7,473(1,7);7,468(1,4);7,448(2,4);7,444(2,3);7,429(3,0);7,426(2,8); 7,411(1,2);7,408(1,1);6,979(5,3);6,972(5,2);5,756(16,0);3,326(8,0);2,512(6,9);2,507(13,8);2,503(18,0);2,498(12,9);2,494(6,1);1,396(0,4);0,0 08(1,0);0,000(24,2);-0,009(0,9) |
| I-1-433: |
| HPLC-MS: logP = 3,06; Masse (m/z): 458,9 (M+H)⁺; ¹H-NMR(400,0 MHz, DMSO-D₆): |
| δ= 11,247(3,0);8,618(3,3);8,612(3,5);8,508(3,6);8,502(3,2);8,264(3,2);8,257(3,1);7,922(2,2);7,902(2,5);7,479(2,4);7,475(2,8);7,466(4,9);7,4 56(0,5);7,232(1,0);7,223(1,0);7,218(1,0);7,212(1,1);7,209(1,1);7,203(0,9);7,199(1,0);7,189(0,8);6,973(3,3);6,967(3,2);5,754(16,0);3,324(11, 4);2,511(6,1);2,506(11,9);2,502(15,2);2,497(10,7);2,493(4,9);1,397(1,4);0,008(1,0);0,000(20,8);-0,009(0,7) |
| I-1-434: |
| HPLC-MS: logP = 2,61; Masse (m/z): 387,1 (M+H)⁺; ¹H-NMR(400,0 MHz, DMSO-D₆): |
| δ= 20,006(0,8);11,548(11,0);8,484(10,9);8,478(11,9);8,361(9,9);8,354(10,2);8,285(3,2);8,279(3,0);8,264(3,5);8,258(6,2);8,252(3,5);8,237(3, 5);8,231(3,2);7,851(1,7);7,831(3,9);7,817(4,0);7,797(2,6);7,636(3,6);7,608(4,1);7,586(3,1);7,489(7,2);7,470(6,6);6,958(13,5);6,951(13,6);4,5 93(0,7);4,578(1,4);4,565(0,9);3,511(16,0);3,491(1,8);3,477(2,2);3,465(1,1);3,423(3,0);3,411(3,4);3,399(2,7);3,331(4647,8);2,711(1,1);2,675( 9,2);2,671(12,2);2,667(8,9);2,541(137,4);2,524(38,2);2,511(696,8);2,506(1362,8);2,502(1781,8);2,497(1302,9);2,493(634,2);2,367(0,8);2,33 3(8,5);2,329(11,6);2,324(8,2);2,289(0,7);2,074(5,0);1,336(2,7);1,298(1,3);1,259(1,9);1,249(3,5);1,235(6,8);1,148(1,2);0,907(0,6);0,888(1,2); 0,869(0,7);0,854(0,7);0,000(33,3) |
| I-1-435: |
| HPLC-MS: logP = 1,83; Masse (m/z): 431,0 (M+H)⁺; ¹H-NMR(400,0 MHz, DMSO-D₆): |
| δ= 10,817(2,9);8,475(2,7);8,469(2,9);8,429(5,8);8,315(2,3);8,310(2,2);8,308(2,2);8,278(0,9);8,272(0,8);8,257(0,9);8,251(1,6);8,245(0,8);8,2 30(0,9);8,224(0,8);6,968(3,8);6,961(3,8);3,895(16,0);3,513(0,5);3,336(50,5);2,544(5,8);2,527(0,3);2,522(0,5);2,514(6,8);2,509(13,9);2,504(1 8,6);2,500(13,6);2,495(6,5);0,000(1,0) |
| I-1-436: |
| HPLC-MS: logP = 2,61; Masse (m/z): 387,1 (M+H)⁺; ¹H-NMR(400,0 MHz, DMSO-D₆): |
| δ= 11,486(13,3);8,482(11,5);8,476(12,0);8,357(11,1);8,351(10,9);8,283(3,2);8,277(3,1);8,262(3,6);8,255(6,0);8,249(3,4);8,234(3,3);8,228(3, 0);7,810(4,0);7,797(4,6);7,789(5,8);7,779(6,6);7,775(9,9);7,757(5,3);7,751(5,4);7,671(3,3);7,665(3,0);7,650(5,7);7,644(5,0);7,629(2,6);7,622 (2,2);6,978(16,0);6,971(15,7);3,516(1,3);3,339(118,8);3,000(0,8);2,715(0,6);2,575(0,4);2,570(0,5);2,545(152,8);2,528(1,3);2,523(1,9);2,515( 17,8);2,510(35,2);2,506(46,8);2,501(34,4);2,496(16,7);2,371(0,6);2,078(0,4);1,233(0,3);0,000(2,9) |
| I-1-437: |
| HPLC-MS: logP = 2,93; Masse (m/z): 403,1 (M+H)⁺; ¹H-NMR(400,0 MHz, DMSO-D₆): |
| δ= 11,536(12,7);8,484(12,3);8,478(12,9);8,362(11,1);8,355(11,1);8,285(3,6);8,279(3,3);8,264(3,8);8,258(6,7);8,252(3,5);8,237(3,6);8,231(3, 3);7,872(8,8);7,864(10,0);7,860(11,0);7,851(13,5);7,790(6,7);7,787(6,2);7,768(3,9);7,765(3,8);6,971(16,0);6,964(15,9);3,514(1,5);3,365(0,9) ;3,338(229,4);2,998(0,4);2,714(0,4);2,678(0,4);2,673(0,5);2,669(0,4);2,563(0,4);2,544(105,3);2,532(0,7);2,527(1,6);2,522(2,3);2,513(29,3);2 ,509(58,9);2,504(78,3);2,500(57,2);2,495(27,6);2,370(0,4);2,336(0,4);2,331(0,5);2,326(0,4);1,233(0,4);0,000(3,8) |
| I-1-438: siehe Synthesebeispiel 48 |
| I-1-439: |
| HPLC-MS: logP = 2,85; Masse (m/z): 383,1 (M+H)⁺; ¹H-NMR(400,0 MHz, DMSO-D₆): |
| δ= 11,442(7,7);8,316(6,4);8,310(5,9);8,179(3,5);8,157(3,9);8,153(3,9);8,131(3,5);7,832(4,0);7,812(4,9);7,779(1,4);7,762(4,0);7,743(3,7);7,7 09(3,2);7,689(9,0);7,669(4,4);6,964(9,3);6,958(9,2);3,322(182,2);2,680(0,5);2,675(1,0);2,671(1,5);2,666(1,0);2,662(0,5);2,524(4,0);2,519(6, 2);2,511(76,9);2,506(156,5);2,502(210,7);2,497(154,4);2,492(73,5);2,464(16,0);2,458(15,4);2,456(15,4);2,337(0,5);2,333(1,1);2,328(1,4);2,3 24(1,0);2,319(0,5);1,989(0,6);1,398(1,0);1,235(0,4);1,175(0,3);0,008(0,5);0,000(17,4);-0,009(0,5) |
| I-1-440: |
| HPLC-MS: logP = 2,38; Masse (m/z): 384,1 (M+H)⁺; ¹H-NMR(400,0 MHz, DMSO-D₆): |
| δ= 11,623(7,4);8,844(3,9);8,834(3,8);8,832(3,8);8,343(6,0);8,337(5,9);8,209(3,5);8,206(3,7);8,187(7,3);8,165(3,8);8,160(3,8);8,139(3,4);7,8 35(3,6);7,824(3,5);7,816(3,3);7,804(3,2);6,969(9,5);6,963(9,4);5,758(2,2);3,322(233,2);2,680(1,0);2,675(2,2);2,670(3,0);2,666(2,2);2,661(1, 0);2,524(8,5);2,519(13,4);2,510(166,2);2,506(336,3);2,501(444,8);2,497(318,7);2,492(149,9);2,467(14,8);2,465(16,0);2,459(15,4);2,457(15, 1);2,337(1,0);2,333(2,2);2,328(3,0);2,324(2,2);2,319(1,0);1,146(0,3);0,146(0,9);0,008(7,8);0,000(238,1);-0,009(7,2);-0,150(0,9) |
| I-1-441: |
| HPLC-MS: logP = 2,56; Masse (m/z): 351,0 (M+H)⁺; ¹H-NMR(400,0 MHz, DMSO-D₆): |
| δ= 11,671(7,3);8,340(6,1);8,333(6,0);8,316(0,6);8,185(3,4);8,163(3,9);8,159(3,8);8,137(3,4);7,607(0,9);7,590(2,1);7,586(1,8);7,573(1,5);7,5 69(3,8);7,552(1,9);7,548(2,3);7,531(1,0);7,242(1,3);7,235(6,7);7,215(9,4);7,194(5,5);7,187(1,1);6,971(8,9);6,964(8,8);5,756(0,9);3,322(178, 1);2,675(1,3);2,671(1,8);2,666(1,3);2,524(6,0);2,511(96,3);2,506(188,7);2,502(249,0);2,497(183,7);2,493(89,7);2,465(16,0);2,460(15,4);2,45 8(15,1);2,337(0,6);2,333(1,2);2,328(1,7);2,324(1,2);2,319(0,6);1,336(0,7);1,298(0,5);1,259(0,7);1,250(0,8);1,235(0,5);0,000(3,9) |
| I-1-442: |
| HPLC-MS: logP = 2,71; Masse (m/z): 395,0 (M+H)⁺; ¹H-NMR(400,0 MHz, DMSO-D₆): |
| δ= 11,339(7,6);8,315(6,0);8,308(5,8);8,178(3,2);8,156(3,7);8,152(3,8);8,130(3,2);7,699(4,5);7,697(4,5);7,679(5,3);7,677(5,2);7,545(2,9);7,5 |
| 40(3,3);7,526(5,2);7,521(5,3);7,484(2,5);7,482(2,7);7,466(5,1);7,463(5,0);7,447(2,8);7,444(2,6);7,420(3,2);7,415(3,3);7,400(4,0);7,396(3,9); 7,382(1,8);7,377(1,6);6,984(7,9);6,977(7,8);5,756(1,9);3,322(145,3);2,675(1,0);2,671(1,4);2,666(1,1);2,524(4,2);2,510(81,1);2,506(158,6);2, 502(209,1);2,497(155,2);2,493(77,1);2,464(16,0);2,458(15,8);2,333(1,1);2,328(1,4);2,324(1,1);1,336(0,4);1,298(0,3);1,259(0,5);1,250(0,4);1 ,235(0,4);0,000(2,9) |
| I-1-443: |
| HPLC-MS: logP = 2,16; Masse (m/z): 341,1 (M+H)⁺; ¹H-NMR(400,0 MHz, DMSO-D₆): |
| δ= 11,676(12,3);8,520(0,6);8,514(0,8);8,499(13,2);8,493(13,8);8,483(0,8);8,463(4,6);8,378(10,6);8,376(10,8);8,371(10,8);8,370(10,4);8,316( 10,5);8,309(5,4);8,303(4,5);8,288(5,0);8,282(8,8);8,276(4,6);8,261(4,8);8,255(4,4);8,170(5,0);7,939(11,2);7,936(11,0);7,458(10,6);7,456(10, 7);7,016(0,8);7,009(0,8);6,990(16,0);6,983(15,8);6,812(0,7);5,757(0,9);4,038(0,7);4,020(0,7);3,324(113,5);2,681(0,4);2,676(0,8);2,672(1,2); 2,667(0,8);2,663(0,4);2,525(3,4);2,520(5,4);2,512(64,3);2,507(128,7);2,503(170,4);2,498(123,2);2,494(58,3);2,431(1,9);2,338(0,4);2,334(0,8 );2,329(1,2);2,325(0,8);2,320(0,4);1,989(3,2);1,397(0,6);1,336(1,3);1,299(0,5);1,259(0,7);1,250(1,7);1,234(0,5);1,193(0,9);1,175(1,7);1,157( 0,9);0,008(1,8);0,000(53,2);-0,009(1,7) |
| I-1-444: |
| HPLC-MS: logP = 2,65; Masse (m/z): 373,0 (M+H)⁺; ¹H-NMR(400,0 MHz, DMSO-D₆): |
| δ= 11,594(2,5);8,495(3,7);8,489(3,9);8,382(3,1);8,377(3,1);8,316(1,1);8,307(1,3);8,301(1,2);8,286(1,4);8,280(2,5);8,274(1,3);8,259(1,3);8,2 53(1,2);8,019(1,3);7,874(3,2);7,730(1,6);6,951(2,4);6,944(2,4);3,322(526,6);2,680(1,1);2,675(2,3);2,670(3,2);2,666(2,3);2,661(1,0);2,541(11 ,1);2,524(8,2);2,519(12,8);2,511(177,2);2,506(361,6);2,501(478,3);2,497(338,8);2,492(156,9);2,337(1,1);2,333(2,3);2,328(3,1);2,324(2,2);2, 319(1,0);2,280(16,0);1,259(0,4);1,235(1,0);0,008(1,6);0,000(51,8);-0,009(1,5) |
| I-1-445: |
| HPLC-MS: logP = 1,60; Masse (m/z): 333,2 (M+H)⁺; ¹H-NMR(400,0 MHz, CD₃CN): |
| δ= 8,526(0,7);8,269(2,1);8,263(2,1);8,170(1,7);8,164(1,7);7,699(0,7);7,693(0,7);7,679(0,8);7,673(1,3);7,667(0,7);7,652(0,7);7,646(0,7);7,63 6(0,3);6,991(2,4);6,985(2,3);5,448(1,1);3,685(15,2);3,671(0,3);3,233(0,9);3,030(0,9);2,469(0,4);2,464(0,5);2,459(0,4);2,428(15,9);2,357(16, 0);2,174(55,4);2,120(0,3);2,114(0,5);2,108(0,6);2,102(0,4);1,965(5,5);1,959(8,4);1,953(33,9);1,947(58,3);1,940(75,4);1,934(51,7);1,928(26, 6);1,842(0,3);1,775(0,3);1,769(0,4);0,008(1,5);0,000(34,5);-0,009(1,3) |
| I-1-446: |
| HPLC-MS: logP = 1,54; Masse (m/z): 319,1 (M+H)⁺; ¹H-NMR(400,0 MHz, DMSO-D₆): |
| δ= 10,654(2,6);8,470(2,5);8,464(2,6);8,445(4,6);8,292(2,0);8,285(2,0);8,275(0,8);8,269(0,8);8,254(0,9);8,248(1,5);8,241(0,8);8,227(0,8);8,2 20(0,7);6,969(3,2);6,963(3,2);5,757(0,5);3,792(15,2);3,323(75,2);2,675(0,5);2,671(0,7);2,666(0,5);2,524(1,8);2,519(2,8);2,511(38,3);2,506(7 7,4);2,502(102,8);2,497(74,5);2,493(35,6);2,363(16,0);2,333(0,5);2,328(0,7);2,324(0,5);2,075(0,4);0,008(1,4);0,000(44,6);-0,009(1,4) |
| I-1-447: |
| HPLC-MS: logP = 2,03; Masse (m/z): 364,1 (M+H)⁺; ¹H-NMR(400,0 MHz, DMSO-D₆): |
| δ= 11,987(2,1);8,493(2,3);8,487(2,4);8,410(2,1);8,404(2,1);8,315(0,7);8,298(0,7);8,292(0,6);8,277(0,8);8,271(1,3);8,265(0,7);8,250(0,7);8,2 44(0,7);7,021(2,9);7,014(2,9);3,845(16,0);3,371(0,4);3,325(432,6);2,995(1,0);2,680(0,6);2,675(1,3);2,671(1,7);2,666(1,3);2,662(0,6);2,541(5 9,5);2,524(5,1);2,511(97,8);2,506(197,2);2,502(259,9);2,497(187,0);2,493(89,1);2,449(14,3);2,338(0,6);2,333(1,2);2,328(1,7);2,324(1,2);2,3 19(0,6);1,235(1,1);0,008(0,6);0,000(15,6);-0,009(0,5) |
| I-1-448: |
| HPLC-MS: logP = 1,83; Masse (m/z): 364,1 (M+H)⁺; ¹H-NMR(400,0 MHz, DMSO-D₆): |
| δ= 11,480(2,8);8,481(2,8);8,475(3,0);8,360(2,5);8,353(2,5);8,316(0,4);8,284(0,9);8,278(0,8);8,263(1,0);8,257(1,6);8,250(0,9);8,235(0,9);8,2 29(0,8);6,973(3,3);6,967(3,2);3,870(16,0);3,323(93,8);2,675(0,7);2,671(0,9);2,666(0,7);2,610(15,4);2,541(0,5);2,524(2,6);2,511(55,6);2,506( 109,1);2,502(140,4);2,497(100,3);2,493(48,4);2,333(0,7);2,329(0,9);2,324(0,7);0,146(0,4);0,008(3,9);0,000(94,2);-0,008(3,9);-0,150(0,4) |
| I-1-449: |
| HPLC-MS: logP = 1,67; Masse (m/z): 306,1 (M+H)⁺; ¹H-NMR(601,6 MHz, DMF): |
| δ= 10,909(0,4);8,499(1,9);8,495(1,9);8,453(1,9);8,4493(1,8);8,4488(1,8);8,261(0,7);8,257(0,6);8,248(0,7);8,243(1,3);8,239(0,6);8,230(0,7);8 ,225(0,6);8,131(4,4);8,025(4,3);7,074(2,1);7,069(2,0);5,817(0,4);4,286(16,0);3,471(4,4);2,952(4,4);2,922(2,2);2,919(4,3);2,916(6,3);2,912(4, 3);2,909(2,0);2,784(4,2);2,751(2,5);2,748(4,8);2,745(6,8);2,741(4,8);2,738(2,4);2,616(0,7);0,005(0,4);0,000(10,9) |
| I-1-450: |
| HPLC-MS: logP = 3,65; Masse (m/z): 491,1 (M+H)⁺; ¹H-NMR(400,0 MHz, DMSO-D₆): |
| δ= 12,141(3,6);8,498(3,6);8,492(3,8);8,420(3,2);8,413(3,2);8,315(1,1);8,307(1,2);8,300(1,2);8,286(1,3);8,279(2,3);8,273(1,1);8,258(1,2);8,2 52(1,1);6,990(4,6);6,983(4,5);4,002(16,0);3,920(0,4);3,414(0,4);3,392(0,5);3,387(0,5);3,374(0,8);3,359(1,4);3,326(1280,7);3,296(1,1);3,273( 0,4);2,711(0,4);2,680(1,2);2,675(2,4);2,671(3,4);2,666(2,4);2,662(1,1);2,541(83,9);2,524(8,4);2,519(13,1);2,511(187,5);2,506(384,4);2,502( 509,9);2,497(363,2);2,493(169,8);2,367(0,3);2,338(1,1);2,333(2,4);2,328(3,3);2,324(2,3);2,319(1,1);2,289(0,6);1,258(0,4);1,235(1,1);0,008( 0,6);0,000(18,6);-0,009(0,6) |
| I-1-451: |
| HPLC-MS: logP = 2,79; Masse (m/z): 441,0 (M+H)⁺; ¹H-NMR(400,0 MHz, DMSO-D₆): |
| δ= 11,728(4,9);8,484(4,7);8,478(5,0);8,364(4,3);8,357(4,3);8,292(1,5);8,286(1,3);8,271(1,6);8,265(2,7);8,258(1,4);8,243(1,5);8,237(1,3);6,9 30(6,0);6,923(5,9);5,758(3,3);4,127(16,0);3,323(109,4);2,680(0,4);2,675(0,9);2,671(1,2);2,666(0,9);2,662(0,4);2,524(3,5);2,511(66,0);2,506( 130,7);2,502(170,6);2,497(122,5);2,493(58,0);2,333(0,8);2,329(1,2);2,324(0,8);2,320(0,4);0,008(0,9);0,000(25,5);-0,009(0,8) |
| I-1-452: |
| HPLC-MS: logP = 2,27; Masse (m/z): 349,1 (M+H)⁺; ¹H-NMR(400,0 MHz, DMSO-D₆): |
| δ= 11,199(3,1);8,487(2,8);8,480(3,0);8,335(2,6);8,329(2,5);8,317(0,9);8,297(0,9);8,291(0,8);8,270(1,5);8,249(1,1);8,238(3,1);8,234(3,1);7,8 90(3,6);7,885(3,5);6,955(3,2);6,948(3,2);3,977(16,0);3,403(0,4);3,325(350,6);2,670(3,1);2,506(393,9);2,502(484,1);2,498(358,1);2,445(0,5); 2,421(0,4);2,412(0,4);2,332(2,3);2,328(2,9);1,988(0,4);1,259(0,5);1,235(0,8);0,146(0,5);0,000(90,0);-0,150(0,4) |
| I-1-453: |
| HPLC-MS: logP = 1,69; Masse (m/z): 350,1 (M+H)⁺; ¹H-NMR(400,0 MHz, DMSO-D₆): |
| δ= 11,534(2,8);8,891(5,4);8,482(2,8);8,476(2,9);8,371(2,5);8,364(2,5);8,316(0,9);8,286(0,8);8,280(0,7);8,264(0,9);8,258(1,6);8,252(0,8);8,2 37(0,8);8,231(0,8);6,985(3,2);6,978(3,1);5,756(1,2);3,947(16,0);3,324(384,8);2,675(1,9);2,671(2,5);2,666(1,8);2,541(1,6);2,510(151,1);2,50 6(293,3);2,502(379,5);2,497(271,7);2,493(130,3);2,333(1,8);2,328(2,4);2,324(1,8);0,146(1,3);0,008(11,8);0,000(287,0);-0,008(10,7);-0,150(1,3) |
| I-1-454: |
| HPLC-MS: logP = 1,58; Masse (m/z): 350,0 (M+H)⁺; ¹H-NMR(400,0 MHz, DMSO-D₆): |
| δ= 11,335(3,1);8,480(2,8);8,474(3,1);8,365(2,7);8,359(2,7);8,316(0,4);8,284(0,9);8,277(0,8);8,262(1,0);8,256(1,7);8,250(0,9);8,235(0,9);8,2 29(0,8);8,085(5,0);6,984(3,3);6,977(3,3);3,930(16,0);3,324(123,4);2,675(0,7);2,671(0,9);2,666(0,7);2,541(0,5);2,524(2,2);2,506(108,4);2,50 2(142,0);2,498(103,9);2,333(0,7);2,329(0,9);2,324(0,7);0,146(0,4);0,008(3,1);0,000(89,8);-0,008(3,8);-0,150(0,4) |
| I-1-455: |
| HPLC-MS: logP = 3,37; Masse (m/z): 381,1 (M+H)⁺; ¹H-NMR(400,0 MHz, DMSO-D₆): |
| δ= 11,542(4,1);8,491(5,3);8,485(5,6);8,368(4,2);8,361(4,2);8,315(1,2);8,300(1,6);8,293(1,5);8,279(1,8);8,272(3,0);8,266(1,6);8,251(1,6);8,2 45(1,4);7,689(8,6);6,987(3,7);6,981(3,7);4,537(0,8);4,522(1,3);4,500(1,3);4,485(0,9);3,458(0,3);3,440(0,4);3,419(0,7);3,395(0,9);3,328(1742 ,5);3,284(1,2);3,255(0,4);2,995(0,8);2,675(3,3);2,671(4,4);2,667(3,2);2,541(14,1);2,524(12,6);2,511(266,7);2,506(516,9);2,502(665,9);2,498 (479,9);2,493(233,4);2,333(3,2);2,329(4,3);2,324(3,1);2,290(0,5);1,875(0,8);1,856(1,1);1,840(1,5);1,821(1,6);1,800(1,3);1,781(0,4);1,765(0, 3);1,746(1,2);1,732(1,5);1,728(1,4);1,714(1,8);1,698(1,0);1,680(0,7);1,421(12,4);1,405(12,3);1,298(0,5);1,259(0,7);1,235(2,1);0,726(7,4);0,7 08(16,0);0,689(6,9);0,008(0,8);0,000(20,1);-0,008(0,7) |
| I-1-456: |
| HPLC-MS: logP = 3,28; Masse (m/z): 473,0 (M+H)⁺; ¹H-NMR(400,0 MHz, DMSO-D₆): |
| δ= 11,474(5,2);8,492(5,7);8,486(6,0);8,364(4,3);8,358(4,3);8,315(1,7);8,297(1,8);8,291(1,6);8,276(1,9);8,269(3,3);8,263(1,8);8,248(1,8);8,2 42(1,6);7,630(11,0);6,989(4,7);6,983(4,7);4,461(1,0);4,445(1,4);4,424(1,4);4,409(1,0);4,394(0,4);3,479(0,4);3,466(0,3);3,443(0,6);3,431(0,5) ;3,417(0,7);3,408(0,8);3,388(1,3);3,328(2368,1);3,293(1,9);3,280(1,2);3,253(0,5);2,676(3,9);2,671(5,3);2,666(3,9);2,662(1,9);2,541(29,9);2, 524(13,6);2,511(303,8);2,506(613,8);2,502(810,3);2,497(583,1);2,493(277,8);2,419(0,3);2,333(3,8);2,329(5,2);2,324(3,8);2,290(0,7);1,868(0 ,8);1,849(1,1);1,833(1,5);1,812(1,6);1,793(1,3);1,775(0,4);1,733(1,2);1,719(1,5);1,715(1,5);1,701(1,9);1,682(1,1);1,667(0,8);1,406(12,4);1,3 90(12,3);1,355(0,4);1,298(0,7);1,259(1,0);1,235(3,1);0,854(0,4);0,721(7,4);0,702(16,0);0,684(7,0);0,008(0,6);0,000(20,3);-0,009(0,6) |
| I-1-457: |
| HPLC-MS: logP = 2,13; Masse (m/z): 383,1 (M+H)⁺; ¹H-NMR(400,0 MHz, DMSO-D₆): |
| δ= 20,013(0,3);11,562(12,8);8,707(9,4);8,703(10,5);8,695(10,5);8,691(10,4);8,489(14,9);8,483(16,0);8,364(11,6);8,358(11,9);8,292(5,0);8,2 86(4,6);8,271(5,3);8,265(9,4);8,258(5,0);8,244(5,1);8,237(4,7);8,096(7,5);8,091(7,9);8,076(8,5);8,072(8,3);7,544(8,5);7,532(8,6);7,525(8,4); 7,513(8,1);7,011(14,4);7,004(14,6);4,137(4,1);4,109(12,8);4,080(13,4);4,052(4,6);3,473(0,5);3,442(0,7);3,337(3631,0);3,211(1,0);3,147(0,6) ;2,995(0,6);2,681(2,5);2,676(5,2);2,671(7,2);2,667(5,2);2,662(2,5);2,542(42,8);2,525(22,7);2,520(34,8);2,511(399,6);2,507(806,5);2,502(106 5,4);2,498(765,9);2,493(362,6);2,338(2,4);2,334(5,0);2,329(6,9);2,324(4,9);2,320(2,3);2,291(0,4);1,258(0,3);1,235(0,7);1,147(0,7);0,008(2,2 );0,000(64,0);-0,009(1,7) |
| I-1-458: |
| HPLC-MS: logP = 2,53; Masse (m/z): 367,1 (M+H)⁺; ¹H-NMR(400,0 MHz, DMSO-D₆): |
| δ= 11,161(13,3);8,480(14,0);8,474(14,8);8,344(11,6);8,337(11,6);8,316(0,8);8,280(4,5);8,274(4,2);8,259(4,8);8,253(8,4);8,247(4,5);8,232(4, 5);8,226(4,1);7,636(7,0);7,632(8,1);7,617(8,2);7,613(9,0);7,585(4,2);7,581(3,9);7,565(7,4);7,562(6,9);7,546(5,9);7,541(5,1);7,386(7,7);7,364 (6,4);7,362(6,7);7,345(10,8);7,343(11,1);7,326(4,9);7,324(4,9);7,277(9,6);7,257(8,4);7,202(16,0);7,017(8,1);6,997(14,8);6,990(14,6);5,756(1 ,7);3,323(236,9);2,680(0,9);2,675(1,8);2,671(2,5);2,666(1,8);2,662(0,9);2,524(6,9);2,519(10,7);2,511(137,6);2,506(279,5);2,502(370,1);2,49 7(267,1);2,493(127,5);2,338(0,8);2,333(1,8);2,328(2,4);2,324(1,7);2,319(0,8);1,989(1,1);1,298(1,3);1,259(1,9);1,235(1,1);1,193(0,3);1,175(0 ,6);0,146(0,4);0,008(3,2);0,000(99,9);-0,009(3,1);-0,150(0,4) |
| I-1-459: |
| HPLC-MS: logP = 2,50; Masse (m/z): 351,1 (M+H)⁺; ¹H-NMR(400,0 MHz, DMSO-D₆): |
| δ= 11,485(11,9);8,495(15,5);8,489(16,0);8,361(11,9);8,355(11,7);8,296(4,6);8,290(4,2);8,275(5,0);8,269(8,5);8,263(4,5);8,248(4,6);8,242(4, 1);7,794(5,5);7,776(7,5);7,762(6,3);7,744(9,7);7,709(3,3);7,706(3,7);7,691(8,9);7,687(7,7);7,673(12,4);7,669(10,6);7,654(6,4);7,635(2,0);7,4 93(5,4);7,354(11,7);7,215(5,8);7,008(11,8);7,002(11,5);3,508(0,5);3,496(0,3);3,474(0,5);3,457(0,5);3,439(0,8);3,410(1,3);3,345(1104,0);3,3 04(1,9);3,288(1,0);3,270(0,4);3,223(0,3);3,001(1,9);2,718(1,4);2,682(1,8);2,677(2,3);2,673(1,7);2,610(0,3);2,607(0,4);2,548(321,5);2,517(14 6,6);2,513(274,7);2,508(346,5);2,504(247,4);2,500(118,6);2,467(0,7);2,374(1,3);2,340(1,6);2,335(2,2);2,330(1,5);2,298(0,3);1,250(0,4);1,24 0(0,9);0,006(0,3) |
| I-1-460: |
| HPLC-MS: logP = 1,77; Masse (m/z): 352,0 (M+H)⁺; ¹H-NMR(400,0 MHz, DMSO-D₆): |
| δ= 11,621(14,7);8,811(8,5);8,808(8,9);8,799(9,1);8,796(8,6);8,490(14,6);8,484(15,3);8,373(13,0);8,367(12,9);8,316(1,7);8,293(4,4);8,287(4, 1);8,272(4,8);8,266(8,4);8,260(4,4);8,245(4,5);8,239(4,1);8,193(7,7);8,174(8,2);7,703(6,1);7,691(6,3);7,683(6,0);7,671(5,6);7,333(6,4);7,198 (14,3);7,063(7,0);7,011(16,0);7,005(15,8);4,056(0,4);4,038(1,0);4,020(1,1);4,002(0,3);3,321(311,5);3,175(0,5);3,161(0,5);2,675(4,2);2,671(5 ,7);2,666(4,1);2,662(2,0);2,541(3,4);2,524(14,6);2,510(316,6);2,506(634,8);2,502(831,9);2,497(594,2);2,493(281,7);2,337(1,9);2,333(4,0);2, 328(5,5);2,324(4,0);1,989(4,4);1,235(0,6);1,193(1,2);1,175(2,3);1,157(1,2);0,146(3,1);0,008(23,1);0,000(669,5);-0,009(22,8);-0,150(3,1) |
| I-1-461: |
| HPLC-MS: logP = 2,14; Masse (m/z): 348,0 (M+H)⁺; ¹H-NMR(400,0 MHz, DMSO-D₆): |
| δ= 11,368(2,1);8,583(1,5);8,579(1,7);8,571(1,7);8,567(1,7);8,486(2,5);8,480(2,7);8,354(2,2);8,348(2,2);8,290(0,7);8,284(0,7);8,269(0,8);8,2 63(1,4);8,256(0,8);8,242(0,7);8,235(0,7);7,980(1,3);7,976(1,4);7,961(1,5);7,957(1,5);7,236(1,5);7,224(1,6);7,217(1,6);7,205(1,5);6,996(1,5); 6,991(1,5);3,328(72,2);3,175(0,9);3,162(0,9);2,671(0,4);2,506(46,3);2,502(61,4);2,498(46,5);2,464(16,0);2,329(0,4);0,000(1,4) |
| I-1-462: |
| HPLC-MS: logP = 1,69; Masse (m/z): 379,1 (M+H)⁺; ¹H-NMR(400,0 MHz, CD₃CN): |
| δ= 8,264(0,7);8,258(0,7);8,214(0,6);8,208(0,6);8,061(0,3);8,058(0,5);8,041(0,4);8,038(0,5);7,767(0,5);7,749(0,5);7,745(0,4);7,726(0,3);7,72 2(0,5);7,703(1,2);7,687(0,6);7,684(0,6);7,681(0,6);7,661(0,5);7,011(0,7);7,004(0,7);3,297(5,6);2,502(16,0);2,156(1,4);1,964(0,7);1,958(1,0); 1,952(4,2);1,946(7,4);1,940(9,6);1,934(6,6);1,927(3,4);0,000(4,6) |
| I-1-463: |
| HPLC-MS: logP = 2,77; Masse (m/z): 385,1 (M+H)⁺; ¹H-NMR(400,0 MHz, DMSO-D₆): |
| δ= 11,367(13,5);8,483(14,3);8,477(15,1);8,348(12,1);8,342(12,1);8,317(0,8);8,285(4,4);8,279(4,1);8,264(4,8);8,258(8,2);8,252(4,4);8,237(4, 5);8,231(4,0);7,982(0,3);7,716(7,2);7,712(8,2);7,697(8,8);7,693(9,5);7,660(3,9);7,656(3,8);7,640(8,0);7,636(7,5);7,620(6,7);7,616(5,9);7,520 (7,3);7,501(11,6);7,485(11,6);7,465(6,1);7,447(0,4);6,986(15,1);6,980(14,9);5,757(16,0);4,038(0,5);4,020(0,5);3,323(175,0);2,680(1,0);2,67 5(2,1);2,671(2,8);2,667(2,1);2,662(1,0);2,524(8,4);2,511(154,2);2,506(305,6);2,502(399,3);2,497(287,5);2,493(137,6);2,338(0,9);2,333(1,9); 2,329(2,6);2,324(1,9);2,320(0,9);1,989(2,0);1,352(0,3);1,336(2,5);1,298(0,8);1,259(1,2);1,250(3,1);1,235(1,0);1,193(0,6);1,175(1,1);1,157(0, 6);1,148(0,3);1,141(0,3);1,124(0,6);1,001(0,6);0,146(1,0);0,008(8,1);0,000(221,7);-0,009(7,2);-0,150(0,9) |
| I-1-464: |
| HPLC-MS: logP = 2,53; Masse (m/z): 386,1 (M+H)⁺; ¹H-NMR(400,0 MHz, DMSO-D₆): |
| δ= 11,554(10,5);8,494(11,0);8,487(16,0);8,482(7,5);8,474(6,9);8,470(6,7);8,380(9,3);8,374(9,1);8,297(3,3);8,291(3,0);8,276(3,5);8,270(6,0); 8,263(3,2);8,248(3,4);8,242(3,3);8,234(6,8);8,229(6,7);8,215(7,3);8,210(6,7);7,560(6,9);7,547(6,8);7,541(6,7);7,528(6,4);7,007(12,3);7,001( 12,0);3,381(0,5);3,365(1,3);3,338(403,0);3,306(0,6);3,299(0,4);2,687(0,4);2,682(0,8);2,678(1,1);2,673(0,8);2,669(0,4);2,548(28,7);2,531(3,5 );2,526(5,7);2,518(63,8);2,513(126,2);2,509(164,6);2,504(117,3);2,499(55,3);2,344(0,4);2,340(0,8);2,335(1,1);2,331(0,7);2,326(0,4);1,240(0 ,5) |
| I-1-465: |
| HPLC-MS: logP = 2,44; Masse (m/z): 387,0 (M+H)⁺; ¹H-NMR(400,0 MHz, CD₃CN): |
| δ= 8,840(3,8);8,829(4,9);8,791(0,6);8,282(9,8);8,276(11,9);8,270(10,6);8,258(7,4);8,212(1,0);8,206(1,0);8,181(0,3);8,061(0,8);8,054(0,7);7, 846(5,7);7,828(8,0);7,807(1,8);7,786(1,3);7,776(2,1);7,757(6,9);7,740(16,0);7,718(10,1);7,697(12,4);7,677(6,4);7,671(5,2);7,648(2,1);7,622( 1,4);7,603(1,3);7,584(0,6);7,570(1,3);7,550(0,9);7,520(0,7);7,502(0,7);7,493(0,4);7,482(0,4);7,476(0,3);7,462(0,3);7,455(0,5);7,019(1,1);7,0 12(1,0);6,248(0,4);6,235(0,3);6,219(0,5);6,206(0,3);6,161(0,4);6,152(0,4);5,869(0,3);5,459(0,3);5,448(0,3);4,193(0,3);2,619(0,4);2,569(0,5); 2,466(0,9);2,352(1,3);2,315(2,0);2,309(1,8);2,270(1,7);2,241(1,7);2,179(1,5);2,132(1,3);2,126(1,2);2,119(1,7);2,113(2,3);2,107(2,7);2,101(2, 1);2,095(1,4);2,086(0,9);1,964(52,9);1,958(32,2);1,952(140,9);1,946(247,6);1,940(322,7);1,933(221,8);1,927(114,2);1,915(3,4);1,900(1,8);1, 843(0,8);1,792(0,7);1,786(0,6);1,780(1,2);1,774(1,8);1,768(2,3);1,762(1,6);1,756(1,0);1,730(0,4);1,718(0,4);1,705(0,4);1,340(0,5);1,295(0,4) ;1,270(1,3);1,130(0,6);1,064(0,4);0,888(0,4);0,881(0,4);0,871(0,4);0,866(0,4);0,856(0,4);0,146(0,8);0,008(6,8);0,000(188,6);-0,009(6,5);-0,150(0,9) |
| I-1-466: |
| HPLC-MS: logP = 3,18; Masse (m/z): 385,1 (M+H)⁺; ¹H-NMR(400,0 MHz, CD₃CN): |
| δ= 10,724(1,0);8,330(0,4);8,325(0,4);8,301(4,7);8,295(4,8);8,270(3,7);8,263(3,7);8,209(0,4);8,204(0,4);7,754(2,6);7,734(3,3);7,726(1,8);7,7 19(1,7);7,705(1,9);7,699(3,6);7,693(2,9);7,678(3,7);7,675(3,5);7,658(2,2);7,610(6,0);7,604(6,9);7,586(2,6);7,567(1,1);7,551(3,3);7,532(2,4); 7,484(0,4);7,445(0,3);5,450(0,5);5,444(0,4);3,831(0,5);2,470(0,4);2,465(0,5);2,460(0,4);2,266(0,6);2,248(1,0);2,171(287,5);2,120(2,6);2,114 (2,6);2,107(2,7);2,101(2,0);2,095(1,4);2,044(0,5);2,029(0,5);2,008(0,4);1,972(2,2);1,964(12,7);1,958(17,6);1,952(99,2);1,946(180,5);1,940(2 44,9);1,934(168,5);1,928(86,7);1,781(0,7);1,775(1,1);1,769(1,5);1,762(1,1);1,756(0,6);1,477(6,4);1,437(1,6);1,386(1,3);1,376(0,8);1,371(1,2 );1,363(0,5);1,340(3,3);1,326(0,5);1,285(5,1);1,269(16,0);1,222(0,4);1,216(0,7);1,210(0,4);1,204(0,5);0,898(0,5);0,882(1,3);0,864(0,7);0,837 (0,3);0,146(1,2);0,008(12,1);0,000(291,0);-0,009(12,1);-0,025(0,5);-0,150(1,2) |
| I-1-467: |
| HPLC-MS: logP = 2,96; Masse (m/z): 383,1 (M+H)⁺; ¹H-NMR(400,0 MHz, DMSO-D₆): |
| δ= 8,496(3,5);8,412(4,6);8,407(4,7);8,395(3,7);8,317(0,3);8,295(1,0);8,273(1,9);8,252(1,2);8,219(1,4);8,213(1,4);8,192(2,4);8,171(1,4);8,16 5(1,3);8,143(4,5);8,137(4,3);7,914(2,2);7,894(2,7);7,853(1,1);7,835(2,4);7,817(1,8);7,755(2,0);7,736(4,7);7,716(6,0);7,698(2,4);7,567(4,5);7, 558(5,3);7,549(4,2);7,388(2,5);7,382(2,5);7,368(2,2);7,135(3,6);7,130(3,5);5,968(4,5);5,963(4,4);5,758(1,8);3,444(21,8);3,324(332,6);3,196( 16,0);2,680(0,9);2,676(1,9);2,671(2,7);2,666(1,9);2,662(0,9);2,524(8,1);2,520(12,6);2,511(150,8);2,507(302,6);2,502(396,3);2,497(279,2);2, 493(129,4);2,338(0,9);2,333(1,9);2,329(2,6);2,324(1,9);2,320(0,9);1,351(0,4);1,258(0,5);1,235(1,0);0,146(0,9);0,008(7,2);0,000(215,4);-0,009(6,4);-0,150(0,9) |
| I-1-468: |
| HPLC-MS: logP = 3,23; Masse (m/z): 397,1 (M+H)⁺; ¹H-NMR(400,0 MHz, DMSO-D₆): |
| δ= 8,501(1,2);8,415(4,9);8,410(5,1);8,383(1,3);8,317(0,5);8,291(0,4);8,270(0,7);8,244(0,5);8,219(1,5);8,213(1,5);8,192(2,8);8,171(2,1);8,16 6(2,1);8,151(5,2);8,145(5,4);8,078(1,5);8,073(1,7);7,895(1,2);7,870(1,9);7,864(2,0);7,832(1,1);7,819(1,2);7,794(0,5);7,767(1,3);7,741(2,9);7, 717(4,5);7,702(3,4);7,696(3,2);7,554(2,4);7,535(6,5);7,527(6,4);7,518(5,1);7,343(3,4);7,335(3,5);7,322(2,6);7,047(1,3);6,933(0,6);6,060(5,2) ;6,054(5,4);5,757(0,7);5,722(1,6);5,717(1,7);4,206(0,6);4,189(0,6);4,128(0,8);4,111(2,0);4,094(2,1);4,077(0,7);3,937(3,2);3,833(0,7);3,480(0 ,5);3,322(131,8);2,675(2,4);2,671(3,2);2,667(2,5);2,506(379,2);2,502(482,3);2,497(374,3);2,333(2,4);2,328(3,2);2,324(2,5);1,352(0,7);1,336 (1,2);1,319(0,7);1,260(2,2);1,243(4,7);1,227(9,5);1,210(16,0);1,192(7,9);1,122(3,3);1,107(2,6);0,146(1,3);0,000(278,2);-0,150(1,4) |
| I-1-469: |
| HPLC-MS: logP = 3,38; Masse (m/z): 409,1 (M+H)⁺; ¹H-NMR(400,0 MHz, CD₃CN): |
| δ= 8,277(1,0);8,270(0,8);8,244(0,8);8,201(3,6);8,196(3,5);8,041(0,5);8,025(0,5);8,012(0,4);7,965(2,0);7,959(2,2);7,948(3,9);7,942(3,8);7,83 0(1,1);7,824(1,3);7,821(1,2);7,815(1,3);7,809(1,2);7,761(0,7);7,752(0,4);7,740(1,1);7,733(1,3);7,728(1,5);7,719(2,2);7,704(3,6);7,698(4,3);7, 691(4,5);7,685(4,3);7,669(5,0);7,652(1,7);7,646(1,9);7,640(1,6);7,620(2,3);7,600(1,6);7,594(1,3);7,582(1,0);7,563(1,0);7,484(5,2);7,475(5,4) ;7,465(5,3);7,447(1,4);7,424(0,5);7,385(1,3);7,379(1,2);7,371(0,9);7,359(1,5);7,353(1,8);7,342(3,2);7,328(2,8);7,077(0,8);7,020(0,4);7,013(0 ,5);6,980(0,4);6,564(0,9);6,078(0,3);6,052(0,5);6,035(0,7);6,015(4,5);6,009(4,9);5,985(2,0);5,969(2,2);5,956(1,9);5,943(1,9);5,928(1,1);5,91 3(0,7);5,900(0,5);5,885(0,4);5,846(2,2);5,840(2,1);5,815(0,6);5,808(0,5);5,797(0,4);5,791(0,4);5,438(0,4);5,434(0,7);5,431(0,6);5,427(0,3);5, 395(0,4);5,391(0,6);5,387(0,6);5,328(1,3);5,312(2,9);5,296(1,9);5,285(3,0);5,282(2,8);5,270(2,5);5,256(2,3);5,189(2,7);5,163(2,7);5,110(0,3) ;5,080(0,7);5,060(0,7);4,971(0,5);4,928(0,6);4,925(0,6);4,825(0,7);4,821(1,1);4,818(0,7);4,810(0,7);4,807(1,1);4,803(0,7);4,667(0,7);4,639(0 ,4);4,576(7,4);4,478(0,5);4,470(0,4);4,465(0,5);4,118(0,4);4,086(2,4);4,068(6,5);4,050(6,4);4,032(2,2);3,549(1,3);3,095(0,4);3,077(0,4);3,03 2(0,3);2,135(12,8);2,119(0,5);2,113(0,5);2,107(0,6);2,101(0,5);1,971(27,7);1,964(6,0);1,958(8,6);1,952(36,2);1,946(63,4);1,939(82,4);1,933( 56,7);1,927(29,4);1,864(0,3);1,808(0,3);1,780(0,4);1,774(0,6);1,768(0,7);1,762(0,5);1,756(0,4);1,614(0,3);1,558(0,3);1,542(0,4);1,525(0,4);1 ,372(1,2);1,360(0,5);1,353(0,6);1,342(0,6);1,269(10,2);1,221(8,4);1,214(1,4);1,203(16,0);1,198(9,7);1,191(3,1);1,186(8,7);1,178(2,3);1,173( 4,3);1,155(2,3);1,139(0,6);1,110(0,6);1,092(1,0);1,076(0,9);1,043(0,4);1,038(0,4);1,033(0,4);1,025(2,1);1,015(0,4);1,007(3,9);0,998(0,5);0,9 89(2,0);0,980(0,4);0,969(0,3);0,950(0,4);0,933(0,4);0,925(0,4);0,897(1,2);0,888(2,1);0,881(2,6);0,876(2,0);0,863(1,9);0,858(2,2);0,840(1,4); 0,080(0,4);0,000(4,7) |
| I-1-470: |
| HPLC-MS: logP = 3,05; Masse (m/z): 407,0 (M+H)⁺; ¹H-NMR(400,0 MHz, DMSO-D₆): |
| δ= 8,511(1,5);8,419(13,8);8,416(13,8);8,316(1,7);8,302(0,8);8,275(1,1);8,219(4,4);8,193(8,3);8,177(15,0);8,173(16,0);7,909(1,3);7,855(1,3); 7,817(0,8);7,731(8,6);7,722(7,8);7,663(0,4);7,562(13,5);7,551(14,5);7,541(12,5);7,351(6,8);7,343(7,4);7,061(1,5);6,144(13,0);6,139(12,7);4, 734(15,4);4,647(0,5);4,565(0,8);4,342(0,7);3,323(299,4);3,238(13,2);2,676(3,4);2,671(4,6);2,667(3,3);2,524(13,3);2,511(282,2);2,507(556,0 );2,502(720,8);2,497(515,7);2,493(245,9);2,333(3,5);2,329(4,7);2,324(3,4);0,008(1,8);0,000(48,9);-0,009(1,6) |
| I-1-471: |
| HPLC-MS: logP = 3,62; Masse (m/z): 423,1 (M+H)⁺; ¹H-NMR(400,0 MHz, DMSO-D₆): |
| δ= 8,502(1,6);8,415(15,1);8,409(15,3);8,316(1,7);8,267(1,1);8,240(1,0);8,219(4,6);8,213(4,4);8,192(8,1);8,171(4,6);8,166(4,3);8,145(15,2);8 ,138(14,8);7,882(1,2);7,828(1,2);7,812(1,1);7,730(8,5);7,718(9,0);7,708(8,0);7,540(14,5);7,529(16,0);7,518(14,1);7,327(7,5);7,318(7,8);7,30 7(6,4);7,021(1,6);6,047(15,5);6,041(15,2);5,756(3,7);3,819(6,9);3,710(0,5);3,670(0,7);3,651(0,8);3,635(0,8);3,604(0,4);3,474(0,8);3,322(187 ,3);2,676(2,5);2,671(3,5);2,667(2,5);2,662(1,2);2,541(1,9);2,525(10,1);2,511(209,5);2,507(422,5);2,502(555,8);2,498(398,2);2,493(190,6);2, 437(0,5);2,431(0,5);2,408(0,4);2,338(1,3);2,333(2,6);2,329(3,5);2,324(2,6);2,239(0,6);1,235(1,2);1,149(3,6);1,131(5,3);1,120(4,0);1,113(4,0) ;1,101(2,3);1,081(0,9);1,041(0,9);0,463(14,4);0,444(13,7);0,396(0,8);0,334(2,2);0,323(2,2);0,284(15,7);0,274(14,9);0,146(0,4);0,025(2,3);0, 008(3,3);0,000(65,5);-0,009(2,7) |
| I-1-472: |
| HPLC-MS: logP = 2,80; Masse (m/z): 408,1 (M+H)⁺; ¹H-NMR(400,0 MHz, DMSO-D₆): |
| δ= 8,441(8,2);8,316(0,9);8,250(3,0);8,226(5,1);8,204(9,4);7,953(0,4);7,779(5,0);7,616(9,0);7,470(4,5);6,043(7,2);5,757(16,0);5,040(12,9);4, 743(0,5);3,324(120,7);2,676(1,1);2,672(1,5);2,667(1,1);2,663(0,5);2,542(1,0);2,525(4,4);2,512(89,2);2,507(180,6);2,503(238,3);2,498(170,2 );2,494(80,9);2,338(0,5);2,334(1,1);2,329(1,5);2,325(1,1);2,321(0,6);2,075(0,6);0,000(0,9) |
| I-1-473: |
| HPLC-MS: logP = 2,62; Masse (m/z): 403,1 (M+H)⁺; ¹H-NMR(400,0 MHz, CD₃CN): |
| δ= 8,690(4,5);8,365(11,3);8,296(11,3);8,292(11,2);7,846(6,2);7,827(8,0);7,748(15,9);7,715(10,1);7,695(7,9);7,427(0,6);7,420(0,7);7,241(0,6 );7,236(0,5);7,171(0,5);7,165(0,4);5,446(2,0);2,138(44,8);2,119(0,5);2,113(0,8);2,107(1,0);2,101(0,7);2,094(0,4);1,964(6,0);1,958(7,3);1,952 (60,8);1,946(113,6);1,939(157,6);1,933(106,8);1,927(53,7);1,920(1,3);1,914(0,5);1,780(0,3);1,774(0,7);1,768(0,9);1,762(0,6);1,387(0,6);1,3 83(0,5);1,372(13,7);1,340(3,1);1,285(4,3);1,276(16,0);1,270(3,2);1,216(0,5);0,881(0,5);0,146(1,2);0,008(11,2);0,000(295,9);-0,009(8,7);-0,150(1,2) |
| I-1-474: |
| HPLC-MS: logP = 2,61; Masse (m/z): 449,0 (M+H)⁺; ¹H-NMR(400,0 MHz, CD₃CN): |
| δ= 8,633(4,3);8,380(10,0);8,298(9,7);7,846(5,6);7,827(7,2);7,756(12,4);7,717(8,7);7,697(7,1);7,426(0,7);7,420(0,7);7,241(0,6);7,235(0,5);7, 171(0,5);7,165(0,4);5,446(3,4);2,566(1,0);2,134(51,7);2,119(0,6);2,113(0,8);2,107(1,1);2,101(0,8);2,094(0,4);1,964(7,7);1,957(10,4);1,952(6 5,5);1,946(119,4);1,939(162,3);1,933(109,9);1,927(55,5);1,914(0,7);1,780(0,4);1,774(0,7);1,768(0,9);1,762(0,6);1,372(14,1);1,340(2,7);1,28 5(3,9);1,276(16,0);1,271(3,0);1,216(0,6);0,881(0,5);0,146(1,0);0,023(0,4);0,021(0,5);0,020(0,6);0,018(0,7);0,008(9,2);0,000(234,8);-0,007(4,0);-0,009(7,0);-0,013(0,6);-0,014(0,6);-0,015(0,5);-0,0156(0,4);-0,0163(0,4);-0,017(0,4);-0,150(1,0) |
| I-1-475: |
| HPLC-MS: logP = 2,63; Masse (m/z): 414,0 (M+H)⁺; ¹H-NMR(400,0 MHz, CD₃CN): |
| δ= 9,306(3,0);8,987(16,0);8,985(15,8);8,381(0,3);8,367(9,2);8,361(9,3);7,863(5,2);7,843(6,9);7,804(3,5);7,798(4,2);7,788(11,1);7,778(13,7); 7,758(4,1);7,752(3,7);7,742(3,0);7,741(3,1);7,734(2,0);7,728(3,1);7,721(2,9);7,713(1,4);7,708(2,1);7,701(1,0);5,447(1,8);2,149(135,3);2,120 (0,5);2,114(0,6);2,107(0,7);2,101(0,5);1,964(5,1);1,958(6,5);1,952(41,1);1,946(75,1);1,940(101,9);1,934(69,4);1,928(35,3);1,915(0,4);1,775( 0,4);1,769(0,6);1,762(0,4);1,285(0,3);1,269(0,4);0,146(0,5);0,018(0,5);0,0162(0,6);0,0155(0,6);0,013(0,8);0,008(5,2);0,000(141,2);-0,007(2,8);-0,009(4,6);-0,013(0,5);-0,150(0,5) |
| I-1-476: |
| HPLC-MS: logP = 2,72; Masse (m/z): 369,0 (M+H)⁺; ¹H-NMR(400,0 MHz, DMSO-D₆): |
| δ= 11,504(1,5);11,462(10,9);8,583(0,3);8,521(5,2);8,515(5,0);8,490(6,7);8,484(15,8);8,478(12,6);8,430(0,8);8,362(10,2);8,355(10,1);8,329(2 ,2);8,322(2,6);8,316(4,2);8,308(3,3);8,301(3,5);8,295(2,2);8,286(4,7);8,280(5,4);8,265(4,8);8,259(7,2);8,253(4,3);8,238(5,3);8,232(3,6);8,20 |
| 2(0,9);8,145(0,5);8,064(0,5);8,014(0,4);8,007(0,5);7,977(0,4);7,961(0,4);7,927(0,4);7,910(0,4);7,807(0,5);7,773(1,1);7,769(1,5);7,760(7,0);7, 756(7,5);7,749(1,8);7,740(8,1);7,736(7,9);7,680(0,5);7,630(0,3);7,561(6,7);7,557(6,5);7,542(10,5);7,538(8,9);7,497(0,6);7,489(1,3);7,478(8, 8);7,470(2,0);7,458(11,8);7,439(5,4);7,356(0,4);7,097(0,9);7,090(1,0);7,027(1,5);7,015(5,7);7,008(5,7);6,997(12,7);6,990(12,7);6,960(0,4);6, 929(0,5);6,904(0,9);6,898(0,7);6,868(0,4);6,810(5,3);6,737(0,8);6,179(0,5);5,757(3,0);5,589(0,7);4,143(0,3);3,774(0,3);3,621(0,4);3,593(0,4) ;3,539(0,5);3,529(0,7);3,511(0,5);3,411(0,8);3,322(817,4);3,264(2,0);3,237(0,7);3,189(1,3);3,114(0,7);3,105(0,7);3,078(1,1);3,035(1,0);2,99 4(1,1);2,989(0,9);2,978(0,8);2,966(1,0);2,941(1,0);2,916(2,0);2,900(2,2);2,882(1,1);2,860(1,4);2,843(0,9);2,825(0,9);2,749(0,8);2,726(1,5);2, 709(1,2);2,675(10,5);2,671(14,0);2,666(10,5);2,541(94,9);2,524(52,2);2,510(827,8);2,506(1578,9);2,502(2019,5);2,497(1471,5);2,493(729,1 );2,430(16,0);2,368(0,4);2,333(9,7);2,328(12,8);2,324(9,3);2,288(1,4);1,234(1,1);1,147(1,3);1,131(0,3);0,909(0,4);0,854(0,3);0,146(2,0);0,00 8(20,4);0,000(500,3);-0,008(20,3);-0,149(2,0) |
| I-1-477: |
| HPLC-MS: logP = 4,47; Masse (m/z): 542,9 (M+H)⁺; ¹H-NMR(400,0 MHz, DMSO-D₆): |
| δ= 8,521(0,5);8,515(0,5);8,490(0,7);8,483(0,8);8,473(7,7);8,467(8,0);8,322(8,2);8,315(8,3);8,301(0,4);8,295(0,4);8,283(2,3);8,277(2,1);8,26 2(2,5);8,256(3,8);8,251(2,3);8,236(2,2);8,230(2,0);7,759(0,5);7,756(0,5);7,739(1,0);7,729(6,5);7,726(14,6);7,723(14,5);7,720(6,8);7,710(6,9) ;7,707(15,3);7,703(16,0);7,635(0,4);7,449(0,5);7,431(9,8);7,411(14,1);7,392(7,7);7,015(0,5);7,008(0,4);6,810(0,6);6,800(9,3);6,793(9,2);5,7 57(5,1);3,322(171,4);2,675(1,9);2,671(2,5);2,666(1,9);2,662(0,9);2,541(10,5);2,524(8,7);2,510(148,0);2,506(286,4);2,502(371,5);2,497(271, 6);2,493(133,8);2,430(1,6);2,333(1,8);2,328(2,5);2,324(1,8);0,146(0,4);0,008(3,3);0,000(85,3);-0,008(3,4);-0,150(0,4) |
| I-1-478: |
| HPLC-MS: logP = 1,88; Masse (m/z): 336,0 (M+H)⁺; ¹H-NMR(400,0 MHz, DMSO-D₆): |
| δ= 11,038(2,5);8,483(2,7);8,476(2,8);8,327(2,2);8,322(2,2);8,291(0,9);8,285(0,8);8,270(0,9);8,264(1,6);8,258(0,8);8,243(0,9);8,237(0,8);6,9 27(3,3);6,921(3,2);5,757(2,1);3,323(70,8);2,675(0,7);2,671(1,0);2,666(0,8);2,649(16,0);2,555(18,1);2,524(2,9);2,511(49,5);2,506(97,6);2,50 2(127,4);2,497(92,7);2,493(45,0);2,333(0,6);2,328(0,8);2,324(0,6);0,008(1,1);0,000(28,5);-0,009(1,0) |
| I-1-479: |
| HPLC-MS: logP = 2,51; Masse (m/z): 369,1 (M+H)⁺; ¹H-NMR(400,0 MHz, DMSO-D₆): |
| δ= 11,604(6,0);8,484(6,4);8,478(6,7);8,367(5,7);8,360(5,6);8,285(2,1);8,278(1,9);8,264(2,2);8,257(3,8);8,251(2,0);8,236(2,1);8,230(1,9);7,5 63(6,5);7,558(8,0);7,540(16,0);7,498(8,0);7,481(5,1);7,475(3,9);7,466(0,4);7,458(2,8);7,001(8,2);6,994(8,1);3,405(0,4);3,377(1,2);3,343(385 ,4);3,300(0,6);3,289(0,4);2,677(0,5);2,672(0,6);2,668(0,4);2,542(2,7);2,526(2,0);2,512(36,4);2,508(71,9);2,503(94,1);2,499(67,9);2,494(32,4 );2,334(0,4);2,330(0,6);2,325(0,4);0,000(2,2) |
| I-1-480: |
| HPLC-MS: logP = 2,51; Masse (m/z): 329,1 (M+H)⁺; ¹H-NMR(400,0 MHz, CD₃CN): |
| δ= 9,331(3,0);8,249(5,7);8,245(5,7);8,212(7,3);8,205(7,2);7,669(3,7);7,663(3,7);7,656(0,4);7,649(3,9);7,643(6,7);7,636(3,8);7,622(3,7);7,61 6(3,6);7,180(4,1);7,160(7,2);7,141(7,1);7,085(7,8);7,078(7,7);7,053(0,4);7,035(16,0);7,016(11,7);5,447(3,3);2,432(0,7);2,275(112,3);2,237(1 ,8);2,156(117,0);2,125(0,4);2,120(0,5);2,114(1,1);2,107(0,6);2,101(0,4);1,964(4,4);1,958(5,5);1,952(35,9);946(65,2);1,940(88,4);1,934(61, 0);1,928(31,0);1,915(0,3);1,774(0,4);1,768(0,5);1,762(0,3);1,340(0,4);1,285(0,7);1,269(1,7);1,254(0,5);0,146(0,4);0,008(3,7);0,000(104,2);-0,009(2,9);-0,150(0,4) |
| I-1-481: |
| HPLC-MS: logP = 2,22; Masse (m/z): 343,1 (M+H)⁺; ¹H-NMR(400,0 MHz, DMSO-D₆): |
| δ= 8,293(3,7);8,287(3,8);8,072(1,2);8,066(1,1);8,051(1,3);8,045(2,2);8,039(1,2);8,024(1,4);8,015(4,8);8,008(4,4);7,820(2,1);7,807(0,4);7,80 0(3,2);7,739(1,1);7,736(1,1);7,720(2,6);7,702(2,0);7,699(1,8);7,674(4,6);7,615(2,9);7,611(3,4);7,598(3,2);7,591(2,5);7,580(1,3);5,758(2,0);5, 421(4,4);5,414(4,4);3,324(51,5);2,675(0,4);2,671(0,6);2,667(0,4);2,524(2,2);2,511(31,9);2,507(61,3);2,502(78,9);2,497(56,4);2,493(26,6);2, 333(0,4);2,329(0,5);2,324(0,3);1,989(0,5);1,906(16,0);1,299(1,8);1,259(2,5);1,235(0,9);0,008(1,3);0,000(30,3);-0,009(0,9) |
| I-1-482: |
| HPLC-MS: logP = 1,77; Masse (m/z): 382,0 (M+H)⁺; ¹H-NMR(400,0 MHz, DMSO-D₆): |
| δ= 11,525(11,7);8,485(13,0);8,479(16,0);8,470(8,4);8,465(8,0);8,370(10,6);8,364(10,5);8,333(0,4);8,326(0,4);8,316(3,3);8,288(3,8);8,282(3, 6);8,267(4,1);8,261(6,9);8,254(3,6);8,239(3,8);8,233(3,3);8,126(0,3);8,099(0,3);8,056(0,4);8,048(0,4);8,000(7,7);7,995(7,9);7,981(8,7);7,977 (8,1);7,565(8,4);7,553(8,0);7,546(7,8);7,534(7,6);7,000(13,9);6,994(13,7);5,825(0,5);5,819(0,4);5,756(5,9);5,213(0,5);3,395(0,3);3,381(0,4); 3,321(476,1);2,752(0,4);2,740(0,5);2,679(3,5);2,675(6,9);2,671(9,5);2,666(7,1);2,644(0,6);2,524(30,1);2,510(548,2);2,506(1095,4);2,502(14 33,5);2,497(1029,5);2,492(494,2);2,337(3,1);2,333(6,6);2,328(9,0);2,324(6,5);0,147(0,4);0,008(3,2);0,000(94,4);-0,009(3,3);-0,149(0,4) |
| I-1-483: |
| HPLC-MS: logP = 2,51; Masse (m/z): 364,1 (M+H)⁺; ¹H-NMR(400,0 MHz, DMSO-D₆): |
| δ= 11,327(2,2);8,481(2,2);8,475(2,3);8,347(1,9);8,341(1,9);8,283(0,7);8,277(0,7);8,262(0,8);8,256(1,4);8,249(0,7);8,235(0,8);8,228(0,7);7,4 35(2,8);7,413(3,1);7,148(2,4);7,141(2,8);7,065(1,8);7,057(1,5);7,043(1,6);7,035(1,4);6,996(2,7);6,989(2,7);3,805(16,0);3,329(65,6);2,542(0, 4);2,525(0,6);2,520(1,0);2,511(12,0);2,507(24,3);2,502(32,1);2,498(23,0);2,493(10,7);0,000(5,8) |
| I-1-484: |
| HPLC-MS: logP = 2,38; Masse (m/z): 353,1 (M+H)⁺; ¹H-NMR(400,0 MHz, DMSO-D₆): |
| δ= 11,649(5,5);8,486(5,6);8,480(5,8);8,371(5,1);8,364(5,0);8,288(1,8);8,282(1,7);8,267(1,9);8,261(3,4);8,255(1,7);8,240(1,8);8,234(1,6);7,5 58(1,3);7,542(1,6);7,537(3,1);7,521(3,2);7,516(2,3);7,501(2,2);7,428(5,2);7,408(3,5);7,373(2,4);7,371(2,3);7,350(4,0);7,329(1,9);7,327(1,8); 6,993(7,0);6,987(6,9);5,757(16,0);3,322(50,0);3,028(0,5);2,813(0,5);2,675(0,8);2,671(1,0);2,666(0,8);2,524(3,3);2,511(61,4);2,506(118,6);2, 502(153,3);2,497(110,0);2,493(52,7);2,333(0,8);2,328(1,0);2,324(0,7);2,319(0,4);1,336(0,9);1,298(0,4);1,259(0,6);1,249(1,1);1,235(0,4);0,1 46(0,4);0,008(3,7);0,000(82,2);-0,009(2,8);-0,150(0,4) |
| I-1-485: |
| HPLC-MS: logP = 1,89; Masse (m/z): 337,0 (M+H)⁺; ¹H-NMR(400,0 MHz, DMSO-D₆): |
| δ= 11,635(9,8);8,786(0,4);8,772(13,7);8,766(15,8);8,702(16,0);8,695(13,3);8,497(10,5);8,491(10,8);8,409(9,8);8,403(9,6);8,300(3,1);8,294(2 ,8);8,279(3,4);8,273(5,7);8,266(3,0);8,252(3,1);8,245(2,8);7,016(12,8);7,009(12,5);3,339(200,7);2,682(0,5);2,678(0,7);2,673(0,5);2,548(34,4 );2,531(2,2);2,517(41,0);2,513(79,5);2,508(102,4);2,504(73,7);2,500(35,5);2,340(0,5);2,335(0,7);2,331(0,5) |
| I-1-486: |
| HPLC-MS: logP = 2,68; Masse (m/z): 341,1 (M+H)⁺; ¹H-NMR(400,0 MHz, DMSO-D₆): |
| δ= 11,154(6,6);8,478(6,9);8,472(7,1);8,326(6,0);8,320(6,0);8,278(2,1);8,272(2,0);8,256(2,5);8,251(3,9);8,245(2,4);8,229(2,2);8,224(2,0);7,4 06(4,1);7,387(5,1);7,375(2,8);7,356(4,8);7,337(3,0);7,235(3,5);7,217(5,4);7,198(2,3);7,022(6,3);7,016(6,3);6,988(5,4);6,969(4,9);4,038(0,5); 4,020(0,5);3,322(67,7);2,671(1,1);2,502(177,6);2,328(1,1);2,253(0,7);2,240(1,6);2,232(2,0);2,220(2,9);2,207(2,1);2,199(1,7);2,186(0,8);1,98 9(1,9);1,397(16,0);1,193(0,5);1,175(1,0);1,157(0,5);0,944(2,1);0,933(5,9);0,929(6,5);0,918(4,4);0,912(6,2);0,908(6,3);0,898(2,6);0,700(2,7); 0,686(8,2);0,674(7,6);0,662(2,4);0,146(0,3);0,000(66,2);-0,150(0,3) |
| I-1-487: |
| HPLC-MS: logP = 2,69; Masse (m/z): 347,1 (M+H)⁺; ¹H-NMR(400,0 MHz, DMSO-D₆): |
| δ= 11,432(4,2);8,481(4,3);8,475(4,5);8,347(3,6);8,340(3,6);8,315(0,8);8,281(1,5);8,275(1,4);8,261(1,6);8,254(2,8);8,248(1,4);8,233(1,5);8,2 27(1,3);7,438(1,1);7,423(1,3);7,418(2,0);7,403(2,0);7,398(1,5);7,383(1,4);7,168(3,3);7,148(2,9);7,142(1,8);7,119(2,7);7,098(1,4);7,006(5,6); 7,000(5,6);3,394(0,4);3,327(714,8);3,326(732,3);3,286(0,5);2,680(1,5);2,675(4,1);2,671(4,1);2,666(2,9);2,661(1,8);2,656(5,0);2,637(5,1);2,6 18(1,8);2,541(34,6);2,524(10,1);2,519(15,4);2,511(203,4);2,506(415,9);2,502(549,7);2,497(392,9);2,492(185,1);2,454(0,5);2,419(0,4);2,338( 1,1);2,333(2,5);2,328(3,5);2,324(2,5);2,319(1,2);2,074(15,6);1,298(0,6);1,259(0,9);1,244(0,5);1,235(1,3);1,196(7,0);1,177(16,0);1,158(6,9);0 ,008(2,4);0,000(81,5);-0,009(2,5) |
| I-1-488: |
| HPLC-MS: logP = 2,69; Masse (m/z): 329,1 (M+H)⁺; ¹H-NMR(400,0 MHz, DMSO-D₆): |
| δ= 11,140(3,2);8,485(4,5);8,479(4,6);8,332(3,1);8,326(3,1);8,283(1,4);8,277(1,3);8,262(1,5);8,256(2,5);8,250(1,3);8,235(1,4);8,229(1,3);7,4 55(2,1);7,435(3,0);7,414(3,0);7,411(2,5);7,395(2,3);7,392(1,9);7,334(3,6);7,317(2,3);7,295(2,0);7,292(1,7);7,276(2,9);7,274(2,6);7,258(1,2); 7,255(1,1);7,012(3,0);7,006(3,0);3,434(0,4);3,429(0,4);3,416(0,5);3,408(0,5);3,349(446,7);3,298(0,7);2,786(1,5);2,767(4,5);2,749(4,7);2,730 (1,6);2,718(1,2);2,682(0,6);2,678(0,8);2,673(0,6);2,583(0,4);2,572(0,8);2,548(246,6);2,531(2,8);2,518(49,0);2,513(94,0);2,509(120,2);2,504( 86,9);2,500(42,3);2,374(1,1);2,340(0,6);2,335(0,8);2,331(0,6);1,241(0,4);1,195(7,3);1,176(16,0);1,157(7,1) |
| I-1-489: |
| HPLC-MS: logP = 2,42; Masse (m/z): 331,1 (M+H)⁺; ¹H-NMR(400,0 MHz, DMSO-D₆): |
| δ= 11,212(4,2);8,765(5,0);8,759(5,2);8,591(4,9);8,585(4,5);8,491(4,3);8,485(4,5);8,381(4,1);8,375(4,0);8,316(0,4);8,296(1,3);8,290(1,2);8,2 75(1,4);8,269(2,4);8,263(1,3);8,248(1,3);8,242(1,1);7,025(5,0);7,018(4,9);3,325(444,3);3,286(0,3);3,104(1,9);3,085(5,9);3,067(6,1);3,048(2, 0);2,675(1,9);2,671(2,5);2,666(1,9);2,541(2,1);2,524(7,6);2,510(154,0);2,506(297,2);2,502(383,3);2,497(279,7);2,493(137,9);2,333(1,8);2,32 8(2,4);2,324(1,8);2,074(7,7);1,298(0,6);1,284(7,6);1,265(16,0);1,247(7,6);1,235(1,2);0,008(2,5);0,000(62,0);-0,008(2,2) |
| I-1-490: |
| HPLC-MS: logP = 1,29; Masse (m/z): 330,1 (M+H)⁺; ¹H-NMR(400,0 MHz, DMSO-D₆): |
| δ= 11,352(2,0);8,589(1,4);8,584(1,5);8,577(1,5);8,572(1,5);8,484(2,3);8,478(2,5);8,351(1,9);8,345(1,9);8,316(0,7);8,285(0,7);8,279(0,7);8,2 64(0,9);8,258(1,5);8,252(1,2);8,237(0,9);8,231(0,7);7,859(1,1);7,855(1,2);7,840(1,3);7,836(1,3);7,324(1,3);7,312(1,3);7,305(1,3);7,292(1,2); 7,017(2,0);7,011(2,0);3,327(785,0);2,909(1,0);2,890(2,9);2,871(3,0);2,852(1,0);2,675(3,0);2,671(4,2);2,666(3,1);2,662(1,5);2,541(11,4);2,52 4(12,2);2,519(19,0);2,511(236,8);2,506(479,5);2,502(634,6);2,497(460,5);2,492(222,5);2,419(0,4);2,337(1,4);2,333(2,9);2,328(4,1);2,324(3, 0);2,289(0,5);2,074(16,0);1,298(0,5);1,258(0,7);1,241(4,3);1,222(8,7);1,203(3,9);1,148(0,4);0,146(0,3);0,008(3,1);0,000(87,1);-0,009(2,4);-0,150(0,4) |
| I-1-491: |
| HPLC-MS: logP = 2,57; Masse (m/z): 387,1 (M+H)⁺; ¹H-NMR(400,0 MHz, DMSO-D₆): |
| δ= 11,702(10,0);8,486(9,8);8,480(10,4);8,376(9,7);8,369(9,8);8,285(2,7);8,279(2,5);8,264(2,9);8,258(5,1);8,251(2,7);8,237(2,7);8,230(2,5);7 ,778(0,8);7,757(2,6);7,748(1,5);7,738(5,3);7,722(8,2);7,701(16,0);7,684(4,6);7,679(3,9);6,981(12,1);6,974(12,0);3,518(1,0);3,343(97,1);3,00 2(0,4);2,716(0,5);2,571(0,3);2,563(0,7);2,546(116,6);2,530(0,6);2,525(0,9);2,516(12,3);2,512(25,3);2,507(34,0);2,502(25,0);2,498(12,2);2,3 72(0,5);0,000(1,4) |
| I-1-492: |
| HPLC-MS: logP = 2,56; Masse (m/z): 443,9 (M+H)⁺; ¹H-NMR(400,0 MHz, DMSO-D₆): |
| δ= 11,544(6,4);8,486(6,4);8,480(6,7);8,368(5,7);8,362(5,6);8,288(2,0);8,282(1,8);8,267(2,1);8,261(3,7);8,255(1,9);8,240(2,0);8,234(1,8);7,7 39(4,8);7,737(4,8);7,720(5,2);7,718(5,0);7,379(1,9);7,377(1,9);7,356(4,5);7,335(3,0);7,333(2,7);7,285(2,5);7,270(3,0);7,265(3,2);7,250(3,3); 7,244(1,7);7,229(1,5);6,994(7,9);6,987(7,8);3,323(53,2);2,675(0,7);2,671(1,0);2,666(0,7);2,524(3,7);2,511(59,4);2,506(113,0);2,502(143,6); 2,497(101,7);2,493(47,6);2,337(0,3);2,333(0,7);2,328(0,9);2,324(0,6);1,989(0,9);1,398(16,0);1,175(0,5);0,008(3,0);0,000(72,0);-0,009(2,3) |
| I-1-493: |
| HPLC-MS: logP = 2,31; Masse (m/z): 319,1 (M+H)⁺; ¹H-NMR(400,0 MHz, DMSO-D₆): |
| δ= 11,232(10,4);8,483(15,3);8,477(16,0);8,354(11,8);8,347(11,6);8,286(4,6);8,280(4,3);8,265(5,0);8,259(8,7);8,253(4,5);8,238(4,6);8,232(4, 2);7,701(3,5);7,697(4,0);7,683(6,7);7,678(7,5);7,664(4,0);7,659(4,2);7,603(2,2);7,598(2,2);7,589(2,6);7,585(5,0);7,582(3,9);7,580(3,8);7,578 (3,4);7,570(4,2);7,566(3,9);7,564(5,2);7,559(2,9);7,550(2,9);7,546(2,5);7,348(5,9);7,328(13,2);7,324(7,6);7,311(12,6);7,309(11,8);7,303(5,1) ;7,301(4,9);7,293(6,3);7,290(5,2);7,000(10,8);6,994(10,6);3,441(0,5);3,429(0,5);3,344(925,4);3,268(0,4);2,996(1,7);2,713(1,0);2,682(0,6);2, 677(1,1);2,672(1,5);2,668(1,1);2,663(0,5);2,576(0,4);2,543(275,3);2,526(4,7);2,521(7,4);2,512(85,2);2,508(171,2);2,503(226,8);2,499(163,8 );2,494(77,8);2,369(1,0);2,339(0,5);2,335(1,1);2,330(1,5);2,326(1,1);2,321(0,5);0,000(2,7) |
| I-1-494: |
| HPLC-MS: logP = 2,53; Masse (m/z): 433,0 (M+H)⁺; ¹H-NMR(400,0 MHz, DMSO-D₆): |
| δ= 11,058(15,7);8,492(14,4);8,486(15,8);8,343(13,4);8,336(13,9);8,295(4,1);8,289(4,1);8,274(4,6);8,268(7,9);8,263(4,7);8,247(4,2);8,241(4, 0);7,807(15,1);7,793(16,0);7,410(14,0);7,396(13,2);6,994(9,7);6,989(10,0);6,966(0,5);3,481(0,4);3,437(0,5);3,426(0,5);3,421(0,6);3,336(696 ,2);3,284(1,3);3,259(0,9);3,218(0,3);3,002(1,1);2,718(3,7);2,677(2,7);2,654(0,7);2,634(0,7);2,596(2,0);2,548(688,0);2,509(383,3);2,433(1,2); 2,374(3,7);2,335(2,5);2,297(0,4);1,241(1,2);1,155(0,4) |
| I-1-495: |
| HPLC-MS: logP = 1,79; Masse (m/z): 428,0 (M+H)⁺; ¹H-NMR(400,0 MHz, DMSO-D₆): |
| δ= 13,527(0,3);11,480(13,3);10,125(0,3);8,493(13,0);8,487(13,7);8,429(8,8);8,424(9,5);8,417(9,7);8,412(9,2);8,397(0,5);8,376(11,6);8,370(1 1,6);8,294(4,1);8,288(3,7);8,273(4,3);8,267(7,6);8,261(3,9);8,246(4,1);8,240(3,7);7,839(8,5);7,834(8,7);7,820(10,1);7,815(9,3);7,534(0,5);7, 527(9,9);7,515(9,3);7,508(8,8);7,496(8,7);7,209(0,9);7,081(1,0);7,006(16,0);6,999(15,9);6,954(0,7);3,891(0,3);3,876(0,4);3,802(0,4);3,743(0 ,4);3,739(0,3);3,691(0,4);3,681(0,4);3,665(0,5);3,647(0,5);3,631(0,4);3,608(0,4);3,577(0,4);3,568(0,5);3,554(0,6);3,537(0,7);3,505(0,8);3,49 8(0,8);3,484(0,6);3,466(1,4);3,458(1,2);3,443(1,4);3,430(1,3);3,414(1,8);3,395(3,2);3,341(3202,7);3,309(7,4);3,295(3,7);3,276(1,5);3,258(0, 9);3,232(0,8);3,220(0,8);3,200(0,5);3,192(0,6);3,138(0,3);3,102(0,3);3,098(0,4);3,024(0,4);3,002(0,5);2,718(1,3);2,687(2,4);2,682(5,1);2,678 (7,1);2,673(5,2);2,669(2,6);2,639(0,5);2,605(0,7);2,599(0,7);2,590(0,8);2,569(1,8);2,548(310,4);2,531(22,0);2,526(34,8);2,518(413,6);2,513( 826,0);2,509(1080,9);2,504(770,7);2,500(365,0);2,442(0,8);2,421(0,5);2,411(0,4);2,391(0,4);2,374(1,2);2,357(0,4);2,344(2,3);2,340(5,0);2,3 35(6,8);2,331(4,9);2,326(2,1);2,297(0,8);2,253(0,4);1,440(0,5);1,295(0,4);1,265(0,5);1,251(1,2);1,243(2,5);1,162(0,4);1,154(0,7);-1,685(0,3);-3,450(0,4) |
| I-1-496: |
| HPLC-MS: logP = 2,55; Masse (m/z): 331,1 (M+H)⁺; ¹H-NMR(400,0 MHz, DMSO-D₆): |
| δ= 10,622(2,6);8,482(2,9);8,476(3,0);8,329(2,2);8,322(2,2);8,316(0,5);8,286(1,0);8,280(0,9);8,265(1,1);8,259(1,9);8,253(1,0);8,238(1,0);8,2 32(0,9);7,773(1,5);7,769(1,6);7,754(1,6);7,750(1,6);7,557(0,9);7,553(0,9);7,539(1,2);7,536(1,4);7,532(1,2);7,518(1,1);7,514(1,0);7,216(2,2); 7,196(1,9);7,105(1,3);7,103(1,3);7,087(2,3);7,085(2,2);7,068(1,2);7,066(1,1);7,011(2,9);7,004(2,8);3,941(16,0);3,767(0,4);3,324(38,4);2,671 (0,4);2,525(1,2);2,520(1,8);2,511(19,4);2,507(38,7);2,502(51,0);2,497(36,7);2,493(17,5);2,329(0,3);1,336(0,4);1,250(0,6);0,008(1,0);0,000(2 8,8);-0,009(0,9) |
| I-1-497: |
| HPLC-MS: logP = 2,35; Masse (m/z): 390,0 (M+H)⁺; ¹H-NMR(400,0 MHz, DMSO-D₆): |
| δ= 11,855(1,7);8,488(2,9);8,482(3,1);8,367(2,5);8,361(2,4);8,317(0,4);8,297(0,9);8,291(0,9);8,276(1,0);8,270(1,7);8,264(0,9);8,249(0,9);8,2 43(0,8);6,934(1,6);6,928(1,6);5,757(0,4);3,322(176,6);2,759(16,0);2,675(2,2);2,671(2,9);2,666(2,1);2,541(6,7);2,523(11,8);2,510(170,2);2,5 06(326,4);2,501(421,8);2,497(306,9);2,493(151,0);2,333(2,0);2,328(2,8);2,324(2,0);0,146(0,4);0,008(4,1);0,000(97,2);-0,008(3,7);-0,150(0,5) |
| I-1-498: |
| HPLC-MS: logP = 2,17; Masse (m/z): 339,0 (M+H)⁺; ¹H-NMR(400,0 MHz, DMSO-D₆): |
| δ= 10,506(2,1);8,466(2,1);8,465(2,2);8,459(2,3);8,288(1,9);8,281(1,9);8,273(0,9);8,267(0,8);8,252(0,9);8,246(1,5);8,239(0,8);8,225(0,8);8,2 18(0,8);6,851(2,6);6,844(2,6);5,756(0,4);4,289(1,9);4,281(1,5);4,278(2,0);4,275(1,5);4,266(2,0);3,324(33,6);3,045(2,0);3,037(1,5);3,033(2,1) ;3,030(1,5);3,022(1,9);2,525(0,5);2,520(0,8);2,512(10,8);2,507(21,7);2,502(28,8);2,498(20,8);2,493(9,7);2,013(16,0);0,000(8,1) |
| I-1-499: |
| HPLC-MS: logP = 2,36; Masse (m/z): 315,2 (M+H)⁺; ¹H-NMR(400,0 MHz, DMSO-D₆): |
| δ= 11,101(2,5);8,479(3,6);8,473(3,8);8,327(2,6);8,321(2,6);8,279(1,2);8,273(1,1);8,258(1,3);8,252(2,2);8,246(1,2);8,231(1,2);8,225(1,1);7,4 84(1,8);7,465(2,0);7,395(0,8);7,392(0,8);7,376(2,1);7,373(2,1);7,358(1,8);7,354(1,6);7,293(3,0);7,284(1,9);7,274(2,1);7,265(2,4);7,246(0,9); 7,010(2,1);7,004(2,1);5,757(6,0);3,322(23,1);2,675(0,4);2,671(0,5);2,666(0,3);2,524(1,6);2,510(30,4);2,506(57,8);2,502(73,8);2,497(53,5);2, 493(26,1);2,396(16,0);2,333(0,4);2,328(0,5);2,324(0,4);1,336(0,5);1,250(0,6);0,008(1,5);0,000(36,4);-0,008(1,4) |
| I-1-500: |
| HPLC-MS: logP = 2,06; Masse (m/z): 373,1 (M+H)⁺; ¹H-NMR(400,0 MHz, DMSO-D₆): |
| δ= 10,918(4,5);8,488(5,1);8,482(5,3);8,348(4,3);8,342(4,3);8,298(1,5);8,292(1,4);8,277(1,6);8,271(2,8);8,264(1,5);8,250(1,5);8,244(1,4);7,2 71(2,3);7,137(5,3);7,002(2,5);6,922(4,9);6,916(4,8);4,180(0,7);3,822(16,0);3,540(0,9);3,335(197,1);2,718(0,5);2,682(0,8);2,678(1,1);2,673(0 ,7);2,548(117,1);2,531(3,7);2,518(65,5);2,513(129,3);2,509(168,3);2,504(120,5);2,500(57,3);2,374(0,5);2,340(0,8);2,336(1,1);2,331(0,8);1,2 42(0,5) |
| I-1-501: |
| HPLC-MS: logP = 2,20; Masse (m/z): 371,1 (M+H)⁺; ¹H-NMR(400,0 MHz, DMSO-D₆): |
| δ= 11,704(11,9);9,091(10,1);9,085(11,9);9,031(12,3);9,025(10,1);8,902(0,4);8,893(0,3);8,497(12,5);8,490(13,1);8,410(11,6);8,403(11,4);8,2 96(3,7);8,290(3,4);8,275(4,0);8,269(7,0);8,263(3,7);8,248(3,8);8,242(3,4);6,994(16,0);6,987(15,8);3,418(0,4);3,408(0,5);3,355(473,6);3,299( 0,5);2,683(0,5);2,679(0,6);2,674(0,4);2,549(47,3);2,532(2,0);2,527(3,2);2,519(37,9);2,514(75,7);2,509(98,6);2,505(70,5);2,500(33,2);2,341( 0,5);2,336(0,6);2,332(0,5) |
| I-1-502: |
| HPLC-MS: logP = 2,38; Masse (m/z): 370,0 (M+H)⁺; ¹H-NMR(400,0 MHz, DMSO-D₆): |
| δ= 11,474(10,8);8,924(6,2);8,91 2(6,3);8,494(11,1);8,488(11,4);8,383(10,8);8,376(16,0);8,356(6,6);8,294(3,2);8,288(2,9);8,273(3,5);8,267(5, 9);8,261(3,1);8,246(3,1);8,240(2,9);7,810(4,3);7,798(4,3);7,790(4,1);7,778(3,9);6,989(13,9);6,982(13,6);3,390(0,5);3,346(297,6);3,316(0,9); 2,719(1,2);2,684(0,4);2,679(0,6);2,674(0,5);2,593(0,3);2,572(1,0);2,549(274,0);2,532(2,2);2,527(3,1);2,519(36,3);2,514(71,1);2,510(92,1);2, 505(66,2);2,501(31,8);2,376(1,3);2,341(0,5);2,337(0,6);2,332(0,4);1,240(0,4) |
| I-1-503: |
| HPLC-MS: logP = 2,61; Masse (m/z): 341,0 (M+H)⁺; ¹H-NMR(400,0 MHz, DMSO-D₆): |
| δ= 10,972(10,5);8,495(11,6);8,488(12,1);8,357(9,8);8,350(9,7);8,303(3,4);8,297(3,2);8,282(3,7);8,276(6,4);8,270(3,4);8,255(3,5);8,249(3,1); 7,929(14,9);7,916(15,4);7,217(16,0);7,204(15,5);6,944(12,8);6,937(12,6);3,337(356,3);3,310(0,8);2,682(0,8);2,678(1,0);2,673(0,7);2,548(34, 3);2,517(66,5);2,513(126,1);2,509(160,9);2,504(116,0);2,500(56,6);2,340(0,8);2,335(1,1);2,331(0,8);1,240(0,4) |
| I-1-504: |
| HPLC-MS: logP = 2,16; Masse (m/z): 336,0 (M+H)⁺; ¹H-NMR(400,0 MHz, DMSO-D₆): |
| δ= 11,380(12,2);8,628(0,3);8,614(9,7);8,611(10,0);8,602(10,1);8,599(9,9);8,507(0,4);8,493(13,2);8,487(13,8);8,382(12,0);8,376(12,0);8,295( 3,8);8,289(3,6);8,274(4,1);8,268(7,2);8,261(3,9);8,246(3,8);8,240(3,5);8,105(9,1);8,101(9,3);8,084(10,0);8,081(9,6);7,612(10,1);7,600(9,7);7 ,591(9,2);7,579(9,2);7,027(0,4);7,013(16,0);7,006(16,0);3,375(0,6);3,370(0,6);3,353(5,0);3,339(234,0);3,310(0,5);2,682(0,5);2,678(0,7);2,67 3(0,5);2,548(9,3);2,531(2,7);2,526(4,2);2,518(40,2);2,513(79,8);2,509(105,1);2,504(76,9);2,500(37,7);2,340(0,5);2,335(0,7);2,331(0,5);1,23 8(0,3) |
| I-1-505: |
| HPLC-MS: logP = 1,50; Masse (m/z): 331,1 (M+H)⁺; ¹H-NMR(400,0 MHz, DMSO-D₆): |
| δ= 11,667(2,1);9,259(2,6);9,246(2,7);8,491(2,1);8,485(2,3);8,385(1,9);8,378(1,9);8,316(0,5);8,295(0,7);8,289(0,6);8,274(0,7);8,268(1,3);8,2 61(0,7);8,247(0,7);8,241(0,7);7,761(2,7);7,748(2,7);7,015(2,7);7,008(2,7);3,326(667,3);3,081(1,0);3,062(3,0);3,043(3,1);3,024(1,0);2,995(0, 5);2,680(1,2);2,675(2,5);2,671(3,5);2,666(2,5);2,661(1,2);2,541(12,6);2,524(9,9);2,519(15,2);2,511(195,0);2,506(395,4);2,502(520,7);2,497( 370,7);2,492(174,1);2,419(0,3);2,337(1,2);2,333(2,5);2,328(3,4);2,324(2,4);2,319(1,1);2,074(16,0);1,308(3,9);1,298(0,7);1,289(8,6);1,271(3, 8);1,258(0,7);1,244(0,5);1,235(1,1);1,148(0,3);0,146(0,4);0,008(3,0);0,000(91,5);-0,009(2,5);-0,150(0,3) |
| I-1-506: |
| HPLC-MS: logP = 2,65; Masse (m/z): 387,1 (M+H)⁺; ¹H-NMR(400,0 MHz, DMSO-D₆): |
| δ= 11,525(12,5);8,485(12,6);8,479(13,1);8,361(11,1);8,355(11,0);8,286(4,1);8,280(3,8);8,265(4,4);8,259(7,7);8,252(4,0);8,238(4,1);8,231(3, 7);7,931(4,6);7,918(4,8);7,909(5,2);7,896(5,0);7,681(4,5);7,674(4,9);7,659(4,7);7,652(4,7);7,570(2,5);7,565(2,2);7,549(4,7);7,543(3,9);7,528 (2,3);7,522(1,9);6,971(16,0);6,965(15,9);3,512(2,4);3,492(0,4);3,478(0,4);3,424(0,6);3,411(0,7);3,397(0,7);3,388(0,8);3,334(794,3);2,995(0, 4);2,681(0,6);2,676(1,3);2,672(1,7);2,667(1,3);2,662(0,6);2,542(38,7);2,525(4,8);2,520(7,3);2,512(95,5);2,507(193,4);2,502(257,2);2,498(18 6,6);2,493(88,4);2,338(0,6);2,334(1,2);2,329(1,7);2,325(1,2);2,320(0,5);2,074(0,7);1,249(0,4);1,235(0,9);0,000(4,9) |
| I-1-507: |
| HPLC-MS: logP = 2,69; Masse (m/z): 433,0 (M+H)⁺; ¹H-NMR(400,0 MHz, DMSO-D₆): |
| δ= 11,088(9,2);8,496(9,8);8,490(10,2);8,355(7,7);8,348(7,7);8,301(3,0);8,295(2,8);8,280(3,2);8,274(5,6);8,268(2,9);8,253(3,0);8,247(2,7);7, 808(14,7);7,796(15,3);7,285(16,0);7,272(15,2);6,951(11,2);6,944(11,1);3,365(0,4);3,335(236,3);3,315(0,6);2,682(0,7);2,678(0,9);2,673(0,6); 2,548(79,9);2,531(2,8);2,526(4,5);2,518(52,9);2,513(106,2);2,509(139,2);2,504(99,2);2,499(46,7);2,374(0,3);2,344(0,3);2,340(0,7);2,335(0,9 );2,331(0,6);1,240(0,4) |
| I-1-508: |
| HPLC-MS: logP = 3,02; Masse (m/z): 406,1 (M+H)⁺; ¹H-NMR(400,0 MHz, DMSO-D₆): |
| δ= 11,995(2,5);8,492(2,5);8,486(2,8);8,414(2,4);8,407(2,5);8,297(0,8);8,291(0,7);8,276(0,8);8,270(1,5);8,264(0,8);8,249(0,8);8,243(0,7);7,0 21(3,1);7,014(3,1);3,857(16,0);3,326(251,2);2,995(0,5);2,754(4,0);2,737(4,3);2,675(0,6);2,671(0,9);2,666(0,7);2,541(47,1);2,524(2,2);2,506( 97,0);2,502(127,5);2,497(94,3);2,493(47,3);2,333(0,6);2,328(0,8);2,324(0,6);2,052(0,4);2,035(0,8);2,018(1,0);2,001(0,8);1,984(0,4);1,235(0, 5);0,946(14,4);0,930(14,0);0,000(4,9) |
| I-1-509: |
| HPLC-MS: logP = 2,72; Masse (m/z): 392,1 (M+H)⁺; ¹H-NMR(400,0 MHz, DMSO-D₆): |
| δ= 11,970(2,8);8,492(2,5);8,486(2,7);8,409(2,3);8,402(2,4);8,315(0,3);8,297(0,7);8,291(0,7);8,276(0,8);8,269(1,4);8,263(0,7);8,248(0,8);8,2 42(0,7);7,016(3,0);7,010(3,0);3,855(16,0);3,520(0,4);3,503(1,1);3,486(1,5);3,469(1,2);3,452(0,5);3,325(243,8);2,995(0,4);2,675(0,8);2,671(1 ,0);2,666(0,8);2,541(46,0);2,524(2,6);2,510(59,1);2,506(119,6);2,502(158,6);2,497(116,6);2,493(57,8);2,333(0,7);2,329(1,0);2,324(0,8);1,28 0(15,5);1,263(15,3);1,236(0,6);0,008(0,5);0,000(15,5);-0,008(0,6) |
| I-1-510: |
| HPLC-MS: logP = 2,38; Masse (m/z): 321,0 (M+H)⁺; ¹H-NMR(400,0 MHz, DMSO-D₆): |
| δ= 10,801(2,4);8,488(2,6);8,482(2,7);8,327(2,0);8,321(2,0);8,293(0,9);8,287(0,8);8,272(0,9);8,266(1,6);8,260(0,8);8,245(0,9);8,239(0,8);7,6 82(2,8);7,669(2,9);7,025(2,8);7,013(2,7);6,940(3,2);6,933(3,2);3,408(0,4);3,350(409,2);3,303(0,5);3,298(0,5);2,718(0,3);2,682(0,4);2,678(0, 6);2,673(0,4);2,548(92,8);2,531(1,9);2,526(3,1);2,518(35,8);2,513(70,6);2,508(91,3);2,504(65,3);2,499(30,9);2,473(16,0);2,374(0,3);2,340(0 ,4);2,335(0,6);2,331(0,4) |
| I-1-511: |
| HPLC-MS: logP = 2,61; Masse (m/z): 374,1 (M+H)⁺; ¹H-NMR(400,0 MHz, DMSO-D₆): |
| δ= 11,788(0,9);8,491(1,9);8,485(2,0);8,387(1,7);8,380(1,7);8,316(0,8);8,300(0,6);8,294(0,6);8,279(0,7);8,273(1,1);8,267(0,6);8,251(0,6);8,2 46(0,5);6,961(2,3);6,955(2,3);3,323(629,4);2,679(1,7);2,675(3,5);2,670(4,8);2,666(3,5);2,661(1,7);2,541(3,7);2,524(14,5);2,519(22,5);2,510( 272,2);2,506(546,9);2,501(719,1);2,497(518,4);2,492(247,3);2,418(12,0);2,337(1,5);2,333(3,3);2,328(4,6);2,324(3,3);2,319(1,5);2,074(16,0); 1,298(0,7);1,258(0,9);1,244(0,4);1,236(0,7);1,147(0,5);0,146(0,8);0,008(6,3);0,000(196,3);-0,009(6,1);-0,150(0,8) |
| I-1-512: |
| HPLC-MS: logP = 3,11; Masse (m/z): 406,1 (M+H)⁺; ¹H-NMR(400,0 MHz, DMSO-D₆): |
| δ= 11,975(1,1);8,491(1,0);8,485(1,1);8,405(1,0);8,398(1,0);8,315(0,4);8,275(0,4);8,269(0,6);8,248(0,3);7,003(1,3);6,997(1,2);3,830(6,8);3,3 53(0,8);3,325(347,8);3,294(0,3);2,995(0,5);2,675(0,9);2,671(1,2);2,666(0,8);2,662(0,4);2,541(32,0);2,524(3,1);2,511(71,5);2,506(139,7);2,5 |
| 02(179,3);2,497(127,2);2,493(59,8);2,337(0,4);2,333(0,9);2,328(1,1);2,324(0,8);1,396(16,0);1,235(0,8);0,008(0,6);0,000(16,6);-0,009(0,6) |
| I-1-513: |
| HPLC-MS: logP = 3,89; Masse (m/z): 395,1 (M+H)⁺; ¹H-NMR(400,0 MHz, DMSO-D₆): |
| δ= 11,415(0,7);8,488(0,9);8,482(1,0);8,363(0,7);8,356(0,7);8,274(0,3);8,267(0,6);6,975(0,5);6,969(0,5);3,844(5,8);3,327(91,5);2,541(0,5);2, 524(0,6);2,520(0,9);2,511(14,7);2,506(30,2);2,502(40,0);2,497(28,6);2,493(13,5);1,355(16,0);0,000(3,5) |
| I-1-514: |
| HPLC-MS: logP = 2,13; Masse (m/z): 369,1 (M+H)⁺; ¹H-NMR(400,0 MHz, DMSO-D₆): |
| δ= 11,655(2,2);8,491(4,2);8,485(4,5);8,369(3,2);8,363(3,3);8,315(1,1);8,297(1,4);8,291(1,2);8,276(1,4);8,270(2,5);8,264(1,3);8,249(1,4);8,2 43(1,2);7,145(1,3);7,008(3,0);6,978(2,0);6,973(2,0);6,871(1,5);3,993(0,4);3,847(16,0);3,388(0,4);3,374(0,6);3,326(1334,4);3,282(1,0);3,275( 0,7);3,246(0,4);2,995(1,1);2,675(2,6);2,671(3,6);2,666(2,6);2,541(37,1);2,524(8,4);2,519(13,0);2,511(200,2);2,506(410,1);2,502(544,2);2,49 7(389,5);2,493(184,5);2,423(0,5);2,337(1,2);2,333(2,6);2,328(3,5);2,324(2,5);2,319(1,2);2,289(0,7);2,262(14,1);1,298(0,4);1,259(0,6);1,235( 2,0);0,008(0,6);0,000(16,9);-0,009(0,5) |
| I-1-515: |
| HPLC-MS: logP = 2,00; Masse (m/z): 370,1 (M+H)⁺; ¹H-NMR(400,0 MHz, DMSO-D₆): |
| δ= 11,699(11,2);8,987(16,0);8,974(9,5);8,972(9,4);8,961(9,0);8,494(1 0,4);8,488(12,7);8,386(9,4);8,379(11,4);8,294(3,6);8,287(3,8);8,273(4, 1);8,266(7,3);8,260(4,8);8,245(4,0);8,239(3,9);7,892(8,9);7,879(9,9);7,000(13,0);6,993(15,1);3,555(0,3);3,550(0,3);3,533(0,4);3,454(0,9);3,4 46(1,0);3,429(1,3);3,425(1,3);3,360(1310,0);3,352(358,2);3,299(2,3);3,281(1,3);3,272(0,9);3,262(0,8);3,256(0,7);3,237(0,6);3,228(0,7);3,21 7(0,6);3,207(0,6);3,198(0,5);3,002(1,3);2,719(2,3);2,688(0,5);2,683(0,9);2,679(1,4);2,674(1,2);2,607(0,3);2,601(0,4);2,587(0,9);2,583(0,9);2, 579(0,9);2,576(1,1);2,573(1,1);2,549(641,4);2,542(88,5);2,539(84,7);2,528(8,3);2,519(70,7);2,514(154,9);2,510(223,4);2,505(190,9);2,501(1 28,5);2,462(0,8);2,426(0,4);2,418(0,4);2,395(0,3);2,386(0,3);2,375(2,4);2,370(0,7);2,368(0,7);2,345(0,6);2,341(1,1);2,336(1,5);2,332(1,3);2, 327(0,9);1,240(0,6) |
| I-1-516: |
| HPLC-MS: logP = 2,98; Masse (m/z): 423,0 (M+H)⁺; ¹H-NMR(601,6 MHz, DMSO-D₆): |
| δ= 11,830(14,2);10,109(0,4);8,486(13,4);8,482(13,7);8,418(0,4);8,405(0,4);8,391(12,3);8,387(12,0);8,314(1,5);8,281(3,9);8,277(3,8);8,268(4 ,3);8,263(7,5);8,259(3,8);8,249(4,0);8,245(3,5);8,232(0,5);8,077(2,7);8,066(3,1);8,051(2,5);6,961(16,0);6,956(15,9);6,200(0,4);6,143(0,5);6, 138(0,4);5,755(0,8);3,320(392,5);2,617(1,9);2,613(2,6);2,610(2,1);2,541(0,5);2,523(4,1);2,520(4,8);2,517(4,4);2,508(135,6);2,505(302,4);2, 502(429,8);2,499(317,3);2,496(152,4);2,448(0,4);2,389(2,1);2,386(2,7);2,383(2,0);1,523(0,4);1,339(0,4);1,234(0,4);0,096(0,5);0,005(2,4);0, 000(97,9);-0,006(4,1);-0,100(0,5);-1,806(0,4) |
| I-1-517: |
| HPLC-MS: logP = 2,53; Masse (m/z): 397,0 (M+H)⁺; ¹H-NMR(400,0 MHz, DMSO-D₆): |
| δ= 11,351(1,9);8,490(2,7);8,484(2,8);8,365(2,1);8,359(2,1);8,315(1,0);8,297(0,9);8,291(0,8);8,276(1,0);8,270(1,6);8,263(0,9);8,249(0,9);8,2 42(0,8);6,979(1,4);6,974(1,3);3,871(16,0);3,354(0,7);3,323(551,5);2,679(1,0);2,675(2,1);2,671(2,9);2,666(2,1);2,662(0,9);2,541(5,7);2,524(6 ,9);2,519(10,6);2,510(162,2);2,506(330,6);2,501(436,1);2,497(310,9);2,492(146,9);2,337(1,0);2,333(2,0);2,328(2,9);2,324(2,1);2,319(1,0);2, 288(0,4);2,161(14,8);1,259(0,4);1,234(0,7);0,008(1,1);0,000(36,9);-0,009(1,1) |
| I-1-518: |
| HPLC-MS: logP = 2,61; Masse (m/z): 397,0 (M+H)⁺; ¹H-NMR(400,0 MHz, DMSO-D₆): |
| δ= 11,474(2,1);8,491(4,4);8,485(4,6);8,369(3,4);8,363(3,4);8,315(1,3);8,299(1,4);8,293(1,3);8,278(1,5);8,271(2,6);8,265(1,3);8,251(1,4);8,2 44(1,2);7,661(8,1);6,987(2,6);6,981(2,6);4,290(1,7);4,272(5,3);4,254(5,4);4,235(1,8);3,322(718,9);3,279(0,6);2,679(1,4);2,675(3,0);2,670(4, 2);2,666(3,1);2,661(1,6);2,541(15,3);2,524(18,3);2,510(235,9);2,506(480,5);2,501(643,1);2,497(474,8);2,492(235,1);2,337(1,4);2,333(3,0);2, 328(4,2);2,323(3,1);2,319(1,5);2,288(0,6);1,359(7,2);1,341(16,0);1,323(7,0);1,298(0,4);1,258(0,5);1,233(1,0);0,008(1,8);0,000(52,7);-0,009(2,1) |
| I-1-519: |
| HPLC-MS: logP = 2,93; Masse (m/z): 413,0 (M+H)⁺; ¹H-NMR(400,0 MHz, DMSO-D₆): |
| δ= 11,387(2,0);8,490(2,9);8,484(3,1);8,360(2,3);8,354(2,3);8,315(0,5);8,297(0,9);8,291(0,9);8,276(1,0);8,270(1,8);8,264(0,9);8,249(1,0);8,2 43(0,9);6,978(1,6);6,973(1,6);4,223(1,0);4,205(3,3);4,187(3,3);4,169(1,1);3,359(0,5);3,325(474,0);3,295(0,7);2,675(1,1);2,671(1,6);2,666(1, 2);2,541(5,2);2,524(3,6);2,511(88,2);2,506(182,2);2,502(243,4);2,497(178,3);2,493(87,5);2,333(1,2);2,329(1,6);2,324(1,2);2,173(16,0);1,338 (4,4);1,320(9,8);1,302(4,4);1,235(0,5);0,008(0,4);0,000(11,8);-0,008(0,4) |
| I-1-520: |
| HPLC-MS: logP = 2,15; Masse (m/z): 385,0 (M+H)⁺; ¹H-NMR(400,0 MHz, DMSO-D₆): |
| δ= 10,367(2,6);8,479(2,4);8,473(2,5);8,334(2,2);8,327(2,3);8,316(0,6);8,289(0,8);8,283(0,7);8,268(0,8);8,262(1,5);8,255(0,8);8,241(0,8);8,2 34(0,7);8,119(5,3);6,930(2,5);6,924(2,4);3,937(16,0);3,323(139,8);2,671(1,4);2,666(1,0);2,541(0,8);2,506(166,2);2,502(211,3);2,497(153,0); 2,328(1,3);2,324(1,0);0,146(0,6);0,008(5,5);0,000(141,0);-0,009(5,4);-0,150(0,6) |
| I-1-521: |
| HPLC-MS: logP = 2,23; Masse (m/z): 385,0 (M+H)⁺; ¹H-NMR(400,0 MHz, DMSO-D₆): |
| δ= 11,435(1,6);8,491(2,5);8,485(2,7);8,373(2,0);8,367(2,1);8,314(0,8);8,297(0,7);8,291(0,7);8,276(0,8);8,270(1,5);8,264(0,8);8,249(0,8);8,2 43(0,7);7,648(4,6);6,982(1,5);3,937(16,0);3,330(1166,4);2,675(2,0);2,671(2,7);2,667(2,0);2,541(6,1);2,524(7,5);2,511(151,5);2,506(301,2);2 ,502(394,8);2,497(286,7);2,493(139,0);2,333(1,9);2,329(2,6);2,324(1,9);2,290(0,3);1,258(0,4);1,233(0,7);0,000(8,1) |
| I-1-522: |
| HPLC-MS: logP = 3,19; Masse (m/z): 451,0 (M+H)⁺; ¹H-NMR(400,0 MHz, DMSO-D₆): |
| δ= 11,777(3,2);8,497(3,4);8,491(3,6);8,401(3,0);8,395(3,0);8,315(1,5);8,304(1,1);8,298(1,0);8,283(1,1);8,277(1,9);8,271(1,0);8,256(1,1);8,2 |
| 50(0,9);6,998(3,4);6,991(3,3);4,022(16,0);3,406(0,4);3,374(0,7);3,324(1094,0);3,281(0,6);2,680(1,4);2,675(3,0);2,671(4,1);2,666(3,0);2,662( 1,4);2,541(10,2);2,524(10,2);2,519(16,1);2,511(233,2);2,506(473,3);2,502(624,0);2,497(448,3);2,493(212,8);2,338(1,4);2,333(2,9);2,328(4,0 );2,324(2,9);2,320(1,4);2,289(0,6);1,298(0,4);1,259(0,6);1,235(1,4);0,008(1,1);0,000(36,8);-0,008(1,2) |
| I-1-523: |
| HPLC-MS: logP = 3,18; Masse (m/z): 399,0 (M+H)⁺; ¹H-NMR(400,0 MHz, DMSO-D₆): |
| δ= 10,849(3,1);8,478(3,3);8,472(3,5);8,326(2,8);8,320(2,8);8,284(1,0);8,278(0,9);8,263(1,1);8,256(1,9);8,250(1,0);8,235(1,0);8,229(0,9);6,9 50(2,3);6,943(2,3);5,757(1,6);3,327(126,0);2,675(0,6);2,671(0,9);2,667(0,7);2,510(54,2);2,506(108,6);2,502(143,0);2,498(104,2);2,493(52,0 );2,400(16,0);2,333(0,7);2,329(0,9);2,324(0,7);2,251(15,4);2,233(0,6);1,351(0,3);1,259(0,4);1,234(0,7);0,008(1,3);0,000(31,0);-0,008(1,3) |
| I-1-524: |
| HPLC-MS: logP = 2,30; Masse (m/z): 384,9 (M+H)⁺; ¹H-NMR(400,0 MHz, DMSO-D₆): |
| δ= 11,223(11,1);8,483(12,1);8,477(12,8);8,339(10,6);8,332(10,6);8,316(0,7);8,288(3,6);8,282(3,3);8,267(3,9);8,261(6,8);8,255(3,8);8,240(16 ,0);8,232(14,5);7,776(14,7);7,768(14,1);6,978(7,5);6,972(7,5);5,757(4,0);3,328(283,1);3,304(0,8);2,676(1,4);2,671(1,8);2,542(0,9);2,507(22 3,5);2,502(289,7);2,498(210,9);2,440(0,3);2,333(1,3);2,329(1,8);1,235(6,3);0,853(0,6);0,147(0,3);0,008(2,6);0,000(66,7);-0,008(2,8) |
| I-1-525: |
| HPLC-MS: logP = 2,84; Masse (m/z): 389,0 (M+H)⁺; ¹H-NMR(400,0 MHz, DMSO-D₆): |
| δ= 11,735(2,7);8,495(3,8);8,489(3,8);8,384(3,0);8,378(2,9);8,315(0,6);8,304(1,1);8,298(1,0);8,283(1,2);8,277(2,0);8,271(1,1);8,256(1,1);8,2 50(1,0);7,984(1,2);7,840(2,8);7,696(1,4);6,970(2,9);6,964(2,8);3,388(0,5);3,328(805,5);2,675(1,4);2,671(1,9);2,666(1,4);2,541(8,2);2,510(11 6,4);2,506(221,4);2,502(286,3);2,497(208,7);2,493(101,9);2,333(1,4);2,329(1,8);2,324(1,4);2,290(0,4);2,269(16,0);1,234(0,6);0,000(8,1) |
| I-1-526: |
| HPLC-MS: logP = 2,42; Masse (m/z): 353,0 (M+H)⁺; ¹H-NMR(400,0 MHz, DMSO-D₆): |
| δ= 10,387(3,9);8,481(3,9);8,475(4,1);8,338(3,4);8,331(3,4);8,316(0,6);8,291(1,3);8,285(1,2);8,270(1,4);8,264(2,5);8,258(1,3);8,243(1,3);8,2 37(1,2);8,183(10,3);6,941(3,9);6,934(3,8);4,230(1,9);4,211(6,1);4,193(6,2);4,175(2,0);3,323(310,6);2,680(0,6);2,675(1,2);2,671(1,7);2,666(1 ,2);2,662(0,6);2,541(4,0);2,524(3,9);2,519(6,1);2,511(94,7);2,506(194,4);2,502(257,6);2,497(184,0);2,493(86,8);2,337(0,6);2,333(1,2);2,328 (1,7);2,324(1,2);2,319(0,5);1,453(7,3);1,435(16,0);1,417(7,2);1,234(0,5);0,008(0,6);0,000(21,0);-0,009(0,7) |
| I-1-527: |
| HPLC-MS: logP = 2,93; Masse (m/z): 367,0 (M+H)⁺; ¹H-NMR(400,0 MHz, DMSO-D₆): |
| δ= 11,354(2,2);8,490(2,8);8,484(3,0);8,361(2,3);8,355(2,2);8,297(0,9);8,290(0,8);8,276(1,0);8,270(1,7);8,263(0,9);8,249(0,9);8,242(0,8);6,9 75(1,4);6,971(1,4);4,236(1,0);4,218(3,3);4,200(3,4);4,182(1,1);3,376(0,4);3,331(300,3);2,676(0,4);2,671(0,6);2,667(0,5);2,542(2,8);2,525(1, 5);2,511(35,2);2,507(70,9);2,502(93,4);2,498(67,7);2,493(32,8);2,334(0,5);2,329(0,6);2,325(0,5);2,177(16,0);1,339(4,3);1,321(9,7);1,303(4, 3);0,000(1,8) |
| I-1-528: |
| HPLC-MS: logP = 3,11; Masse (m/z): 412,0 (M+H)⁺; ¹H-NMR(400,0 MHz, DMSO-D₆): |
| δ= 12,011(3,1);8,501(2,9);8,495(3,0);8,416(2,6);8,409(2,6);8,315(0,4);8,311(0,9);8,305(0,8);8,290(0,9);8,284(1,6);8,278(0,9);8,263(0,9);8,2 57(0,8);7,007(3,4);7,001(3,4);4,901(0,4);4,885(1,1);4,868(1,6);4,852(1,2);4,835(0,4);3,324(142,5);2,995(2,2);2,676(0,5);2,671(0,7);2,667(0, 5);2,541(90,6);2,524(1,6);2,520(2,4);2,511(38,9);2,507(80,6);2,502(107,3);2,497(77,0);2,493(36,5);2,368(0,3);2,333(0,5);2,329(0,7);2,324(0 ,5);1,478(16,0);1,462(16,0);1,235(0,4);0,000(8,1) |
| I-1-529: |
| HPLC-MS: logP = 2,23; Masse (m/z): 339,0 (M+H)⁺; ¹H-NMR(400,0 MHz, DMSO-D₆): |
| δ= 11,413(1,4);8,492(2,5);8,486(2,5);8,374(1,9);8,368(1,9);8,300(0,8);8,294(0,7);8,279(0,8);8,273(1,4);8,267(0,8);8,252(0,8);8,246(0,7);7,6 58(4,7);6,985(1,2);6,980(1,1);3,939(16,0);3,322(85,9);2,675(0,5);2,671(0,6);2,666(0,5);2,541(2,1);2,524(1,8);2,511(38,7);2,506(76,8);2,502( 100,6);2,497(71,7);2,492(33,8);2,333(0,5);2,328(0,6);2,324(0,5);0,008(0,3);0,000(8,8) |
| I-1-530: |
| HPLC-MS: logP = 2,50; Masse (m/z): 384,0 (M+H)⁺; ¹H-NMR(400,0 MHz, DMSO-D₆): |
| δ= 11,918(0,7);11,103(2,4);8,500(0,7);8,490(2,4);8,484(2,5);8,414(0,6);8,408(0,6);8,361(2,0);8,354(2,0);8,315(0,8);8,300(0,8);8,294(0,8);8, 284(0,5);8,279(1,0);8,273(1,5);8,267(0,8);8,252(0,8);8,246(0,7);7,001(0,7);6,995(0,7);6,948(1,8);6,941(1,8);5,756(0,8);4,242(16,0);4,075(4, 0);3,322(231,2);2,675(1,4);2,670(2,0);2,666(1,5);2,541(3,6);2,524(8,8);2,510(108,9);2,506(219,9);2,501(292,2);2,497(214,6);2,493(105,6);2, 333(1,4);2,328(1,9);2,324(1,4);1,233(0,5);0,146(0,7);0,008(6,7);0,000(170,1);-0,009(6,2);-0,150(0,7) |
| I-1-531: |
| HPLC-MS: logP = 3,42; Masse (m/z): 381,1 (M+H)⁺; ¹H-NMR(400,0 MHz, DMSO-D₆): |
| δ= 11,334(1,3);8,489(2,6);8,483(2,7);8,365(2,1);8,359(2,1);8,315(0,6);8,297(0,8);8,291(0,7);8,276(0,9);8,270(1,5);8,263(0,8);8,249(0,8);8,2 42(0,7);6,974(1,3);3,882(16,0);3,323(324,1);2,675(1,4);2,670(2,0);2,666(1,5);2,541(4,7);2,524(7,6);2,519(7,7);2,510(108,7);2,506(224,1);2, 501(296,3);2,497(215,9);2,493(105,2);2,337(0,6);2,333(1,4);2,328(1,9);2,324(1,4);1,656(1,3);1,637(2,4);1,619(2,4);1,600(1,3);1,234(0,6);0, 947(3,8);0,929(7,8);0,911(3,5);0,008(0,8);0,000(26,4);-0,009(0,9) |
| I-1-532: |
| HPLC-MS: logP = 3,09; Masse (m/z): 381,1 (M+H)⁺; ¹H-NMR(400,0 MHz, DMSO-D₆): |
| δ= 10,274(2,5);8,479(2,4);8,473(2,5);8,332(2,1);8,326(2,1);8,316(0,6);8,289(0,8);8,283(0,7);8,268(0,8);8,262(1,4);8,255(0,8);8,241(0,8);8,2 34(0,7);6,929(2,5);6,923(2,5);3,897(16,0);3,324(194,2);2,715(1,7);2,696(2,9);2,677(2,5);2,671(1,6);2,666(1,1);2,541(0,8);2,524(3,6);2,511(7 9,5);2,506(158,1);2,502(205,3);2,497(146,8);2,493(70,2);2,333(1,0);2,328(1,3);2,324(1,0);1,617(1,1);1,598(2,0);1,580(2,0);1,561(1,1);0,946 (3,6);0,928(7,4);0,910(3,2);0,146(0,6);0,008(5,3);0,000(136,9);-0,008(4,9);-0,150(0,6) |
| I-1-533: |
| HPLC-MS: logP = 2,85; Masse (m/z): 367,0 (M+H)⁺; ¹H-NMR(400,0 MHz, DMSO-D₆): |
| δ= 10,389(5,3);8,481(5,3);8,475(5,5);8,341(4,7);8,335(4,7);8,318(0,4);8,292(1,6);8,286(1,4);8,271(1,7);8,265(2,9);8,259(1,5);8,244(1,5);8,2 38(1,4);8,180(12,1);6,942(4,7);6,936(4,7);5,758(0,4);4,146(4,1);4,128(8,1);4,111(4,1);3,328(43,9);2,672(0,4);2,508(47,6);2,503(61,7);2,499( 44,8);2,330(0,4);1,905(0,5);1,887(2,5);1,869(5,0);1,851(5,1);1,833(2,6);1,815(0,6);0,886(0,3);0,874(7,8);0,856(16,0);0,837(7,2);0,008(0,6);0 ,000(13,7) |
| I-1-534: |
| HPLC-MS: logP = 2,98; Masse (m/z): 367,0 (M+H)⁺; ¹H-NMR(400,0 MHz, DMSO-D₆): |
| δ= 11,326(1,9);8,490(2,5);8,484(2,6);8,365(1,9);8,358(1,8);8,316(0,5);8,298(0,9);8,292(0,8);8,277(0,9);8,271(1,6);8,264(0,8);8,250(0,9);8,2 43(0,8);6,975(1,1);6,970(1,1);3,883(16,0);3,322(194,5);2,680(0,4);2,675(0,9);2,671(1,2);2,666(0,9);2,661(0,4);2,597(1,1);2,578(3,6);2,559(3 ,7);2,541(5,8);2,524(2,8);2,519(4,6);2,511(69,3);2,506(141,5);2,502(186,1);2,497(131,7);2,492(61,0);2,338(0,4);2,333(0,9);2,328(1,2);2,324 (0,9);2,319(0,4);1,235(0,5);1,209(4,4);1,190(9,6);1,172(4,2);0,008(0,5);0,000(17,2);-0,009(0,5) |
| I-1-535: |
| HPLC-MS: logP = 3,19; Masse (m/z): 448,1 (M+H)⁺; ¹H-NMR(400,0 MHz, DMSO-D₆): |
| δ= 12,285(3,4);8,505(3,6);8,499(3,9);8,435(3,4);8,428(3,4);8,315(1,6);8,309(1,0);8,294(1,2);8,288(2,0);8,282(1,1);8,267(1,1);8,261(1,1);7,0 11(3,1);7,005(3,1);4,137(16,0);3,329(1158,1);2,995(1,5);2,676(1,7);2,671(2,3);2,667(1,6);2,541(57,7);2,524(6,6);2,511(130,2);2,507(257,5); 2,502(336,2);2,497(239,4);2,493(112,6);2,333(1,6);2,329(2,2);2,324(1,6);2,290(0,3);1,298(0,3);1,259(0,4);1,235(1,4);0,000(6,8) |
| I-1-536: |
| HPLC-MS: logP = 2,76; Masse (m/z): 398,1 (M+H)⁺; ¹H-NMR(400,0 MHz, DMSO-D₆): |
| δ= 12,250(1,6);8,505(3,6);8,499(3,9);8,432(3,3);8,425(3,4);8,315(1,9);8,310(1,1);8,295(1,1);8,288(2,0);8,282(1,1);8,267(1,1);8,261(1,0);7,0 10(2,8);7,003(2,9);4,124(16,0);3,381(0,9);3,329(1809,3);3,271(0,9);3,257(0,7);3,216(0,4);2,676(2,7);2,671(3,7);2,667(2,8);2,541(10,1);2,52 4(9,7);2,511(207,6);2,507(421,9);2,502(559,4);2,497(405,9);2,493(196,3);2,333(2,7);2,329(3,7);2,324(2,7);2,290(0,5);1,298(0,5);1,259(0,7); 1,235(1,3);0,000(14,8);-0,008(0,4) |
| I-1-537: |
| HPLC-MS: logP = 2,45; Masse (m/z): 349,0 (M+H)⁺; ¹H-NMR(400,0 MHz, DMSO-D₆): |
| δ= 11,856(7,5);8,494(15,3);8,488(16,0);8,384(13,3);8,383(13,2);8,378(13,4);8,316(1,1);8,303(5,2);8,297(4,7);8,282(5,5);8,276(9,6);8,270(5, 0);8,255(5,2);8,249(4,7);6,995(8,0);6,989(7,8);5,757(3,7);3,357(0,6);3,322(480,7);2,680(3,2);2,675(4,6);2,671(6,6);2,666(6,7);2,661(4,2);2,6 46(5,5);2,634(3,4);2,626(3,2);2,613(1,7);2,593(0,5);2,582(0,5);2,576(0,6);2,541(2,5);2,524(16,2);2,519(25,2);2,511(297,1);2,506(593,5);2,5 02(785,5);2,497(567,9);2,492(269,0);2,338(1,8);2,333(3,8);2,328(5,2);2,324(3,7);2,319(1,7);1,351(0,6);1,335(1,8);1,298(1,3);1,259(2,0);1,2 49(2,9);1,244(2,9);1,231(8,5);1,224(14,4);1,218(8,7);1,210(7,4);1,203(14,7);1,196(12,0);1,189(13,2);1,183(15,0);1,177(15,0);1,171(8,9);1,1 57(1,9);1,147(1,1);0,146(0,9);0,008(7,5);0,000(225,1);-0,009(7,0);-0,150(0,9) |
| I-1-538: |
| HPLC-MS: logP = 2,03; Masse (m/z): 323,0 (M+H)⁺; ¹H-NMR(400,0 MHz, DMSO-D₆): |
| δ= 11,162(2,5);8,489(3,1);8,483(3,3);8,367(2,7);8,361(2,7);8,315(0,4);8,299(1,0);8,293(0,9);8,278(1,1);8,272(1,9);8,266(1,0);8,251(1,0);8,2 45(0,9);7,620(3,3);7,609(3,3);6,965(2,7);6,958(2,7);3,968(16,0);3,327(374,8);3,303(0,7);2,676(0,8);2,671(1,1);2,667(0,8);2,541(2,3);2,524(3 ,0);2,511(61,7);2,507(122,4);2,502(159,4);2,498(113,9);2,493(54,1);2,333(0,7);2,329(1,0);2,324(0,7);2,320(0,3);0,008(0,4);0,000(12,8);-0,009(0,4) |
| I-1-539: |
| HPLC-MS: logP = 3,28; Masse (m/z): 473,0 (M+H)⁺; ¹H-NMR(400,0 MHz, DMSO-D₆): |
| δ= 11,439(2,3);8,491(2,7);8,485(3,1);8,363(2,1);8,357(2,3);8,314(0,8);8,296(0,8);8,290(0,8);8,275(0,9);8,268(1,6);8,263(0,9);8,248(0,9);8,2 41(0,8);7,615(6,2);6,975(1,9);6,969(1,9);4,085(3,4);4,066(3,5);3,420(0,4);3,330(1398,6);3,226(0,4);2,676(2,3);2,671(3,1);2,667(2,3);2,541(1 1,4);2,524(7,5);2,511(168,6);2,507(341,8);2,502(452,0);2,498(328,4);2,494(161,0);2,333(2,1);2,329(2,9);2,325(2,1);2,290(0,4);2,090(0,4);2, 072(0,8);2,055(1,0);2,038(0,8);2,021(0,4);1,298(0,3);1,258(0,4);1,235(1,4);0,815(16,0);0,799(15,5);0,000(8,5) |
| I-1-540: |
| HPLC-MS: logP = 2,20; Masse (m/z): 431,0 (M+H)⁺; ¹H-NMR(400,0 MHz, DMSO-D₆): |
| δ= 11,393(1,2);8,493(2,8);8,486(3,0);8,372(2,1);8,365(2,2);8,315(1,3);8,297(0,9);8,291(0,8);8,276(0,9);8,270(1,6);8,264(0,9);8,249(0,9);8,2 43(0,8);7,599(5,3);6,990(1,5);6,984(1,5);3,939(16,0);3,323(791,9);2,675(3,1);2,671(4,2);2,666(3,2);2,541(8,9);2,524(11,2);2,511(241,7);2,5 06(488,2);2,502(644,7);2,497(467,3);2,493(229,9);2,333(3,1);2,328(4,1);2,324(3,0);2,288(0,6);1,298(0,4);1,259(0,5);1,233(1,2);0,008(1,6);0 ,000(46,7);-0,009(1,6) |
| I-1-541: |
| HPLC-MS: logP = 2,93; Masse (m/z): 459,0 (M+H)⁺; ¹H-NMR(400,0 MHz, DMSO-D₆): |
| δ= 11,431(3,6);8,492(4,6);8,486(4,9);8,365(3,5);8,359(3,5);8,315(0,4);8,297(1,4);8,291(1,3);8,276(1,5);8,270(2,6);8,264(1,4);8,249(1,4);8,2 43(1,3);7,611(9,7);6,982(2,8);6,976(2,8);4,218(2,9);4,200(5,7);4,183(3,0);3,359(0,4);3,324(329,0);2,995(0,3);2,675(1,0);2,671(1,4);2,666(1, 1);2,662(0,5);2,541(11,2);2,524(3,3);2,511(79,7);2,506(163,6);2,502(218,0);2,497(159,9);2,493(78,7);2,337(0,5);2,333(1,0);2,328(1,4);2,324 (1,0);1,785(0,5);1,767(2,2);1,748(4,3);1,731(4,4);1,713(2,3);1,694(0,5);1,235(0,8);0,820(7,5);0,802(16,0);0,783(7,0);0,000(10,5);-0,008(0,4) |
| I-1-542: |
| HPLC-MS: logP = 2,22; Masse (m/z): 307,0 (M+H)⁺; ¹H-NMR(400,0 MHz, DMSO-D₆): |
| δ= 11,909(7,2);8,500(14,3);8,494(14,6);8,388(12,3);8,387(12,9);8,381(12,4);8,380(12,2);8,310(4,6);8,303(4,2);8,289(5,1);8,282(8,6);8,276(4 ,5);8,261(4,7);8,255(4,2);6,963(16,0);6,956(15,7);5,757(3,1);5,336(0,4);5,325(0,6);5,313(0,3);3,427(0,3);3,396(0,5);3,390(0,6);3,330(776,8); 2,710(0,6);2,680(0,9);2,676(1,8);2,671(2,4);2,667(1,7);2,662(0,8);2,547(94,3);2,524(8,4);2,511(137,0);2,507(265,0);2,502(344,8);2,497(250, |
| 9);2,493(121,2);2,407(0,5);2,381(0,5);2,338(0,8);2,333(1,6);2,329(2,2);2,324(1,6);2,320(0,7);2,027(0,6);2,009(1,1);1,989(1,1);1,973(0,5);1,4 54(0,4);1,336(2,3);1,298(1,0);1,258(2,2);1,249(4,7);1,235(5,0);1,187(0,3);1,153(0,3);1,148(0,4);0,870(0,5);0,854(1,6);0,836(0,6);0,008(1,1); 0,000(27,2);-0,009(1,0) |
| I-1-543: |
| HPLC-MS: logP = 2,00; Masse (m/z): 306,1 (M+H)⁺; ¹H-NMR(400,0 MHz, DMSO-D₆): |
| δ= 11,494(2,0);8,684(4,8);8,496(2,5);8,490(2,7);8,357(2,3);8,351(2,4);8,305(0,7);8,299(0,7);8,284(0,8);8,278(1,4);8,272(0,8);8,257(0,8);8,2 51(0,7);6,941(2,6);6,935(2,6);3,331(62,7);3,329(60,7);2,672(0,3);2,507(42,4);2,503(54,1);2,498(39,8);2,329(0,3);2,281(16,0);0,000(6,9);-0,008(0,4) |
| I-1-544: |
| HPLC-MS: logP = 1,29; Masse (m/z): 306,1 (M+H)⁺; ¹H-NMR(400,0 MHz, CD₃CN): |
| δ= 9,678(0,5);8,326(3,3);8,286(2,0);8,280(2,0);8,229(1,8);8,222(1,8);7,725(0,7);7,719(0,7);7,705(0,8);7,699(1,4);7,692(0,7);7,678(0,7);7,67 2(0,7);6,996(2,4);6,989(2,3);3,973(16,0);2,135(21,1);2,107(0,4);1,964(4,1);1,958(6,2);1,952(26,9);1,946(46,9);1,940(60,9);1,934(41,5);1,92 7(21,2);1,768(0,4);0,008(1,1);0,000(31,0);-0,009(1,0) |
| I-1-545: |
| HPLC-MS: logP = 1,92; Masse (m/z): 337,0 (M+H)⁺; ¹H-NMR(400,0 MHz, DMSO-D₆): |
| δ= 11,690(6,9);9,260(15,6);9,147(16,0);8,494(6,7);8,488(7,1);8,403(6,4);8,397(6,5);8,316(0,9);8,299(2,1);8,292(1,9);8,278(2,2);8,271(3,9);8 ,265(2,1);8,250(2,1);8,244(2,0);6,995(7,9);6,988(7,9);5,756(1,4);3,324(317,6);2,675(1,7);2,671(2,4);2,666(1,8);2,565(0,8);2,541(1,2);2,524( 5,4);2,506(284,6);2,502(372,5);2,497(273,9);2,333(1,8);2,329(2,4);2,324(1,8);1,258(0,4);1,234(0,6);0,146(0,6);0,008(5,0);0,000(142,6);-0,008(5,5);-0,150(0,6) |
| I-1-546: |
| HPLC-MS: logP = 2,15; Masse (m/z): 323,1 (M+H)⁺; ¹H-NMR(400,0 MHz, DMSO-D₆): |
| δ= 11,269(1,9);8,496(2,2);8,490(2,3);8,377(1,9);8,370(1,9);8,304(0,7);8,298(0,7);8,283(0,8);8,277(1,4);8,271(0,7);8,256(0,8);8,250(0,7);6,9 96(1,8);6,990(1,8);3,403(0,8);3,322(72,1);3,033(0,8);2,910(1,1);2,903(16,0);2,891(1,0);2,732(0,7);2,675(0,4);2,671(0,5);2,666(0,4);2,524(1, 6);2,511(31,0);2,506(60,5);2,502(78,5);2,497(56,7);2,493(27,5);2,333(0,4);2,329(0,5);2,324(0,4);0,146(0,4);0,008(5,3);0,000(103,4);-0,009(4,1);-0,150(0,5) |
| I-1-547: |
| HPLC-MS: logP = 1,37; Masse (m/z): 306,1 (M+H)⁺; ¹H-NMR(400,0 MHz, DMSO-D₆): |
| δ= 12,237(1,0);8,385(1,0);8,379(1,0);8,198(1,5);8,179(1,5);8,167(0,7);8,160(1,2);8,153(1,0);8,146(0,4);8,140(0,3);6,961(1,5);6,942(1,4);6,8 51(1,1);6,844(1,1);3,313(1,4);3,157(13,5);3,096(0,3);2,725(1,6);2,485(6,8);2,481(8,5);2,477(6,4);1,995(5,6);0,961(0,5);-0,024(0,9) |
| I-1-548: |
| HPLC-MS: logP = 2,10; Masse (m/z): 321.1 (M+H)⁺; ¹H-NMR [DMSO-D₆] 1.76 - 1.82 (m, 2H), 1.98 (s, 3H), 2.31 - 2.34 (m, 2H), 3.94 - 3.97 (m, 2H), 6.88 (d, 1H), 8.21 - 8.26 (m, 2H), 8.46 (d, 1H), 10.15 (s, 1H). |
| I-1-549: |
| HPLC-MS: logP = 2,42; Masse (m/z): 411,1 (M+H)⁺; ¹H-NMR(400,0 MHz, DMSO-D₆): |
| δ= 11,425(0,5);11,359(11,9);8,586(0,6);8,580(0,8);8,567(8,5);8,563(9,0);8,555(9,0);8,551(8,7);8,364(8,6);8,360(8,4);8,344(9,2);8,340(8,5);8 ,218(14,5);8,212(14,1);7,836(6,8);7,817(8,2);7,783(2,4);7,766(6,9);7,747(6,3);7,711(5,6);7,692(16,0);7,673(7,7);7,477(8,9);7,465(8,7);7,457 (8,4);7,445(8,3);6,949(0,6);6,943(0,7);6,921(15,7);6,915(15,3);3,327(570,7);3,285(0,4);2,995(0,4);2,711(0,4);2,679(0,9);2,675(1,7);2,671(2, 3);2,666(1,7);2,662(0,8);2,541(107,5);2,524(7,5);2,510(143,4);2,506(276,2);2,502(354,1);2,497(253,6);2,493(121,1);2,367(0,5);2,333(1,7);2, 328(2,3);2,324(1,6);2,319(0,8);2,074(2,4);1,258(0,4);1,235(0,8);0,008(1,8);0,000(44,9);-0,009(1,5) |
| I-1-550: |
| HPLC-MS: logP = 2,34; Masse (m/z): 414,1 (M+H)⁺; ¹H-NMR(400,0 MHz, DMSO-D₆): |
| δ= 11,366(11,2);8,920(6,4);8,918(6,4);8,908(6,4);8,572(8,9);8,568(9,4);8,560(9,3);8,556(9,2);8,369(15,1);8,365(10,2);8,349(16,0);8,345(10, 5);8,244(14,2);8,238(14,1);7,804(4,4);7,802(4,5);7,790(4,5);7,783(4,2);7,782(4,1);7,771(4,0);7,770(3,9);7,482(9,8);7,471(9,3);7,462(9,1);7,4 51(9,1);6,929(15,9);6,922(15,7);3,453(0,3);3,427(0,4);3,420(0,5);3,396(0,8);3,335(1022,8);3,297(0,8);2,680(0,7);2,676(1,5);2,671(2,0);2,66 7(1,5);2,662(0,7);2,542(85,1);2,525(6,4);2,520(9,9);2,511(117,1);2,507(234,5);2,502(307,5);2,498(219,7);2,493(103,6);2,338(0,7);2,334(1,4 );2,329(2,0);2,325(1,4);2,320(0,6);2,074(1,3);1,298(0,3);1,258(0,5);1,235(0,8);0,008(0,3);0,000(10,8);-0,009(0,3) |
| I-1-551: |
| HPLC-MS: logP = 2,13; Masse (m/z): 379,0 (M+H)⁺; ¹H-NMR(400,0 MHz, DMSO-D₆): |
| δ= 11,650(0,4);11,583(10,1);8,603(0,5);8,597(0,5);8,570(9,0);8,566(9,5);8,559(9,5);8,555(9,3);8,368(9,3);8,364(9,2);8,348(10,1);8,344(9,2); 8,237(14,4);8,230(14,2);7,776(0,4);7,609(1,5);7,592(3,4);7,587(2,9);7,575(2,3);7,571(6,1);7,566(2,3);7,554(3,0);7,549(3,6);7,533(1,6);7,484 (10,1);7,473(9,6);7,464(9,4);7,453(9,5);7,250(1,7);7,247(2,0);7,240(11,0);7,220(14,6);7,200(8,9);7,192(1,6);6,957(0,5);6,950(0,6);6,931(16, 0);6,924(15,8);3,399(0,6);3,379(1,1);3,334(980,8);3,279(0,3);2,712(0,5);2,680(0,7);2,676(1,4);2,671(1,9);2,667(1,4);2,662(0,6);2,541(152,4) ;2,525(6,2);2,520(9,8);2,511(109,8);2,507(218,6);2,502(285,8);2,498(203,4);2,493(95,2);2,367(0,5);2,338(0,6);2,333(1,4);2,329(1,8);2,324(1 ,3);2,320(0,6);2,074(1,8);1,258(0,4);1,235(0,6);0,008(0,4);0,000(11,8);-0,009(0,4) |
| I-1-552: |
| HPLC-MS: logP = 2,31; Masse (m/z): 423,0 (M+H)⁺; ¹H-NMR(400,0 MHz, DMSO-D₆): |
| δ= 11,326(0,5);11,260(12,8);8,586(0,6);8,579(0,8);8,566(8,7);8,562(9,5);8,555(9,2);8,551(9,3);8,362(8,7);8,359(8,9);8,342(9,3);8,339(9,0);8 ,215(14,7);8,208(14,7);7,773(0,4);7,752(0,4);7,704(8,3);7,701(8,6);7,684(9,7);7,682(9,7);7,548(5,6);7,544(6,7);7,530(9,8);7,525(10,1);7,489 (4,9);7,486(5,3);7,476(10,4);7,471(10,5);7,468(11,0);7,465(11,2);7,456(9,5);7,452(6,2);7,449(5,7);7,445(9,2);7,433(0,7);7,422(6,1);7,418(6, 2);7,403(7,3);7,398(7,2);7,384(3,4);7,379(3,1);6,969(0,6);6,962(0,6);6,940(16,0);6,934(15,9);3,330(609,4);2,995(0,9);2,711(1,4);2,675(1,3); |
| 2,671(1,8);2,666(1,3);2,662(0,7);2,586(0,3);2,541(354,6);2,524(6,4);2,511(101,4);2,506(202,1);2,502(265,6);2,497(194,3);2,493(95,7);2,367 (1,4);2,337(0,6);2,333(1,3);2,328(1,7);2,324(1,3);2,320(0,6);2,074(8,2);1,258(0,4);1,235(0,9);0,008(0,8);0,000(23,2);-0,009(0,8) |
| I-1-553: |
| HPLC-MS: logP = 2,23; Masse (m/z): 379,0 (M+H)⁺; ¹H-NMR(400,0 MHz, DMSO-D₆): |
| δ= 11,344(0,5);11,278(13,1);8,588(0,6);8,581(0,7);8,567(8,9);8,563(9,5);8,555(9,3);8,551(9,3);8,363(9,0);8,359(9,0);8,343(9,7);8,339(9,1);8 ,216(15,3);8,210(15,1);7,775(0,4);7,754(0,4);7,579(6,9);7,575(7,5);7,561(9,4);7,556(10,3);7,550(6,5);7,533(11,5);7,530(11,9);7,510(4,9);7,5 06(5,3);7,492(8,8);7,488(7,7);7,477(10,2);7,472(5,9);7,466(10,9);7,457(9,6);7,445(15,3);7,430(8,7);7,427(8,4);7,412(3,2);7,409(3,0);6,972(0 ,5);6,965(0,6);6,943(16,0);6,937(15,7);3,438(0,3);3,421(0,4);3,413(0,5);3,333(1101,5);3,280(0,4);2,995(0,9);2,711(2,1);2,675(1,8);2,671(2,4 );2,666(1,7);2,662(0,9);2,541(484,3);2,524(8,6);2,511(141,2);2,506(277,7);2,502(362,3);2,497(261,9);2,493(126,5);2,367(2,1);2,337(0,8);2,3 33(1,7);2,329(2,3);2,324(1,7);2,320(0,8);2,074(2,5);1,258(0,4);1,235(0,9);0,008(0,5);0,000(14,9);-0,008(0,5) |
| I-1-554: |
| HPLC-MS: logP = 2,42; Masse (m/z): 471,0 (M+H)⁺; ¹H-NMR(400,0 MHz, DMSO-D₆): |
| δ= 11,199(7,6);8,585(0,3);8,579(0,4);8,567(5,4);8,563(5,8);8,555(5,7);8,551(5,6);8,363(5,4);8,359(5,4);8,343(5,8);8,339(5,4);8,215(9,0);8,2 08(8,9);7,923(6,0);7,903(7,0);7,501(0,9);7,499(0,9);7,481(5,6);7,479(6,3);7,475(12,3);7,465(16,0);7,455(6,9);7,444(5,5);7,243(0,4);7,231(2, 9);7,223(2,8);7,217(2,8);7,212(3,2);7,209(3,0);7,203(2,5);7,197(2,7);7,189(2,3);6,938(9,8);6,932(9,6);3,328(489,1);2,680(0,6);2,675(1,2);2,6 71(1,6);2,666(1,2);2,662(0,6);2,541(65,9);2,524(5,0);2,519(7,7);2,511(94,1);2,506(189,5);2,501(249,3);2,497(178,3);2,492(84,3);2,337(0,6); 2,333(1,2);2,328(1,6);2,324(1,2);2,319(0,5);2,074(2,3);1,258(0,3);1,235(0,6);0,008(1,1);0,000(36,1);-0,009(1,1) |
| I-1-555: |
| HPLC-MS: logP = 3,37; Masse (m/z): 384,1 (M+H)⁺; ¹H-NMR(400,0 MHz, DMSO-D₆): |
| δ= 11,405(6,5);11,339(0,5);8,234(3,6);8,227(6,7);8,221(3,6);8,203(0,6);8,197(0,6);7,842(3,9);7,822(4,9);7,789(1,4);7,771(3,9);7,752(5,2);7, 730(4,1);7,718(3,7);7,714(3,7);7,709(3,1);7,697(8,6);7,678(4,4);7,616(1,8);7,612(1,9);7,596(4,0);7,579(2,3);7,575(2,1);7,521(0,4);7,518(0,4) ;7,502(0,5);7,498(0,4);7,401(2,5);7,398(2,5);7,381(4,2);7,378(4,3);7,361(1,9);7,357(1,9);7,282(0,6);6,963(7,5);6,957(7,5);6,927(0,6);6,921(0 ,6);5,757(16,0);3,813(0,7);3,795(0,7);3,657(0,8);3,325(48,1);2,676(0,4);2,671(0,6);2,667(0,5);2,507(68,9);2,502(87,7);2,498(64,8);2,333(0,4 );2,329(0,6);2,324(0,4);1,336(0,6);1,259(0,4);1,250(0,7);1,234(1,0);1,216(1,5);1,199(0,7);0,008(1,7);0,000(31,2);-0,008(1,5) |
| I-1-556: |
| HPLC-MS: logP = 3,12; Masse (m/z): 352,1 (M+H)⁺; ¹H-NMR(400,0 MHz, DMSO-D₆): |
| δ= 11,624(11,3);11,560(1,0);8,315(0,4);8,252(6,4);8,245(11,9);8,239(6,8);8,210(1,2);8,203(1,1);7,752(3,3);7,749(3,4);7,731(6,9);7,714(4,0); 7,710(3,8);7,625(3,2);7,621(3,5);7,615(2,5);7,605(7,6);7,601(6,8);7,594(5,0);7,588(5,6);7,584(5,7);7,577(7,2);7,560(3,7);7,556(3,9);7,540(1, 7);7,532(1,0);7,511(1,0);7,405(4,5);7,402(4,4);7,385(7,8);7,382(7,5);7,364(3,7);7,361(3,5);7,307(0,7);7,296(0,3);7,286(1,0);7,265(0,7);7,245 (9,9);7,224(16,0);7,204(8,9);6,968(12,9);6,961(13,0);6,933(1,2);6,927(1,1);5,756(1,5);3,819(0,4);3,802(1,2);3,784(1,3);3,766(0,4);3,646(1,3) ;3,323(181,9);2,671(1,8);2,667(1,4);2,506(221,1);2,502(274,8);2,498(211,3);2,329(1,8);2,324(1,4);1,336(1,1);1,298(0,6);1,259(0,8);1,250(1, 2);1,235(0,5);1,223(1,4);1,205(2,8);1,188(1,5);0,146(0,4);0,000(78,6);-0,150(0,4) |
| I-1-557: |
| HPLC-MS: logP = 3,31; Masse (m/z): 394,0 (M+H)⁺; ¹H-NMR(400,0 MHz, DMSO-D₆): |
| δ= 11,303(15,1);11,241(1,2);8,316(0,3);8,232(8,0);8,226(14,6);8,219(7,9);8,202(1,6);8,196(1,4);7,751(4,3);7,747(4,6);7,730(9,0);7,709(15,2 );7,689(12,2);7,662(0,5);7,644(0,5);7,628(0,4);7,615(4,2);7,611(4,4);7,594(9,1);7,578(5,7);7,574(4,9);7,551(6,6);7,547(7,2);7,532(12,0);7,52 8(11,9);7,491(6,3);7,475(11,5);7,473(11,6);7,456(6,1);7,454(6,0);7,428(7,2);7,424(7,5);7,409(9,5);7,404(12,0);7,390(4,7);7,382(10,7);7,379( 10,2);7,361(4,5);7,358(4,6);7,304(0,8);7,292(0,4);7,283(1,2);7,263(0,6);6,980(16,0);6,974(15,8);6,946(1,5);6,940(1,3);5,757(1,3);3,826(0,5); 3,809(1,5);3,791(1,6);3,774(0,5);3,654(1,6);3,324(136,1);2,671(1,8);2,506(212,6);2,502(265,7);2,498(197,4);2,333(1,2);2,329(1,6);1,352(0,7 );1,298(0,5);1,259(0,8);1,234(3,1);1,217(3,6);1,199(1,6);1,188(0,3);0,146(0,5);0,000(81,9);-0,150(0,4) |
| I-1-558: |
| HPLC-MS: logP = 2,64; Masse (m/z): 395,0 (M+H)⁺; ¹H-NMR(400,0 MHz, DMSO-D₆): |
| δ= 11,483(14,0);11,419(1,4);10,742(0,4);10,678(0,5);8,489(8,8);8,484(9,8);8,477(9,5);8,472(9,4);8,316(0,5);8,251(7,6);8,244(14,3);8,238(7, 4);8,215(1,6);8,209(1,6);8,147(0,3);8,140(0,6);8,109(0,6);8,102(0,7);8,002(9,0);7,998(9,1);7,984(10,1);7,979(9,4);7,751(3,9);7,747(4,2);7,72 9(7,8);7,712(4,6);7,708(4,7);7,623(3,9);7,619(4,1);7,602(9,1);7,599(6,1);7,586(5,4);7,582(5,6);7,572(10,0);7,560(9,6);7,553(9,3);7,541(8,7); 7,529(1,1);7,525(1,0);7,508(1,2);7,505(1,1);7,480(0,5);7,476(0,5);7,406(5,3);7,403(5,3);7,386(9,1);7,382(8,9);7,365(4,4);7,362(4,2);7,344(0, 4);7,337(0,4);7,316(0,4);7,307(1,0);7,287(1,6);7,271(1,0);7,267(0,8);7,250(0,5);7,186(0,4);6,983(16,0);6,977(15,8);6,956(0,5);6,949(1,7);6,9 42(1,5);6,836(0,5);6,829(0,5);6,797(0,7);6,791(0,6);5,757(13,4);3,824(0,6);3,806(1,7);3,789(1,8);3,777(0,5);3,771(0,7);3,759(10);3,742(10) ;3,651(1,9);3,628(0,5);3,323(238,3);3,288(0,4);2,676(1,8);2,671(2,4);2,667(1,8);2,511(154,4);2,506(289,1);2,502(367,7);2,497(266,1);2,455( 1,1);2,439(0,7);2,417(0,8);2,333(1,9);2,329(2,4);2,324(1,7);2,044(3,1);2,038(3,4);1,989(0,4);1,351(0,5);1,336(4,0);1,298(1,6);1,259(2,3);1,2 50(5,0);1,235(3,2);1,230(3,0);1,212(4,2);1,194(2,8);1,176(2,5);1,158(1,2);0,854(0,4);0,146(0,7);0,008(6,8);0,000(140,6);-0,009(5,2);-0,150(0,6) |
| I-1-559: |
| HPLC-MS: logP = 3,30; Masse (m/z): 350,0 (M+H)⁺; ¹H-NMR(400,0 MHz, DMSO-D₆): |
| δ= 11,319(14,4);11,258(0,5);8,316(0,9);8,234(7,7);8,227(14,6);8,221(7,7);8,201(0,7);7,751(3,9);7,747(4,3);7,730(8,1);7,71 3(4,5);7,709(4,6); 7,616(4,1);7,612(4,2);7,595(9,0);7,592(6,1);7,578(12,9);7,564(10,2);7,559(14,1);7,538(12,7);7,535(13,6);7,516(5,6);7,512(6,0);7,498(9,8);7, 494(8,6);7,478(5,2);7,474(4,4);7,453(7,6);7,450(7,8);7,435(9,6);7,431(9,7);7,416(3,8);7,413(3,8);7,403(5,5);7,399(5,6);7,382(9,1);7,379(9,4) ;7,362(4,1);7,358(4,2);7,304(0,4);7,292(0,4);7,284(0,5);6,982(16,0);6,976(15,8);6,955(0,4);6,949(0,8);6,942(0,5);5,757(2,5);4,038(0,4);4,02 0(0,4);3,805(0,6);3,788(0,6);3,651(1,8);3,321(196,8);2,890(1,0);2,731(0,8);2,675(3,0);2,671(4,1);2,666(3,0);2,662(1,5);2,524(12,9);2,510(23 7,3);2,506(468,1);2,502(612,8);2,497(446,5);2,493(218,2);2,333(2,9);2,328(4,0);2,324(2,9);1,989(1,4);1,398(0,8);1,351(1,2);1,298(0,8);1,25 9(1,2);1,231(2,3);1,213(1,6);1,196(0,8);1,175(0,8);1,157(0,5);0,146(1,5);0,008(12,2);0,000(323,3);-0,008(10,9);-0,150(1,5) |
| I-1-560: |
| HPLC-MS: logP = 3,41; Masse (m/z): 435,0 (M+H)⁺; ¹H-NMR(400,0 MHz, CD₃CN): |
| δ= 9,493(3,5);8,735(8,9);8,732(9,0);8,320(8,7);8,316(8,8);8,296(11,0);8,289(11,2);7,803(5,4);7,784(6,7);7,726(1,4);7,716(1,4);7,707(5,2);7, 693(7,5);7,690(7,1);7,679(16,0);7,662(6,3);7,659(6,0);7,657(6,0);7,642(1,7);7,639(1,7);7,065(11,6);7,058(11,6);5,448(2,1);2,154(32,6);2,10 8(0,4);1,972(0,4);1,965(2,5);1,959(3,2);1,953(23,3);1,947(43,6);1,941 (60,9);1,935(42,8);1,929(22,5);1,770(0,4);1,372(4,3);1,340(0,4);1,285( 0,6);1,276(4,8);0,008(0,6);0,000(17,9);-0,009(0,8) |
| I-1-561: |
| HPLC-MS: logP = 3,30; Masse (m/z): 401,0 (M+H)⁺; ¹H-NMR(400,0 MHz, CD₃CN): |
| δ= 9,455(4,7);8,734(11,2);8,732(11,3);8,321(10,4);8,317(10,4);8,299(11,4);8,292(11,5);7,592(6,4);7,576(7,4);7,573(7,6);7,499(1,8);7,495(3, 1);7,475(16,0);7,461(9,3);7,456(8,4);7,441(3,3);7,436(3,0);7,426(0,5);7,414(7,0);7,408(6,0);7,395(7,2);7,390(6,7);7,378(3,6);7,373(3,3);7,08 9(11,0);7,083(11,0);5,448(1,8);2,144(27,6);2,115(0,4);2,108(0,6);2,102(0,4);1,965(3,7);1,959(4,4);1,953(34,0);1,947(63,6);1,941(88,7);1,93 5(61,8);1,929(32,2);1,776(0,4);1,769(0,5);1,763(0,3);1,372(4,6);1,340(0,4);1,285(0,6);1,276(5,0);0,008(0,9);0,000(27,8);-0,009(1,1) |
| I-1-562: |
| HPLC-MS: logP = 1,98; Masse (m/z): 368,1 (M+H)⁺; ¹H-NMR(400,0 MHz, DMSO-D₆): |
| δ= 11,547(12,6);8,845(7,5);8,835(7,5);8,536(8,5);8,532(8,8);8,524(8,9);8,521(8,7);8,316(0,8);8,287(15,4);8,281(15,3);8,221(9,6);8,217(13,5 );8,201(10,6);8,197(15,0);7,838(6,3);7,826(6,3);7,818(5,9);7,806(5,5);7,556(8,8);7,544(8,5);7,536(8,2);7,524(8,0);6,943(16,0);6,937(15,6);3, 322(94,2);2,675(1,7);2,671(2,2);2,667(1,6);2,506(258,7);2,502(327,5);2,497(237,6);2,333(1,6);2,329(2,1);2,324(1,5);2,075(0,5);0,146(0,8);0 ,008(7,7);0,000(166,2);-0,008(6,4);-0,149(0,8) |
| I-1-563: |
| HPLC-MS: Masse (m/z): 317,0 (M+H)⁺; ¹H-NMR(400,0 MHz, DMSO-D₆): |
| δ= 10,546(3,9);8,526(2,7);8,522(2,8);8,515(2,9);8,511(2,8);8,214(7,0);8,208(5,0);8,195(3,0);8,191(2,8);7,542(2,8);7,530(2,7);7,522(2,6);7,5 10(2,5);6,919(4,8);6,913(4,8);6,807(4,5);3,902(1,6);3,347(1,2);3,328(152,1);2,676(0,5);2,671(0,7);2,667(0,5);2,524(22,8);2,511(45,2);2,507( 91,4);2,502(119,1);2,498(85,3);2,493(41,2);2,333(0,5);2,329(0,7);2,324(0,5);2,238(16,0) |
| I-1-564: |
| HPLC-MS: logP = 2,12; Masse (m/z): 335,1 (M+H)⁺; ¹H-NMR(400,0 MHz, DMSO-D₆): |
| δ= 13,913(0,7);11,602(10,9);8,537(7,9);8,533(7,8);8,525(8,3);8,521(7,8);8,316(0,7);8,283(13,0);8,277(13,0);8,222(8,0);8,218(7,7);8,202(8,6 );8,198(7,8);7,608(1,5);7,592(3,5);7,587(3,3);7,571(6,2);7,558(9,3);7,547(9,0);7,538(8,0);7,533(2,4);7,526(7,6);7,239(10,1);7,219(16,0);7,19 9(9,8);7,178(0,9);6,945(13,7);6,938(13,6);5,756(0,7);3,322(32,0);2,675(1,7);2,671(2,2);2,666(1,5);2,506(269,1);2,502(337,6);2,497(244,0);2 ,437(0,5);2,333(1,8);2,329(2,2);1,337(0,4);1,299(0,4);259(0,5);1,250(0,6);1,234(0,4);1,146(0,3);0,000(2,0) |
| I-1-565: |
| HPLC-MS: logP = 1,68; Masse (m/z): 377,9 (M+H)⁺; ¹H-NMR(400,0 MHz, DMSO-D₆): |
| δ= 11,458(11,7);8,535(8,6);8,532(9,0);8,524(9,0);8,520(8,8);8,481(8,0);8,476(8,6);8,469(8,5);8,464(8,3);8,315(2,2);8,282(15,0);8,275(14,8); 8,220(8,9);8,216(8,8);8,200(9,6);8,196(9,0);8,002(8,2);7,997(8,4);7,983(9,3);7,978(8,7);7,565(9,0);7,555(13,2);7,546(9,9);7,543(10,4);7,535 (16,0);7,523(8,7);6,959(15,8);6,953(15,6);3,321(223,6);2,675(3,2);2,671(4,4);2,666(3,2);2,662(1,5);2,524(12,4);2,511(257,8);2,506(517,6);2 ,502(678,7);2,497(489,0);2,493(234,8);2,337(1,6);2,333(3,3);2,328(4,5);2,324(3,2);0,146(0,7);0,008(5,8);0,000(175,4);-0,008(6,3);-0,150(0,8) |
| I-1-566: |
| HPLC-MS: logP = 2,46; Masse (m/z): 431,0 (M+H)⁺; ¹H-NMR(400,0 MHz, DMSO-D₆): |
| δ= 10,988(9,2);8,536(8,0);8,532(8,7);8,524(8,6);8,520(8,7);8,316(1,3);8,251(13,5);8,244(13,7);8,222(8,1);8,218(8,3);8,202(8,9);8,198(8,6);7 ,799(15,0);7,785(16,0);7,554(9,0);7,542(8,8);7,534(8,5);7,522(8,4);7,396(10,0);7,382(9,5);6,939(6,0);6,934(6,0);4,038(0,8);4,020(0,8);3,321 (134,1);2,680(1,5);2,675(3,2);2,671(4,4);2,666(3,3);2,662(1,6);2,524(16,2);2,511(257,4);2,506(514,2);2,502(678,5);2,497(492,5);2,493(241, 0);2,337(1,6);2,333(3,2);2,328(4,4);2,324(3,2);1,989(3,7);1,236(0,6);1,193(1,0);1,175(2,0);1,157(1,1);0,000(7,8) |
| I-1-567: |
| HPLC-MS: logP = 3,15; Masse (m/z): 386,0 (M+H)⁺; ¹H-NMR(400,0 MHz, DMSO-D₆): |
| δ= 11,570(11,4);8,542(10,5);8,535(10,7);7,837(5,6);7,819(7,8);7,788(2,0);7,771(5,7);7,752(5,3);7,719(4,9);7,701(13,8);7,683(6,6);7,366(16, 0);7,029(11,0);7,022(11,1);4,102(0,4);4,089(0,4);3,326(149,7);3,176(1,4);3,163(1,4);2,699(0,4);2,692(0,4);2,675(0,7);2,671(0,9);2,667(0,7); 2,541(57,3);2,532(55,5);2,507(97,5);2,502(130,4);2,498(101,0);2,379(0,3);2,369(0,3);2,333(0,6);2,329(0,9);2,325(0,7);1,233(0,7);1,178(0,5) ;0,146(0,5);0,008(4,4);0,000(103,4);-0,150(0,5) |
| I-1-568 siehe Synthesebeispiel 36 |
| I-1-569 siehe Synthesebeispiel 31 |
| I-1-570 siehe Synthesebeispiel 40 |
| I-1-571 siehe Synthesebeispiel 33 |
| I-1-572: |
| HPLC-MS: logP = 2,92; Masse (m/z): 372,1 (M+H)⁺; ¹H-NMR(400,0 MHz, DMSO-D₆): |
| δ= 11,595(3,4);8,578(3,2);8,572(3,2);8,322(3,3);8,302(3,4);7,839(1,6);7,820(2,4);7,790(0,6);7,772(1,7);7,753(1,7);7,720(1,5);7,705(3,7);7,6 86(1,8);7,406(3,3);7,386(3,1);7,047(3,3);7,040(3,3);3,326(4,9);2,595(16,0);2,507(13,0);2,503(16,3);2,499(11,9);0,007(0,6);0,000(11,1);-0,001(10,5);-0,008(0,5) |
| I-1-573 siehe Synthesebeispiel 39 |
| I-1-574 siehe Synthesebeispiel 43 |
| I-1-575 siehe Synthesebeispiel 25 |
| I-1-576: |
| HPLC-MS: logP = 2,96; Masse (m/z): 476,9 (M+H)⁺; ¹H-NMR(400,0 MHz, CD₃CN): |
| δ= 9,540(1,7);8,293(4,0);8,287(3,9);7,929(4,7);7,917(5,4);7,777(2,3);7,769(4,2);7,759(7,3);7,747(2,9);7,691(0,6);7,674(1,8);7,658(3,8);7,65 4(3,9);7,648(3,9);7,636(4,7);7,623(1,9);7,050(4,8);7,043(4,6);5,447(16,0);2,156(18,3);1,972(1,1);1,964(1,0);1,958(2,3);1,953(9,0);1,946(15, 6);1,940(19,8);1,934(13,8);1,928(7,1);1,203(0,5);1,172(0,4);1,006(0,4);0,000(23,8) |
| I-1-577: |
| HPLC-MS: logP = 2,33; Masse (m/z): 368,0 (M+H)⁺; ¹H-NMR(400,0 MHz, DMSO-D₆): |
| δ= 11,571(13,2);8,482(8,3);8,478(9,1);8,470(9,0);8,465(8,9);8,446(13,0);8,439(12,8);8,428(0,5);8,420(0,3);8,384(5,7);8,381(9,4);8,372(6,1); 8,370(9,5);8,233(8,7);8,228(8,7);8,214(9,6);8,210(8,8);8,030(4,6);8,027(4,5);8,010(5,2);8,006(5,1);8,001(4,9);7,998(4,8);7,981(4,9);7,978(4, 8);7,555(9,0);7,543(9,0);7,536(8,9);7,524(10,1);7,518(6,2);7,515(5,6);7,506(8,5);7,497(4,9);7,494(4,9);7,486(4,1);7,012(16,0);7,006(15,7);4, 118(0,4);4,104(1,1);4,091(1,2);4,078(0,4);3,330(51,8);3,179(5,5);3,166(5,4);2,679(0,4);2,674(0,5);2,670(0,4);2,545(0,4);2,528(1,4);2,514(30 ,4);2,510(61,4);2,505(80,3);2,501 (56,9);2,496(26,4);2,337(0,4);2,332(0,5);2,327(0,4);1,232(0,4);1,160(0,4);1,142(0,8);1,125(0,4);1,025(0,4); 1,007(0,8);0,989(0,4);0,146(0,5);0,015(0,4);0,008(4,2);0,000(114,1);-0,009(3,7);-0,150(0,5) |
| I-1-578: |
| HPLC-MS: logP = 1,57; Masse (m/z): 363,9 (M+H)⁺; ¹H-NMR(400,0 MHz, DMSO-D₆): |
| δ= 11,543(12,3);8,482(9,1);8,477(9,8);8,470(9,8);8,465(9,5);8,441(12,7);8,434(12,6);8,380(5,5);8,377(9,2);8,374(5,6);8,368(5,9);8,365(9,3); 8,316(1,5);8,027(4,6);8,023(4,6);8,006(6,3);8,003(13,9);7,998(14,0);7,995(5,9);7,984(10,6);7,979(11,7);7,974(5,3);7,565(10,0);7,553(9,7);7, 546(9,4);7,534(9,3);7,522(5,0);7,514(5,6);7,511(5,3);7,502(8,5);7,493(4,7);7,490(4,7);7,482(4,1);7,007(16,0);7,000(15,9);5,756(6,5);3,365(0 ,4);3,322(277,4);3,102(0,4);3,083(0,4);2,675(3,4);2,671(4,6);2,666(3,3);2,662(1,6);2,524(14,5);2,511(265,0);2,506(529,4);2,502(695,0);2,49 7(498,0);2,493(238,6);2,337(1,6);2,333(3,3);2,328(4,5);2,324(3,2);2,319(1,5);1,790(1,0);1,236(0,5);0,146(0,8);0,008(7,6);0,000(221,1);-0,009(7,8);-0,150(0,9) |
| I-1-579: |
| HPLC-MS: logP = 1,53; Masse (m/z): 318,1 (M+H)⁺; ¹H-NMR(400,0 MHz, CD₃CN): |
| δ= 9,529(4,4);8,463(9,7);8,459(9,8);8,451(10,4);8,447(9,6);8,313(15,0);8,307(15,8);8,298(12,4);8,286(11,8);7,969(9,9);7,965(9,5);7,950(10, 8);7,946(10,0);7,750(5,7);7,748(5,1);7,729(6,8);7,727(6,6);7,722(6,4);7,701(6,3);7,698(5,6);7,429(10,0);7,417(10,4);7,410(10,0);7,398(9,3); 7,389(5,9);7,380(7,5);7,378(6,6);7,369(9,9);7,360(6,6);7,357(5,9);7,349(4,7);7,049(16,0);7,042(15,8);5,448(2,6);2,149(37,6);2,114(0,4);2,10 8(0,4);1,965(2,4);1,953(23,6);1,947(43,1);1,941(59,2);1,934(41,4);1,928(21,5);1,769(0,3);0,000(4,0) |
| I-1-580: |
| HPLC-MS: logP = 2,36; Masse (m/z): 369,1 (M+H)⁺; ¹H-NMR(400,0 MHz, DMSO-D₆): |
| δ= 14,226(0,5);11,712(9,7);8,441(9,1);8,434(9,2);8,377(6,5);8,366(6,6);8,026(3,2);8,023(3,2);8,005(3,6);8,002(3,7);7,997(3,5);7,994(3,4);7, 976(3,5);7,973(3,4);7,772(0,8);7,753(2,6);7,744(1,9);7,734(5,4);7,719(8,8);7,697(16,0);7,680(4,8);7,675(4,5);7,523(3,1);7,514(3,9);7,512(3, 8);7,502(5,5);7,493(3,4);7,491(3,4);7,482(2,7);6,979(10,5);6,972(10,5);5,757(1,1);3,324(6,8);2,672(0,8);2,507(108,0);2,503(137,3);2,498(10 3,0);2,329(0,9);0,000(0,8) |
| I-1-581: |
| HPLC-MS: logP = 2,30; Masse (m/z): 431,9 (M+H)⁺; ¹H-NMR(400,0 MHz, DMSO-D₆): |
| δ= 11,070(6,4);8,405(5,8);8,399(5,9);8,377(4,8);8,368(3,2);8,365(4,9);8,025(2,5);8,021(2,5);8,004(2,8);8,001(2,7);7,996(2,6);7,992(2,5);7,9 75(2,7);7,972(2,6);7,801(9,5);7,794(0,7);7,787(10,1);7,517(2,5);7,509(3,0);7,506(2,8);7,497(4,4);7,488(2,5);7,485(2,5);7,477(2,1);7,416(7,3) ;7,402(6,9);7,001(3,8);6,995(3,8);5,757(0,6);4,117(1,2);4,104(3,4);4,091(3,5);4,078(1,2);3,331(12,5);3,179(16,0);3,166(15,4);2,527(0,8);2,5 14(11,5);2,509(22,3);2,505(29,0);2,500(21,1);2,496(10,3);0,000(1,6) |
| I-1-582: |
| HPLC-MS: logP = 2,16; Masse (m/z): 398,9 (M+H)⁺; ¹H-NMR(400,0 MHz, DMSO-D₆): |
| δ= 10,989(9,3);8,406(8,9);8,400(8,9);8,382(4,2);8,379(7,0);8,376(4,3);8,370(4,4);8,367(7,1);8,365(4,1);8,031(3,4);8,028(3,4);8,011(3,8);8,0 07(3,8);8,003(3,6);7,999(3,5);7,982(3,7);7,979(3,6);7,938(14,8);7,934(14,8);7,523(3,8);7,514(4,3);7,511(4,0);7,502(6,5);7,493(3,5);7,490(3, 6);7,482(3,1);6,953(11,7);6,946(11,6);6,921(16,0);6,917(15,5);5,761(3,4);3,339(7,6);2,531(0,5);2,518(9,6);2,513(19,2);2,509(25,2);2,504(18 ,1);2,500(8,6);0,000(5,2) |
| I-1-583 siehe Synthesebeispiel 42 |
| I-1-584 siehe Synthesebeispiel 30 |
| I-1-585 siehe Synthesebeispiel 37 |
| I-1-586 siehe Synthesebeispiel 38 |
| I-1-587: |
| HPLC-MS: logP = 2,91; Masse (m/z): 444,8 (M+H)⁺; ¹H-NMR(400,0 MHz, DMSO-D₆): |
| δ= 11,489(2,0);8,471(1,9);8,467(2,0);8,411(1,8);8,404(1,7);8,372(1,2);8,367(1,1);8,345(1,2);8,340(1,1);7,836(1,0);7,817(1,4);7,785(0,4);7,7 67(1,1);7,748(1,0);7,715(0,9);7,697(2,5);7,679(1,2);7,009(2,1);7,003(2,1);5,757(16,0);3,330(1,8);2,509(5,8);2,505(7,3);2,500(5,3);1,397(0,9) |
| ;0,000(3,0) |
| I-1-588: |
| HPLC-MS: logP = 2,60; Masse (m/z): 448,8 (M+H)⁺; ¹H-NMR(400,0 MHz, DMSO-D₆): |
| δ= 11,712(6,0);8,477(6,3);8,472(7,1);8,432(5,7);8,425(5,7);8,383(4,4);8,378(3,9);8,356(4,3);8,350(4,0);7,612(0,8);7,596(1,7);7,591(1,5);7,5 79(1,2);7,574(3,2);7,570(1,2);7,558(1,6);7,553(1,9);7,537(0,8);7,247(1,0);7,240(5,6);7,220(7,8);7,199(4,7);7,192(1,0);7,010(7,4);7,003(7,4); 5,757(16,0);4,101(0,4);4,088(0,5);3,324(30,2);3,176(1,9);3,163(2,0);2,676(0,4);2,672(0,5);2,667(0,4);2,525(1,7);2,520(2,6);2,512(29,9);2,50 7(59,8);2,503(78,4);2,498(56,2);2,494(26,7);2,334(0,4);2,329(0,5);2,325(0,4);1,145(0,3);0,000(2,4) |
| I-1-589: |
| HPLC-MS: logP = 2,78; Masse (m/z): 351,0 (M+H)⁺; ¹H-NMR(400,0 MHz, DMSO-D₆): |
| δ= 11,387(6,8);8,470(6,6);8,465(6,6);8,408(6,1);8,402(5,8);8,371(4,0);8,366(3,4);8,344(3,9);8,339(3,4);7,703(4,6);7,683(5,2);7,553(3,0);7,5 48(3,1);7,534(5,0);7,530(4,7);7,489(2,6);7,470(5,1);7,452(2,8);7,425(3,2);7,420(2,9);7,405(3,9);7,401(3,5);7,386(1,7);7,382(1,4);7,027(7,0); 7,020(6,6);5,758(16,0);3,328(4,0);2,508(21,8);2,504(26,0);2,500(18,4);0,000(6,3) |
| I-1-590: |
| HPLC-MS: logP = 2,74; Masse (m/z): 442,9 (M+H)⁺; ¹H-NMR(400,0 MHz, DMSO-D₆): |
| δ= 11,404(5,5);8,470(5,5);8,465(5,9);8,410(4,9);8,403(4,8);8,372(3,5);8,367(3,1);8,345(3,5);8,340(3,2);7,585(2,7);7,581(2,9);7,566(3,6);7,5 62(3,7);7,552(2,1);7,549(2,4);7,532(4,8);7,529(4,9);7,513(2,2);7,508(2,3);7,495(3,7);7,490(3,1);7,475(2,1);7,470(1,7);7,448(2,9);7,445(2,8); 7,430(3,7);7,427(3,5);7,412(1,4);7,408(1,3);7,030(5,9);7,023(5,8);5,758(16,0);3,329(4,6);2,526(0,5);2,513(8,9);2,509(17,3);2,504(22,2);2,50 0(15,8);2,495(7,5);1,231(0,4);0,000(6,2) |
| I-1-591: |
| HPLC-MS: logP = 2,98; Masse (m/z): 353,1 (M+H)⁺; ¹H-NMR(400,0 MHz, DMSO-D₆): |
| δ= 11,746(4,7);8,475(4,8);8,470(5,4);8,433(4,3);8,426(4,3);8,377(3,2);8,372(2,9);8,350(3,2);8,344(3,0);7,778(0,4);7,758(1,1);7,748(0,7);7,7 38(2,3);7,723(3,2);7,700(7,0);7,683(2,0);7,678(1,9);7,005(5,5);6,998(5,5);5,759(16,0);3,333(3,1);2,529(0,3);2,516(5,9);2,511(11,7);2,507(15 ,3);2,502(11,1);2,498(5,4);1,992(0,5);1,396(2,0);0,000(0,5) |
| I-1-592: |
| HPLC-MS: logP = 2,99; Masse (m/z): 394,9 (M+H)⁺; ¹H-NMR(400,0 MHz, DMSO-D₆): |
| δ= 11,093(6,2);8,475(6,3);8,470(6,8);8,393(5,3);8,386(5,5);8,379(4,2);8,374(3,6);8,352(3,9);8,347(3,6);7,802(7,6);7,788(8,0);7,410(6,5);7,3 96(6,1);7,016(3,7);7,010(3,6);5,758(16,0);3,326(9,6);2,512(16,3);2,508(31,2);2,503(40,2);2,499(29,1);0,008(0,6);0,000(14,7);-0,008(0,5) |
| I-1-593: |
| HPLC-MS: logP = 2,89; Masse (m/z): 385,0 (M+H)⁺; ¹H-NMR(400,0 MHz, DMSO-D₆): |
| δ= 11,323(5,9);8,471(5,6);8,466(6,1);8,407(5,0);8,400(4,9);8,372(3,7);8,367(3,3);8,345(3,6);8,340(3,4);7,921(4,5);7,901(5,0);7,500(0,5);7,4 79(5,3);7,476(5,8);7,467(10,0);7,457(1,0);7,233(2,0);7,224(1,9);7,219(2,0);7,213(2,3);7,211(2,1);7,204(1,8);7,200(2,0);7,191(1,6);7,024(6,0) ;7,017(5,9);5,757(16,0);3,326(7,4);2,525(0,7);2,512(12,6);2,507(24,5);2,503(31,7);2,498(22,8);2,494(11,0);0,008(0,6);0,000(14,7);-0,009(0,4) |
| I-1-594: |
| HPLC-MS: logP = 2,34; Masse (m/z): 359,0 (M+H)⁺; ¹H-NMR(400,0 MHz, DMSO-D₆): |
| δ= 19,999(0,4);11,791(6,4);9,293(16,0);9,239(15,8);9,048(0,5);8,780(0,6);8,756(6,8);8,749(6,8);8,315(2,4);7,850(3,3);7,831(5,5);7,801(1,3); 7,783(3,6);7,764(3,7);7,734(3,9);7,722(5,6);7,705(3,3);7,159(6,4);7,151(6,2);5,756(6,5);3,319(151,2);2,675(5,7);2,670(7,7);2,666(5,7);2,540 (4,9);2,510(464,4);2,506(895,7);2,501(1158,0);2,497(833,7);2,492(404,2);2,332(5,3);2,328(7,3);2,323(5,3);0,146(1,9);0,008(20,7);0,000(408 ,1);-0,008(16,0);-0,150(1,8) |
| I-1-595: |
| HPLC-MS: logP = 2,03; Masse (m/z): 327,1 (M+H)⁺; ¹H-NMR(400,0 MHz, DMSO-D₆): |
| δ= 11,980(0,5);9,306(1,0);9,248(1,0);8,775(0,5);8,768(0,5);7,262(0,4);7,243(0,6);7,222(0,4);7,165(0,6);7,158(0,6);5,755(16,0);3,321(15,5);2 ,524(0,5);2,511(12,0);2,506(23,8);2,502(30,9);2,497(22,0);2,493(10,4);0,008(0,5);0,000(13,8);-0,009(0,5) |
| I-1-596: |
| HPLC-MS: logP = 2,88; Masse (m/z): 351,1 (M+H)⁺; ¹H-NMR(400,0 MHz, DMSO-D₆): |
| δ= 11,423(2,8);8,528(3,4);8,521(3,3);8,493(2,8);8,486(2,7);7,967(0,9);7,960(0,8);7,947(1,3);7,945(1,4);7,939(1,2);7,937(1,3);7,924(1,1);7,9 17(1,0);7,847(1,5);7,828(2,0);7,807(1,8);7,797(2,2);7,784(1,7);7,778(1,9);7,775(2,0);7,759(1,5);7,725(1,3);7,707(3,7);7,688(1,8);6,954(3,7); 6,948(3,5);4,119(1,3);4,106(3,5);4,093(3,5);4,079(1,2);3,334(16,7);3,179(16,0);3,166(15,0);2,514(7,1);2,509(12,3);2,505(15,2);2,500(10,6); 2,496(5,1);0,008(0,9);0,000(10,4);-0,009(0,4) |
| I-1-597: |
| HPLC-MS: logP = 2,32; Masse (m/z): 352,0 (M+H)⁺; ¹H-NMR(400,0 MHz, DMSO-D₆): |
| δ= 11,587(11,2);8,859(6,7);8,847(6,7);8,546(15,5);8,540(15,6);8,500(11,6);8,493(11,9);8,225(6,4);8,206(6,9);7,972(3,4);7,965(3,3);7,950(5, 5);7,943(5,4);7,929(4,5);7,922(4,3);7,851(6,0);7,839(6,0);7,832(5,7);7,820(5,4);7,805(7,1);7,796(7,4);7,783(5,6);7,773(5,4);6,959(16,0);6,95 3(16,0);5,758(1,7);4,102(0,7);4,089(0,7);3,327(36,1);3,178(2,8);3,165(2,7);2,678(0,4);2,673(0,5);2,669(0,4);2,543(0,3);2,526(1,4);2,513(31, 1);2,509(63,5);2,504(83,9);2,500(60,4);2,495(29,1);2,335(0,4);2,331(0,5);2,326(0,4);0,008(1,2);0,000(36,5);-0,008(1,3) |
| I-1-598: |
| HPLC-MS: logP = 2,61; Masse (m/z): 319,1 (M+H)⁺; ¹H-NMR(400,0 MHz, DMSO-D₆): |
| δ= 11,642(11,7);8,543(14,4);8,537(14,4);8,499(11,7);8,492(11,9);7,974(3,4);7,967(3,3);7,952(5,7);7,945(5,5);7,931(4,6);7,924(4,3);7,811(7, 0);7,801(7,3);7,788(5,5);7,779(5,3);7,622(1,5);7,605(3,4);7,601(3,2);7,584(6,3);7,567(3,4);7,563(3,8);7,547(1,7);7,257(2,3);7,251(10,9);7,23 |
| 1(16,0);7,210(9,2);7,203(2,1);6,958(15,5);6,951(15,4);3,324(79,5);2,676(0,7);2,672(0,9);2,668(0,7);2,542(0,5);2,525(2,3);2,512(57,2);2,507( 111,1);2,503(143,0);2,498(104,4);2,494(52,2);2,334(0,7);2,330(0,9);2,325(0,7);0,008(3,5);0,000(71,0);-0,008(3,5) |
| I-1-599: |
| HPLC-MS: logP = 2,78; Masse (m/z): 409,0 (M+H)⁺; ¹H-NMR(400,0 MHz, DMSO-D₆): |
| δ= 11,265(4,0);8,526(4,3);8,520(4,2);8,490(3,7);8,482(3,7);7,967(1,1);7,960(1,1);7,945(2,0);7,934(3,5);7,924(1,7);7,914(3,8);7,810(2,1);7,8 01(2,2);7,788(1,7);7,778(1,6);7,512(0,3);7,491(3,9);7,488(4,2);7,479(6,9);7,470(0,8);7,244(1,4);7,235(1,4);7,231(1,5);7,224(1,6);7,222(1,5); 7,215(1,3);7,212(1,5);7,202(1,2);6,974(4,6);6,967(4,4);5,757(16,0);3,330(7,3);2,513(5,9);2,509(11,4);2,504(14,7);2,500(10,3);2,495(4,9);0,0 00(3,6) |
| I-1-600: |
| HPLC-MS: logP = 2,13; Masse (m/z): 352,1 (M+H)⁺; ¹H-NMR(400,0 MHz, DMSO-D₆): |
| δ= 11,559(3,9);8,9382(16,0);8,9378(16,0);8,581(4,9);8,575(4,9);7,848(2,2);7,828(2,7);7,796(0,7);7,779(2,1);7,760(1,9);7,725(1,7);7,704(4,7 );7,685(2,3);6,999(5,3);6,992(5,3);5,758(2,3);3,327(25,0);2,526(0,6);2,521(0,9);2,512(11,2);2,508(22,5);2,503(29,7);2,499(21,3);2,494(10,0) ;0,008(0,4);0,000(13,6);-0,009(0,4) |
| I-1-601: |
| HPLC-MS: logP = 1,73; Masse (m/z): 353,0 (M+H)⁺; ¹H-NMR(400,0 MHz, DMSO-D₆): |
| δ= 11,734(3,8);8,944(16,0);8,861(1,9);8,859(2,1);8,849(2,0);8,847(2,0);8,598(4,8);8,591(4,8);8,223(1,8);8,221(1,9);8,203(2,1);8,201(2,1);7, 854(1,9);7,842(1,9);7,835(1,8);7,823(1,7);6,997(5,5);6,990(5,4);5,757(0,6);3,323(61,1);2,676(0,4);2,671(0,6);2,667(0,4);2,525(1,8);2,520(2, 8);2,511(30,7);2,507(61,6);2,502(81,3);2,498(58,4);2,493(27,4);2,334(0,4);2,329(0,5);2,324(0,4);0,000(7,4) |
| I-1-602: |
| HPLC-MS: logP = 1,83; Masse (m/z): 320,0 (M+H)⁺; ¹H-NMR(400,0 MHz, DMSO-D₆): |
| δ= 11,776(3,6);8,944(16,0);8,598(5,1);8,591(5,1);7,623(0,5);7,606(1,2);7,601(1,0);7,589(0,8);7,585(2,1);7,581(0,8);7,568(1,1);7,564(1,3);7, 547(0,6);7,258(0,7);7,251(3,8);7,231(5,4);7,211(3,1);7,204(0,6);7,000(5,6);6,994(5,5);3,324(29,4);2,672(0,4);2,525(1,3);2,512(20,6);2,507(4 0,1);2,503(52,3);2,498(38,0);2,494(18,3);2,330(0,3);0,008(0,8);0,000(20,5);-0,009(0,7) |
| I-1-603: |
| HPLC-MS: logP = 1,94; Masse (m/z): 362,0 (M+H)⁺; ¹H-NMR(400,0 MHz, DMSO-D₆): |
| δ= 11,446(3,9);8,937(16,0);8,580(4,1);8,573(4,0);7,713(2,6);7,712(2,7);7,694(3,1);7,692(3,1);7,560(1,7);7,556(1,8);7,541(2,9);7,537(2,9);7, 499(1,5);7,496(1,6);7,480(3,1);7,478(3,0);7,462(1,6);7,459(1,5);7,434(2,0);7,429(2,0);7,414(2,4);7,410(2,3);7,396(1,1);7,391(1,0);7,017(4,3) ;7,010(4,2);4,038(0,5);4,020(0,5);3,322(59,5);2,675(0,5);2,671(0,7);2,666(0,5);2,510(43,3);2,506(82,4);2,502(106,7);2,497(78,5);2,493(39,2 );2,333(0,6);2,328(0,7);2,324(0,5);1,989(2,3);1,193(0,6);1,175(1,2);1,157(0,6);0,008(0,4);0,000(8,2);-0,008(0,3) |
| I-1-604: |
| HPLC-MS: logP = 1,91; Masse (m/z): 318,0 (M+H)⁺; ¹H-NMR(400,0 MHz, DMSO-D₆): |
| δ= 11,469(3,9);8,937(16,0);8,582(4,2);8,576(4,1);7,593(2,1);7,590(2,2);7,575(2,8);7,571(2,8);7,562(1,8);7,559(1,9);7,542(3,9);7,540(3,8);7, 522(1,8);7,518(1,8);7,504(3,0);7,500(2,6);7,484(1,6);7,480(1,3);7,458(2,4);7,455(2,3);7,440(3,0);7,437(2,8);7,422(1,2);7,418(1,0);7,022(4,2) ;7,016(4,1);5,757(10,3);3,324(20,9);2,507(33,6);2,503(42,6);2,498(31,4);0,000(2,0) |
| I-1-605: |
| HPLC-MS: logP = 2,05; Masse (m/z): 410,0 (M+H)⁺; ¹H-NMR(400,0 MHz, DMSO-D₆): |
| δ= 11,386(3,8);9,032(1,4);8,936(16,0);8,907(2,2);8,580(3,9);8,573(3,9);7,933(3,2);7,913(3,7);7,510(0,4);7,508(0,4);7,491(3,0);7,489(3,4);7, 485(3,8);7,477(6,9);7,467(0,7);7,381(0,7);7,377(0,7);7,242(1,6);7,233(1,4);7,228(1,4);7,222(1,7);7,219(1,6);7,213(1,3);7,209(1,4);7,200(1,3) ;7,020(4,1);7,013(4,0);6,615(0,6);5,756(9,4);5,421(0,9);5,416(0,8);3,325(31,9);2,525(0,9);2,512(13,9);2,507(27,1);2,503(35,4);2,498(25,7);2 ,494(12,4);1,989(0,9);1,175(0,5);0,000(2,2) |
| I-1-606 siehe Synthesebeispiel 44 |
| I-1-607 siehe Synthesebeispiel 34 |
| I-1-608 siehe Synthesebeispiel 35 |
| I-1-609: |
| HPLC-MS: Masse (m/z): 284,1 (M+H)⁺; ¹H-NMR(400,0 MHz, DMSO-D₆): |
| δ= 10,845(3,9);10,029(0,4);9,579(0,4);8,887(1,0);8,875(1,0);8,867(1,0);8,863(1,0);8,855(1,5);8,843(9,9);8,831(9,7);8,679(0,4);8,672(0,5);8, 656(0,4);8,649(0,4);8,639(0,3);8,632(0,4);8,624(0,3);8,594(4,2);8,587(4,3);8,322(0,5);7,919(0,5);7,485(0,4);7,473(0,6);7,460(0,7);7,439(2,7) ;7,427(4,6);7,415(2,4);7,019(4,5);7,012(4,5);6,923(0,4);6,916(0,4);6,849(4,7);3,909(12,1);3,395(0,9);3,389(0,8);3,337(100,1);3,174(4,7);2,7 12(0,8);2,677(1,3);2,535(22,2);2,513(184,6);2,508(236,8);2,504(179,1);2,443(1,5);2,422(0,8);2,396(0,6);2,389(0,6);2,371(0,5);2,339(1,3);2, 335(1,6);2,304(0,4);2,290(0,5);2,248(16,0);1,915(1,2);1,836(1,1);1,819(0,3);1,799(0,4);1,575(0,3);1,241(0,3);0,005(0,4) |
| I-1-610: |
| HPLC-MS: logP = 3,00; Masse (m/z): 382,1 (M+H)⁺; ¹H-NMR(601,6 MHz, DMSO-D₆): |
| δ= 11,222(11,4);8,056(8,8);8,053(8,9);7,654(6,7);7,641(7,4);7,628(1,4);7,617(2,9);7,613(2,7);7,603(5,3);7,593(2,9);7,589(3,4);7,578(1,5);7, 545(2,2);7,543(2,4);7,531(6,7);7,521(6,4);7,519(6,3);7,496(9,5);7,485(8,2);7,475(7,2);7,462(2,9);7,460(2,7);7,396(9,1);7,382(16,0);7,368(7, 7);6,940(12,3);6,935(12,4);4,029(2,4);4,010(7,6);3,991(8,1);3,971(2,8);3,331(356,1);2,996(0,5);2,654(0,8);2,617(1,4);2,613(1,9);2,611(1,4); 2,541(225,8);2,523(3,2);2,520(4,1);2,517(4,2);2,508(99,9);2,505(213,4);2,502(294,6);2,499(217,0);2,496(103,9);2,425(0,9);2,389(1,4);2,386 (1,9);2,383(1,4);2,076(0,4);1,235(0,8);0,005(2,2);0,000(69,8);-0,006(2,3) |
| I-1-611: |
| HPLC-MS: logP = 2,53; Masse (m/z): 374,1 (M+H)⁺; ¹H-NMR(400,0 MHz, DMSO-D₆): |
| δ= 10,987(7,9);8,038(9,0);8,032(8,8);7,637(1,3);7,622(2,9);7,616(2,5);7,606(2,1);7,600(5,7);7,594(2,3);7,584(2,7);7,579(3,6);7,563(1,6);7,3 94(9,6);7,373(16,0);7,352(7,4);6,809(8,1);6,803(8,0);6,779(3,3);6,648(7,6);6,517(3,7);4,386(9,1);4,375(10,7);4,364(9,3);3,339(103,8);3,304( 0,3);3,193(9,1);3,182(10,6);3,171(8,6);2,543(57,8);2,526(0,8);2,512(16,0);2,508(31,7);2,503(41,0);2,499(29,5);2,495(14,3);0,000(2,9) |
| I-1-612: |
| HPLC-MS: logP = 3,53; Masse (m/z): 411,1 (M+H)⁺; ¹H-NMR(400,0 MHz, DMSO-D₆): |
| δ= 12,182(6,1);11,414(7,9);8,081(12,1);8,061(6,4);7,997(5,0);7,978(5,3);7,661(3,1);7,641(5,9);7,622(4,2);7,613(4,1);7,591(2,7);7,575(1,1);7 ,406(7,8);7,385(16,0);7,364(7,9);6,920(7,4);6,915(7,6);3,325(43,8);2,671(0,8);2,502(115,1);2,328(0,7);2,074(0,5);1,234(0,3);-0,001(26,4) |
| I-1-613: |
| HPLC-MS: logP = 2,72; Masse (m/z): 396,0 (M+H)⁺; ¹H-NMR(601,6 MHz, DMSO-D₆): |
| δ= 19,968(0,6);11,302(1,1);8,073(1,1);7,743(0,7);7,734(0,7);7,728(0,7);7,720(0,7);7,603(0,6);7,526(0,7);7,521(0,8);7,512(0,7);7,507(0,8);7, 395(1,1);7,381(1,9);7,367(1,0);7,300(0,8);6,937(2,0);6,933(2,1);3,329(1933,2);2,616(11,4);2,613(16,0);2,610(11,6);2,541(53,2);2,523(25,7); 2,520(33,1);2,516(34,2);2,508(809,0);2,505(1748,8);2,502(2415,0);2,499(1771,5);2,496(834,3);2,389(11,2);2,386(15,6);2,383(11,3);2,286(0 ,6);2,076(0,8);1,298(1,0);1,258(1,5);1,235(4,3);0,854(0,7);0,097(2,1);0,005(17,1);0,000(554,1);-0,006(18,0);-0,100(2,3) |
| I-1-614: |
| HPLC-MS: logP = 2,86; Masse (m/z): 402,0 (M+H)⁺; ¹H-NMR(400,0 MHz, DMSO-D₆): |
| δ= 11,484(11,8);8,079(9,4);8,074(9,4);7,909(7,6);7,889(9,2);7,827(7,4);7,808(9,9);7,712(5,4);7,692(8,2);7,672(3,4);7,636(1,3);7,620(2,7);7, 615(2,6);7,599(5,5);7,583(2,8);7,578(3,5);7,562(1,6);7,400(9,2);7,379(16,0);7,358(7,2);6,924(13,3);6,918(13,3);5,758(3,4);3,329(71,3);2,67 1(0,9);2,506(114,6);2,502(145,6);2,498(107,8);2,329(0,9);1,235(0,4);1,141(0,5);1,030(0,5);0,008(2,4);0,000(56,9) |
| I-1-615: |
| HPLC-MS: logP = 2,42; Masse (m/z): 322,1 (M+H)⁺; ¹H-NMR(400,0 MHz, DMSO-D₆): |
| δ= 9,625(2,3);7,996(1,8);7,990(1,8);7,605(0,6);7,599(0,5);7,589(0,4);7,584(1,2);7,578(0,5);7,568(0,6);7,563(0,8);7,547(0,3);7,382(2,0);7,36 1(3,4);7,340(1,5);6,825(2,9);6,819(2,8);4,171(1,7);4,162(2,4);4,157(2,1);4,152(2,5);4,066(2,5);4,060(2,1);4,055(2,4);4,046(1,7);3,337(54,7); 2,542(4,0);2,511(8,1);2,507(15,8);2,503(20,3);2,498(14,6);2,197(16,0);0,000(1,3) |
| I-1-616: |
| HPLC-MS: logP = 1,80; Masse (m/z): 370,0 (M+H)⁺; ¹H-NMR(400,0 MHz, DMSO-D₆): |
| δ= 10,832(3,1);8,317(0,4);8,049(3,1);7,634(0,4);7,618(0,9);7,612(0,9);7,597(1,8);7,580(1,0);7,575(1,2);7,560(0,5);7,396(3,0);7,375(5,1);7,3 54(2,4);6,846(3,0);6,841(3,1);5,757(2,8);4,736(2,6);4,723(2,9);4,709(2,8);3,597(2,8);3,584(2,9);3,570(2,7);3,328(96,3);2,671(1,6);2,506(198 ,1);2,502(252,4);2,329(1,6);2,028(16,0);1,259(0,4);1,235(0,5);0,146(0,4);0,000(89,8);-0,150(0,4) |
| I-1-617: |
| HPLC-MS: logP = 2,72; Masse (m/z): 374,9 (M+H)⁺; ¹H-NMR(400,0 MHz, DMSO-D₆): |
| δ= 11,520(7,9);8,452(8,3);8,445(8,4);7,932(1,7);7,916(2,2);7,911(4,1);7,895(4,2);7,890(2,9);7,874(2,3);7,842(4,5);7,822(5,6);7,790(1,6);7,7 73(4,4);7,754(4,1);7,720(3,7);7,699(16,0);7,679(10,0);7,545(3,4);7,523(6,0);7,501(3,1);7,038(8,5);7,031(8,5);5,757(8,9);3,326(82,1);2,671(0 ,6);2,667(0,5);2,507(75,0);2,502(97,7);2,498(73,2);2,329(0,6);1,989(0,6);1,175(0,3);0,146(0,6);0,008(6,6);0,000(128,8);-0,008(6,7);-0,150(0,6) |
| I-1-618: |
| HPLC-MS: logP = 2,26; Masse (m/z): 375,9 (M+H)⁺; ¹H-NMR(400,0 MHz, DMSO-D₆): |
| δ= 11,665(15,1);8,854(8,8);8,842(8,8);8,472(15,5);8,466(15,4);8,317(1,0);8,220(8,2);8,201(9,0);7,936(3,3);7,920(4,2);7,915(7,5);7,899(7,7); 7,894(5,0);7,878(4,3);7,845(7,2);7,833(7,3);7,826(6,8);7,814(6,3);7,708(12,6);7,687(10,3);7,553(6,3);7,531(11,0);7,509(5,5);7,043(16,0);7,0 37(15,9);5,758(3,0);3,328(363,0);3,037(0,7);2,671(3,4);2,667(2,6);2,541(2,2);2,506(412,2);2,502(525,5);2,498(388,1);2,333(2,6);2,329(3,3); 1,299(0,7);1,259(1,2);1,235(1,3);0,146(0,9);0,008(8,3);0,000(200,8);-0,149(1,0) |
| I-1-619: |
| HPLC-MS: logP = 2,45; Masse (m/z): 343,0 (M+H)⁺; ¹H-NMR(400,0 MHz, DMSO-D₆): |
| δ= 11,735(11,8);8,469(14,5);8,462(14,6);7,936(3,0);7,920(3,5);7,915(7,0);7,899(7,0);7,894(4,6);7,878(4,0);7,709(10,8);7,688(8,9);7,619(1,5 );7,602(3,3);7,598(3,2);7,581(6,3);7,564(3,4);7,560(4,1);7,553(5,5);7,543(2,1);7,531(9,4);7,509(4,6);7,253(2,1);7,246(10,9);7,226(16,0);7,20 6(9,1);7,199(1,9);7,046(15,1);7,039(15,2);5,757(3,3);4,038(0,4);4,020(0,5);3,326(90,4);2,676(0,6);2,672(0,8);2,667(0,6);2,542(0,4);2,525(2, 3);2,511(47,8);2,507(96,5);2,503(127,0);2,498(91,9);2,494(44,8);2,334(0,6);2,329(0,8);2,325(0,6);1,989(2,0);1,235(0,5);1,193(0,5);1,175(1, 0);1,158(0,5);0,146(0,7);0,008(6,4);0,000(164,4);-0,009(6,4);-0,150(0,8) |
| I-1-620: |
| HPLC-MS: logP = 2,61; Masse (m/z): 384,9 (M+H)⁺; ¹H-NMR(400,0 MHz, DMSO-D₆): |
| δ= 11,431(15,3);8,450(15,9);8,443(16,0);8,316(0,4);7,931(3,4);7,915(4,3);7,910(8,0);7,894(8,1);7,889(5,4);7,873(4,5);7,707(10,9);7,697(13, 7);7,688(12,9);7,676(10,9);7,551(6,4);7,546(10,5);7,533(11,5);7,528(12,4);7,522(13,2);7,500(6,9);7,495(6,4);7,476(11,3);7,457(5,8);7,431(6 ,8);7,427(6,7);7,412(8,5);7,407(8,3);7,393(3,7);7,388(3,3);7,055(16,0);7,049(15,9);5,756(1,8);4,038(0,4);4,020(0,4);3,325(165,0);2,675(0,9); 2,671(1,2);2,667(0,9);2,506(142,6);2,502(185,6);2,498(135,4);2,333(0,9);2,329(1,2);2,325(0,9);1,989(1,7);1,397(0,4);1,259(0,5);1,234(0,6); 1,193(0,5);1,175(0,9);1,157(0,5);0,146(1,2);0,008(10,3);0,000(240,6);-0,008(10,1);-0,150(1,2) |
| I-1-621: |
| HPLC-MS: logP = 2,57; Masse (m/z): 340,9 (M+H)⁺; ¹H-NMR(400,0 MHz, DMSO-D₆): |
| δ= 11,444(15,0);8,450(16,0);8,444(16,0);8,316(0,4);7,932(3,5);7,915(4,5);7,911(8,2);7,894(8,3);7,890(5,6);7,873(4,6);7,697(12,5);7,676(10, |
| 4);7,582(7,5);7,578(8,2);7,563(10,5);7,559(14,8);7,545(8,5);7,538(14,5);7,535(14,8);7,523(13,5);7,514(7,1);7,500(15,9);7,497(9,8);7,480(5, 4);7,476(4,5);7,454(7,9);7,451(7,8);7,436(9,9);7,433(9,7);7,418(3,7);7,415(3,5);7,057(15,9);7,051(15,9);5,756(5,7);3,325(140,0);2,675(1,0); 2,671(1,3);2,667(1,0);2,506(151,5);2,502(197,2);2,498(143,7);2,333(0,9);2,329(1,2);2,324(0,9);1,989(0,8);1,259(0,4);1,234(0,8);1,175(0,4); 0,146(1,1);0,007(10,3);0,000(219,7);-0,008(9,9);-0,150(1,1) |
| I-1-622: |
| HPLC-MS: logP = 1,95; Masse (m/z): 342,0 (M+H)⁺; ¹H-NMR(400,0 MHz, DMSO-D₆): |
| δ= 11,597(13,9);11,528(0,5);8,527(8,6);8,523(9,3);8,515(9,2);8,511(9,1);8,469(15,7);8,463(15,8);8,354(0,6);8,347(0,5);8,316(1,4);8,077(8,7 );8,072(8,9);8,058(9,6);8,053(9,2);7,936(3,4);7,920(4,0);7,915(7,6);7,899(7,8);7,894(5,0);7,878(4,4);7,832(0,4);7,819(0,4);7,783(0,3);7,705( 11,9);7,684(9,9);7,555(10,9);7,543(9,8);7,537(9,9);7,530(11,9);7,525(10,4);7,508(5,5);7,061(16,0);7,054(15,9);7,013(0,5);7,007(0,6);5,757( 1,2);3,324(396,8);3,038(0,4);2,675(3,0);2,671(4,1);2,666(2,9);2,524(11,9);2,511(244,1);2,506(484,8);2,502(632,6);2,497(450,9);2,493(215,8 );2,333(3,0);2,329(4,1);2,324(3,0);2,074(0,9);1,299(0,5);1,259(0,8);1,235(0,8);0,146(2,3);0,008(19,6);0,000(508,7);-0,008(18,2);-0,150(2,5) |
| I-1-623: |
| HPLC-MS: logP = 2,68; Masse (m/z): 393,0 (M+H)⁺; ¹H-NMR(400,0 MHz, DMSO-D₆): |
| δ= 11,751(10,1);8,469(11,2);8,463(11,1);8,316(0,7);7,932(2,5);7,916(3,0);7,911(5,4);7,895(5,6);7,890(3,6);7,874(3,1);7,781(0,9);7,761(2,6); 7,752(1,7);7,742(5,1);7,727(7,4);7,711(10,4);7,704(16,0);7,690(9,1);7,682(4,3);7,548(4,1);7,526(7,3);7,503(3,6);7,044(0,5);7,031(12,0);7,02 4(11,8);3,324(199,3);3,295(0,5);2,680(0,7);2,676(1,3);2,671(1,8);2,667(1,4);2,558(0,6);2,511(113,7);2,506(221,7);2,502(287,0);2,498(207,1 );2,333(1,4);2,329(1,8);2,325(1,4);1,259(0,5);1,235(0,5);0,146(1,2);0,008(11,1);0,000(251,8);-0,009(10,5);-0,027(0,5);-0,030(0,5);-0,150(1,2) |
| I-1-624: |
| HPLC-MS: logP = 2,79; Masse (m/z): 438,8 (M+H)⁺; ¹H-NMR(400,0 MHz, DMSO-D₆): |
| δ= 20,005(0,4);11,109(10,4);8,659(0,6);8,652(0,6);8,521(0,4);8,493(0,4);8,434(12,9);8,428(12,8);8,316(6,1);8,308(1,2);8,301(1,1);8,277(0,4 );8,157(0,4);8,140(1,5);7,997(0,4);7,939(2,8);7,923(3,4);7,918(6,4);7,902(6,7);7,897(4,2);7,881(3,6);7,821(0,9);7,809(15,2);7,795(16,0);7,78 4(1,1);7,771(0,8);7,696(9,5);7,675(8,2);7,650(1,0);7,555(4,5);7,532(8,1);7,511(4,2);7,389(13,3);7,375(12,3);7,347(0,4);7,285(0,7);7,271(0,6) ;7,236(0,4);7,215(0,5);7,192(0,4);7,170(0,4);7,062(0,7);7,055(0,9);7,038(7,0);7,032(6,8);6,965(0,4);6,359(0,7);6,347(1,1);6,340(1,1);5,757(6 ,0);3,411(0,6);3,384(0,7);3,324(1622,0);3,092(2,7);3,058(1,0);3,035(3,2);2,946(3,2);2,679(5,0);2,675(10,3);2,671(14,3);2,666(10,1);2,662(4, 7);2,541(6,4);2,524(38,4);2,519(61,1);2,511(807,8);2,506(1644,2);2,502(2160,1);2,497(1531,6);2,493(717,1);2,427(1,0);2,338(4,8);2,333(10 ,1);2,328(14,0);2,324(10,1);2,212(0,4);2,074(1,3);1,259(0,6);1,235(1,6);0,146(8,1);0,008(67,0);0,000(1989,3);-0,009(68,7);-0,081(0,7);-0,150(8,5) |
| I-1-625 |
| HPLC-MS: logP = 2,63; Masse (m/z):433.0 (M+H)⁺; ¹H-NMR [DMSO-D₆] 7.05-7.06 (m, 1H), 7.20-7.25 (m, 1H), 7.46-7.52 (m, 3H), 7.68-7.70 (m, 1H), 7.87-7.93 (m, 2H), 8.45 (d, 1H), 11.38 (s, 1H). |
| I-1-626: |
| HPLC-MS: logP = 2,67; Masse (m/z): 375,1 (M+H)⁺; ¹H-NMR(400,0 MHz, DMSO-D₆): |
| δ= 11,444(15,0);8,337(15,5);8,330(15,7);8,316(0,7);8,071(7,0);8,064(7,3);8,051(7,2);8,043(7,2);7,850(4,8);7,838(12,2);7,827(11,2);7,818(11 ,6);7,815(12,6);7,795(6,0);7,788(7,8);7,775(7,4);7,768(13,6);7,751(8,2);7,716(6,3);7,693(12,8);7,673(7,3);6,990(15,9);6,984(16,0);5,756(3,5 );3,772(0,5);3,325(125,6);2,675(0,9);2,671(1,2);2,667(0,9);2,506(139,1);2,502(183,6);2,498(135,7);2,333(0,8);2,329(1,1);2,324(0,9);1,989(0 ,3);1,259(0,4);1,235(1,1);0,146(1,1);0,008(9,6);0,000(235,1);-0,008(10,4);-0,150(1,1) |
| I-1-627: |
| HPLC-MS: logP = 2,21; Masse (m/z): 375,9 (M+H)⁺; ¹H-NMR(400,0 MHz, DMSO-D₆): |
| δ= 20,011(0,4);11,597(15,1);8,849(8,5);8,838(8,6);8,407(0,8);8,400(0,8);8,356(15,1);8,350(15,7);8,316(2,5);8,283(0,5);8,264(0,4);8,210(8,1 );8,191(9,0);8,110(1,0);8,076(6,6);8,069(7,1);8,055(6,9);8,048(7,0);7,998(0,6);7,976(0,7);7,940(0,5);7,858(4,7);7,841(8,6);7,835(11,1);7,829 (8,8);7,822(15,5);7,809(6,7);7,800(6,1);7,793(5,4);7,780(6,5);7,773(6,2);7,757(3,2);7,750(3,0);7,639(0,4);7,617(0,5);7,604(0,5);7,559(0,8);7, 553(0,8);7,342(0,4);7,333(0,5);7,310(0,4);6,995(15,7);6,988(16,0);6,313(0,7);6,307(0,7);6,283(0,6);5,756(4,3);5,618(0,7);5,612(0,7);4,945(1 ,2);3,325(881,4);3,025(1,5);2,997(1,6);2,934(1,6);2,671(7,1);2,506(829,8);2,502(1086,7);2,498(822,1);2,328(7,1);1,235(0,7);0,146(6,1);0,00 8(51,9);0,000(1266,1);-0,150(6,2) |
| I-1-628: |
| HPLC-MS: logP = 2,39; Masse (m/z): 343,0 (M+H)⁺; ¹H-NMR(400,0 MHz, DMSO-D₆): |
| δ= 11,662(12,5);8,353(14,6);8,347(14,6);8,316(0,4);8,076(6,4);8,068(6,6);8,055(6,6);8,048(6,5);7,859(4,3);7,847(4,8);7,837(8,9);7,824(8,5); 7,800(5,3);7,793(4,9);7,780(5,8);7,773(5,3);7,758(2,7);7,751(2,5);7,613(1,5);7,597(3,3);7,592(3,1);7,575(6,2);7,559(3,3);7,554(3,7);7,538(1, 6);7,249(2,0);7,242(10,9);7,222(16,0);7,202(9,2);7,195(2,0);6,997(15,4);6,991(15,4);5,757(4,3);4,056(0,6);4,038(1,8);4,020(1,9);4,002(0,6); 3,325(107,8);3,024(0,6);2,857(0,5);2,676(0,7);2,671(1,0);2,667(0,7);2,524(2,8);2,511(56,8);2,507(114,6);2,502(151,2);2,498(109,0);2,493(5 2,7);2,333(0,7);2,329(1,0);2,325(0,7);1,989(8,0);1,397(0,6);1,193(2,1);1,175(4,2);1,157(2,1);0,146(0,9);0,008(7,9);0,000(205,6);-0,009(7,8);-0,150(0,9) |
| I-1-629: |
| HPLC-MS: logP = 2,55; Masse (m/z): 384,9 (M+H)⁺; ¹H-NMR(400,0 MHz, DMSO-D₆): |
| δ= 11,351(10,5);8,335(11,3);8,328(11,3);8,316(0,5);8,070(5,2);8,063(5,3);8,049(5,3);8,042(5,3);7,846(3,0);7,833(3,6);7,823(7,2);7,811(6,9); 7,795(4,5);7,788(4,1);7,775(4,9);7,768(4,5);7,752(2,1);7,745(2,0);7,706(6,7);7,704(6,7);7,686(7,8);7,684(7,6);7,547(4,1);7,542(4,7);7,528(7, 7);7,523(7,8);7,492(3,8);7,489(4,0);7,473(7,6);7,471(7,2);7,455(3,9);7,452(3,6);7,427(4,8);7,422(4,8);7,407(5,8);7,403(5,6);7,389(2,7);7,384 (2,4);7,008(11,9);7,001(11,8);5,756(16,0);4,056(1,2);4,038(3,6);4,020(3,7);4,002(1,2);3,324(64,7);2,675(0,5);2,671(0,7);2,666(0,5);2,541(0, 4);2,524(2,2);2,511(42,8);2,506(85,1);2,502(110,9);2,497(79,1);2,493(37,7);2,333(0,5);2,329(0,7);2,324(0,5);1,989(15,8);1,397(5,7);1,193(4 ,3);1,175(8,6);1,157(4,2);1,045(0,5);1,030(0,5);0,146(0,7);0,008(6,9);0,000(166,9);-0,009(6,2);-0,150(0,7) |
| I-1-630: |
| HPLC-MS: logP = 2,52; Masse (m/z): 341,0 (M+H)⁺; ¹H-NMR(400,0 MHz, DMSO-D₆): |
| δ= 11,366(10,9);8,335(11,6);8,329(11,7);8,316(0,6);8,071(5,5);8,064(5,7);8,050(5,7);8,043(5,7);7,846(3,1);7,833(3,7);7,823(7,4);7,811(7,1); 7,795(4,7);7,788(4,4);7,775(5,1);7,768(4,7);7,753(2,2);7,746(2,1);7,577(5,1);7,573(5,7);7,558(7,8);7,554(11,2);7,535(9,3);7,532(9,7);7,514( 4,1);7,510(4,4);7,496(7,1);7,492(6,1);7,476(4,0);7,472(3,2);7,451(5,6);7,448(5,6);7,433(7,0);7,429(6,9);7,415(2,6);7,411(2,5);7,010(12,2);7, 004(12,2);5,756(16,0);4,056(0,6);4,038(1,7);4,020(1,8);4,002(0,6);3,325(92,6);2,675(0,6);2,671(0,9);2,666(0,6);2,541(0,4);2,524(2,5);2,511( 48,8);2,506(99,4);2,502(131,4);2,497(94,2);2,493(44,9);2,333(0,6);2,329(0,8);2,324(0,6);1,989(7,6);1,397(1,3);1,234(0,4);1,193(2,1);1,175( 4,1);1,157(2,0);0,146(0,8);0,008(7,2);0,000(187,3);-0,009(6,7);-0,150(0,8) |
| I-1-631: |
| HPLC-MS: logP = 1,91; Masse (m/z): 342,1 (M+H)⁺; ¹H-NMR(400,0 MHz, DMSO-D₆): |
| δ= 11,528(13,9);8,524(8,2);8,519(8,9);8,512(8,9);8,507(8,7);8,354(15,3);8,347(15,3);8,316(2,7);8,076(7,3);8,070(13,8);8,066(10,5);8,055(8, 6);8,052(11,9);8,048(14,7);7,872(0,4);7,854(4,2);7,842(4,8);7,831(9,5);7,819(9,0);7,801(5,8);7,793(5,3);7,781(6,3);7,773(5,8);7,758(2,8);7,7 51(2,7);7,552(8,9);7,540(8,7);7,534(8,6);7,522(8,3);7,013(16,0);7,007(16,0);5,844(0,4);5,838(0,5);5,233(0,6);3,324(675,9);2,997(0,7);2,675( 4,7);2,671(6,5);2,666(4,7);2,541(3,0);2,524(17,9);2,510(372,2);2,506(747,8);2,502(976,6);2,497(698,4);2,493(333,4);2,333(4,6);2,328(6,4);2 ,324(4,6);2,074(1,9);0,146(3,7);0,008(30,3);0,000(825,1);-0,009(29,7);-0,150(3,6) |
| I-1-632: |
| HPLC-MS: logP = 2,64; Masse (m/z): 393,0 (M+H)⁺; ¹H-NMR(400,0 MHz, DMSO-D₆): |
| δ= 11,679(10,7);8,355(11,9);8,348(11,7);8,072(5,3);8,064(5,4);8,051(5,4);8,043(5,2);7,865(3,7);7,853(4,2);7,842(7,0);7,830(6,7);7,797(4,1); 7,790(3,8);7,777(5,5);7,770(4,6);7,755(4,3);7,747(3,7);7,738(5,3);7,722(7,8);7,701(16,0);7,684(4,6);7,678(3,9);6,984(12,3);6,978(12,2);5,75 7(1,7);3,325(82,1);2,676(0,6);2,671(0,8);2,667(0,6);2,541(0,5);2,511(52,9);2,507(101,6);2,502(130,2);2,498(93,0);2,333(0,7);2,329(0,9);2,3 25(0,6);1,989(1,2);1,260(0,4);1,193(0,3);1,175(0,6);1,157(0,3);0,146(0,6);0,008(6,1);0,000(124,0);-0,008(4,6);-0,150(0,6) |
| I-1-633: |
| HPLC-MS: logP = 2,73; Masse (m/z): 438,8 (M+H)⁺; ¹H-NMR(400,0 MHz, DMSO-D₆): |
| δ= 11,046(10,4);8,427(0,3);8,324(11,9);8,317(12,3);8,187(0,5);8,180(0,5);8,112(0,6);8,082(5,2);8,075(5,3);8,062(5,3);8,055(5,3);7,847(3,0); 7,835(3,7);7,825(7,7);7,812(7,9);7,805(13,9);7,801(6,1);7,792(16,0);7,781(5,5);7,773(5,1);7,758(2,4);7,751(2,0);7,733(0,4);7,696(0,4);7,676 (0,4);7,511(0,3);7,386(11,2);7,372(10,6);7,266(0,3);7,253(0,4);7,246(0,5);7,233(0,4);6,993(6,8);6,987(6,6);6,595(0,4);6,290(0,4);6,283(0,4); 5,757(12,1);3,324(197,3);3,039(1,1);3,026(1,0);3,014(0,6);2,999(1,2);2,934(1,0);2,675(1,8);2,671(2,3);2,666(1,7);2,541(1,2);2,506(279,3);2, 502(358,7);2,497(256,6);2,333(1,7);2,329(2,3);2,324(1,7);1,235(0,4);0,146(1,4);0,008(11,5);0,000(303,0);-0,009(12,0);-0,150(1,3) |
| I-1-634: |
| HPLC-MS: logP = 2,57; Masse (m/z): 433,0 (M+H)⁺; ¹H-NMR(400,0 MHz, DMSO-D₆): |
| δ= 11,294(10,1);8,336(10,0);8,329(10,0);8,316(0,8);8,071(4,8);8,064(4,9);8,050(4,9);8,043(4,9);7,924(7,4);7,904(8,3);7,848(2,8);7,835(3,3); 7,825(6,4);7,813(6,0);7,796(4,0);7,789(3,6);7,776(4,4);7,769(4,1);7,753(1,9);7,746(1,7);7,501(1,4);7,482(6,4);7,472(9,3);7,466(16,0);7,454( 2,2);7,250(0,3);7,236(3,3);7,229(3,2);7,220(3,5);7,216(4,0);7,209(3,1);7,201(2,9);7,193(2,6);7,007(10,3);7,000(10,3);5,756(3,6);4,055(0,7);4 ,038(2,1);4,020(2,1);4,002(0,7);3,323(134,2);2,671(1,5);2,506(176,0);2,502(229,1);2,497(168,3);2,328(1,4);2,324(1,1);1,989(9,0);1,398(0,4) ;1,259(0,4);1,235(0,6);1,193(2,5);1,175(4,8);1,157(2,4);0,146(0,9);0,008(8,2);0,000(189,4);-0,008(8,2);-0,150(0,9) |
| I-1-635: |
| HPLC-MS: logP = 2,80; Masse (m/z): 383,1 (M+H)⁺; ¹H-NMR(601,6 MHz, DMSO-D₆): |
| δ= 11,396(0,9);11,342(11,1);8,486(15,4);8,482(16,0);8,337(11,4);8,333(11,4);8,298(1,6);8,283(4,7);8,279(4,4);8,269(5,0);8,265(8,8);8,261(4 ,7);8,251(4,8);8,247(4,4);8,237(2,7);8,227(0,8);8,214(0,4);7,661(8,9);7,648(9,6);7,560(0,5);7,549(3,8);7,547(4,3);7,535(9,6);7,524(8,3);7,52 2(8,0);7,505(2,3);7,495(13,5);7,491(9,8);7,483(7,2);7,478(10,2);7,466(3,7);7,464(3,4);7,027(1,6);7,023(1,6);6,991(13,4);6,986(13,5);4,024(3 ,3);4,005(10,2);3,986(10,7);3,967(3,8);3,330(740,6);2,654(0,3);2,617(4,2);2,614(5,8);2,610(4,3);2,608(2,0);2,541(107,4);2,523(9,6);2,520(1 2,2);2,517(12,2);2,508(293,4);2,505(638,6);2,502(886,8);2,499(648,0);2,496(303,5);2,425(0,5);2,389(4,2);2,386(5,7);2,383(4,2);2,076(0,4);1 ,298(0,5);1,258(0,7);1,235(2,0);0,854(0,4);0,096(0,9);0,005(6,4);0,000(218,1);-0,006(7,0);-0,100(0,8) |
| I-1-636: |
| HPLC-MS: logP = 2,31; Masse (m/z): 375,0 (M+H)⁺; ¹H-NMR(400,0 MHz, DMSO-D₆): |
| δ= 11,101(11,1);8,477(15,8);8,471(16,0);8,320(14,0);8,314(13,5);8,288(4,9);8,282(4,4);8,267(5,4);8,261(9,0);8,255(4,8);8,240(4,9);8,234(4, 4);6,855(10,9);6,849(10,5);6,767(4,7);6,636(11,5);6,505(5,5);4,389(12,5);4,379(14,5);4,367(12,6);3,333(181,6);3,196(12,5);3,185(14,3);3,17 4(11,6);2,998(0,4);2,677(0,4);2,673(0,6);2,668(0,4);2,543(53,4);2,513(37,1);2,508(71,2);2,504(90,8);2,499(64,8);2,495(31,1);2,335(0,4);2,3 31(0,6);2,326(0,4);1,234(0,5);0,000(7,8) |
| I-1-637: |
| HPLC-MS: logP = 2,50; Masse (m/z): 397,0 (M+H)⁺; ¹H-NMR(601,6 MHz, DMSO-D₆): |
| δ= 11,433(6,9);8,485(12,8);8,481(13,4);8,359(11,9);8,354(11,8);8,312(0,7);8,282(3,6);8,278(3,5);8,268(3,9);8,264(6,9);8,260(3,8);8,250(3,8 );8,246(3,7);8,219(0,4);7,742(6,2);7,734(6,5);7,727(6,6);7,719(6,3);7,523(6,3);7,518(6,6);7,508(6,7);7,503(6,3);7,317(3,7);7,312(3,5);7,303( 6,5);7,298(5,8);7,289(3,5);7,284(3,0);7,020(1,0);7,015(1,0);6,989(15,9);6,985(16,0);3,330(826,5);2,996(0,4);2,654(0,9);2,617(3,9);2,614(5,4 );2,611(4,0);2,541(226,1);2,523(8,7);2,520(11,0);2,517(11,5);2,507(274,7);2,505(587,5);2,502(810,6);2,499(609,9);2,425(1,0);2,389(3,8);2,3 86(5,3);2,383(3,9);1,298(0,4);1,258(0,6);1,235(2,2);0,854(0,3);0,097(0,7);0,005(5,5);0,000(168,9);-0,006(6,3);-0,100(0,7) |
| I-1-638: |
| HPLC-MS: logP = 2,70; Masse (m/z): 403,0 (M+H)⁺; ¹H-NMR(400,0 MHz, DMSO-D₆): |
| δ= 11,627(14,4);11,483(0,9);8,484(13,8);8,478(14,6);8,367(12,7);8,361(12,8);8,317(0,7);8,287(4,1);8,280(3,9);8,266(4,4);8,259(7,7);8,253(4 ,1);8,238(4,2);8,232(3,8);8,079(0,8);7,907(9,1);7,887(11,0);7,826(8,7);7,806(12,0);7,713(6,5);7,693(9,8);7,673(4,0);7,599(0,5);7,400(0,7);7, 379(1,3);7,358(0,6);6,980(16,0);6,973(15,9);6,923(1,1);6,917(1,1);5,758(1,3);3,328(207,6);3,101(0,5);3,086(0,4);2,676(1,5);2,671(2,1);2,66 |
| 7(1,5);2,507(244,3);2,502(317,7);2,498(231,5);2,333(1,5);2,329(2,0);2,325(1,5);1,259(0,4);1,235(0,7);1,191(0,8);1,173(1,4);1,158(0,9);1,14 1(0,6);1,030(0,5);0,146(0,6);0,008(5,7);0,000(133,5);-0,008(5,4);-0,150(0,6) |
| I-1-639: |
| HPLC-MS: logP = 2,16; Masse (m/z): 323,1 (M+H)⁺; ¹H-NMR(400,0 MHz, DMSO-D₆): |
| δ= 9,672(2,2);8,462(2,4);8,456(2,4);8,293(2,2);8,287(2,1);8,270(0,7);8,264(0,6);8,249(0,8);8,243(1,3);8,236(0,7);8,221(0,7);8,215(0,6);6,88 3(2,8);6,877(2,7);4,183(1,7);4,174(2,4);4,169(2,1);4,163(2,5);4,084(2,6);4,079(2,1);4,074(2,4);4,065(1,6);3,345(19,7);2,545(7,8);2,514(3,6); 2,510(6,9);2,506(8,8);2,501(6,2);2,497(2,9);2,199(16,0);0,000(0,5) |
| I-1-640 siehe Synthesebeispiel 23 |
| I-1-641 siehe Synthesebeispiel 32 |
| I-1-642: |
| HPLC-MS: logP = 3,40; Masse (m/z): 445,0 (M+H)⁺; ¹H-NMR(400,0 MHz, DMSO-D₆): |
| δ= 11,458(4,1);8,430(3,2);8,424(3,4);8,417(5,1);8,405(5,1);8,080(1,3);7,852(0,3);7,837(2,1);7,830(0,7);7,819(3,0);7,791(2,3);7,786(1,8);7,7 78(2,8);7,769(4,3);7,759(2,6);7,756(2,3);7,750(2,3);7,714(1,8);7,697(4,8);7,679(2,3);7,639(2,1);7,627(4,0);7,614(2,1);7,450(0,5);7,442(3,7); 7,437(1,2);7,425(1,4);7,420(6,9);7,415(1,4);7,403(1,1);7,398(3,3);7,390(0,3);7,144(0,4);6,998(4,5);6,991(4,5);4,056(1,2);4,038(3,7);4,020(3, 8);4,003(1,3);3,326(23,2);2,671(0,3);2,511(20,3);2,507(40,2);2,503(52,7);2,498(38,1);2,494(18,4);2,329(0,3);1,989(16,0);1,193(4,3);1,175(8 ,5);1,157(4,2);0,008(0,9);0,000(22,5);-0,009(0,8) |
| I-1-643 siehe Synthesebeispiel 24 |
| I-1-644: |
| HPLC-MS: logP = 0,99; Masse (m/z): 304,1 (M+H)⁺; ¹H-NMR(400,0 MHz, DMSO-D₆): |
| δ= 10,958(1,2);8,035(0,9);8,032(0,9);8,029(0,9);7,937(1,9);7,935(1,8);7,829(1,7);7,598(0,6);7,576(0,4);7,397(1,1);7,376(1,8);7,355(0,8);6,9 05(1,6);6,899(1,6);4,123(1,2);4,109(3,7);4,096(3,8);4,083(1,3);3,877(7,2);3,341(27,3);3,177(16,0);3,164(15,3);2,513(3,4);2,508(6,8);2,504(8 ,8);2,499(6,4);2,495(3,1);0,008(0,6);0,000(13,6);-0,008(0,6) |
| I-1-645: |
| HPLC-MS: logP = 2,41; Masse (m/z): 303,1 (M+H)⁺; ¹H-NMR(400,0 MHz, DMSO-D₆): |
| δ= 10,571(2,6);8,001(2,0);7,996(2,0);7,611(0,6);7,605(0,6);7,595(0,5);7,589(1,3);7,584(0,5);7,573(0,6);7,568(0,8);7,552(0,4);7,390(2,2);7,3 69(3,7);7,348(1,7);7,188(1,6);7,184(1,8);7,178(1,8);7,174(1,7);7,009(1,6);7,004(2,4);6,999(1,6);6,899(3,2);6,893(3,2);6,064(1,8);6,058(1,9); 6,055(1,9);6,048(1,7);5,757(1,7);3,896(16,0);3,330(11,0);2,511(5,0);2,507(9,8);2,503(12,8);2,498(9,2);2,494(4,6) |
| I-1-646: |
| HPLC-MS: logP = 0,70; Masse (m/z): 305,0 (M+H)⁺; ¹H-NMR(400,0 MHz, DMSO-D₆): |
| δ= 11,065(3,1);8,480(2,8);8,474(2,9);8,320(2,5);8,314(2,5);8,288(0,8);8,282(0,8);8,267(0,9);8,261(1,6);8,255(0,9);8,240(0,8);8,234(0,8);7,9 85(4,6);7,839(4,1);6,967(3,2);6,960(3,2);3,878(16,0);3,330(8,2);2,508(14,0);2,504(18,1);2,500(13,7) |
| I-1-647: |
| HPLC-MS: logP = 2,16; Masse (m/z): 304,1 (M+H)⁺; ¹H-NMR(400,0 MHz, DMSO-D₆): |
| δ= 10,673(2,9);8,472(2,6);8,466(2,7);8,291(2,3);8,285(2,3);8,276(0,9);8,270(0,8);8,255(0,9);8,249(1,5);8,243(0,8);8,228(0,8);8,222(0,8);7,2 41(1,7);7,237(1,8);7,231(1,8);7,227(1,8);7,021(1,7);7,017(2,5);7,012(1,7);6,962(3,1);6,956(3,1);6,075(1,7);6,069(1,9);6,065(1,9);6,059(1,7); 3,898(16,0);3,332(19,5);2,508(11,1);2,504(14,2);2,500(10,4) |
| I-4-1 |
| HPLC-MS: logP = 2,75; Masse (m/z): 336.1 (M+H)⁺; ¹H-NMR [CD₃CN] 3.45 (t, 2H), 3.79 (t, 2H), 6.93 - 7.01 (m, 2H), 7.10 - 7.18 (m, 1H), 7.39 - 7.51 (m, 3H), 7.54 - 7.57 (m, 1H), 9.01 (br. s, 1H). |
| I-4-2 |
| siehe Synthesebeispiel 6 |
| I-4-3 |
| HPLC-MS: logP = 2,59; Masse (m/z): 338.1 (M+H)⁺; ¹H-NMR [DMSO-D₆] 3.41 (t, 2H), 3.76 (t, 2H), 7.06 - 7.12 (m, 2H), 7.17 - 7.23 (m, 3H), 7.53 - 7.61 (m, 1H), 11.46 (br. s, 1H). |
| I-4-4: |
| HPLC-MS: logP = 2,31; Masse (m/z): 428,0 (M+H)⁺; ¹H-NMR(400,0 MHz, CD₃CN): |
| δ= 8,772(2,9);7,888(4,7);7,886(4,5);7,868(4,9);7,866(4,6);7,455(2,4);7,453(2,2);7,437(5,6);7,434(5,3);7,418(3,8);7,415(3,5);7,382(0,4);7,33 8(4,8);7,333(5,2);7,319(3,6);7,314(3,5);7,214(1,0);7,199(2,3);7,195(3,8);7,191(4,1);7,184(1,4);7,177(6,7);7,172(5,3);7,164(1,9);7,161(2,0);7, 157(5,3);7,153(2,7);7,142(1,6);7,133(0,4);7,036(0,7);7,030(1,1);7,020(6,5);7,014(0,9);7,010(1,0);6,999(8,3);6,997(8,4);6,987(1,2);6,976(4,5) ;6,965(0,9);6,961(0,6);6,954(0,4);6,932(0,6);6,910(0,3);3,815(2,6);3,812(4,3);3,809(2,5);3,799(5,4);3,796(8,9);3,792(5,0);3,783(3,0);3,780(4 ,6);3,776(2,6);2,816(8,0);2,800(16,0);2,784(7,5);2,626(0,5);2,132(38,3);2,119(0,6);2,113(0,7);2,107(0,9);2,100(0,6);1,971(1,2);1,964(4,9);1, 957(6,0);1,952(53,4);1,945(100,4);1,939(140,1);1,933(95,4);1,927(48,4);1,920(1,6);1,914(0,8);1,780(0,3);1,774(0,6);1,768(0,9);1,762(0,6);1 ,437(5,1);1,372(0,8);1,285(0,4);1,277(1,0);1,270(0,4);1,203(0,6);0,146(1,0);0,008(9,1);0,000(253,6);-0,009(7,8);-0,150(1,1) |
| I-4-5: |
| HPLC-MS: logP = 2,21; Masse (m/z): 316,1 (M+H)⁺; ¹H-NMR(400,0 MHz, CD₃CN): |
| δ= 8,768(3,7);7,470(0,3);7,426(0,4);7,420(0,4);7,371(2,1);7,368(2,3);7,349(10,6);7,331(10,4);7,307(0,6);7,287(0,8);7,270(1,0);7,267(1,0);7, 246(7,6);7,228(7,7);7,207(2,5);7,183(1,3);7,168(2,3);7,163(2,1);7,161(1,8);7,153(1,6);7,147(4,2);7,141(2,3);7,133(2,0);7,130(2,1);7,126(3,2) ;7,111(1,8);7,095(0,5);7,087(0,5);7,074(0,5);7,068(0,4);7,062(0,4);7,058(0,4);7,054(0,5);7,039(0,4);7,029(0,9);7,023(1,3);7,012(6,9);7,003(1 ,3);6,990(9,4);6,989(9,4);6,978(1,6);6,968(5,2);6,956(1,2);6,952(1,1);6,940(1,1);6,927(0,5);6,918(0,7);6,905(0,4);6,882(0,4);3,832(0,5);3,81 3(4,9);3,797(9,8);3,781(5,3);3,692(0,5);3,669(0,9);3,645(0,6);3,130(0,6);3,106(1,1);3,082(0,7);2,881(0,4);2,765(8,2);2,749(16,0);2,733(7,8); 2,452(0,7);2,430(0,7);2,407(1,2);2,391(1,5);2,353(0,4);2,343(0,4);2,308(1,5);2,275(38,7);2,248(0,8);2,225(4,0);2,136(25,8);2,119(0,4);2,112 (0,6);2,106(0,6);2,100(0,4);1,963(2,8);1,957(3,5);1,951(27,3);1,945(50,8);1,939(70,1);1,933(47,4);1,926(24,0);1,912(1,0);1,767(0,4);1,599(0 ,6);1,372(5,8);1,362(0,5);1,340(2,7);1,294(0,7);1,285(3,4);1,276(7,0);1,271(1,8);1,263(0,8);1,260(0,7);1,243(0,4);1,215(1,3);1,197(2,4);1,17 9(1,2);1,001(1,2);0,983(2,3);0,966(1,1);0,146(0,6);0,008(5,4);0,000(132,4);-0,009(4,1);-0,150(0,6) |
| I-4-6: |
| HPLC-MS: logP = 4,45; Masse (m/z): 434,1 (M+H)⁺; ¹H-NMR(400,0 MHz, CD₃CN): |
| δ= 7,618(1,8);7,598(1,9);7,381(0,7);7,378(0,7);7,362(1,9);7,359(1,8);7,343(1,4);7,340(1,2);7,261(1,3);7,242(2,2);7,235(2,3);7,223(1,0);7,21 6(1,7);7,153(0,4);7,148(0,4);7,132(0,9);7,126(0,3);7,118(0,4);7,111(0,6);6,949(1,5);6,928(2,2);6,906(1,1);3,841(1,2);3,816(2,6);3,791(1,4);3, 159(1,4);3,134(2,7);3,109(1,2);2,352(16,0);2,141(6,7);1,963(0,5);1,957(0,7);1,951(4,8);1,945(8,9);1,939(12,2);1,933(8,3);1,927(4,2);1,341(0 ,3);1,285(0,4);0,008(0,9);0,000(19,9);-0,009(0,6) |
| I-4-7: |
| HPLC-MS: logP = 2,34; Masse (m/z): 370,1 (M+H)⁺; ¹H-NMR(400,0 MHz, CD₃CN): |
| δ= 8,897(2,7);7,763(3,1);7,745(4,3);7,698(1,3);7,682(3,7);7,680(3,6);7,664(4,0);7,659(3,5);7,655(3,1);7,653(3,5);7,636(3,3);7,634(3,2);7,61 7(1,4);7,604(0,7);7,594(0,5);7,541(3,9);7,540(3,9);7,524(3,0);7,523(3,2);7,240(0,3);7,218(1,1);7,203(2,1);7,198(1,5);7,196(1,5);7,188(1,2);7, 182(4,0);7,176(1,6);7,168(1,5);7,166(1,7);7,161(2,9);7,146(1,5);7,035(0,7);7,030(1,2);7,020(6,6);7,014(1,1);7,009(1,1);6,997(8,9);6,986(1,4) ;6,975(4,8);6,965(0,9);6,960(0,7);6,945(0,5);6,922(0,7);6,901(0,4);3,797(2,7);3,794(4,5);3,791(2,8);3,781(5,5);3,778(9,1);3,775(5,3);3,765(3 ,0);3,762(4,7);3,759(2,7);3,445(0,7);3,427(0,4);3,279(0,5);3,266(0,5);2,754(8,2);2,738(16,0);2,721(7,6);2,146(9,8);1,963(0,8);1,957(1,1);1,9 51(8,9);1,945(16,6);1,939(23,0);1,932(15,7);1,926(8,0);1,373(2,1);1,341(0,9);1,285(1,2);1,276(2,8);1,270(1,5);1,171(0,5);1,006(0,5);0,008(1 ,9);0,000(47,8);-0,009(1,5) |
| I-4-8: |
| HPLC-MS: logP = 2,37; Masse (m/z): 378,0 (M+H)⁺; ¹H-NMR(400,0 MHz, CD₃CN): |
| δ= 8,819(2,3);7,650(4,1);7,631(4,4);7,629(4,4);7,498(3,4);7,495(3,7);7,478(4,5);7,474(4,7);7,458(0,7);7,450(1,6);7,445(4,2);7,439(9,8);7,43 2(7,3);7,422(8,8);7,421(8,8);7,404(1,5);7,401(1,4);7,397(0,6);7,384(3,2);7,377(2,6);7,368(2,1);7,364(3,1);7,361(2,2);7,358(2,5);7,348(3,5);7, 343(3,3);7,328(3,8);7,325(3,8);7,308(2,4);7,304(2,2);7,290(0,4);7,286(0,4);7,259(0,3);7,253(0,5);7,245(0,6);7,236(0,6);7,231(0,4);7,149(2,8) ;7,146(2,9);7,130(3,7);7,127(3,8);7,111(1,9);7,107(1,9);5,447(2,0);3,813(5,6);3,797(11,7);3,780(6,0);3,390(0,4);3,373(0,9);3,355(0,4);2,876( 7,8);2,860(16,0);2,843(7,4);2,713(0,5);2,172(41,2);2,169(40,4);1,964(1,2);1,958(1,5);1,952(12,1);1,946(22,9);1,940(32,1);1,934(22,5);1,928 (12,0);1,372(2,2);1,340(0,8);1,285(1,0);1,276(2,5);1,271(1,0);0,008(0,7);0,000(19,6) |
| I-2-1 |
| siehe Synthesebeispiel 20 |
| I-2-2 |
| HPLC-MS: logP = 2,89; Masse (m/z): 333.0 (M+H)⁺; 1H-NMR [CD₃CN] 7.43-7.47 (m, 1H), 7.48 7.55 (m, 4H), 7.60-7.66 (m, 3H), 8.38 (s, 1H), 9.50 (br. s, 1H). |
| I-2-3 |
| HPLC-MS: logP = 3,03; Masse (m/z): 424.9 (M+H)⁺; ¹H-NMR [CD₃CN] 7.22 - 7.26 (m, 1H), 7.48 7.55 (m, 4H), 7.61 - 7.66 (m, 2H), 7.97-7.99 (m, 1H), 8.38 (s, 1H), 9.43 (br. s, 1H). |
| I-2-4 |
| HPLC-MS: logP = 2,54; Masse (m/z): 368.0 (M+H)⁺; ¹H-NMR [CD₃CN] 7.48-7.56 (m, 2H), 7.62 - 7.66 (m, 2H), 7.73 - 7.76 (m, 1H), 8.11 - 8.13 (m, 1H), 8.36 (s, 1H), 8.83 - 8.84 (m, 1H), 9.68 (br. s, 1H). |
| I-2-5 |
| HPLC-MS: logP = 2,20; Masse (m/z): 334.0 (M+H)⁺; ¹H-NMR [CD₃CN] 7.46 - 7.56 (m, 3H), 7.56 - 7.66 (m, 2H), 8.01 - 8.03 (m, 1H), 8.38 (s, 1H), 8.51 - 8.53 (m, 1H), 9.64 (br. s, 1H). |
| I-2-6 |
| HPLC-MS: logP = 3,03; Masse (m/z): 410.9 (M+H)⁺; ¹H-NMR [CD₃CN] 7.45 - 7.47 (m, 1H), 7.53 - 7.56 (m, 1H), 7.58 - 7.60 (m, 1H), 7.69 - 7.77 (m, 3H), 7.81 - 7.85 (m, 2H), 8.35 (s, 1H), 9.50 (br. s, 1H). |
| I-2-7 |
| HPLC-MS: logP = 2,93; Masse (m/z): 376.9 (M+H)⁺; 1H-NMR [CD₃CN] 7.42 - 7.64 (m, 7H), 7.81 - 7.83 (m, 1H), 8.37 (s, 1H), 9.59 (br. s, 1H). |
| I-2-8 |
| HPLC-MS: logP = 2,58; Masse (m/z): 411.9 (M+H)⁺; ¹H-NMR [CD₃CN] 7.43 - 7.49 (m, 1H), 7.53 - 7.61 (m, 2H), 7.73 - 7.76 (m, 1H), 7.81 - 7.83 (m, 1H), 8.11 - 8.13 (m, 1H), 8.35 (s, 1H), 8.83 - 8.84 (m, 1H), 9.67 (br. s, 1H). |
| I-2-9 |
| HPLC-MS: logP = 2,29; Masse (m/z): 377.9 (M+H)⁺; ¹H-NMR [CD₃CN] 7.44 - 7.50 (m, 2H), 7.53 - 7.61 (m, 2H), 7.81 - 7.83 (m, 1H), 8.01 - 8.03 (m, 1H), 8.37 (s, 1H), 8.51 - 8.53 (m, 1H), 9.62 (br. s, 1H). |
| I-2-10 |
| HPLC-MS: logP = 2,38; Masse (m/z): 368.1 (M+H)⁺; ¹H-NMR [DMSO-D₆] 7.70 - 7.81 (m, 4H), 7.87 - 7.88 (m, 1H), 8.32 - 8.34 (m, 1H), 8.42 (s, 1H), 8.61 - 8.63 (m, 1H), 11.85 (br. s, 1H). |
| I-2-11 |
| HPLC-MS: logP = 2,60; Masse (m/z): 402.0 (M+H)⁺; ¹H-NMR [CD₃CN] 7.58 - 7.61 (m, 1H), 7.72 - 7.76 (m, 3H), 7.84 - 7.86 (m, 1H), 8.12 - 8.14 (m, 1H), 8.55 - 8.56 (m, 1H), 8.94 (br. s, 1H). |
| I-2-12 |
| HPLC-MS: logP = 2.22; Masse (m/z): 334,0 (M+H)⁺; ¹H-NMR [DMSO-D₆] 7.45 - 7.49 (m, 1H), 7.51 - 7.60 (m, 2H), 7.64 - 7.66 (m, 1H), 7.70 - 7.73 (m, 1H), 8.32 - 8.34 (m, 1H), 8.44 (s, 1H), 8.61 - 8.63 (m, 1H), 11.77 (s, 1H). |
| I-2-13 |
| HPLC-MS: logP = 2.26; Masse (m/z): 377,9 (M+H)⁺; ¹H-NMR [DMSO-D₆] 7.43 - 7.47 (m, 1H), 7.49 - 7.53 (m, 1H), 7.60 - 7.63 (m, 1H), 7.71 - 7.75 (m, 2H), 8.32 - 8.34 (m, 1H), 8.44 (s, 1H), 8.61 - 8.63 (m, 1H), 11.75 (s, 1H). |
| I-2-14 |
| HPLC-MS: logP = 2.36; Masse (m/z): 425,9 (M+H)⁺; ¹H-NMR [DMSO-D₆] 7.23 - 7.28 (m, 1H), 7.49 - 7.56 (m, 2H), 7.70 - 7.72 (m, 1H), 7.94 - 7.96 (m, 1H), 8.32 - 8.34 (m, 1H), 8.43 (s, 1H), 8.61 - 8.63 (m, 1H), 11.69 (s, 1H). |
| I-2-15 |
| HPLC-MS: logP = 2.09; Masse (m/z): 336,1 (M+H)⁺; ¹H-NMR [DMSO-D₆] 7.25 - 7.30 (m, 2H), 7.59 - 7.67 (m, 1H), 7.71 - 7.74 (m, 1H), 8.32 - 8.35 (m, 1H), 8.44 (s, 1H), 8.62 - 8.63 (m, 1H), 12.07 (s, 1H). |
| I-2-16 |
| HPLC-MS: logP = 2.19; Masse (m/z): 389,9 (M+H)⁺; ¹H-NMR [DMSO-D₆] 3.88 (s, 3H), 7.70 - 7.73 (m, 1H), 8.31 - 8.34 (m, 1H), 8.38 (s, 1H), 8.61 - 8.63 (m, 1H), 11.60 (s, 1H). |
| I-2-17 |
| HPLC-MS: logP = 2.46; Masse (m/z):412,0 (M+H)⁺; 1H-NMR [DMSO-D₆] 7.61 - 7.65 (m, 1H), 7.72 - 7.83 (m, 3H), 7.87 - 7.88 (m, 1H), 8.39 (s, 1H), 8.45 - 8.47 (m, 1H), 8.64 - 8.66 (m, 1H), 11.84 (s, 1H). |
| I-2-18 |
| HPLC-MS: logP = 2.43; Masse (m/z): 469,8 (M+H)⁺; ¹H-NMR [DMSO-D₆] 7.23 - 7.28 (m, 1H), 7.49 - 7.56 (m, 2H), 7.61 - 7.64 (m, 1H), 7.94 - 7.96 (m, 1H), 8.40 (s, 1H), 8.44 - 8.47 (m, 1H), 8.64 - 8.65 (m, 1H), 11.68 (s, 1H). |
| I-2-19: |
| HPLC-MS: logP = 3,21; Masse (m/z): 401,0 (M+H)⁺; ¹H-NMR(400,0 MHz, DMSO-D₆): |
| δ= 11,142(10,9);7,903(4,9);7,884(7,4);7,855(2,3);7,837(5,7);7,818(6,2);7,811(6,5);7,807(6,4);7,792(11,9);7,787(10,1);7,773(16,0);7,764(8,2 );7,673(2,6);7,670(2,9);7,655(6,0);7,651(5,8);7,635(4,5);7,631(4,3);7,621(5,3);7,618(5,3);7,602(6,0);7,599(6,0);7,583(2,5);3,324(66,4);2,675 (0,9);2,671(1,2);2,666(0,9);2,662(0,4);2,524(4,5);2,511(67,9);2,506(132,1);2,502(171,5);2,497(123,4);2,493(59,6);2,333(0,8);2,329(1,1);2,3 24(0,8);1,989(0,4);1,397(1,3);1,336(1,5);1,299(2,5);1,259(3,5);1,249(1,5);1,235(1,4);0,008(0,4);0,000(9,1) |
| I-2-20: |
| HPLC-MS: logP = 4,87; Masse (m/z): 573,0 (M+H)⁺; ¹H-NMR(400,0 MHz, DMSO-D₆): |
| δ= 7,924(9,9);7,903(16,0);7,880(11,4);7,840(5,8);7,824(11,5);7,805(6,5);7,783(7,7);7,764(9,3);7,745(3,5);7,698(3,5);7,694(3,5);7,677(8,7);7 ,675(7,2);7,664(4,7);7,660(5,9);7,656(5,0);7,652(4,2);7,642(9,4);7,637(9,4);7,633(3,7);7,618(3,8);7,613(2,4);7,580(5,2);7,576(4,9);7,561(5,8 );7,557(5,5);7,542(2,3);7,538(2,1);3,831(0,3);3,812(0,3);3,789(0,4);3,770(0,5);3,750(0,3);3,329(18,1);3,179(0,5);3,166(0,5);2,678(0,4);2,674 (0,5);2,669(0,3);2,527(2,2);2,514(31,1);2,509(59,6);2,505(76,3);2,500(54,7);2,496(26,5);2,336(0,4);2,332(0,5);2,327(0,4);1,339(4,1);1,301(2 ,0);1,259(2,9);1,250(5,0);1,235(2,1);0,000(4,5) |
| I-2-21: |
| HPLC-MS: logP = 2,93; Masse (m/z): 381,0 (M+H)⁺; ¹H-NMR(400,0 MHz, CD₃CN): |
| δ= 8,847(1,0);7,854(1,4);7,834(1,9);7,760(2,3);7,752(4,9);7,732(0,4);7,721(0,9);7,712(1,0);7,701(1,0);7,692(0,8);7,680(0,4);7,649(1,1);7,64 3(1,0);7,635(0,9);7,630(1,2);7,625(1,9);7,622(1,5);7,615(1,1);7,610(1,0);7,607(1,1);7,605(1,1);7,597(1,7);7,528(0,6);7,516(1,9);7,510(2,8);7, 502(2,9);7,492(2,1);7,475(0,4);5,446(2,0);2,367(16,0);2,141(22,9);1,963(0,7);1,952(7,5);1,945(13,8);1,939(19,2);1,933(13,2);1,927(6,7);0,0 00(1,4) |
| I-2-22: |
| HPLC-MS: logP = 4,54; Masse (m/z): 553,2 (M+H)⁺; ¹H-NMR(400,0 MHz, CD₃CN): |
| δ= 7,815(2,2);7,797(3,1);7,699(1,4);7,695(2,7);7,691(2,6);7,684(9,7);7,682(8,0);7,675(3,1);7,665(1,5);7,656(1,4);7,646(1,2);7,633(0,5);7,55 |
| 2(0,7);7,548(0,9);7,532(1,8);7,528(2,0);7,515(0,9);7,511(1,0);7,497(1,6);7,493(1,5);7,477(0,8);7,473(0,8);7,463(1,1);7,458(1,0);7,444(1,5);7, 439(1,4);7,425(0,9);7,421(0,8);7,347(1,7);7,343(1,5);7,328(1,3);7,323(1,1);2,386(16,0);2,146(9,3);1,964(0,6);1,957(1,0);1,952(8,9);1,946(16 ,7);1,939(23,3);1,933(16,0);1,927(8,2);1,436(6,0);1,372(3,1);1,341(0,6);1,285(0,8);1,276(3,5);0,000(1,6) |
| I-2-23: |
| HPLC-MS: logP = 3,64; Masse (m/z): 550,9 (M+H)⁺; ¹H-NMR(400,0 MHz, CD₃CN): |
| δ= 8,489(11,3);8,484(11,6);8,477(11,6);8,472(11,4);8,050(11,4);8,045(11,2);8,031(12,2);8,026(11,6);7,778(4,5);7,775(4,8);7,759(5,7);7,755( 5,9);7,572(1,6);7,568(1,9);7,553(5,2);7,549(4,8);7,534(6,1);7,530(5,4);7,522(3,8);7,516(6,0);7,502(4,3);7,497(7,6);7,490(7,9);7,485(5,2);7,4 78(2,6);7,472(16,0);7,466(4,2);7,460(12,3);7,453(11,8);7,441(11,3);7,426(0,4);7,420(0,4);7,241(0,4);7,235(0,4);5,448(4,6);2,464(0,4);2,269( 0,4);2,224(0,6);2,161(62,5);2,114(0,5);2,108(0,6);2,102(0,4);2,042(0,4);1,965(5,5);1,959(9,0);1,953(39,4);1,947(68,6);1,940(89,5);1,934(60, 7);1,928(30,8);1,915(0,3);1,775(0,4);1,769(0,5);1,763(0,4);1,386(0,7);1,372(9,0);1,340(2,1);1,285(3,0);1,276(10,2);1,270(1,9);1,216(0,4);0,8 81(0,4);0,146(0,6);0,008(6,2);0,000(143,9);-0,009(4,3);-0,150(0,6) |
| I-2-24: |
| HPLC-MS: logP = 2,92; Masse (m/z): 439,0 (M+H)⁺; ¹H-NMR(400,0 MHz, CD₃CN): |
| δ= 8,793(1,0);7,989(2,0);7,970(2,1);7,650(1,2);7,637(1,3);7,626(2,9);7,614(1,7);7,603(1,8);7,574(1,3);7,555(2,4);7,532(1,9);7,513(4,6);7,50 1(3,7);7,493(3,1);7,475(0,6);7,256(1,1);7,239(1,9);7,219(0,9);2,424(16,0);2,143(18,3);1,971(0,4);1,964(1,3);1,957(1,9);1,952(12,6);1,946(23 ,4);1,939(32,3);1,933(22,4);1,927(11,7);1,372(3,1);1,340(0,7);1,284(1,0);1,276(3,3);1,220(0,4);0,000(0,9) |
| I-2-25: |
| HPLC-MS: logP = 4,68; Masse (m/z): 668,9 (M+H)⁺; ¹H-NMR(400,0 MHz, CD₃CN): |
| δ= 7,922(2,8);7,921(2,9);7,902(3,0);7,901(3,0);7,608(2,2);7,604(2,4);7,589(3,0);7,585(3,7);7,581(1,4);7,565(1,5);7,561(1,7);7,526(0,6);7,52 1(0,7);7,507(1,3);7,503(1,3);7,488(0,9);7,483(0,9);7,478(1,0);7,474(1,0);7,464(1,8);7,462(1,9);7,459(1,7);7,455(1,5);7,445(3,1);7,443(3,2);7, 436(0,8);7,426(1,6);7,424(1,6);7,388(1,6);7,383(1,4);7,368(1,1);7,364(1,0);7,192(1,6);7,188(1,6);7,172(2,5);7,169(2,6);7,153(1,3);7,149(1,4) ;5,447(6,0);2,443(16,0);2,286(0,6);2,146(27,6);1,964(0,5);1,958(0,8);1,952(7,2);1,946(13,6);1,940(19,11,933(13,1););1,927(6,7);1,372(2,4);1 ,340(0,4);1,284(0,6);1,276(2,7);1,263(0,6);1,216(0,5);0,000(1,3) |
| I-2-26: |
| HPLC-MS: logP = 3,22; Masse (m/z): 444,9 (M+H)⁺; ¹H-NMR(400,0 MHz, CD₃CN): |
| δ= 8,914(3,7);7,860(6,0);7,844(9,7);7,830(7,3);7,758(15,3);7,735(5,1);7,715(4,7);7,651(4,2);7,632(6,7);7,592(4,0);7,573(7,0);7,554(3,9);7,5 17(4,8);7,497(6,6);7,480(2,9);7,447(0,4);7,426(0,8);7,420(0,8);7,262(0,4);7,257(0,4);7,241(0,6);7,236(0,5);7,171(0,5);7,165(0,5);7,150(0,3); 5,447(1,4);2,144(88,5);2,119(0,6);2,113(0,9);2,107(1,0);2,101(0,7);2,095(0,4);1,964(7,6);1,958(11,2);1,952(60,9);1,946(110,3);1,940(148,5) ;1,933(101,8);1,927(52,0);1,914(0,8);1,780(0,3);1,774(0,6);1,768(0,9);1,762(0,6);1,756(0,4);1,402(0,4);1,396(0,4);1,386(0,6);1,372(14,2);1, 340(2,3);1,284(3,2);1,276(16,0);1,216(0,5);0,881(0,3);0,146(0,8);0,008(7,3);0,000(194,2);-0,009(6,2);-0,150(0,8) |
| I-2-27: |
| HPLC-MS: logP = 4,87; Masse (m/z): 617,0 (M+H)⁺; ¹H-NMR(400,0 MHz, CD₃CN): |
| δ= 7,837(6,5);7,819(8,5);7,816(8,0);7,779(0,4);7,769(2,5);7,765(3,8);7,745(16,0);7,741(10,2);7,726(14,8);7,722(12,9);7,704(8,4);7,686(5,6); 7,682(5,1);7,668(1,9);7,664(1,7);7,552(1,5);7,549(1,8);7,534(4,8);7,530(5,0);7,515(5,3);7,511(4,8);7,504(3,8);7,499(5,1);7,485(4,1);7,480(5, 3);7,473(0,4);7,466(1,9);7,461(1,7);7,443(6,0);7,438(5,4);7,424(3,6);7,419(3,7);2,467(0,3);2,463(0,5);2,458(0,3);2,269(0,5);2,148(89,1);2,11 9(0,6);2,113(0,8);2,107(1,0);2,101(0,7);2,095(0,4);1,964(8,3);1,958(13,5);1,952(65,0);1,946(116,5);1,940(154,0);1,933(105,1);1,927(53,8);1 ,914(0,8);1,780(0,4);1,774(0,7);1,768(0,9);1,762(0,6);1,756(0,3);1,437(2,0);1,376(1,5);1,372(6,0);1,340(1,2);1,285(1,6);1,276(6,7);1,217(0,4 );0,146(1,0);0,008(8,3);0,000(245,9);-0,009(8,2);-0,150(1,0) |
| I-2-28: |
| HPLC-MS: logP = 2,77; Masse (m/z): 445,9 (M+H)⁺; ¹H-NMR(400,0 MHz, CD₃CN): |
| δ= 8,999(3,6);8,855(6,0);8,844(6,1);8,816(0,7);8,156(5,6);8,137(6,1);8,100(0,6);7,852(5,8);7,832(6,5);7,771(4,5);7,759(5,2);7,752(4,8);7,74 0(4,5);7,723(0,9);7,711(0,7);7,693(0,4);7,680(0,5);7,651(4,3);7,632(7,4);7,596(3,9);7,577(6,8);7,558(3,7);7,521(4,5);7,502(6,3);7,484(2,9);7, 464(0,8);7,458(0,8);7,447(0,7);7,426(1,0);7,420(0,9);7,261(0,4);7,256(0,4);7,241(0,7);7,235(0,7);7,171(0,5);7,165(0,5);7,150(0,4);7,144(0,3) ;5,447(0,4);2,141(55,3);2,113(2,5);2,107(2,2);2,101(1,5);2,095(0,9);1,964(9,6);1,958(15,8);1,952(80,9);1,946(144,9);1,940(193,5);1,934(133 ,4);1,927(69,0);1,780(0,5);1,774(0,9);1,768(1,2);1,762(0,8);1,756(0,5);1,386(0,8);1,372(15,0);1,340(4,2);1,285(5,5);1,276(16,0);1,217(0,6);0 ,881(0,6);0,858(0,4);0,146(1,0);0,008(9,4);0,000(227,6);-0,009(9,5);-0,150(1,1) |
| I-2-29: |
| HPLC-MS: logP = 3,09; Masse (m/z): 412,9 (M+H)⁺; ¹H-NMR(400,0 MHz, CD₃CN): |
| δ= 8,843(1,6);7,844(3,2);7,825(3,6);7,644(4,1);7,588(2,8);7,569(5,0);7,549(4,4);7,537(5,5);7,515(6,5);7,495(6,1);7,472(4,0);7,453(3,1);7,42 6(1,5);7,420(1,2);7,261(0,4);7,256(0,4);7,240(0,6);7,235(0,6);7,171(0,5);7,165(0,5);7,150(0,3);7,143(0,3);5,446(0,7);2,139(37,2);2,119(0,4); 2,113(0,5);2,107(0,7);2,101(0,4);1,964(4,9);1,958(7,3);1,952(39,9);1,946(71,4);1,1,939(95,7);1,933(65,2);1,927(33,2);1,914(0,4);1,774(0,4);1, 768(0,6);1,762(0,4);1,432(1,3);1,386(0,7);1,372(15,0);1,340(2,8);1,284(3,7);1,276(16,0);1,216(0,8);0,881(0,4);0,857(0,4);0,146(0,6);0,008(4 ,8);0,000(127,1);-0,009(3,7);-0,149(0,6) |
| I-2-30: |
| HPLC-MS: logP = 4,87; Masse (m/z): 550,9 (M+H)⁺; ¹H-NMR(400,0 MHz, CD₃CN): |
| δ= 7,774(2,2);7,771(2,3);7,755(2,8);7,751(2,8);7,685(3,0);7,682(4,9);7,679(3,7);7,666(3,5);7,664(5,0);7,663(4,8);7,661(4,2);7,560(0,8);7,55 6(1,0);7,541(2,6);7,537(2,8);7,522(2,8);7,518(2,6);7,512(0,7);7,508(2,2);7,503(2,8);7,495(0,5);7,488(2,3);7,484(3,2);7,474(0,9);7,463(11,2); 7,460(5,3);7,454(16,0);7,451(8,7);7,446(1,2);7,440(2,0);7,435(2,2);7,431(1,0);7,425(1,2);7,414(4,3);7,403(5,0);7,395(3,7);7,393(2,8);7,386( 2,7);7,383(2,8);7,374(1,9);5,446(0,8);2,547(1,0);2,295(0,3);2,137(11,6);1,964(2,5);1,958(4,0);1,952(18,6);1,946(32,8);1,940(43,2);1,933(29, 8);1,927(15,3);1,372(2,4);1,340(0,4);1,284(0,6);1,276(2,6);1,217(0,3);0,015(0,3);0,008(2,5);0,000(69,8);-0,009(2,3) |
| I-2-31: |
| HPLC-MS: logP = 2,46; Masse (m/z): 411,9 (M+H)⁺; ¹H-NMR(400,0 MHz, CD₃CN): |
| δ= 9,068(2,5);8,524(4,9);8,045(3,9);8,028(4,2);7,848(4,7);7,828(5,3);7,650(3,1);7,632(4,7);7,591(3,9);7,573(6,6);7,554(3,8);7,517(5,4);7,49 8(9,7);7,482(6,7);7,426(1,0);7,420(1,0);7,411(0,4);7,399(0,3);7,393(0,3);7,261(0,4);7,256(0,4);7,240(0,6);7,235(0,6);7,171(0,5);7,165(0,5);7, 150(0,3);2,465(0,4);2,460(0,5);2,455(0,4);2,158(128,3);2,120(0,7);2,114(0,9);2,107(1,1);2,101(0,8);2,095(0,4);1,964(8,6);1,958(14,3);1952( 67,7);1,946(119,5);1,940(156,8);1,934(106,8);1,928(54,6);1,915(1,0);1,781(0,4);1,775(0,7);1,769(1,0);1,762(0,7);1,756(0,4);1,386(1,1);1,37 2(14,4);1,340(3,8);1,308(0,4);1,297(0,7);1,284(5,1);1,276(16,0);1,270(3,4);1,217(0,7);1,200(0,6);1,017(0,5);0,963(0,4);0,881(0,6);0,856(0,5) ;0,708(0,4);0,146(1,1);0,008(9,6);0,000(260,2);-0,009(8,5);-0,019(0,5);-0,150(1,2) |
| I-2-32: |
| HPLC-MS: logP = 3,24; Masse (m/z): 502,9 (M+H)⁺; ¹H-NMR(400,0 MHz, CD₃CN): |
| δ= 8,786(1,3);7,997(1,8);7,978(1,9);7,846(2,2);7,827(2,4);7,634(1,8);7,589(2,2);7,572(4,2);7,554(3,4);7,540(2,1);7,516(3,8);7,496(3,6);7,47 7(1,4);7,448(0,4);7,426(0,8);7,420(0,8);7,262(1,4);7,256(1,6);7,248(1,9);7,241(1,7);7,235(1,6);7,171(0,5);7,165(0,6);7,150(0,4);7,143(0,4);5, 446(0,5);4,055(0,5);2,134(27,2);2,119(0,4);2,113(0,5);2,107(0,5);2,101(0,4);1,971(1,1);1,964(4,1);1,958(6,4);1,952(33,2);1,946(59,2);1,939( 78,9);1,933(54,2);1,927(27,9);1,774(0,3);1,768(0,5);1,437(1,0);1,432(0,4);1,386(0,6);1,372(15,5);1,340(2,9);1,285(4,0);1,276(16,0);1,216(1, 6);1,203(0,5);0,881(0,4);0,146(0,5);0,008(4,5);0,000(110,1);-0,009(4,0);-0,150(0,5) |
| I-2-33: |
| HPLC-MS: logP = 3,06; Masse (m/z): 471,0 (M+H)⁺; ¹H-NMR(400,0 MHz, CD₃CN): |
| δ= 9,482(2,7);8,371(16,0);7,986(6,8);7,967(6,9);7,833(5,6);7,830(5,7);7,813(6,3);7,810(6,3);7,611(3,1);7,607(3,7);7,591(7,6);7,587(7,8);7,5 71(4,4);7,568(4,4);7,553(8,0);7,550(8,3);7,534(11,2);7,529(15,7);7,511(6,6);7,509(6,6);7,492(2,3);7,490(2,3);7,483(4,4);7,479(4,1);7,463(5, 2);7,460(4,8);7,445(2,6);7,440(2,4);7,264(3,4);7,258(3,4);7,246(4,0);7,244(4,4);7,241(4,4);7,238(3,9);7,226(2,8);7,221(2,7);2,462(0,4);2,148 (212,3);2,146(201,8);2,119(0,9);2,113(1,2);2,107(1,3);2,101(0,9);2,095(0,5);1,964(12,7);1,958(20,2);1,952(86,6);1,946(151,0);1,940(197,2); 1,933(135,1);1,927(69,2);1,780(0,5);1,774(0,8);1,768(1,1);1,762(0,8);1,756(0,4);0,146(1,2);0,008(12,3);0,000(262,3);-0,009(9,9);-0,150(1,2) |
| I-2-34: |
| HPLC-MS: logP = 5,06; Masse (m/z): 732,7 (M+H)⁺; ¹H-NMR(400,0 MHz, CD₃CN): |
| δ= 7,934(7,3);7,932(7,5);7,914(7,8);7,912(7,8);7,782(3,0);7,778(3,2);7,763(3,6);7,759(3,9);7,664(6,0);7,660(6,3);7,645(7,5);7,641(7,4);7,56 9(1,0);7,565(1,2);7,550(3,3);7,546(3,2);7,531(3,7);7,527(3,3);7,515(2,2);7,510(3,7);7,496(2,8);7,490(7,0);7,487(5,3);7,483(5,5);7,478(3,6);7, 470(8,9);7,468(8,7);7,460(2,5);7,451(4,2);7,449(4,4);7,420(0,4);7,212(4,3);7,208(4,4);7,192(6,8);7,188(6,8);7,173(3,6);7,169(3,6);5,446(1,7) ;4,068(0,5);4,050(0,5);2,306(0,4);2,140(20,6);2,113(0,4);2,107(0,5);2,101(0,3);1,971(2,4);1,964(3,9);1,958(6,4);1,952(31,1);1,946(55,0);1,9 40(72,4);1,933(49,2);1,927(25,2);1,914(0,4);1,768(0,4);1,436(16,0);1,386(0,3);1,371(4,8);1,340(0,9);1,284(1,2);1,276(5,1);1,221(0,6);1,204( 1,11,186(0,6);0,146(0,6);0,008(4,8);0,000(132,6);-0,009(4,1);-0,150(0,6) |
| I-2-35 siehe Synthesebeispiel 21 |
| I-2-36: |
| HPLC-MS: logP = 2,71; Masse (m/z): 381,0 (M+H)⁺; ¹H-NMR(400,0 MHz, DMSO-D₆): |
| δ= 11,736(7,7);8,464(16,0);7,773(0,9);7,757(2,0);7,751(2,2);7,742(6,1);7,735(4,2);7,725(6,0);7,722(6,6);7,714(2,6);7,698(1,1);7,627(3,7);7, 622(4,1);7,608(5,1);7,603(5,4);7,526(2,3);7,523(2,6);7,507(5,3);7,505(5,6);7,488(6,5);7,485(8,6);7,464(12,2);7,446(5,5);7,442(8,8);7,427(2, 0);7,423(1,8);5,757(4,0);3,327(14,2);2,957(0,4);2,511(18,0);2,507(35,2);2,503(45,8);2,498(33,8);2,087(1,6);1,990(0,3);0,008(1,3);0,000(30, 5);-0,008(1,2) |
| I-2-37: |
| HPLC-MS: logP = 2,09; Masse (m/z): 380,0 (M+H)⁺; ¹H-NMR(400,0 MHz, DMSO-D₆): |
| δ= 11,907(8,8);8,526(4,9);8,522(5,6);8,515(5,4);8,510(5,4);8,476(16,0);8,089(4,9);8,084(5,2);8,070(5,5);8,065(5,4);7,777(0,9);7,761(2,0);7, 756(2,0);7,739(3,8);7,723(2,1);7,718(2,5);7,702(1,1);7,609(4,9);7,597(5,0);7,590(5,0);7,578(4,5);7,490(6,1);7,469(10,8);7,447(5,0);7,355(0, 4);7,333(0,6);5,758(1,2);5,461(0,4);3,328(16,4);2,672(0,3);2,508(43,2);2,504(54,1);2,499(42,0);2,330(0,3);2,087(0,8);0,000(0,8) |
| I-2-38: |
| HPLC-MS: logP = 2,66; Masse (m/z): 335,0 (M+H)⁺; ¹H-NMR(400,0 MHz, DMSO-D₆): |
| δ= 11,753(7,9);8,470(16,0);7,774(0,9);7,758(2,1);7,752(1,8);7,736(3,9);7,731(1,6);7,720(2,0);7,715(2,4);7,699(1,1);7,659(4,3);7,656(4,3);7, 641(5,3);7,637(5,1);7,595(3,1);7,593(3,3);7,575(7,2);7,573(6,9);7,555(3,2);7,551(3,2);7,537(5,1);7,533(4,5);7,517(2,7);7,513(2,3);7,486(9,9) ;7,465(15,1);7,449(3,2);7,444(5,7);5,758(2,2);3,330(10,7);2,508(30,3);2,504(37,8);2,499(27,3);2,087(0,8);0,000(26,6);-0,008(1,1) |
| I-2-39: |
| HPLC-MS: logP = 2,88; Masse (m/z): 387,0 (M+H)⁺; ¹H-NMR(400,0 MHz, DMSO-D₆): |
| δ= 12,099(5,7);8,465(16,0);7,828(0,5);7,809(1,7);7,800(1,1);7,789(3,6);7,776(6,4);7,754(10,5);7,739(5,2);7,731(3,6);7,723(1,8);7,718(2,2);7 ,702(1,1);7,603(0,4);7,492(5,1);7,470(8,8);7,449(4,0);7,446(2,8);7,376(0,5);7,355(0,9);7,333(1,6);5,758(1,8);5,462(0,8);3,327(8,0);2,960(2,0 );2,673(0,3);2,526(1,2);2,513(20,5);2,509(40,7);2,504(53,3);2,500(38,8);2,495(18,9);2,331(0,3);0,000(1,2) |
| I-2-40: |
| HPLC-MS: logP = 2,90; Masse (m/z): 432,9 (M+H)⁺; ¹H-NMR(400,0 MHz, DMSO-D₆): |
| δ= 11,440(9,1);8,458(13,3);8,393(0,6);7,844(12,1);7,830(12,8);7,772(1,1);7,756(2,5);7,750(2,2);7,740(1,8);7,734(4,8);7,728(2,5);7,718(2,3); 7,713(3,0);7,697(1,4);7,488(7,2);7,467(12,5);7,444(16,0);7,430(10,8);7,134(0,7);7,120(0,6);5,757(1,5);3,326(19,4);2,677(0,3);2,672(0,5);2,6 68(0,4);2,526(1,6);2,512(30,0);2,508(60,1);2,503(79,2);2,499(58,5);2,495(29,4);2,335(0,4);2,330(0,5);2,326(0,4);0,000(1,7) |
| I-2-41: |
| HPLC-MS: logP = 2,81; Masse (m/z): 427,0 (M+H)⁺: ¹H-NMR(400,0 MHz, DMSO-D₆): |
| δ= 11,679(7,8);8,465(16,0);7,961(5,4);7,942(5,6);7,773(0,9);7,757(1,9);7,751(1,7);7,741(1,3);7,735(3,6);7,730(1,4);7,719(1,8);7,714(2,2);7, 698(1,0);7,559(1,9);7,554(2,6);7,540(6,5);7,535(6,3);7,528(4,4);7,527(4,4);7,509(5,1);7,489(4,7);7,485(5,7);7,464(9,6);7,443(4,5);7,275(2,6) ;7,269(2,7);7,255(3,6);7,250(3,6);7,237(2,3);7,232(2,2);5,757(4,2);3,333(39,2);2,512(11,3);2,508(22,1);2,503(28,8);2,499(20,7);2,495(9,9);2 ,087(1,1);0,008(0,9);0,000(21,4);-0,009(0,7) |
| I-2-42: |
| HPLC-MS: logP = 3,02; Masse (m/z): 402,1 (M+H)⁺; ¹H-NMR(400,0 MHz, DMSO-D₆): |
| δ= 12,014(3,8);8,987(2,7);8,985(2,6);8,983(2,6);8,981(2,6);8,529(8,6);8,498(0,6);8,480(1,6);8,474(1,6);8,457(1,7);8,452(1,7);8,169(2,7);8,1 48(2,4);7,894(2,0);7,874(2,5);7,841(0,8);7,822(1,7);7,806(2,2);7,786(3,2);7,772(2,1);7,755(1,7);7,737(0,7);5,759(16,0);3,328(8,6);2,528(0,6) 2,523(0,9);2,515(10,1);2,510(19,8);2,505(26,0);2,501(18,8);2,496(8,9);1,397(0,9);1,338(2,5);1,301(0,5);1,260(0,8);1,251 (3,1);1,123(0,5);0, 984(0,5);0,008(0,9);0,000(23,4);-0,009(0,7) |
| I-2-43: |
| HPLC-MS: logP = 3,04; Masse (m/z): 460,0 (M+H)⁺; ¹H-NMR(400,0 MHz, DMSO-D₆): |
| δ= 11,850(8,8);8,986(6,2);8,984(6,2);8,983(6,2);8,980(6,1);8,539(16,0);8,479(3,7);8,473(3,7);8,456(4,3);8,451(4,1);8,1 68(6,2);8,146(5,4);7, 972(6,8);7,953(6,9);7,564(1,4);7,558(2,6);7,545(8,7);7,540(13,0);7,523(6,2);7,521(6,2);7,504(2,0);7,502(2,0);7,288(3,8);7,282(3,6);7,271(3, 6);7,268(4,2);7,265(4,0);7,262(3,9);7,251(3,0);7,245(3,1);5,757(1,3);3,325(84,7);2,676(0,6);2,672(0,8);2,667(0,5);2,525(2,5);2,520(3,9);2,51 2(42,4);2,507(83,7);2,503(110,3);2,498(79,8);2,494(37,8);2,334(0,6);2,330(0,7);2,325(0,5);1,233(0,4);0,008(1,0);0,000(27,0);-0,009(0,8) |
| I-2-44: |
| HPLC-MS: logP = 2,18; Masse (m/z): 352,1 (M+H)⁺; ¹H-NMR(400,0 MHz, DMSO-D₆): |
| δ= 11,905(6,1);8,481(2,6);8,478(4,0);8,476(2,7);8,469(3,0);8,467(4,7);8,462(16,0);8,150(2,2);8,146(2,2);8,129(2,5);8,125(2,6);8,123(2,5);8, 119(2,2);8,102(2,4);8,098(2,3);7,883(3,1);7,864(4,0);7,829(0,9);7,811(2,7);7,794(3,7);7,776(5,3);7,761(3,8);7,742(2,7);7,725(1,1);7,711(2,6) ;7,702(2,9);7,700(2,6);7,691(4,3);7,681(2,3);7,679(2,3);7,670(2,0);5,758(1,5);4,039(0,8);4,021(0,9);3,326(24,9);2,754(0,4);2,742(0,4);2,673( 0,4);2,526(1,1);2,521(1,7);2,513(20,7);2,508(41,2);2,503(53,8);2,499(38,0);2,494(17,5);2,330(0,4);1,990(3,9);1,337(1,5);1,300(0,3);1,259(0, 5);1,250(1,9);1,234(0,6);1,193(1,1);1,175(2,2);1,158(1,1);0,008(1,2);0,000(33,9);-0,009(1,0) |
| I-2-45: |
| HPLC-MS: logP = 1,76; Masse (m/z): 352,9 (M+H)⁺; ¹H-NMR(400,0 MHz, DMSO-D₆): |
| δ= 12,069(5,8);8,893(3,2);8,881(3,2);8,482(3,9);8,474(16,0);8,302(3,1);8,283(3,3);8,154(1,9);8,151(1,9);8,133(2,1);8,130(2,4);8,128(2,3);8, 124(1,9);8,107(2,1);8,103(2,0);7,890(2,8);7,878(2,8);7,870(2,7);7,858(2,5);7,717(1,9);7,708(2,5);7,706(2,3);7,696(3,5);7,687(2,1);7,685(2,0) ;7,676(1,6);3,328(28,6);3,177(0,6);3, 164(0,6);2,526(0,9);2,513(18,0);2,508(35,9);2,504(47,0);2,499(33,7);2,495(16,1);0,008(1,2);0,000(33,5 5 );-0,009(1,1) |
| I-2-46: |
| HPLC-MS: logP = 2,04; Masse (m/z): 363,9 (M+H)⁺; ¹H-NMR(400,0 MHz, DMSO-D₆): |
| δ= 11,804(6,0);8,476(16,0);8,466(4,4);8,147(2,1);8,143(2,0);8,126(2,4);8,122(2,8);8,120(2,6);8,116(2,2);8,099(2,4);8,096(2,2);7,747(3,9);7, 745(3,8);7,728(4,7);7,725(4,3);7,710(2,2);7,701(2,8);7,698(2,5);7,689(3,9);7,680(2,4);7,677(2,2);7,668(1,9);7,624(2,9);7,620(3,2);7,606(4,1) ;7,601(4,3);7,592(0,4);7,530(2,0);7,528(2,0);7,512(4,5);7,509(4,2);7,493(2,7);7,490(2,4);7,470(2,9);7,465(3,0);7,451(3,5);7,446(3,4);7,432(1 ,6);7,427(1,4);7,373(0,6);5,757(13,5);5,571(0,3);4,057(0,9);4,039(2,9);4,021(2,9);4,004(1,0);3,336(102,3);2,527(0,7);2,513(15,4);2,509(30,7 );2,504(39,9);2,500(28,4);2,496(13,7);1,990(12,5);1,194(3,3);1,176(6,6);1,158(3,2);0,008(1,6);0,000(40,4);-0,009(1,5) |
| I-2-47: |
| HPLC-MS: logP = 1,49; Masse (m/z): 365,0 (M+H)⁺; ¹H-NMR(400,0 MHz, DMSO-D₆): |
| δ= 11,976(5,7);8,528(3,6);8,523(3,8);8,516(3,9);8,511(3,7);8,494(0,3);8,482(16,0);8,469(3,8);8,152(1,9);8,148(1,8);8,131(2,2);8,127(2,5);8, 125(2,3);8,121(2,0);8,104(2,1);8,100(2,0);8,081(3,6);8,076(3,7);8,062(4,1);8,057(3,8);7,715(1,9);7,706(2,5);7,703(2,2);7,694(3,5);7,685(2,1) ;7,683(2,0);7,674(1,6);7,611(3,9);7,599(3,8);7,592(3,7);7,580(3,6);5,756(6,7);3,324(56,1);2,676(0,4);2,672(0,5);2,668(0,3);2,542(0,3);2,525( 1,4);2,512(28,8);2,507(56,4);2,503(72,6);2,498(51,9);2,494(24,9);2,334(0,3);2,330(0,4);2,325(0,3);0,146(0,4);0,008(3,4);0,000(83,3);-0,009(3,1);-0,150(0,4) |
| I-2-48: |
| HPLC-MS: logP = 1,99; Masse (m/z): 318,0 (M+H)⁺; ¹H-NMR(400,0 MHz, DMSO-D₆): |
| δ= 11,819(5,6);8,478(16,0);8,466(4,2);8,147(2,1);8,144(2,1);8,126(2,4);8,123(2,6);8,120(2,5);8,117(2,2);8,099(2,3);8,096(2,3);7,709(2,4);7, 700(2,7);7,698(2,6);7,689(4,2);7,680(2,3);7,677(2,3);7,668(2,0);7,656(3,1);7,652(3,2);7,637(3,9);7,633(4,0);7,597(2,1);7,593(2,5);7,577(5,2) ;7,574(5,4);7,558(2,5);7,554(2,6);7,540(3,9);7,535(3,5);7,520(2,1);7,515(1,8);7,489(3,1);7,486(3,1);7,471(4,1);7,467(4,0);7,452(1,6);7,449(1 ,5);5,757(2,8);4,039(0,5);4,021(0,5);3,328(41,2);2,672(0,3);2,526(1,0);2,512(19,3);2,508(38,8);2,503(51,0);2,499(36,4);2,494(17,3);1,990(2, 1);1,193(0,7);1,175(1,3);1,158(0,6);0,008(1,9);0,000(49,7);-0,009(1,8) |
| I-2-49: |
| HPLC-MS: logP = 2,26; Masse (m/z): 370,0 (M+H)⁺; ¹H-NMR(400,0 MHz, DMSO-D₆): |
| δ= 12,170(5,7);8,483(4,0);8,474(16,0);8,461(0,7);8,155(1,9);8,152(2,1);8,135(2,2);8,131(2,5);8,129(2,4);8,125(2,1);8,108(2,2);8,104(2,1);8, 059(0,3);7,832(0,5);7,811(1,5);7,802(0,9);7,792(3,1);7,778(5,1);7,755(7,9);7,738(2,3);7,732(2,7);7,719(2,1);7,710(2,5);7,707(2,4);7,698(3,7) ;7,689(2,1);7,686(2,1);7,677(1,8);7,614(0,4);7,602(0,4);7,594(0,3);7,582(0,4);7,376(1,0);5,760(1,6);5,575(0,6);4,058(0,6);4,041(1,7);4,023(1 ,7);4,005(0,6);3,332(12,9);2,529(0,5);2,515(11,8);2,511(23,1);2,506(30,0);2,502(21,6);1,992(7,3);1,195(1,9);1,177(3,9);1,159(1,9);0,008(1,2 );0,000(29,7);-0,009(1,0) |
| I-2-50: |
| HPLC-MS: logP = 2,20; Masse (m/z): 415,9 (M+H)⁺; ¹H-NMR(400,0 MHz, DMSO-D₆): |
| δ= 11,536(8,6);8,477(16,0);8,464(5,3);8,147(2,6);8,144(2,6);8,126(3,0);8,123(3,2);8,120(3,1);8,117(2,7);8,099(2,9);8,096(2,8);7,850(9,6);7, |
| 836(10,3);7,703(2,7);7,694(3,2);7,691(3,1);7,682(4,8);7,673(2,7);7,671(2,7);7,662(2,2);7,476(9,7);7,463(9,0);5,757(0,4);3,329(61,9);2,674(0 ,3);2,513(19,2);2,509(38,1);2,505(49,9);2,500(36,1);2,496(17,6);1,990(0,5);0,008(1,8);0,000(45,1);-0,008(1,7) |
| I-2-51: |
| HPLC-MS: logP = 2,14; Masse (m/z): 410,0 (M+H)⁺; ¹H-NMR(400,0 MHz, DMSO-D₆): |
| δ= 11,745(6,8);8,474(16,0);8,469(4,1);8,466(4,9);8,449(0,3);8,147(2,3);8,143(2,2);8,126(2,6);8,122(2,9);8,120(2,8);8,117(2,4);8,099(2,5);8, 096(2,4);7,963(5,1);7,944(5,3);7,709(2,4);7,700(3,1);7,698(2,8);7,689(4,2);7,679(2,5);7,677(2,4);7,668(2,0);7,556(1,1);7,551(2,0);7,537(6,4) ;7,531(9,8);7,514(4,6);7,512(4,5);7,495(1,5);7,493(1,4);7,372(0,4);7,277(2,4);7,272(2,4);7,260(2,7);7,258(3,0);7,254(3,0);7,240(2,0);7,235(2 ,0);5,756(14,7);4,039(0,7);4,021(0,7);3,333(94,6);2,513(17,3);2,508(33,6);2,504(43,3);2,499(31,0);2,495(15,1);1,990(3,0);1,193(0,8);1,176( 1,5);1,158(0,7);0,008(1,7);0,000(40,5);-0,008(1,6) |
| I-2-52: |
| HPLC-MS: logP = 3,74; Masse (m/z): 540,1 (M+H)⁺; ¹H-NMR(400,0 MHz, DMSO-D₆): |
| δ= 8,600(3,9);8,596(4,1);8,588(4,1);8,585(4,1);8,424(16,0);8,256(4,0);8,252(4,0);8,235(4,4);8,231(4,2);8,023(4,7);8,004(5,4);7,867(5,2);7,8 47(6,4);7,792(2,6);7,790(2,7);7,774(5,8);7,771(5,5);7,755(3,9);7,736(4,6);7,724(8,0);7,716(4,5);7,704(8,9);7,686(1,9);3,326(20,7);2,672(0,4) ;2,525(1,3);2,521(1,9);2,512(23,3);2,508(47,0);2,503(62,0);2,498(44,2);2,494(21,0);2,330(0,4);1,337(1,3);1,300(0,3);1,259(0,6);1,250(1,6);1 ,235(0,5);1,091(0,4);0,000(7,9) |
| I-2-53: |
| HPLC-MS: logP = 2,33; Masse (m/z): 382,0 (M+H)⁺; ¹H-NMR(400,0 MHz, CD₃CN): |
| δ= 8,899(0,8);8,530(1,7);8,521(1,8);8,101(1,5);8,098(1,5);8,080(1,6);8,078(1,6);7,855(1,5);7,836(1,9);7,769(3,2);7,758(4,3);7,736(0,4);7,72 3(0,9);7,712(1,1);7,703(1,0);7,692(0,8);7,683(0,4);7,554(1,4);7,543(1,4);7,534(1,4);7,522(1,3);4,068(0,6);4,050(0,6);2,385(16,0);2,142(10,7) ;1,971(2,8);1,964(0,8);1,952(10,4);1,946(19,4);1,939(27,2);1,933(18,7);1,927(9,5);1,372(4,0);1,340(0,8);1,285(1,2);1,277(4,5);1,221 (0,7);1, 203(1,4);1,186(0,7);0,000(3,4) |
| I-2-54: |
| HPLC-MS: logP = 3,93; Masse (m/z): 554,1 (M+H)⁺; ¹H-NMR(400,0 MHz, CD₃CN): |
| δ= 8,491 (1,5);8,487(1,5);8,479(1,6);8,475(1,5);8,009(1,5);8,006(1,5);7,989(1,7);7,985(1,6);7,808(2,4);7,790(3,2);7,697(4,6);7,687(8,8);7,67 2(2,2);7,660(1,8);7,653(1,4);7,641(1,3);7,630(0,5);7,554(1,7);7,542(1,6);7,534(1,5);7,522(1,5);2,409(16,0);2,138(12,5);2,137(12,4);1,964(0, 9);1,952(11,7);1,946(21,9);1,939(30,7);1,933(21,1);1,927(10,8);1,372(4,0);1,341(0,8);1,285(1,0);1,276(4,3);0,000(3,7) |
| I-2-55: |
| HPLC-MS: logP = 1,95; Masse (m/z): 369,0 (M+H)⁺; ¹H-NMR(400,0 MHz, DMSO-D₆): |
| δ= 11,994(1,1);8,890(2,9);8,881(2,8);8,879(2,9);8,630(4,1);8,626(4,3);8,618(4,3);8,615(4,3);8,434(16,0);8,346(4,2);8,342(4,2);8,326(4,7);8, 322(4,4);8,317(0,4);8,307(2,6);8,305(2,8);8,288(3,0);8,285(3,0);7,887(2,7);7,875(2,7);7,867(2,5);7,855(2,5);7,735(4,8);7,724(4,5);7,715(4,4) ;7,703(4,3);3,327(33,8);3,176(0,6);3,164(0,6);2,672(0,4);2,525(1,1);2,521(1,7);2,512(21,3);2,508(43,0);2,503(56,8);2,498(40,9);2,494(19,4); 2,330(0,4) |
| I-2-56: |
| HPLC-MS: logP = 1,66; Masse (m/z): 369,1 (M+H)⁺; ¹H-NMR(400,0 MHz, DMSO-D₆): |
| δ= 11,930(5,8);8,630(4,9);8,626(5,2);8,618(5,2);8,615(5,2);8,566(4,2);8,561(4,5);8,554(4,5);8,549(4,4);8,527(0,5);8,522(0,5);8,515(0,5);8,5 10(0,5);8,448(16,0);8,444(2,8);8,345(5,0);8,341(5,0);8,325(5,5);8,321(5,2);8,158(4,3);8,153(4,4);8,139(4,8);8,134(4,5);8,086(0,5);8,081(0,5) ;8,067(0,5);8,062(0,5);7,735(5,6);7,724(5,3);7,715(5,2);7,703(5,1);7,610(0,5);7,594(4,7);7,582(4,6);7,575(4,5);7,563(4,4);5,757(0,6);3,324(2 1,5);2,676(0,4);2,671(0,5);2,667(0,4);2,525(1,6);2,512(29,7);2,507(58,9);2,502(77,0);2,498(55,1);2,493(26,1);2,334(0,4);2,329(0,5);2,325(0, 3);0,008(2,1);0,000(57,1);-0,009(1,8) |
| I-2-57: |
| HPLC-MS: logP = 2,48; Masse (m/z): 335,0 (M+H)⁺; ¹H-NMR(400,0 MHz, DMSO-D₆): |
| δ= 12,107(6,3);8,630(4,7);8,627(4,9);8,619(4,9);8,615(4,9);8,434(16,0);8,347(4,8);8,343(4,8);8,326(5,3);8,323(5,0);7,829(0,5);7,809(1,5);7, 800(0,9);7,790(3,4);7,776(5,4);7,754(9,0);7,737(7,7);7,731(3,0);7,725(5,3);7,716(5,0);7,705(4,9);4,117(0,4);4,103(1,1);4,090(1,1);4,077(0,4) ;3,329(21,5);3,178(5,2);3,165(5,0);2,526(0,8);2,521(1,2);2,513(14,6);2,509(29,3);2,504(38,6);2,499(27,7);2,495(13,2);1,338(0,8);1,250(1,0); 0,008(1,0);0,000(27,6);-0,009(0,9) |
| I-2-58: |
| HPLC-MS: logP = 2,44; Masse (m/z): 386,0 (M+H)⁺; ¹H-NMR(400,0 MHz, DMSO-D₆): |
| δ= 11,470(3,1);8,626(2,6);8,622(2,8);8,614(2,7);8,610(2,7);8,430(4,9);8,341(2,7);8,337(2,7);8,320(2,9);8,317(2,8);7,846(5,0);7,833(5,3);7,7 25(3,0);7,714(2,9);7,705(2,7);7,694(2,8);7,454(4,4);7,440(4,2);3,323(22,8);2,671(0,4);2,524(1,2);2,520(1,8);2,511(21,9);2,507(44,0);2,502(5 7,8);2,497(41,3);2,493(19,6);2,329(0,4);2,075(16,0);0,008(1,3);0,000(36,9);-0,009(1,2) |
| I-2-59: |
| HPLC-MS: logP = 2,28; Masse (m/z): 439,9 (M+H)⁺; ¹H-NMR(400,0 MHz, CD₃CN): |
| δ= 8,788(1,4);8,529(2,2);8,519(2,2);8,097(1,8);8,077(2,0);7,990(2,0);7,970(2,1);7,583(1,3);7,564(2,4);7,552(2,0);7,540(2,2);7,532(3,0);7,51 6(2,5);7,496(1,0);7,426(0,5);7,420(0,5);7,257(1,2);7,240(2,1);7,220(0,9);7,171(0,4);7,165(0,3);5,446(0,7);4,068(0,9);4,050(0,9);2,438(16,0); 2,136(22,3);1,971(3,9);1,964(0,8);1,952(10,2);1,946(19,1);1,939(26,8);1,933(18,5);1,927(9,6);1,372(8,9);1,340(1,9);1,285(2,5);1,276(9,5);1, 221(1,1);1,216(0,3);1,203(2,0);1,185(1,0);0,000(2,4) |
| I-2-60: |
| HPLC-MS: logP = 2,57; Masse (m/z): 459,9 (M+H)⁺; ¹H-NMR(400,0 MHz, CD₃CN): |
| δ= 8,818(1,7);8,562(4,4);8,560(4,4);8,551(4,5);8,135(3,4);8,115(3,6);8,040(0,5);8,019(0,7);7,998(3,0);7,978(3,0);7,612(4,6);7,600(4,9);7,59 1(5,0);7,580(5,6);7,563(2,9);7,540(2,5);7,522(3,0);7,503(1,3);7,481(0,4);7,470(0,6);7,458(0,5);7,449(0,6);7,426(0,6);7,420(0,6);7,269(1,8);7, |
| 262(1,6);7,250(2,9);7,241(1,8);7,235(1,9);7,171(0,5);7,165(0,4);6,986(0,5);5,446(0,8);4,597(0,4);3,598(0,3);2,133(31,8);2,119(0,5);2,113(0, 5);2,107(0,7);2,101(0,5);1,971(1,3);1,964(4,1);1,952(40,8);1,946(73,8);1,940(99,7);1,933(67,6);1,927(34,1);1,914(0,7);1,774(0,5);1,768(0,6) ;1,762(0,4);1,386(1,1);1,372(14,7);1,340(5,7);1,310(1,1);1,294(1,9);1,285(8,3);1,276(16,0);1,271(7,7);1,221(0,6);1,216(1,4);1,204(0,6);1,18 6(0,4);0,898(0,7);0,881(1,7);0,874(1,1);0,864(1,1);0,857(1,3);0,838(0,8);0,000(6,2) |
| I-2-61: |
| HPLC-MS: logP = 4,03; Masse (m/z): 669,9 (M+H)⁺; ¹H-NMR(400,0 MHz, CD₃CN): |
| δ= 8,511(1,4);8,507(1,4);8,499(1,5);8,495(1,4);8,043(1,5);8,039(1,4);8,022(1,6);8,019(1,5);7,910(3,2);7,890(3,4);7,614(2,4);7,610(2,4);7,59 5(3,1);7,591(3,0);7,565(1,5);7,553(1,4);7,545(1,4);7,533(1,3);7,462(1,9);7,460(1,8);7,443(3,5);7,424(1,8);7,422(1,7);7,185(1,7);7,181(1,6);7, 165(2,9);7,162(2,7);7,146(1,5);7,142(1,3);5,446(1,5);2,467(16,0);2,133(18,1);1,972(0,5);1,964(0,7);1,952(9,5);1,946(17,8);1,939(24,7);1,93 3(17,0);1,927(8,7);1,372(2,9);1,341(0,6);1,285(0,8);1,276(3,1);0,000(2,4) |
| I-2-62: |
| HPLC-MS: logP = 2,77; Masse (m/z): 370,0 (M+H)⁺;¹H-NMR(400,0 MHz, DMSO-D₆): |
| δ= 11,988(6,4);8,511(16,0);8,459(1,5);8,452(1,9);8,440(2,3);8,433(2,9);8,422(1,3);8,415(2,1);8,401(4,6);8,396(4,8);8,389(3,2);7,879(3,5);7, 860(4,2);7,826(1,0);7,805(3,3);7,790(3,6);7,763(8,3);7,743(4,6);7,724(1,1);5,756(0,9);3,323(34,1);2,676(0,5);2,672(0,6);2,667(0,5);2,525(2, 0);2,512(36,4);2,507(72,3);2,503(94,3);2,498(67,4);2,493(32,1);2,334(0,4);2,329(0,6);2,325(0,4);1,352(0,6);1,336(2,4);1,299(0,8);1,259(1,3) ;1,250(2,6);1,234(0,8);1,188(0,3);0,008(2,3);0,000(62,3);-0,009(2,0) |
| I-2-63: siehe Synthesebeispiel 45 |
| I-2-64: |
| HPLC-MS: logP = 2,54; Masse (m/z): 402,9 (M+H)⁺; ¹H-NMR(400,0 MHz, DMSO-D₆): |
| δ= 12,039(7,3);8,888(3,7);8,878(3,6);8,720(7,2);8,715(8,3);8,655(8,1);8,649(6,8);8,459(16,0);8,316(0,9);8,299(3,5);8,281(3,8);7,884(3,1);7, 872(3,1);7,865(2,9);7,853(2,8);3,320(137,4);2,675(2,0);2,671(2,7);2,666(2,0);2,524(7,9);2,510(156,8);2,506(305,6);2,501(398,3);2,497(293, 2);2,493(143,3);2,333(2,0);2,328(2,7);2,324(2,0);2,102(0,3);1,336(1,9);1,298(0,3);1,259(0,5);1,250(2,3);1,135(0,4);0,989(0,4);0,000(2,0) |
| I-2-65: |
| HPLC-MS: logP = 3,04; Masse (m/z): 459,9 (M+H)⁺; ¹H-NMR(400,0 MHz, DMSO-D₆): |
| δ= 11,722(8,3);8,716(8,8);8,711(10,3);8,649(8,8);8,643(7,5);8,470(0,5);8,459(16,0);8,444(0,7);7,961(5,5);7,942(5,7);7,880(0,3);7,553(1,3);7 ,547(2,2);7,534(7,3);7,529(11,1);7,511(5,0);7,509(5,1);7,493(1,7);7,490(1,7);7,451(0,3);7,444(0,3);7,337(0,4);7,332(0,4);7,276(3,0);7,270(3, 0);7,258(3,2);7,256(3,5);7,253(3,3);7,250(3,3);7,239(2,6);7,233(2,5);7,187(0,7);7,182(0,8);7,176(0,4);5,756(7,2);3,322(49,2);2,676(0,5);2,67 1(0,8);2,666(0,5);2,524(2,3);2,520(3,6);2,511(41,0);2,506(82,0);2,502(108,7);2,497(78,3);2,493(36,7);2,333(0,5);2,329(0,7);2,324(0,5);1,33 6(7,0);1,299(1,5);1,259(2,4);1,249(9,4);1,234(0,8);1,195(0,6);1,187(0,3);1,177(1,3);1,159(0,6);1,013(0,5);0,995(1,2);0,977(0,5);0,146(0,3);0, 008(3,2);0,000(90,9);-0,009(2,7);-0,150(0,4) |
| I-2-66: |
| HPLC-MS: logP = 2,43; Masse (m/z): 370,0 (M+H)⁺; ¹H-NMR(400,0 MHz, DMSO-D₆): |
| δ= 11,900(6,8);11,873(0,6);8,770(0,9);8,638(0,6);8,632(0,7);8,582(6,3);8,576(6,6);8,511(0,5);8,462(16,0);8,436(0,3);8,404(2,2);8,397(2,1);8 ,389(0,4);8,383(2,3);8,377(3,5);8,372(2,1);8,357(2,2);8,351(2,0);7,882(3,5);7,862(4,4);7,827(1,1);7,808(3,1);7,792(3,9);7,772(5,7);7,761(3,6 );7,757(4,0);7,742(3,3);7,724(1,2);5,756(7,4);3,324(31,3);2,676(0,5);2,672(0,6);2,667(0,4);2,525(2,1);2,520(3,2);2,512(34,8);2,507(69,2);2,5 03(90,5);2,498(64,6);2,494(30,5);2,334(0,4);2,330(0,6);2,325(0,4);1,989(1,2);1,352(0,7);1,337(3,0);1,299(1,1);1,259(1,6);1,250(3,5);1,234(1 ,0);1,193(0,4);1,175(0,7);1,158(0,4);0,008(2,2);0,000(63,5);-0,009(2,1) |
| I-2-67: |
| HPLC-MS: logP = 2,00; Masse (m/z): 371,0 (M+H)⁺; ¹H-NMR(400,0 MHz, DMSO-D₆): |
| δ= 12,060(7,9);8,890(4,1);8,879(4,1);8,586(6,9);8,580(7,3);8,472(16,0);8,409(2,2);8,403(2,1);8,388(2,4);8,382(3,8);8,377(2,2);8,362(2,2);8, 356(2,0);8,316(0,6);8,296(3,9);8,278(4,3);7,887(3,5);7,875(3,5);7,867(3,3);7,855(3,2);5,756(3,5);3,321(104,9);2,675(1,5);2,671(2,0);2,666(1 ,5);2,541(1,0);2,524(5,1);2,510(117,0);2,506(231,7);2,502(303,0);2,497(221,1);2,493(108,8);2,333(1,5);2,329(2,0);2,324(1,5);0,146(1,1);0,0 08(9,9);0,000(246,6);-0,008(9,3);-0,150(1,2) |
| I-2-68: |
| HPLC-MS: logP = 2,27; Masse (m/z): 382,0 (M+H)⁺; ¹H-NMR(400,0 MHz, DMSO-D₆): |
| δ= 11,802(7,3);8,582(6,5);8,576(6,7);8,477(13,5);8,401(2,2);8,395(2,0);8,380(2,4);8,375(3,6);8,369(2,1);8,354(2,1);8,348(1,9);7,747(4,4);7, 727(5,1);7,725(4,6);7,622(3,2);7,618(3,4);7,603(4,6);7,599(4,5);7,530(2,3);7,527(2,2);7,511(5,1);7,509(4,5);7,493(3,0);7,490(2,6);7,470(3,2) ;7,465(3,2);7,450(4,0);7,446(3,7);7,431(1,8);7,427(1,5);5,757(16,0);3,328(19,5);2,509(22,6);2,504(28,4);2,500(20,2);1,990(0,7);1,396(0,4);1 ,178(0,4);1,176(0,5);1,161(0,7);0,993(0,6);0,008(0,5);0,000(10,7);-0,009(0,4) |
| I-2-69: |
| HPLC-MS: logP = 1,71; Masse (m/z): 382,9 (M+H)⁺; ¹H-NMR(400,0 MHz, DMSO-D₆): |
| δ= 11,973(7,3);8,586(6,8);8,580(7,4);8,529(4,4);8,524(5,0);8,517(4,8);8,512(4,8);8,484(16,0);8,406(2,2);8,400(2,1);8,385(2,5);8,380(3,8);8, 374(2,4);8,359(2,2);8,353(2,0);8,079(4,4);8,074(4,8);8,060(5,0);8,056(4,9);7,611(4,8);7,599(4,7);7,592(4,6);7,580(4,3);4,114(0,3);4,101(1,0) ;4,088(1,0);4,074(0,4);3,326(70,4);3,177(3,9);3,163(3,8);2,673(0,4);2,508(46,7);2,503(61,5);2,499(46,2);2,330(0,4);0,008(1,7);0,000(37,9) |
| I-2-70: |
| HPLC-MS: logP = 2,22; Masse (m/z): 336,0 (M+H)⁺; ¹H-NMR(400,0 MHz, DMSO-D₆): |
| δ= 11,818(7,9);8,582(7,7);8,576(8,0);8,481(16,0);8,402(2,6);8,395(2,4);8,381(2,8);8,375(4,2);8,370(2,5);8,355(2,6);8,349(2,3);7,655(3,9);7, 651(4,1);7,636(5,1);7,632(5,0);7,597(2,7);7,594(3,1);7,577(6,8);7,574(6,8);7,558(3,3);7,554(3,3);7,540(5,1);7,536(4,4);7,521(2,8);7,516(2,3) |
| ;7,490(4,1);7,486(3,8);7,471(5,3);7,468(5,0);7,453(2,2);7,449(2,0);5,757(8,1);3,328(19,6);2,948(0,6);2,527(0,6);2,513(13,9);2,509(27,4);2,5 04(35,2);2,500(24,7);2,495(11,5);1,991 (1,0);1,397(0,7);1,176(0,6);1,173(0,4);1,155(0,8);1,138(0,4);1,006(0,3);0,988(0,7);0,970(0,3);0,008(0 ,6);0,000(14,6);-0,009(0,5) |
| I-2-71: |
| HPLC-MS: logP = 2,51; Masse (m/z): 388,0 (M+H)⁺; ¹H-NMR(400,0 MHz, DMSO-D₆): |
| δ= 12,166(6,6);8,587(6,2);8,580(6,6);8,475(16,0);8,408(2,2);8,402(2,0);8,387(2,3);8,382(3,5);8,376(2,1);8,362(2,2);8,355(2,0);7,832(0,5);7, 811(1,5);7,802(0,9);7,792(3,1);7,777(5,0);7,754(8,2);7,737(2,3);7,731(2,7);5,758(4,8);3,330(26,6);2,528(0,5);2,515(11,2);2,510(22,7);2,506( 29,8);2,501(21,1);2,497(9,8);1,991(0,7);1,397(0,8);1,177(0,4);0,000(1,8) |
| I-2-72: |
| HPLC-MS: logP = 2,44; Masse (m/z): 433,9 (M+H)⁺; ¹H-NMR(400,0 MHz, DMSO-D₆): |
| δ= 11,525(10,8);8,580(10,3);8,574(11,0);8,476(16,0);8,402(3,5);8,396(3,2);8,381(3,6);8,377(4,6);8,371(3,6);8,356(3,4);8,349(3,0);7,848(13, 6);7,834(14,4);7,769(0,4);7,756(0,5);7,735(0,7);7,721(0,8);7,468(13,5);7,454(12,7);7,127(0,7);7,113(0,8);6,941(0,5);6,928(0,5);5,757(5,9);4, 039(0,7);4,021(0,7);3,325(73,9);2,677(0,4);2,673(0,6);2,668(0,4);2,543(0,5);2,526(1,7);2,513(34,4);2,508(68,7);2,504(89,4);2,499(63,3);2,4 95(29,6);2,335(0,4);2,330(0,5);2,326(0,4);1,990(2,9);1,397(1,2);1,193(0,8);1,176(1,9);1,158(1,4);1,006(0,6);0,000(4,6) |
| I-2-73: |
| HPLC-MS: logP = 2,39; Masse (m/z): 427,9 (M+H)⁺; ¹H-NMR(400,0 MHz, DMSO-D₆): |
| δ= 11,738(5,5);8,581(5,5);8,575(5,9);8,471(11,4);8,402(1,7);8,396(1,6);8,381(1,9);8,376(3,0);8,370(1,8);8,355(1,8);8,349(1,6);8,315(0,6);7, 960(4,4);7,941(4,4);7,551(0,8);7,545(1,5);7,532(5,8);7,526(8,6);7,511(3,9);7,510(3,9);7,492(1,2);7,276(2,1);7,270(2,0);7,259(2,2);7,256(2,5) ;7,253(2,5);7,250(2,3);7,239(1,8);7,233(1,7);3,321(141,3);2,942(0,7);2,675(1,1);2,671(1,5);2,666(1,1);2,541(0,9);2,524(3,8);2,510(86,2);2,5 06(171,5);2,502(223,6);2,497(160,3);2,493(77,4);2,333(1,1);2,328(1,5);2,324(1,1);1,989(1,4);1,398(16,0);1,193(0,4);1,175(0,8);1,157(0,4);0 ,146(0,4);0,008(2,9);0,000(80,1);-0,008(3,0);-0,150(0,4) |
| I-2-74: |
| HPLC-MS: logP = 3,28; Masse (m/z): 436,0 (M+H)⁺; ¹H-NMR(400,0 MHz, DMSO-D₆): |
| δ= 11,968(6,7);9,050(4,1);9,048(4,7);9,045(4,7);9,043(4,1);9,034(0,4);8,881(4,6);8,880(4,8);8,876(4,5);8,526(16,0);7,889(3,0);7,869(3,9);7, 834(0,7);7,816(2,5);7,800(3,9);7,797(3,8);7,787(5,2);7,771(3,2);7,769(3,0);7,751(2,3);7,749(2,4);7,747(2,2);7,734(1,0);7,732(1,0);7,729(0,9) ;7,716(0,3);5,758(8,3);3,329(22,1);2,528(0,6);2,523(1,0);2,514(11,4);2,510(22,8);2,505(30,4);2,500(21,9);2,496(10,2);1,339(1,3);1,301(0,7); 1,260(1,0);1,250(1,4);1,233(0,9);1,123(0,4);0,984(0,5);0,008(0,8);0,000(24,4);-0,009(0,7) |
| I-2-75: |
| HPLC-MS: logP = 3,28; Masse (m/z): 493,9 (M+H)⁺; ¹H-NMR(400,0 MHz, DMSO-D₆): |
| δ= 11,805(8,4);9,047(6,3);9,044(6,3);8,879(6,5);8,875(6,1);8,534(16,0);7,967(5,6);7,948(5,8);7,563(1,6);7,558(2,4);7,544(7,0);7,538(7,2);7, 536(6,2);7,533(5,2);7,51 8(5,1);7,516(5,2);7,499(1,8);7,497(1,8);7,282(2,9);7,277(3,0);7,264(3,3);7,262(3,6);7,259(3,5);7,257(3,5);7,245(2,5) ;7,239(2,4);5,757(6,5);3,328(105,2);3,310(0,6);2,677(0,3);2,672(0,4);2,525(1,6);2,512(25,0);2,508(48,3);2,503(62,9);2,498(45,7);2,494(21,9 );2,330(0,4);1,235(0,6);0,008(0,5);0,000(11,6);-0,009(0,4) |
| I-2-76: |
| HPLC-MS: logP = 2,40; Masse (m/z): 370,0 (M+H)⁺; ¹H-NMR(400,0 MHz, DMSO-D₆): |
| δ= 11,998(7,3);8,892(4,0);8,880(3,9);8,470(16,0);8,31 6(3,7);8,299(4,0);8,297(4,0);7,887(3,3);7,875(3,4);7,868(3,2);7,856(3,0);7,778(0,8);7, 762(1,8);7,756(1,6);7,746(1,2);7,741(3,4);7,735(1,3);7,725(1,7);7,719(2,2);7,703(1,0);7,604(0,4);7,492(5,3);7,471(9,2);7,450(4,3);7,377(0,5) ;7,356(0,9);7,335(1,7);5,759(0,3);5,465(0,8);3,334(18,9);3,179(1,1);3,166(1,1);2,528(0,5);2,514(12,6);2,510(25,4);2,505(33,5);2,501(24,3);2 ,497(11,9);0,008(0,7);0,000(17,6);-0,008(0,6) |
| I-2-77: |
| HPLC-MS: logP = 2,59; Masse (m/z): 337,0 (M+H)⁺; ¹H-NMR(400,0 MHz, DMSO-D₆): |
| δ= 12,063(11,5);8,481(14,2);8,479(15,2);8,476(16,0);7,781(0,9);7,778(1,0);7,762(3,3);7,745(4,6);7,741(4,9);7,724(3,8);7,719(2,6);7,708(1,3 );7,704(1,2);7,668(1,0);7,665(1,1);7,649(3,7);7,631(5,0);7,628(5,1);7,611(4,0);7,593(1,2);7,590(1,1);7,495(7,9);7,473(1 3,4);7,471(13,1);7,44 9(6,8);7,296(7,3);7,293(7,4);7,276(13,1);7,273(13,1);7,255(6,3);7,252(6,6);5,764(1,8);5,762(2,0);5,759(2,1);3,336(28,5);3,334(30,6);2,674(0 ,4);2,510(66,0);2,505(64,1);2,336(0,4);2,332(0,4);0,005(49,3);0,003(48,7);0,000(50,2) |
| I-2-78: |
| HPLC-MS: logP = 2,22; Masse (m/z): 355,1 (M+H)⁺; ¹H-NMR(400,0 MHz, DMSO-D₆): |
| δ= 11,486(3,8);8,592(3,9);8,419(8,0);7,765(0,4);7,749(0,9);7,743(0,8);7,733(0,6);7,728(1,7);7,722(0,6);7,712(0,8);7,706(1,0);7,690(0,5);7,4 95(1,9);7,487(2,7);7,465(4,6);7,444(2,1);7,360(3,5);7,225(1,6);5,758(0,5);3,968(16,0);3,332(44,8);3,006(0,8);2,513(12,8);2,508(24,4);2,504( 30,9);2,500(21,7);2,495(10,1);0,007(0,7);0,000(12,2);-0,009(0,4) |
| I-2-79: |
| HPLC-MS: logP = 2,60; Masse (m/z): 370,9 (M+H)⁺; ¹H-NMR(400,0 MHz, DMSO-D₆): |
| δ= 12,149(4,7);9,134(5,2);9,128(6,1);9,079(6,3);9,073(5,1);8,511(0,3);8,497(16,0);7,785(0,8);7,769(1,6);7,763(1,4);7,753(1,1);7,748(3,2);7, 742(1,2);7,731(1,5);7,726(2,0);7,710(0,9);7,499(4,8);7,478(8,3);7,457(3,8);7,454(2,6);5,760(1,4);3,339(13,6);2,975(0,5);2,531(0,4);2,517(8, 1);2,513(16,4);2,508(21,6);2,504(15,5);2,499(7,3);0,008(0,4);0,000(12,4);-0,009(0,4) |
| I-2-80: |
| HPLC-MS: logP = 2,78; Masse (m/z): 370,0 (M+H)⁺; ¹H-NMR(400,0 MHz, DMSO-D₆): |
| δ= 11,930(7,0);8,958(4,3);8,947(4,3);8,469(16,0);8,421(4,1);8,402(4,4);7,856(2,9);7,843(2,9);7,836(2,8);7,823(2,6);7,779(0,9);7,763(1,8);7, 757(1,7);7,747(1,3);7,741(3,6);7,736(1,4);7,726(1,8);7,720(2,2);7,704(1,0);7,493(5,6);7,472(9,8);7,451(4,5);7,332(0,3);5,758(7,4);3,330(45, |
| 4);2,673(0,4);2,513(23,6);2,508(46,7);2,504(60,9);2,500(43,9);2,331(0,4);1,233(0,3);0,008(2,3);0,000(57,5);-0,009(2,1) |
| I-2-81: |
| HPLC-MS: logP = 2,36; Masse (m/z): 370,0 (M+H)⁺; ¹H-NMR(400,0 MHz, DMSO-D₆): |
| δ= 12,093(0,6);9,120(3,0);9,046(1,8);9,033(1,9);8,464(5,2);8,104(1,3);8,100(1,0);8,091(1,0);8,087(1,3);7,883(1,7);7,870(1,7);7,763(0,6);7,7 57(0,5);7,747(0,4);7,741(1,1);7,736(0,4);7,725(0,5);7,720(0,7);7,492(1,7);7,471(2,9);7,450(1,4);7,331(0,3);6,592(1,4);6,588(1,0);6,579(1,0); 6,575(1,4);3,329(11,6);2,946(16,0);2,525(0,7);2,508(22,8);2,503(29,5);2,499(21,4);1,990(1,0);1,235(0,4);1,175(0,5);0,008(1,0);0,000(25,6);-0,008(1,1) |
| I-2-82: |
| HPLC-MS: logP = 2,95; Masse (m/z): 375,0 (M+H)⁺; ¹H-NMR(400,0 MHz, DMSO-D₆): |
| δ= 11,997(6,7);8,436(16,0);7,993(5,7);7,980(6,1);7,779(1,0);7,763(2,1);7,757(1,8);7,747(1,4);7,741(4,1);7,735(1,5);7,725(1,9);7,720(2,5);7, 704(1,2);7,500(12,0);7,496(4,4);7,493(6,9);7,487(10,9);7,472(10,8);7,450(5,0);7,447(3,2);5,758(6,3);3,329(36,3);2,673(0,4);2,527(1,1);2,51 3(22,2);2,509(44,8);2,504(58,7);2,500(42,0);2,495(20,1);2,331(0,4);0,008(2,5);0,000(66,7);-0,009(2,5) |
| I-2-83: |
| HPLC-MS: logP = 2,36; Masse (m/z): 370,0 (M+H)⁺; ¹H-NMR(400,0 MHz, DMSO-D₆): |
| δ= 12,077(3,7);9,083(8,7);9,012(4,2);9,000(4,3);8,477(14,7);8,103(1,2);8,099(0,9);8,091(0,9);8,087(1,2);7,933(4,9);7,920(4,7);7,779(0,7);7, 763(1,5);7,757(1,3);7,747(1,1);7,742(2,9);7,736(1,1);7,726(1,4);7,720(1,8);7,704(0,8);7,494(4,4);7,473(7,7);7,452(3,6);6,590(1,3);6,586(0,9) ;6,578(0,9);6,574(1,3);5,758(0,5);3,329(32,2);2,946(16,0);2,673(0,3);2,513(20,1);2,508(40,0);2,504(52,1);2,499(37,0);2,495(17,4);2,330(0,3 );1,990(0,6);0,008(2,1);0,000(53,2);-0,009(1,9) |
| I-2-84: |
| HPLC-MS: logP = 2,28; Masse (m/z): 371,0 (M+H)⁺; ¹H-NMR(400,0 MHz, DMSO-D₆): |
| δ= 11,997(6,7);8,964(4,3);8,952(4,4);8,591(7,1);8,585(7,4);8,495(0,3);8,483(16,0);8,471(0,4);8,423(4,2);8,411(2,6);8,404(6,0);8,390(2,5);8, 385(3,8);8,379(2,4);8,364(2,2);8,358(2,1);7,859(2,9);7,847(2,9);7,839(2,8);7,827(2,6);5,760(3,2);3,338(40,7);2,954(1,4);2,511(22,6);2,507(2 9,2);2,503(21,5);1,992(0,7);1,177(0,4);0,007(1,1);0,000(27,4);-0,008(1,4) |
| I-2-85: |
| HPLC-MS: logP = 2,50; Masse (m/z): 376,0 (M+H)⁺; |
| ¹H-NMR(400,0 MHz, DMSO-D₆): |
| δ= 12,078(7,2);8,587(7,8);8,581(8,2);8,459(0,4);8,448(16,0);8,411(2,5);8,405(2,3);8,390(2,7);8,385(4,2);8,379(2,4);8,364(2,4);8,358(2,2);7, 998(5,8);7,985(6,1);7,503(9,7);7,490(9,2);5,758(1,4);3,332(55,4);2,510(35,3);2,505(45,2);2,501(32,4);0,008(1,9);0,000(44,2);-0,008(1,8) |
| I-5-1 |
| I-5-2 |
| siehe Synthesebeispiel 22 |
| I-5-3: |
| HPLC-MS: logP = 3,09; Masse (m/z): 429,0 (M+H)⁺; ¹H-NMR(400,0 MHz, DMSO-D₆): |
| δ= 11,274(11,8);8,586(12,2);8,580(12,5);8,356(3,1);8,352(3,1);8,331(5,7);8,316(2,4);8,310(3,2);8,305(2,9);8,032(11,5);8,011(12,2);7,929(14 ,5);7,908(16,0);7,770(6,9);7,751(7,9);7,688(5,8);7,670(7,1);7,577(7,1);7,558(12,6);7,538(12,2);7,516(4,6);7,476(5,0);7,457(6,6);7,439(2,9);7 ,358(9,7);7,339(13,8);7,321(7,6);7,320(7,8);5,757(14,0);3,324(114,0);2,687(2,8);2,680(0,9);2,674(3,7);2,666(1,4);2,662(0,7);2,541(1,3);2,52 4(6,1);2,511(106,3);2,506(212,0);2,502(277,8);2,497(198,5);2,493(94,1);2,338(0,6);2,333(1,3);2,328(1,7);2,324(1,3);2,086(5,7);1,755(0,4);1 ,233(1,1);0,146(1,0);0,008(9,1);0,000(232,6);-0,009(8,0);-0,150(1,0) |
| I-5-4: |
| HPLC-MS: logP = 2,67; Masse (m/z): 427,0 (M+H)⁺; ¹H-NMR(400,0 MHz, DMSO-D₆): |
| δ= 11,256(3,7);8,643(2,5);8,634(2,5);8,205(2,0);8,188(2,1);8,055(2,6);8,035(2,8);7,953(0,9);7,768(2,3);7,749(2,6);7,730(2,0);7,718(2,1);7,7 10(2,1);7,698(1,8);7,673(2,0);7,655(2,4);7,549(1,3);7,531(2,6);7,522(2,2);7,502(3,1);7,483(2,5);7,474(1,8);7,454(2,1);7,436(1,0);7,414(0,5); 7,392(0,5);7,365(0,5);7,337(0,8);7,332(0,9);7,318(3,5);7,310(3,6);7,293(4,2);7,290(4,3);7,272(2,6);7,182(1,0);7,176(0,6);5,757(16,0);3,325( 98,5);2,890(6,8);2,731(5,5);2,675(0,6);2,671(0,8);2,666(0,6);2,524(2,6);2,510(42,6);2,506(82,0);2,502(106,3);2,497(77,2);2,493(37,2);2,333 (0,5);2,328(0,7);2,324(0,5);1,397(0,3);1,352(1,1);1,336(11,6);1,317(0,4);1,298(6,1);1,258(8,6);1,249(13,2);1,234(1,7);1,225(1,0);1,187(0,5); 0,008(0,6);0,000(14,6);-0,009(0,5) |
| I-5-5: |
| HPLC-MS: logP = 3,98; Masse (m/z): 497,0 (M+H)⁺; ¹H-NMR(400,0 MHz, DMSO-D₆): |
| δ= 11,349(7,9);9,015(8,3);8,792(6,5);8,315(1,6);8,055(11,9);8,035(12,8);7,905(14,0);7,883(16,0);7,771(4,5);7,752(5,1);7,689(4,0);7,672(4,8 );7,594(6,8);7,591(7,0);7,576(9,1);7,573(11,4);7,555(9,5);7,552(9,3);7,535(5,0);7,517(3,3);7,479(3,8);7,461(4,8);7,442(2,5);7,384(9,2);7,383 (9,5);7,365(13,7);7,347(7,7);7,345(7,6);3,379(0,6);3,327(402,0);3,325(467,8);2,680(1,7);2,675(3,6);2,671(4,9);2,666(3,6);2,662(1,7);2,593(0 ,4);2,568(0,6);2,524(13,0);2,519(20,4);2,511(266,0);2,506(535,6);2,502(713,4);2,497(518,8);2,493(246,5);2,435(0,3);2,338(1,6);2,333(3,5);2 ,328(4,8);2,324(3,4);2,319(1,6);2,074(4,3);1,187(0,6);1,148(0,5);0,008(2,3);0,000(75,6);-0,009(2,3) |
| I-5-6: |
| HPLC-MS: logP = 3,05; Masse (m/z): 428,9 (M+H)⁺; ¹H-NMR(400,0 MHz, DMSO-D₆): |
| δ= 11,307(10,7);8,437(8,1);8,402(3,7);8,053(7,8);8,033(8,3);7,952(1,8);7,933(0,4);7,772(5,1);7,753(5,8);7,672(4,3);7,654(5,4);7,576(16,0);7 ,561(10,0);7,538(7,1);7,518(3,6);7,479(3,9);7,460(5,2);7,441(2,4);7,352(4,3);7,349(3,9);7,333(6,8);7,317(3,4);7,314(3,5);5,757(1,2);3,862(1, 1);3,325(54,6);3,023(0,3);3,017(0,5);3,007(0,5);2,890(9,9);2,730(9,3);2,701(0,6);2,689(0,6);2,670(0,9);2,501(138,1);2,328(0,9);2,086(0,9);1, |
| 954(0,3);1,939(0,8);1,754(1,2);1,745(0,3);1,737(0,3);1,729(0,7);1,722(0,3);1,233(0,6);0,146(0,4);0,000(66,9);-0,150(0,4) |
| I-5-7: |
| HPLC-MS: logP = 3,39; Masse (m/z): 437,0 (M+H)⁺; ¹H-NMR(400,0 MHz, DMSO-D₆): |
| δ= 11,491(11,8);8,584(0,5);8,579(0,5);8,453(10,2);8,424(5,6);8,406(3,1);8,400(2,4);8,317(0,5);8,014(0,3);8,003(0,3);7,991(0,3);7,980(0,3);7 ,908(7,3);7,888(9,5);7,848(16,0);7,839(13,1);7,815(2,0);7,778(3,9);7,765(4,9);7,757(4,9);7,745(3,4);7,738(2,1);7,690(9,3);7,684(9,9);7,668( 10,6);7,661(11,0);7,511(4,6);7,505(4,1);7,489(8,0);7,482(6,7);7,466(3,7);7,460(3,3);5,758(5,9);3,324(134,6);2,675(1,8);2,671(2,4);2,666(1,7 );2,541(1,2);2,524(8,2);2,511(141,8);2,506(275,5);2,502(353,2);2,497(253,0);2,493(121,7);2,333(1,7);2,329(2,3);2,324(1,6);1,234(0,8);0,000 (0,8) |
| I-5-8: siehe Synthesebeispiel 47 |
| I-5-9: |
| HPLC-MS: logP = 3,11; Masse (m/z): 417,0 (M+H)⁺; ¹H-NMR(400,0 MHz, DMSO-D₆): |
| δ= 11,497(9,5);8,897(5,0);8,885(4,9);8,345(4,7);8,327(5,0);7,993(6,0);7,973(6,5);7,902(4,2);7,890(4,2);7,882(4,0);7,870(3,8);7,800(4,4);7,7 94(4,5);7,783(3,4);7,780(4,1);7,776(5,6);7,763(0,4);7,678(3,4);7,673(2,9);7,670(2,9);7,661(5,1);7,654(6,9);7,644(1,6);7,639(2,4);7,626(6,0); 7,620(5,1);7,614(6,5);7,608(9,7);7,601(4,3);7,595(4,6);7,591(3,8);7,577(1,3);7,572(0,9);7,502(3,0);7,483(5,2);7,465(4,0);7,463(3,9);7,297(5, 0);7,279(6,5);7,260(3,9);7,259(3,9);7,222(7,5);7,200(6,4);5,757(16,0);3,327(152,8);2,676(0,6);2,671(0,9);2,667(0,6);2,541(0,5);2,524(2,4);2, 511(48,1);2,507(96,2);2,502(126,0);2,497(89,9);2,493(42,5);2,333(0,6);2,329(0,8);2,324(0,6);1,397(0,4);0,008(1,6);0,000(41,2);-0,009(1,3) |
| I-5-10: |
| HPLC-MS: logP = 3,26; Masse (m/z): 461,0 (M+H)⁺; ¹H-NMR(400,0 MHz, DMSO-D₆): |
| δ= 11,200(16,0);8,433(5,3);8,419(5,8);8,408(5,8);8,394(5,3);8,316(1,7);8,070(4,1);8,054(5,2);8,033(4,0);7,906(7,6);7,886(11,6);7,859(7,4);7 ,841(6,2);7,781(13,6);7,764(13,0);7,615(4,0);7,602(4,7);7,594(8,2);7,582(8,3);7,574(6,0);7,562(5,5);7,454(6,9);7,446(7,2);7,433(5,9);7,425( 5,4);7,164(11,1);7,144(11,4);7,138(12,0);7,119(10,2);5,756(5,7);3,322(510,5);2,675(3,8);2,671(5,3);2,666(3,8);2,541(3,0);2,524(14,5);2,510 (303,2);2,506(597,5);2,501(773,7);2,497(549,5);2,492(257,3);2,333(3,7);2,328(5,1);2,324(3,6);2,074(1,8);1,236(0,3);0,146(1,0);0,008(8,2);0 ,000(229,1);-0,009(7,4);-0,150(1,0) |
| I-5-11: |
| HPLC-MS: logP = 2,99; Masse (m/z): 429,1 (M+H)⁺; ¹H-NMR(400,0 MHz, DMSO-D₆): |
| δ= 11,378(16,0);8,435(4,8);8,421(5,1);8,410(5,1);8,396(4,8);8,315(2,1);8,102(4,8);8,087(5,3);8,079(5,3);8,063(4,7);7,669(1,4);7,653(3,3);7, 649(3,4);7,632(5,9);7,617(5,5);7,611(4,8);7,606(4,2);7,597(6,8);7,585(5,9);7,577(4,3);7,565(3,7);7,454(6,3);7,445(6,7);7,433(5,2);7,424(5,0) ;7,301(9,7);7,281(15,9);7,261(8,3);7,204(0,4);7,164(8,6);7,144(8,7);7,138(9,3);7,119(8,0);5,756(1,3);4,038(0,3);4,020(0,3);3,550(0,8);3,536( 0,7);3,509(4,2);3,495(0,7);3,482(0,7);3,408(0,5);3,392(0,5);3,382(0,6);3,369(0,8);3,322(542,0);2,675(4,0);2,671(5,5);2,666(4,0);2,662(2,0);2 ,631(0,4);2,541(3,7);2,524(14,2);2,519(21,5);2,511(292,8);2,506(593,0);2,502(781,0);2,497(556,3);2,493(260,6);2,337(1,6);2,333(3,6);2,328 (5,0);2,324(3,5);1,989(1,4);1,298(0,6);1,259(1,1);1,234(3,6);1,193(0,5);1,175(0,9);1,157(0,5);1,050(2,0);1,034(2,0);1,020(0,3);0,854(0,6);0,8 36(0,4);0,807(0,4);0,146(1,8);0,008(13,3);0,000(407,8);-0,008(12,8);-0,150(1,7) |
| I-3-1: siehe Synthesebeispiel 46 |
| I-3-2: |
| HPLC-MS: logP = 2,68; Masse (m/z): 335,1 (M+H)⁺; ¹H-NMR(400,0 MHz, DMSO-D₆): |
| δ= 10,802(10,4);7,610(1,4);7,593(3,2);7,589(3,0);7,572(5,8);7,555(3,3);7,551(3,5);7,534(1,8);7,531(1,6);7,515(2,7);7,509(2,3);7,499(2,1);7, 494(5,5);7,488(2,5);7,476(2,6);7,472(3,9);7,457(1,8);7,402(9,0);7,364(10,0);7,342(16,0);7,321(6,8);7,260(2,3);7,254(10,0);7,234(14,6);7,21 4(8,3);7,206(1,8);6,955(7,7);6,313(8,7);6,309(9,2);6,305(9,0);6,301(7,9);4,121(0,5);4,108(1,3);4,095(1,4);4,082(0,5);3,335(183,4);3,176(6,2) ;3,163(5,9);2,677(0,5);2,672(0,7);2,668(0,5);2,511(42,8);2,507(77,4);2,503(97,5);2,499(71,9);2,334(0,5);2,330(0,6);2,325(0,5);1,337(0,4);1, 226(0,5);0,008(0,3);0,000(5,7) |
| I-3-3: |
| HPLC-MS: logP = 2,96; Masse (m/z): 424,9 (M+H)⁺; ¹H-NMR(400,0 MHz, DMSO-D₆): |
| δ= 10,802(10,5);7,610(1,4);7,593(3,2);7,589(3,0);7,572(5,8);7,556(3,2);7,551(3,6);7,534(1,8);7,531(1,6);7,515(2,7);7,510(2,3);7,499(2,0);7, 493(5,5);7,488(2,5);7,476(2,6);7,472(3,9);7,457(1,8);7,405(9,0);7,364(10,0);7,342(16,0);7,321(7,4);7,298(0,6);7,261(2,1);7,254(10,0);7,235( 14,6);7,214(8,4);7,207(1,9);6,956(7,7);6,315(8,5);6,311(9,3);6,307(9,1);6,303(8,2);4,119(0,5);4,106(1,5);4,093(1,5);4,080(0,5);3,330(20,7);3 ,177(6,7);3,164(6,4);2,676(0,3);2,672(0,4);2,668(0,3);2,507(45,6);2,503(58,5);2,499(43,6);2,330(0,4);1,339(1,1);1,233(0,4);1,226(1,5);0,000 (4,7) |
| I-3-4: |
| HPLC-MS: logP = 2,97; Masse (m/z): 363,0 (M+H)⁺; ¹H-NMR(400,0 MHz, DMSO-D₆): |
| δ= 10,484(2,3);7,508(0,5);7,501(0,5);7,492(0,4);7,486(1,1);7,481(0,6);7,472(0,5);7,469(0,6);7,465(0,9);7,447(2,9);7,425(3,0);7,396(1,8);7,3 59(2,1);7,348(0,5);7,338(3,2);7,317(1,3);7,119(2,3);7,111(3,2);7,069(1,8);7,062(1,5);7,047(1,6);7,039(1,5);6,927(1,6);6,329(1,7);6,325(1,9); 6,322(2,1);6,318(2,0);3,804(16,0);3,323(34,4);2,670(0,5);2,666(0,4);2,524(1,2);2,519(1,8);2,510(22,0);2,506(47,2);2,501(66,3);2,497(54,3); 2,492(32,5);2,328(0,4);2,324(0,4);2,075(0,5);0,000(0,7) |
| I-3-5: |
| HPLC-MS: logP = 2,52; Masse (m/z): 368,0 (M+H)⁺; ¹H-NMR(400,0 MHz, DMSO-D₆): |
| δ= 10,705(11,0);8,849(6,4);8,839(6,2);8,837(6,3);8,192(6,0);8,174(6,5);8,172(6,6);7,852(5,7);7,840(5,6);7,832(5,3);7,820(5,0);7,529(1,2);7, 514(2,6);7,509(2,0);7,507(2,1);7,498(1,8);7,492(5,4);7,487(2,3);7,478(2,1);7,475(2,4);7,471(4,0);7,456(1,8);7,385(8,4);7,373(1,9);7,368(2,6) ;7,362(10,1);7,341(16,0);7,320(6,4);6,955(7,1);6,320(8,2);6,316(9,0);6,312(8,9);6,308(8,3);5,758(0,6);3,325(92,7);2,676(0,7);2,671(1,0);2,6 66(0,7);2,541(0,4);2,524(3,0);2,511(54,5);2,506(108,3);2,502(142,7);2,497(104,9);2,493(51,3);2,338(0,4);2,333(0,7);2,329(1,0);2,324(0,7);0 ,000(2,5) |
| I-3-6: |
| HPLC-MS: logP = 2,96; Masse (m/z): 430,9 (M+H)⁺; ¹H-NMR(400,0 MHz, DMSO-D₆): |
| δ= 10,453(9,0);7,992(0,5);7,987(0,5);7,931(15,0);7,927(14,9);7,513(1,1);7,498(2,3);7,494(1,6);7,491(1,7);7,482(1,5);7,477(4,5);7,471(1,9);7 ,463(1,6);7,459(1,9);7,455(3,4);7,440(1,8);7,430(4,8);7,425(6,3);7,365(1,3);7,360(2,0);7,355(8,4);7,343(1,4);7,333(12,9);7,323(1,3);7,313(5, 2);7,306(1,5);7,204(0,4);6,949(0,7);6,945(0,7);6,926(1,9);6,922(3,8);6,919(4,9);6,915(5,4);6,909(3,7);6,906(2,0);6,893(16,0);6,889(15,8);6,4 91(6,8);6,487(7,1);6,483(7,0);6,479(6,6);5,757(13,2);3,821(0,5);3,327(14,6);2,672(0,4);2,526(1,5);2,512(24,8);2,508(49,5); 2,503(65,0);2,49 9(46,5);2,494(21,8);2,330(0,4);2,325(0,3);1,233(0,4);0,008(1,5);0,000(40,7);-0,009(1,3) |
| I-3-7: |
| HPLC-MS: logP = 3,07; Masse (m/z): 414,9 (M+H)⁺; ¹H-NMR(400,0 MHz, DMSO-D₆): |
| δ= 10,224(11,0);8,316(0,6);7,822(14,7);7,808(15,4);7,518(1,3);7,502(2,7);7,497(2,1);7,495(2,2);7,486(1,9);7,481(5,5);7,475(2,4);7,467(2,2); 7,464(2,5);7,460(4,0);7,444(2,0);7,419(8,5);7,367(1,6);7,356(10,2);7,335(16,0);7,314(6,3);7,308(2,0);7,284(15,4);7,271(14,7);6,926(7,2);6,3 80(8,4);6,377(9,0);6,373(8,9);6,369(8,1);5,756(3,0);3,321(26,0);2,675(1,3);2,671(1,7);2,666(1,3);2,524(6,2);2,510(97,7);2,506(190,1);2,502( 247,7);2,497(180,7);2,493(88,6);2,333(1,1);2,328(1,6);2,324(1,1); 1,989(0,6);1,398(0,3);1,384(2,4);1,234(0,6);1,175(0,4);0,000(2,1) |

### 1) Methodenbesehreibung zur Bestimmung der logP Werte (Ameisensäure Methode)

Die Bestimmung der in der Tabelle angegebenen logP-Werte erfolgte gemäß EEC-Directive 79/831 Annex V.A8 durch HPLC (High Performance Liquid Chromatography) an einer Phasenumkehrsäule (C 18). Temperatur: 55°C.

Eluenten für die Bestimmung im sauren Bereich (pH 3,4):
Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

### 2) Messung der NMR-Spektren ausgewählter Beispiele

Die NMR-Spektren wurden mit einem Bruker Avance 400, ausgestattet mit einem Durchflussprobenkopf (60 µl Volumen), bestimmt. Als Lösungsmittel wurden CD₃CN oder DMSO-D₆ verwendet, wobei als Referenz Tetramethylsilan (0.00 ppm) eingesetzt wurde. In Einzelfällen wurden die NMR Spektren mit einem Bruker Avance II 600 bestimmt. Als Lösungsmittel wurden CD₃CN oder DMSO-D₆ verwendet, wobei als Referenz Tetramethylsilan (0.00 ppm) eingesetzt wurde.

Die NMR-Daten ausgewählter Beispiele werden entweder in klassischer Form (d-Werte, Anzahl der H-Atome, Multiplettaufspaltung) oder als NMR-Peak-Listen aufgeführt.

Die Aufstpaltung der Signale wurde wie folgt beschrieben: s (Singulett), d (Duplett), t (Triplett), q (Quartett), quin (Quintett), m (Multiplett).

### NMR-Peak-Listenverfahren

Wenn die ¹H-NMR-Daten ausgewählter Beispiele in Form von ¹H-NMR-Peaklisten notiert werden, wird zu jedem Signalpeak erst der d-Wert in ppm und dann die Signalintensität in runden Klammern aufgeführt. Die d-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensität;);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

### Anwendungsbeispiele

Die folgenden Beispiele zeigen die insektizide, akarizide und nematizide Wirkung der erfindungsgemäßen Verbindungen. Dabei beziehen sich die genannten erfindungsgemäßen Verbindungen auf die in den Tabellen 1 bis 5 mit den entsprechenden Referenzzeichen, z. B. I-1-1, aufgeführten Verbindungen:

### Beispiel 1:

### Boophilus microplus - Test (DIP)

Testtiere: adulte gesogene Weibchen von *Boophilus microplus* Stamm Parkhurst - (SP- resistent)

| | |
|---|---|
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 Zecken werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf im Klimaschrank aufbewahrt. Eine Wirkung von 100 % bedeutet, dass keine der Zecken fertile Eier gelegt hat.

Bei diesem Test zeigten z.B. die folgenden Verbindungen der Herstellungsbeispiele eine Wirkung von 80% bei einer Aufwandmenge von 100 ppm : I-1-32, I-1-82

Bei diesem Test zeigten z.B. die folgenden Verbindungen der Herstellungsbeispiele eine Wirkung von 90% bei einer Aufwandmenge von 100 ppm : I-1-39, I-1-53, I-1-91

Bei diesem Test zeigten z.B. die folgenden Verbindungen der Herstellungsbeispiele eine Wirkung von 100% bei einer Aufwandmenge von 100 ppm : I-1-5, I-1-33, I-1-48, I-1-83, I-1-96, I-1-99, I-2-1

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigten z.B. die folgenden Verbindungen der Herstellungsbeispiele eine Wirkung von 80 % bei einer Aufwandmenge von 20 µg / Tier : I-1-55

Bei diesem Test zeigten z.B. die folgenden Verbindungen der Herstellungsbeispiele eine Wirkung von 100 % bei einer Aufwandmenge von 20 µg / Tier :

| | | | | | | | | | |
|---|---|---|---|---|---|---|---|---|---|
| I-1-4 | I-1-20 | I-1-33 | I-1-52 | I-1-69 | I-1-85 | I-1-102 | I-1-128 | I-1-240 | I-3-1 |
| I-1-5 | I-1-21 | I-1-37 | I-1-53 | I-1-73 | I-1-90 | I-1-103 | I-1-131 | I-1-253 | I-3-2 |
| I-1-6 | I-1-22 | I-1-39 | I-1-54 | I-1-75 | I-1-91 | I-1-107 | I-1-135 | I-1-254 | |
| I-1-9 | I-1-23 | I-1-43 | I-1-63 | I-1-81 | I-1-93 | I-1-109 | I-1-136 | I-1-562 | |
| I-1-11 | I-1-24 | I-1-48 | I-1-65 | I-1-82 | I-1-96 | I-1-110 | I-1-145 | I-2-1 | |
| I-1-13 | I-1-25 | I-1-50 | I-1-67 | I-1-83 | I-1-99 | I-1-113 | I-1-186 | I-2-10 | |
| I-1-14 | I-1-32 | I-1-51 | I-1-68 | I-1-84 | I-1-101 | I-1-122 | I-1-238 | I-2-2 | |

### Ctenocephalides felis - Oraltest (CTECFE)

| | |
|---|---|
| Lösungsmittel: | 1 Gewichtsteil Dimethylsulfoxid |

Zwecks Herstellung einer zweckmäßigen Wirkstoffzubereitung vermischt man 10 mg Wirkstoff mit 0,5 ml Dimethylsulfoxid. Ein Teil des Konzentrats wird mit citriertem Rinderblut verdünnt und die gewünschte Konzentration hergestellt.

Ca. 20 nüchterne adulte Flöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass kein Floh abgetötet wurde.

Bei diesem Test zeigten z.B. die folgenden Verbindungen der Herstellungsbeispiele eine Wirkung von 80 % bei einer Aufwandmenge von 100 ppm : I-1-91, I-1-99

Bei diesem Test zeigten z.B. die folgenden Verbindungen der Herstellungsbeispiele eine Wirkung von 85 % bei einer Aufwandmenge von 100 ppm : I-1-128

Bei diesem Test zeigten z.B. die folgenden Verbindungen der Herstellungsbeispiele eine Wirkung von 90 % bei einer Aufwandmenge von 100 ppm : I-1-131, I-2-1, I-2-10

### Lucilia cuprina-Test (LUCICU)

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit ca 20 *Lucilia cuprina* Larven besetzt.

Nach 2 Tage wird die Abtötung in % bestimmt. Dabei bedeutet 100 %, dass alle Larven abgetötet wurden; 0 % bedeutet, dass keine Larven abgetötet wurden.

Bei diesem Test zeigten z.B. die folgenden Verbindungen der Herstellungsbeispiele eine Wirkung von 80 % bei einer Aufwandmenge von 100 ppm : I-1-63, I-1-186, I-2-2, I-3-1

Bei diesem Test zeigten z.B. die folgenden Verbindungen der Herstellungsbeispiele eine Wirkung von 90 % bei einer Aufwandmenge von 100 ppm : I-1-20, I-1-37, I-1-51, I-1-69, I-1-145

Bei diesem Test zeigten z.B. die folgenden Verbindungen der Herstellungsbeispiele eine Wirkung von 95 % bei einer Aufwandmenge von 100 ppm : I-1-82

Bei diesem Test zeigten z.B. die folgenden Verbindungen der Herstellungsbeispiele eine Wirkung von 100 % bei einer Aufwandmenge von 100 ppm :

| | | | | | | | | |
|---|---|---|---|---|---|---|---|---|
| I-1-5 | I-1-32 | I-1-50 | I-1-65 | I-1-81 | I-1-93 | I-1-107 | I-1-128 | I-1-562 |
| I-1-6 | I-1-33 | I-1-52 | I-1-67 | I-1-83 | I-1-96 | I-1-109 | I-1-131 | I-2-1 |
| I-1-11 | I-1-39 | I-1-53 | I-1-73 | I-1-85 | I-1-99 | I-1-110 | I-1-135 | |
| I-1-14 | I-1-43 | I-1-54 | I-1-75 | I-1-91 | I-1-101 | I-1-113 | I-1-254 | |

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica*-Adulten besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigten z.B. die folgenden Verbindungen der Herstellungsbeispiele eine Wirkung von 85 % bei einer Aufwandmenge von 20 ppm : I-1-91

Bei diesem Test zeigten z.B. die folgenden Verbindungen der Herstellungsbeispiele eine Wirkung von 90 % bei einer Aufwandmenge von 20 ppm : I-1-99

Bei diesem Test zeigten z.B. die folgenden Verbindungen der Herstellungsbeispiele eine Wirkung von 90 % bei einer Aufwandmenge von 100 ppm : I-1-562

### Cooperia curticei - Test (COOPCU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration.

Gefäße mit der Wirkstoffzubereitung der gewünschten Konzentration werden mit ca. 40 Nematodenlarven (*Cooperia curticei)* besetzt.

Nach 5 Tagen wird die Abtötung in % bestimmt. Dabei bedeutet 100 %, dass alle Larven abgetötet wurden; 0 % bedeutet, dass keine der Larven abgetötet wurde.

Bei diesem Test zeigten z.B. die folgenden Verbindungen der Herstellungsbeispiele eine Wirkung von 80 % bei einer Aufwandmenge von 4 ppm: I-2-7, I-1-284

Bei diesem Test zeigten z.B. die folgenden Verbindungen der Herstellungsbeispiele eine Wirkung von 80 % bei einer Aufwandmenge von 20 ppm:

| | | | | | | | | | |
|---|---|---|---|---|---|---|---|---|---|
| I-1-44 | I-1-219 | I-1-288 | I-1-421 | I-1-495 | I-1-591 | I-2-18 | I-2-58 | I-2-66 | I-3-3 |
| I-1-156 | | | | | | | | | |

Bei diesem Test zeigten z.B. die folgenden Verbindungen der Herstellungsbeispiele eine Wirkung von 80 % bei einer Aufwandmenge von 100 ppm: I-1-22, I-1-69, I-1-121, I-1-211

Bei diesem Test zeigten z.B. die folgenden Verbindungen der Herstellungsbeispiele eine Wirkung von 90 % bei einer Aufwandmenge von 4 ppm: I-1-221, I-1-555

Bei diesem Test zeigten z.B. die folgenden Verbindungen der Herstellungsbeispiele eine Wirkung von 90 % bei einer Aufwandmenge von 20 ppm:

| | | | | | | | |
|---|---|---|---|---|---|---|---|
| I-1-63 | I-1-465 | I-1-484 | I-1-488 | I-1-501 | I-1-587 | I-2-2 | I-2-49 |
| I-1-284 | I-1-470 | I-1-487 | I-1-499 | I-1-581 | I-1-593 | I-2-37 | |

Bei diesem Test zeigten z.B. die folgenden Verbindungen der Herstellungsbeispiele eine Wirkung von 90 % bei einer Aufwandmenge von 100 ppm:

| | | | | | | |
|---|---|---|---|---|---|---|
| I-1-11 | I-1-20 | I-1-39 | I-1-54 | I-1-91 | I-1-135 | I-1-562 |
| I-1-13 | I-1-32 | I-1-51 | I-1-79 | I-1-107 | I-1-136 | I-2-6 |
| I-1-14 | I-1-36 | I-1-52 | I-1-81 | I-1-132 | I-1-138 | I-4-3 |

Bei diesem Test zeigten z.B. die folgenden Verbindungen der Herstellungsbeispiele eine Wirkung von 100 % bei einer Aufwandmenge von 4 ppm: I-2-1

Bei diesem Test zeigten z.B. die folgenden Verbindungen der Herstellungsbeispiele eine Wirkung von 100 % bei einer Aufwandmenge von 20 ppm:

| | | | | | | | |
|---|---|---|---|---|---|---|---|
| I-1-6 | I-1-142 | I-1-277 | I-1-468 | I-1-502 | I-2-17 | I-2-40 | I-2-62 |
| I-1-9 | I-1-253 | I-1-335 | I-1-472 | I-1-507 | I-2-35 | I-2-41 | I-3-1 |
| I-1-10 | I-1-254 | I-1-459 | I-1-492 | I-1-575 | I-2-36 | I-2-44 | I-3-6 |
| I-1-64 | I-1-259 | I-1-467 | I-1-494 | I-1-580 | I-2-38 | I-2-57 | I-3-7 |

Bei diesem Test zeigten z.B. die folgenden Verbindungen der Herstellungsbeispiele eine Wirkung von 100 % bei einer Aufwandmenge von 100 ppm:

| | | | | | | | | |
|---|---|---|---|---|---|---|---|---|
| I-1-5 | I-1-62 | I-1-73 | I-1-85 | I-1-103 | I-1-119 | I-1-133 | I-1-146 | I-2-5 |
| I-1-33 | I-1-65 | I-1-75 | I-1-92 | I-1-104 | I-1-128 | I-1-140 | I-1-151 | I-2-10 |
| I-1-46 | I-1-66 | I-1-82 | I-1-93 | I-1-105 | I-1-129 | I-1-143 | I-1-162 | |
| I-1-50 | I-1-67 | I-1-83 | I-1-96 | I-1-109 | I-1-130 | I-1-144 | I-1-491 | |
| I-1-53 | I-1-71 | I-1-84 | I-1-99 | I-1-113 | I-1-131 | I-1-145 | I-1-506 | |

### Haemonchus contortus - Test (HAEMCO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration.

Gefäße mit der Wirkstoffzubereitung der gewünschten Konzentration werden mit ca 40 Larven des Roten Magenwurmes (*Haemonchus contortus*) besetzt.

Nach 5 Tagen wird die Abtötung in % bestimmt. Dabei bedeutet 100 %, dass alle Larven abgetötet wurden; 0 % bedeutet, dass keine der Larven abgetötet wurde.

Bei diesem Test zeigten z.B. die folgenden Verbindungen der Herstellungsbeispiele eine Wirkung von 80 % bei einer Aufwandmenge von 4 ppm: I-2-1, I-2-51

Bei diesem Test zeigten z.B. die folgenden Verbindungen der Herstellungsbeispiele eine Wirkung von 80 % bei einer Aufwandmenge von 20 ppm:

| | | | | | | | |
|---|---|---|---|---|---|---|---|
| I-1-9 | I-1-63 | I-1-335 | I-1-467 | I-1-502 | I-1-554 | I-1-591 | I-2-55 |
| I-1-36 | I-1-259 | I-1-427 | I-1-484 | I-1-549 | I-1-589 | I-2-12 | I-2-66 |

Bei diesem Test zeigten z.B. die folgenden Verbindungen der Herstellungsbeispiele eine Wirkung von 80 % bei einer Aufwandmenge von 100 ppm:

| | | | | | | | |
|---|---|---|---|---|---|---|---|
| I-1-10 | I-1-20 | I-1-46 | I-1-81 | I-1-96 | I-1-129 | I-1-151 | I-1-562 |
| I-1-11 | I-1-33 | I-1-71 | I-1-85 | I-1-99 | I-1-136 | I-1-162 | |

Bei diesem Test zeigten z.B. die folgenden Verbindungen der Herstellungsbeispiele eine Wirkung von 90 % bei einer Aufwandmenge von 4 ppm: I-1-221, I-2-49

Bei diesem Test zeigten z.B. die folgenden Verbindungen der Herstellungsbeispiele eine Wirkung von 90 % bei einer Aufwandmenge von 20 ppm:

| | | | | | | | | | |
|---|---|---|---|---|---|---|---|---|---|
| I-1-64 | I-1-186 | I-1-277 | I-1-366 | I-1-472 | I-1-582 | I-1-593 | I-2-48 | I-2-62 | I-3-7 |
| I-1-66 | I-1-252 | I-1-288 | I-1-378 | I-1-492 | I-1-587 | I-2-13 | I-2-58 | I-3-6 | |

Bei diesem Test zeigten z.B. die folgenden Verbindungen der Herstellungsbeispiele eine Wirkung von 90 % bei einer Aufwandmenge von 100 ppm:

| | | | | | | |
|---|---|---|---|---|---|---|
| I-1-13 | I-1-36 | I-1-73 | I-1-83 | I-1-103 | I-1-133 | I-1-491 |
| I-1-35 | I-1-54 | I-1-82 | I-1-84 | I-1-132 | I-1-251 | I-2-5 |

Bei diesem Test zeigten z.B. die folgenden Verbindungen der Herstellungsbeispiele eine Wirkung von 100 % bei einer Aufwandmenge von 20 ppm:

| | | | | | | |
|---|---|---|---|---|---|---|
| I-1-68 | I-1-494 | I-1-581 | I-2-18 | I-2-38 | I-2-49 | I-3-1 |
| I-1-142 | I-1-575 | I-2-14 | I-2-35 | I-2-41 | I-2-50 | I-3-3 |
| I-1-219 | I-1-580 | I-2-17 | I-2-36 | I-2-44 | I-2-51 | I-3-5 |

Bei diesem Test zeigten z.B. die folgenden Verbindungen der Herstellungsbeispiele eine Wirkung von 100 % bei einer Aufwandmenge von 100 ppm:

| | | | | | | | | | |
|---|---|---|---|---|---|---|---|---|---|
| I-1-66 | I-1-104 | I-1-105 | I-1-130 | I-1-140 | I-1-143 | I-1-144 | I-1-145 | I-1-146 | I-2-10 |

### Meloidogyne incognita- Test (MELGIN)

| | | |
|---|---|---|
| Lösungsmittel: | 125,0 | Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigten z. B. die folgenden Verbindungen der Herstellungsbeispiele Wirkung von 80 % bei einer Aufwandmenge von 20 ppm : I-1-137

Bei diesem Test zeigten z. B. die folgenden Verbindungen der Herstellungsbeispiele Wirkung von 90 % bei einer Aufwandmenge von 20 ppm :

| | | | | | | | | | |
|---|---|---|---|---|---|---|---|---|---|
| I-1-6 | I-1-130 | I-1-255 | I-1-354 | I-1-419 | I-1-492 | I-1-535 | I-1-641 | I-2-56 | I-4-4 |
| I-1-20 | I-1-131 | I-1-256 | I-1-395 | I-1-438 | I-1-499 | I-1-540 | I-2-2 | I-2-68 | |
| I-1-59 | I-1-147 | I-1-274 | I-1-398 | I-1-455 | I-1-502 | I-1-555 | I-2-12 | I-2-70 | |
| I-1-77 | I-1-190 | I-1-284 | I-1-409 | I-1-466 | I-1-512 | I-1-588 | I-2-18 | I-2-76 | |
| I-1-100 | I-1-192 | I-1-308 | I-1-410 | I-1-471 | I-1-518 | I-1-596 | I-2-23 | I-2-80 | |
| I-1-120 | I-1-205 | I-1-335 | I-1-415 | I-1-482 | I-1-528 | I-1-598 | I-2-37 | I-2-84 | |
| I-1-125 | I-1-254 | I-1-345 | I-1-418 | I-1-484 | I-1-531 | I-1-599 | I-2-52 | I-3-1 | |

Bei diesem Test zeigten z. B. die folgenden Verbindungen der Herstellungsbeispiele Wirkung von 100 % bei einer Aufwandmenge von 20 ppm :

| | | | | | | | | | |
|---|---|---|---|---|---|---|---|---|---|
| I-1-3 | I-1-74 | I-1-86 | I-1-117 | I-1-142 | I-1-459 | I-1-574 | I-2-11 | I-2-46 | I-2-79 |
| I-1-14 | I-1-75 | I-1-90 | I-1-121 | I-1-143 | I-1-465 | I-1-575 | I-2-13 | I-2-48 | I-2-82 |
| I-1-50 | I-1-76 | I-1-91 | I-1-122 | I-1-144 | I-1-468 | I-1-591 | I-2-17 | I-2-49 | I-2-83 |
| I-1-57 | I-1-81 | I-1-92 | I-1-128 | I-1-145 | I-1-470 | I-1-622 | I-2-35 | I-2-51 | |
| I-1-65 | I-1-82 | I-1-93 | I-1-129 | I-1-146 | I-1-472 | I-1-626 | I-2-36 | I-2-62 | |
| I-1-67 | I-1-83 | I-1-95 | I-1-135 | I-1-152 | I-1-488 | I-1-629 | I-2-38 | I-2-66 | |
| I-1-70 | I-1-84 | I-1-96 | I-1-140 | I-1-191 | I-1-491 | I-1-631 | I-2-41 | I-2-67 | |
| I-1-73 | I-1-85 | I-1-99 | I-1-141 | I-1-208 | I-1-519 | I-2-10 | I-2-44 | I-2-71 | |

### Myzus persicae - Sprühtest (MYZUPK)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekiueusis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 80 % bei einer Aufwandmenge von 500 g/ha : I-1-25

Bei diesem Test zeigten z. B. die folgenden Verbindungen der Herstellungsbeispiele Wirkung von 90 % bei einer Aufwandmenge von 500 g/ha : I-1-63, I-1-80, I-1-133, I-1-145, I-1-280, I-2-39, I-2-58

Bei diesem Test zeigten z. B. die folgenden Verbindungen der Herstellungsbeispiele Wirkung von 90 % bei einer Aufwandmenge von 20 g/ha : I-1-86

### Phaedon cochleariae - Sprühtest (PHAECO)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 83 % bei einer Aufwandmenge von 500 g/ha :

| | | | | | | |
|---|---|---|---|---|---|---|
| I-1-10 | I-1-19 | I-1-77 | I-1-108 | I-1-275 | I-1-415 | I-1-433 |
| I-1-16 | I-1-34 | I-1-102 | I-1-124 | I-1-317 | I-1-426 | I-1-551 |

Bei diesem Test zeigten z. B. die folgenden Verbindungen der Herstellungsbeispiele Wirkung von 100 % bei einer Aufwandmenge von 500 g/ha :

| | | | | | | | | | |
|---|---|---|---|---|---|---|---|---|---|
| I-1-1 | I-1-28 | I-1-50 | I-1-74 | I-1-103 | I-1-132 | I-1-259 | I-1-555 | I-2-8 | I-3-1 |
| I-1-3 | I-1-29 | I-1-51 | I-1-75 | I-1-107 | I-1-133 | I-1-260 | I-1-556 | I-2-9 | I-3-2 |
| I-1-4 | I-1-31 | I-1-52 | I-1-76 | I-1-109 | I-1-134 | I-1-261 | I-1-559 | I-2-10 | I-3-5 |
| I-1-5 | I-1-32 | I-1-53 | I-1-78 | I-1-110 | I-1-135 | I-1-262 | I-1-562 | I-2-12 | I-4-1 |
| I-1-6 | I-1-33 | I-1-54 | I-1-81 | I-1-111 | I-1-136 | I-1-263 | I-1-564 | I-2-13 | I-4-2 |
| I-1-7 | I-1-35 | I-1-55 | I-1-82 | I-1-113 | I-1-137 | I-1-264 | I-1-565 | I-2-15 | I-4-3 |
| I-1-9 | I-1-36 | I-1-57 | I-1-83 | I-1-114 | I-1-138 | I-1-335 | I-1-566 | I-2-17 | |
| I-1-11 | I-1-37 | I-1-58 | I-1-84 | I-1-117 | I-1-168 | I-1-352 | I-1-578 | I-2-35 | |
| I-1-12 | I-1-38 | I-1-60 | I-1-85 | I-1-118 | I-1-190 | I-1-372 | I-1-580 | I-2-36 | |
| I-1-13 | I-1-39 | I-1-61 | I-1-86 | I-1-119 | I-1-231 | I-1-384 | I-1-596 | I-2-37 | |
| I-1-14 | I-1-40 | I-1-63 | I-1-90 | I-1-120 | I-1-238 | I-1-395 | I-1-599 | I-2-38 | |
| I-1-15 | I-1-41 | I-1-65 | I-1-91 | I-1-121 | I-1-240 | I-1-403 | I-1-642 | I-2-39 | |
| I-1-17 | I-1-42 | I-1-66 | I-1-92 | I-1-122 | I-1-243 | I-1-406 | I-2-1 | I-2-41 | |
| I-1-20 | I-1-43 | I-1-67 | I-1-93 | I-1-123 | I-1-248 | I-1-408 | I-2-2 | I-2-44 | |
| I-1-21 | I-1-44 | I-1-68 | I-1-94 | I-1-125 | I-1-253 | I-1-410 | I-2-3 | I-2-45 | |
| I-1-22 | I-1-45 | I-1-69 | I-1-95 | I-1-127 | I-1-254 | I-1-411 | I-2-4 | I-2-46 | |
| I-1-23 | I-1-46 | I-1-70 | I-1-96 | I-1-128 | I-1-255 | I-1-417 | I-2-5 | I-2-49 | |
| I-1-24 | I-1-48 | I-1-72 | I-1-99 | I-1-129 | I-1-256 | I-1-428 | I-2-6 | I-2-55 | |
| I-1-25 | I-1-49 | I-1-73 | I-1-101 | I-1-131 | I-1-258 | I-1-550 | I-2-7 | I-2-57 | |

### Spodoptera frugiperda - Sprühtest (SPODFR)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigten z. B. die folgenden Verbindungen der Herstellungsbeispiele Wirkung von 83 % bei einer Aufwandmenge von 100 g/ha : I-1-118

Bei diesem Test zeigten z. B. die folgenden Verbindungen der Herstellungsbeispiele Wirkung von 83 % bei einer Aufwandmenge von 500 g/ha : I-1-17, I-1-29, I-1-34

Bei diesem Test zeigten z. B. die folgenden Verbindungen der Herstellungsbeispiele Wirkung von 100 % bei einer Aufwandmenge von 500 g/ha : I-1-35, I-1-75, I-1-136, I-1-259, I-1-403, I-2-41

### Tetranychus urticae - Sprühtest, OP-resistent (TETRUR)

| | | |
|---|---|---|
| Lösungsmittel: | 78,0 | GewichtsteileAceton |
| | 1,5 | Gewichtsteile Dimethylformamid |
| | | |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*), die von allen Stadien der Gemeinen Spinnmilbe (*tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 80 % bei einer Aufwandmenge von 500g /ha : I-1-10, I-1-18, I-1-39, I-1-248

Bei diesem Test zeigten z. B. die folgenden Verbindungen der Herstellungsbeispiele eine Wirkung von 85 % bei einer Aufwandmenge von 500g /ha: I-1-598

Bei diesem Test zeigten z. B. die folgenden Verbindungen der Herstellungsbeispiele eine Wirkung von 90 % bei einer Aufwandmenge von 500g /ha :

| | | | | | | | | | |
|---|---|---|---|---|---|---|---|---|---|
| I-1-7 | I-1-51 | I-1-73 | I-1-103 | I-1-116 | I-1-188 | I-1-238 | I-1-281 | I-1-360 | I-1-472 |
| I-1-11 | I-1-52 | I-1-79 | I-1-107 | I-1-145 | I-1-203 | I-1-259 | I-1-338 | I-1-370 | I-1-558 |
| I-1-26 | I-1-53 | I-1-80 | I-1-109 | I-1-157 | I-1-212 | I-1-264 | I-1-354 | I-1-433 | |
| I-1-32 | I-1-69 | I-1-99 | I-1-113 | I-1-186 | I-1-213 | I-1-275 | I-1-355 | I-1-463 | |

Bei diesem Test zeigten z. B. die folgenden Verbindungen der Herstellungsbeispiele eine Wirkung von 90 % bei einer Aufwandmenge von 100g /ha: I-1-43

Bei diesem Test zeigten z. B. die folgenden Verbindungen der Herstellungsbeispiele eine Wirkung von 95 % bei einer Aufwandmenge von 500g /ha : I-1-210, I-1-258, I-1-262, I-1-263, I-1-428, I-1-596

Bei diesem Test zeigten z. B. die folgenden Verbindungen der Herstellungsbeispiele eine Wirkung von 100 % bei einer Aufwandmenge von 500g /ha :

| | | | | | | | | | |
|---|---|---|---|---|---|---|---|---|---|
| I-1-5 | I-1-20 | I-1-35 | I-1-48 | I-1-66 | I-1-83 | I-1-185 | I-1-253 | I-1-372 | I-2-62 |
| I-1-6 | I-1-33 | I-1-37 | I-1-64 | I-1-67 | I-1-101 | I-1-214 | I-1-254 | I-1-403 | |

## Patentansprüche

1. Nicht-therapeutische Verwendung von Verbindungen der Formel (I) 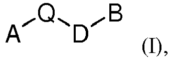 worin
A für einen Rest aus der Reihe 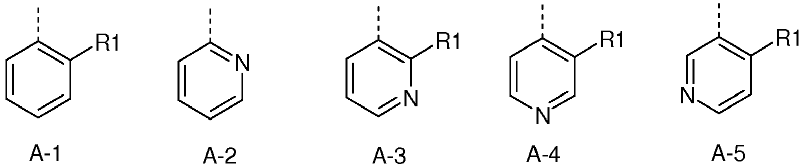 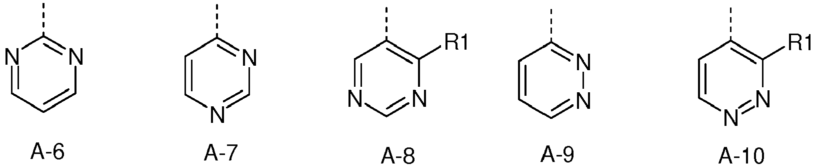 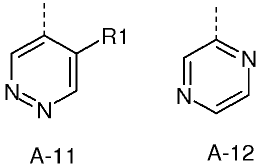 steht, in dem die gestrichelte Linie die Bindung zu Q bedeutet und worin A weiterhin m Substituenten R2 trägt,
Q für einen Rest aus der Reihe 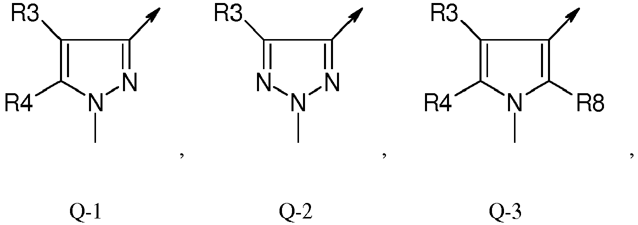 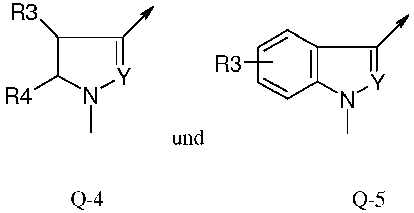 steht, worin der Stickstoff mit dem Ring A verknüpft ist und der Pfeil jeweils die Bindung zu D bedeutet, und
D für den Rest der Formel 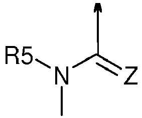 steht, worin der Stickstoff mit Q verbunden ist und der Pfeil die Bindung zu B bedeutet,
B für einen Rest aus der Reihe 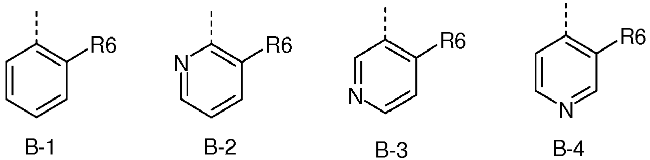 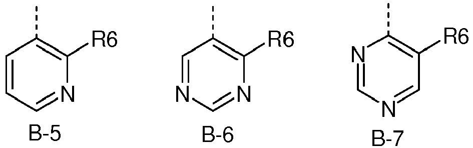 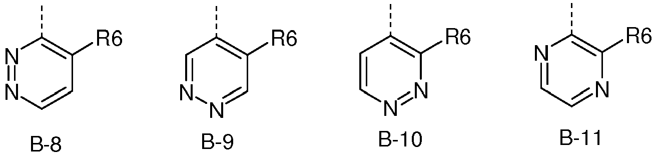 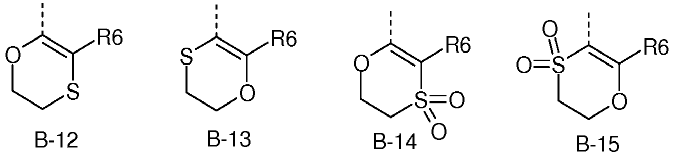 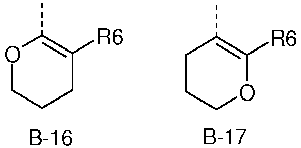 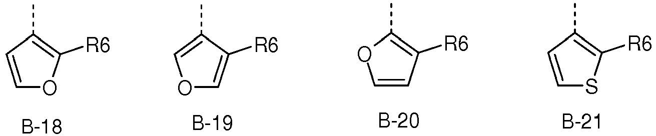 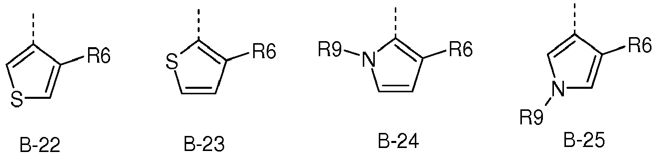 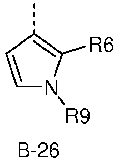 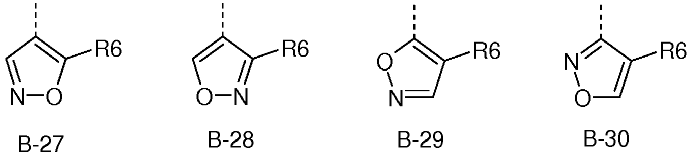 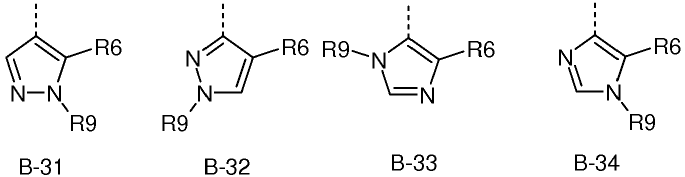 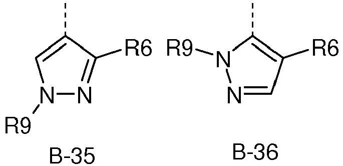 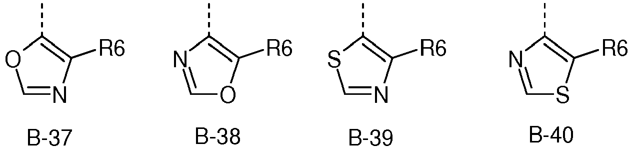 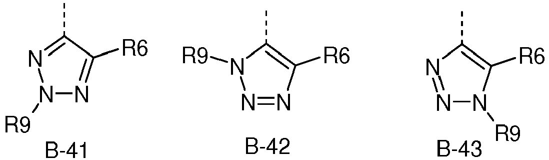 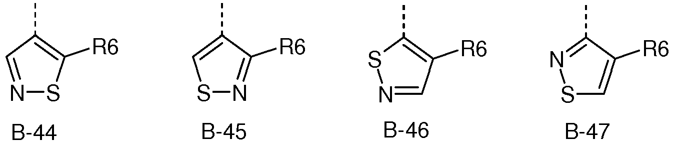 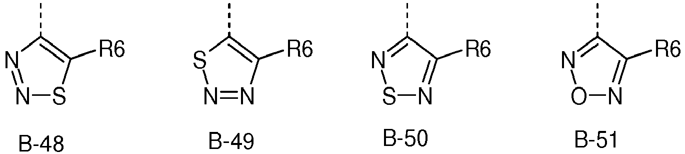 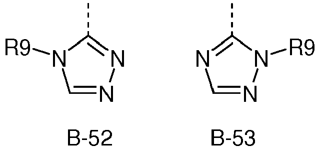 steht, worin die gestrichelte Linie die Bindung zu D bedeutet und wobei B weiterhin n Substituenten R7 trägt,
Y im Rest Q-4 für CR8 oder für Stickstoff steht,
Y im Rest Q-5 für Stickstoff steht,
Z für Sauerstoff oder Schwefel steht,
R1 für einen Rest aus der Reihe Halogen, Cyano, Nitro, Amino, Hydroxy, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, C₄-C₁₂-Bicycloalkyl, Aryl, Aryloxy, Arylamino, Arylthio, Heteroaryl, Heteroaryloxy, Heteroarylamino und Heteroarylthio steht, wobei die Substituenten unabhängig voneinander ausgewählt sind aus Halogen, Cyano, Nitro, Hydroxy, Amino, C₁-C₆-Alkyl, C₁-C₆-Alkoxy, C₃-C₆-Cycloalkyl, C₁-C₆-Halogenalkoxy, C₁-C₆-Alkylthio, Aryl, Aryloxy, Arylthio, Heteroaryl, Heteroaryloxy und Heteroarylthio,
R2 für einen Rest aus der Reihe Halogen, Cyano, Nitro, Amino, Hydroxy, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenoxy, C₃-C₆-Alkinoxy, C₃-C₆-Cycloalkoxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, Di-(C₁-C₆-alkyl)-amino, C₃-C₆-Alkenylamino, C₃-C₆-Alkinylamino, C₃-C₆-Cycloalkylamino, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, C₄-C₁₂-Bicycloalkyl, Aryl, Aryloxy, Arylamino, Arylthio, Heteroaryl, Heteroaryloxy, Heteroarylamino und Heteroarylthio steht, wobei die Substituenten unabhängig voneinander ausgewählt sind aus Halogen, Cyano, Nitro, Hydroxy, Amino, C₁-C₆-Alkyl, C₁-C₆-Alkoxy, C₃-C₆-Cycloalkyl, C₁-C₆-Halogenalkoxy und C₁-C₆-Alkylthio, Aryl, Aryloxy, Arylthio, Heteroaryl, Heteroaryloxy und Heteroarylthio,
R3 für einen Rest aus der Reihe Wasserstoff, Halogen, Cyano, Nitro, Amino, Hydroxy, C₁-C₆-Alkyl, C₁-C₆-Alkoxy, C₁-C₆-Halogenalkyl und C₁-C₆-Halogenalkoxy steht,
R4 für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy steht,
R5 für einen Rest aus der Reihe Wasserstoff, C₁-C₆-Alkyl, C₃-C₆-Cycloalkyl, C₁-C₆-Halogenalkyl, C₂-C₆-Alkenyl, C₃-C₆-Alkinyl, C₃-C₆-Cycloalkyl-C₁-C₆-alkyl, Cyano-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl und C₁-C₆-Halogenalkylsulfonyl oder für C(=O)-B steht,
R6 für einen Rest aus der Reihe Wasserstoff (nur in den Resten B-26, B-33, B-36 und B-42), Halogen, Cyano, Nitro, Amino, Hydroxy, Carboxyl, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenoxy, C₃-C₆-Alkinoxy, C₃-C₆-Cycloalkoxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Halogenalkylcarbonylamino, C₁-C₆-Alkylthiocarbonylamino, C₄-C₁₂-Bicycloalkyl, Aryl, Aryloxy, Heteroaryl, Heteroaryloxy steht, wobei die Substituenten unabhängig voneinander ausgewählt sind aus Halogen, Cyano, Nitro, Hydroxy, Amino, C₁-C₆-Alkyl, C₁-C₆-Alkoxy, C₃-C₆-Cycloalkyl, C₁-C₆-Halogenalkoxy und C₁-C₆-Alkylthio,
R7 für einen Rest aus der Reihe Halogen, Nitro, Cyano, Amino, Hydroxy, für gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, C₁-C₆-Alkoxy, C₃-C₆-Alkenoxy, C₃-C₆-Alkinoxy, C₃-C₆-Cycloalkoxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylcarbonylamino, C₂-C₆-Alkenylcarbonylamino, C₂-C₆-Alkinylcarbonylamino, C₃-C₆-Cycloalkylcarbonylamino, C₁-C₆-Alkoxycarbonylamino, C₁-C₆-Alkylsulfonylamino, C₁-C₆-Alkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl, Heteroaryloxy, C₄-C₁₂-Bicycloalkyl steht, wobei die Substituenten unabhängig voneinander ausgewählt sind aus Halogen, Cyano, Nitro, Hydroxy, Amino, C₁-C₆-Alkyl, C₁-C₆-Alkoxy, C₃-C₆-Cycloalkyl, C₁-C₆-Halogenalkoxy und C₁-C₆-Alkylthio,
R8 für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy steht,
R9 für einen Rest aus der Reihe Wasserstoff, C₁-C₆-Alkyl, C₃-C₆-Cycloalkyl, Halogenalkyl, C₃-C₆-Alkenyl, C₃-C₆-Alkinyl, C₃-C₆-Cycloalkyl-C₁-C₆-alkyl, Cyano-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl und C₁-C₆-Halogenalkylsulfonyl steht,
m für eine Zahl aus der Reihe 0, 1, 2 und 3 steht, wobei für m > 1 die Reste R2 gleich oder verschieden sein können und
n für eine Zahl aus der Reihe 0, 1, 2 und 3 steht, wobei für n > 1 die Reste R7 gleich oder verschieden sein können, zur Bekämpfung von tierischen Schädlingen.

2. Nicht-therapeutische Verwendung von Verbindungen der Formel (I) gemäß Anspruch 1, in welchen
A für einen Rest aus der Reihe 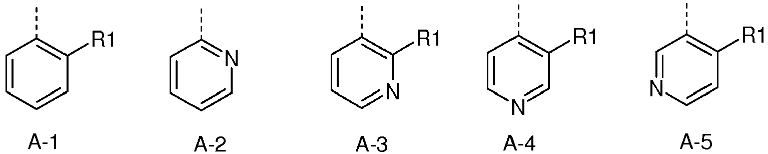 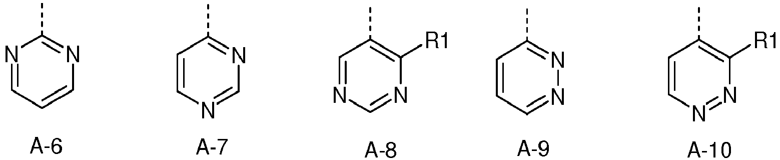 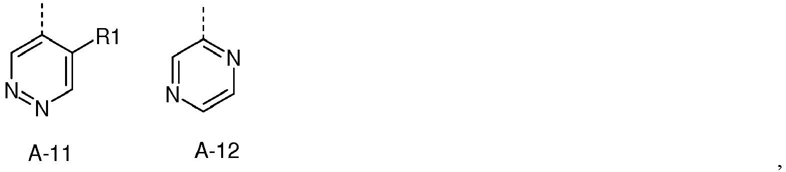 steht, in dem die gestrichelte Linie die Bindung zu Q bedeutet und wobei A weiterhin m Substituenten R2 trägt,
Q für einen Rest aus der Reihe 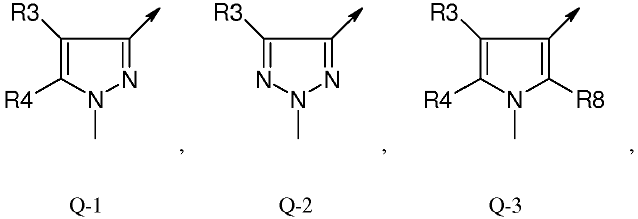 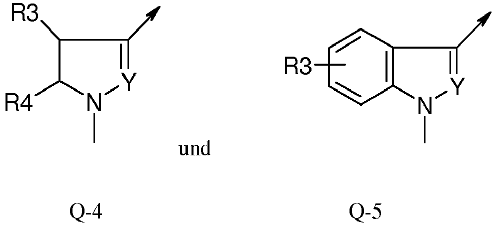
D steht, worin der Stickstoff mit dem Ring A verknüpft ist und der Pfeil jeweils die Bindung zu bedeutet,
D für den Rest der Formel 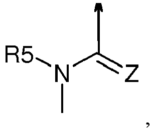 steht, worin der Stickstoff mit Q verbunden ist und der Pfeil die Bindung zu B bedeutet
B für einen Rest aus der Reihe 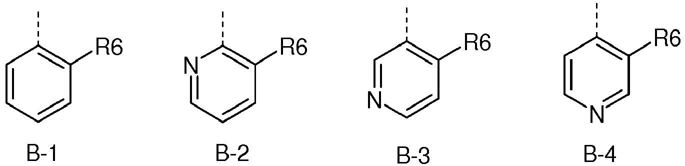 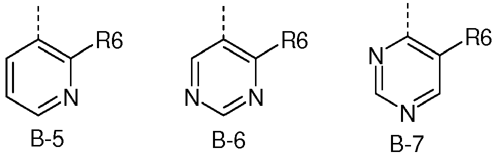 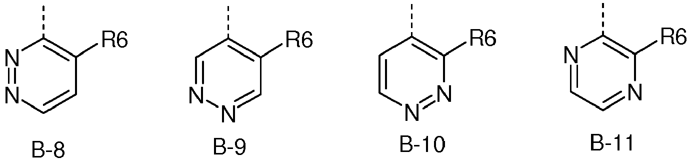 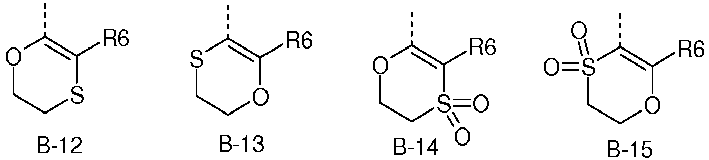 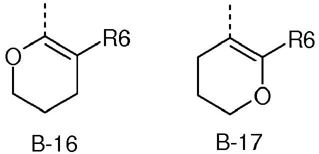 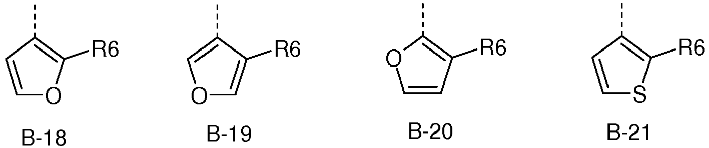 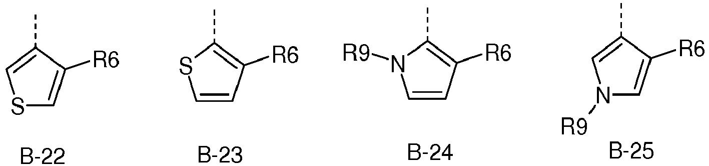 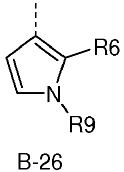 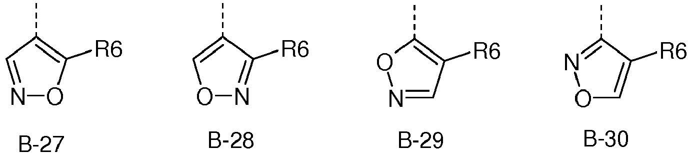 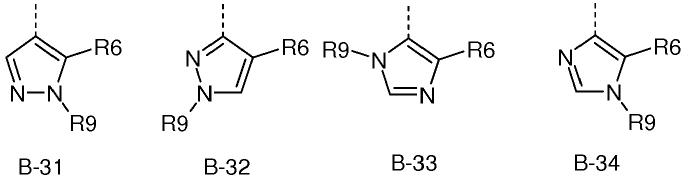 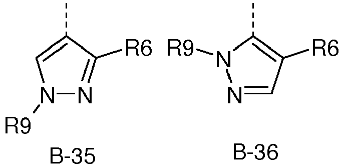 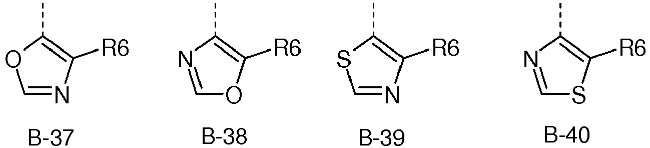 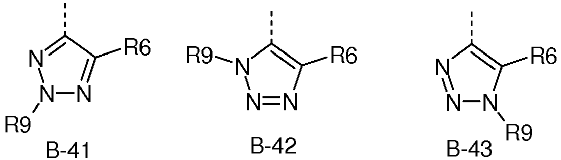 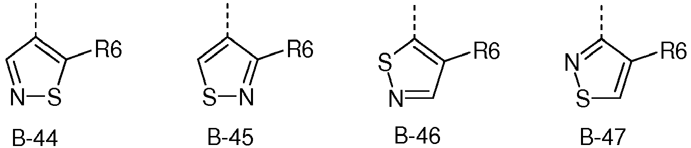 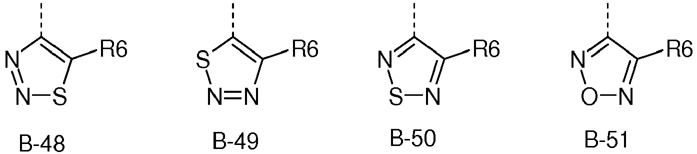 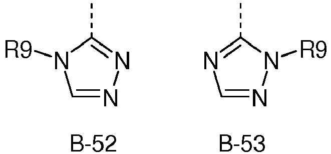 steht, worin die gestrichelte Linie die Bindung zu D bedeutet und wobei B weiterhin n Substituenten R7 trägt,
Y im Rest Q-4 für CR8 oder für Stickstoff steht,
Y im Rest Q-5 für Stickstoff steht,
Z für Sauerstoff oder Schwefel steht,
R1 für einen Rest aus der Reihe Halogen, Cyano, Nitro, Amino, Hydroxy, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl und Heteroaryloxy steht,
R2 für einen Rest aus der Reihe Halogen, Cyano, Nitro, Amino, Hydroxy, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, Di-(C₁-C₆-alkyl)-amino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl und Heteroaryloxy steht,
R3 für einen Rest aus der Reihe Wasserstoff, Halogen, Cyano, Nitro, Amino, C₁-C₆-Alkyl und C₁-C₆-Halogenalkyl steht,
R4 für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy steht,
R5 für einen Rest aus der Reihe Wasserstoff, C₁-C₆-Alkyl, C₁-C₆-Halogenalkyl, Cyano-C₁-C₆-alkyl, C₂-C₆-Alkenyl, C₃-C₆-Alkinyl, C₃-C₆-Cycloalkyl, C₃-C₆-Cycloalkyl-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl, C₁-C₆-Halogengenalkylsufonyl und C(=O)-B steht,
R6 für einen Rest aus der Reihe Wasserstoff (nur in den Resten B-26, B-33, B-36 und B-42), Halogen, Cyano, Nitro, Amino, Hydroxy, Carboxyl, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₄-Halogenalkylcarbonylamino, C₁-C₆-Alkylthiocarbonylamino, Aryl, Aryloxy, Heteroaryl und Heteroaryloxy steht,
R7 für einen Rest aus der Reihe Halogen, Cyano, Nitro, Amino, Hydroxy, C₁-C₆-Alkyl, C₁-C₆-Halogenalkyl, Cyano-C₁-C₆-alkyl, C₂-C₆-Alkenyl, C₃-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylcarbonyloxy, C₂-C₆-Alkenylcarbonyloxy, C₂-C₆-Alkinylcarbonyloxy, C₃-C₆-Cycloalkylcarbonyloxy, C₁-C₆-Alkoxycarbonyloxy, C₁-C₆-Alkylsulfonyloxy, C₁-C₆-Alkylamino, C₃-C₆-Alkenylamino, C₃-C₆-Alkinylamino, C₃-C₆-Cycloalkylamino, C₁-C₆-Alkylthio, C₁-C₆-Halogenalkylthio, C₃-C₆-Alkenylthio, C₃-C₆-Alkinylthio, C₃-C₆-Cycloalkylthio, C₁-C₆-Alkylsulfinyl, C₁-C₆-Alkylsulfonyl, C₁-C₆-Alkylcarbonyl, C₁-C₆-Alkoxyimino-C₁-C₆-alkyl, C₁-C₆-Alkoxycarbonyl, Aminocarbonyl, C₁-C₆-Alkylaminocarbonyl, Di-(C₁-C₆-alkyl)-aminocarbonyl, Aminothiocarbonyl, C₁-C₆-Alkylaminosulfonyl, C₁-C₆-Alkylsulfonylamino, C₁-C₆-Alkylcarbonylamino, C₁-C₆-Alkylthiocarbonylamino, Aryl, Aryloxy, Heteroaryl und Heteroaryloxy steht,
R8 für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy steht,
R9 für einen Rest aus der Reihe Wasserstoff, C₁-C₆-Alkyl, C₃-C₆-Cycloalkyl, C₁-C₆-Halogenalkyl, C₃-C₆-Alkenyl, C₃-C₆-Alkinyl, C₃-C₆-Cycloalkyl-C₁-C₆-alkyl, Cyano-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl und C₁-C₆-Halogenalkylsulfonyl steht,
m für eine Zahl aus der Reihe 0, 1, 2 und 3 steht, wobei für m > 1 die Reste R2 gleich oder verschieden sein können und
n für eine Zahl aus der Reihe 0, 1, 2 und 3 steht, wobei für n > 1 die Reste R7 gleich oder verschieden sein können.

3. Nicht-therapeutische Verwendung von Verbindungen der Formel (I) gemäß Anspruch 1, in welchen
A für einen Rest aus der Reihe 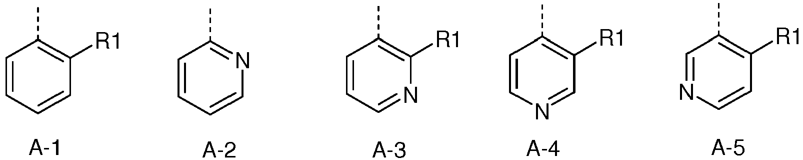 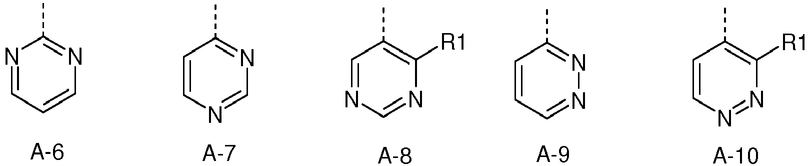 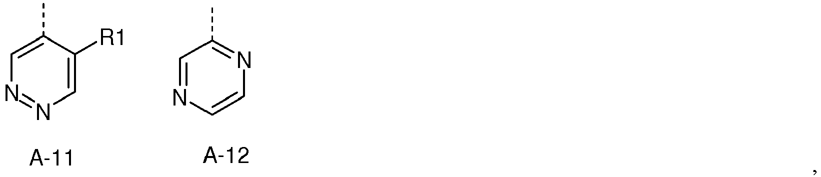 steht, in dem die gestrichelte Linie die Bindung zu Q bedeutet und wobei A weiterhin m Substituenten R2 trägt,
Q für einen Rest aus der Reihe 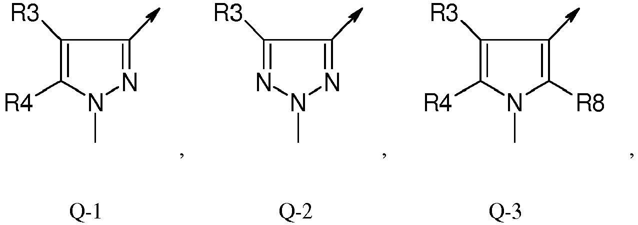 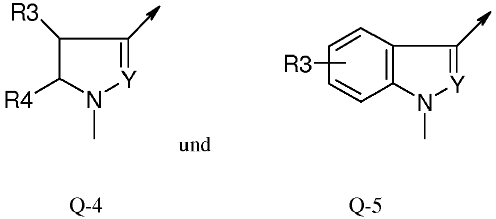 steht, worin der Stickstoff mit dem Ring A verknüpft ist und der Pfeil jeweils die Bindung zu D bedeutet,
D für den Rest der Formel 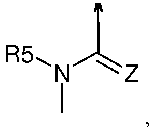 steht, worin der Stickstoff mit Q verbunden ist und der Pfeil die Bindung zu B bedeutet,
B für einen Rest aus der Reihe 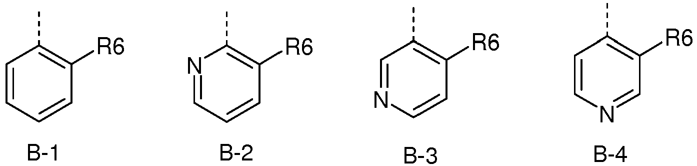 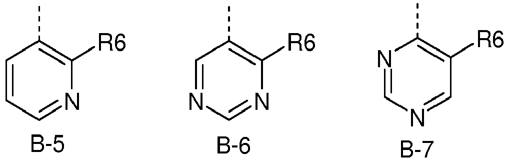 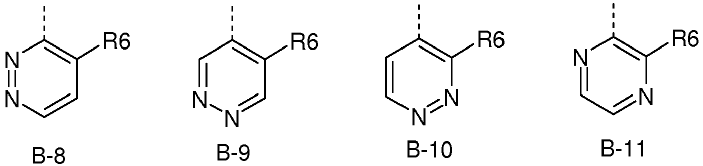 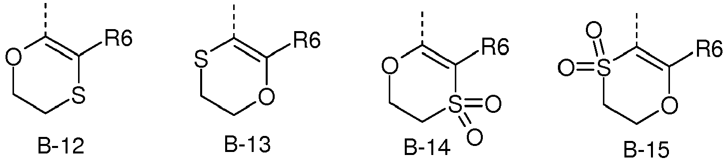 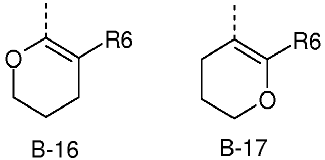 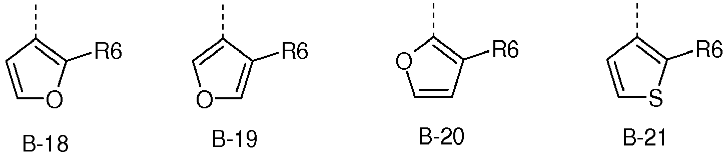 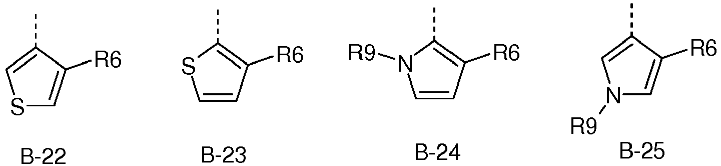 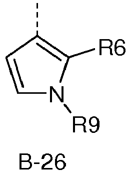 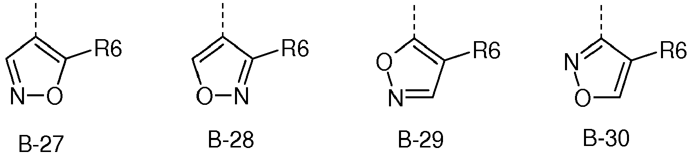 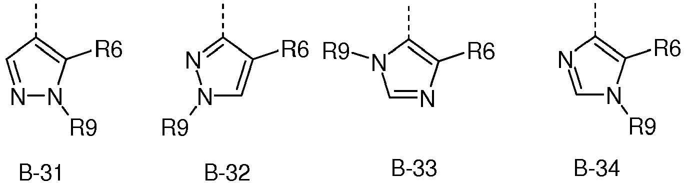 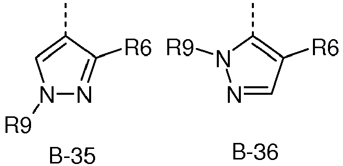 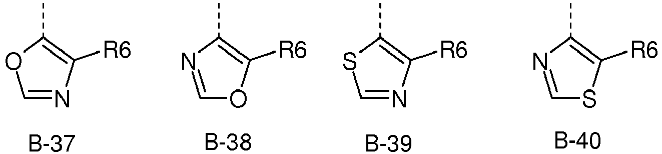 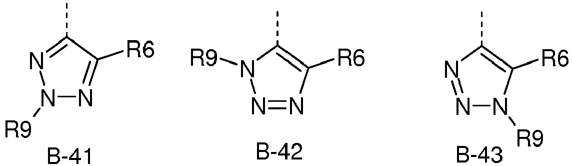 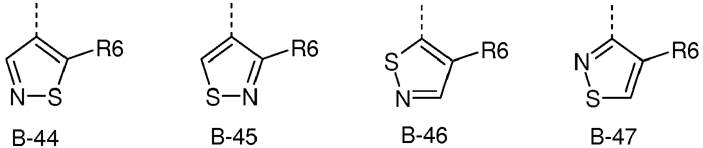 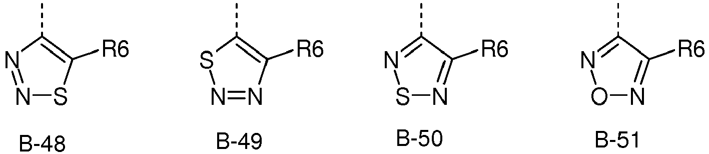 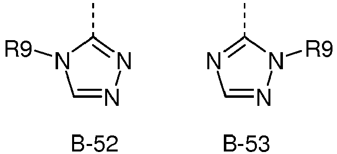 steht, worin die gestrichelte Linie die Bindung zu D bedeutet und wobei B weiterhin n Substituenten R7 trägt,
Y im Rest Q-4 für CR8 oder für Stickstoff steht,
Y im Rest Q-5 für Stickstoff steht,
Z für Sauerstoff oder Schwefel steht,
R1 für einen Rest aus der Reihe Halogen, Cyano, Nitro, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylthio, C₁-C₆-Halogenalkylthio, C₁-C₆-Alkylsulfinyl, C₁-C₆-Alkylsulfonyl, C₁-C₆-Alkoxyimino-C₁-C₆-alkyl, Aryl, Aryloxy, Heteroaryl und Heteroaryloxy steht,
R2 für einen Rest aus der Reihe Halogen, Cyano, Nitro, Amino, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylthio, C₁-C₆-Halogenalkylthio, C₁-C₆-Alkylsulfinyl, C₁-C₆-Alkylsulfonyl, C₁-C₆-Alkylcarbonyl, Di-(C₁-C₆-alkyl)-amino, C₁-C₆-Alkoxyimino-C₁-C₆-alkyl, Aryl, Aryloxy, Heteroaryl und Heteroaryloxy steht,
R3 für einen Rest aus der Reihe Wasserstoff, Halogen, Cyano, Nitro, Amino, C₁-C₆-Alkyl und C₁-C₆-Halogenalkyl steht,
R4 für einen Rest aus der Reihe Wasserstoff, Halogen, Amino, Hydroxy, C₁-C₆-Alkyl, C₁-C₆-Alkoxy, C₁-C₆-Halogenalkyl und C₁-C₆-Halogenalkoxy steht,
R5 für einen Rest aus der Reihe Wasserstoff, C₁-C₆-Alkyl, C₁-C₆-Halogenalkyl, Cyano-C₁-C₆-alkyl, C₂-C₆-Alkenyl, C₃-C₆-Alkinyl, C₃-C₆-Cycloalkyl, C₃-C₆-Cycloalkyl-C₁-C₆-alkyl, C₁-C₆-Alkylcarbonyl, C₂-C₆-Alkenylcarbonyl, C₁-C₆-Halogenalkylcarbonyl, C₂-C₆-Halogenalkenylcarbonyl, C₁-C₆-Alkoxy-C₁-C₆-alkyl, C₁-C₆-Alkoxycarbonyl, C₁-C₆-Alkylsulfonyl, C₁-C₆-Halogenalkylsulfonyl und C(=O)-B steht,
R6 für einen Rest aus der Reihe Wasserstoff (nur in den Resten B-26, B-33, B-36 und B-42), Halogen, Cyano, Nitro, Hydroxy, Carboxyl, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylsulfinyl, C₁-C₆-Alkylthio, C₁-C₆-Halogenalkylthio, C₁-C₆-Alkylsulfonyl, C₁-C₆-Alkylcarbonyl, C₁-C₆-Alkoxyimino-C₁-C₆-alkyl, C₁-C₆-Alkylcarbonylamino, C₁-C₆-Halogenalkylcarbonylamino, Aryl, Aryloxy, Heteroaryl und Heteroaryloxy steht,
R7 für einen Rest aus der Reihe Halogen, Cyano, Nitro, Hydroxy, C₁-C₆-Alkyl, C₁-C₆-Halogenalkyl, Cyano-C₁-C₆-alkyl, C₂-C₆-Alkenyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₃-C₆-Alkenyloxy, C₃-C₆-Alkinyloxy, C₃-C₆-Cycloalkyloxy, C₁-C₆-Alkylthio, C₁-C₆-Halogenalkylthio, C₁-C₆-Alkylsulfinyl, C₁-C₆-Alkylsulfonyl, C₁-C₆-Alkoxyimino-C₁-C₆-alkyl, Aryl, Aryloxy, Heteroaryl und Heteroaryloxy steht,
R8 für einen Rest aus der Reihe Wasserstoff, C₁-C₆-Alkyl, C₁-C₆-Alkoxy, C₁-C₆-Halogenalkyl und C₁-C₆-Halogenalkoxy steht,
R9 für einen Rest aus der Reihe Wasserstoff, C₁-C₆-Alkyl, C₃-C₆-Cycloalkyl und C₁-C₆-Halogenalkyl steht,
m für eine Zahl aus der Reihe 0, 1, 2 und 3 steht, wobei für m > 1 die Reste R2 gleich oder verschieden sein können und
n für eine Zahl aus der Reihe 0, 1, 2 und 3 steht, wobei für n > 1 die Reste R7 gleich oder verschieden sein können.

4. Nicht-therapeutische Verwendung von Verbindungen der Formel (I) gemäß Anspruch 1, in welchen
A für einen Rest aus der Reihe 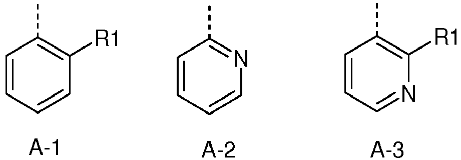 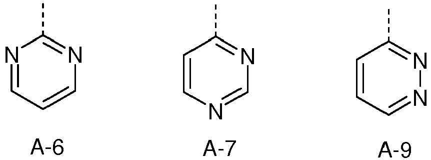 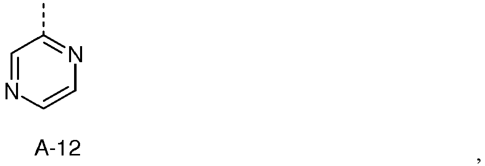 steht, in dem die gestrichelte Linie die Bindung zu Q bedeutet und wobei A weiterhin m Substituenten R2 trägt,
Q für einen Rest aus der Reihe 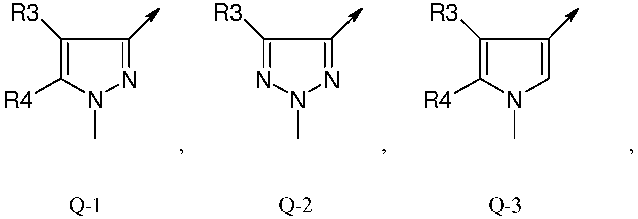 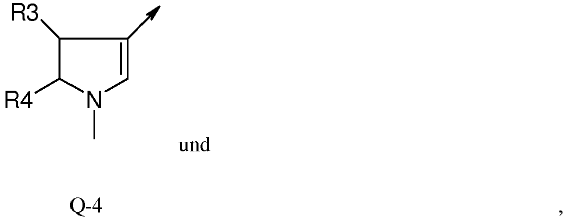 steht, worin der Stickstoff mit dem Ring A verknüpft ist und der Pfeil jeweils die Bindung zu D bedeutet,
D für den Rest der Formel 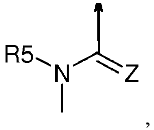 steht, worin der Stickstoff mit Q verbunden ist und der Pfeil die Bindung zu B bedeutet,
B für einen Rest aus der Reihe 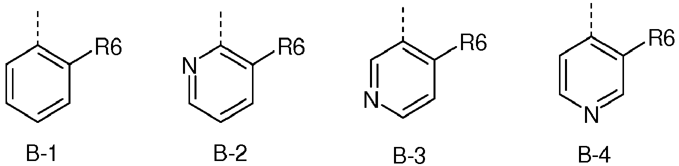 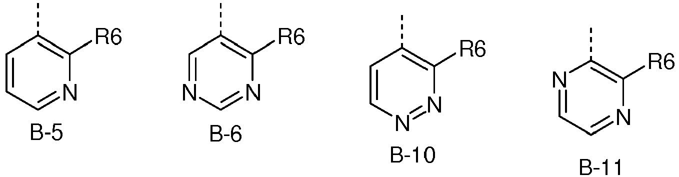 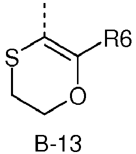 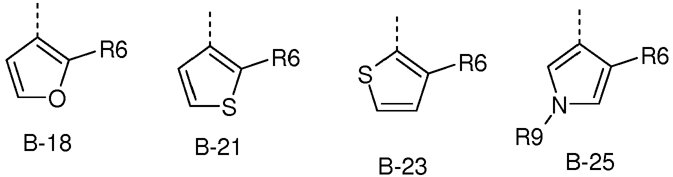 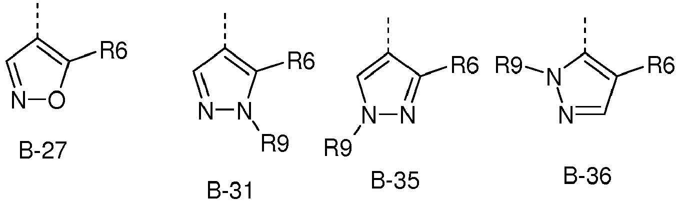 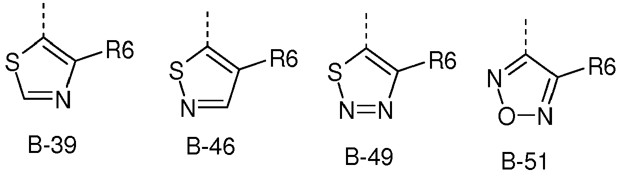 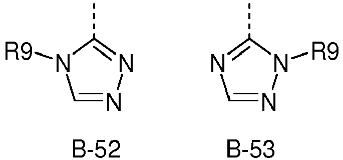 steht, worin die gestrichelte Linie die Bindung zu D bedeutet und wobei B weiterhin n Substituenten R7 trägt,
Z für Sauerstoff oder Schwefel steht,
R1 für einen Rest aus der Reihe Halogen, Cyano, Nitro, C₁-C₄-Alkyl, C₁-C₄-Halogenalkyl, C₁-C₄-Alkoxy und C₁-C₄-Alkylsulfonyl steht,
R2 für einen Rest aus der Reihe Halogen, C₁-C₄-Alkyl und C₁-C₄-Alkoxy steht,
R3 für einen Rest aus der Reihe Wasserstoff und Halogen steht,
R4 für einen Rest aus der Reihe Wasserstoff und C₁-C₄-Alkyl steht,
R5 für einen Rest aus der Reihe Wasserstoff, C₁-C₄-Alkyl, C₃-C₄-Alkenyl, C₃-C₄-Alkinyl, C₃-C₆-Cycloalkyl-C₁-C₄-alkyl, C₁-C₄-Alkylcarbonyl, Cyano-C₁-C₄-alkyl und C(=O)-B steht,
R6 für einen Rest aus der Reihe Halogen, Nitro, Hydroxy, C₁-C₄-Alkyl, C₁-C₄-Alkoxy, C₁-C₄-Alkylthio, C₁-C₄-Alkylsulfonyl, C₁-C₄-Halogenalkyl, C₁-C₄-Halogenalkoxy, C₁-C₄-Halogenalkylthio und Heteroaryl steht,
R7 für einen Rest aus der Reihe Halogen, C₁-C₄-Alkyl und C₁-C₄-Halogenalkyl steht,
R9 für einen Rest aus der Reihe C₁-C₄-Alkyl steht,
m für eine Zahl aus der Reihe 0, 1, 2 und 3 steht, wobei für m > 1 die Reste R2 gleich oder verschieden sein können und
n für eine Zahl aus der Reihe 0 und 1 steht.

5. Nicht-therapeutische Verwendung von Verbindungen der Formel (I) gemäß Anspruch 1, in welchen
A für einen Rest aus der Reihe 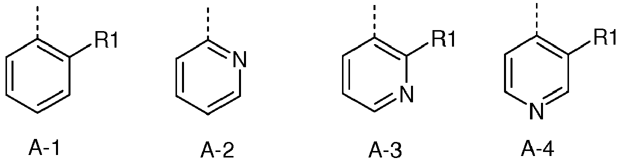 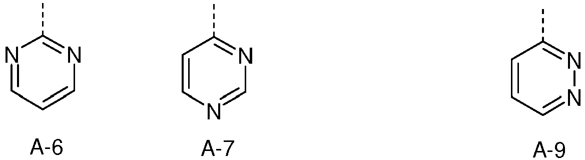 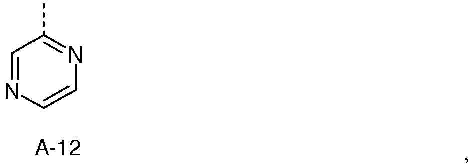 steht, in dem die gestrichelte Linie die Bindung zu Q bedeutet und wobei A weiterhin m Substituenten R2 trägt,
Q für einen Rest aus der Reihe 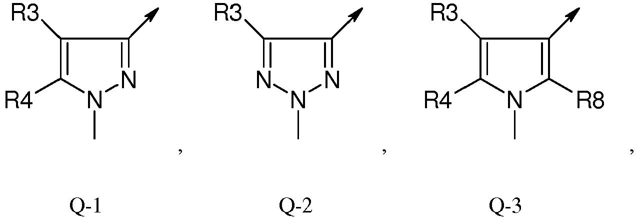 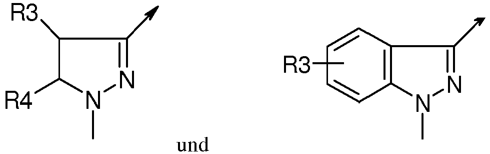 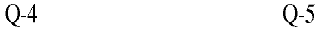
D steht, worin der Stickstoff mit dem Ring A verknüpft ist und der Pfeil jeweils die Bindung zu bedeutet,
D für den Rest der Formel 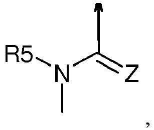 steht, worin der Stickstoff mit Q verbunden ist und der Pfeil die Bindung zu B bedeutet,
B für einen Rest aus der Reihe 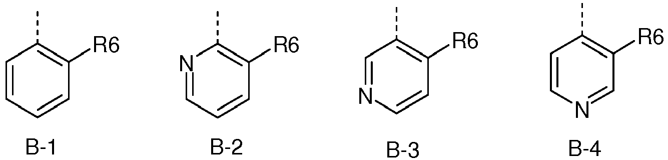 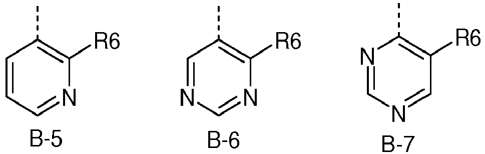 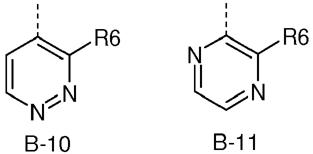 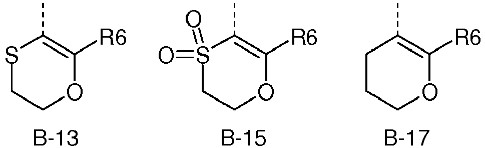 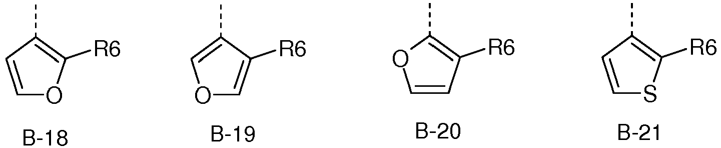 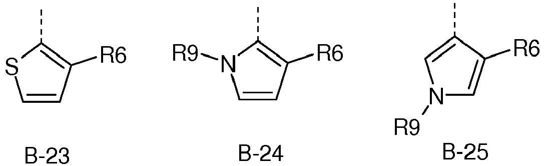 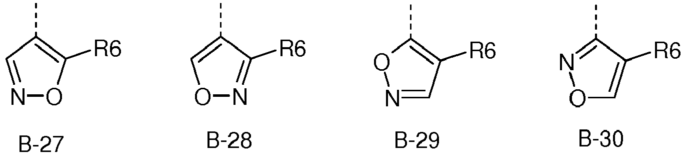 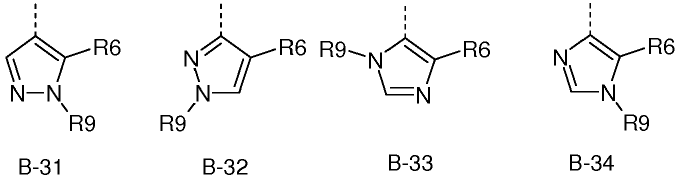 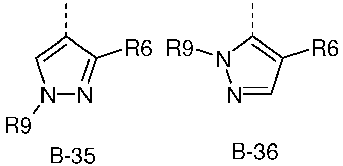 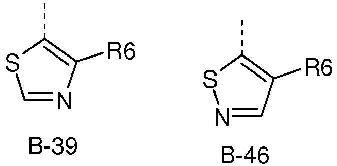 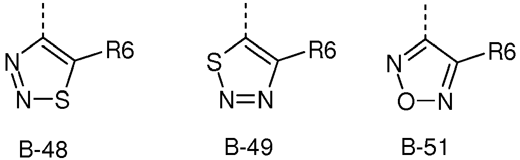 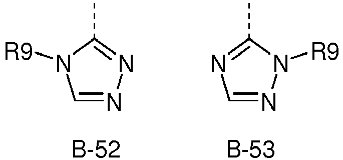 steht, worin die gestrichelte Linie die Bindung zu D bedeutet und wobei B weiterhin n Substituenten R7 trägt,
Z für Sauerstoff oder Schwefel steht,
R1 für einen Rest aus der Reihe Halogen, Cyano, Nitro, C₁-C₄-Alkyl, C₁-C₄-Halogenalkyl, C₁-C₄-Alkoxy und C₁-C₄-Alkylsulfonyl steht,
R2 für einen Rest aus der Reihe Halogen, Cyano, Nitro, Amino, C₁-C₄-Alkyl, C₁-C₄-Halogenalkyl, C₃-C₆-Cycloalkyl, C₁-C₄-Alkoxy, Di-(C₁-C₆-alkyl)-amino, C₁-C₄-Alkylcarbonyl, Aryl steht,
R3 für einen Rest aus der Reihe Wasserstoff, Halogen, Cyano, Nitro, C₁-C₄-Alkyl steht,
R4 für einen Rest aus der Reihe Wasserstoff, Amino, C₁-C₄-Alkyl steht,
R5 für einen Rest aus der Reihe Wasserstoff, C₁-C₄-Alkyl, C₃-C₄-Alkenyl, C₃-C₄-Alkinyl, C₃-C₆-Cycloalkyl-C₁-C₄-alkyl, C₁-C₄-Alkylcarbonyl, Cyano-C₁-C₄-alkyl und C(=O)-B steht,
R6 für einen Rest aus der Reihe Wasserstoff (nur in den Resten B-33 und B-36), Halogen, Cyano, Nitro, Hydroxy, Carboxyl, C₁-C₄-Alkyl, C₁-C₄-Alkoxy, C₁-C₄-Alkylthio, C₁-C₄-Alkylsulfonyl, C₁-C₄-Halogenalkyl, C₃-C₆-Cycloalkyl, C₁-C₄-Halogenalkoxy, C₁-C₄-Halogenalkylthio, C₁-C₄-Alkylcarbonyl, C₁-C₄-Alkylcarbonylamino, C₁-C₄-Halogenalkylcarbonylamino und Heteroaryl steht,
R7 für einen Rest aus der Reihe Halogen, Cyano, Nitro, Hydroxy, C₁-C₄-Alkyl, C₁-C₄-Halogenalkyl und C₁-C₄-Alkoxy steht,
R9 für einen Rest aus der Reihe C₁-C₄-Alkyl und C₁-C₄-Halogenalkyl steht,
m für eine Zahl aus der Reihe 0, 1, 2 und 3 steht, wobei für m > 1 die Reste R2 gleich oder verschieden sein können und
n für eine Zahl aus der Reihe 0, 1, 2 und 3 steht, wobei für n > 1 die Reste R7 gleich oder verschieden sein können.

6. Verbindungen der Formel (I) wie in einem der Ansprüche 1 bis 5 beschrieben, ausgenommen die folgenden Verbindungen 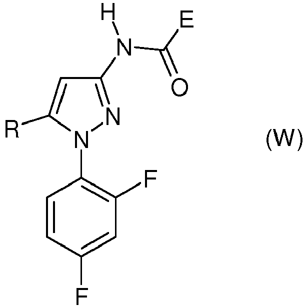
| Nr. | R | E |
|---|---|---|
| W-1 | Ethyl | 2-(Trifluormethyl)phenyl |
| W-2 | Ethyl | 2-Bromphenyl |
| W-3 | Ethyl | 2-Fluorphenyl |
| W-4 | Ethyl | 3-Methyl-2-thienyl |
| W-5 | Ethyl | 2,5-Dimethyl-3-furanyl |
| W-6 | H | 2,4-Dimethoxyphenyl |
| W-7 | H | 2-(Trifluormethyl)phenyl |
| W-8 | H | 2,4-Difluorphenyl |
| W-9 | Methyl | 2-Bromphenyl |
| W-10 | Ethyl | 2,4-Difluorphenyl |
| W-11 | H | 2,5-Dimethyl-3-furanyl |
| W-12 | Methyl | 2,4-Dimethoxyphenyl |

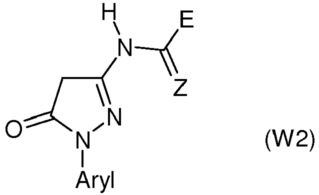
| Nr. | Z | E | Aryl |
|---|---|---|---|
| W2-1 | O | 2-Nitrophenyl | 3,4-Dichlor-2,5-dimethoxyphenyl |
| W2-2 | S | 2,4-Dihydroxyphenyl | 2,4,6-Trichlorphenyl |
| W2-3 | O | 2-Chlor-4-aminophenyl | 2,4,6-Trichlorphenyl |
| W2-5 | O | 2-Chlor-5-aminophenyl | 2,4,6-Trichlorphenyl |
| W2-6 | O | 2-Chlor-5-nitrophenyl | 2,4,6-Trichlorphenyl |
| W2-7 | O | 2-Chlor-3-(1-oxo-2-propen-1-yl)amino | 2,4,6-Trichlorphenyl |
| W2-8 | O | 2-Chlor-5-(2-methyl-1-oxo-2-propenyl)amino | 2,4,6-Trichlorphenyl |
| W2-9 | O | 2-Chlor-5-(1-oxo-2-propen-1-yl)amino | 2,4,6-Trichlorphenyl |

7. Verbindung der Formel (I) wie in Anspruch 1 beschrieben, ausgewählt aus der folgenden Liste:
| **Nr.** | **A** | **Q** | **B** | **R3** | **R4** | **R5** | **Z** |
|---|---|---|---|---|---|---|---|
| 1 | 2-(Trifluormethyl)phe nyl | Q-1 | 2-(Trifluormethyl)phenyl | H | H | H | O |
| 2 | 2-Chlorphenyl | Q-1 | 2-(Trifluormethyl)phenyl | H | H | H | O |
| 3 | 2-Bromphenyl | Q-1 | 2-(Trifluormethyl)phenyl | H | H | H | O |
| 4 | 2-Bromphenyl | Q-1 | 2-Iodphenyl | H | H | H | O |
| 5 | 2,5-Difluorphenyl | Q-1 | 2-(Trifluormethyl)phenyl | H | H | H | O |
| 6 | 2,6-Difluorphenyl | Q-1 | 2-Methylphenyl | H | H | H | O |
| 7 | 2,6-Difluorphenyl | Q-1 | 2-Difluormethylphenyl | H | H | H | O |
| 8 | 2,6-Difluorphenyl | Q-1 | 2-(Trifluormethyl)phenyl | H | H | H | O |
| 9 | 2,6-Difluorphenyl | Q-1 | 2-(Trifluormethyl)phenyl | F | H | H | O |
| 10 | 2,6-Difluorphenyl | Q-1 | 2-Chlorphenyl | H | H | H | O |
| 11 | 2,6-Difluorphenyl | Q-1 | 2-Bromphenyl | H | H | H | O |
| 12 | 2,6-Difluorphenyl | Q-1 | 2-Iodphenyl | H | H | H | O |
| 13 | 2,6-Difluorphenyl | Q-1 | 2,6-Difluorphenyl | H | H | H | O |
| 14 | 2-Ethoxy-6-fluorphenyl | Q-1 | 2-Chlorphenyl | H | H | H | O |
| 15 | 3,5-Difluorpyridin-2-yl | Q-1 | 2-(Trifluormethyl)phenyl | H | H | H | O |
| 16 | 3-Chlor-5-fluorpyridin-2-yl | Q-1 | 2-(Trifluormethyl)phenyl | H | H | H | O |
| 17 | 2,6-Difluorphenyl | Q-1 | 2-Fluor-6-(trifluormethyl)phenyl | H | H | H | O |
| 18 | 2,6-Difluorphenyl | Q-1 | 4-Fluor-2-(trifluormethyl)phenyl | H | H | H | O |
| 19 | 2-Bromphenyl | Q-1 | 2-Fluor-6-(trifluormethyl)phenyl | H | H | H | O |
| 20 | 2-Cyan-4,6-difluorphenyl | Q-1 | 2-(Trifluormethyl)phenyl | H | H | H | O |
| 21 | 2-Cyanphenyl | Q-1 | 2-Iodphenyl | H | H | H | O |
| 22 | 3,5-Difluorpyridin-2-yl | Q-1 | 2-(Trifluormethyl)phenyl | F | H | H | O |
| 23 | 3-Cyanpyridin-2-yl | Q-1 | 2-(Trifluormethyl)phenyl | H | H | H | O |
| 24 | 2-Cyan-4-fluorphenyl | Q-1 | 2-(Trifluormethyl)phenyl | H | H | H | O |
| 25 | 2,6-Difluorphenyl | Q-2 | 2-(Trifluormethyl)phenyl | H | - | H | O |

8. Mittel, **gekennzeichnet durch** einen Gehalt von mindestens einer Verbindung der Formel (I) gemäß Anspruch 6 oder 7.

9. Nicht-therapeutische Verfahren zum Bekämpfen von Schädlingen, **dadurch gekennzeichnet, dass** man eine Verbindung der Formel (I) wie in Ansprüchen 1 bis 5 beschrieben oder gemäß Anspruch 6 oder 7 oder ein Mittel gemäß Anspruch 8 auf die Schädlinge und/oder ihren Lebensraum einwirken lässt.

10. Verfahren gemäß Anspruch 9, **dadurch gekennzeichnet, dass** es sich bei den Schädlingen um Insekten, Spinnentiere und Nematoden handelt.

11. Verbindungen der Formel (I) wie in Ansprüchen 1 bis 5 beschrieben oder gemäß Anspruch 6 oder 7 zur Verwendung als Antiendoparasitikum.

12. Verbindungen der Formel (I) wie in Ansprüchen 1 bis 5 beschrieben oder gemäß Anspruch 6 oder 7 zur Verwendung als Antiektoparasitikum.

13. 2-(3,5-Difluorpyridin-2-yl)-2H-1,2,3-triazol-4-amin.

14. 1-(3,5-Difluorpyridin-2-yl)-1H-pyrazol-3-amin.

15. N-[1-(2,6-Difluorphenyl)-4,5-dihydro-1H-pyrazol-3-yl]acetamid.

16. N-[1-(2,6-Difluorphenyl)-1H-pyrazol-3-yl]acetamid.

17. 1-(2,6-Difluorphenyl)-1H-pyrazol-3-amin.

18. 1-(2,6-Difluorphenyl)-4,5-dihydro-1H-pyrazol-3-amin.

## Claims

1. Non-therapeutic use of compounds of the formula (I) 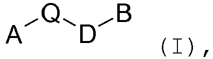 in which
A represents a radical from the group consisting of 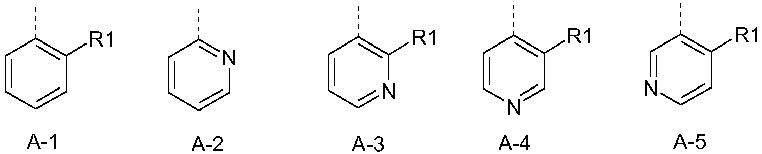 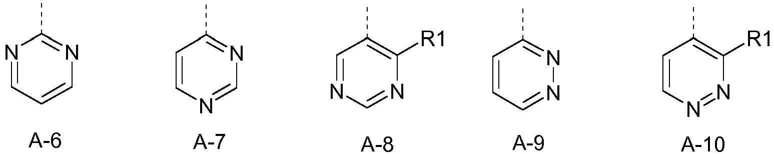 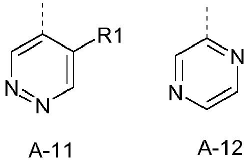 in which the broken line represents the bond to Q and in which A furthermore carries m substituents R2,
Q represents a radical from the group consisting of 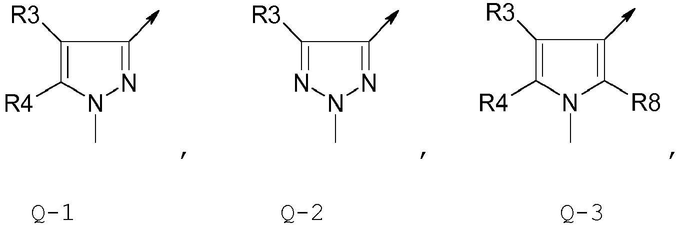 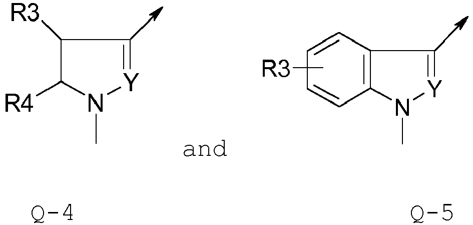 in which the nitrogen is attached to ring A and the arrow in each case represents the bond to D and
D represents the radical of the formula 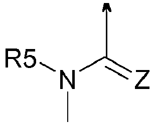 in which the nitrogen is attached to Q and the arrow represents the bond to B,
B represents a radical from the group consisting of 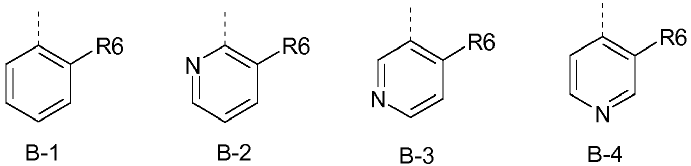 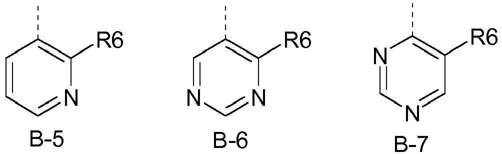 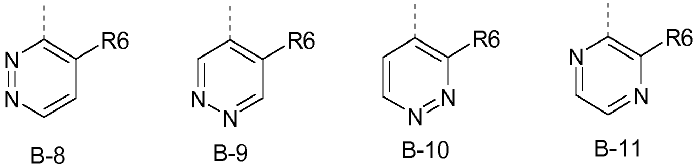 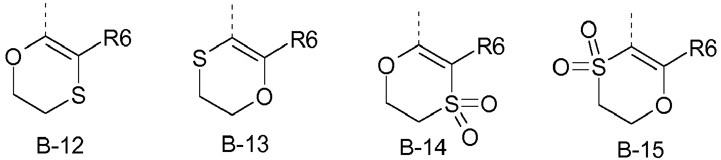 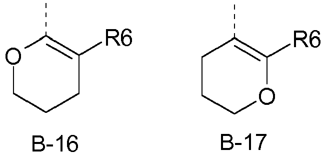 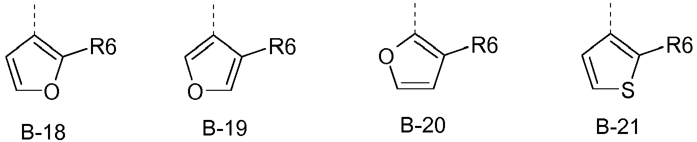 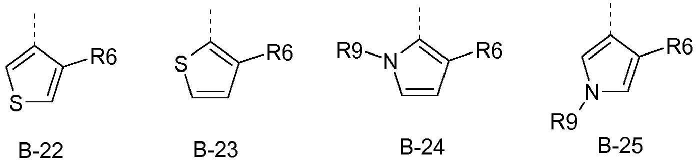 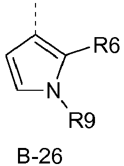 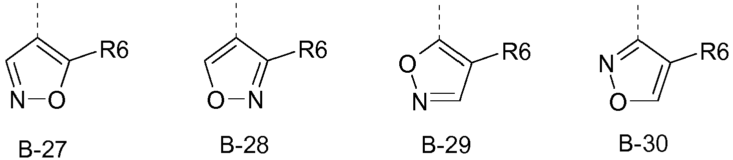 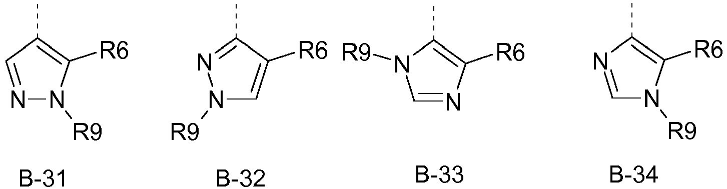 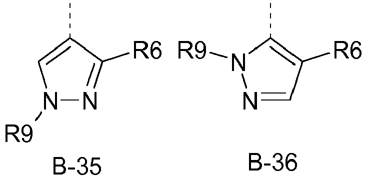 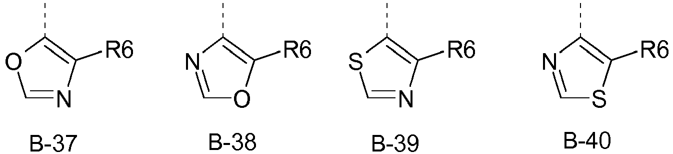 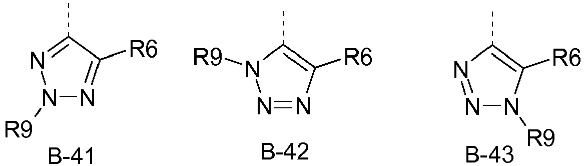 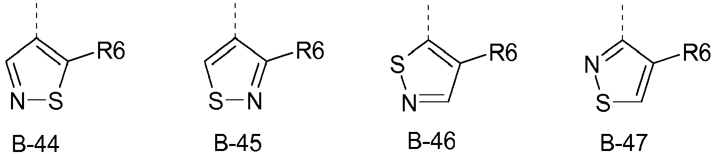 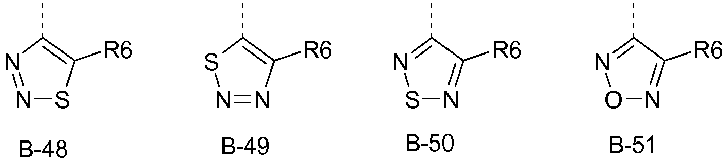 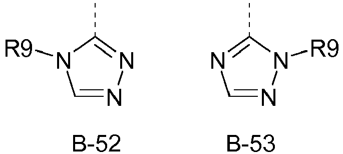 in which the broken line represents the bond to D and in which B furthermore carries n substituents R7,
Y in radical Q-4 represents CR8 or represents nitrogen,
Y in radical Q-5 represents nitrogen,
Z represents oxygen or sulphur,
R1 represents a radical from the group consisting of halogen, cyano, nitro, amino, hydroxy, represents C₁-C₆-alkyl, C₂-C₆-alkenyl, C₂-C₆-alkynyl, C₃-C₆-cycloalkyl, C₁-C₆-alkoxy, C₃-C₆-alkenyloxy, C₃-C₆-alkynyloxy, C₃-C₆-cycloalkyloxy, C₁-C₆-alkyl-carbonyloxy, C₂-C₆-alkenylcarbonyloxy, C₂-C₆-alkynylcarbonyloxy, C₃-C₆-cycloalkylcarbonyloxy, C₁-C₆-alkoxycarbonyloxy, C₁-C₆-alkylsulphonyloxy, C₁-C₆-alkylamino, C₃-C₆-alkenylamino, C₃-C₆-alkynylamino, C₃-C₆-cycloalkylamino, C₁-C₆-alkyl-carbonylamino, C₂-C₆-alkenylcarbonylamino, C₂-C₆-alkynylcarbonylamino, C₃-C₆-cycloalkylcarbonyl-amino, C₁-C₆-alkoxycarbonylamino, C₁-C₆-alkyl-sulphonylamino, C₁-C₆-alkylthio, C₃-C₆-alkenylthio, C₃-C₆-alkynylthio, C₃-C₆-cycloalkylthio, C₁-C₆-alkylsulphinyl, C₁-C₆-alkylsulphonyl, C₁-C₆-alkylcarbonyl, C₁-C₆-alkoxyimino-C₁-C₆-alkyl, C₁-C₆-alkoxycarbonyl, aminocarbonyl, C₁-C₆-alkyl-aminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₆-alkylthiocarbonylamino, C₄-C₁₂-bicycloalkyl, aryl, aryloxy, arylamino, arylthio, heteroaryl, heteroaryloxy, heteroarylamino and heteroarylthio, each of which is optionally substituted by one or more identical or different substituents, where the substituents independently of one another are selected from the group consisting of halogen, cyano, nitro, hydroxy, amino, C₁-C₆-alkyl, C₁-C₆-alkoxy, C₃-C₆-cycloalkyl, C₁-C₆-haloalkoxy, C₁-C₆-alkylthio, aryl, aryloxy, arylthio, heteroaryl, heteroaryloxy and heteroarylthio,
R1 represents a radical from the group consisting of halogen, cyano, nitro, amino, hydroxy, represents C₁-C₆-alkyl, C₂-C₆-alkenyl, C₂-C₆-alkynyl, C₃-C₆-cycloalkyl, C₁-C₆-alkoxy, C₃-C₆-alkenoxy, C₃-C₆-alkynoxy, C₃-C₆-cycloalkoxy, C₁-C₆-alkylcarbonyloxy, C₂-C₆-alkenylcarbonyloxy, C₂-C₆-alkynylcarbonyloxy, C₃-C₆-cycloalkylcarbonyloxy, C₁-C₆-alkoxycarbonyl-oxy, C₁-C₆-alkylsulphonyloxy, C₁-C₆-alkylamino, di-(C₁-C₆-alkyl)-amino, C₁-C₆-alkenylamino, C₃-C₆-alkynylamino, C₃-C₆-cycloalkylamino, C₃-C₆-alkylcarbonylamino, C₁-C₆-alkenylcarbonylamino, C₂-C₆-alkynylcarbonylamino, C₃-C₆-cycloalkylcarbonyl-amino, C₁-C₆-alkoxycarbonylamino, C₁-C₆-alkyl-sulphonylamino, C₁-C₆-alkylthio, C₃-C₆-alkenylthio, C₃-C₆-alkynylthio, C₃-C₆-cycloalkylthio, C₁-C₆-alkylsulphinyl, C₁-C₆-alkylsulphonyl, C₁-C₆-alkylcarbonyl, C₁-C₆-alkoxyimino-C₁-C₆-alkyl, C₁-C₆-alkoxycarbonyl, aminocarbonyl, C₁-C₆-alkyl-aminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₆-alkylthiocarbonylamino, C₄-C₁₂-bicycloalkyl, aryl, aryloxy, arylamino, arylthio, heteroaryl, heteroaryloxy, heteroarylamino and heteroarylthio, each of which is optionally substituted by one or more identical or different substituents, where the substituents independently of one another are selected from the group consisting of halogen, cyano, nitro, hydroxy, amino, C₁-C₆-alkyl, C₁-C₆-alkoxy, C₃-C₆-cycloalkyl, C₁-C₆-haloalkoxy and C₁-C₆-alkylthio, aryl, aryloxy, arylthio, heteroaryl, heteroaryloxy and heteroarylthio,
R3 represents a radical from the group consisting of hydrogen, halogen, cyano, nitro, amino, hydroxy, C₁-C₆-alkyl, C₁-C₆-alkoxy, C₁-C₆-haloalkyl and C₁-C₆-haloalkoxy,
R4 represents a radical from the group consisting of hydrogen, halogen, amino, hydroxy, C₁-C₆-alkyl, C₁-C₆-alkoxy, C₁-C₆-haloalkyl and C₁-C₆-haloalkoxy,
R5 represents a radical from the group consisting of hydrogen, C₁-C₆-alkyl, C₃-C₆-cycloalkyl, C₁-C₆-haloalkyl, C₂-C₆-alkenyl, C₃-C₆-alkynyl, C₃-C₆-cycloalkyl-C₁-C₆-alkyl, cyano-C₁-C₆-alkyl, C₁-C₆-alkylcarbonyl, C₂-C₆-alkenylcarbonyl, C₁-C₆-haloalkylcarbonyl, C₂-C₆-haloalkenylcarbonyl, C₁-C₆-alkoxy-C₁-C₆-alkyl, C₁-C₆-alkoxycarbonyl, C₁-C₆-alkylsulphonyl and C₁-C₆-haloalkylsulphonyl or represents C(=O)-B,
R6 represents a radical from the group consisting of hydrogen (only in the radicals B-26, B-33, B-36 and B-42), halogen, cyano, nitro, amino, hydroxy, carboxyl, represents C₁-C₆-alkyl, C₂-C₆-alkenyl, C₂-C₆-alkynyl, C₃-C₆-cycloalkyl, C₁-C₆-alkoxy, C₃-C₆-alkenoxy, C₃-C₆-alkynoxy, C₃-C₆-cycloalkoxy, C₁-C₆-alkylcarbonyloxy, C₂-C₆-alkenylcarbonyloxy, C₂-C₆-alkynylcarbonyloxy, C₃-C₆-cycloalkylcarbonyloxy, C₁-C₆-alkoxycarbonyloxy, C₁-C₆-alkylsulphonyloxy, C₁-C₆-alkylcarbonylamino, C₂-C₆-alkenylcarbonyl-amino, C₂-C₆-alkynylcarbonylamino, C₃-C₆-cycloalkylcarbonylamino, C₁-C₆-alkoxycarbonylamino, C₁-C₆-alkylsulphonylamino, C₁-C₆-alkylthio, C₁-C₆-alkylsulphinyl, C₁-C₆-alkylsulphonyl, C₁-C₆-alkylcarbonyl, C₁-C₆-alkoxyimino-C₁-C₆-alkyl, C₁-C₆-alkoxycarbonyl, aminocarbonyl, C₁-C₆-alkylaminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₆-haloalkylcarbonylamino, C₁-C₆-alkylthio-carbonylamino, C₄-C₁₂-bicycloalkyl, aryl, aryloxy, heteroaryl, heteroaryloxy, each of which is optionally substituted by one or more identical or different substituents, where the substituents independently of one another are selected from the group consisting of halogen, cyano, nitro, hydroxy, amino, C₁-C₆-alkyl, C₁-C₆-alkoxy, C₃-C₆-cycloalkyl, C₁-C₆-haloalkoxy and C₁-C₆-alkylthio,
R7 represents a radical from the group consisting of halogen, nitro, cyano, amino, hydroxy, represents C₁-C₆-alkyl, C₂-C₆-alkenyl, C₂-C₆-alkynyl, C₃-C₆-cycloalkyl, C₁-C₆-alkoxy, C₃-C₆-alkenoxy, C₃-C₆-alkynoxy, C₃-C₆-cycloalkoxy, C₁-C₆-alkylcarbonyloxy, C₂-C₆-alkenylcarbonyloxy, C₂-C₆-alkynylcarbonyloxy, C₃-C₆-cycloalkylcarbonyloxy, C₁-C₆-alkoxy-carbonyloxy, C₁-C₆-alkylsulphonyloxy, C₁-C₆-alkylcarbonylamino, C₂-C₆-alkenylcarbonylamino, C₂-C₆-alkynylcarbonylamino, C₃-C₆-cycloalkylcarbonyl-amino, C₁-C₆-alkoxycarbonylamino, C₁-C₆-alkyl-sulphonylamino, C₁-C₆-alkylthio, C₁-C₆-alkyl-sulphinyl, C₁-C₆-alkylsulphonyl, C₁-C₆-alkyl-carbonyl, C₁-C₆-alkoxyimino-C₁-C₆-alkyl, C₁-C₆-alkoxycarbonyl, aminocarbonyl, C₁-C₆-alkyl-aminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₆-alkylthiocarbonylamino, aryl, aryloxy, heteroaryl, heteroaryloxy, C₄-C₁₂-bicycloalkyl, each of which is optionally substituted by one or more identical or different substituents, where the substituents independently of one another are selected from the group consisting of halogen, cyano, nitro, hydroxy, amino, C₁-C₆-alkyl, C₁-C₆-alkoxy, C₃-C₆-cycloalkyl, C₁-C₆-haloalkoxy and C₁-C₆-alkylthio,
R8 represents a radical from the group consisting of hydrogen, halogen, amino, hydroxy, C₁-C₆-alkyl, C₁-C₆-alkoxy, C₁-C₆-haloalkyl and C₁-C₆-haloalkoxy,
R9 represents a radical from the group consisting of hydrogen, C₁-C₆-alkyl, C₃-C₆-cycloalkyl, haloalkyl, C₃-C₆-alkenyl, C₂-C₆-alkynyl, C₃-C₆-cycloalkyl-C₁-C₆-alkyl, cyano-C₁-C₆-alkyl, C₁-C₆-alkylcarbonyl, C₂-C₆-alkenylcarbonyl, C₁-C₆-haloalkylcarbonyl, C₂-C₆-haloalkenylcarbonyl, C₁-C₆-alkoxy-C₁-C₆-alkyl, C₁-C₆-alkoxycarbonyl, C₁-C₆-alkylsulphonyl and C₁-C₆-haloalkylsulphonyl,
m represents a number from the group consisting of 0, 1, 2 and 3, where for m > 1 the radicals R2 may be identical or different and
n represents a number from the group consisting of 0, 1, 2 and 3, where for n > 1 the radicals R7 may be identical or different,
for controlling animal pests.

2. Non-therapeutic use of compounds of the formula (I) according to Claim 1 in which
A represents a radical from the group consisting of 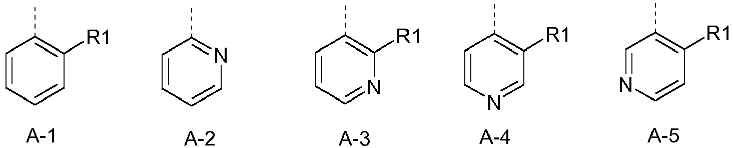 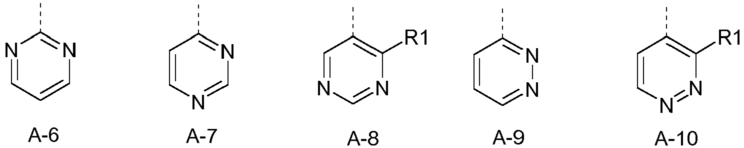 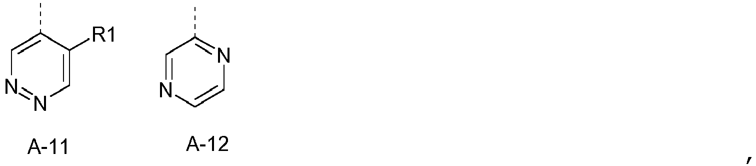 in which the broken line represents the bond to Q and where A furthermore carries m substituents R2,
Q represents a radical from the group consisting of 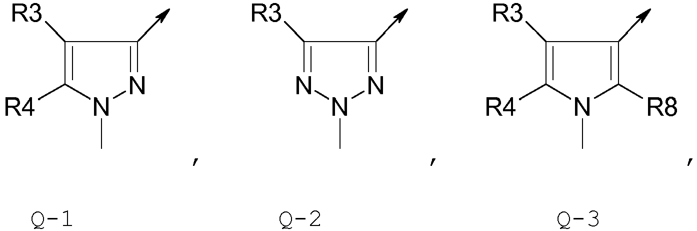 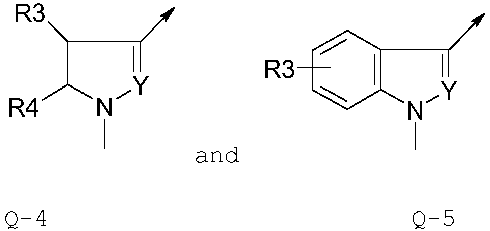 in which the nitrogen is attached to ring A and the arrow in each case represents the bond to D,
D represents the radical of the formula 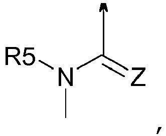 in which the nitrogen is attached to Q and the arrow represents the bond to B,
B represents a radical from the group consisting of 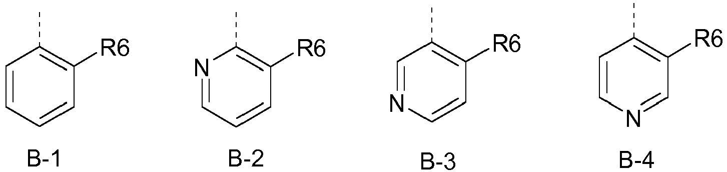 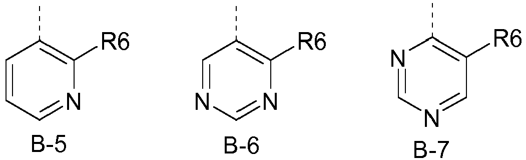 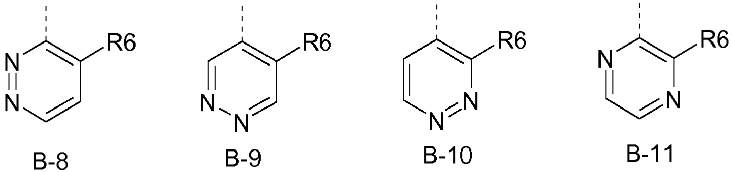 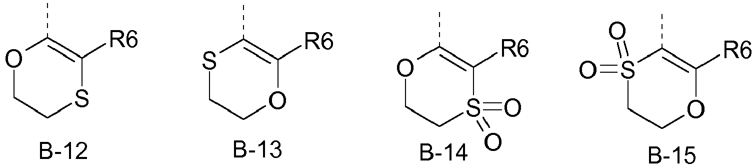 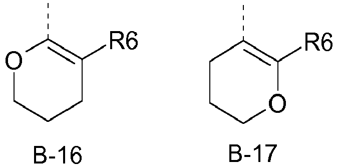 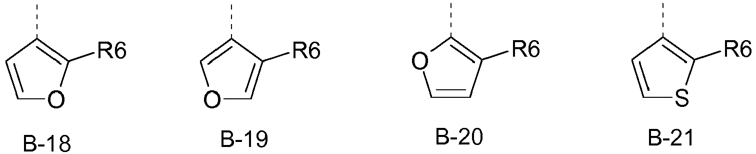 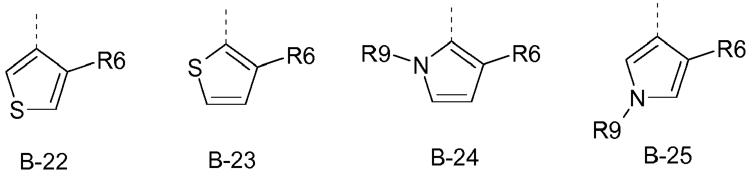 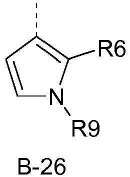 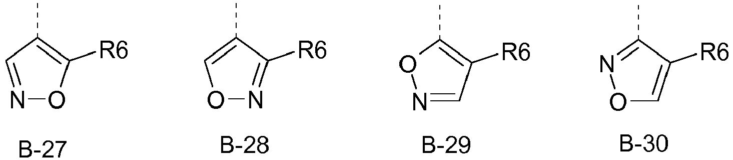 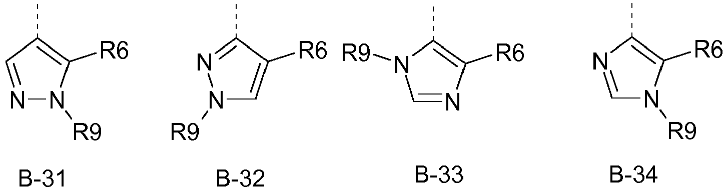 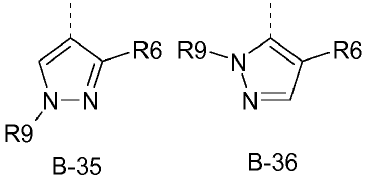 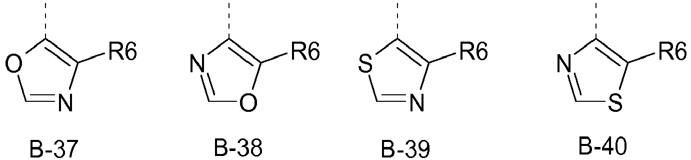 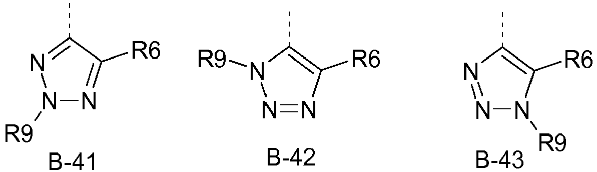 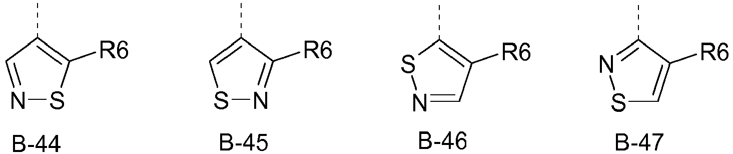 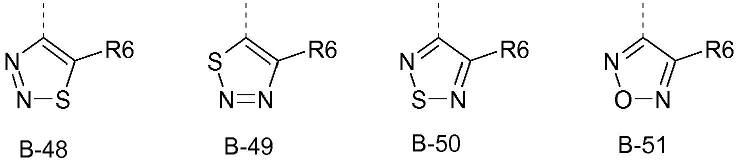 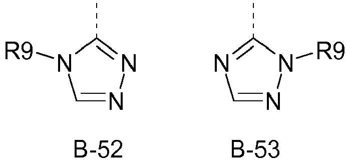 in which the broken line represents the bond to D and in which B furthermore carries n substituents R7,
Y in radical Q-4 represents CR8 or represents nitrogen,
Y in radical Q-5 represents nitrogen,
Z represents oxygen or sulphur,
R1 represents a radical from the group consisting of halogen, cyano, nitro, amino, hydroxy, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-cycloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylcarbonyloxy, C₂-C₆-alkenylcarbonyloxy, C₂-C₆-alkynylcarbonyloxy, C₃-C₆-cycloalkyl-carbonyloxy, C₁-C₆-alkoxycarbonyloxy, C₁-C₆-alkylsulphonyloxy, C₁-C₆-alkylamino, C₃-C₆-alkenylamino, C₃-C₆-alkynylamino, C₃-C₆-cycloalkylamino, C₁-C₆-alkylthio, C₁-C₆-haloalkylthio, C₃-C₆-alkenylthio, C₃-C₆-alkynylthio, C₃-C₆-cycloalkylthio, C₁-C₆-alkylsulphinyl, C₁-C₆-alkylsulphonyl, C₁-C₆-alkylcarbonyl, C₁-C₆-alkoxyimino-C₁-C₆-alkyl, C₁-C₆-alkoxycarbonyl, aminocarbonyl, C₁-C₆-alkylaminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₆-alkylthiocarbonyl-amino, aryl, aryloxy, heteroaryl and heteroaryloxy,
R2 represents a radical from the group consisting of halogen, cyano, nitro, amino, hydroxy, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-cycloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylcarbonyloxy, C₂-C₆-alkenylcarbonyloxy, C₂-C₆-alkynylcarbonyloxy, C₃-C₆-cycloalkyl-carbonyloxy, C₁-C₆-alkoxycarbonyloxy, C₁-C₆-alkylsulphonyloxy, C₁-C₆-alkylamino, di-(C₁-C₆-alkyl)-amino, C₃-C₆-alkenylamino, C₃-C₆-alkynylamino, C₃-C₆-cycloalkylamino, C₁-C₆-alkylthio, C₁-C₆-haloalkylthio, C₃-C₆-alkenylthio, C₃-C₆-alkynylthio, C₃-C₆-cycloalkylthio, C₁-C₆-alkylsulphinyl, C₁-C₆-alkylsulphonyl, C₁-C₆-alkylcarbonyl, C₁-C₆-alkoxyimino-C₁-C₆-alkyl, C₁-C₆-alkoxycarbonyl, aminocarbonyl, C₁-C₆-alkylaminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₆-alkylthiocarbonylamino, aryl, aryloxy, heteroaryl and heteroaryloxy,
R3 represents a radical from the group consisting of hydrogen, halogen, cyano, nitro, amino, C₁-C₆-alkyl and C₁-C₆-haloalkyl,
R4 represents a radical from the group consisting of hydrogen, halogen, amino, hydroxy, C₁-C₆-alkyl, C₁-C₆-alkoxy, C₁-C₆-haloalkyl and C₁-C₆-haloalkoxy,
R5 represents a radical from the group consisting of hydrogen, C₁-C₆-alkyl, C₁-C₆-haloalkyl, cyano-C₁-C₆-alkyl, C₂-C₆-alkenyl, C₃-C₆-alkynyl, C₃-C₆-cycloalkyl, C₃-C₆-cycloalkyl-C₁-C₆-alkyl, C₁-C₆-alkylcarbonyl, C₂-C₆-alkenylcarbonyl, C₁-C₆-haloalkylcarbonyl, C₂-C₆-haloalkenylcarbonyl, C₁-C₆-alkoxy-C₁-C₆-alkyl, C₁-C₆-alkoxycarbonyl, C₁-C₆-alkylsulphonyl, C₁-C₆-haloalkylsulphonyl and C(=O)-B,
R6 represents a radical from the group consisting of hydrogen (only in the radicals B-26, B-33, B-36 and B-42), halogen, cyano, nitro, amino, hydroxy, carboxyl, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-CyCloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylcarbonyloxy, C₂-C₆-alkenylcarbonyloxy, C₂-C₆-alkynylcarbonyloxy, C₃-C₆-cycloalkylcarbonyloxy, C₁-C₆-alkoxycarbonyloxy, C₁-C₆-alkylsulphonyloxy, C₁-C₆-alkylamino, C₃-C₆-alkenylamino, C₃-C₆-alkynylamino, C₃-C₆-cycloalkylamino, C₁-C₆-alkylthio, C₁-C₆-haloalkylthio, C₃-C₆-alkenylthio, C₃-C₆-alkynylthio, C₃-C₆-cycloalkylthio, C₁-C₆-alkylsulphinyl, C₁-C₆-alkylsulphonyl, C₁-C₆-alkylcarbonyl, C₁-C₆-alkoxyimino-C₁-C₆-alkyl, C₁-C₆-alkoxycarbonyl, aminocarbonyl, C₁-C₆-alkylaminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₄-haloalkylcarbonylamino, C₁-C₆-alkylthiocarbonylamino, aryl, aryloxy, heteroaryl and heteroaryloxy,
R7 represents a radical from the group consisting of halogen, cyano, nitro, amino, hydroxy, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-cycloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylcarbonyloxy, C₂-C₆-alkenylcarbonyloxy, C₂-C₆-alkynylcarbonyloxy, C₃-C₆-cycloalkyl-carbonyloxy, C₁-C₆-alkoxycarbonyloxy, C₁-C₆-alkylsulphonyloxy, C₁-C₆-alkylamino, C₃-C₆-alkenylamino, C₃-C₆-alkynylamino, C₃-C₆-cycloalkylamino, C₁-C₆-alkylthio, C₁-C₆-haloalkylthio, C₃-C₆-alkenylthio, C₃-C₆-alkynylthio, C₃-C₆-cycloalkylthio, C₁-C₆-alkylsulphinyl, C₁-C₆-alkylsulphonyl, C₁-C₆-alkylcarbonyl, C₁-C₆-alkoxyimino-C₁-C₆-alkyl, C₁-C₆-alkoxycarbonyl, aminocarbonyl, C₁-C₆-alkylaminocarbonyl, di-(C₁-C₆-alkyl)-aminocarbonyl, aminothiocarbonyl, C₁-C₆-alkylaminosulphonyl, C₁-C₆-alkylsulphonylamino, C₁-C₆-alkylcarbonylamino, C₁-C₆-alkylthiocarbonyl-amino, aryl, aryloxy, heteroaryl and heteroaryloxy,
R8 represents a radical from the group consisting of hydrogen, halogen, amino, hydroxy, C₁-C₆-alkyl, C₁-C₆-alkoxy, C₁-C₆-haloalkyl and C₁-C₆-haloalkoxy,
R9 represents a radical from the group consisting of hydrogen, C₁-C₆-alkyl, C₃-C₆-cycloalkyl, C₁-C₆-haloalkyl, C₃-C₆-alkenyl, C₂-C₆-alkynyl, C₃-C₆-cycloalkyl-C₁-C₆-alkyl, cyano-C₁-C₆-alkyl, C₁-C₆-alkylcarbonyl, C₂-C₆-alkenylcarbonyl, C₁-C₆-haloalkylcarbonyl, C₂-C₆-haloalkenylcarbonyl, C₁-C₆-alkoxy-C₁-C₆-alkyl, C₁-C₆-alkoxycarbonyl, C₁-C₆-alkylsulphonyl and C₁-C₆-haloalkylsulphonyl,
m represents a number from the group consisting of 0, 1, 2 and 3, where for m > 1 the radicals R2 may be identical or different and
n represents a number from the group consisting of 0, 1, 2 and 3, where for n > 1 the radicals R7 may be identical or different.

3. Non-therapeutic use of compounds of the formula (I) according to Claim 1 in which
A represents a radical from the group consisting of 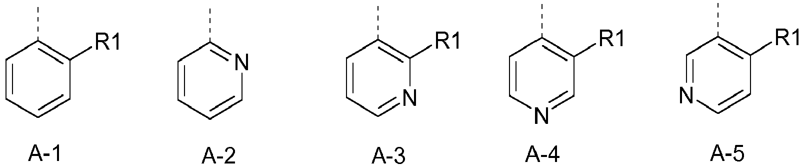 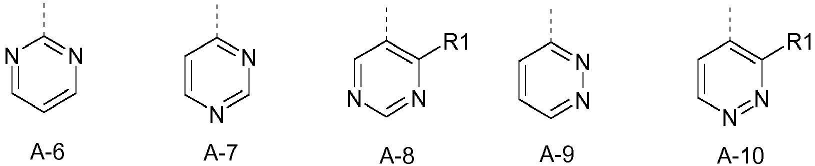 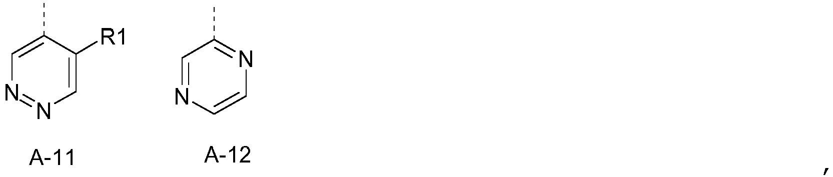 in which the broken line represents the bond to Q and where A furthermore carries m substituents R2,
Q represents a radical from the group consisting of 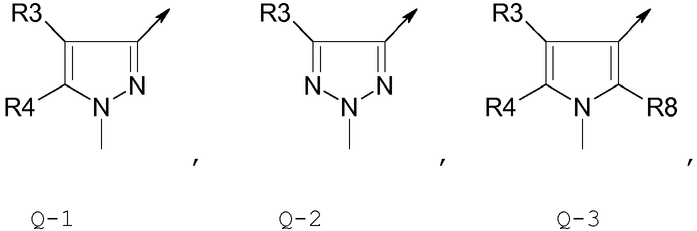 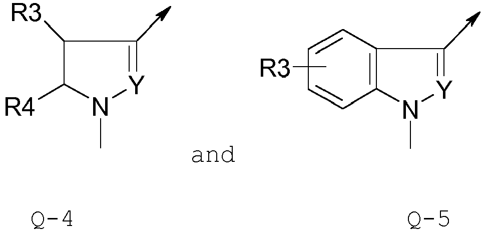 in which the nitrogen is attached to ring A and the arrow in each case represents the bond to D,
D represents the radical of the formula 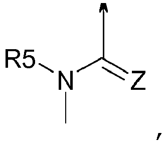 in which the nitrogen is attached to Q and the arrow represents the bond to B,
B represents a radical from the group consisting of 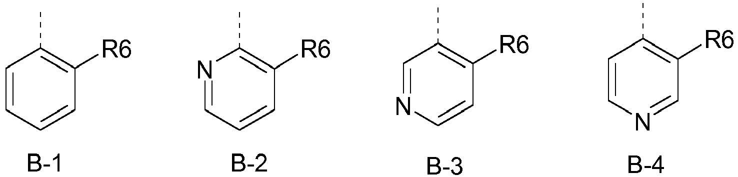 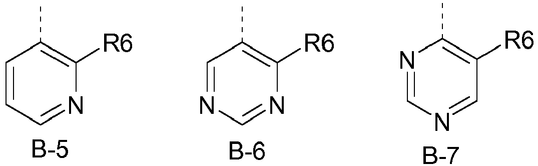 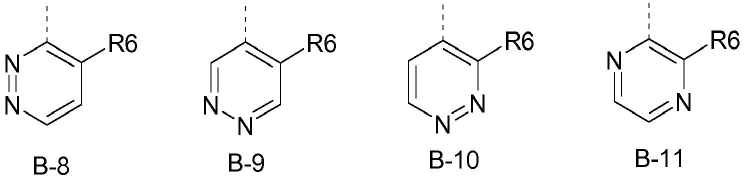 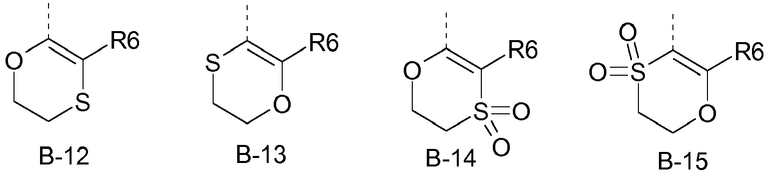 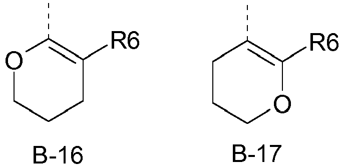 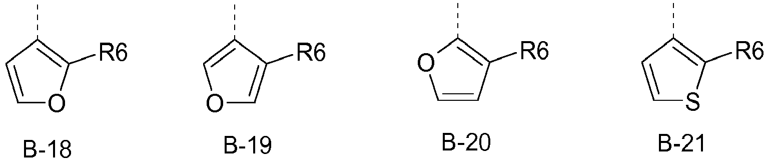 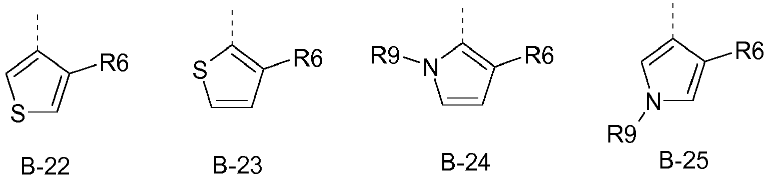 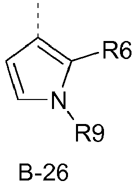 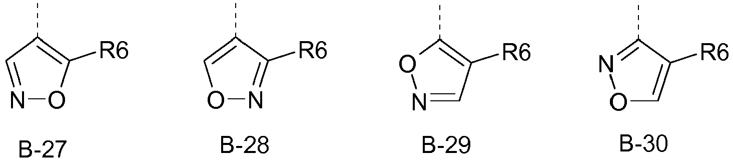 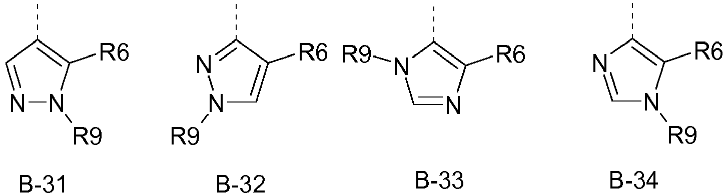 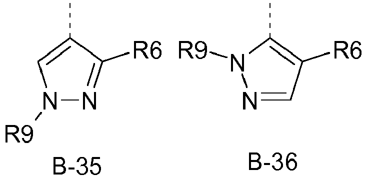 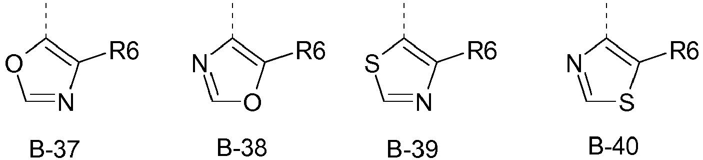 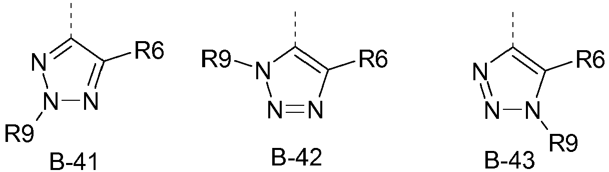 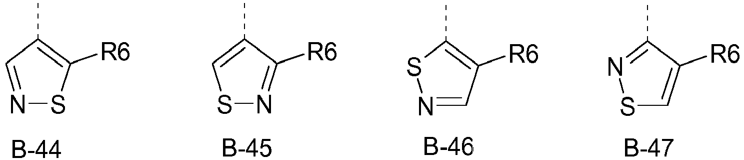 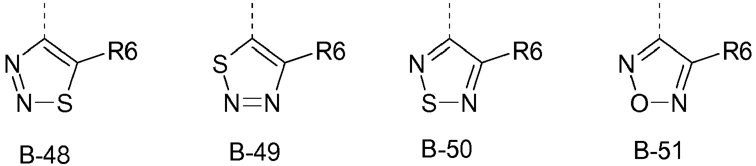 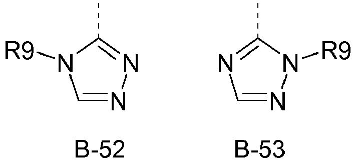 in which the broken line represents the bond to D and in which B furthermore carries n substituents R7,
Y in radical Q-4 represents CR8 or represents nitrogen,
Y in radical Q-5 represents nitrogen,
Z represents oxygen or sulphur,
R1 represents a radical from the group consisting of halogen, cyano, nitro, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-cycloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylthio, C₁-C₆-haloalkylthio, C₁-C₆-alkylsulphinyl, C₁-C₆-alkylsulphonyl, C₁-C₆-alkoxyimino-C₁-C₆-alkyl, aryl, aryloxy, heteroaryl and heteroaryloxy,
R2 represents a radical from the group consisting of halogen, cyano, nitro, amino, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-cycloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylthio, C₁-C₆-haloalkylthio, C₁-C₆-alkylsulphinyl, C₁-C₆-alkylsulphonyl, C₁-C₆-alkylcarbonyl, di-(C₁-C₆-alkyl)-amino, C₁-C₆-alkoxyimino-C₁-C₆-alkyl, aryl, aryloxy, heteroaryl and heteroaryloxy,
R3 represents a radical from the group consisting of hydrogen, halogen, cyano, nitro, amino, C₁-C₆-alkyl and C₁-C₆-haloalkyl,
R4 represents a radical from the group consisting of hydrogen, halogen, amino, hydroxy, C₁-C₆-alkyl, C₁-C₆-alkoxy, C₁-C₆-haloalkyl and C₁-C₆-haloalkoxy,
R5 represents a radical from the group consisting of hydrogen, C₁-C₆-alkyl, C₁-C₆-haloalkyl, cyano-C₁-C₆-alkyl, C₂-C₆-alkenyl, C₃-C₆-alkynyl, C₃-C₆-cycloalkyl, C₃-C₆-cycloalkyl-C₁-C₆-alkyl, C₁-C₆-alkylcarbonyl, C₂-C₆-alkenylcarbonyl, C₁-C₆-haloalkylcarbonyl, C₂-C₆-haloalkenylcarbonyl, C₁-C₆-alkoxy-C₁-C₆-alkyl, C₁-C₆-alkoxycarbonyl, C₁-C₆-alkylsulphonyl, C₁-C₆-haloalkylsulphonyl and C(=O)-B,
R6 represents a radical from the group consisting of hydrogen (only in the radicals B-26, B-33, B-36 and B-42), halogen, cyano, nitro, hydroxy, carboxyl, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-cycloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylsulphinyl, C₁-C₆-alkylthio,. C₁-C₆-haloalkylthio, C₁-C₆-alkylsulphonyl, C₁-C₆-alkylcarbonyl, C₁-C₆-alkoxyimino-C₁-C₆-alkyl, C₁-C₆-alkylcarbonylamino, C₁-C₆-haloalkylcarbonylamino, aryl, aryloxy, heteroaryl and heteroaryloxy,
R7 represents a radical from the group consisting of halogen, cyano, nitro, hydroxy, C₁-C₆-alkyl, C₁-C₆-haloalkyl, cyano-C₁-C₆-alkyl, C₂-C₆-alkenyl, C₂-C₆-alkynyl, C₃-C₆-cycloalkyl, C₃-C₆-cycloalkyl-C₁-C₆-alkyl, C₁-C₆-alkoxy, C₁-C₆-haloalkoxy, C₃-C₆-alkenyloxy, C₃-C₆-alkynyloxy, C₃-C₆-cycloalkyloxy, C₁-C₆-alkylthio, C₁-C₆-haloalkylthio, C₁-C₆-alkylsulphinyl, C₁-C₆-alkylsulphonyl, C₁-C₆-alkoxyimino-C₁-C₆-alkyl, aryl, aryloxy, heteroaryl and heteroaryloxy,
R8 represents a radical from the group consisting of hydrogen, C₁-C₆-alkyl, C₁-C₆-alkoxy, C₁-C₆-haloalkyl and C₁-C₆-haloalkoxy,
R9 represents a radical from the group consisting of hydrogen, C₁-C₆-alkyl, C₃-C₆-cycloalkyl and C₁-C₆-haloalkyl,
m represents a number from the group consisting of 0, 1, 2 and 3, where for m > 1 the radicals R2 may be identical or different and
n represents a number from the group consisting of 0, 1, 2 and 3, where for n > 1 the radicals R7 may be identical or different.

4. Non-therapeutic use of compounds of the formula (I) according to Claim 1 in which
A represents a radical from the group consisting of 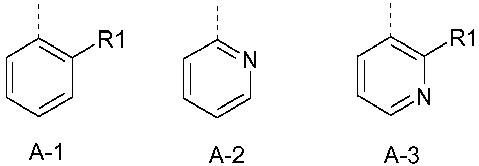 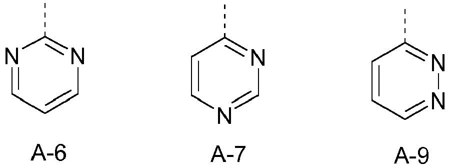 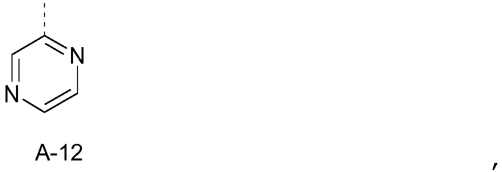 in which the broken line represents the bond to Q and where A furthermore carries m substituents R2,
Q represents a radical from the group consisting of 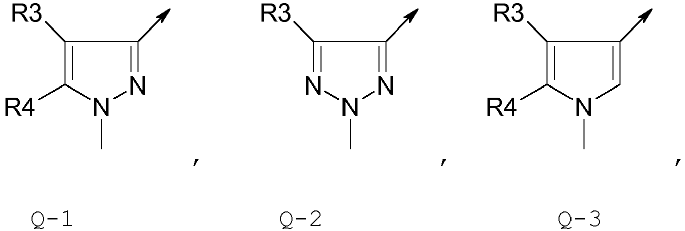 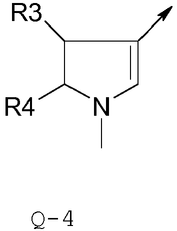 and , in which the nitrogen is attached to ring A and the arrow in each case represents the bond to D,
D represents the radical of the formula 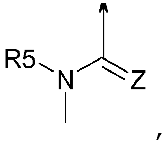 in which the nitrogen is attached to Q and the arrow represents the bond to B,
B represents a radical from the group consisting of 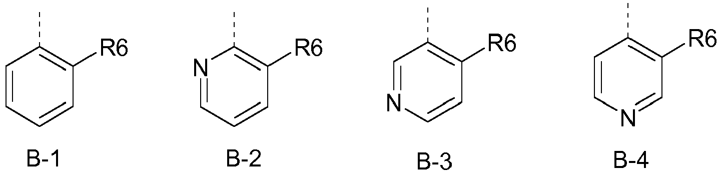 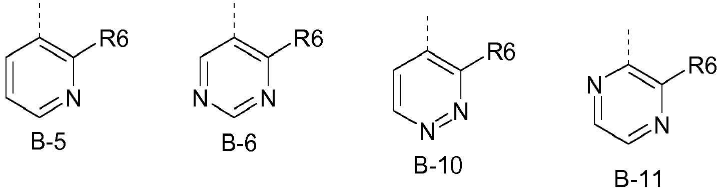 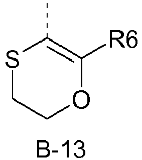 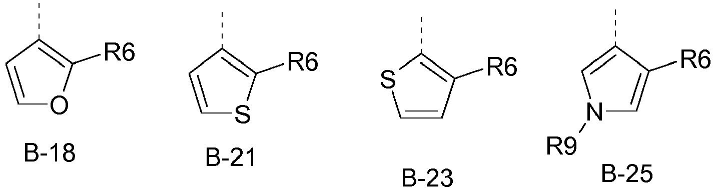 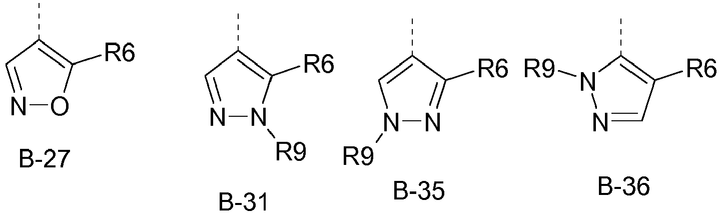 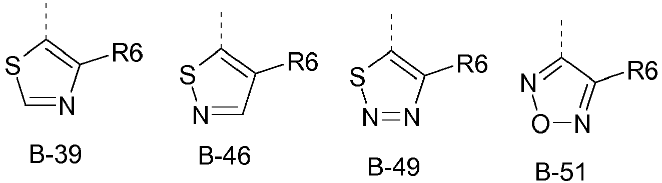 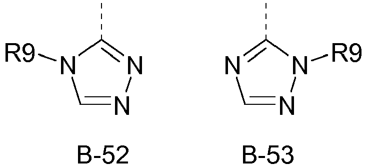 in which the broken line represents the bond to D and in which B furthermore carries n substituents R7,
Z represents oxygen or sulphur,
R1 represents a radical from the group consisting of halogen, cyano, nitro, C₁-C₄-alkyl, C₁-C₄-haloalkyl, C₁-C₄-alkoxy and C₁-C₄-alkylsulphonyl,
R2 represents a radical from the group consisting of halogen, C₁-C₄-alkyl and C₁-C₄-alkoxy,
R3 represents a radical from the group consisting of hydrogen and halogen,
R4 represents a radical from the group consisting of hydrogen and C₁-C₄-alkyl,
R5 represents a radical from the group consisting of hydrogen, C₁-C₄-alkyl, C₃-C₄-alkenyl, C₃-C₄-alkynyl, C₃-C₆-cycloalkyl-C₁-C₄-alkyl, C₁-C₄-alkylcarbonyl, cyano-C₁-C₄-alkyl and C(=O)-B,
R6 represents a radical from the group consisting of halogen, nitro, hydroxy, C₁-C₄-alkyl, C₁-C₄-alkoxy, C₁-C₄-alkylthio, C₁-C₄-alkylsulphonyl, C₁-C₄-haloalkyl, C₁-C₄-haloalkoxy, C₁-C₄-haloalkylthio and heteroaryl,
R7 represents a radical from the group consisting of halogen, C₁-C₄-alkyl and C₁-C₄-haloalkyl,
R9 represents a radical from the group consisting of C₁-C₄-alkyl,
m represents a number from the group consisting of 0, 1, 2 and 3, where for m > 1 the radicals R2 may be identical or different and
n represents a number from the group consisting of 0 and 1.

5. Non-therapeutic use of compounds of the formula (I) according to Claim 1 in which
A represents a radical from the group consisting of 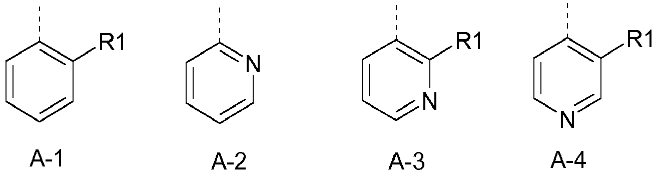 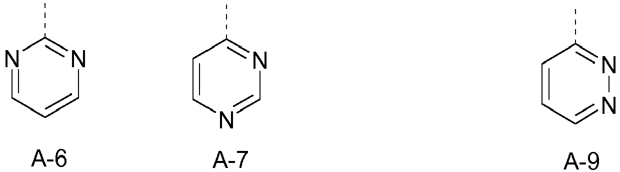 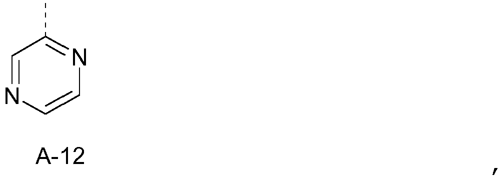 in which the broken line represents the bond to Q and where A furthermore carries m substituents R2,
Q represents a radical from the group consisting of 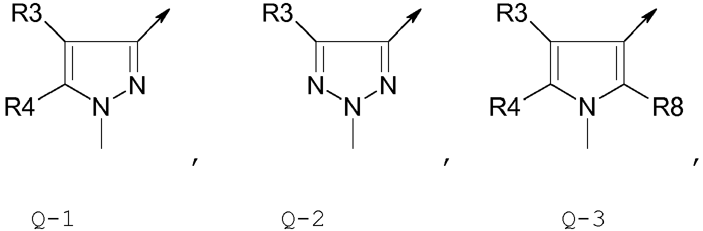 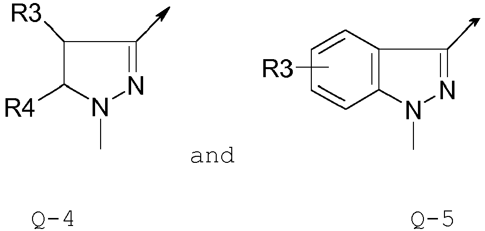 in which the nitrogen is attached to ring A and the arrow in each case represents the bond to D,
D represents the radical of the formula 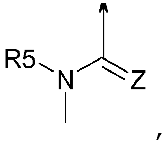 in which the nitrogen is attached to Q and the arrow represents the bond to B,
B represents a radical from the group consisting of 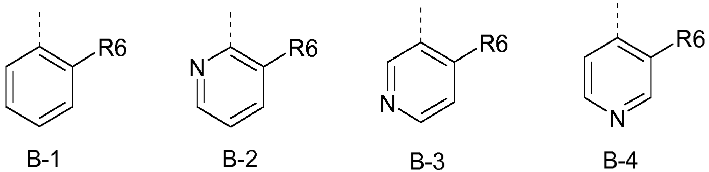 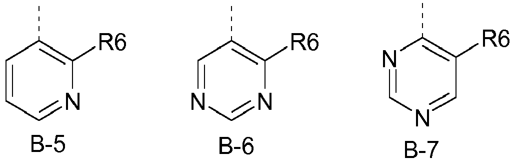 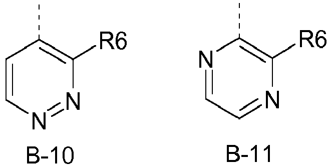 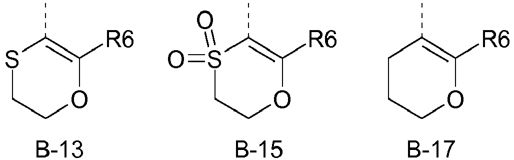 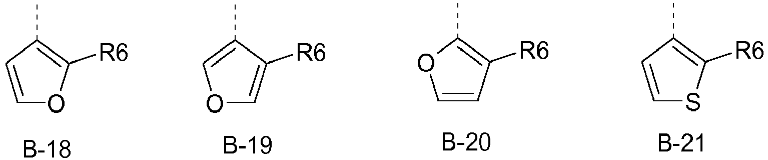 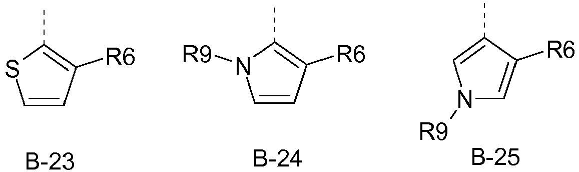 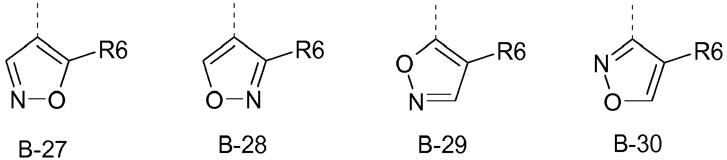 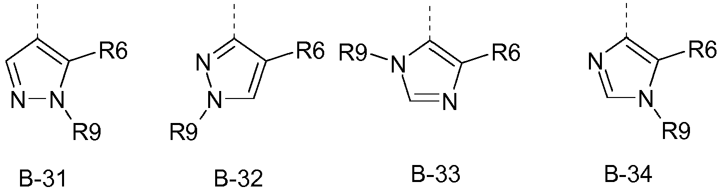 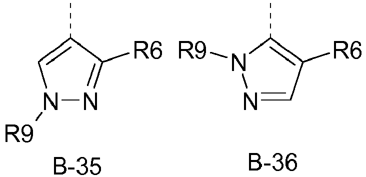 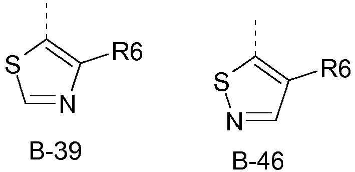 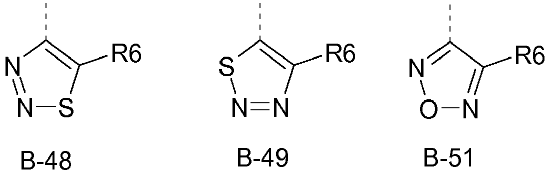 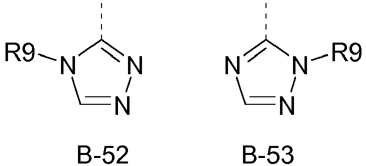 in which the broken line represents the bond to D and in which B furthermore carries n substituents R7,
Z represents oxygen or sulphur,
R1 represents a radical from the group consisting of halogen, cyano, nitro, C₁-C₄-alkyl, C₁-C₄-haloalkyl, C₁-C₄-alkoxy and C₁-C₄-alkylsulphonyl,
R2 represents a radical from the group consisting of halogen, cyano, nitro, amino, C₁-C₄-alkyl, C₁-C₄-haloalkyl, C₃-C₆-cycloalkyl, C₁-C₄-alkoxy, di-(C₁-C₆-alkyl)-amino, C₁-C₄-alkylcarbonyl, aryl,
R3 represents a radical from the group consisting of hydrogen, halogen, cyano, nitro, C₁-C₄-alkyl,
R4 represents a radical from the group consisting of hydrogen, amino, C₁-C₄-alkyl,
R5 represents a radical from the group consisting of hydrogen, C₁-C₄-alkyl, C₃-C₄-alkenyl, C₃-C₄-alkynyl, C₃-C₆-cycloalkyl-C₁-C₄-alkyl, C₁-C₄-alkylcarbonyl, cyano-C₁-C₄-alkyl and C(=O)-B,
R6 represents a radical from the group consisting of hydrogen (only in the radicals B-33 and B-36), halogen, cyano, nitro, hydroxy, carboxyl, C₁-C₄-alkyl, C₁-C₄-alkoxy, C₁-C₄-alkylthio, C₁-C₄-alkylsulphonyl, C₁-C₄-haloalkyl, C₃-C₆-cycloalkyl, C₁-C₄-haloalkoxy, C₁-C₄-haloalkylthio, C₁-C₄-alkylcarbonyl, C₁-C₄-alkylcarbonylamino, C₁-C₄-haloalkylcarbonylamino and heteroaryl,
R7 represents a radical from the group consisting of halogen, cyano, nitro, hydroxy, C₁-C₄-alkyl, C₁-C₄-haloalkyl and C₁-C₄-alkoxy,
R9 represents a radical from the group consisting of C₁-C₄-alkyl and C₁-C₄-haloalkyl,
m represents a number from the group consisting of 0, 1, 2 and 3, where for m > 1 the radicals R2 may be identical or different and
n represents a number from the group consisting of 0, 1, 2 and 3, where for n > 1 the radicals R7 may be identical or different.

6. Compounds of the formula (I) as described in any of Claims 1 to 5, except for the following compounds 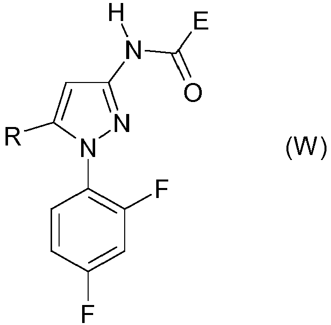
| No. | R | E |
|---|---|---|
| W-1 | ethyl | 2-(trifluoromethyl)phenyl |
| W-2 | ethyl | 2-bromophenyl |
| W-3 | ethyl | 2-fluorophenyl |
| W-4 | ethyl | 3-methyl-2-thienyl |
| W-5 | ethyl | 2,5-dimethyl-3-furanyl |
| W-6 | H | 2,4-dimethoxyphenyl |
| W-7 | H | 2-(trifluoromethyl)phenyl |
| W-8 | H | 2,4-difluorophenyl |
| W-9 | methyl | 2-bromophenyl |
| W-10 | ethyl | 2,4-difluorophenyl |
| W-11 | H | 2,5-dimethyl-3-furanyl |
| W-12 | methyl | 2,4-dimethoxyphenyl |

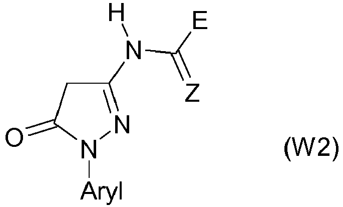
| No. | Z | E | aryl |
|---|---|---|---|
| W2-1 | O | 2-nitrophenyl | 3,4-dichloro-2,5-dimethoxyphenyl |
| W2-2 | S | 2,4-dihydroxyphenyl | 2,4,6-trichlorophenyl |
| W2-3 | O | 2-chloro-4-aminophenyl | 2,4,6-trichlorophenyl |
| W2-5 | O | 2-chloro-5-aminophenyl | 2,4,6-trichlorophenyl |
| W2-6 | O | 2-chloro-5-nitrophenyl | 2,4,6-trichlorophenyl |
| W2-7 | O | 2-chloro-3-(1-oxo-2-propen-1-yl)amino | 2,4,6-trichlorophenyl |
| W2-8 | O | 2-chloro-5-(2-methyl-1-oxo-2-propenyl)amino | 2,4,6-trichlorophenyl |
| W2-9 | O | 2-chloro-5-(1-oxo-2-propen-1-yl)amino | 2,4,6-trichlorophenyl |

7. Compound of the formula (I) as described in Claim 1, selected from the list below:
| **No**. | **A** | **Q** | **B** | **R3** | **R4** | **R5** | **Z** |
|---|---|---|---|---|---|---|---|
| 1 | 2-(trifluoromethyl)-phenyl | Q-1 | 2-(trifluoromethyl)-phenyl | H | H | H | O |
| 2 | 2-chlorophenyl | Q-1 | 2-(trifluoromethyl)-phenyl | H | H | H | O |
| 3 | 2-bromophenyl | Q-1 | 2-(trifluoromethyl)-phenyl | H | H | H | O |
| 4 | 2-bromophenyl | Q-1 | 2-iodophenyl | H | H | H | O |
| 5 | 2,5-difluorophenyl | Q-1 | 2-(trifluoromethyl)-phenyl | H | H | H | O |
| 6 | 2,6-difluorophenyl | Q-1 | 2-methylphenyl | H | H | H | O |
| 7 | 2,6-difluorophenyl | Q-1 | 2-difluoromethylphenyl | H | H | H | O |
| 8 | 2,6-difluorophenyl | Q-1 | 2-(trifluoromethyl)-phenyl | H | H | H | O |
| 9 | 2,6-difluorophenyl | Q-1 | 2-(trifluoromethyl)-phenyl | F | H | H | O |
| 10 | 2,6-difluorophenyl | Q-1 | 2-chlorophenyl | H | H | H | O |
| 11 | 2,6-difluorophenyl | Q-1 | 2-bromophenyl | H | H | H | O |
| 12 | 2,6-difluorophenyl | Q-1 | 2-iodophenyl | H | H | H | O |
| 13 | 2,6-difluorophenyl | Q-1 | 2,6-difluorophenyl | H | H | H | O |
| 14 | 2-ethoxy-6-fluorophenyl | Q-1 | 2-chlorophenyl | H | H | H | O |
| 15 | 3,5-difluoro-pyridin-2-yl | Q-1 | 2-(trifluoromethyl)-phenyl | H | H | H | O |
| 16 | 3-chloro-5-fluoro-pyridin-2-yl | Q-1 | 2-(trifluoromethyl)-phenyl | H | H | H | O |
| 17 | 2,6-difluorophenyl | Q-1 | 2-fluoro-6-(trifluoromethyl)phenyl | H | H | H | O |
| 18 | 2,6-difluorophenyl | Q-1 | 4-fluoro-2-(trifluoromethyl)phenyl | H | H | H | O |
| 19 | 2-bromophenyl | Q-1 | 2-fluoro-6-(trifluoromethyl)phenyl | H | H | H | O |
| 20 | 2-cyano-4,6-difluorophenyl | Q-1 | 2-(trifluoromethyl)-phenyl | H | H | H | O |
| 21 | 2-cyanophenyl | Q-1 | 2-iodophenyl | H | H | H | O |
| 22 | 3,5-difluoro-pyridin-2-yl | Q-1 | 2-(trifluoromethyl)-phenyl | F | H | H | O |
| 23 | 3-cyanopyridin-2-yl | Q-1 | 2-(trifluoromethyl)-phenyl | H | H | H | O |
| 24 | 2-cyano-4-fluorophenyl | Q-1 | 2-(trifluoromethyl)-phenyl | H | H | H | O |
| 25 | 2,6-difluorophenyl | Q-2 | 2-(trifluoromethyl)-phenyl | H | - | H | O |

8. Compositions, **characterized in that** they comprise at least one compound of the formula (I) according to Claim 6 or 7.

9. Non-therapeutic method for controlling pests, **characterized in that** a compound of the formula (I) as described in Claims 1 to 5 or according to Claim 6 or 7 or a composition according to Claim 8 is allowed to act on the pests and/or their habitat.

10. Process according to Claim 9, **characterized in that** the pests are insects, arachnids and nematodes.

11. Compounds of the formula (I) as described in Claims 1 to 5 or according to Claim 6 or 7 for use as antiendoparasitic.

12. Compounds of the formula (I) as described in Claims 1 to 5 or according to Claim 6 or 7 for use as antiectoparasitic.

13. 2-(3,5-Difluoropyridin-2-yl)-2H-1,2,3-triazole-4-amine.

14. 1-(3,5-Difluoropyridin-2-yl)-1H-pyrazole-3-amine.

15. N-[1-(2,6-Difluorophenyl)-4,5-dihydro-1H-pyrazol-3-yl]acetamide.

16. N-[1-(2,6-Difluorophenyl)-1H-pyrazol-3-yl]-acetamide.

17. 1-(2,6-Difluorophenyl)-1H-pyrazole-3-amine.

18. 1-(2,6-Difluorophenyl)-4,5-dihydro-1H-pyrazole-3-amine.

## Revendications

1. Utilisation non thérapeutique de composés de formule (I) 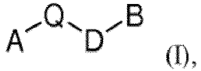 dans laquelle
A représente un radical de la série 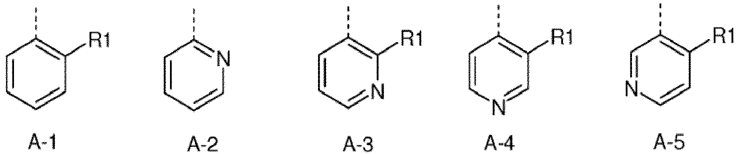 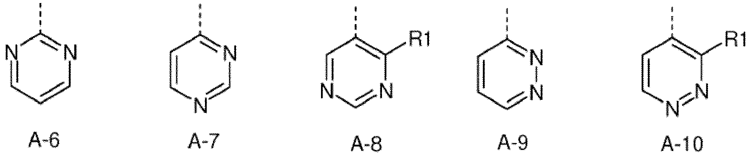 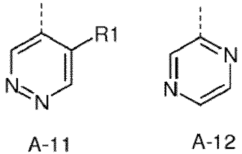 la ligne en pointillés signifiant la liaison à Q et A portant en outre m substituants R2,
Q représente un radical de la série 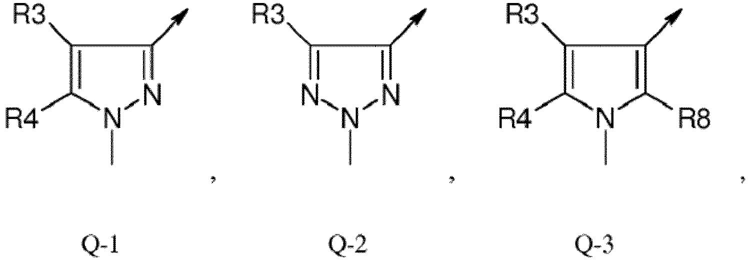 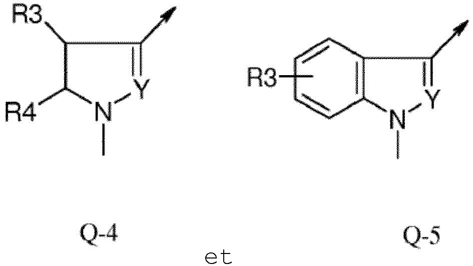 l'azote étant relié avec le cycle A et la flèche signifiant à chaque fois la liaison à D, et
D représente un radical de formule 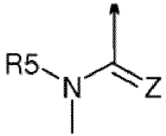 dans laquelle l'azote est relié avec Q et la flèche signifie la liaison à B,
B représente un radical de la série 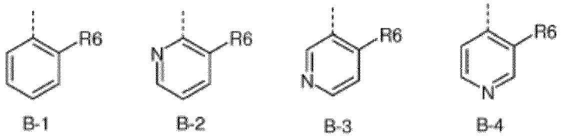 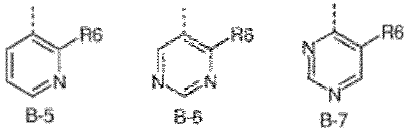 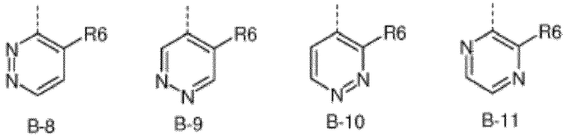 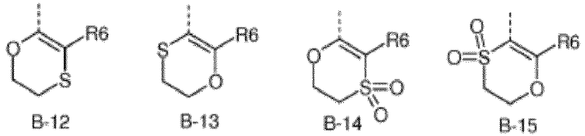 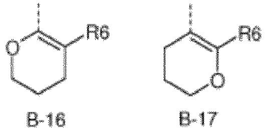 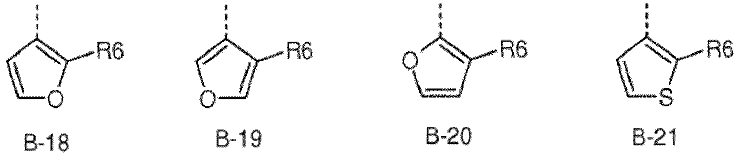 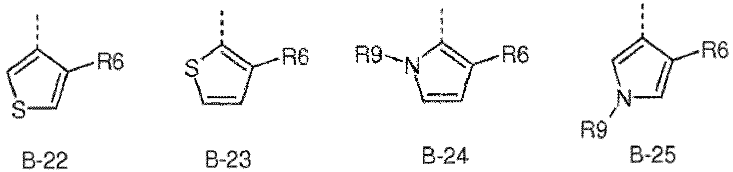 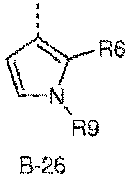 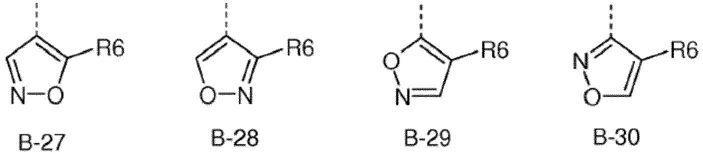 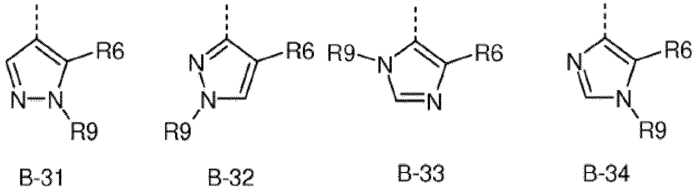 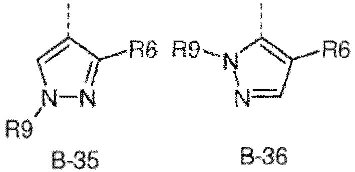 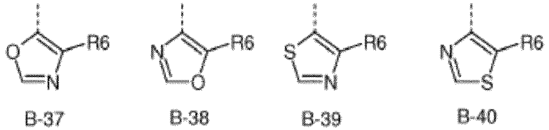 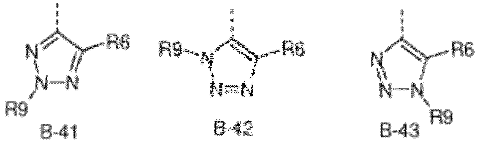 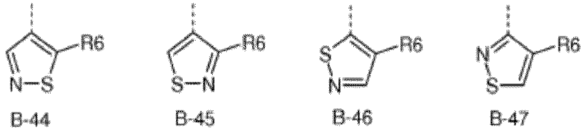 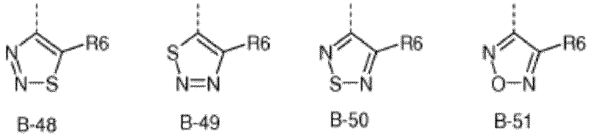 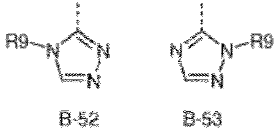 la ligne en pointillés signifiant la liaison à D et B portant en outre n substituants R7,
Y dans le radical Q-4 représente CR8 ou azote,
Y dans le radical Q-5 représente azote,
Z représente oxygène ou soufre,
R1 représente un radical de la série constituée par halogène, cyano, nitro, amino, hydroxy ; alkyle en C₁-C₆, alcényle en C₂-C₆, alcynyle en C₂-C₆, cycloalkyle en C₃-C₆, alcoxy en C₁-C₆, alcényloxy en C₃-C₆, alcynyloxy en C₃-C₆, cycloalkyloxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylamino en C₁-C₆, alcénylamino en C₃-C₆, alcynylamino en C₃-C₆, cycloalkylamino en C₃-C₆, alkylcarbonylamino en C₁-C₆, alcénylcarbonylamino en C₂-C₆, alcynylcarbonylamino en C₂-C₆, cycloalkylcarbonylamino en C₃-C₆, alcoxycarbonylamino en C₁-C₆, alkylsulfonylamino en C₁-C₆, alkylthio en C₁-C₆, alcénylthio en C₃-C₆, alcynylthio en C₃-C₆, cycloalkylthio en C₃-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di-(alkyle en C₁-C₆)-aminocarbonyle, aminothiocarbonyle, alkylaminosulfonyle en C₁-C₆, alkylsulfonylamino en C₁-C₆, alkylcarbonylamino en C₁-C₆, alkylthiocarbonylamino en C₁-C₆, bicycloalkyle en C₄-C₁₂, aryle, aryloxy, arylamino, arylthio, hétéroaryle, hétéroaryloxy, hétéroarylamino et hétéroarylthio, éventuellement substitués une ou plusieurs fois, de manière identique ou différente, les substituants étant choisis indépendamment les uns des autres parmi halogène, cyano, nitro, hydroxy, amino, alkyle en C₁-C₆, alcoxy en C₁-C₆, cycloalkyle en C₃-C₆, halogénoalcoxy en C₁-C₆, alkylthio en C₁-C₆, aryle, aryloxy, arylthio, hétéroaryle, hétéroaryloxy et hétéroarylthio,
R2 représente un radical de la série constituée par halogène, cyano, nitro, amino, hydroxy ; alkyle en C₁-C₆, alcényle en C₂-C₆, alcynyle en C₂-C₆, cycloalkyle en C₃-C₆, alcoxy en C₁-C₆, alcényloxy en C₃-C₆, alcynyloxy en C₃-C₆, cycloalcoxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylamino en C₁-C₆, di- (alkyle en C₁-C₆)-amino, alcénylamino en C₃-C₆, alcynylamino en C₃-C₆, cycloalkylamino en C₃-C₆, alkylcarbonylamino en C₁-C₆, alcénylcarbonylamino en C₂-C₆, alcynylcarbonylamino en C₂-C₆, cycloalkylcarbonylamino en C₃-C₆, alcoxycarbonylamino en C₁-C₆, alkylsulfonylamino en C₁-C₆, alkylthio en C₁-C₆, alcénylthio en C₃-C₆, alcynylthio en C₃-C₆, cycloalkylthio en C₃-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di-(alkyle en C₁-C₆)-aminocarbonyle, aminothiocarbonyle, alkylaminosulfonyle en C₁-C₆, alkylsulfonylamino en C₁-C₆, alkylcarbonylamino en C₁-C₆, alkylthiocarbonylamino en C₁-C₆, bicycloalkyle en C₄-C₁₂, aryle, aryloxy, arylamino, arylthio, hétéroaryle, hétéroaryloxy, hétéroarylamino et hétéroarylthio, éventuellement substitués une ou plusieurs fois, de manière identique ou différente, les substituants étant choisis indépendamment les uns des autres parmi halogène, cyano, nitro, hydroxy, amino, alkyle en C₁-C₆, alcoxy en C₁-C₆, cycloalkyle en C₃-C₆, halogénoalcoxy en C₁-C₆ et alkylthio en C₁-C₆, aryle, aryloxy, arylthio, hétéroaryle, hétéroaryloxy et hétéroarylthio, R3 représente un radical de la série constituée par hydrogène, halogène, cyano, nitro, amino, hydroxy, alkyle en C₁-C₆, alcoxy en C₁-C₆, halogénoalkyle en C₁-C₆ et halogénoalcoxy en C₁-C₆,
R4 représente un radical de la série constituée par hydrogène, halogène, amino, hydroxy, alkyle en C₁-C₆, alcoxy en C₁-C₆, halogénoalkyle en C₁-C₆ et halogénoalcoxy en C₁-C₆,
R5 représente un radical de la série constituée par hydrogène, alkyle en C₁-C₆, cycloalkyle en C₃-C₆, halogénoalkyle en C₁-C₆, alcényle en C₂-C₆, alcynyle en C₃-C₆, cycloalkyle en C₃-C₆-alkyle en C₁-C₆, cyano-alkyle en C₁-C₆, alkylcarbonyle en C₁-C₆, alcénylcarbonyle en C₂-C₆, halogénoalkylcarbonyle en C₁-C₆, halogénoalcénylcarbonyle en C₂-C₆, alcoxy en C₁-C₆-alkyle en C₁-C₆, alcoxycarbonyle en C₁-C₆, alkylsulfonyle en C₁-C₆ et halogénoalkylsulfonyle en C₁-C₆ ou C(=O)-B,
R6 représente un radical de la série constituée par hydrogène (uniquement dans les radicaux B-26, B-33, B-36 et B-42), halogène, cyano, nitro, amino, hydroxy, carboxyle ; alkyle en C₁-C₆, alcényle en C₂-C₆, alcynyle en C₂-C₆, cycloalkyle en C₃-C₆, alcoxy en C₁-C₆, alcényloxy en C₃-C₆, alcynyloxy en C₃-C₆, cycloalcoxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylcarbonylamino en C₁-C₆, alcénylcarbonylamino en C₂-C₆, alcynylcarbonylamino en C₂-C₆, cycloalkylcarbonylamino en C₃-C₆, alcoxycarbonylamino en C₁-C₆, alkylsulfonylamino en C₁-C₆, alkylthio en C₁-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di- (alkyle en C₁-C₆) - aminocarbonyle, aminothiocarbonyle, alkylaminosulfonyle en C₁-C₆, alkylsulfonylamino en C₁-C₆, alkylcarbonylamino en C₁-C₆, halogénoalkylcarbonylamino en C₁-C₆, alkylthiocarbonylamino en C₁-C₆, bicycloalkyle en C₄-C₁₂, aryle, aryloxy, hétéroaryle, hétéroaryloxy, éventuellement substitués une ou plusieurs fois, de manière identique ou différente, les substituants étant choisis indépendamment les uns des autres parmi halogène, cyano, nitro, hydroxy, amino, alkyle en C₁-C₆, alcoxy en C₁-C₆, cycloalkyle en C₃-C₆, halogénoalcoxy en C₁-C₆ et alkylthio en C₁-C₆,
R7 représente un radical de la série constituée par halogène, nitro, cyano, amino, hydroxy ; alkyle en C₁-C₆, alcényle en C₂-C₆, alcynyle en C₂-C₆, cycloalkyle en C₃-C₆, alcoxy en C₁-C₆, alcényloxy en C₃-C₆, alcynyloxy en C₃-C₆, cycloalcoxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylcarbonylamino en C₁-C₆, alcénylcarbonylamino en C₂-C₆, alcynylcarbonylamino en C₂-C₆, cycloalkylcarbonylamino en C₃-C₆, alcoxycarbonylamino en C₁-C₆, alkylsulfonylamino en C₁-C₆, alkylthio en C₁-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di- (alkyle en C₁-C₆) - aminocarbonyle, aminothiocarbonyle, alkylaminosulfonyle en C₁-C₆, alkylsulfonylamino en C₁-C₆, alkylcarbonylamino en C₁-C₆, alkylthiocarbonylamino en C₁-C₆, aryle, aryloxy, hétéroaryle, hétéroaryloxy, bicycloalkyle en C₄-C₁₂, éventuellement substitués une ou plusieurs fois, de manière identique ou différente, les substituants étant choisis indépendamment les uns des autres parmi halogène, cyano, nitro, hydroxy, amino, alkyle en C₁-C₆, alcoxy en C₁-C₆, cycloalkyle en C₃-C₆, halogénoalcoxy en C₁-C₆ et alkylthio en C₁-C₆,
R8 représente un radical de la série constituée par hydrogène, halogène, amino, hydroxy, alkyle en C₁-C₆, alcoxy en C₁-C₆, halogénoalkyle en C₁-C₆ et halogénoalcoxy en C₁-C₆,
R9 représente un radical de la série constituée par hydrogène, alkyle en C₁-C₆, cycloalkyle en C₃-C₆, halogénoalkyle, alcényle en C₃-C₆, alcynyle en C₃-C₆, cycloalkyle en C₃-C₆-alkyle en C₁-C₆, cyano-alkyle en C₁-C₆, alkylcarbonyle en C₁-C₆, alcénylcarbonyle en C₂-C₆, halogénoalkylcarbonyle en C₁-C₆, halogénoalcénylcarbonyle en C₂-C₆, alcoxy en C₁-C₆-alkyle en C₁-C₆, alcoxycarbonyle en C₁-C₆, alkylsulfonyle en C₁-C₆ et halogénoalkylsulfonyle en C₁-C₆,
m représente un nombre de la série constituée par 0, 1, 2 et 3, pour m > 1, les radicaux R2 pouvant être identiques ou différents, et
n représente un nombre de la série constituée par 0, 1, 2 et 3, pour n > 1, les radicaux R7 pouvant être identiques ou différents,
pour lutter contre des animaux nuisibles.

2. Utilisation non thérapeutique de composés de formule (I) selon la revendication 1, dans laquelle
A représente un radical de la série 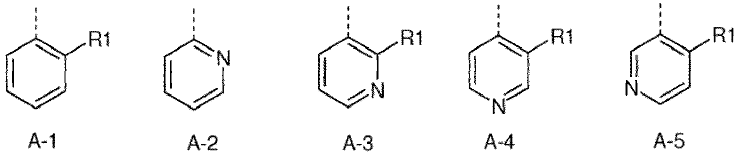 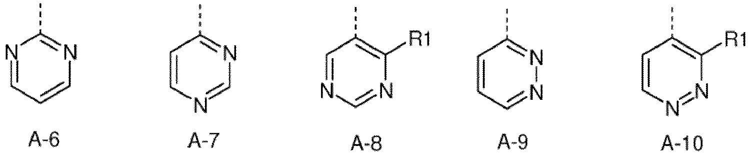 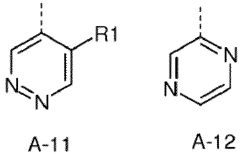 la ligne en pointillés signifiant la liaison à Q et A portant en outre m substituants R2,
Q représente un radical de la série 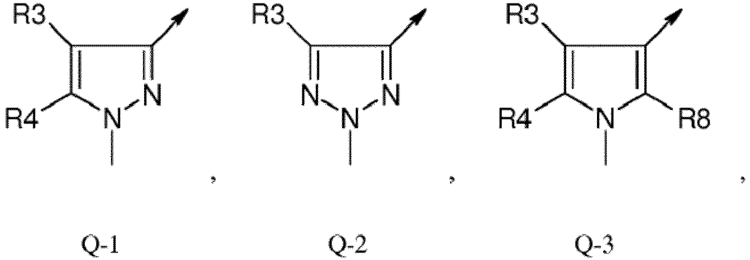 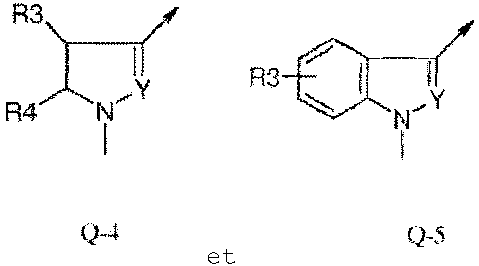 l'azote étant relié avec le cycle A et la flèche signifiant à chaque fois la liaison à D,
D représente un radical de formule 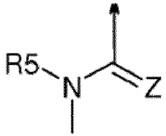 dans laquelle l'azote est relié avec Q et la flèche signifie la liaison à B,
B représente un radical de la série 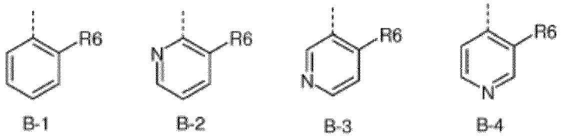 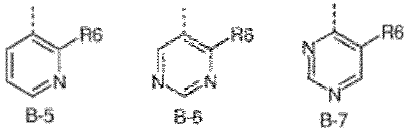 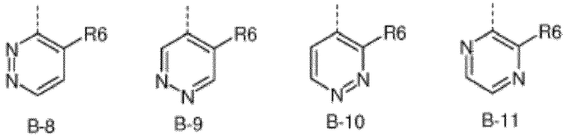 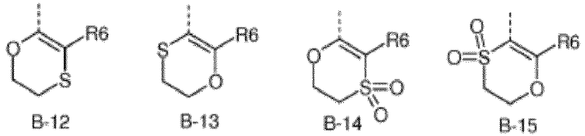 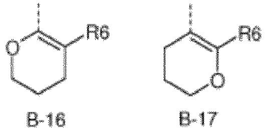 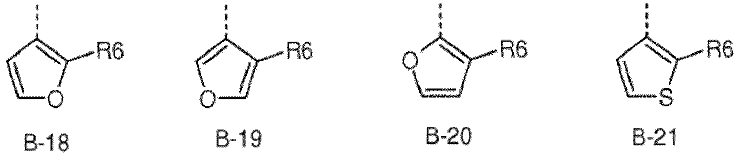 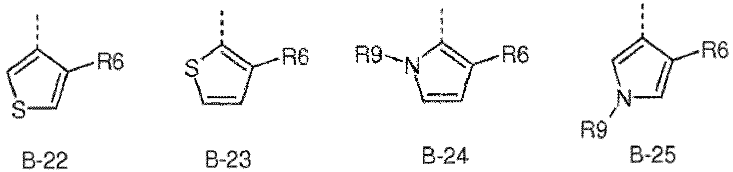 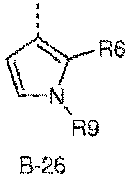 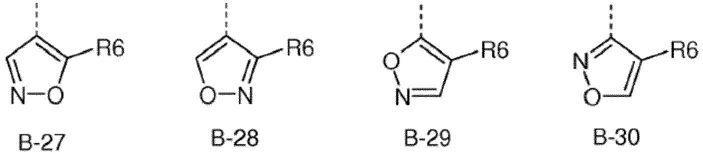 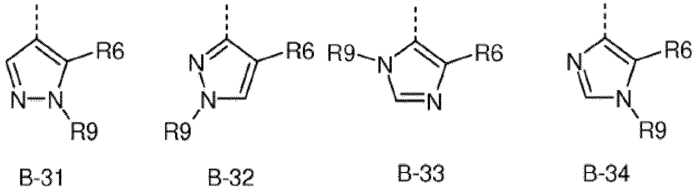 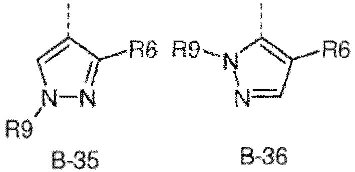 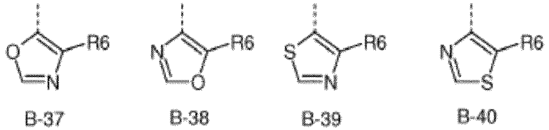 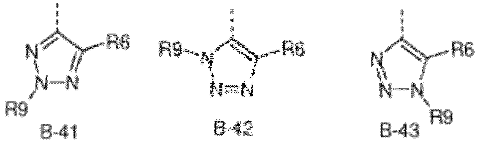 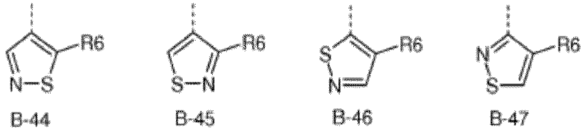 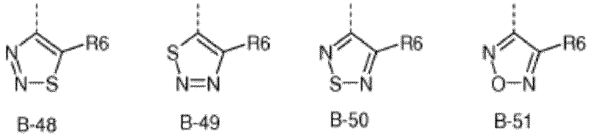 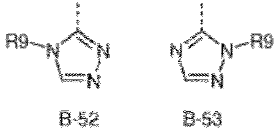 la ligne en pointillés signifiant la liaison à D et B portant en outre n substituants R7,
Y dans le radical Q-4 représente CR8 ou azote,
Y dans le radical Q-5 représente azote,
Z représente oxygène ou soufre,
R1 représente un radical de la série constituée par halogène, cyano, nitro, amino, hydroxy, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylamino en C₁-C₆, alcénylamino en C₃-C₆, alcynylamino en C₃-C₆, cycloalkylamino en C₃-C₆, alkylthio en C₁-C₆, halogénoalkylthio en C₁-C₆, alcénylthio en C₃-C₆, alcynylthio en C₃-C₆, cycloalkylthio en C₃-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di- (alkyle en C₁-C₆)-aminocarbonyle, aminothiocarbonyle, alkylaminosulfonyle en C₁-C₆, alkylsulfonylamino en C₁-C₆, alkylcarbonylamino en C₁-C₆, alkylthiocarbonylamino en C₁-C₆, aryle, aryloxy, hétéroaryle et hétéroaryloxy,
R2 représente un radical de la série constituée par halogène, cyano, nitro, amino, hydroxy, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylamino en C₁-C₆, di- (alkyle en C₁-C₆)-amino, alcénylamino en C₃-C₆, alcynylamino en C₃-C₆, cycloalkylamino en C₃-C₆, alkylthio en C₁-C₆, halogénoalkylthio en C₁-C₆, alcénylthio en C₃-C₆, alcynylthio en C₃-C₆, cycloalkylthio en C₃-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di-(alkyle en C₁-C₆)-aminocarbonyle, aminothiocarbonyle, alkylaminosulfonyle en C₁-C₆, alkylsulfonylamino en C₁-C₆, alkylcarbonylamino en C₁-C₆, alkylthiocarbonylamino en C₁-C₆, aryle, aryloxy, hétéroaryle et hétéroaryloxy,
R3 représente un radical de la série constituée par hydrogène, halogène, cyano, nitro, amino, alkyle en C₁-C₆ et halogénoalkyle en C₁-C₆,
R4 représente un radical de la série constituée par hydrogène, halogène, amino, hydroxy, alkyle en C₁-C₆, alcoxy en C₁-C₆, halogénoalkyle en C₁-C₆ et halogénoalcoxy en C₁-C₆,
R5 représente un radical de la série constituée par hydrogène, alkyle en C₁-C₆, halogénoalkyle en C₁-C₆, cyano-alkyle en C₁-C₆, alcényle en C₂-C₆, alcynyle en C₃-C₆, cycloalkyle en C₃-C₆, cycloalkyle en C₃-C₆-alkyle en C₁-C₆, alkylcarbonyle en C₁-C₆, alcénylcarbonyle en C₂-C₆, halogénoalkylcarbonyle en C₁-C₆, halogénoalcénylcarbonyle en C₂-C₆, alcoxy en C₁-C₆-alkyle en C₁-C₆, alcoxycarbonyle en C₁-C₆, alkylsulfonyle en C₁-C₆, halogénoalkylsulfonyle en C₁-C₆ et C(=O)-B,
R6 représente un radical de la série constituée par hydrogène (uniquement dans les radicaux B-26, B-33, B-36 et B-42), halogène, cyano, nitro, amino, hydroxy, carboxyle, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylamino en C₁-C₆, alcénylamino en C₃-C₆, alcynylamino en C₃-C₆, cycloalkylamino en C₃-C₆, alkylthio en C₁-C₆, halogénoalkylthio en C₁-C₆, alcénylthio en C₃-C₆, alcynylthio en C₃-C₆, cycloalkylthio en C₃-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di- (alkyle en C₁-C₆) - aminocarbonyle, aminothiocarbonyle, alkylaminosulfonyle en C₁-C₆, alkylsulfonylamino en C₁-C₆, alkylcarbonylamino en C₁-C₆, halogénoalkylcarbonylamino en C₁-C₄, alkylthiocarbonylamino en C₁-C₆, aryle, aryloxy, hétéroaryle et hétéroaryloxy,
R7 représente un radical de la série constituée par halogène, cyano, nitro, amino, hydroxy, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylcarbonyloxy en C₁-C₆, alcénylcarbonyloxy en C₂-C₆, alcynylcarbonyloxy en C₂-C₆, cycloalkylcarbonyloxy en C₃-C₆, alcoxycarbonyloxy en C₁-C₆, alkylsulfonyloxy en C₁-C₆, alkylamino en C₁-C₆, alcénylamino en C₃-C₆, alcynylamino en C₃-C₆, cycloalkylamino en C₃-C₆, alkylthio en C₁-C₆, halogénoalkylthio en C₁-C₆, alcénylthio en C₃-C₆, alcynylthio en C₃-C₆, cycloalkylthio en C₃-C₆, alkylsulfinyle en C₁-C₆, alkylsulfonyle en C₁-C₆, alkylcarbonyle en C₁-C₆, alcoxyimino en C₁-C₆-alkyle en C₁-C₆, alcoxycarbonyle en C₁-C₆, aminocarbonyle, alkylaminocarbonyle en C₁-C₆, di- (alkyle en C₁-C₆)-aminocarbonyle, aminothiocarbonyle, alkylaminosulfonyle en C₁-C₆, alkylsulfonylamino en C₁-C₆, alkylcarbonylamino en C₁-C₆, alkylthiocarbonylamino en C₁-C₆, aryle, aryloxy, hétéroaryle et hétéroaryloxy,
R8 représente un radical de la série constituée par hydrogène, halogène, amino, hydroxy, alkyle en C₁-C₆, alcoxy en C₁-C₆, halogénoalkyle en C₁-C₆ et halogénoalcoxy en C₁-C₆,
R9 représente un radical de la série constituée par hydrogène, alkyle en C₁-C₆, cycloalkyle en C₃-C₆, halogénoalkyle en C₁-C₆, alcényle en C₃-C₆, alcynyle en C₃-C₆, cycloalkyle en C₃-C₆-alkyle en C₁-C₆, cyano-alkyle en C₁-C₆, alkylcarbonyle en C₁-C₆, alcénylcarbonyle en C₂-C₆, halogénoalkylcarbonyle en C₁-C₆, halogénoalcénylcarbonyle en C₂-C₆, alcoxy en C₁-C₆-alkyle en C₁-C₆, alcoxycarbonyle en C₁-C₆, alkylsulfonyle en C₁-C₆ et halogénoalkylsulfonyle en C₁-C₆,
m représente un nombre de la série constituée par 0, 1, 2 et 3, pour m > 1, les radicaux R2 pouvant être identiques ou différents, et
n représente un nombre de la série constituée par 0, 1, 2 et 3, pour n > 1, les radicaux R7 pouvant être identiques ou différents.

3. Utilisation non thérapeutique de composés de formule (I) selon la revendication 1, dans laquelle
A représente un radical de la série 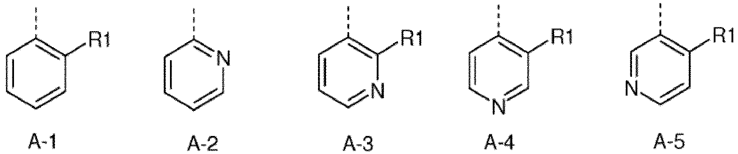 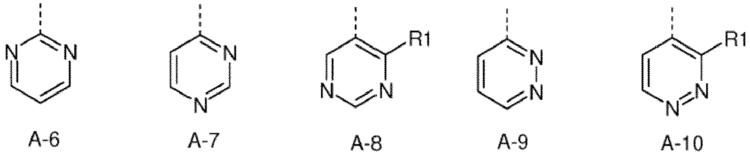 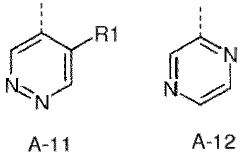 la ligne en pointillés signifiant la liaison à Q et A portant en outre m substituants R2,
Q représente un radical de la série 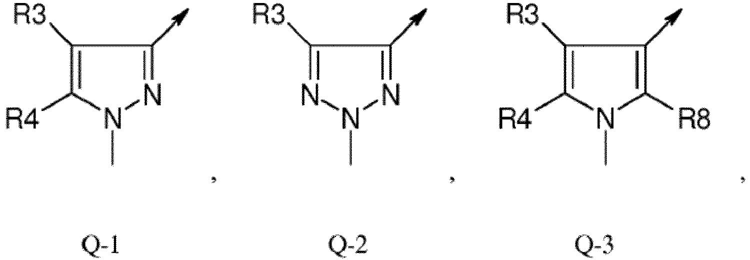 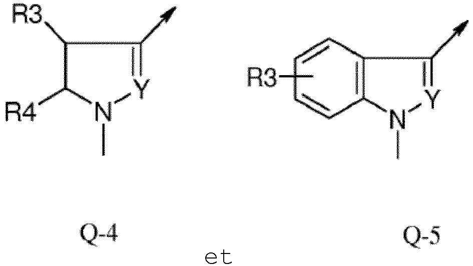 l'azote étant relié avec le cycle A et la flèche signifiant à chaque fois la liaison à D,
D représente un radical de formule 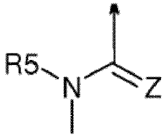 dans laquelle l'azote est relié avec Q et la flèche signifie la liaison à B,
B représente un radical de la série 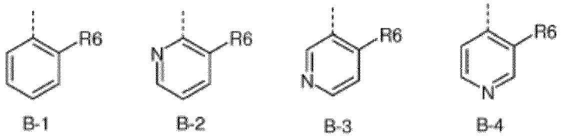 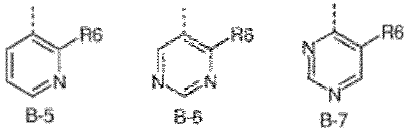 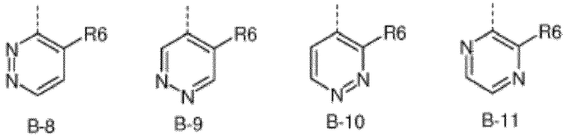 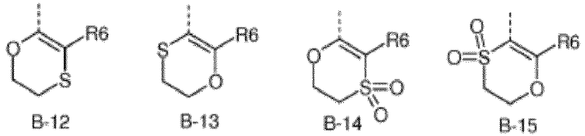 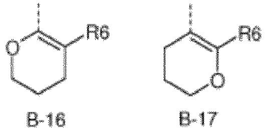 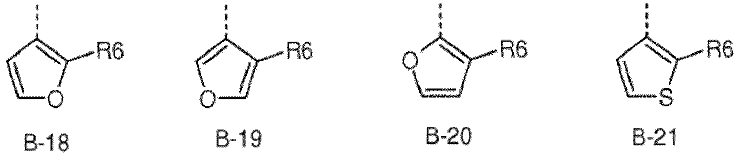 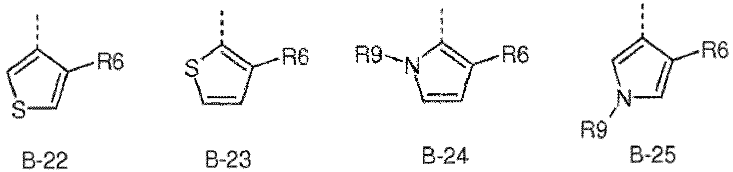 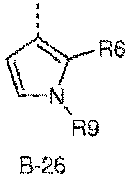 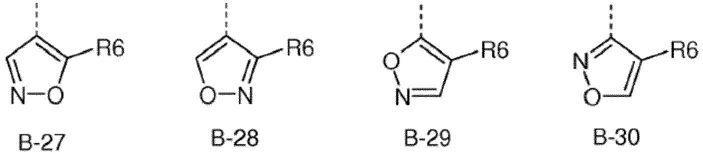 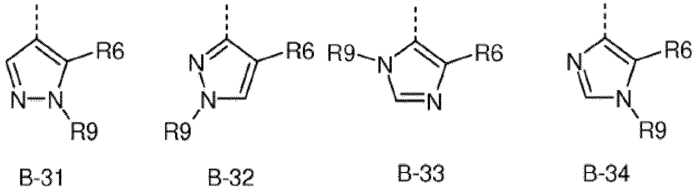 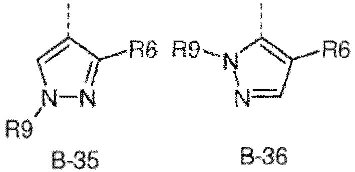 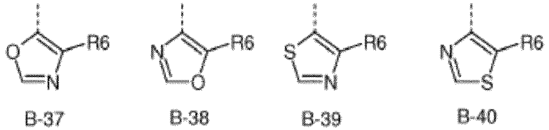 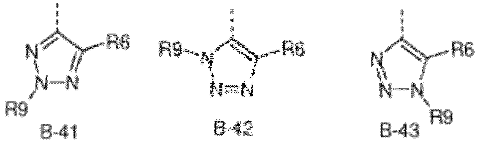 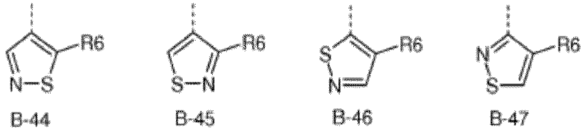 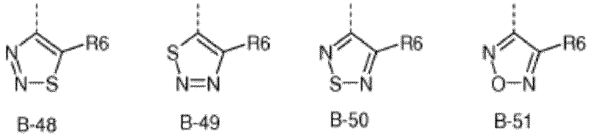 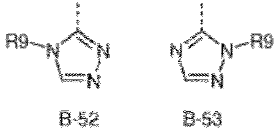 la ligne en pointillés signifiant la liaison à D et B portant en outre n substituants R7,
Y dans le radical Q-4 représente CR8 ou azote,
Y dans le radical Q-5 représente azote,
Z représente oxygène ou soufre,
R1 représente un radical de la série constituée par halogène, cyano, nitro, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylthio en C₁-C₆, halogénoalkylthio en C₁-C₆, alkylsulfinyle en C₁-C₆, alkylsulfonyle en C₁-C₆, alcoxyimino en C₁-C₆-alkyle en C₁-C₆, aryle, aryloxy, hétéroaryle et hétéroaryloxy,
R2 représente un radical de la série constituée par halogène, cyano, nitro, amino, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylthio en C₁-C₆, halogénoalkylthio en C₁-C₆, alkylsulfinyle en C₁-C₆, alkylsulfonyle en C₁-C₆, alkylcarbonyle en C₁-C₆, di-(alkyle en C₁-C₆)-amino, alcoxyimino en C₁-C₆-alkyle en C₁-C₆, aryle, aryloxy, hétéroaryle et hétéroaryloxy,
R3 représente un radical de la série constituée par hydrogène, halogène, cyano, nitro, amino, alkyle en C₁-C₆ et halogénoalkyle en C₁-C₆,
R4 représente un radical de la série constituée par hydrogène, halogène, amino, hydroxy, alkyle en C₁-C₆, alcoxy en C₁-C₆, halogénoalkyle en C₁-C₆ et halogénoalcoxy en C₁-C₆,
R5 représente un radical de la série constituée par hydrogène, alkyle en C₁-C₆, halogénoalkyle en C₁-C₆, cyano-alkyle en C₁-C₆, alcényle en C₂-C₆, alcynyle en C₃-C₆, cycloalkyle en C₃-C₆, cycloalkyle en C₃-C₆-alkyle en C₁-C₆, alkylcarbonyle en C₁-C₆, alcénylcarbonyle en C₂-C₆, halogénoalkylcarbonyle en C₁-C₆, halogénoalcénylcarbonyle en C₂-C₆, alcoxy en C₁-C₆-alkyle en C₁-C₆, alcoxycarbonyle en C₁-C₆, alkylsulfonyle en C₁-C₆, halogénoalkylsulfonyle en C₁-C₆ et C(=O)-B,
R6 représente un radical de la série constituée par hydrogène (uniquement dans les radicaux B-26, B-33, B-36 et B-42), halogène, cyano, nitro, hydroxy, carboxyle, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylsulfinyle en C₁-C₆, alkylthio en C₁-C₆, halogénoalkylthio en C₁-C₆, alkylsulfonyle en C₁-C₆, alkylcarbonyle en C₁-C₆, alcoxyimino en C₁-C₆-alkyle en C₁-C₆, alkylcarbonylamino en C₁-C₆, halogénoalkylcarbonylamino en C₁-C₆, aryle, aryloxy, hétéroaryle et hétéroaryloxy,
R7 représente un radical de la série constituée par halogène, cyano, nitro, hydroxy, alkyle en C₁-C₆, halogénoalkyle en C₁-C₆, cyano-alkyle en C₁-C₆, alcényle en C₂-C₆, alcynyle en C₂-C₆, cycloalkyle en C₃-C₆, cycloalkyle en C₃-C₆-alkyle en C₁-C₆, alcoxy en C₁-C₆, halogénoalcoxy en C₁-C₆, alcényloxy en C₃-C₆, alcynyloxy en C₃-C₆, cycloalkyloxy en C₃-C₆, alkylthio en C₁-C₆, halogénoalkylthio en C₁-C₆, alkylsulfinyle en C₁-C₆, alkylsulfonyle en C₁-C₆, alcoxyimino en C₁-C₆-alkyle en C₁-C₆, aryle, aryloxy, hétéroaryle et hétéroaryloxy,
R8 représente un radical de la série constituée par hydrogène, alkyle en C₁-C₆, alcoxy en C₁-C₆, halogénoalkyle en C₁-C₆ et halogénoalcoxy en C₁-C₆ R9 représente un radical de la série constituée par hydrogène, alkyle en C₁-C₆, cycloalkyle en C₃-C₆ et halogénoalkyle en C₁-C₆,
m représente un nombre de la série constituée par 0, 1, 2 et 3, pour m > 1, les radicaux R2 pouvant être identiques ou différents, et
n représente un nombre de la série constituée par 0, 1, 2 et 3, pour n > 1, les radicaux R7 pouvant être identiques ou différents.

4. Utilisation non thérapeutique de composés de formule (I) selon la revendication 1, dans laquelle
A représente un radical de la série 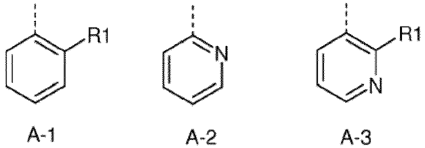 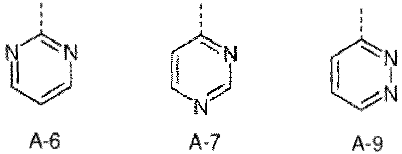 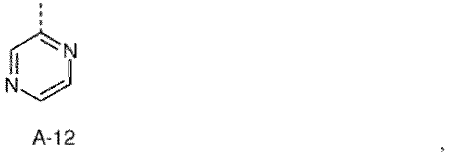 la ligne en pointillés signifiant la liaison à Q et A portant en outre m substituants R2,
Q représente un radical de la série 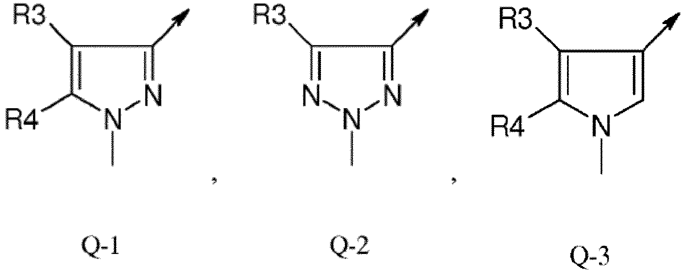 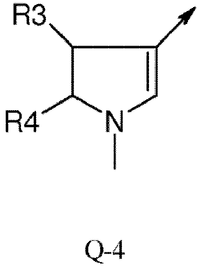 et
l'azote étant relié avec le cycle A et la flèche signifiant à chaque fois la liaison à D,
D représente un radical de formule 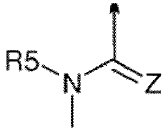 dans laquelle l'azote est relié avec Q et la flèche signifie la liaison à B,
B représente un radical de la série 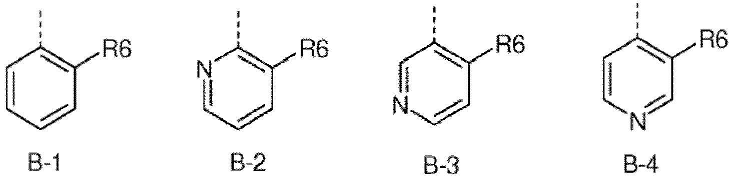 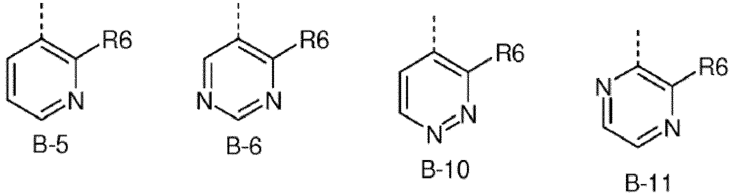 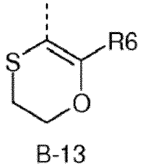 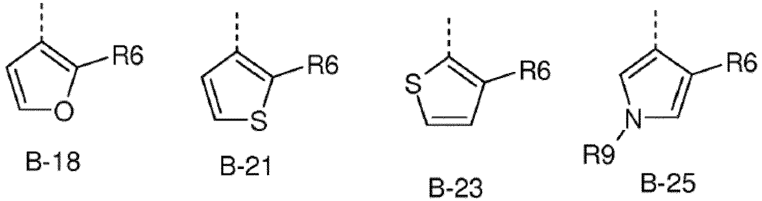 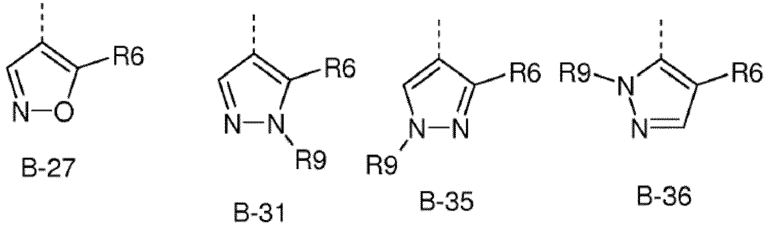 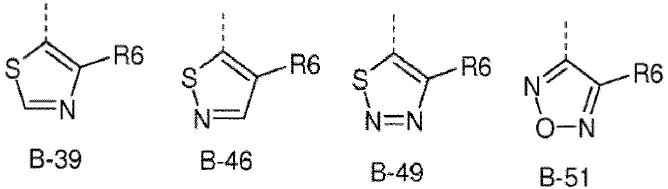 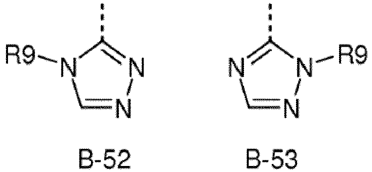 la ligne en pointillés signifiant la liaison à D et B portant en outre n substituants R7,
Z représente oxygène ou soufre,
R1 représente un radical de la série constituée par halogène, cyano, nitro, alkyle en C₁-C₄, halogénoalkyle en C₁-C₄, alcoxy en C₁-C₄ et alkylsulfonyle en C₁-C₄,
R2 représente un radical de la série constituée par halogène, alkyle en C₁-C₄ et alcoxy en C₁-C₄,
R3 représente un radical de la série constituée par hydrogène et halogène,
R4 représente un radical de la série constituée par hydrogène et alkyle en C₁-C₄,
R5 représente un radical de la série constituée par hydrogène, alkyle en C₁-C₄, alcényle en C₃-C₄, alcynyle en C₃-C₄, cycloalkyle en C₃-C₆-alkyle en C₁-C₄, alkylcarbonyle en C₁-C₄, cyano-alkyle en C₁-C₄ et C(=O)-B,
R6 représente un radical de la série constituée par halogène, nitro, hydroxy, alkyle en C₁-C₄, alcoxy en C₁-C₄, alkylthio en C₁-C₄, alkylsulfonyle en C₁-C₄, halogénoalkyle en C₁-C₄, halogénoalcoxy en C₁-C₄, halogénoalkylthio en C₁-C₄ et hétéroaryle,
R7 représente un radical de la série constituée par halogène, alkyle en C₁-C₄ et halogénoalkyle en C₁-C₄,
R9 représente un radical de la série constituée par alkyle en C₁-C₄,
m représente un nombre de la série constituée par 0, 1, 2 et 3, pour m > 1, les radicaux R2 pouvant être identiques ou différents, et
n représente un nombre de la série constituée par 0 et 1.

5. Utilisation non thérapeutique de composés de formule (I) selon la revendication 1, dans laquelle
A représente un radical de la série 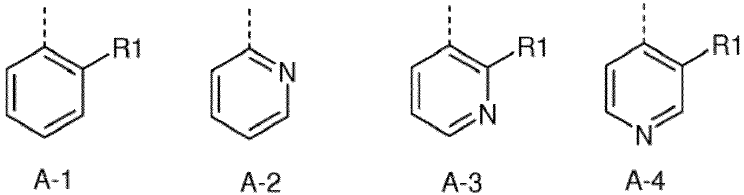 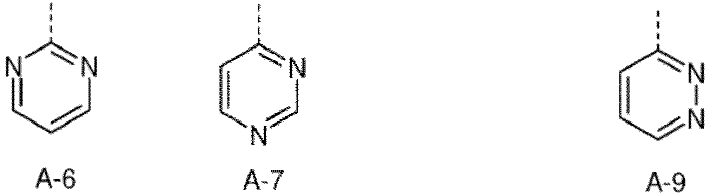 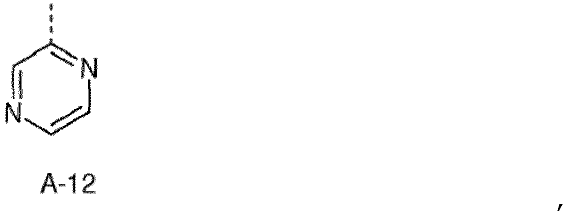 la ligne en pointillés signifiant la liaison à Q et A portant en outre m substituants R2,
Q représente un radical de la série 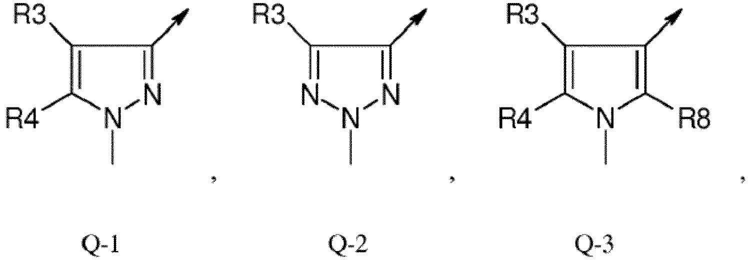 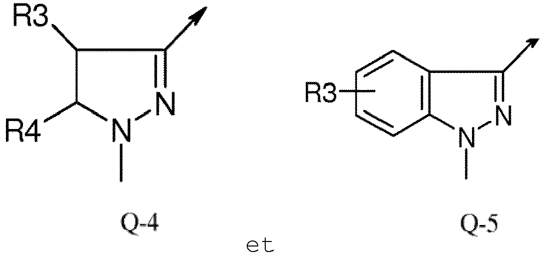 l'azote étant relié avec le cycle A et la flèche signifiant à chaque fois la liaison à D,
D représente un radical de formule 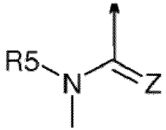 dans laquelle l'azote est relié avec Q et la flèche signifie la liaison à B,
B représente un radical de la série 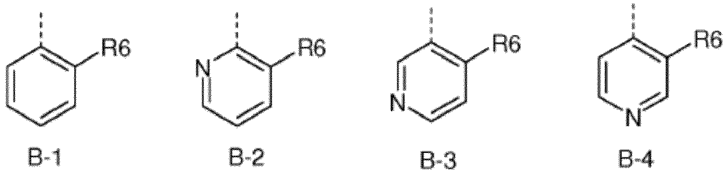 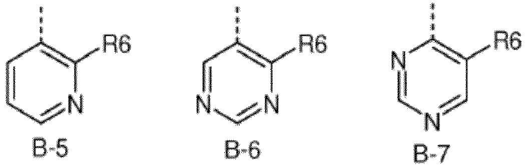 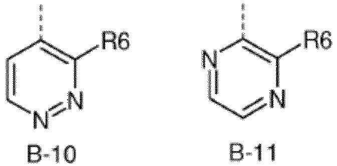 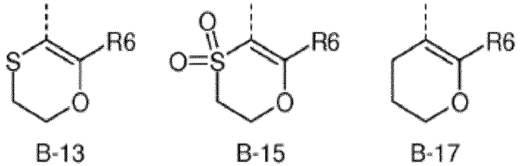 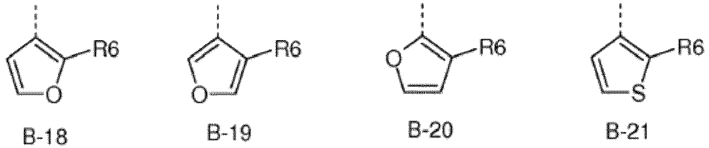 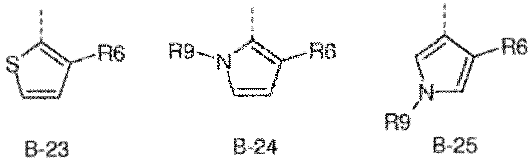 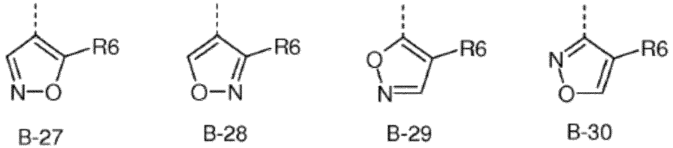 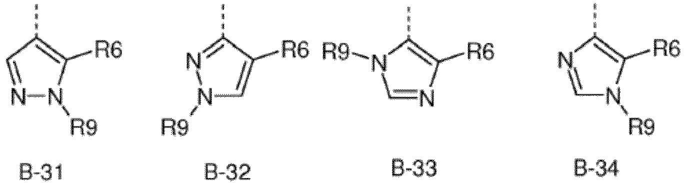 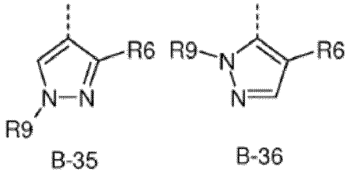 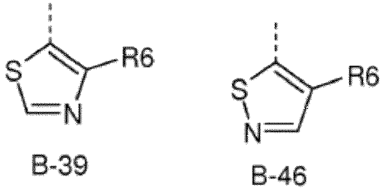 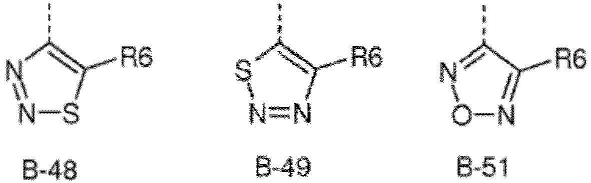 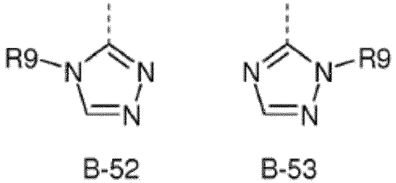 la ligne en pointillés signifiant la liaison à D et B portant en outre n substituants R7,
Z représente oxygène ou soufre,
R1 représente un radical de la série constituée par halogène, cyano, nitro, alkyle en C₁-C₄, halogénoalkyle en C₁-C₄, alcoxy en C₁-C₄ et alkylsulfonyle en C₁-C₄,
R2 représente un radical de la série constituée par halogène, cyano, nitro, amino, alkyle en C₁-C₄, halogénoalkyle en C₁-C₄, cycloalkyle en C₃-C₆, alcoxy en C₁-C₄, di- (alkyle en C₁-C₆)-amino, alkylcarbonyle en C₁-C₄, aryle,
R3 représente un radical de la série constituée par hydrogène, halogène, cyano, nitro, alkyle en C₁-C₄,
R4 représente un radical de la série constituée par hydrogène, amino et alkyle en C₁-C₄,
R5 représente un radical de la série constituée par hydrogène, alkyle en C₁-C₄, alcényle en C₃-C₄, alcynyle en C₃-C₄, cycloalkyle en C₃-C₆-alkyle en C₁-C₄, alkylcarbonyle en C₁-C₄, cyano-alkyle en C₁-C₄ et C(=O)-B,
R6 représente un radical de la série constituée par hydrogène (uniquement dans les radicaux B-33 et B-36), halogène, cyano, nitro, hydroxy, carboxyle, alkyle en C₁-C₄, alcoxy en C₁-C₄, alkylthio en C₁-C₄, alkylsulfonyle en C₁-C₄, halogénoalkyle en C₁-C₄, cycloalkyle en C₃-C₆, halogénoalcoxy en C₁-C₄, halogénoalkylthio en C₁-C₄, alkylcarbonyle en C₁-C₄, alkylcarbonylamino en C₁-C₄, halogénoalkylcarbonylamino en C₁-C₄ et hétéroaryle,
R7 représente un radical de la série constituée par halogène, cyano, nitro, hydroxy, alkyle en C₁-C₄, halogénoalkyle en C₁-C₄ et alcoxy en C₁-C₄,
R9 représente un radical de la série constituée par alkyle en C₁-C₄ et halogénoalkyle en C₁-C₄,
m représente un nombre de la série constituée par 0, 1, 2 et 3, pour m > 1, les radicaux R2 pouvant être identiques ou différents, et
n représente un nombre de la série constituée par 0, 1, 2 et 3, pour n > 1, les radicaux R7 pouvant être identiques ou différents.

6. Composés de formule (I) tels que décrits dans l'une quelconque des revendications 1 à 5, à l'exception des composés suivantes : 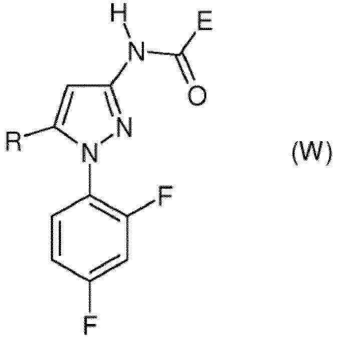
| No | R | E |
|---|---|---|
| W-1 | éthyle | 2-(trifluorométhyl)phényle |
| W-2 | éthyle | 2-bromophényle |
| W-3 | éthyle | 2-fluorophényle |
| W-4 | éthyle | 3-méthyl-2-thiényle |
| W-5 | éthyle | 2,5-diméthyl-3-furanyle |
| W-6 | H | 2,4-diméthoxyphényle |
| W-7 | H | 2-(trifluorométhyl)phényle |
| W-8 | H | 2,4-difluorophényle |
| W-9 | méthyle | 2-bromophényle |
| W-10 | éthyle | 2,4-difluorophényle |
| W-11 | H | 2,5-diméthyl-3-furanyle |
| W-12 | méthyle | 2,4-diméthoxyphényle |

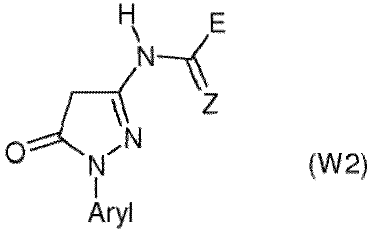
| No | Z | E | Aryle |
|---|---|---|---|
| W2-1 | O | 2-nitrophényle | 3,4-dichloro-2,5-diméthoxyphényle |
| W2-2 | S | 2,4-dihydroxyphényle | 2,4,6-trichlorophényle |
| W2-3 | O | 2-chloro-4-aminophényle | 2,4,6-trichlorophényle |
| W2-5 | O | 2-chloro-5-aminophényle | 2,4,6-trichlorophényle |
| W2-6 | O | 2-chloro-5-nitrophényle | 2,4,6-trichlorophényle |
| W2-7 | O | 2-chloro-3-(1-oxo-2-propén-1-yl)amino | 2,4,6-trichlorophényle |
| W2-8 | O | 2-chloro-5-(2-méthyl-1-oxo-2-propényl)amino | 2,4,6-trichlorophényle |
| W2-9 | O | 2-chloro-5-(1-oxo-2-propén-1-yl)amino | 2,4,6-trichlorophényle |

7. Composé de formule (I) tel que décrit dans la revendication 1, choisi dans la liste suivante :
| N° | A | Q | B | R 3 | R 4 | R 5 | Z |
|---|---|---|---|---|---|---|---|
| 1 | 2-(trifluorométhyl) phényle | Q-1 | 2-(trifluorométhyl) phényle | H | H | H | O |
| 2 | 2-chlorophényle | Q-1 | 2-(trifluorométhyl) phényle | H | H | H | O |
| 3 | 2-bromophényle | Q-1 | 2-(trifluorométhyl) phényle | H | H | H | O |
| 4 | 2-bromophényle | Q-1 | 2-iodophényle | H | H | H | O |
| 5 | 2,5-difluorophényle | Q-1 | 2-(trifluorométhyl) phényle | H | H | H | O |
| 6 | 2,6-difluorophényle | Q-1 | 2-méthylphényle | H | H | H | O |
| 7 | 2,6-difluorophényle | Q-1 | 2-difluorométhylphényle | H | H | H | O |
| 8 | 2,6-difluorophényle | Q-1 | 2-(trifluorométhyl) phényle | H | H | H | O |
| 9 | 2,6-difluorophényle | Q-1 | 2-(trifluorométhyl) phényle | F | H | H | O |
| 10 | 2,6-difluorophényle | Q-1 | 2-chlorophényle | H | H | H | O |
| 11 | 2,6-difluorophényle | Q-1 | 2-bromophényle | H | H | H | O |
| 12 | 2,6-difluorophényle | Q-1 | 2-iodophényle | H | H | H | O |
| 13 | 2,6-difluorophényle | Q-1 | 2,6-difluorophényle | H | H | H | O |
| 14 | 2-éthoxy-6-fluorophényle | Q-1 | 2-chlorophényle | H | H | H | O |
| 15 | 3,5-difluoropyridin-2-yle | Q-1 | 2-(trifluorométhyl) phényle | H | H | H | O |
| 16 | 3-chloro-5-fluoropyridin-2-yle | Q-1 | 2-(trifluorométhyl) phényle | H | H | H | O |
| 17 | 2,6-difluorophényle | Q-1 | 2-fluoro-6-(trifluorométhyl) phényle | H | H | H | O |
| 18 | 2,6-difluorophényle | Q-1 | 4-fluoro-2-(trifluorométhyl) phényle | H | H | H | O |
| 19 | 2-bromophényle | Q-1 | 2-fluoro-6-(trifluorométhyl) phényle | H | H | H | O |
| 20 | 2-cyano-4,6-difluorophényle | Q-1 | 2-(trifluorométhyl) phényle | H | H | H | O |
| 21 | 2-cyanophényle | Q-1 | 2-iodophényle | H | H | H | O |
| 22 | 3,5-difluoropyridin-2-yle | Q-1 | 2-(trifluorométhyl) phényle | F | H | H | O |
| 23 | 3-cyanopyridin-2-yle | Q-1 | 2-(trifluorométhyl) phényle | H | H | H | O |
| 24 | 2-cyano-4-fluorophényle | Q-1 | 2-(trifluorométhyl) phényle | H | H | H | O |
| 25 | 2,6-difluorophényle | Q-2 | 2-(trifluorométhyl) phényle | H | H- | H | O |

8. Agent, **caractérisé par** une teneur en au moins un composé de formule (I) selon la revendication 6 ou 7.

9. Procédé non thérapeutique de lutte contre des nuisibles, **caractérisé en ce qu'**un composé de formule (I) tel que décrit dans les revendications 1 à 5 ou selon la revendication 6 ou 7 ou un agent selon la revendication 8 est laissé agir sur les nuisibles et/ou leur habitat.

10. Procédé selon la revendication 9, **caractérisé en ce que** les nuisibles sont des insectes, des araignées et des nématodes.

11. Composés de formule (I) tels que décrits dans les revendications 1 à 5 ou selon la revendication 6 ou 7, destinés à une utilisation en tant qu'anti-endoparasitique.

12. Composés de formule (I) tels que décrits dans les revendications 1 à 5 ou selon la revendication 6 ou 7, destinés à une utilisation en tant qu'anti-ectoparasitique.

13. 2-(3,5-Difluoropyridin-2-yl)-2H-1,2,3-triazol-4-amine.

14. 1-(3,5-Difluoropyridin-2-yl)-1H-pyrazol-3-amine.

15. N-[1-(2,6-Difluorophényl)-4,5-dihydro-1H-pyrazol-3-yl]acétamide.

16. N-[1-(2,6-Difluorophényl)-1H-pyrazol-3-yl]acétamide.

17. 1-(2,6-Difluorophényl)-1H-pyrazol-3-amine.

18. 1-(2,6-Difluorophényl)-4,5-dihydro-1H-pyrazol-3-amine.
